# EUROPEAN PATENT SPECIFICATION

(11) **EP 3 443 001 B1**
(45) Date of publication and mention of the grant of the patent: **30.04.2025**
(21) Application number: 17782955.3
(22) Date of filing: 11.04.2017
(51) Int. Cl.: C07K 14/705, C07K 19/00, C12N 5/0783, C12N 5/10, C12N 15/62

(54) **REGULATED BIOCIRCUIT SYSTEMS**
REGULIERTE BIOSCHALTKREISSYSTEME
SYSTÈMES DE BIOCIRCUITS RÉGULÉS

(30) Priority: 11.04.2016 US 201662320864 P; 03.03.2017 US 201762466596 P
(43) Date of publication of application: 20.02.2019
(73) Proprietor: Obsidian Therapeutics, Inc., Cambridge, MA 02138 (US)
(72) Inventor: BARRETT, Peter, Cambridge, MA 02139 (US); GLADSTONE, Michael, N., Cambridge, MA 02139 (US); KASSUM, Tariq, A., Cambridge, MA 02138-5335 (US); SURI, Vipin, Belmont, MA 02478 (US); LI, Dan, Jun, Cambridge, MA 02138-5335 (US); SUN, Dexue, Cambridge, MA 02138-5335 (US); DOLINSKI, Brian, Cambridge, MA 02138-5335 (US)
(74) Representative: Hoffmann Eitle
(86) International application number: PCT/US2017/026950
(87) International publication number: WO 2017/180587

(56) References cited:
- WO-A1-2015/150771
- WO-A1-2016/012623
- WO-A2-2015/142675
- US-A1- 2012 178 168
- US-A1- 2014 010 791
- CHU B W ET AL: "Recent progress with FKBP-derived destabilizing domains", BIOORGANIC & MEDICINAL CHEMISTRY LETTERS, PERGAMON, AMSTERDAM, NL, vol. 18, no. 22, 15 November 2008 (2008-11-15), pages 5941 - 5944, XP025627175, ISSN: 0960-894X, [retrieved on 20080912], DOI: 10.1016/J.BMCL.2008.09.043
- LIANG-CHUAN WANG ET AL: "Overcoming intrinsic inhibitory pathways to augment the antineoplastic activity of adoptively transferred T cells: Re-tuning your CAR before hitting a rocky road", ONCOIMMUNOLOGY, vol. 2, no. 11, 1 November 2013 (2013-11-01), pages e26492, XP055191167, DOI: 10.4161/onci.26492

## Description

### FIELD OF THE INVENTION

The invention relates to regulatable and tunable biocircuit systems for the development of controlled and/or regulated therapeutic systems.

### BACKGROUND OF THE INVENTION

Gene therapy is revolutionizing medicine and offering new promise for the treatment of previously intractable conditions. However, current technologies do not allow titration of the timing or levels of target protein induction. This has rendered many potential gene therapy applications difficult or impossible to safely and effectively deploy.

Inadequate exogenous and/or endogenous gene control is a critical issue in numerous gene therapy settings. This lack of tunability also makes it difficult to safely express proteins with narrow or uncertain therapeutic windows or those requiring more titrated or transient expression.

One approach to regulated protein expression or function is the use of Destabilizing Domains (DDs). Destabilizing domains are small protein domains that can be appended to a target protein of interest. DDs render the attached protein of interest unstable in the absence of a DD-binding ligand such that the protein is rapidly degraded by the ubiquitin-proteasome system of the cell (Stankunas, K., et al., (2003). Mol. Cell 12, 1615-1624; Banaszynski, et al., (2006) Cell; 126(5): 995-1004; reviewed in Banaszynski, L.A., and Wandless, T.J. (2006) Chem. Biol.; 13, 11-21 and Rakhit R, Navarro R, Wand less TJ (2014) Chem Biol. Sep 18;21(9):1238-52). However, when a specific small molecule ligand binds its intended DD as a ligand binding partner, the instability is reversed and protein function is restored. Such a system is herein referred to as a biocircuit, with the canonical DD-containing biocircuit described above being the prototypical model biocircuit.

It is believed that improvements of biocircuits, including those containing DDs can form the basis of a new class of cell and gene therapies that employ tunable and temporal control of gene expression and function. Such novel moieties are described by the present inventors as stimulus response elements (SREs) which act in the context of an effector module to complete a biocircuit arising from a stimulus and ultimately producing a signal or outcome. When properly formatted with a polypeptide payload, and when activated by a particular stimulus, e.g., a small molecule, biocircuit systems can be used to regulate transgene and/or protein levels either up or down by perpetuating a stabilizing signal or destabilizing signal. This approach has many advantages over existing methods of regulating protein function and/or expression, which are currently focused on top level transcriptional regulation via inducible promoters.

Beyond the initial work on destabilizing domains (Banaszynski, et al., (2006) Cell; 126(5): 995-1004; US Patent 8,173,792 and US Patent 8,530,636), is the development of expanded biocircuit systems such as those taught in the present application including stimulus response elements (SREs) which go far beyond the destabilizing or dimerization domains of the art. Such therapies represent a significant improvement on existing gene therapy strategies, and could also expand the universe of protein therapeutics that can be safely and effectively incorporated into gene therapy modalities, including applications that have previously been considered unsuitable for therapeutic use.

### SUMMARY OF THE INVENTION

The invention provides an effector module comprising a first component and a second component, wherein said first component is a stimulus response element (SRE) comprising an FKBP or E.coli DHFR (ecDHFR) destabilizing domain (DD), each DD having two or more mutations relative to a wild-type FKBP or ecDHFR protein sequence, wherein the FKBP DD comprises the mutations (F36V, L106P) or (E31G, F36V, R71G, K105E); and the ecDHFR DD comprises the mutations (R12Y, Y1001) or (R12H, E129K) and wherein said second component is a payload construct comprising a CD19 CAR payload; and wherein said effector module is responsive to at least one stimulus.

The invention also provides a polynucleotide encoding the effector module of the present invention as well as an expression vector comprising the polynucleotide of the present invention. The present invention also provides a cell comprising the expression vector of the present invention.

The present invention also provides a pharmaceutical composition comprising the effector module, polynucleotide, expression vector or cell of the present invention, wherein the pharmaceutical composition is for us in a method of treating a disease or disorder in a subject.

The invention provides a regulatable human T cell or T cell population engineered to express an effector module, wherein, the effector module comprises a chimeric antigen receptor (CAR) that recognizes a CD19 antigen and a FKBP or ecDHFR destabilizing domain (DD), each DD having two or more mutations relative to a wild-type FKBP or ecDHFR protein sequence, wherein the FKBP DD comprises the mutations (F36V, L106P) or (E31G, F36V, R71G, K105E); and the ecDHFR DD comprises the mutations (R12Y, Y100I) or (R12H, E129K). The regulatable human T cell or T cell population of the present invention may be for use in a method of treating a patient in need thereof, the method comprising administration of the regulatable human T cell or T cell population.

Further, the invention provides a method of producing a regulatable human T-cell or population thereof, the method comprising,
(a) contacting an isolated population of T-cells with a polynucleotide encoding one or more effector modules such that the effector module can be expressed in the contacted population;
(b) causing the level of the payload encoded by said one or more effector modules to be modulated upon exposure of the expressed effector module with one or more stimuli;
wherein at least one of the one or more effector modules comprises a stimulus response element (SRE) and a payload construct, wherein the payload construct comprises a CD19 CAR payload and the SRE comprises a FKBP or ecDHFR destabilizing domain (DD), each DD having two or more mutations relative to a wild-type FKBP or ecDHFR protein sequence, wherein the FKBP DD comprises the mutations (F36V, L106P) or (E31G, F36V, R71G, K105E) and the ecDHFR DD comprises the mutations (R12Y, Y100I) or (R12H, E129K). In one aspect, the level of the payload encoded by said one or more effector modules is upregulated upon exposure to the stimulus.

Further aspects of the invention are provided in the claims.

In one aspect, the effector module may comprise a signal sequence, a cleavage and/or processing feature, a targeting and/or penetrating peptide, and/or a linker.

In one aspect, the effector module of the biocircuit system may comprise a signal sequence selected from those listed in Table 2, a cleavage and/or processing feature selected from those listed in Table 3, a targeting and/or penetrating peptide selected from those listed in Tables 5 or 6, and/or a linker selected from those listed in Tables 7 and 8.

The polynucleotide encoding an effector module may be a DNA molecule. The polynucleotide may have a region such as, but not limited to, a region comprising the sequence of any known microRNAs. The polynucleotide may have a region such as, but not limited to, a region comprising the sequence of any of the microRNAs listed in Table 9, the reverse complement of the microRNAs listed in Table 9, or the microRNA anti-seed region of any of the microRNAs listed in Table 9.

The polynucleotide encoding an effector module may be a messenger RNA (mRNA) molecule. The polynucleotide may have a region such as, but not limited to, a region comprising the sequence of any of the microRNAs listed in Table 9, the reverse complement of the microRNAs listed in Table 9, or the microRNA anti-seed region of any of the microRNAs listed in Table 9. The mRNA molecule may comprise one or more chemical modifications such as, but not limited to, a sugar, nucleobase or backbone modifications. The modifications may be naturally or non-naturally occurring modifications.

At least one region of the polynucleotide may be codon optimized. As a non-limiting example, the region encoding the first component of the effector module is codon optimized. As another non-limiting example, the region encoding the second component of the effector module is codon optimized.

The polynucleotide may be a DNA molecule. The polynucleotide may have a region such as, but not limited to, a region comprising the sequence of any known microRNA. The polynucleotide may have a region such as, but not limited to, a region comprising the sequence of any of the microRNAs listed in Table 9, the reverse complement of the microRNAs listed in Table 9, or the microRNA anti-seed region of any of the microRNAs listed in Table 9.

The polynucleotide may be a messenger RNA (mRNA) molecule. The polynucleotide may have a region such as, but not limited to, a region comprising the sequence of any of the known microRNAs. The polynucleotide may have a region such as, but not limited to, a region comprising the sequence of any of the microRNAs listed in Table 9, the reverse complement of the microRNAs listed in Table 9, or the microRNA anti-seed region of any of the microRNAs listed in Table 9. The mRNA molecule may comprise one or more chemical modifications such as, but not limited to, a sugar, nucleobase or backbone modifications. The modifications may be naturally or non-naturally occurring modifications.

The pharmaceutical composition may be administered via a route such as, but not limited to intravenous (into a vein).

In one aspect, the T-cells are primary T-cells. In another aspect, the T cell may be, but is not limited to, cytotoxic T-cells, helper T-cells, memory T-cells, regulatory T-cells, tissue infiltrating lymphocytes and combinations thereof. In one aspect, the cell population may be obtained from a subject suffering from, being treated for, diagnosed with, at risk of developing, or suspected of having a disorder selected from the group consisting of an immune disorder (including autoimmune disorders), a hypoproliferative condition including cancer, an infectious disease, a non-infectious disease, and graft vs. host disease.

The treatment may comprise adoptive immunotherapy. In one aspect, prior to administration, the regulatable human T cell or T cell population may be expanded.

### BRIEF DESCRIPTION OF THE DRAWINGS

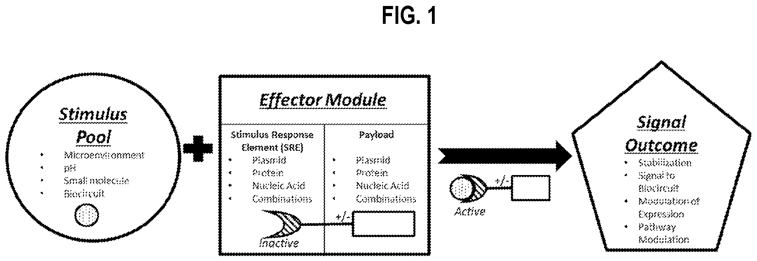

FIG. 1 shows an overview diagram of a biocircuit system. The biocircuit comprises a stimulus and at least one effector module responsive to a stimulus, where the response to the stimulus produces a signal or outcome. The effector module comprises at least one stimulus response element (SRE) and one payload.

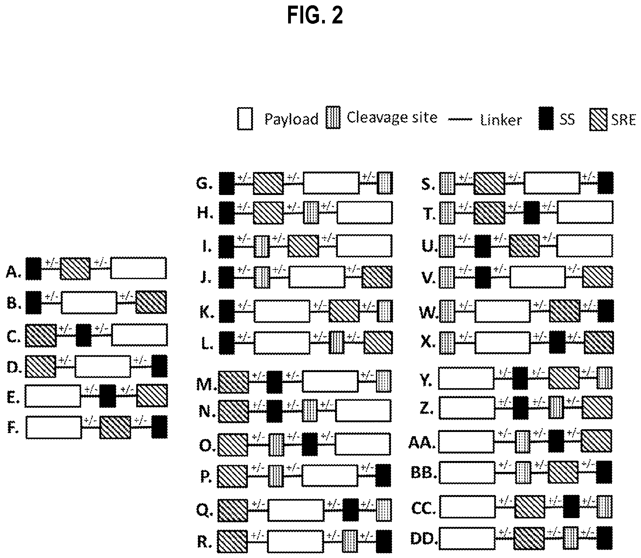

FIG. 2 shows representative effector modules carrying one payload. The signal sequence (SS), SRE and payload may be located or positioned in various arrangements without (A to F) or with (G to Z, and AA to DD) a cleavage site. An optional linker may be inserted between each component of the effector module.

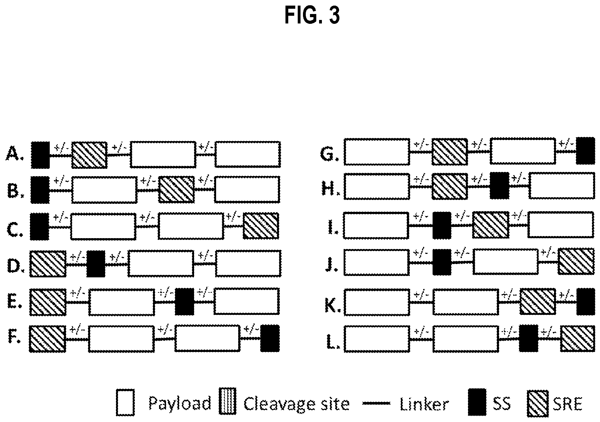

FIG. 3 shows representative effector modules carrying two payloads without a cleavage site. The two payloads may be either directly linked to each other or separated.

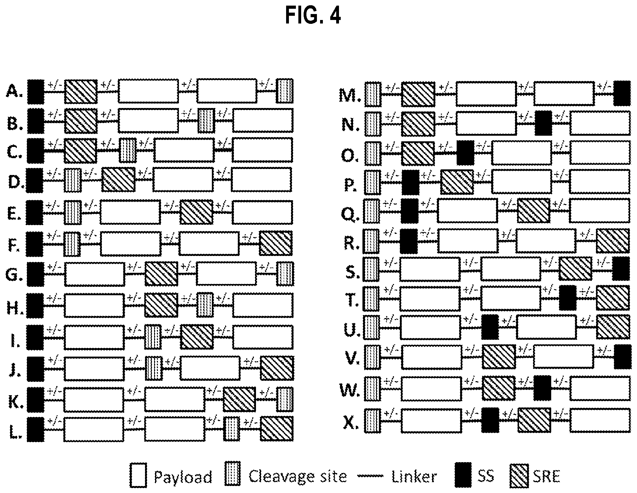

FIG. 4 shows representative effector modules carrying two payloads with a cleavage site. In one embodiment, an SS is positioned at the N-terminus of the construct, while other components: SRE, two payloads and the cleavage site may be located at different positions (A to L). In another embodiment, the cleavage site is positioned at the N-terminus of the construct (M to X). An optional linker may be inserted between each component of the effector module.

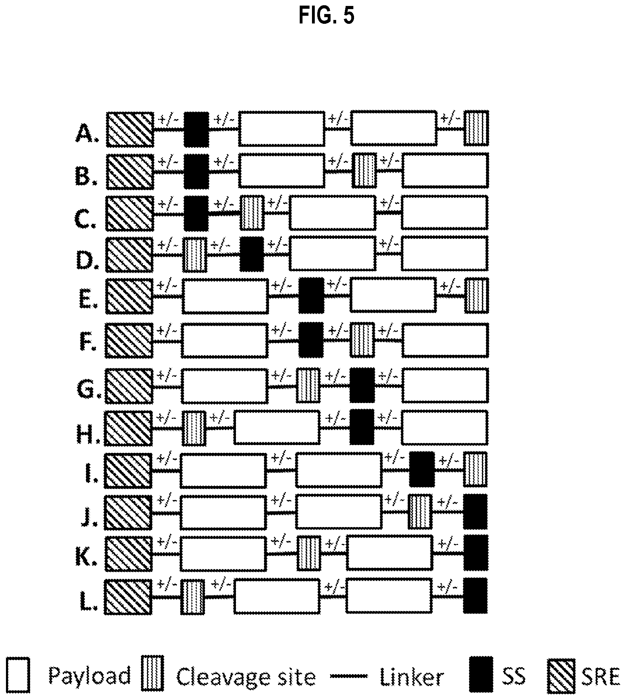

FIG. 5 shows effector modules of the invention carrying two payloads, where an SRE is positioned at the N-terminus of the construct (A to L), while SS, two payloads and the cleavage site can be in any configuration. An optional linker may be inserted between each component of the effector module.

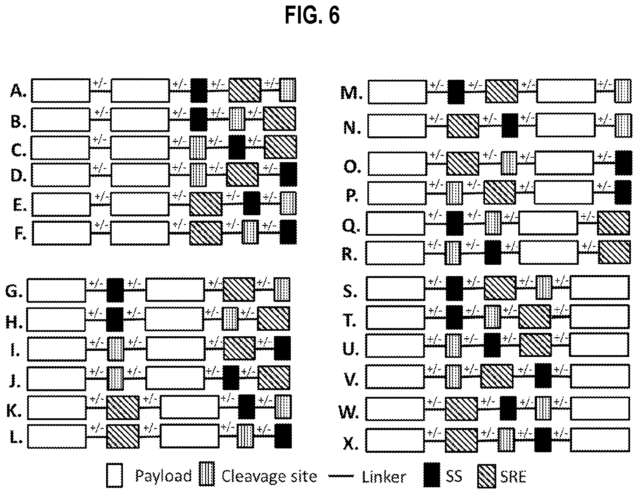

FIG. 6 shows effector modules of the invention carrying two payloads, where either the two payloads (A to F) or one of the two payloads (G to X) is positioned at the N-terminus of the construct (A to L), while SS, SRE and the cleavage site can be in any configuration. An optional linker may be inserted between each component of the effector module.

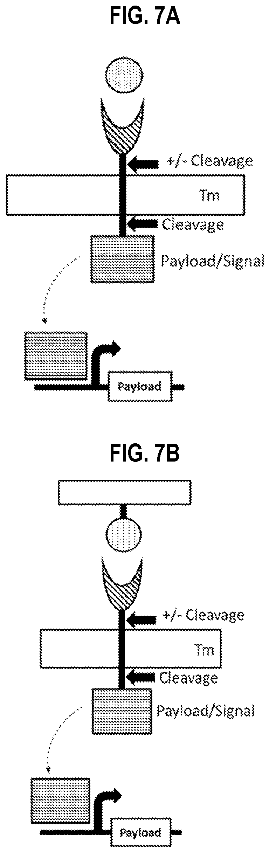

FIG. 7 depicts representative configurations of the stimulus and effector module within a biocircuit system. A trans-membrane effector module is activated either by a free stimulus (FIG. 7A) or a membrane bound stimulus (FIG. 7B) which binds to SRE. The response to the stimulus causes the cleavage of the intracellular signal/payload, which activates downstream effector/payload.

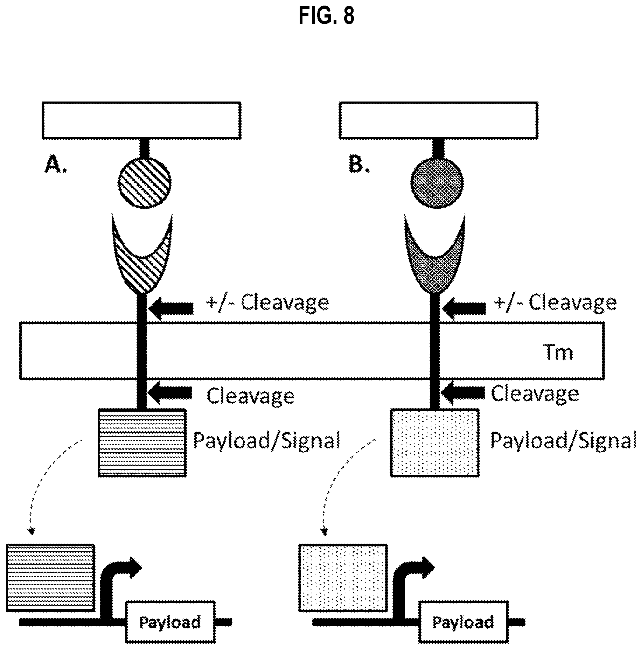

FIG. 8 depicts a dual stimulus-dual presenter biocircuit system, where two bound stimuli (A and B) from two different presenters (e.g., different cells) bind to two different effector modules in a single receiver (e.g., another single cell) simultaneously and create a dual-signal to downstream payloads.

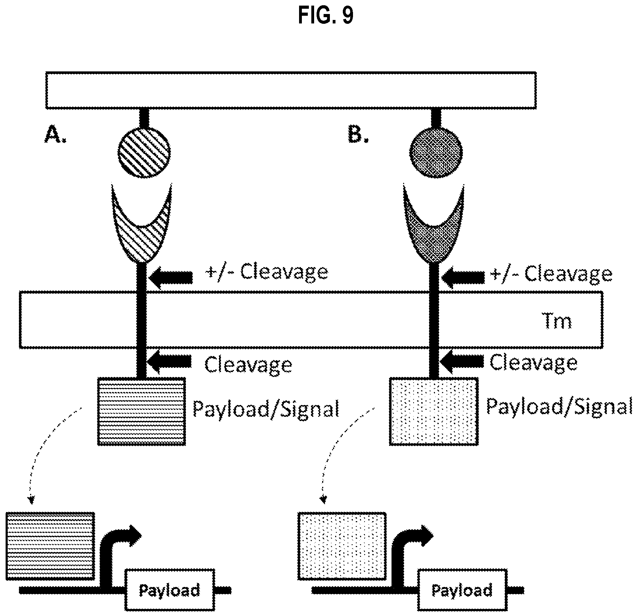

FIG. 9 depicts a dual stimulus-single presenter biocircuit system, where two bound stimuli (A and B) from the same presenter (e.g., a single cell) bind to two different effector modules in another single cell simultaneously and create a dual-signal.

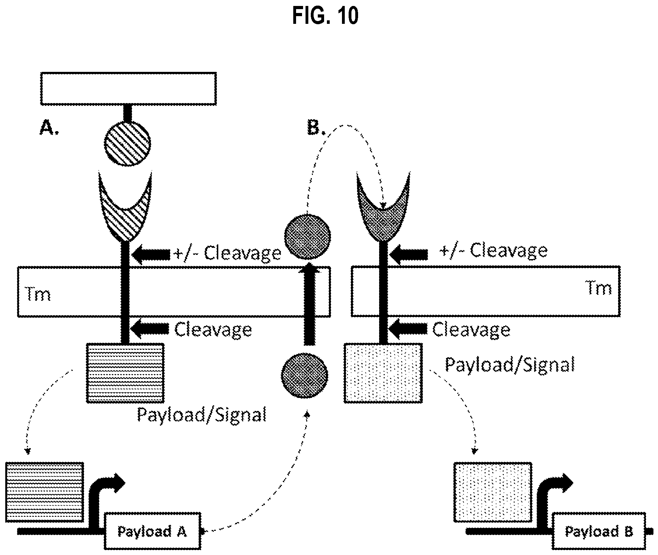

FIG. 10 depicts a single-stimulus-bridged receiver biocircuit system. In this configuration, a bound stimulus (A) binds to an effector module in the bridge cell and creates a signal to activate a payload which is a stimulus (B) for another effector module in the final receiver (e.g., another cell).

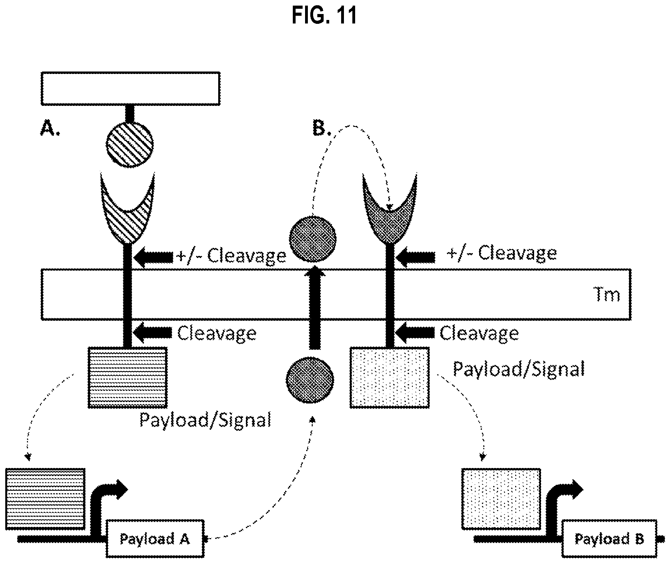

FIG. 11 depicts a single stimulus-single receiver biocircuit system, wherein the single receiver contains the two effector modules which are sequentially activated by a single stimulus.

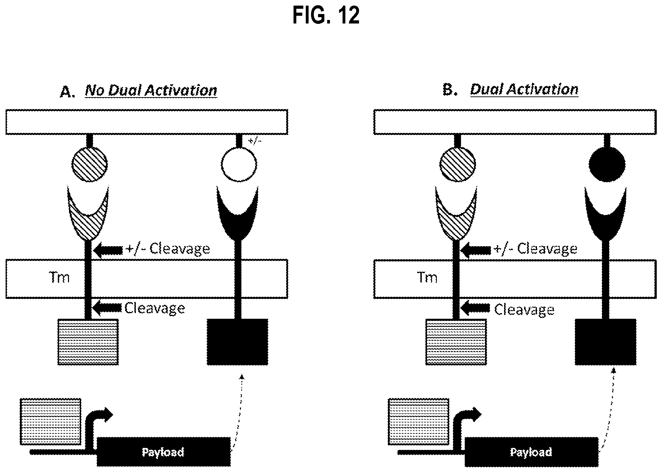

FIG. 12 depicts a biocircuit system which requires a dual activation. In this embodiment, one stimulus must bind the transmembrane effector module first to prime the receiver cell being activated by the other stimulus. The receiver only activates when it senses both stimuli (B).

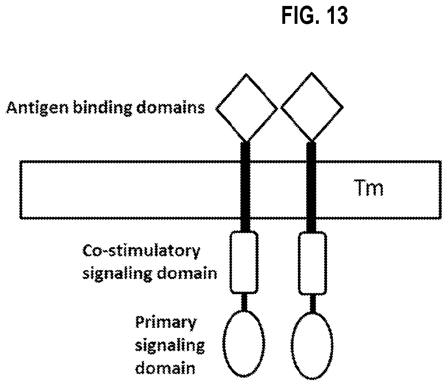

FIG. 13 depicts a standard effector module of a chimeric antigen receptor (CAR) system which comprises an antigen binding domain as an SRE, and signaling domain(s) as payload.

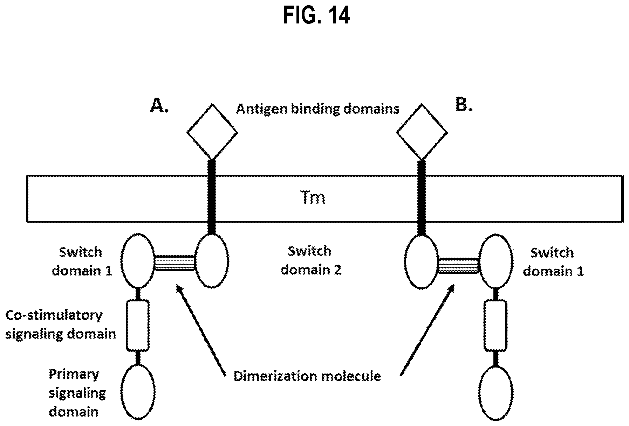

FIG. 14 depicts the structure design of a regulatable CAR system, where the trans-membrane effector modules comprise antigen binding domains sensing an antigen and a first switch domain and the intracellular module comprises a second switch domain and signaling domains. A stimulus (e.g., a dimerization small molecule) can dimerize the first and second switch domains and assemble an activated CAR system.

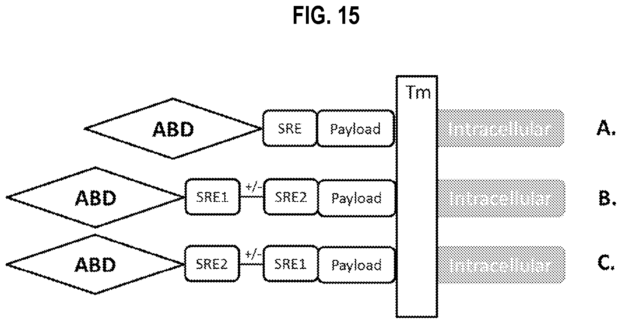

FIG. 15 shows schematic representation of CAR systems having one (A) or two (B and C) SREs incorporated into the effector module.

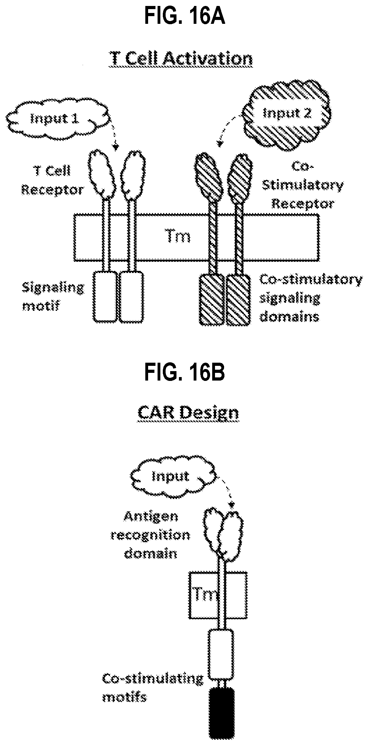

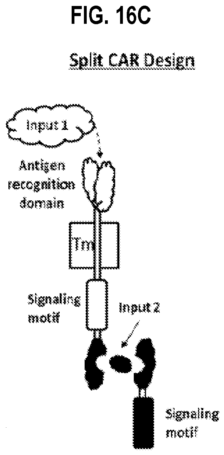

FIG. 16 depicts a split CAR design to control T cell activation by a dual stimulus (e.g., an antigen and small molecule). FIG. 16A shows normal T cell activation which entails a dual activation of TCR and co-stimulatory receptor. The regular CAR design (FIG. 16B) combines the antigen recognition domain with TCR signaling motif and co-stimulatory motif in a single molecule. The split CAR system separates the components of the regular CAR into two separate effector modules which can be reassembled when a heterodimerizing small molecule (stimulus) is present (FIG. 16C).

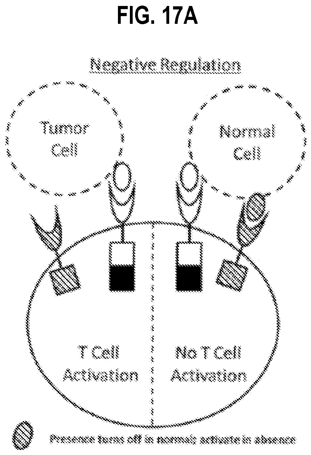

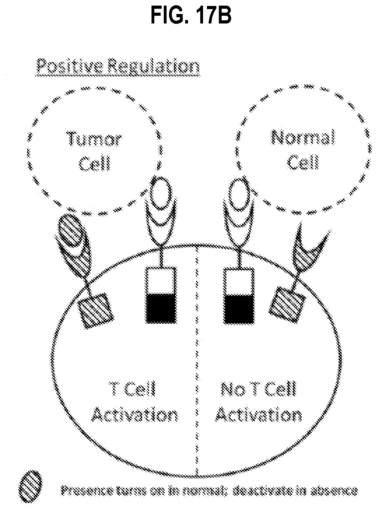

FIG. 17 depicts the positive and negative regulation of CAR engineered T cell activation. The absence or presence of a second stimulus can negatively (FIG. 17A) or positively (FIG. 17B) control T cell activation.

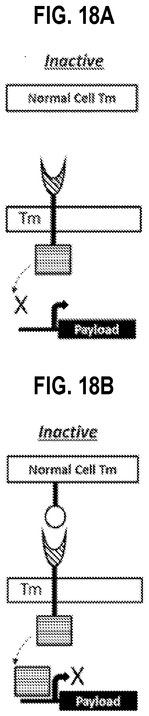

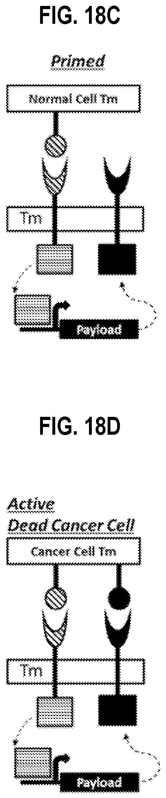

FIG. 18 shows schematic representation of gated activation of CAR engineered T cells. If a normal cell that has no stimulus (e.g., an antigen) (FIG. 18A) or an antigen that cannot bind to the trans-membrane effector module (FIG. 18B), or only an antigen that activates the trans-membrane effector module and primes the receiver T cell to express the second effector (FIG. 18C), the receiver T cell remains inactive. When both stimuli (e.g. two antigens) that bind the trans-membrane effector module and the primed effector, are present on the presenter cell (e.g. a cancer cell), the T cell is activated (FIG 18D).

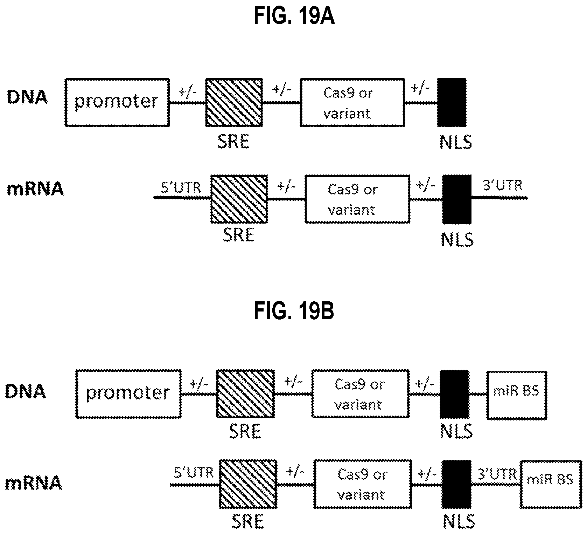

Reference FIG. 19 shows representative effector modules having Cas9 or variant Cas9 as the payload and a nuclear localization signal (NLS) without a miR binding site (miR BS) (FIG. 19A) or with a miR binding site (FIG. 19B).

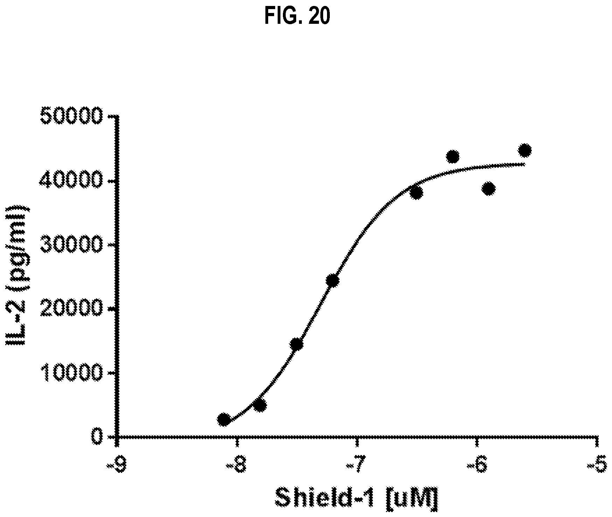

Reference FIG. 20 is a line graph depicting DD-IL2 levels in response to varying concentrations of Shield-1.

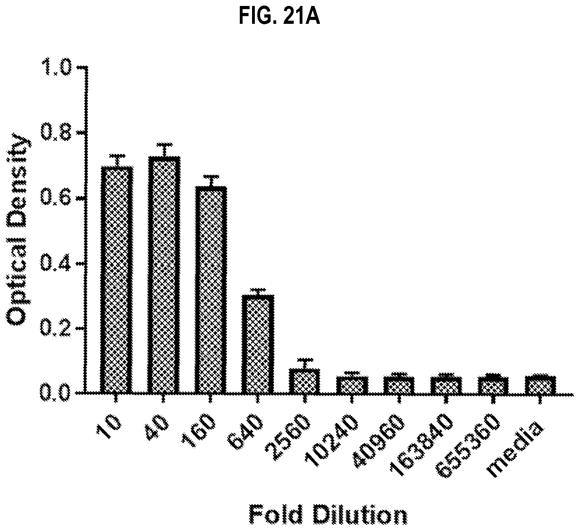

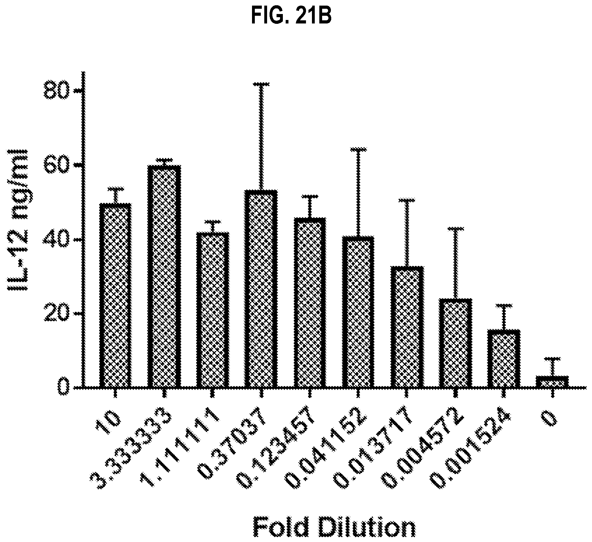

Reference FIG. 21A is a bar graph depicting DD-IL12 levels in the various dilutions of media derived from cells expressing DD-IL12. FIG. 21B is a bar graph depicting the Shield-1 dose responsive induction of DD- IL12.

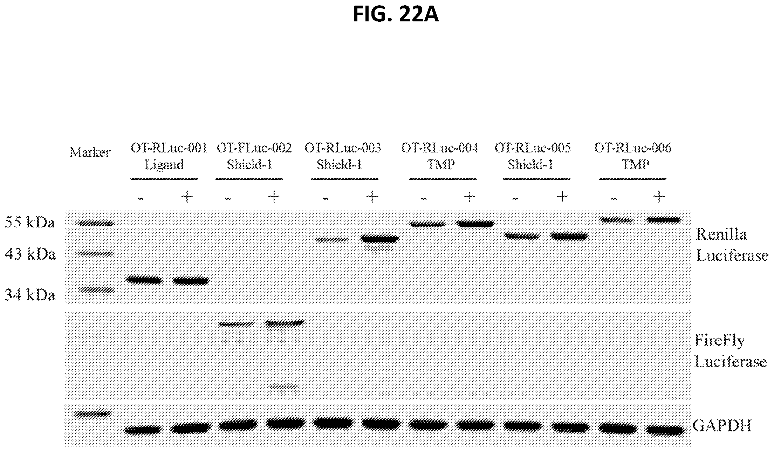

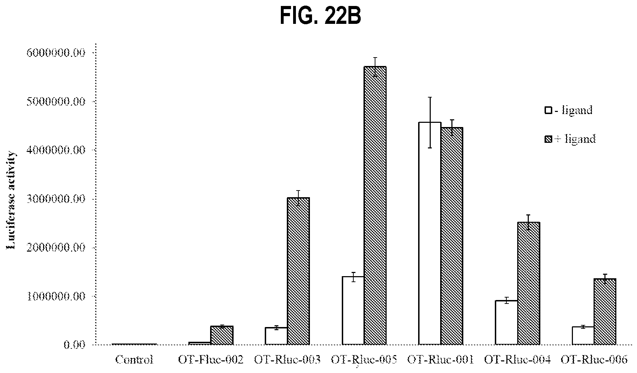

Reference FIG. 22A is a western blot depicting luciferase levels in DD-luciferase expressing cells. FIG. 22B depicts luciferase activity.

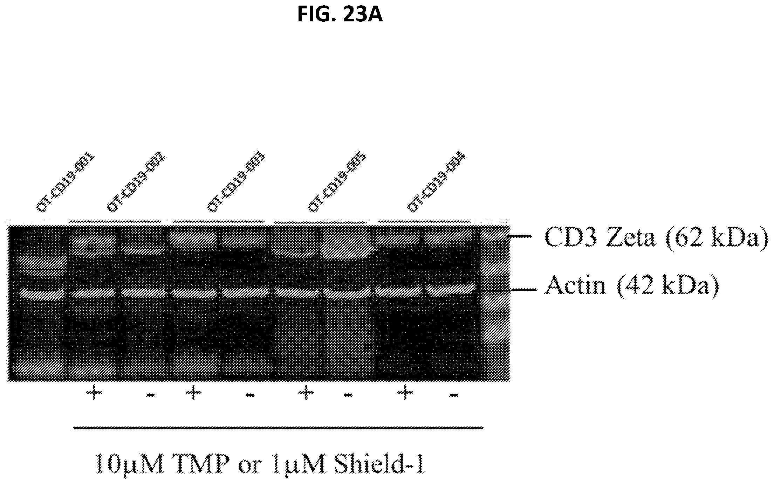

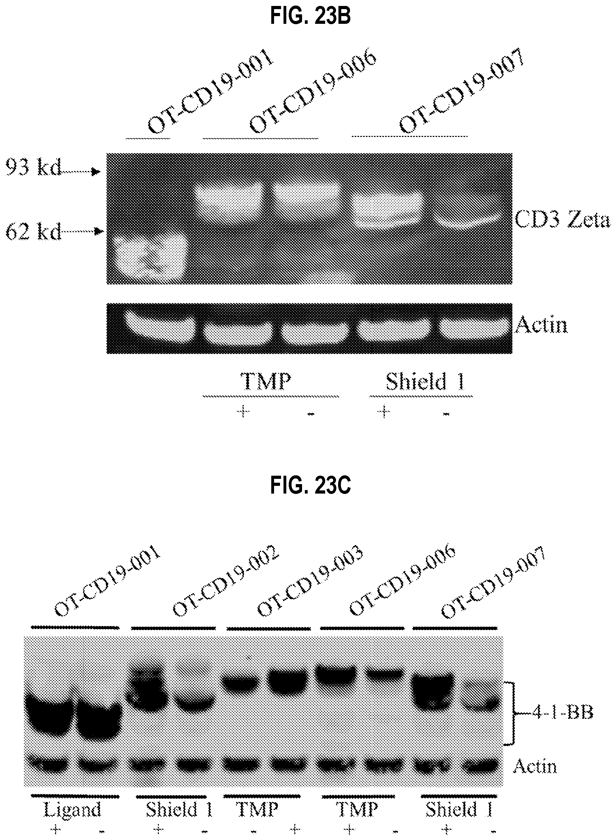

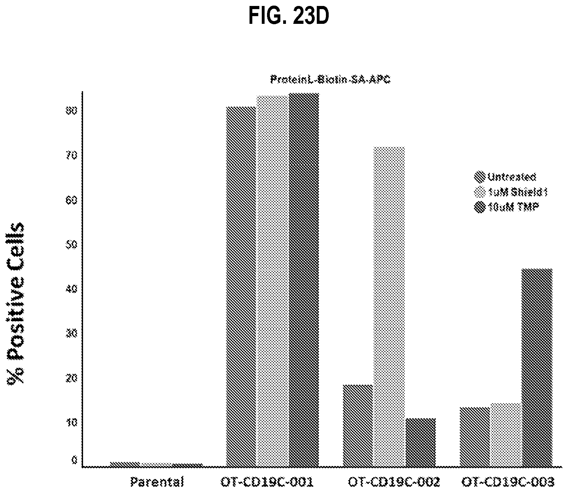

FIG. 23A and FIG. 23B are western blots depicting CD3 zeta levels in CD19 CAR expressing cells. FIG. 23C is a western blot depicting 41-BB levels in CD19 CAR expressing cells. FIG. 23D is a bar graph depicting the surface expression of CD19 CAR.

### DETAILED DESCRIPTION OF THE INVENTION

### I. INTRODUCTION

Strategies for the conditional regulation of gene expression and protein function have been described in the art and have focused predominantly on the manipulation of gene promoters to alter expression levels. However, such strategies suffer from delays between administration of the stimulus, most often a ligand, and the ultimate response from the system-owing to the delay for signals to reach the nucleus and effect transcriptional changes.

Further, knock down strategies aimed at muting the DNA or destruction of mRNA suffer from a long wait time for the ultimate desired drop in protein levels and function, owing in part to residual protein present and longer protein half-lives for destruction.

The longer lag times between administration of a particular perturbation agent and the response of the natural systems involving transcription, translation and protein degradation limit the application of the methods whose mechanism of action is subject to such natural processing times.

It is more advantageous to target the protein molecule directly. Strategies which directly trigger a cell's natural degradation systems have been developed. One such system relies on temperature sensitivity (for DHFR and the ligand, methotrexate) (Dohmen RJ, et al., Science. 1994; 263:1273-1276; and Lévy F, et al., Eur. J. Biochem. 1999; 259:244-252).

Others have used reversible systems employing a rapamycin derivative for the regulation of GSK-3β kinase fused to an unstable triple-mutant of the FRB domain (FRB*) (Stankunas et al., Mol Cell. 2003; 12:1615-1624 and Liu et al., Nature. 2007; 446:79-82).

Banaszynski, et al., developed a cell-permeable ligand systems using mutants of FKBP12 protein which were engineered to be unstable in the absence of a high-affinity ligand, Shield-1. (Banaszynski et al., Cell. 2006; 126:995-1004). They termed these unstable domains, destabilizing domains (DDs).

Subsequently, *E. coli* dihydrofolate reductase (ecDHFR) was explored as a candidate protein from which to design destabilizing domains. One inhibitor of DHFR, trimethoprim (TMP), inhibits ecDHFR much more potently than mammalian DHFR and this differential responsiveness makes this protein-ligand pair ideal for development for use as a biocircuit (Iwamoto, et al., Chem Biol. (2010) September 24; 17(9): 981-988).

Post-translational control has been of great interest in several gene therapy and cell therapy areas including immune-oncology applications and the expanding area of stem cell technology (Reviewed in Rakhit, et al., Chem Biol. 2014 September 18; 21(9): 1238-1252).

Most recently protein switches useful as biosensors as well as new chimeric antigen receptors and other small molecule stabilization frameworks have been disclosed (An W, et al. (2015), PLoS ONE (2015) 10(12): e0145783. doi: 10.1371/journal.pone.0145783; Nicholes, et al., Protein Engineering, Design & Selection, 2016, vol. 29 no. 2, pp. 77-85; Nath, et al., Biochemical and Biophysical Research Communications 470 (2016) 411e416); Stevers, et al., PNAS, 2016, vol. 119, no. 9, pp. E112-1161; Juillerat, A. et al., Sci. Rep. (2016), 6, 18950; Roybal, Cell, (2016), vol. 164, pp. 1-10; and Morsut, Cell, (2016), vol. 164, pp. 1-12).

But collectively, these may all be characterized as simple on-off switches, even when combined with one another switch, e.g., the propagation of a single input or effect.

The present disclosure expands upon the early understandings of destabilizing domain research toward the development of new regulatable biocircuit systems and their methods of use. Such biocircuits include broader spectrum tunable stimulus response elements (SREs) which may be exploited alone or in concert with tunable proteins thus providing increased modularity and flexibility.

### II. COMPOSITIONS OF THE INVENTION

The present invention provides the effector module of claim 1.

As used herein, a "biocircuit" or "biocircuit system" is defined as a circuit within or useful in biologic systems comprising a stimulus and at least one effector module responsive to a stimulus, where the response to the stimulus produces at least one signal or outcome within, between, as an indicator of, or on a biologic system. Biologic systems are generally understood to be any cell, tissue, organ, organ system or organism, whether animal, plant, fungi, bacterial, or viral. It is also understood that biocircuits may be artificial circuits which employ the stimuli or effector modules taught by the present invention and effect signals or outcomes in acellular environments such as with diagnostic, reporter systems, devices, assays or kits. The artificial circuits may be associated with one or more electronic, magnetic, or radioactive components or parts.

The present invention includes a destabilizing domain (DD) biocircuit system.

### Effector Modules, SREs and Payloads

As used herein, an "effector module" is a single or multi-component construct or complex comprising at least (a) one or more stimulus response elements and (b) one or more payloads.

As used herein a "stimulus response element (SRE)" is a component of an effector module which is joined, attached, linked to or associated with one or more payloads of the effector module and in some instances, is responsible for the responsive nature of the effector module to one or more stimuli. As used herein, the "responsive" nature of an SRE to a stimulus may be characterized by a covalent or non-covalent interaction, a direct or indirect association or a structural or chemical reaction to the stimulus. Further, the response of any SRE to a stimulus may be a matter of degree or kind. The response may be a partial response. The response may be a reversible response. The response may ultimately lead to a regulated signal or output. Such output signal may be of a relative nature to the stimulus, e.g., producing a modulatory effect of between 1% and 100% or a factored increase or decrease such as 2-fold, 3-fold, 4-fold, 5-fold, 10-fold or more.

In some aspects, the modulation of protein level refers to modulation of level by at least about 20%, such as by at least about 30%, 40%, 50%, 60%, 70%, 80%, 85%, 90%, 95% and 100%, or at least 20-30%, 20-40%, 20-50%, 20-60%, 20-70%, 20-80%, 20-90%, 20-95%, 20-100%, 30-40%, 30-50%, 30-60%, 30-70%, 30-80%, 30-90%, 30-95%, 30-100%, 40-50%, 40-60%, 40-70%, 40-80%, 40-90%, 40-95%, 40-100%, 50-60%, 50-70%, 50-80%, 50-90%, 50-95%, 50-100%, 60-70%, 60-80%, 60-90%, 60-95%, 60-100%, 70-80%, 70-90%, 70-95%, 70-100%, 80-90%, 80-95%, 80-100%, 90-95%, 90-100% or 95-100%.

As used herein a "payload "or "target payload" or "payload of interest (POI)" is defined as any protein or nucleic acid whose function is to be altered.

The effector modules, including their SREs and payloads of the present invention may exist as a whole polypeptide, a plurality of polypeptides or fragments of polypeptides, which independently may be encoded by one or more nucleic acids, a plurality of nucleic acids, fragments of nucleic acids or variants of any of the aforementioned.

As used herein, the term "polypeptide" refers to a polymer of amino acid residues (natural or unnatural) linked together most often by peptide bonds. The term, as used herein, refers to proteins, polypeptides, and peptides of any size, structure, or function. In some instances, the polypeptide encoded is smaller than about 50 amino acids and the polypeptide is then termed a peptide. If the polypeptide is a peptide, it will be at least about 2, 3, 4, or at least 5 amino acid residues long. Thus, polypeptides include gene products, naturally occurring polypeptides, synthetic polypeptides, homologs, orthologs, paralogs, fragments and other equivalents, variants, and analogs of the foregoing. A polypeptide may be a single molecule or may be a multi-molecular complex such as a dimer, trimer or tetramer. They may also comprise single chain or multichain polypeptides and may be associated or linked. The term polypeptide may also apply to amino acid polymers in which one or more amino acid residues are an artificial chemical analogue of a corresponding naturally occurring amino acid.

As used herein, the term "polypeptide variant" refers to molecules which differ in their amino acid sequence from a native or reference sequence. The amino acid sequence variants may possess substitutions, deletions, and/or insertions at certain positions within the amino acid sequence, as compared to a native or reference sequence. Ordinarily, variants will possess at least about 50% identity (homology) to a native or reference sequence, and preferably, they will be at least about 80%, more preferably at least about 90% identical (homologous) to a native or reference sequence.

As used herein, the term "variant mimic" refers to a variant which contains one or more amino acids which would mimic an activated sequence. For example, glutamate may serve as a mimic for phospho-threonine and/or phospho-serine. Alternatively, variant mimics may result in deactivation or in an inactivated product containing the mimic, e.g., phenylalanine may act as an inactivating substitution for tyrosine; or alanine may act as an inactivating substitution for serine. The amino acid sequences of the effector modules including their SREs or payloads of the invention may comprise naturally occurring amino acids and as such may be considered to be proteins, peptides, polypeptides, or fragments thereof. Alternatively, the effector modules including their SREs or payloads may comprise both naturally and non-naturally occurring amino acids.

As used herein, the term "amino acid sequence variant" refers to molecules with some differences in their amino acid sequences as compared to a native or starting sequence. The amino acid sequence variants may possess substitutions, deletions, and/or insertions at certain positions within the amino acid sequence. As used herein, the terms "native" or "starting" when referring to sequences are relative terms referring to an original molecule against which a comparison may be made. Native or starting sequences should not be confused with wild type sequences. Native sequences or molecules may represent the wild-type (that sequence found in nature) but do not have to be identical to the wild-type sequence.

Ordinarily, variants will possess at least about 70% homology to a native sequence, and preferably, they will be at least about 80%, more preferably at least about 90% homologous to a native sequence.

As used herein, the term "homology" as it applies to amino acid sequences is defined as the percentage of residues in the candidate amino acid sequence that are identical with the residues in the amino acid sequence of a second sequence after aligning the sequences and introducing gaps, if necessary, to achieve the maximum percent homology. Methods and computer programs for the alignment are well known in the art. It is understood that homology depends on a calculation of percent identity but may differ in value due to gaps and penalties introduced in the calculation.

As used herein, the term "homolog" as it applies to amino acid sequences is meant the corresponding sequence of other species having substantial identity to a second sequence of a second species.

As used herein, the term "analog" is meant to include polypeptide variants which differ by one or more amino acid alterations, e.g., substitutions, additions or deletions of amino acid residues that still maintain the properties of the parent polypeptide.

As used herein, the term "derivative" is used synonymously with the term "variant" and refers to a molecule that has been modified or changed in any way relative to a reference molecule or starting molecule.

The present invention contemplates several types of effector modules including their SREs or payloads which are amino acid based including variants and derivatives. These include substitutional, insertional, deletional and covalent variants and derivatives. As such, included within the scope of this invention are effector modules including their SREs or payloads comprising substitutions, insertions, additions, deletions and/or covalent modifications. For example, sequence tags or amino acids, such as one or more lysines, can be added to peptide sequences of the invention (e.g., at the N-terminal or C-terminal ends). Sequence tags can be used for peptide purification or localization. Lysines can be used to increase peptide solubility or to allow for biotinylation. Alternatively, amino acid residues located at the carboxy and amino terminal regions of the amino acid sequence of a peptide or protein may optionally be deleted providing for truncated sequences. Certain amino acids (e.g., C-terminal or N-terminal residues) may alternatively be deleted depending on the use of the sequence, as for example, expression of the sequence as part of a larger sequence which is soluble, or linked to a solid support.

"Substitutional variants" when referring to proteins are those that have at least one amino acid residue in a native or starting sequence removed and a different amino acid inserted in its place at the same position. The substitutions may be single, where only one amino acid in the molecule has been substituted, or they may be multiple, where two or more amino acids have been substituted in the same molecule.

As used herein, the term "conservative amino acid substitution" refers to the substitution of an amino acid that is normally present in the sequence with a different amino acid of similar size, charge, or polarity. Examples of conservative substitutions include the substitution of a non-polar (hydrophobic) residue such as isoleucine, valine and leucine for another non-polar residue. Likewise, examples of conservative substitutions include the substitution of one polar (hydrophilic) residue for another such as between arginine and lysine, between glutamine and asparagine, and between glycine and serine. Additionally, the substitution of a basic residue such as lysine, arginine or histidine for another, or the substitution of one acidic residue such as aspartic acid or glutamic acid for another acidic residue are additional examples of conservative substitutions. Examples of non-conservative substitutions include the substitution of a non-polar (hydrophobic) amino acid residue such as isoleucine, valine, leucine, alanine, methionine for a polar (hydrophilic) residue such as cysteine, glutamine, glutamic acid or lysine and/or a polar residue for a non-polar residue.

As used herein, the term "insertional variants" when referring to proteins are those with one or more amino acids inserted immediately adjacent to an amino acid at a particular position in a native or starting sequence. As used herein, the term "immediately adjacent" refers to an adjacent amino acid that is connected to either the alpha-carboxy or alpha-amino functional group of a starting or reference amino acid.

As used herein, the term "deletional variants" when referring to proteins, are those with one or more amino acids in the native or starting amino acid sequence removed. Ordinarily, deletional variants will have one or more amino acids deleted in a particular region of the molecule.

As used herein, the term "derivatives," as referred to herein includes variants of a native or starting protein comprising one or more modifications with organic proteinaceous or non-proteinaceous derivatizing agents, and post-translational modifications. Covalent modifications are traditionally introduced by reacting targeted amino acid residues of the protein with an organic derivatizing agent that is capable of reacting with selected side-chains or terminal residues, or by harnessing mechanisms of post-translational modifications that function in selected recombinant host cells. The resultant covalent derivatives are useful in programs directed at identifying residues important for biological activity, for immunoassays, or for the preparation of anti-protein antibodies for immunoaffinity purification of the recombinant glycoprotein. Such modifications are within the ordinary skill in the art and are performed without undue experimentation.

Features of the proteins of the present invention include surface manifestations, local conformational shape, folds, loops, half-loops, domains, half-domains, sites, termini or any combination thereof. As used herein, the term "features" when referring to proteins are defined as distinct amino acid sequence-based components of a molecule.

As used herein, the term "surface manifestation" when referring to proteins refers to a polypeptide based component of a protein appearing on an outermost surface.

As used herein, the term "local conformational shape" when referring to proteins refers to a polypeptide based structural manifestation of a protein which is located within a definable space of the protein.

As used herein, the term "fold," when referring to proteins, refers to the resultant conformation of an amino acid sequence upon energy minimization. A fold may occur at the secondary or tertiary level of the folding process. Examples of secondary level folds include beta sheets and alpha helices. Examples of tertiary folds include domains and regions formed due to aggregation or separation of energetic forces. Regions formed in this way include hydrophobic and hydrophilic pockets, and the like.

As used herein, the term "turn" as it relates to protein conformation, refers to a bend which alters the direction of the backbone of a peptide or polypeptide and may involve one, two, three or more amino acid residues.

As used herein, the term "loop," when referring to proteins, refers to a structural feature of a peptide or polypeptide which reverses the direction of the backbone of a peptide or polypeptide and comprises four or more amino acid residues. Oliva et al. have identified at least 5 classes of protein loops (Oliva, B. et al., An automated classification of the structure of protein loops. J Mol Biol. 1997. 266(4):814-30.)

As used herein, the term "half-loop," when referring to proteins, refers to a portion of an identified loop having at least half the number of amino acid resides as the loop from which it is derived. It is understood that loops may not always contain an even number of amino acid residues. Therefore, in those cases where a loop contains or is identified to comprise an odd number of amino acids, a half-loop of the odd-numbered loop will comprise the whole number portion or next whole number portion of the loop (number of amino acids of the loop/2+/-0.5 amino acids). For example, a loop identified as a 7 amino acid loop could produce half-loops of 3 amino acids or 4 amino acids (7/2=3.5+/-0.5 being 3 or 4).

As used herein, the term "domain," when referring to proteins, refers to a motif of a polypeptide having one or more identifiable structural or functional characteristics or properties (e.g., binding capacity, serving as a site for protein-protein interactions.)

As used herein, the term "half-domain," when referring to proteins, refers to a portion of an identified domain having at least half the number of amino acid resides as the domain from which it is derived. It is understood that domains may not always contain an even number of amino acid residues. Therefore, in those cases where a domain contains or is identified to comprise an odd number of amino acids, a half-domain of the odd-numbered domain will comprise the whole number portion or next whole number portion of the domain (number of amino acids of the domain/2+/-0.5 amino acids). For example, a domain identified as a 7 amino acid domain could produce half-domains of 3 amino acids or 4 amino acids (7/2=3.5+/-0.5 being 3 or 4). It is also understood that sub-domains may be identified within domains or half-domains, these subdomains possessing less than all of the structural or functional properties identified in the domains or half domains from which they were derived. It is also understood that the amino acids that comprise any of the domain types herein need not be contiguous along the backbone of the polypeptide (i.e., nonadjacent amino acids may fold structurally to produce a domain, half-domain or subdomain).

As used herein, the terms "site," as it pertains to amino acid based embodiments is used synonymously with "amino acid residue" and "amino acid side chain". A site represents a position within a peptide or polypeptide that may be modified, manipulated, altered, derivatized or varied within the polypeptide based molecules of the present invention.

As used herein, the terms "termini" or "terminus," when referring to proteins refers to an extremity of a peptide or polypeptide. Such extremity is not limited only to the first or final site of the peptide or polypeptide but may include additional amino acids in the terminal regions. The polypeptide based molecules of the present invention may be characterized as having both an N-terminus (terminated by an amino acid with a free amino group (NH2)) and a C-terminus (terminated by an amino acid with a free carboxyl group (COOH)).

Polypeptides or proteins are in some cases made up of multiple polypeptide chains brought together by disulfide bonds or by non-covalent forces (multimers, oligomers). These sorts of proteins will have multiple N- and C-termini. Alternatively, the termini of the polypeptides may be modified such that they begin or end, as the case may be, with a non-polypeptide based moiety such as an organic conjugate.

Once any of the features have been identified or defined as a component of a biocircuit system component, stimulus, effector module including the SREs or payloads of the invention, any of several manipulations and/or modifications of these features may be performed by moving, swapping, inverting, deleting, randomizing or duplicating. Furthermore, it is understood that manipulation of features may result in the same outcome as a modification to the compositions of the invention. For example, a manipulation which involved deleting a domain would result in the alteration of the length of a molecule just as modification of a nucleic acid to encode less than a full-length molecule would.

Modifications and manipulations can be accomplished by methods known in the art such as site directed mutagenesis. The resulting modified molecules may then be tested for activity using in vitro or in vivo assays such as those described herein or any other suitable screening assay known in the art.

In some embodiments, compositions of the present invention may comprise one or more atoms that are isotopes. As used herein, the term "isotope" refers to a chemical element that has one or more additional neutrons. In some embodiments, compounds of the present invention may be deuterated. As used herein, the term "deuterate" refers to the process of replacing one or more hydrogen atoms in a substance with deuterium isotopes. Deuterium isotopes are isotopes of hydrogen. The nucleus of hydrogen contains one proton while deuterium nuclei contain both a proton and a neutron. The effector modules including their SREs or payloads of the present invention may be deuterated in order to change one or more physical property, such as stability, or to allow pharmaceutical compositions, biocircuits, biocircuit components, effector modules including their SREs or payloads to be used in diagnostic and/or experimental applications.

Effector modules may be designed to operate in groups of one, two, three, four or more modules. When more than one effector module is utilized in a biocircuit, it is known as an effector module system of that biocircuit.

At the protein level, any of the biocircuit components may comprise one or more post-translational modifications (PTM). Such PTMs may occur intracellularly after administration of a protein-based biocircuit component or upon or after translation of a biocircuit component administered as a nucleic acid encoding said biocircuit component.

Post translational modifications (PTMs) of the present invention include, but are not limited to acetylation, phosphorylation, ubiquitination, carboxylation, deamidation, deamination, deacetylation, dihydroxylation, dephosphorylation, formylation, gamma-carboxyglutamation, glutathionylation, glycation, hydroxylation, methylation, nitration, sumoylation, N- or O-transglutamination, glycosylation and farnesylation.

Effector modules, including their SREs and payloads, may independently have 1, 2, 3, 4, 5, 6, 7, 8, 9, 10 or more PTMs which are the same or different.

Effector modules may be designed to include one or more structural or functional domain, repeat, or motif of a protein family. Such domains, repeats and motifs are categorized by protein family; and representative families are given in the EMBL-EBI database, located at http://www.ebi.ac.uk/.

### Polynucleotides of the invention

Effector modules, their SREs and payloads, may be nucleic acid-based. The term "nucleic acid," in its broadest sense, includes any compound and/or substance that comprise a polymer of nucleotides, e.g., linked nucleosides. These polymers are often referred to as polynucleotides. Exemplary nucleic acids or polynucleotides of the invention include, but are not limited to, ribonucleic acids (RNAs), deoxyribonucleic acids (DNAs), threose nucleic acids (TNAs), glycol nucleic acids (GNAs), peptide nucleic acids (PNAs), locked nucleic acids (LNAs, including **LNA** having a β- D-ribo configuration, α-**LNA** having an α-L-ribo configuration (a diastereomer of LNA), 2'-amino-LNA having a 2'-amino functionalization, and 2'-amino- α-LNA having a 2'-amino functionalization) or hybrids thereof.

In some embodiments, the nucleic acid molecule is a messenger RNA (mRNA). As used herein, the term "messenger RNA" (mRNA) refers to any polynucleotide which encodes a polypeptide of interest and which is capable of being translated to produce the encoded polypeptide of interest in vitro, in vivo, in situ or ex vivo. Polynucleotides of the invention may be mRNA or any nucleic acid molecule and may or may not be chemically modified.

Traditionally, the basic components of an mRNA molecule include at least a coding region, a 5'UTR, a 3'UTR, a 5' cap and a poly-A tail. Building on this wild type modular structure, the present invention expands the scope of functionality of traditional mRNA molecules by providing payload constructs which maintain a modular organization, but which comprise one or more structural and/or chemical modifications or alterations which impart useful properties to the

polynucleotide, for example tenability of function. As used herein, a "structural" feature or modification is one in which two or more linked nucleosides are inserted, deleted, duplicated, inverted or randomized in a polynucleotide without significant chemical modification to the nucleosides themselves. Because chemical bonds will necessarily be broken and reformed to effect a structural modification, structural modifications are of a chemical nature and hence are chemical modifications. However, structural modifications will result in a different sequence of nucleotides. For example, the polynucleotide "ATCG" may be chemically modified to "AT-5meC-G". The same polynucleotide may be structurally modified from "ATCG" to "ATCCCG". Here, the dinucleotide "CC" has been inserted, resulting in a structural modification to the polynucleotide.

In some embodiments, polynucleotides of the present invention may harbor 5'UTR sequences which play a role in translation initiation. 5'UTR sequences may include features such as Kozak sequences which are commonly known to be involved in the process by which the ribosome initiates translation of genes, Kozak sequences have the consensus XCCR(A/G) CCAUG, where R is a purine (adenine or guanine) three bases upstream of the start codon (AUG) and X is any nucleotide. In one embodiment, the Kozak sequence is ACCGCC. By engineering the features that are typically found in abundantly expressed genes of target cells or tissues, the stability and protein production of the polynucleotides of the invention can be enhanced.

Further provided are polynucleotides, which may contain an internal ribosome entry site (IRES) which play an important role in initiating protein synthesis in the absence of 5' cap structure in the polynucleotide. An IRES may act as the sole ribosome binding site, or may serve as one of the multiple binding sites. Polynucleotides of the invention containing more than one functional ribosome binding site may encode several peptides or polypeptides that are translated independently by the ribosomes giving rise to bicistronic and/or multicistronic nucleic acid molecules.

According to the present invention, effector modules, their SREs or payloads may be linked together through the 3'-end using nucleotides which are modified at the 3'-terminus. Chemical conjugation may be used to control the stoichiometry of delivery into cells. Polynucleotides encoding effector modules, their SREs or payloads can be designed to be conjugated to other polynucleotides, dyes, intercalating agents (e.g. acridines), cross-linkers (e.g. psoralene, mitomycin C), porphyrins (TPPC4, texaphyrin, Sapphyrin), polycyclic aromatic hydrocarbons (e.g., phenazine, dihydrophenazine), artificial endonucleases (e.g. EDTA), alkylating agents, phosphate, amino, mercapto, PEG (e.g., PEG-40K), MPEG, [MPEG]₂, polyamino, alkyl, substituted alkyl, radiolabeled markers, enzymes, haptens (e.g. biotin), transport/absorption facilitators (e.g., aspirin, vitamin E, folic acid), synthetic ribonucleases, proteins, e.g., glycoproteins, or peptides, e.g., molecules having a specific affinity for a co-ligand, or antibodies e.g., an antibody, that binds to a specified cell type such as a cancer cell, endothelial cell, or bone cell, hormones and hormone receptors, non-peptidic species, such as lipids, lectins, carbohydrates, vitamins, cofactors, or a drug. They may also be conjugated to, administered with, or further encode one or more of RNAi agents, siRNAs, shRNAs, miRNAs, miRNA binding sites, antisense RNAs, ribozymes, catalytic DNA, tRNA, RNAs that induce triple helix formation, aptamers or vectors, and the like.

Once any of the features have been identified or defined as a desired effector modules, their SREs or payloads to be encoded by the polynucleotides the invention, any of several manipulations and/or modifications of these features may be performed by moving, swapping, inverting, deleting, randomizing or duplicating. Furthermore, it is understood that manipulation of features may result in the same outcome as a modification to the molecules of the invention. For example, a manipulation which involved deleting a domain would result in the alteration of the length of a molecule just as modification of a nucleic acid to encode less than a full-length molecule would.

Modifications and manipulations can be accomplished by methods known in the art such as, but not limited to, site directed mutagenesis. The resulting modified molecules may then be tested for activity using in vitro or in vivo assays such as those described herein or any other suitable screening assay known in the art.

The term "identity" as known in the art, refers to a relationship between two or more sequences, as determined by comparing the sequences. In the art, identity also means the degree of sequence relatedness between sequences, as determined by the number of matches between strings of two or more residues (amino acid or nucleic acid). Identity measures the percent of identical matches between two or more sequences with gap alignments (if any) addressed by a particular mathematical model or computer program (i.e., "algorithms"). Identity of related sequences can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988).

In some embodiments, the variant sequence may have the same or a similar activity as the reference sequence. Alternatively, the variant may have an altered activity (e.g., increased or decreased) relative to a reference sequence. Generally, variants of a particular polynucleotide or polypeptide of the invention will have at least about 40%, 45%, 50%, 55%, 60%, 65%, 70%, 75%, 80%, 85%, 90%, 91%, 92%, 93%, 94%, 95%, 96%, 97%, 98%, 99% but less than 100% sequence identity to that particular reference polynucleotide or polypeptide as determined by sequence alignment programs and parameters described herein and known to those skilled in the art. Such tools for alignment include those of the BLAST suite (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402.)

### RNA Binding Domains

In one embodiment, the polynucleotides of the invention comprise at least one RNA-binding motif such as, but not limited to a RNA-binding domain (RBD).

RNA binding proteins (RBPs) can regulate numerous aspects of co- and post-transcription gene expression such as, but not limited to, RNA splicing, localization, translation, turnover, polyadenylation, capping, modification, export and localization. RNA-binding domains (RBDs), such as, but not limited to, RNA recognition motif (RR) and hnRNP K-homology (KH) domains, typically regulate the sequence association between RBPs and their RNA targets (Ray et al. Nature 2013. 499:172-177). In one embodiment, RBDs can bind short RNA sequences. In another embodiment, the RBDs can recognize structure RNAs.

### Exosome Quantification

In one embodiment, the polynucleotides of the present invention may be quantified in exosomes derived from one or more bodily fluid. As used herein "bodily fluids" include peripheral blood, serum, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, and umbilical cord blood. Alternatively, exosomes may be retrieved from an organ selected from the group consisting of lung, heart, pancreas, stomach, intestine, bladder, kidney, ovary, testis, skin, colon, breast, prostate, brain, esophagus, liver, and placenta.

It is advantageous to correlate the level of polynucleotides with one or more clinical phenotypes or with an assay for a human disease biomarker. The assay may be performed using construct specific probes, cytometry, qRT-PCR, real-time PCR, PCR, flow cytometry, electrophoresis, mass spectrometry, or combinations thereof while the exosomes may be isolated using immunohistochemical methods such as enzyme linked immunosorbent assay (ELISA) methods. Exosomes may also be isolated by size exclusion chromatography, density gradient centrifugation, differential centrifugation, nanomembrane ultrafiltration, immunoabsorbent capture, affinity purification, microfluidic separation, or combinations thereof.

### Chemical modifications to polynucleotides

According to the present invention, the terms "modification" or, as appropriate, "modified" polynucleotides refer to modification with respect to A, G, U (T in DNA) or C nucleotides.

Modifications of the polynucleotides of the invention may be on the nucleoside base and/or sugar portion of the nucleosides which comprise the polynucleotide. In some embodiments, multiple modifications are included in the modified nucleic acid or in one or more individual nucleoside or nucleotide. For example, modifications to a nucleoside may include one or more modifications to the nucleobase and the sugar. Modifications to the polynucleotides of the present invention may include any of those taught in, for example, International Publication WO2013052523.

As described herein "nucleoside" is defined as a compound containing a sugar molecule (e.g., a pentose or ribose) or a derivative thereof in combination with an organic base (e.g., a purine or pyrimidine) or a derivative thereof (also referred to herein as "nucleobase"). As described herein, "nucleotide" is defined as a nucleoside including a phosphate group.

The modified nucleotides, which may be incorporated into a polynucleotide can be modified on the internucleoside linkage (e.g., phosphate backbone). Herein, in the context of the polynucleotide backbone, the phrases "phosphate" and "phosphodiester" are used interchangeably. Backbone phosphate groups can be modified by replacing one or more of the oxygen atoms with a different substituent. Further, the modified nucleosides and nucleotides can include the wholesale replacement of an unmodified phosphate moiety with another internucleoside linkage. Examples of modified phosphate groups include, but are not limited to, phosphorothioate, phosphoroselenates, boranophosphates, boranophosphate esters, hydrogen phosphonates, phosphoramidates, phosphorodiamidates, alkyl or aryl phosphonates, and phosphotriesters. Phosphorodithioates have both non-linking oxygens replaced by sulfur. The phosphate linker can also be modified by the replacement of a linking oxygen with nitrogen (bridged phosphoramidates), sulfur (bridged phosphorothioates), and carbon (bridged methylene-phosphonates). Other modifications which may be used are taught in, for example, International Application WO2013052523.

### Nucleotide and Nucleosides modifications

Chemical modifications and/or substitution of the nucleotides or nucleobases of the polynucleotides of the invention which are useful in the present invention include, but are not limited to: (±)1-(2-Hydroxypropyl)pseudouridine TP, (2R)-1-(2-Hydroxypropyl)pseudouridine TP, (2S)-1-(2-Hydroxypropyl)pseudouridine TP, (E)-5-(2-Bromo-vinyl)ara-uridine TP, (E)-5-(2-Bromo-vinyl)cytidine TP, (E)-5-(2-Bromo-vinyl)uridine TP, (Z)-5-(2-Bromo-vinyl)ara-uridine TP, (Z)-5-(2-Bromo-vinyl)uridine TP, 1 (aminoalkylamino-carbonylethylenyl)-2(thio)-pseudouracil, 1 (aminoalkylaminocarbonylethylenyl)-2,4-(dithio)pseudouracil, 1 (aminoalkylaminocarbonylethylenyl)-4 (thio)pseudouracil, 1 (aminoalkylaminocarbonylethylenyl)-pseudouracil, 1 (aminocarbonylethylenyl)-2(thio)-pseudouracil, 1 (aminocarbonylethylenyl)-2,4-(dithio)pseudouracil, 1 (aminocarbonylethylenyl)-4 (thio)pseudouracil, 1 (aminocarbonylethylenyl)-pseudouracil, 1 substituted 2(thio)-pseudouracil, 1 substituted 2,4-(dithio)pseudouracil, 1 substituted 4 (thio)pseudouracil, 1 substituted pseudouracil, 1-(2,2,2-Trifluoroethyl)-pseudo-UTP , 1-(2,2,3,3,3-Pentafluoropropyl)pseudouridine TP, 1-(2,2-Diethoxyethyl)pseudouridine TP, 1-(2,4,6-Trimethylbenzyl)pseudouridine TP, 1-(2,4,6-Trimethyl-benzyl)pseudo-UTP , 1-(2,4,6-Trimethylphenyl)pseudo-UTP , 1-(2-Amino-2-carboxyethyl)pseudo-UTP, 1-(2-Amino-ethyl)pseudo-UTP, 1-(2-Hydroxyethyl)pseudouridine TP, 1-(2-Methoxyethyl)pseudouridine TP, 1-(3,4-Bis-trifluoromethoxybenzyl)pseudouridine TP, 1-(3,4-Dimethoxybenzyl)pseudouridine TP, 1-(3-Amino-3-carboxypropyl)pseudo-UTP , 1-(3-Amino-propyl)pseudo-UTP, 1-(3-Cyclopropyl-prop-2-ynyl)pseudouridine TP, 1-(4-Amino-4-carboxybutyl)pseudo-UTP, 1-(4-Amino-benzyl)pseudo-UTP, 1-(4-Amino-butyl)pseudo-UTP, 1-(4-Amino-phenyl)pseudo-UTP , 1-(4-Azidobenzyl)pseudouridine TP, 1-(4-Bromobenzyl)pseudouridine TP, 1-(4-Chlorobenzyl)pseudouridine TP, 1-(4-Fluorobenzyl)pseudouridine TP, 1-(4-lodobenzyl)pseudouridine TP, 1-(4-Methanesulfonylbenzyl)pseudouridine TP, 1-(4-Methoxybenzyl)pseudouridine TP, 1-(4-Methoxy-benzyl)pseudo-UTP, 1-(4-Methoxy-phenyl)pseudo-UTP , 1-(4-Methylbenzyl)pseudouridine TP, 1-(4-Methylbenzyl)pseudo-UTP , 1-(4-Nitrobenzyl)pseudouridine TP, 1-(4-Nitro-benzyl)pseudo-UTP, 1(4-Nitro-phenyl)pseudo-UTP , 1-(4-Thiomethoxybenzyl)pseudouridine TP, 1-(4-Trifluoromethoxybenzyl)pseudouridine TP, 1-(4-Trifluoromethylbenzyl)pseudouridine TP, 1-(5-Amino-pentyl)pseudo-UTP, 1-(6-Amino-hexyl)pseudo-UTP, 1-(aminoalkylamino-carbonylethylenyl)-2-(thio)-pseudouracil, 1-(aza)-2-(thio)-3-(aza)-phenoxazin-1-yl, 1,2'-O-dimethyladenosine, 1,2'-O-dimethylguanosine, 1,2'-O-dimethylinosine, 1,3-(diaza)-2-(oxo)-phenthiazin-1-yl, 1,3-(diaza)-2-(oxo)-phenoxazin-1-yl, 1,3,5-(triaza)-2,6-(dioxa)-naphthalene, 1,6-Dimethyl-pseudo-UTP, 1-[3-(2-{2-[2-(2-Aminoethoxy)-ethoxy]-ethoxy}-ethoxy)-propionyl]pseudouridine TP, 1-{3-[2-(2-Aminoethoxy)-ethoxy]-propionyl } pseudouridine TP, 1-Acetylpseudouridine TP, 1-Alkyl-6-(1-propynyl)-pseudo-UTP, 1-Alkyl-6-(2-propynyl)-pseudo-UTP, 1-Alkyl-6-allyl-pseudo-UTP, 1-Alkyl-6-ethynyl-pseudo-UTP, 1-Alkyl-6-homoallyl-pseudo-UTP, 1-Alkyl-6-vinyl-pseudo-UTP, 1-Allylpseudouridine TP, 1-Aminomethyl-pseudo-UTP , 1-Benzoylpseudouridine TP, 1-Benzyloxymethylpseudouridine TP, 1-Benzyl-pseudo-UTP, 1-Biotinyl-PEG2-pseudouridine TP, 1-Biotinylpseudouridine TP, 1-Butyl-pseudo-UTP , 1-carboxymethyl-pseudouridine, 1-Cyanomethylpseudouridine TP, 1-Cyclobutylmethyl-pseudo-UTP , 1-Cyclobutyl-pseudo-UTP, 1-Cycloheptylmethyl-pseudo-UTP , 1-Cycloheptyl-pseudo-UTP , 1-Cyclohexylmethyl-pseudo-UTP , 1-Cyclohexyl-pseudo-UTP, 1-Cyclooctylmethyl-pseudo-UTP , 1-Cyclooctyl-pseudo-UTP , 1-Cyclopentylmethyl-pseudo-UTP , 1-Cyclopentyl-pseudo-UTP , 1-Cyclopropylmethyl-pseudo-UTP , 1-Cyclopropyl-pseudo-UTP , 1-Deazaadenosine TP, 1-Ethyl-pseudo-UTP, 1-Hexyl-pseudo-UTP , 1-Homoallylpseudouridine TP, 1-Hydroxymethylpseudouridine TP, 1-iso-propyl-pseudo-UTP , 1-Me-2-thio-pseudo-UTP, 1-Me-4-thio-pseudo-UTP, 1-Me-alpha-thio-pseudo-UTP , 1-Me-GTP, 1-Methanesulfonylmethylpseudouridine TP, 1-Methoxymethylpseudouridine TP, 1-methyl-1-deaza-pseudoisocytidine, 1-methyl-1-deaza-pseudouridine, 1-Methyl-3-(3-amino-3-carboxypropyl) pseudouridine TP, 1-Methyl-3-(3-amino-3-carboxypropyl)pseudo-UTP , 1-methyl-3-(3-amino-5-carboxypropyl)pseudouridine, 1-Methyl-6-(2,2,2-Trifluoroethyl)pseudo-UTP, 1-Methyl-6-(4-morpholino)-pseudo-UTP, 1-Methyl-6-(4-thiomorpholino)-pseudo-UTP, 1-Methyl-6-(substituted phenyl)pseudo-UTP, 1-Methyl-6-amino-pseudo-UTP, 1-Methyl-6-azido-pseudo-UTP, 1-Methyl-6-bromo-pseudo-UTP, 1-Methyl-6-butyl-pseudo-UTP, 1-Methyl-6-chloro-pseudo-UTP, 1-Methyl-6-cyano-pseudo-UTP, 1-Methyl-6-dimethylamino-pseudo-UTP, 1-Methyl-6-ethoxy-pseudo-UTP, 1-Methyl-6-ethylcarboxylate-pseudo-UTP, 1-Methyl-6-ethyl-pseudo-UTP, 1-Methyl-6-fluoro-pseudo-UTP, 1-Methyl-6-formyl-pseudo-UTP, 1-Methyl-6-hydroxyamino-pseudo-UTP, 1-Methyl-6-hydroxy-pseudo-UTP, 1-Methyl-6-iodo-pseudo-UTP, 1-Methyl-6-iso-propyl-pseudo-UTP, 1-Methyl-6-methoxy-pseudo-UTP, 1-Methyl-6-methylamino-pseudo-UTP, 1-Methyl-6-phenyl-pseudo-UTP, 1-Methyl-6-propyl-pseudo-UTP, 1-Methyl-6-tert-butyl-pseudo-UTP, 1-methyl-6-thio-guanosine, 1-Methyl-6-trifluoromethoxy-pseudo-UTP, 1-Methyl-6-trifluoromethyl-pseudo-UTP, 1-methyladenosine, 1-methylguanosine, 1-methylinosine, 1-methylpseduouridine, 1-methyl-pseudoisocytidine, 1-methyl-pseudouridine, 1-Methyl-pseudo-UTP , 1-Morpholinomethylpseudouridine TP, 1-Pentyl-pseudo-UTP , 1-Phenyl-pseudo-UTP , 1-Pivaloylpseudouridine TP, 1-Propargylpseudouridine TP, 1-Propyl-pseudo-UTP , 1-propynyl-pseudouridine, 1-propynyl-uridine, 1-p-tolyl-pseudo-UTP , 1-taurinomethyl-1-methyl-uridine, 1-taurinomethyl-4-thio-uridine, 1-taurinomethyl-pseudouridine, 1-tert-Butyl-pseudo-UTP , 1-Thiomethoxymethylpseudouridine TP, 1-Thiomorpholinomethylpseudouridine TP, 1-Trifluoroacetylpseudouridine TP, 1-Trifluoromethyl-pseudo-UTP , 1-Vinylpseudouridine TP, 2 (amino)adenine, 2 (amino)purine, 2 (aminopropyl)adenine, 2 (methylthio) N6 (isopentenyl)adenine, 2 (propyl)guanine, 2 (thio)pseudouracil, 2' deoxy uridine, 2' fluorouridine, 2-(alkyl)adenine, 2-(alkyl)guanine, 2-(aminoalkyl)adenine, 2-(aminopropyl)adenine, 2-(halo)adenine, 2-(propyl)adenine, 2-(thio)cytosine, 2-(thio)uracil, 2-, 6-, 7- or 8-position of the purine base (A/G/inosine), 2,2'-anhydro-cytidine TP hydrochloride, 2,2'-anhydro-uridine TP, 2,2-dimethyl-guanosine, 2,4-(dithio)psuedouracil, 2,4,5-(trimethyl)phenyl, 2,4-diaminopurine, 2,6-(diamino)purine, 2,6-diaminopurine, 2' methyl, 2'amino, 2'azido, 2'fluro-cytidine, 2' methyl, 2'amino, 2'azido, 2'fluro-adenine, 2' methyl, 2'amino, 2'azido, 2'fluro-guanosine, 2'-Amino-2'-deoxy-ATP, 2'-Amino-2'-deoxy-CTP, 2'-Amino-2'-deoxy-GTP, 2'-Amino-2'-deoxy-UTP, 2'-Azido-2'-deoxy-ATP, 2'-Azido-2'-deoxy-CTP, 2'-Azido-2'-deoxy-GTP, 2'-Azido-2'-deoxy-UTP, 2'-Azido-deoxyuridine TP, 2'-bromo-deoxyuridine TP, 2'-F-5-Methyl-2'-deoxy-UTP, 2'Fluor-N4-Bz-cytidine TP, 2'Fluoro-N2-isobutyl-guanosine TP, 2'Fluoro-N4-Acetyl-cytidine TP, 2'Fluoro-N6-Bz-deoxyadenosine TP, 2'Fluro-N2-isobutyl-guanosine TP, 2'methyl, 2'amino, 2'azido, 2'fluro-uridine, 2'-OMe-2-Amino-ATP, 2'-OMe-5-Me-UTP, 2'-OMe-6-Me-UTP, 2'-OMe-pseudo-UTP, 2'-O-methyladenosine, 2'O-methyl-N2-isobutyl-guanosine TP, 2'-O-Methyl-N4-Acetyl-cytidine TP, 2'O-methyl-N4-Bz-cytidine TP, 2'O-methyl-N6-Bz-deoxyadenosine TP, 2'-O-methylpseudouridine, 2'-O-methyluridine, 2' deoxy uridine, 2' fluorouridine, 2'-O-methyladenosine, 2'-O-methylcytidine, 2'-O-methylguanosine, 2'-O-methyluridine, 2'-a-Ethynyladenosine TP, 2'-a-Ethynylcytidine TP, 2'-a-Ethynylguanosine TP, 2'-a-Ethynyluridine TP, 2'-amino-2'-deoxy-, 2'-amino-2'-deoxyadenosine, 2'-amino-2'-deoxycytidine, 2'-amino-2'-deoxyguanosine, 2'-amino-2'-deoxyribose, 2'-amino-2'-deoxyuridine, 2-amino-6-Chloro-purine, 2-Amino-A/U/G/C, 2-aminoadenine, 2-Aminoadenosine TP, 2-Amino-ATP, 2'-aminopropargyl, 2-aminopurine, 2-Amino-riboside-TP, 2'-araadenosine, 2'-aracytidine, 2'-arauridine, 2'-a-Trifluoromethyladenosine TP, 2'-a-Trifluoromethylcytidine TP, 2'-a-Trifluoromethylguanosine TP, 2'-a-Trifluoromethyluridine TP, 2-aza-inosinyl, 2'-azido-2'- deoxyuridine, 2'-Azido-2'-deoxyadenosine, 2'-azido-2'-deoxycytidine, 2'-azido-2'-deoxyguanosine, 2'-azido-2'-deoxyribose, 2'-azido-2'-deoxyuridine, 2'-Azido-2α-deoxyadenosine, 2-Azidoadenosine TP, 2'-b-Ethynyladenosine TP, 2'-b-Ethynylcytidine TP, 2'-b-Ethynylguanosine TP, 2'-b-Ethynyluridine TP, 2-Bromoadenosine TP, 2'-b-Trifluoromethyladenosine TP, 2'-b-Trifluoromethylcytidine TP, 2'-b-Trifluoromethylguanosine TP, 2'-b-Trifluoromethyluridine TP, 2'-C-alkyl oligoribonucleotide, 2-Chloroadenosine TP, 2'-deoxy- uridines, 2'-Deoxy-2',2'-difluoroadenosine TP, 2'-Deoxy-2',2'-difluorocytidine TP, 2'-Deoxy-2',2'-difluoroguanosine TP, 2'-Deoxy-2',2'-difluorouridine TP, 2'-Deoxy-2'-a-aminoadenosine TP, 2'-Deoxy-2'-a-aminocytidine TP, 2'-Deoxy-2'-a-aminoguanosine TP, 2'-Deoxy-2'-a-aminouridine TP, 2'-Deoxy-2'-a-azidoadenosine TP, 2'-Deoxy-2'-a-azidocytidine TP, 2'-Deoxy-2'-a-azidoguanosine TP, 2'-Deoxy-2'-a-azidouridine TP, 2'-Deoxy-2'-a-mercaptoadenosine TP, 2'-Deoxy-2'-a-mercaptocytidine TP, 2'-Deoxy-2'-a-mercaptoguanosine TP, 2'-Deoxy-2'-a-mercaptouridine TP, 2'-Deoxy-2'-a-thiomethoxyadenosine TP, 2'-Deoxy-2'-a-thiomethoxycytidine TP, 2'-Deoxy-2'-a-thiomethoxyguanosine TP, 2'-Deoxy-2'-a-thiomethoxyuridine TP, 2'-Deoxy-2'-b-aminoadenosine TP, 2'-Deoxy-2'-b-aminocytidine TP, 2'-Deoxy-2'-b-aminoguanosine TP, 2'-Deoxy-2'-b-aminouridine TP, 2'-Deoxy-2'-b-azidoadenosine TP, 2'-Deoxy-2'-b-azidocytidine TP, 2'-Deoxy-2'-b-azidoguanosine TP, 2'-Deoxy-2'-b-azidouridine TP, 2'-Deoxy-2'-b-bromoadenosine TP, 2'-Deoxy-2'-b-bromocytidine TP, 2'-Deoxy-2'-b-bromoguanosine TP, 2'-Deoxy-2'-b-bromouridine TP, 2'-Deoxy-2'-b-chloroadenosine TP, 2'-Deoxy-2'-b-chlorocytidine TP, 2'-Deoxy-2'-b-chloroguanosine TP, 2'-Deoxy-2'-b-chlorouridine TP, 2'-Deoxy-2'-b-fluoroadenosine TP, 2'-Deoxy-2'-b-fluorocytidine TP, 2'-Deoxy-2'-b-fluoroguanosine TP, 2'-Deoxy-2'-b-fluorouridine TP, 2'-Deoxy-2'-b-iodoadenosine TP, 2'-Deoxy-2'-b-iodocytidine TP, 2'-Deoxy-2'-b-iodoguanosine TP, 2'-Deoxy-2'-b-iodouridine TP, 2'-Deoxy-2'-b-mercaptoadenosine TP, 2'-Deoxy-2'-b-mercaptocytidine TP, 2'-Deoxy-2'-b-mercaptoguanosine TP, 2'-Deoxy-2'-b-mercaptouridine TP, 2'-Deoxy-2'-b-thiomethoxyadenosine TP, 2'-Deoxy-2'-b-thiomethoxycytidine TP, 2'-Deoxy-2'-b-thiomethoxyguanosine TP, 2'-Deoxy-2'-b-thiomethoxyuridine TP, 2'-deoxy-2'-C-alkyl oligoribonucleotide, 2'-deoxy-2'-deamine oligoribonucleotide, 2'-deoxy-2'-fluoro-oligoribonucleotide, 2'-deoxy-A/U/G/C, 2'-deoxyuridine, 2'-fluoro- uridines, 2'-fluoro-2'-deoxy, 2'-fluoro-2'-deoxyadenosine, 2'-fluoro-2'-deoxycytidine, 2'-fluoro-2'-deoxyguanosine, 2'fluoro-2'-deoxyribose, 2'-fluoro-2'-deoxyuridine, 2'-fluoro-A/U/G/C, 2-Fluoroadenosine TP, 2'fluoroC/2thioU, 2'-fluoro-modified bases, 2'-fluorothymidine, 2-lodoadenosine TP, 2'MeA/2thioU, 2'MeC/2thioU, 2'MeG/2thioU, 2-Mercaptoadenosine TP, 2-methoxy-4-thio-pseudouridine, 2-methoxy-4-thio-uridine, 2-methoxy-5-methyl-cytidine, 2-methoxy-adenine, 2-methoxy-cytidine, 2'-methoxyethyl, 2-methoxyuridine, 2-methyladenosine, 2-methyl-guanosine, 2-methylpseudouridine, 2-methylthio-adenine, 2-methylthio-N6-(cis-hydroxyisopentenyl)adenosine, 2-methylthio-N6-hydroxynorvalyl carbamoyladenosine, 2-methylthio-N6-methyladenosine, 2-methylthio-N6-threonyl carbamoyladenosine, 2'-methyluridine, 2'-O- methyl-2'-deoxyguanosine, 2'-O-(3-aminopropyl), 2'-O-alkenyl-A/U/G/C, 2'-O-alkinyl-A/U/G/C, 2'-O-alkyl-oligoribonucleotide, 2'-O-allyl-A/U/G/C, 2'-O-butyl-A/U/G/C, 2'-O-fluoro (2'-OF), 2'-OH-ara-adenosine TP, 2'-OH-ara-cytidine TP, 2'-OH-ara-guanosine TP, 2'-OH-ara-uridine TP, 2'-O-methyl (2'-OMe), 2'-O-methyl adenosine, 2'-O-methyl guanosine, 2'-O-Methyl inosine, 2'-O-methyl uridine, 2'-O-methyl-2-aminoadenosine, 2'-O-methyl-2'-deoxy-, 2'-O-Methyl-2'-deoxyadenosine, 2'-O-methyl-2'-deoxycytidine, 2'-O-Methyl-2'-deoxyguanosine, 2'-O-methyl-2'-deoxyuridine, 2'-O-Methyl-5-(1-propynyl)cytidine TP, 2'-O-Methyl-5-(1-propynyl)uridine TP, 2'-O-methyl-5-methyluridine, 2'-O-methylinosine, 2'-O-methylpseudouridine, 2'-O-methyl-ribose, 2'-O-propyl-A/U/G/C, 2'-O-ribosyladenosine (phosphate), 2'-O-ribosylguanosine (phosphate), 2-oxo-7-aminopyridopyrimidin-3-yl, 2-oxo-pyridopyrimidine-3-yl, 2'-position of the sugar of adenosine, 2'-position of the sugar of cytidine, 2'-position of the sugar of guanosine, 2'-position of the sugar of inosine, 2'-position of the sugar of uridine, 2'-propinyl-A/U/G/C, 2-pyridinone, 2-thio-1-methyl-1-deaza-pseudouridine, 2-thio-1-methyl-pseudouridine, 2-thio-2'-O-methyluridine, 2-thio-5-aza-uridine, 2-thio-5-methyl-cytidine, 2-thiocytidine, 2-thio-dihydropseudouridine, 2-thio-dihydrouridine, 2-thio-pseudouridine, 2thioU/2'aminoC, 2thioU/2'azidoG, 2-thiouridine, 2-thio-zebularine, 2-Trifluoromethyladenosine TP, 3 (3 amino-3 carboxypropyl)uracil, 3 (deaza) 5 (aza)cytosine, 3 (methyl)cytosine, 3 nitropyrrole, 3-(3-amino-3-carboxypropyl)uridine, 3-(3-Amino-3-carboxypropyl)-Uridine TP, 3-(alkyl)cytosine, 3-(deaza) 5 (aza)cytosine, 3-(methyl)-7-(propynyl)isocarbostyrilyl, 3-(methyl)cytidine, 3-(methyl)isocarbostyrilyl, 3,2'-O-dimethyluridine, 3,2'-O-Dimethyluridine TP, 3-Alkyl-pseudo-UTP, 3-Deaza-3-bromoadenosine TP, 3-Deaza-3-chloroadenosine TP, 3-Deaza-3-fluoroadenosine TP, 3-Deaza-3-iodoadenosine TP, 3-Deazaadenosine TP, 3'-Ethynylcytidine TP, 3-methylcytidine, 3-Methyl-pseudo-Uridine TP, 3-methyluridine, 4 (thio)pseudouracil, 4-(fluoro)-6-(methyl)benzimidazole, 4-(methyl)benzimidazole, 4-(methyl)indolyl, 4-(thio)pseudouracil, 4-(thio)uracil, 4-, 5- or 6-position of the pyrimidine base (C/U), 4,2'-O-dimethylcytidine, 4,6-(dimethyl)indolyl, 4-acetyl-cytosine, 4'-Azidoadenosine TP, 4'-Azidocytidine TP, 4'-Azidoguanosine TP, 4'-Azidouridine TP, 4'-Carbocyclic adenosine TP, 4'-Carbocyclic cytidine TP, 4'-Carbocyclic guanosine TP, 4'-Carbocyclic uridine TP, 4-demethylwyosine, 4'-Ethynyladenosine TP, 4'-Ethynylcytidine TP, 4'-Ethynylguanosine TP, 4'-Ethynyluridine TP, 4-methoxy-1-methyl-pseudoisocytidine, 4-methoxy-2-thio-pseudouridine, 4-methoxy-pseudoisocytidine, 4-methoxy-pseudouridine, 4-methylcytidine, 4-thio-1-methyl-1-deaza-pseudoisocytidine, 4-thio-1-methyl-pseudoisocytidine, 4-thio-1-methyl-pseudouridine, 4-thio-A/U/G/C, 4-thio-pseudoisocytidine, 4-thio-pseudouridine, 4-Thio-pseudo-UTP , 4thioU/2thioU, 4-thiouracil, 4-thiouridine, 5 (1,3-diazole-1-alkyl)uracil, 5 (2-aminopropyl)uracil, 5 (aminoalkyl)uracil, 5 (dimethylaminoalkyl)uracil, 5 (guanidiniumalkyl)uracil, 5 (halo)cytosine, 5 (methoxycarbonylmethyl)-2-(thio)uracil, 5 (methoxycarbonyl-methyl)uracil, 5 (methyl) 2 (thio)uracil, 5 (methyl) 2,4 (dithio)uracil, 5 (methyl) 4 (thio)uracil, 5 (methyl)cytosine, 5 (methylaminomethyl)-2 (thio)uracil, 5 (methylaminomethyl)-2,4 (dithio)uracil, 5 (methylaminomethyl)-4 (thio)uracil, 5 (propynyl)cytosine, 5 (propynyl)uracil, 5 (trifluoromethyl)cytosine, 5 (trifluoromethyl)uracil, 5 nitroindole, 5 substituted pyrimidines, 5-(1-Propynyl)ara-cytidine TP, 5-(1-Propynyl)ara-uridine TP, 5-(2-aminopropyl)uracil, 5-(2-carbomethoxyvinyl)uridine TP, 5-(2-Chloro-phenyl)-2-thiocytidine TP, 5-(2-Furanyl)uridine TP, 5-(4-Amino-phenyl)-2-thiocytidine TP, 5-(alkyl)-2-(thio)pseudouracil, 5-(alkyl)-2,4 (dithio)pseudouracil, 5-(alkyl)-4 (thio)pseudouracil, 5-(alkyl)cytosine, 5-(alkyl)pseudouracil, 5-(alkyl)uracil, 5-(alkynyl)cytosine, 5-(alkynyl)uracil, 5-(allylamino)uracil, 5-(C1-C6)-alkylcytosine, 5-(C1-C6)-alkyluracil, 5-(C2-C6)-alkenylcytosine, 5-(C2-C6)-alkenyluracil, 5-(C2-C6)-alkynylcytosine, 5-(C2-C6)-alkynyluracil, 5-(carboxyhydroxymethyl)pyrimidine methyl ester-, 5-(carboxyhydroxymethyl)uridine, 5-(carboxyhydroxymethyl)uridine methyl ester, 5-(cyanoalkyl)uracil, 5-(dialkylaminoalkyl)uracil, 5-(dimethylaminoalkyl)uracil, 5-(guanidiniumalkyl)uracil, 5-(halo)cytosine, 5-(halo)uracil, 5-(hydroxymethyl)uracil, 5-(iso-Pentenylaminomethyl)- 2-thiouridine TP, 5-(iso-Pentenylaminomethyl)-2'-O-methyluridine TP, 5-(iso-Pentenylaminomethyl)uridine TP, 5-(l,3-diazole-I-alkyl)uracil, 5-(methoxy)uracil, 5-(methoxycarbonylmethyl)-2-(thio)uracil, 5-(methoxycarbonyl-methyl)uracil, 5-(methyl) 2(thio)uracil, 5-(methyl) 2,4 (dithio)uracil, 5-(methyl) 4 (thio)uracil, 5-(methyl)-2-(thio)pseudouracil, 5-(methyl)-2,4 (dithio)pseudouracil, 5-(methyl)-4 (thio)pseudouracil, 5-(methyl)isocarbostyrilyl, 5-(methyl)pseudouracil, 5-(methylaminomethyl)-2 (thio)uracil, 5-(methylaminomethyl)-2,4(dithio)uracil, 5-(methylaminomethyl)-4-(thio)uracil, 5-(propynyl)cytosine, 5-(propynyl)uracil, 5-(trifluoromethyl)cytosine, 5-(trifluoromethyl)uracil, 5,2'-O-dimethylcytidine, 5,2'-O-dimethyluridine, 5,6-dihydro-uridine, 5-aminoallyl-A/U/G/C, 5-Aminoallyl-CTP, 5-Aminoallyl-deoxy-uridine, 5-aminoallyl-uridine, 5-aminomethyl-2-seleno-A/U/G/C, 5-aminomethyl-2-thio-A/U/G/C, 5-aminomethyl-2-thiouridine, 5-aminomethyl-A/U/G/C, 5-aminopropyl-2'-amino-A/U/G/C, 5-aminopropyl-2'-deoxy-A/U/G/C, 5-aminopropyl-2'-fluoro-A/U/G/C, 5-aminopropyl-2'-O-methyl-A/U/G/C, 5-aminopropyl-A/U/G/C, 5-aminouracil, 5-aza-2-thio-zebularine, 5-aza-cytidine, 5-aza-uridine, 5-aza-zebularine, 5-Bromo- 2'-deoxyuridine, 5-Bromo-2'- deoxycytidine, 5-bromo-A/U/G/C, 5-bromo-cytidine, 5-bromo-uridine, 5-carbamoylmethyl-2'-O-methyluridine, 5-carbamoylmethyl-A/U/G/C, 5-carbamoylmethyluridine, 5-Carbamoylmethyluridine TP, 5-carboxyhydroxymethyl-A/U/G/C, 5-carboxyhydroxymethyluridine, 5-carboxyhydroxymethyluridine methyl ester, 5-carboxymethyl-A/U/G/C, 5-carboxymethylaminomethyl-2'-O-methyluridine, 5-carboxymethylaminomethyl-2-thio-A/U/G/C, 5-carboxymethylaminomethyl-2-thiouridine, 5-carboxymethylaminomethyl-A/U/G/C, 5-carboxymethylaminomethyluridine, 5-carboxymethyluridine, 5-chloro-ara-cytosine, 5-chlorocytosine, 5-chlorouracil, 5-Crbamoylmethyluridine TP, 5-Cyanocytidine TP, 5-Cyanouridine TP, 5-Dimethylaminouridine TP, 5-Ethynylara-cytidine TP, 5-Ethynylcytidine TP, 5-fluoro-A/U/G/C, 5-fluorocytosine, 5-fluoro-uridine, 5-formyl-2'-O-methylcytidine, 5-formyl-A/U/G/C, 5-formylcytidine, 5'-Homo-adenosine TP, 5'-Homo-cytidine TP, 5'-Homo-guanosine TP, 5'-Homo-uridine TP, 5-hydro-A/U/G/C, 5-hydroxy-A/U/G/C, 5-hydroxycytosine, 5-hydroxydeoxycytidine, 5-hydroxymethyl-A/U/G/C, 5-hydroxymethylcytidine, 5-hydroxymethyldeoxycytidine, 5-hydroxyuridine, 5-iodo- 2'-deoxycytidine, 5-iodo-2'-fluoro-deoxyuridine TP, 5-iodo-2'-deoxycytidine, 5-iodo-2'-deoxyuridine, 5-iodo-A/U/G/C, 5-iodo-cytidine, 5-iodoU/2thioU, 5-iodoU/4thioU, 5-iodo-uridine, 5-isopentenylaminomethyl-2'-amino-A/U/G/C, 5-isopentenylaminomethyl-2'-deoxy-A/U/G/C, 5-isopentenylaminomethyl-2'-fluoro-A/U/G/C, 5-isopentenylaminomethyl-2'-O-methyl-A/U/G/C, 5-isopentenylaminomethyl-2-thio-2'-amino-A/U/G/C, 5-isopentenylaminomethyl-2-thio-2'-deoxy-A/U/G/C, 5-isopentenylaminomethyl-2-thio-2'-fluoro-A/U/G/C, 5-isopentenylaminomethyl-2-thio-2'-O-methyl-A/U/G/C, 5-isopentenylaminomethyl-2-thio-A/U/G/C, 5-isopentenylaminomethyl-A/U/G/C, 5mC/2thioU, 5mC/2thioU/2'aminoC, 5mC/2thioU/2'azidoG, 5mC/4thioU, 5mC/pseudoU, 5-methoxy-A/U/G/C, 5-methoxyaminomethyl-2-thio-uridine, 5-methoxycarbonyl methyl-A/U/G/C, 5-methoxycarbonylmethyl-2'-O-methyluridine, 5-methoxycarbonylmethyl-2-thioA/U/GIC, 5-methoxycarbonylmethyl-2-thiouridine, 5-methoxycarbonylmethyluridine, 5-Methoxycytidine TP, 5-methoxy-ethoxy-methyl-2'-amino-A/U/G/C, 5-methoxy-ethoxy-methyl-2'-deoxy-A/U/G/C, 5-methoxy-ethoxy-methyl-2'-fluoro-A/U/G/C, 5-methoxy-ethoxy-methyl-2'-O-methyl-A/U/G/C, 5-methoxy-ethoxy-methyl-A/U/G/C, 5-methoxyuridine, 5-methyl-2-thio-A/U/G/C, 5-methyl-2-thiouridine, 5-methyl-A/U/G/C, 5-methylaminomethyl-2-selenouridine, 5-methylaminomethyl-2-thiouridine, 5-methylaminomethyl-A/U/G/C, 5-methylaminomethyluridine, 5-methylcytosine, 5-methyldihydro-A/U/G/C, 5-Methyldihydrouridine, 5-methyluridine, 5-methyl-zebularine, 5-nitro-A/U/G/C, 5-nitroindole, 5-oxyacetic acid methyl ester-A/U/G/C, 5-Oxyacetic acid- Uridine TP, 5-oxyacetic acid-A/U/G/C, 5-Oxyacetic acid-methyl ester-Uridine TP, 5-Phenylethynyluridine TP, 5-propynyl cytosine, 5-propynyl uracil, 5-Propynyl-2'-deoxyuridine, 5-Propynyl-2'-deoxycytidine, 5-propynyl-A/U/G/C, 5-taurinomethy)-2-thiouridine-2'-deoxy-A/U/G/C, 5-taurinomethyl-2'-amino-A/U/G/C, 5-taurinomethyl-2'-fluoro-A/U/G/C, 5-taurinomethyl-2'-O-methyl-A/U/G/C, 5-taurinomethyl-2-thiouridine, 5-taurinomethyl-2-thiouridine-2'-amino-A/U/G/C, 5-taurinomethyl-2-thiouridine-2'-fluoro-A/U/G/C, 5-taurinomethyl-2-thiouridine-2'-O-methyl-A/U/G/C, 5-taurinomethyl-A/U/G/C, 5-taurinomethyluridine, 5-Trideuteromethyl-6-deuterouridine TP, 5-Trifluoromethyl-Cytidine TP, 5-TrifluoromethylUridine TP, 5-uracil, 5-Vinylarauridine TP, 6 (alkyl)adenine, 6 (azo)uracil, 6 (methyl)adenine, 6 (methyl)guanine, 6-(2,2,2-Trifluoroethyl)-pseudo-UTP, 6-(4-Morpholino)-pseudo-UTP, 6-(4-Thiomorpholino)-pseudo-UTP, 6-(alkyl)adenine , 6-(alkyl)guanine, 6-(aza)pyrimidine, 6-(azo)cytosine, 6-(azo)thymine, 6-(azo)uracil, 6-(methyl)-7-(aza)indolyl, 6-(methyl)adenine, 6-(methyl)guanine, 6-(Substituted-Phenyl)-pseudo-UTP, 6-Amino-pseudo-UTP, 6-aza-2'-amino-A/U/G/C, 6-aza-2'-deoxy-A/U/G/C, 6-aza-2'-fluoro-A/U/G/C, 6-aza-2'-O-methyl-A/U/G/C, 6-aza-A/U/G/C, 6-aza-cytidine, 6-azauridine, 6-aza-uridine, 6-Azido-pseudo-UTP, 6-Bromo-pseudo-UTP , 6-Butyl-pseudo-UTP, 6-chloro-7-deaza-guanosine, 6-Chloro-pseudo-UTP , 6-chloro-purine, 6-Cyano-pseudo-UTP, 6-Dimethylamino-pseudo-UTP, 6-Ethoxy-pseudo-UTP, 6-Ethylcarboxylate-pseudo-UTP, 6-Ethyl-pseudo-UTP, 6-Fluoro-pseudo-UTP , 6-Formyl-pseudo-UTP, 6-Hydroxyamino-pseudo-UTP, 6-Hydroxy-pseudo-UTP, 6-lodo-pseudo-UTP , 6-iso-Propyl-pseudo-UTP, 6-mercapto-guanosine, 6-methoxy-guanosine, 6-Methoxy-pseudo-UTP , 6-methyladenosine, 6-Methylamino-pseudo-UTP, 6-methyl-guanosine, 6-methyl-mercaptopurine, 6-Methyl-pseudo-UTP , 6-Phenyl-pseudo-UTP, 6-Phenyl-pseudo-UTP , 6-phenyl-pyrrolo-pyrimidin-2-on-3-yl, 6-Propyl-pseudo-UTP, 6-tert-Butyl-pseudo-UTP, 6-thio-7-deaza-8-aza-guanosine, 6-thio-7-deaza-guanosine, 6-thio-7-methyl-guanosine, 6-thiodeoxyguanosine, 6-thio-guanosine, 6-Trifluoromethoxy-pseudo-UTP, 6-Trifluoromethyl-pseudo-UTP , 7 (alkyl)guanine, 7 (deaza)adenine, 7 (deaza)guanine, 7 (methyl)guanine, 7-(alkyl)guanine, 7-(aminoalkylhydroxy)-1-(aza)-2-(thio)-3-(aza)-phenthiazin-I-yl, 7-(aminoalkylhydroxy)-1-(aza)-2-(thio)-3-(aza)-phenoxazin-1-yl, 7-(aminoalkylhydroxy)-1,3-(diaza)-2-(oxo)-phenoxazin-1-yl, 7-(aminoalkylhydroxy)-l,3-(diaza)-2-(oxo)-phenthiazin-I-yl, 7-(aminoalkylhydroxy)-l,3-(diaza)-2-(oxo)-phenthiazin-1-yl , 7-(aminoalkylhydroxy)-l,3-(diaza)-2-(oxo)-phenoxazin-l-yl, 7-(aminoalkylhydroxy)-l,3-(diaza)-2-(oxo)-phenoxazin-l-yl , 7-(aza)indolyl, 7-(deaza)guanine, 7-(guanidiniumalkylhydroxy)-1-(aza)-2-(thio)-3-(aza)-phenoxazinl-yl, 7-(guanidiniumalkylhydroxy)-1-(aza)-2-(thio)-3-(aza)-phenthiazin-l-yl, 7-(guanidiniumalkylhydroxy)-1-(aza)-2-(thio)-3-(aza)-phenoxazin-1-yl, 7-(guanidiniumalkylhydroxy)-1,3-(diaza)-2-(oxo)-phenoxazin-1-yl, 7-(guanidiniumalkyl-hydroxy)-l,3-(diaza)-2-(oxo)-phenthiazin-l-yl, 7-(guanidiniumalkyl-hydroxy)-l,3-(diaza)-2-(oxo)-phenthiazin-1-yl , 7-(guanidiniumalkylhydroxy)-l,3-(diaza)-2-(oxo)-phenoxazin-I-yl, 7-(methyl)guanine, 7-(propynyl)isocarbostyrilyl, 7-(propynyl)isocarbostyrilyl, propynyl-7-(aza)indolyl, 7-allyl-8-oxoguanosine, 7-aminomethyl-7-deazaguanosine, 7-cyano-7-deazaguanosine, 7-deaza-2,6-diaminopurine, 7-Deaza-2'-amino-A/U/G/C, 7-deaza-2-amino-purine, 7-Deaza-2'-deoxy-A/U/G/C, 7-deaza-2'-deoxy-guanosine, 7-Deaza-2'-fluoro-A/U/G/C, 7-Deaza-2'-O-methyl-A/U/G/C, 7-deaza-7-substituted purine, 7-deaza-8-aza-2,6-diaminopurine, 7-deaza-8-aza-2-aminopurine, 7-deaza-8-aza-adenine, 7-deaza-2-aminopurine, 7-deaza-8-aza-adenosine, 7-deaza-8-aza-guanosine, 7-deaza-8-substituted purine, 7-Deaza-A/U/G/C, 7-deaza-adenine, 7-deaza-adenosine, 7-deaza-guanosine, 7-deaza-inosinyl, 7-methyl-8-oxo-guanosine, 7-methyladenine, 7-methylguanosine, 7-methylinosine, 7-substituted 1-(aza)-2-(thio)-3-(aza)-phenoxazin-1-yl, 7-substituted 1,3-(diaza)-2-(oxo)-phenoxazin-1-yl, 8 (alkenyl)adenine, 8 (alkyl)guanine, 8 (alkynyl)adenine, 8 (alkynyl)guanine, 8 (amino)adenine, 8 (halo)guanine, 8 (thioalkyl)adenine, 8 (thioalkyl)guanine, 8-(alkenyl)adenine, 8-(alkenyl)guanine, 8-(alkyl)adenine, 8-(alkyl)guanine, 8-(alkynyl)adenine, 8-(alkynyl)guanine, 8-(amino)adenine, 8-(amino)guanine, 8-(halo)adenine, 8-(halo)guanine, 8-(hydroxyl)adenine, 8-(hydroxyl)guanine, 8-(thioalkyl)adenine, 8-(thioalkyl)guanine, 8-(thiol)adenine, 8-(thiol)guanine, 8-Aza-2'-amino-A/U/G/C, 8-Aza-2'-deoxy-A/U/G/C, 8-Aza-2'-fluoro-A/U/G/C, 8-Aza-2'-O-methyl-A/U/G/C, 8-Aza-A/U/G/C, 8-Aza-ATP, 8-azapurine, 8-Azido-2 '-amino-A/U/G/C, 8-Azido-2'-deoxy-A/U/G/C, 8-Azido-2'-fluoro-A/U/G/C, 8-Azido-2'-O-methyl-A/U/G/C, 8-Azido-A/U/G/C, 8-azido-adenosine, 8-bromo-adenosine TP, 8-bromo-guanosine TP, 8-mercapto-guanosine, 8-oxo-guanosine, 8-Trifluoromethyladenosine TP, 9-(methyl)-imidizopyridinyl, 9-Deazaadenosine TP, 9-Deazaguanosine TP, allyamino-thymidine, allyamino-uracil, Alpha-thio-pseudo-UTP , aminoindolyl, anthracenyl, archaeosine, aza adenine, aza cytosine, aza guanine, aza thymidine, aza uracil, azidotriphosphate, benzimidazole, beta-D-mannosyl-queosine, bis-ortho-(aminoalkylhydroxy)-6-phenyl-pyrrolo-pyrimidin-2-on-3-yl, bis-ortho-substituted-6-phenyl-pyrrolo-pyrimidin-2-on-3-yl, cytidine 5'-O-(1-thiophosphate), deaza adenine, deaza cytosine, deaza guanine, deaza thymidine, deaza uracil, deoxy-cytosine, deoxy-inosine, deoxyribonucleotides of C5-propynylpyrimidines, deoxyribonucleotides of diaminopurine, deoxyribonucleotides of nitropyrrole, deoxy-thymidine, difluorotolyl, dihydropseudouridine, dihydrouridine, epoxyqueuosine, Formycin A TP, Formycin B TP, galactosyl-queuosine, hydroxywybutosine, hypoxanthine, imidizopyridinyl, inosine, inosinyl, isocarbostyrilyl, isoguanisine, isopentenyladenosine, isowyosine, lysidine, mannosylqueuosine, methylphosphonates, methylphosphoramidates, methylwyosine, N (methyl)guanine, N-(methyl)guanine, N<2>-dimethylguanine, N1-methyl-adenosine, N1-methyl-guanosine, N1-methyl-pseudo-uridine, N2,7,2'-O-trimethylguanosine, N2,7-dimethylguanosine, N2,N2,7-trimethylguanosine, N2,N2-dimethyl-6-thio-guanosine, N2,N2-dimethylguanosine, N2-isobutyl-guanosine TP, N2-methyl-6-thio-guanosine, N2-methylguanosine, N2-substituted purines, N3 (methyl)uracil, N4 (acetyl)cytosine, N4,2'-O-dimethylcytidine, N4,N4-Dimethyl-2'-OMe-Cytidine TP, N4-acetyl-2'-O-methylcytidine, N4-acetylcytidine, N4-alkylcytosine, N4-alkyldeoxycytidine, N4-Amino-cytidine TP, N4-Benzoyl-cytidine TP, N4-ethylcytosine, N4-ethyldeoxycytidine, N4-methylcytidine, N6 (methyl)adenine, N6-([6-aminohexyl]carbamoylmethyl)-adenosine, N6-(19-Amino-pentaoxanonadecyl)adenosine TP, N6-(cis-hydroxyisopentenyl)adenosine, N6-(isopentyl)adenine, N6, N6 (dimethyl)adenine, N6,2'-O-dimethyladenosine, N6,N6,2'-O-trimethyladenosine, N6,N6-dimethyladenosine, N6-acetyladenosine, N6-cis-hydroxy-isopentenyl-adenosine, N6-glycinylcarbamoyladenosine, N6-hydroxynorvalylcarbamoyladenosine, N6-isopentenyladenosine, N6-methyl-2-amino-purine, N6-methyladenosine, N6-methyl-N6-threonylcarbamoyladenosine, N6-substituted purines, N6-threonylcarbamoyladenosine, N7-methylguanosine, N7-methylinosine, N7-methyl-xanthosine, N-alkylated derivative, napthalenyl, nitrobenzimidazolyl, nitroimidazolyl, nitroindazolyl, nitropyrazolyl, nubularine, N-uracil , N-uracil-5-oxyacetic acid, N-uracil-5-oxyacetic acid methyl ester, O6-methylguanosine, O6-substituted purines, O-alkylated derivative, ortho-(aminoalkylhydroxy)-6-phenyl-pyrrolo-pyrimidin-2-on-3-yl, ortho-substituted-6-phenyl-pyrrolo-pyrimidin-2-on-3-yl, Oxoformycin TP, P seudo-UTP-1-2-ethanoic acid, para-(aminoalkylhydroxy)-6-phenyl-pyrrolo-pyrimidin-2-on-3-yl, para-substituted-6-phenyl-pyrrolo-pyrimidin-2-on-3-yl, pentacenyl, peroxywybutosine, phenanthracenyl, phenyl-A/U/G/C, propynyl-7-(aza)indolyl, pseudoisocytidine, Pseudo-iso-cytidine, pseudouracil, pseudouridine, Pseudouridine 1-(4-methylbenzenesulfonic acid) TP, Pseudouridine 1-(4-methylbenzoic acid) TP, Pseudouridine TP 1-[3-(2-ethoxy)]propionic acid, Pseudouridine TP 1-[3-{2-(2-[2-(2-ethoxy)-ethoxy]-ethoxy)-ethoxy}]propionic acid, Pseudouridine TP 1-[3-{2-(2-[2-{2(2-ethoxy)-ethoxy}-ethoxy]-ethoxy)-ethoxy}]propionic acid, Pseudouridine TP 1-[3-{2-(2-[2-ethoxy]-ethoxy)-ethoxy}]propionic acid, Pseudouridine TP 1-[3-{2-(2-ethoxy)-ethoxy}] propionic acid, Pseudouridine TP 1-methylphosphonic acid, Pseudouridine TP 1-methylphosphonic acid diethyl ester, Pseudo-UTP-N1-3-propionic acid, Pseudo-UTP-N1-4-butanoic acid, Pseudo-UTP-N1-5-pentanoic acid, Pseudo-UTP-N1-6-hexanoic acid, Pseudo-UTP-N1-7-heptanoic acid, Pseudo-UTP-N1-methyl-p-benzoic acid, Pseudo-UTP-N1-p-benzoic acid, puromycin, pyrenyl, pyridin-4-one ribonucleoside, pyridopyrimidin-3-yl, pyridopyrimidin-3-yl, 2-oxo-7-amino-pyridopyrimidin-3-yl, pyrrolo-cytidine, pyrrolo-pseudoisocytidine, pyrrolo-pyrimidin-2-on-3-yl, pyrrolopyrimidinyl, pyrrolopyrizinyl, Pyrrolosine TP, queuosine, Sp diasteriomers of ribonucleosid-5'-O-(1-thiotriphosphates), stilbenzyl, substituted 1,2,4-triazoles, substituted 7 deazapurine, tetracenyl, tubercidine, undermodified hydroxywybutosine, uridine 5-oxyacetic acid, uridine 5-oxyacetic acid methyl ester, wybutosine, wyosine, xanthine, Xanthosine-5'-TP, xylo-adenosine, zebularine, α-thio-adenosine, α-thio-cytidine, α-thio-guanosine, and/or α-thio-uridine. In the preceeding list, the abbreviation "TP" stands for triphosphate but it shoud be understood that the mono- and di-phosphates may also be utilized.

Polynucleotides of the present invention may comprise one or more of the modifications taught herein.

Different sugar modifications, nucleotide modifications, and/or internucleoside linkages (e.g., backbone structures) may exist at various positions in the polynucleotide. One of ordinary skill in the art will appreciate that the nucleotide analogs or other modification(s) may be located at any position(s) of a polynucleotide such that the function of the polynucleotide is not substantially decreased. A modification may also be a 5' or 3' terminal modification. The polynucleotide may contain from about 1% to about 100% modified nucleotides (either in relation to overall nucleotide content, or in relation to one or more types of nucleotide, i.e. any one or more of A, G, U or C) or any intervening percentage (e.g., from 1% to 20%, from 1% to 25%, from 1% to 50%, from 1% to 60%, from 1% to 70%, from 1% to 80%, from 1% to 90%, from 1% to 95%, from 10% to 20%, from 10% to 25%, from 10% to 50%, from 10% to 60%, from 10% to 70%, from 10% to 80%, from 10% to 90%, from 10% to 95%, from 10% to 100%, from 20% to 25%, from 20% to 50%, from 20% to 60%, from 20% to 70%, from 20% to 80%, from 20% to 90%, from 20% to 95%, from 20% to 100%, from 50% to 60%, from 50% to 70%, from 50% to 80%, from 50% to 90%, from 50% to 95%, from 50% to 100%, from 70% to 80%, from 70% to 90%, from 70% to 95%, from 70% to 100%, from 80% to 90%, from 80% to 95%, from 80% to 100%, from 90% to 95%, from 90% to 100%, and from 95% to 100%).

In some embodiments, the polynucleotide includes a modified pyrimidine or purine. In some embodiments, the pyrimidine or purine in the polynucleotide molecule may be replaced with from about 1% to about 100% of a modified uracil or modified uridine (e.g., from 1% to 20%, from 1% to 25%, from 1% to 50%, from 1% to 60%, from 1% to 70%, from 1% to 80%, from 1% to 90%, from 1% to 95%, from 10% to 20%, from 10% to 25%, from 10% to 50%, from 10% to 60%, from 10% to 70%, from 10% to 80%, from 10% to 90%, from 10% to 95%, from 10% to 100%, from 20% to 25%, from 20% to 50%, from 20% to 60%, from 20% to 70%, from 20% to 80%, from 20% to 90%, from 20% to 95%, from 20% to 100%, from 50% to 60%, from 50% to 70%, from 50% to 80%, from 50% to 90%, from 50% to 95%, from 50% to 100%, from 70% to 80%, from 70% to 90%, from 70% to 95%, from 70% to 100%, from 80% to 90%, from 80% to 95%, from 80% to 100%, from 90% to 95%, from 90% to 100%, and from 95% to 100% of a modified pyrimidine or purine.

In some embodiments, the polynucleotides may comprise two or more effector module component sequences which are in a pattern such as ABABAB or AABBAABBAABB or ABCABCABC or variants thereof repeated once, twice, or more than three times. In these patterns, each letter, A, B, or C represent a different effector module component.

In yet another embodiment, the polynucleotides may comprise two or more effector module component sequences with each component having one or more sequences. As a non-limiting example, the sequences may be in a pattern such as ABABAB or AABBAABBAABB or ABCABCABC or variants thereof repeated once, twice, or more than three times in each of the regions. As another non-limiting example, the sequences may be in a pattern such as ABABAB or AABBAABBAABB or ABCABCABC or variants thereof repeated once, twice, or more than three times across the entire polynucleotide. In these patterns, each letter, A, B, or C represent a different sequence or component.

### Codon Selection

In some embodiments, one or more codons of the polynucleotides of the present invention may be replaced with other codons encoding the native amino acid sequence to tune the expression of the SREs, through a process referred to as codon selection. Since mRNA codon, and tRNA anticodon pools tend to vary among organisms, cell types, sub cellular locations and over time, the codon selection described herein is a spatiotemporal (ST) codon selection.

In some embodiments of the invention, certain polynucleotide features may be codon optimized. Codon optimization refers to a process of modifying a nucleic acid sequence for enhanced expression in the host cell by replacing at least 1, 2, 3, 4, 5, 10, 15, 20, 25, 50 or more codons of the native sequence with codons that are most frequently used in the genes of that host cell while maintaining the native amino acid sequence. Codon usage may be measured using the Codon Adaptation Index (CAI) which measures the deviation of a coding polynucleotide sequence from a reference gene set. Codon usage tables are available at the Codon Usage Database (http://www.kazusa.or.jp/codon/) and the CAI can be calculated by EMBOSS CAI program (http://emboss.sourceforge.net/). Codon optimization methods are known in the art and may be useful in efforts to achieve one or more of several goals. These goals include to match codon frequencies in target and host organisms to ensure proper folding, bias nucleotide content to alter stability or reduce secondary structures, minimize tandem repeat codons or base runs that may impair gene construction or expression, customize transcriptional and translational control regions, insert or remove protein signaling sequences, remove/add post translation modification sites in encoded protein (e.g. glycosylation sites), add, remove or shuffle protein domains, insert or delete restriction sites, modify ribosome binding sites and degradation sites, to adjust translational rates to allow the various domains of the protein to fold properly, or to reduce or eliminate problem secondary structures within the polynucleotide. Codon optimization tools, algorithms and services are known in the art, and non-limiting examples include services from GeneArt (Life Technologies), DNA2.0 (Menlo Park CA), OptimumGene (GenScript, Piscataway, NJ), algorithms such as but not limited to, DNAWorks v3.2.3 and/or proprietary methods. In one embodiment, a polynucleotide sequence or portion thereof is codon optimized using optimization algorithms. Codon options for each amino acid are well-known in the art as are various species table for optimizing for expression in that particular species.

In some embodiments of the invention, certain polynucleotide features may be codon optimized. For example, a preferred region for codon optimization may be upstream (5') or downstream (3') to a region which encodes a polypeptide. These regions may be incorporated into the polynucleotide before and/or after codon optimization of the payload encoding region or open reading frame (ORF).

After optimization (if desired), the polynucleotides components are reconstituted and transformed into a vector such as, but not limited to, plasmids, viruses, cosmids, and artificial chromosomes.

Spatiotemporal codon selection may impact the expression of the polynucleotides of the invention, since codon composition determines the rate of translation of the mRNA species and its stability. For example, tRNA anticodons to optimized codons are abundant, and thus translation may be enhanced. In contrast, tRNA anticodons to less common codons are fewer and thus translation may proceed at a slower rate. Presnyak et al. have shown that the stability of an mRNA species is dependent on the codon content, and higher stability and thus higher protein expression may be achieved by utilizing optimized codons (Presnyak et al. (2015) Cell 160, 1111-1124). Thus, in some embodiments, ST codon selection may include the selection of optimized codons to enhance the expression of the effector modules of the invention. In other embodiments, spatiotemporal codon selection may involve the selection of codons that are less commonly used in the genes of the host cell to decrease the expression of the compositions of the invention. The ratio of optimized codons to codons less commonly used in the genes of the host cell may also be varied to tune expression.

In some embodiments, certain regions of the polynucleotide may be preferred for codon selection. For example, a preferred region for codon selection may be upstream (5') or downstream (3') to a region which encodes a polypeptide. These regions may be incorporated into the polynucleotide before and/or after codon selection of the payload encoding region or open reading frame (ORF).

The stop codon of the polynucleotides of the present invention may be modified to include sequences and motifs to alter the expression levels of the SREs, payloads and effector modules of the present invention. Such sequences may be incorporated to induce stop codon readthrough, wherein the stop codon may specify amino acids e.g. selenocysteine or pyrrolysine. In other instances, stop codons may be skipped altogether to resume translation through an alternate open reading frame. Stop codon read through may be utilized to tune the expression of components of the effector modules at a specific ratio (e.g.as dictated by the stop codon context). Examples of preferred stop codon motifs include UGAN, UAAN, and UAGN, where N is either C or U.

### Destabilizing domains as stimulus response elements (SREs)

Destabilizing domains described herein or known in the art may be used as SREs in a biocircuit. The presence, absence or an amount of a small molecule ligand that binds to or interacts with the DD, can, upon such binding or interaction modulate the stability of the payload(s) and consequently the function of the payload. Depending on the degree of binding and/or interaction the altered function of the payload may vary, hence providing a "tuning" of the payload function. Table 1 lists a number of exemplary DDs.

**Table 1. Destabilizing Domains (SREs)**

| **SRE No.** | **SRE Name** |
|---|---|
| 1 | Human DHFR (hDHFR) |
| 2 | Human DHFR methotrexate stabilized |
| 3 | Human DHFR trimethoprim stabilized |
| 4 | E. coli DHFR |
| 5 | FKBP |
| 6 | PDE5 Catalytic domain |
| 7 | PPAR gamma ligand binding domain |
| 8 | Carbonic Anhydrase 2 (CA2) |
| 9 | Carbonic Anhydrase 2 acetazolamide stabilized |
| 10 | Carbonic Anhydrase 2 celecoxib stabilized |
| 11 | NAD(P)H Dehydrogenase, Quinone 2 (NQO2) |
| 12 | HMG-CoAR |

### Additional Effector Module Features

### Signal sequences

In addition to the SRE and payload region, effector modules of the invention may further comprise one or more additional features such as one or more signal sequences. Representative signal sequences are given in Table 2.

Signal sequences (sometimes referred to as signal peptides, targeting signals, target peptides, localization sequences, transit peptides, leader sequences or leader peptides) direct proteins (e.g., the effector module of the present invention) to their designated cellular and/or extracellular locations. Protein signal sequences play a central role in the targeting and translocation of nearly all secreted proteins and many integral membrane proteins.

A signal sequence is a short (5-30 amino acids long) peptide present at the N-terminus of the majority of newly synthesized proteins that are destined towards a particular location. Signal sequences can be recognized by signal recognition particles (SRPs) and cleaved using type I and type II signal peptide peptidases. Signal sequences derived from human proteins can be incorporated as a regulatory module of the effector module to direct the effector module to a particular cellular and/or extracellular location (See Table 2). These signal sequences are experimentally verified and can be cleaved (Zhang Z. and Henzel W.J.; "Signal peptide prediction based on analysis of experimentally verified cleavage sites."; Protein Sci. 2004, 13:2819-2824.)

In some embodiments, a signal sequence may be, although not necessarily, located at the N-terminus or C-terminus of the effector module, and may be, although not necessarily, cleaved off the desired effector module to yield a "mature" payload.

In addition to signal sequences naturally occurring such as from a secreted protein, a signal sequence may be a variant modified from a known signal sequence of a protein. For example, U.S. Pat. NOs.: 8,258,102 and 9,133,265 to Sleep disclose a modified albumin signal sequence having a secretion signal and an additional X1-X2-X3-X4-X5- motif which can increase protein secretion; U.S. Pat. NO.: 9,279,007 to Do discloses signal sequences of modified fragments of human immunoglobulin heavy chain binding protein (Bip) that can enhance protein expression and secretion; U.S. Pat. NO.: 8,148,494 to Leonhartsberger et al., discloses a signal peptide with a cleavage site that can be fused with a recombinant protein.

In some instances, the secreted signal sequences may be cytokine signal sequences such as, but not limited to, IL2 signal sequence or a p40 signal sequence.

In some instances, signal sequences directing the payload of interest to the surface membrane of the target cell may be used. Expression of the payload on the surface of the target cell may be useful to limit the diffusion of the payload to non-target *in vivo* environments, thereby potentially improving the safety profile of the payloads. Additionally, the membrane presentation of the payload may allow for physiologically and qualitative signaling as well as stabilization and recycling of the payload for a longer half-life. Membrane sequences may be the endogenous signal sequence of the N terminal component of the payload of interest. Optionally, it may be desirable to exchange this sequence for a different signal sequence. Signal sequences may be selected based on their compatibility with the secretory pathway of the cell type of interest so that the payload is presented on the surface of the T cell. In some embodiments, the signal sequence may be IgE signal sequence, or CD8a signal sequence.

Other signal sequence variants may be used in the present effector module may include those discussed in U.S. patent application publication NOs.: 2007/0141666; PCT patent application publication NOs.: 1993/018181.

In other embodiments, a signal sequence may be a heterogeneous signal sequence from other organisms such as virus, yeast and bacteria, which can direct an effector module to a particular cellular site, such as a nucleus (e.g., EP 1209450). Other examples may include Aspartic Protease (NSP24) signal sequences from *Trichoderma* that can increase secretion of fused protein such as enzymes (e.g., U. S. Pat. NO.: 8,093,016 to Cervin and Kim), bacterial lipoprotein signal sequences (e.g., PCT application publication NO.: 1991/09952 to Lau and Rioux), *E.coli* enterotoxin II signal peptides (e.g., U.S. Pat. NO.: 6,605,697 to Kwon et al.), *E.coli* secretion signal sequence (e.g., U.S. patent publication NO.: 2016/090404 to Malley et al.), a lipase signal sequence from a methylotrophic yeast (e.g., U.S. Pat. NO.: 8,975,041), and signal peptides for DNases derived from *Coryneform bacteria* (e.g., U.S. Pat. NO.: 4,965,197).

Signal sequences may also include nuclear localization signals (NLSs), nuclear export signals (NESs), polarized cell tubulo-vesicular structure localization signals (See, e.g., U.S. Pat. NO.: 8, 993,742; Cour et al., Nucleic Acids Res. 2003, 31(1): 393-396,extracellular localization signals, signals to subcellular locations (e.g. lysosome, endoplasmic reticulum, golgi, mitochondria, plasma membrane and peroxisomes, etc.) (See, e.g., U.S. Pat. NO.: 7,396,811; Negi et al., *Database,* 2015, 1-7.)

**Table 2. Signal Sequences**

| SS No. | Symbol | Signal Sequence Name | Cellular and extracellular location | SEQ ID NO |
|---|---|---|---|---|
| 1 | LYPA3 | 1-O-acylceramide synthase | Lysosome; Secreted | 205368 |
| 2 | TYRP1 | 5,6-dihydroxyindole-2-carboxylic acid oxidase (DHICA oxidase) (Tyrosinase-related protein 1) (TRP-1) | Melanosome membrane; Single-pass type I membrane protein | 205369 |
| 3 | 5NTD | 5'-nucleotidase | Membrane (Single-pass type I membrane protein) | 205370 |
| 4 | GRP78 | 78 kDa glucose-regulated protein | Endoplasmic reticulum lumen; Melanosome; Cytoplasm | 205371 |
| 5 | ACHA | Acetylcholine receptor subunit alpha | transmembrane (Antigen Lipoprotein Membrane Outer membrane Palmitate Signal) | 205372 |
| 6 | ACHB | Acetylcholine receptor subunit beta | | 205373 |
| 7 | ACHE | Acetylcholine receptor subunit epsilon | | 205374 |
| 8 | ACRO | Acrosin | | 205375 |
| 9 | ADA32 | ADAM 32 | | 205376 |
| 10 | ATL1 | ADAMTS-like protein 1 | | 205377 |
| 11 | ADIPO | Adipokine | Secreted (extracellular space) | 205378 |
| 12 | ADML | ADM | Secreted | 205379 |
| 13 | PGCA | Aggrecan core protein | | 205380 |
| 14 | PPBT | Alkaline phosphatase, tissue-nonspecific isozyme | | 205381 |
| 15 | A1AG1 | Alpha-1-acid glycoprotein 1 | Secreted (blood serum; extracellular) | 205382 |
| 16 | A1AG2 | Alpha-1-acid glycoprotein 2 | Secreted (blood serum; extracellular) | 205383 |
| 17 | AACT | Alpha-1-antichymotrypsin | | 205384 |
| 18 | A1AT | Alpha-1-antitrypsin (Alpha-1 protease inhibitor) | Secreted (Endoplasmic reticulum) | 205385 |
| 19 | A1BG | Alpha-1B-glycoprotein | Secreted (blood serum; extracellular) | 205386 |
| 20 | A2AP | Alpha-2-antiplasmin | Secreted | 205387 |
| 21 | FETUA | Alpha-2-HS-glycoprotein (Fetuin-A) (Alpha-2-Z-globulin) | Secreted | 205388 |
| 22 | A2MG | Alpha-2-macroglobulin | | 205389 |
| 23 | AMY2B | Alpha-amylase 2B | | 205390 |
| 24 | FETA | Alpha-fetoprotein | | 205391 |
| 25 | AGAL | Alpha-galactosidase A | Lysosome | 205392 |
| 26 | LALBA | Alpha-lactalbumin (Lactose synthase B protein) | Secreted | 205393 |
| 27 | NAGAB | Alpha-N-acetylgalactosaminidase | Lysosome | 205394 |
| 28 | ANAG | Alpha-N-acetylglucosaminidase | | 205395 |
| 29 | CASA1 | Alpha-S1-casein | Secreted | 205396 |
| 30 | PGFRA | Alpha-type platelet-derived growth factor receptor | | 205397 |
| 31 | AMELX | Amelogenin, X isoform | | 205398 |
| 32 | AMTN | Amelotin | Secreted | 205399 |
| 33 | ABP1 | Amiloride-sensitive amine oxidase [copper-containing] | Secreted, extracellular space | 205400 |
| 34 | A4 | Amyloid beta A4 protein | | 205401 |
| 35 | ANGI | Angiogenin | Secreted (cytoplasmic); Nucleus | 205402 |
| 36 | TIE2 | Angiopoietin-1 receptor | | 205403 |
| 37 | ANGL3 | Angiopoietin-related protein 3 (Angiopoietin-like 3) | Secreted | 205404 |
| 38 | ANGL7 | Angiopoietin-related protein 7 (Angiopoietin-like 7) | Secreted | 205405 |
| 39 | ACET | Angiotensin-converting enzyme, testis-specific isoform | Cell membrane; Single-pass type I membrane | 205406 |
| 40 | ANGT | Angiotensinogen (Serpin A8) | Secreted | 205407 |
| 41 | AGR2 | Anterior gradient protein 2 homolog | Secreted cement gland | 205408 |
| 42 | AGR3 | Anterior gradient protein 3 homolog | Secreted | 205409 |
| 43 | SLPI | Antileukoproteinase (ALP) (Secretory leukocyte protease, inhibitor) | Secreted | 205410 |
| 44 | ANT3 | Antithrombin-III | | 205411 |
| 45 | APOA1 | Apolipoprotein A-I (Apo-AI) | | 205412 |
| 46 | APOA2 | Apolipoprotein A-II (Apo-AII) | Secreted; Transport | 205413 |
| 47 | APOA4 | Apolipoprotein A-IV | | 205414 |
| 48 | APOB | Apolipoprotein B-100 | Membrane (Single-pass type I membrane protein) | 205415 |
| 49 | APOC2 | Apolipoprotein C-2 | | 205416 |
| 50 | APOC3 | Apolipoprotein C-3 | | 205417 |
| 51 | APOC1 | Apolipoprotein C-I | | 205418 |
| 52 | APOD | Apolipoprotein D | | 205419 |
| 53 | APOE | Apolipoprotein E (Apo-E) | Secreted; Transport/VLDL | 205420 |
| 54 | APOM | Apolipoprotein M | | 205421 |
| 55 | APOA | Apolipoprotein(a) | Transport | 205422 |
| 56 | APRIII | Apoptosis-related protein 3 | | 205423 |
| 57 | GHRL | Appetite-regulating hormone | | 205424 |
| 58 | ARSA | Arylsulfatase A | | 205425 |
| 59 | ANF | Atrial natriuretic factor | | 205426 |
| 60 | ANPRA | Atrial natriuretic peptide receptor A | | 205427 |
| 61 | CAP7 | Azurocidin | | 205428 |
| 62 | BPI | Bactericidal permeability-increasing protein (BPI) | membrane | 205429 |
| 63 | BPIL1 | Bactericidal/permeability-increasing protein-like 1 | | 205430 |
| 64 | PRP1 | Basic salivary proline-rich protein 1 | | 205431 |
| 65 | PRB4 | Basic salivary proline-rich protein 4 | | 205432 |
| 66 | BASI | Basigin | | 205433 |
| 67 | CD79A | B-cell antigen receptor complex-associated protein alpha-chain | | 205434 |
| 68 | CD79B | B-cell antigen receptor complex-associated protein beta-chain | | 205435 |
| 69 | NTRK2 | BDNF/NT-3 growth factors receptor | Membrane (Single-pass type I membrane protein) | 205436 |
| 70 | APOH | Beta-2-glycoprotein 1 (Beta-2-glycoprotein I), (Apolipoprotein H) | Secreted | 205437 |
| 71 | B2MG | Beta-2-microglobulin | | 205438 |
| 72 | CASB | Beta-casein | | 205439 |
| 73 | D103A | Beta-defensin 103 (Defensin, beta 103) | Secreted | 205440 |
| 74 | DB127 | Beta-defensin 127 (Defensin, beta 127) | Secreted | 205441 |
| 75 | BGAL | Beta-galactosidase | Lysosome | 205442 |
| 76 | BGLR | Beta-glucuronidase | Lysosome | 205443 |
| 77 | HEXA | Beta-hexosaminidase subunit alpha | | 205444 |
| 78 | MSMB | Beta-microseminoprotein | Secreted (Sperm surface) | 205445 |
| 79 | PDYN | Beta-neoendorphin-dynorphin (Proenkephalin B) | Secreted | 205446 |
| 80 | PGFRB | Beta-type platelet-derived growth factor receptor | Membrane (Single-pass type I membrane protein) | 205447 |
| 81 | BAMBI | BMP and activin membrane-bound inhibitor homolog | Membrane (Single-pass type I membrane protein) | 205448 |
| 82 | PRG2 | Bone marrow proteoglycan | | 205449 |
| 83 | SIAL | Bone sialoprotein 2 (Bone sialoprotein II) (BSP II) | Secreted | 205450 |
| 84 | BOC | Brother of CDO | Cell membrane; transmembrane | 205451 |
| 85 | BT3A3 | Butyrophilin subfamily 3 member A3 | Membrane (Single-pass type I membrane protein) | 205452 |
| 86 | BTNL8 | Butyrophilin-like protein 8 | | 205453 |
| 87 | CA187 | C1orf187 | Secreted | 205454 |
| 88 | C4BPA | C4b-binding protein alpha chain | Secreted | 205455 |
| 89 | C4BPB | C4b-binding protein beta chain | | 205456 |
| 90 | CALRL | Calcitonin gene-related peptide type 1 receptor | Membrane (Single-pass type I membrane protein) | 205457 |
| 91 | CALCR | Calcitonin receptor | Membrane (Single-pass type I membrane protein) | 205458 |
| 92 | CALR | Calreticulin | Endoplasmic reticulum lumen; Cytoplasm | 205459 |
| 93 | CSTN1 | Calsyntenin-1 | | 205460 |
| 94 | CALU | Calumenin | Endoplasmic reticulum lumen; Golgi apparatus; Melanosome | 205461 |
| 95 | CAH4 | Carbonic anhydrase 4 | | 205462 |
| 96 | CAH9 | Carbonic anhydrase 9 | | 205463 |
| 97 | EST2 | Carboxylesterase 2 | | 205464 |
| 98 | CBPA1 | Carboxypeptidase A1 | Secreted (extracellular space) | 205465 |
| 99 | CBPB1 | Carboxypeptidase B | Secreted extracellular space) | 205466 |
| 100 | CBPM | Carboxypeptidase M | | 205467 |
| 101 | CBPN | Carboxypeptidase N catalytic chain | | 205468 |
| 102 | CEAM1 | Carcinoembryonic antigen-related cell adhesion molecule 1, (Biliary glycoprotein 1) (BGP-1) (CD66 antigen) | Cell membrane (lipid anchor) | 205469 |
| 103 | CEAM6 | Carcinoembryonic antigen-related cell adhesion molecule 6 | Cell membrane (lipid anchor) | 205470 |
| 104 | CEAM7 | Carcinoembryonic antigen-related cell adhesion molecule 7 | Cell membrane (lipid anchor) | 205471 |
| 105 | CEAM8 | Carcinoembryonic antigen-related cell adhesion molecule 8 | Cell membrane (lipid anchor) | 205472 |
| 106 | MATN1 | Cartilage matrix protein | | 205473 |
| 107 | CATD | Cathepsin D | | 205474 |
| 108 | CATE | Cathepsin E | | 205475 |
| 109 | CATG | Cathepsin G | | 205476 |
| 110 | CATH | Cathepsin H | | 205477 |
| 111 | CATW | Cathepsin W | | 205478 |
| 112 | MPRD | Cation-dependent mannose-6-phosphate receptor | Lysosome membrane (Single-pass type I membrane protein) | 205479 |
| 113 | MPRI | Cation-independent mannose-6-phosphate receptor | Lysosome membrane (Single-pass type I membrane protein) | 205480 |
| 114 | CCL1 | C-C motif chemokine 1 (Small-inducible cytokine A1) | Secreted | 205481 |
| 115 | CCL13 | C-C motif chemokine 13 | Secreted | 205482 |
| 116 | CCL14 | C-C motif chemokine 14 | Secreted | 205483 |
| 117 | CCL15 | C-C motif chemokine 15 (Small-inducible cytokine A15) | Secreted | 205484 |
| 118 | CCL16 | C-C motif chemokine 16 | Secreted | 205485 |
| 119 | CCL17 | C-C motif chemokine 17 | Secreted | 205486 |
| 120 | CCL18 | C-C motif chemokine 18 | Secreted | 205487 |
| 121 | CCL19 | C-C motif chemokine 19 | Secreted | 205488 |
| 122 | CCL2 | C-C motif chemokine 2 | Secreted | 205489 |
| 123 | CCL20 | C-C motif chemokine 20 | Secreted | 205490 |
| 124 | CCL21 | C-C motif chemokine 21 | Secreted | 205491 |
| 125 | CCL22 | C-C motif chemokine 22 | Secreted | 205492 |
| 126 | CCL23 | C-C motif chemokine 23 | Secreted | 205493 |
| 127 | CCL24 | C-C motif chemokine 24 (Small-inducible cytokine A24) | Secreted | 205494 |
| 128 | CCL26 | C-C motif chemokine 26 | Secreted | 205495 |
| 129 | CCL27 | C-C motif chemokine 27 | Secreted | 205496 |
| 130 | CCL3 | C-C motif chemokine 3 | Secreted | 205497 |
| 131 | CL3L1 | C-C motif chemokine 3-like 1 (Small-inducible cytokine A3-, like 1) | Secreted | 205498 |
| 132 | CCL4 | C-C motif chemokine 4 (Small-inducible cytokine A4) | Secreted | 205499 |
| 133 | CCL5 | C-C motif chemokine 5 | Secreted | 205500 |
| 134 | CCL7 | C-C motif chemokine 7 | Secreted | 205501 |
| 135 | CD109 | CD109 antigen | | 205502 |
| 136 | C16L2 | CD164 sialomucin-like 2 protein | | 205503 |
| 137 | C16L2 | CD164 sialomucin-like 2 protein | Membrane (Single-pass type I membrane protein) | 205504 |
| 138 | CD177 | CD177 antigen | | 205505 |
| 139 | CD180 | CD180 antigen (Lymphocyte antigen 64) | Cell membrane (Single-pass type I membrane protein) | 205506 |
| 140 | CD226 | CD226 antigen | | 205507 |
| 141 | CD27 | CD27 antigen (CD27L receptor) | Membrane (Single-pass type I membrane protein) | 205508 |
| 142 | CD276 | CD276 antigen (Costimulatory molecule) | Membrane (Single-pass type I membrane protein) | 205509 |
| 143 | CD320 | CD320 antigen (8D6 antigen) | Membrane (Single-pass type I membrane protein) | 205510 |
| 144 | CD5L | CD5 antigen-like (SP-alpha) (CT-2) | Secreted | 205511 |
| 145 | CD59 | CD59 glycoprotein | | 205512 |
| 146 | CD83 | CD83 antigen (Cell surface protein HB15) | Transmembrane | 205513 |
| 147 | CD99 | CD99 antigen | | 205514 |
| 148 | CADM3 | Cell adhesion molecule 3 (Immunoglobulin superfamily member,4B) | Membrane (Single-pass type I membrane protein) | 205515 |
| 149 | GPA33 | Cell surface A33 antigen | | 205516 |
| 150 | MUC18 | Cell surface glycoprotein MUC18 | | 205517 |
| 151 | MOX2R | Cell surface glycoprotein OX2 receptor (CD200 cell surface, glycoprotein receptor) | Secreted | 205518 |
| 152 | CER1 | Cerberus | | 205519 |
| 153 | CBLN3 | Cerebellin-3 | | 205520 |
| 154 | CBLN4 | Cerebellin-4 | | 205521 |
| 155 | CERU | Ceruloplasmin (Ferroxidase) | Secreted | 205522 |
| 156 | CH3L1 | Chitinase-3-like protein 1 (Cartilage glycoprotein 39) | Secreted, extracellular space | 205523 |
| 157 | CH3L2 | Chitinase-3-like protein 2 (YKL-39) (Chondrocyte protein,39) | | 205524 |
| 158 | CHIT1 | Chitotriosidase-1 (Chitinase-1) | Secreted; Lysosome (a small portion) | 205525 |
| 159 | CCKN | Cholecystokinin | Secreted | 205526 |
| 160 | CETP | Cholesteryl ester transfer protein | Secreted | 205527 |
| 161 | CHLE | Cholinesterase | | 205528 |
| 162 | CRDL2 | Chordin-like protein 2 (Chordin-related protein 2) | Secreted | 205529 |
| 163 | CGHB | Choriogonadotropin subunit beta | | 205530 |
| 164 | CSH | Chorionic somatomammotropin hormone | | 205531 |
| 165 | CMGA | Chromogranin-A (CgA) (Pituitary secretory protein I) (SP-I) | Secreted (Neuroendocrine and endocrine secretory granules) | 205532 |
| 166 | CMA1 | Chymase | | 205533 |
| 167 | CTRB1 | Chymotrypsinogen B | Secreted (extracellular space) | 205534 |
| 168 | CLUS | Clusterin (Complement-associated protein SP-40,40) | Secreted | 205535 |
| 169 | CLM1 | CMRF35-like molecule 1 | | 205536 |
| 170 | CLM9 | CMRF35-like molecule 9 | | 205537 |
| 171 | FA5 | Coagulation factor V (Activated protein C cofactor) | Secreted | 205538 |
| 172 | FA8 | Coagulation factor VIII | | 205539 |
| 173 | FA11 | Coagulation factor XI (Plasma thromboplastin, antecedent) (PTA) (FXI) | Secreted | 205540 |
| 174 | FA12 | Coagulation factor XII | | 205541 |
| 175 | F13B | Coagulation factor XIII B chain (Protein-glutamine gamma-, glutamyltransferase B chain) | | 205542 |
| 176 | CART | Cocaine- and amphetamine-regulated transcript protein | | 205543 |
| 177 | COL | Colipase | | 205544 |
| 178 | CF126 | Colipase-like protein C6orf126 | | 205545 |
| 179 | CO1A1 | Collagen alpha-1(I) chain | | 205546 |
| 180 | CO3A1 | Collagen alpha-1(III) chain | | 205547 |
| 181 | CO4A1 | Collagen alpha-1(IV) chain | | 205548 |
| 182 | CO9A1 | Collagen alpha-1(IX) chain | Secreted (extracellular space, extracellular matrix) | 205549 |
| 183 | CO6A1 | Collagen alpha-1(VI) chain | | 205550 |
| 184 | COGA1 | Collagen alpha-1(XVI) chain | Secreted (extracellular space, extracellular matrix) | 205551 |
| 185 | CO4A2 | Collagen alpha-2(IV) chain | Secreted (extracellular space, extracellular matrix, basement membrane) | 205552 |
| 186 | CO5A2 | Collagen alpha-2(V) chain | Secreted (extracellular space, extracellular matrix) | 205553 |
| 187 | CTHR1 | Collagen triple helix repeat-containing protein 1 (NMTC1, protein) | Secreted (extracellular space) | 205554 |
| 188 | C1QA | Complement C1q subcomponent subunit A | | 205555 |
| 189 | C1QB | Complement C1q subcomponent subunit B | | 205556 |
| 190 | C1QC | Complement C1q subcomponent subunit C | | 205557 |
| 191 | C1QT5 | Complement C1q tumor necrosis factor-related protein 5 | | 205558 |
| 192 | C1QT5 | Complement C1q tumor necrosis factor-related protein 5 | | 205559 |
| 193 | C1QT6 | Complement C1q tumor necrosis factor-related protein 6 | Secreted | 205560 |
| 194 | C1R | Complement C1r subcomponent | Secreted | 205561 |
| 195 | C1S | Complement C1s subcomponent | Secreted | 205562 |
| 196 | CO2 | Complement C2 (C3/C5 convertase) | Secreted | 205563 |
| 197 | CO3 | Complement C3 | | 205564 |
| 198 | CO4A | Complement C4-A | | 205565 |
| 199 | C1QR1 | Complement component C1q receptor | Secreted | 205566 |
| 200 | CO6 | Complement component C6 | Secreted | 205567 |
| 201 | CO7 | Complement component C7 | Secreted | 205568 |
| 202 | CO8G | Complement component C8 gamma chain | | 205569 |
| 203 | CO9 | Complement component C9 | Transmembrane | 205570 |
| 204 | DAF | Complement decay-accelerating factor | | 205571 |
| 205 | CFAB | Complement factor B (C3/C5 convertase) | Secreted | 205572 |
| 206 | CFAH | Complement factor H | | 205573 |
| 207 | FHR2 | Complement factor H-related protein 2 | | 205574 |
| 208 | CFAI | Complement factor I | | 205575 |
| 209 | CR1 | Complement receptor type 1 | | 205576 |
| 210 | CR2 | Complement receptor type 2 | | 205577 |
| 211 | CNTN1 | Contactin-1 | | 205578 |
| 212 | CNTN2 | Contactin-2 | | 205579 |
| 213 | CBG | Corticosteroid-binding globulin | Secreted | 205580 |
| 214 | COLI | Corticotropin-lipotropin (Pro-opiomelanocortin) (POMC) | | 205581 |
| 215 | CRFR1 | Corticotropin-releasing factor receptor 1 | | 205582 |
| 216 | CRHBP | Corticotropin-releasing factor-binding protein (CRF-binding, protein) | | 205583 |
| 217 | CRP | C-reactive protein | Secreted | 205584 |
| 218 | SG1D1 | cretoglobin family 1D member 1 | | 205585 |
| 219 | SG3A1 | cretoglobin family 3A member 1 | | 205586 |
| 220 | CLC11 | C-type lectin domain family 11 member A (Stem cell growth factor) | Cytoplasm; Secreted. | 205587 |
| 221 | CLC14 | C-type lectin domain family 14 member A (Epidermal growth, factor receptor 5) (EGFR-5) | Membrane (Single-pass type I membrane protein) | 205588 |
| 222 | CDCP1 | CUB domain-containing protein 1 | | 205589 |
| 223 | CXL10 | C-X-C motif chemokine 10 (Small-inducible cytokine B10) | Secreted | 205590 |
| 224 | CXL11 | C-X-C motif chemokine 11 | | 205591 |
| 225 | CXCL2 | C-X-C motif chemokine 2 | | 205592 |
| 226 | CXCL3 | C-X-C motif chemokine 3 | | 205593 |
| 227 | CXCL5 | C-X-C motif chemokine 5 | | 205594 |
| 228 | CXCL6 | C-X-C motif chemokine 6 | | 205595 |
| 229 | CXCL9 | C-X-C motif chemokine 9 | | 205596 |
| 230 | CST9 | Cystatin-9 (Cystatin-like molecule) | Secreted (through the Golgi via the secretory pathway) | 205597 |
| 231 | CST9L | Cystatin-9-like | | 205598 |
| 232 | CYTC | Cystatin-C | Secreted (extracellular space) | 205599 |
| 233 | CYTD | Cystatin-D | Secreted (extracellular space) | 205600 |
| 234 | CYTS | Cystatin-S | | 205601 |
| 235 | CYTT | Cystatin-SA | | 205602 |
| 236 | CYTN | Cystatin-SN | | 205603 |
| 237 | CRIM1 | Cysteine-rich motor neuron 1 protein (CRIM-1) | Cell membrane | 205604 |
| 238 | CRIS1 | Cysteine-rich secretory protein 1 (CRISP-1) | Located in the lumen and epithelium of distal ductus efferentes and epididy | 205605 |
| 239 | CREL2 | Cysteine-rich with EGF-like domain protein 2 | | 205606 |
| 240 | IL2RG | Cytokine receptor common subunit gamma | Membrane (Single-pass type I membrane protein) | 205607 |
| 241 | CRLF1 | Cytokine receptor-like factor 1 | | 205608 |
| 242 | XCL2 | Cytokine SCM-1 beta | | 205609 |
| 243 | CYTL1 | Cytokine-like protein 1 | | 205610 |
| 244 | DLK | Delta-like protein (DLK) (pG2) | Membrane (Single-pass type I membrane protein) | 205611 |
| 245 | DLL4 | Delta-like protein 4 | | 205612 |
| 246 | DNAS1 | Deoxyribonuclease-1 | | 205613 |
| 247 | DCD | Dermcidin | | 205614 |
| 248 | DMKN | Dermokine | | 205615 |
| 249 | DKK1 | Dickkopf-related protein 1 (Dkk-1) (Dickkopf-1) | Secreted | 205616 |
| 250 | DKK3 | Dickkopf-related protein 3 | | 205617 |
| 251 | DKK4 | Dickkopf-related protein 4 (Dkk-4) (Dickkopf-4) | Secreted | 205618 |
| 252 | DPEP1 | Dipeptidase 1 | | 205619 |
| 253 | CATC | Dipeptidyl-peptidase 1 | Lysosome | 205620 |
| 254 | RIB2 | Dolichyl-diphosphooligosaccharideprotein glycosyltransferase 63 kDa, subunit | Endoplasmic reticulum membrane (Single-pass type I membrane protein) | 205621 |
| 255 | RPN1 | Dolichyl-diphosphooligosaccharideprotein glycosyltransferase subunit 1 | | 205622 |
| 256 | RPN2 | Dolichyl-diphosphooligosaccharide--protein glycosyltransferase subunit 2 | | 205623 |
| 257 | INSL4 | Early placenta insulin-like peptide (EPIL) (Placentin), (Insulin-like peptide 4) | Secreted | 205624 |
| 258 | ENPP7 | Ectonucleotide pyrophosphatase/phosphodiesterase family member 7 | | 205625 |
| 259 | EGFL8 | EGF-like domain-containing protein 8 (Epidermal growth, factor-like protein 8) | Secreted | 205626 |
| 260 | ELAF | Elafin (Elastase-specific inhibitor) (ESI) | Secreted | 205627 |
| 261 | ELA2A | Elastase-2A | Secreted | 205628 |
| 262 | ELA2B | Elastase-2B | Secreted | 205629 |
| 263 | EGLN | Endoglin | | 205630 |
| 264 | ERP29 | Endoplasmic reticulum protein ERp29 | | 205631 |
| 265 | ENPL | Endoplasmin (Heat shock protein 90 kDa beta member 1) | Endoplasmic reticulum lumen; Melanosome | 205632 |
| 266 | ESAM | Endothelial cell-selective adhesion molecule | | 205633 |
| 267 | EDN1 | Endothelin-1 | | 205634 |
| 268 | ECP | Eosinophil cationic protein (ECP), (Ribonuclease 3) | Cytoplasmic granule (Matrix of eosinophil's large specific granule) | 205635 |
| 269 | CCL11 | Eotaxin | Secreted | 205636 |
| 270 | EPHA3 | Ephrin type-A receptor 3 | | 205637 |
| 271 | EPHB1 | Ephrin type-B receptor 1 | | 205638 |
| 272 | EPHB6 | Ephrin type-B receptor 6 | | 205639 |
| 273 | EFNA1 | Ephrin-A1 | | 205640 |
| 274 | EFNA4 | Ephrin-A4 | | 205641 |
| 275 | EFNB1 | Ephrin-B1 (EPH-related receptor tyrosine kinase ligand 2) | Membrane | 205642 |
| 276 | EFNB3 | Ephrin-B3 | | 205643 |
| 277 | EGFR | Epidermal growth factor receptor (Receptor, tyrosine-protein kinase ErbB-1) | Membrane (Single-pass type I membrane protein) | 205644 |
| 278 | EPGN | Epigen (Epithelial mitogen) (EPG) | Membrane | 205645 |
| 279 | ERO1A | ERO1-like protein alpha | | 205646 |
| 280 | EPO | Erythropoietin | | 205647 |
| 281 | EPOR | Erythropoietin receptor (EPO-R), Isoform EPOR-S | Secreted and located to the cell surface | 205648 |
| 282 | LYAM2 | E-selectin (Endothelial leukocyte adhesion molecule 1) | Membrane (Single-pass type I membrane protein) | 205649 |
| 283 | SODE | Extracellular superoxide dismutase [Cu-Zn] | Secreted (extracellular) | 205650 |
| 284 | FCRLA | Fc receptor-like and mucin-like 1 (Fc receptor-like A) | Secreted; Cytoplasm | 205651 |
| 285 | FCRL1 | Fc receptor-like protein 1 | | 205652 |
| 286 | FCRL2 | Fc receptor-like protein 2 precursor (FcR-like protein 2) (FcRL2) | Membrane (Single-pass type I membrane protein) | 205653 |
| 287 | FIBA | Fibrinogen alpha chain | Secreted | 205654 |
| 288 | FIBB | Fibrinogen beta chain | Secreted | 205655 |
| 289 | FIBG | Fibrinogen gamma chain | Secreted | 205656 |
| 290 | FGF19 | Fibroblast growth factor 19 (FGF-19) | Secreted | 205657 |
| 291 | FGF21 | Fibroblast growth factor 21 (FGF-21) | Secreted | 205658 |
| 292 | FGF23 | Fibroblast growth factor 23 (FGF-23) (Tumor-derived, hypophosphatemia-inducing factor) | Secreted | 205659 |
| 293 | FGFR3 | Fibroblast growth factor receptor 3 | | 205660 |
| 294 | FGFR4 | Fibroblast growth factor receptor 4 | | 205661 |
| 295 | FGRL1 | Fibroblast growth factor receptor-like 1 | | 205662 |
| 296 | FINC | Fibronectin | | 205663 |
| 297 | FBLN1 | Fibulin-1 | | 205664 |
| 298 | FCN1 | Ficolin-1 (Ficolin-A) (Ficolin-alpha) (M-Ficolin), (Collagen/fibrinogen domain-containing protein 1) | Secreted (on the monocyte surface) | 205665 |
| 299 | FCN3 | Ficolin-3 | | 205666 |
| 300 | FKB14 | FK506-binding protein 14 (Peptidyl-prolyl cis-, trans isomerase) | Endoplasmic reticulum lumen | 205667 |
| 301 | FKBP2 | FK506-binding protein 2 (Peptidyl-prolyl cis-, trans isomerase) (Pease) (Aromatase) (13 kDa FKBP) (FKBP-13). | Endoplasmic reticulum membrane; Peripheral membrane protein | 205668 |
| 302 | FOLR2 | Folate receptor beta | | 205669 |
| 303 | FST | Follistatin (FS) (Activin-binding protein) | Secreted | 205670 |
| 304 | FSTL1 | Follistatin-related protein 1 | | 205671 |
| 305 | FSTL3 | Follistatin-related protein 3 | | 205672 |
| 306 | FSHB | Follitropin subunit beta (Follicle-stimulating hormone beta, subunit) | Secreted | 205673 |
| 307 | X3CL1 | Fractalkine precursor (C-X3-C motif chemokine 1) (Neurotactin) | Membrane; Secreted | 205674 |
| 308 | FZD3 | Frizzled-3 (Fz-3) (hFz3) | Membrane (Multi-pass membrane protein) | 205675 |
| 309 | KV313 | g kappa chain V-III region HIC | | 205676 |
| 310 | GALC | Galactocerebrosidase | Lysosome | 205677 |
| 311 | LG3BP | Galectin-3-binding protein (Lectin galactoside-binding, soluble 3-binding protein) | Secreted (extracellular space, extracellular matrix) | 205678 |
| 312 | LIPG | Gastric triacylglycerol lipase | Secreted | 205679 |
| 313 | PEPC | Gastricsin | | 205680 |
| 314 | GAST | Gastrin | | 205681 |
| 315 | GRP | Gastrin-releasing peptide | Secreted | 205682 |
| 316 | GFRA3 | GDNF family receptor alpha-3 | | 205683 |
| 317 | GELS | Gelsolin | | 205684 |
| 318 | GDN | Glia-derived nexin | Secreted (extracellular space) | 205685 |
| 319 | GPRL1 | GLIPR1-like protein 1 | | 205686 |
| 320 | GLUC | Glucagon | Secreted | 205687 |
| 321 | GLU2B | Glucosidase 2 subunit beta | | 205688 |
| 322 | GLCM | Glucosylceramidase | | 205689 |
| 323 | PAEP | Glycodelin | | 205690 |
| 324 | GLPA | Glycophorin-A (PAS-2) | Membrane (Single-pass type I membrane protein) | 205691 |
| 325 | GLPB | Glycophorin-B (PAS-3) | Membrane (Single-pass type I membrane protein) | 205692 |
| 326 | GLPE | Glycophorin-E | Membrane (Single-pass type I membrane protein) | 205693 |
| 327 | GLHA | Glycoprotein hormones alpha chain | Secreted | 205694 |
| 328 | GPC1 | Glypican-1 | | 205695 |
| 329 | PIGT | GPI transamidase component PIG-T | | 205696 |
| 330 | GPI8 | GPl-anchor transamidase, (GPI transamidase) | Endoplasmic reticulum membrane (Single-pass type I membrane protein) | 205697 |
| 331 | GPR56 | G-protein coupled receptor 56 | | 205698 |
| 332 | CSF3R | Granulocyte colony-stimulating factor receptor (G-CSF-R), (CD114 antigen) | Cell membrane | 205699 |
| 333 | CSF2 | Granulocyte-macrophage colony-stimulating factor (GM-CSF) | Secreted | 205700 |
| 334 | CSF2R | Granulocyte-macrophage colony-stimulating factor receptor subunit alpha | | 205701 |
| 335 | GRAA | Granzyme A | | 205702 |
| 336 | GRAB | Granzyme B | membrane (postsynaptic membrane) | 205703 |
| 337 | GRAH | Granzyme H | | 205704 |
| 338 | GREM1 | Gremlin-1 | | 205705 |
| 339 | PG12B | Group XIIB secretory phospholipase A2-like protein | Secreted | 205706 |
| 340 | SOM2 | Growth hormone variant | | 205707 |
| 341 | GROA | Growth-regulated alpha protein (C-X-C motif chemokine 1) | Secreted | 205708 |
| 342 | GUC2A | Guanylin | | 205709 |
| 343 | HPT | Haptoglobin | Secreted | 205710 |
| 344 | CD34 | Hematopoietic progenitor cell antigen CD34 | Membrane (Single-pass type I membrane protein) | 205711 |
| 345 | HEMO | Hemopexin | | 205712 |
| 346 | HPSE | Heparanase | | 205713 |
| 347 | HEP2 | Heparin cofactor 2 (Heparin cofactor II) | | 205714 |
| 348 | LIPC | Hepatic triacylglycerol lipase | | 205715 |
| 349 | HGF | Hepatocyte growth factor | | 205716 |
| 350 | HGFA | Hepatocyte growth factor activator | Secreted | 205717 |
| 351 | HFE | Hereditary hemochromatosis protein | | 205718 |
| 352 | FCGR1 | High affinity immunoglobulin gamma Fc receptor I (Fc-gamma, RI) | Cell membrane (Single-pass type I membrane protein) | 205719 |
| 353 | HIS1 | Histatin-1 (Histidine-rich protein 1) | | 205720 |
| 354 | HIS3 | Histatin-3 (Histidine-rich protein 3) | Secreted (by serous acinar and demilune cells) | 205721 |
| 355 | HRG | Histidine-rich glycoprotein | | 205722 |
| 356 | 1A01 | HLA class I histocompatibility antigen, A-1 alpha chain | Membrane (Single-pass type I membrane protein) | 205723 |
| 357 | 1A11 | HLA class I histocompatibility antigen, A-11 alpha chain | Membrane (Single-pass type I membrane protein) | 205724 |
| 358 | 1A02 | HLA class I histocompatibility antigen, A-2 alpha chain | Membrane (Single-pass type I membrane protein) | 205725 |
| 359 | 1A23 | HLA class I histocompatibility antigen, A-23 alpha chain | Membrane (Single-pass type I membrane protein) | 205726 |
| 360 | 1A25 | HLA class I histocompatibility antigen, A-25 alpha chain | Membrane (Single-pass type I membrane protein) | 205727 |
| 361 | 1A26 | HLA class I histocompatibility antigen, A-26 alpha chain | Membrane (Single-pass type I membrane protein) | 205728 |
| 362 | 1A30 | HLA class I histocompatibility antigen, A-30 alpha chain | Membrane (Single-pass type I membrane protein) | 205729 |
| 363 | 1A31 | HLA class I histocompatibility antigen, A-31 alpha chain | Membrane (Single-pass type I membrane protein) | 205730 |
| 364 | 1A33 | HLA class I histocompatibility antigen, A-33 alpha chain | Membrane (Single-pass type I membrane protein) | 205731 |
| 365 | 1A34 | HLA class I histocompatibility antigen, A-34 alpha chain | Membrane (Single-pass type I membrane protein) | 205732 |
| 366 | 1A68 | HLA class I histocompatibility antigen, A-68 alpha chain | Membrane (Single-pass type I membrane protein) | 205733 |
| 367 | 1A80 | HLA class I histocompatibility antigen, A-80 alpha chain | Membrane (Single-pass type I membrane protein) | 205734 |
| 368 | HLAE | HLA class I histocompatibility antigen, alpha chain E | | 205735 |
| 369 | HLAF | HLA class I histocompatibility antigen, alpha chain F | | 205736 |
| 370 | 1B15 | HLA class I histocompatibility antigen, B-15 alpha chain | Membrane (Single-pass type I membrane protein) | 205737 |
| 371 | 1B37 | HLA class I histocompatibility antigen, B-37 alpha chain | Membrane (Single-pass type I membrane protein) | 205738 |
| 372 | 1B40 | HLA class I histocompatibility antigen, B-40 alpha chain | Membrane (Single-pass type I membrane protein) | 205739 |
| 373 | 1A45 | HLA class I histocompatibility antigen, B-45 alpha chain | Membrane (Single-pass type I membrane protein) | 205740 |
| 374 | 1B47 | HLA class I histocompatibility antigen, B-47 alpha chain | Membrane (Single-pass type I membrane protein) | 205741 |
| 375 | 1B48 | HLA class I histocompatibility antigen, B-48 alpha chain | Membrane (Single-pass type I membrane protein) | 205742 |
| 376 | 1B52 | HLA class I histocompatibility antigen, B-52 alpha chain | Membrane (Single-pass type I membrane protein) | 205743 |
| 377 | 1B57 | HLA class I histocompatibility antigen, B-57 alpha chain | Membrane (Single-pass type I membrane protein) | 205744 |
| 378 | 1B59 | HLA class I histocompatibility antigen, B-59 alpha chain | Membrane (Single-pass type I membrane protein) | 205745 |
| 379 | 1B07 | HLA class I histocompatibility antigen, B-7 alpha chain | Membrane (Single-pass type I membrane protein) | 205746 |
| 380 | 1B78 | HLA class I histocompatibility antigen, B-78 alpha chain | Membrane (Single-pass type I membrane protein) | 205747 |
| 381 | 1B82 | HLA class I histocompatibility antigen, B-82 alpha chain | Membrane (Single-pass type I membrane protein) | 205748 |
| 382 | 1C01 | HLA class I histocompatibility antigen, Cw-1 alpha chain | Membrane (Single-pass type I membrane protein) | 205749 |
| 383 | 1C14 | HLA class I histocompatibility antigen, Cw-14 alpha chain | Membrane (Single-pass type I membrane protein) | 205750 |
| 384 | 1C15 | HLA class I histocompatibility antigen, Cw-15 alpha chain | Membrane (Single-pass type I membrane protein) | 205751 |
| 385 | 1C16 | HLA class I histocompatibility antigen, Cw-16 alpha chain | Membrane (Single-pass type I membrane protein) | 205752 |
| 386 | 1C17 | HLA class I histocompatibility antigen, Cw-17 alpha chain | Membrane (Single-pass type I membrane protein) | 205753 |
| 387 | 1C18 | HLA class I histocompatibility antigen, Cw-18 alpha chain | Membrane (Single-pass type I membrane protein) | 205754 |
| 388 | 1C03 | HLA class I histocompatibility antigen, Cw-3 alpha chain | Membrane (Single-pass type I membrane protein) | 205755 |
| 389 | 1C04 | HLA class I histocompatibility antigen, Cw-4 alpha chain | Membrane (Single-pass type I membrane protein) | 205756 |
| 390 | 1C05 | HLA class I histocompatibility antigen, Cw-5 alpha chain | Membrane (Single-pass type I membrane protein) | 205757 |
| 391 | 1C07 | HLA class I histocompatibility antigen, Cw-7 alpha chain | Membrane (Single-pass type I membrane protein) | 205758 |
| 392 | 2DOB | HLA class II histocompatibility antigen, DO beta chain | Membrane (Single-pass type I membrane protein) | 205759 |
| 393 | HB2O | HLA class II histocompatibility antigen, DP alpha chain | | 205760 |
| 394 | HB2P | HLA class II histocompatibility antigen, DP(W4) beta chain | | 205761 |
| 395 | HA21 | HLA class II histocompatibility antigen, DQ(1) alpha chain | | 205762 |
| 396 | HB21 | HLA class II histocompatibility antigen, DQ (1) beta chain | Membrane (Single-pass type I membrane protein) | 205763 |
| 397 | HA23 | HLA class II histocompatibility antigen, DQ (3) alpha chain | Membrane (Single-pass type I membrane protein) | 205764 |
| 398 | HB24 | HLA class II histocompatibility antigen, DQ (3) beta chain | Membrane (Single-pass type I membrane protein) | 205765 |
| 399 | HA25 | HLA class II histocompatibility antigen, DQ (5) alpha chain | Membrane (Single-pass type I membrane protein) | 205766 |
| 400 | HA26 | HLA class II histocompatibility antigen, DQ (6) alpha chain | | 205767 |
| 401 | HB22 | HLA class II histocompatibility antigen, DQ(W1.1) beta chain | Membrane (Single-pass type I membrane protein) | 205768 |
| 402 | HA27 | HLA class II histocompatibility antigen, DQ(W3) alpha chain | Membrane (Single-pass type I membrane protein) | 205769 |
| 403 | HB23 | HLA class II histocompatibility antigen, DQ(W3) beta chain | Membrane (Single-pass type I membrane protein) | 205770 |
| 404 | HB25 | HLA class II histocompatibility antigen, DQB1*0602 beta chain | Membrane (Single-pass type I membrane protein) | 205771 |
| 405 | 2DRA | HLA class II histocompatibility antigen, DR alpha chain | Membrane (Single-pass type I membrane protein) | 205772 |
| 406 | HB2B | HLA class II histocompatibility antigen, DR-1 beta chain | Membrane (Single-pass type I membrane protein) | 205773 |
| 407 | HB2C | HLA class II histocompatibility antigen, DR-1 beta chain | Membrane (Single-pass type I membrane protein) | 205774 |
| 408 | 2B11 | HLA class II histocompatibility antigen, DRB1-1 beta chain | Membrane (Single-pass type I membrane protein) | 205775 |
| 409 | 2B1A | HLA class II histocompatibility antigen, DRB1-10 beta chain | Membrane (Single-pass type I membrane protein) | 205776 |
| 410 | 2B1B | HLA class II histocompatibility antigen, DRB1-11 beta chain | Membrane (Single-pass type I membrane protein) | 205777 |
| 411 | 2B14 | HLA class II histocompatibility antigen, DRB1-4 beta chain | Membrane (Single-pass type I membrane protein) | 205778 |
| 412 | 2B17 | HLA class II histocompatibility antigen, DRB1-7 beta chain | Membrane (Single-pass type I membrane protein) | 205779 |
| 413 | 2B19 | HLA class II histocompatibility antigen, DRB1-9 beta chain | Membrane (Single-pass type I membrane protein) | 205780 |
| 414 | 2B32 | HLA class II histocompatibility antigen, DRB3-2 beta chain | Membrane (Single-pass type I membrane protein) | 205781 |
| 415 | HB2K | HLA class II histocompatibility antigen, DR-W53 beta chain | Membrane (Single-pass type I membrane protein) | 205782 |
| 416 | HB2X | HLA class II histocompatibility antigen, DX beta chain | | 205783 |
| 417 | HA22 | HLA class II histocompatibility antigen, DQ (2) alpha chain | Membrane (Single-pass type I membrane protein) | 205784 |
| 418 | HABP2 | Hyaluronan-binding protein 2 | Secreted | 205785 |
| 419 | HV102 | Ig heavy chain V-I region HG3 | Plasm membrane; extracellular space | 205786 |
| 420 | HV104 | Ig heavy chain V-I region ND | Plasm membrane; extracellular space | 205787 |
| 421 | HV103 | Ig heavy chain V-I region V35 | Plasm membrane; extracellular space | 205788 |
| 422 | HV209 | Ig heavy chain V-II region ARH-77 | | 205789 |
| 423 | HV208 | Ig heavy chain V-II region SESS precursor | | 205790 |
| 424 | HV303 | Ig heavy chain V-III region VH26 precursor | | 205791 |
| 425 | KV501 | Ig kappa chain V region EV15 | | 205792 |
| 426 | KV124 | Ig kappa chain V-I region Daudi | | 205793 |
| 427 | KV110 | Ig kappa chain V-I region HK102 | | 205794 |
| 428 | KV123 | Ig kappa chain V-I region Walker | | 205795 |
| 429 | KV205 | Ig kappa chain V-II region GM607 | | 205796 |
| 430 | KV206 | Ig kappa chain V-II region RPMI 6410 | | 205797 |
| 431 | KV308 | Ig kappa chain V-III region CLL | | 205798 |
| 432 | KV312 | Ig kappa chain V-III region HAH | | 205799 |
| 433 | KV311 | Ig kappa chain V-III region IARC/BL41 | | 205800 |
| 434 | KV303 | Ig kappa chain V-III region NG9 | | 205801 |
| 435 | KV309 | Ig kappa chain V-III region VG | | 205802 |
| 436 | KV310 | Ig kappa chain V-III region VH | | 205803 |
| 437 | KV401 | Ig kappa chain V-IV region | | 205804 |
| 438 | KV404 | Ig kappa chain V-IV region B17 | | 205805 |
| 439 | KV403 | Ig kappa chain V-IV region JI | | 205806 |
| 440 | LV001 | Ig lambda chain V region 4A | | 205807 |
| 441 | LV107 | Ig lambda chain V-I region BL2 | | 205808 |
| 442 | LV605 | Ig lambda chain V-VI region EB4 | | 205809 |
| 443 | FCGRN | IgG receptor FcRn large subunit p51 (FcRn) (Neonatal Fc, receptor) | Cell membrane (Single-pass type I membrane protein) | 205810 |
| 444 | INHA | Inhibin alpha chain | | 205811 |
| 445 | INS | Insulin | | 205812 |
| 446 | IGFL1 | Insulin growth factor-like family member 1 | Secreted | 205813 |
| 447 | IGFL3 | Insulin growth factor-like family member 3 | Secreted | 205814 |
| 448 | INSR | Insulin receptor | Cell membrane (Single-pass type I membrane protein) | 205815 |
| 449 | INSL3 | Insulin-like 3 (Leydig insulin-like peptide) | Secreted | 205816 |
| 450 | IGF1R | Insulin-like growth factor 1 receptor | | 205817 |
| 451 | IGF2 | Insulin-like growth factor II | Secreted | 205818 |
| 452 | IBP1 | Insulin-like growth factor-binding protein 1 | Secreted | 205819 |
| 453 | IBP2 | Insulin-like growth factor-binding protein 2 | Secreted | 205820 |
| 454 | IBP3 | Insulin-like growth factor-binding protein 3 | Secreted | 205821 |
| 455 | IBP4 | Insulin-like growth factor-binding protein 4 | Secreted | 205822 |
| 456 | IBP7 | Insulin-like growth factor-binding protein 7 | | 205823 |
| 457 | ALS | Insulin-like growth factor-binding protein complex acid labile chain | | 205824 |
| 458 | INSL5 | Insulin-like peptide INSL5 (Insulin-like peptide 5) | Secreted | 205825 |
| 459 | ITA2 | Integrin alpha-2 (Platelet membrane glycoprotein Ia) | Membrane (Single-pass type I membrane protein) | 205826 |
| 460 | ITA3 | Integrin alpha-3 | Membrane (Single-pass type I membrane protein) | 205827 |
| 461 | ITA4 | Integrin alpha-4 | Membrane (Single-pass type I membrane protein) | 205828 |
| 462 | ITA5 | Integrin alpha-5 | Membrane (Single-pass type I membrane protein) | 205829 |
| 463 | ITA6 | Integrin alpha-6 | Membrane (Single-pass type I membrane protein) | 205830 |
| 464 | ITA7 | Integrin alpha-7 | Membrane (Single-pass type I membrane protein) | 205831 |
| 465 | ITAE | Integrin Alpha-E | | 205832 |
| 466 | ITA2B | Integrin alpha-lib | Membrane (Single-pass type I membrane protein) | 205833 |
| 467 | ITA2B | Integrin alpha-IIb | | 205834 |
| 468 | ITAL | Integrin alpha-L (Leukocyte adhesion glycoprotein LFA-1, alpha chain) (LFA-1A) | Membrane (Single-pass type I membrane protein) | 205835 |
| 469 | ITAM | Integrin alpha-M | | 205836 |
| 470 | ITAV | Integrin alpha-V | | 205837 |
| 471 | ITAX | Integrin alpha-X | | 205838 |
| 472 | ITB1 | Integrin beta-1 | | 205839 |
| 473 | ITB2 | Integrin beta-2 | | 205840 |
| 474 | ITB2 | Integrin beta-2 | Membrane (Single-pass type I membrane protein) | 205841 |
| 475 | ITB4 | Integrin beta-4 (GP150) (CD104 antigen) | Membrane (Single-pass type I membrane protein) | 205842 |
| 476 | ITB7 | Integrin beta-7 | Membrane (Single-pass type I membrane protein) | 205843 |
| 477 | ITLN1 | Intelectin-1 | | 205844 |
| 478 | ITIH4 | Inter-alpha-trypsin inhibitor heavy chain H4 | Secreted | 205845 |
| 479 | ICAM1 | Intercellular adhesion molecule 1 | | 205846 |
| 480 | IFNA1 | Interferon alpha-1/13 | | 205847 |
| 481 | IFN10 | Interferon alpha-10 | | 205848 |
| 482 | IFN14 | Interferon alpha-14 | | 205849 |
| 483 | IFN16 | Interferon alpha-16 | Secreted | 205850 |
| 484 | IFN17 | Interferon alpha-17 | | 205851 |
| 485 | IFNA2 | Interferon alpha-2 | Secreted | 205852 |
| 486 | IFN21 | Interferon alpha-21 | | 205853 |
| 487 | IFNA4 | Interferon alpha-4 | Secreted | 205854 |
| 488 | IFNA5 | Interferon alpha-5 | Secreted | 205855 |
| 489 | IFNA6 | Interferon alpha-6 | Secreted | 205856 |
| 490 | IFNA7 | Interferon alpha-7 | Secreted | 205857 |
| 491 | IFNA8 | Interferon alpha-8 | Secreted | 205858 |
| 492 | IFNB | Interferon beta | Secreted | 205859 |
| 493 | IFNK | Interferon kappa (IFN-kappa) | Secreted | 205860 |
| 494 | IFNW1 | Interferon omega-1 (Interferon alpha-II-1) | Secreted | 205861 |
| 495 | INAR2 | Interferon-alpha/beta receptor beta chain | Membrane (Single-pass type I membrane protein) | 205862 |
| 496 | INGR1 | Interferon-gamma receptor alpha chain | | 205863 |
| 497 | IL20 | Interleukin 20 | | 205864 |
| 498 | IL1RA | Interleukin-1 receptor antagonist protein | Cytoplasm | 205865 |
| 499 | IL1R1 | Interleukin-1 receptor type I | Membrane (Single-pass type I membrane protein) | 205866 |
| 500 | ILRL1 | Interleukin-1 receptor-like 1 (ST2 protein) | Cell membrane (Single-pass type I membrane protein) | 205867 |
| 501 | ILRL2 | Interleukin-1 receptor-like 2 | | 205868 |
| 502 | IL10 | Interleukin-10 | Secreted | 205869 |
| 503 | I10R1 | Interleukin-10 receptor alpha chain | | 205870 |
| 504 | I10R2 | Interleukin-10 receptor beta chain | | 205871 |
| 505 | IL12A | Interleukin-12 subunit alpha | Secreted | 205872 |
| 506 | IL12B | Interleukin-12 subunit beta | Secreted | 205873 |
| 507 | I17RA | Interleukin-17 receptor A | | 205874 |
| 508 | I17RB | Interleukin-17 receptor B | Secreted | 205875 |
| 509 | I17RC | Interleukin-17 receptor C | Cell membrane (Single-pass type I membrane protein) | 205876 |
| 510 | IL17 | Interleukin-17A | | 205877 |
| 511 | IL17F | Interleukin-17F | | 205878 |
| 512 | IL18R | Interleukin-18 receptor 1 | | 205879 |
| 513 | I18BP | Interleukin-18-binding protein (IL-18BP) | Secreted | 205880 |
| 514 | IL19 | Interleukin-19 | Secreted | 205881 |
| 515 | IL2 | Interleukin-2 | Secreted | 205882 |
| 516 | IL2RA | Interleukin-2 receptor alpha chain | Membrane (Single-pass type I membrane protein) | 205883 |
| 517 | IL2RB | Interleukin-2 receptor subunit beta | Membrane (Single-pass type I membrane protein) | 205884 |
| 518 | I20RB | Interleukin-20 receptor beta chain (IL-20R-beta) | Membrane (Single-pass type I membrane protein) | 205885 |
| 519 | IL21R | Interleukin-21 receptor | Membrane (Single-pass type I membrane protein) | 205886 |
| 520 | IL22 | Interleukin-22 | | 205887 |
| 521 | I22RA | Interleukin-22 receptor alpha-2 chain (IL-22R-alpha-2) | Secreted | 205888 |
| 522 | IL24 | Interleukin-24 | Secreted | 205889 |
| 523 | IL25 | Interleukin-25 | Secreted | 205890 |
| 524 | IL3 | Interleukin-3 | Secreted | 205891 |
| 525 | IL31R | Interleukin-31 receptor A | | 205892 |
| 526 | IL4 | Interleukin-4 p | Secreted | 205893 |
| 527 | IL5 | Interleukin-5 | Secreted | 205894 |
| 528 | IL5RA | Interleukin-5 receptor alpha chain (IL-5R-alpha) (CD125,antigen) | Membrane (Single-pass type I membrane protein) | 205895 |
| 529 | IL6 | Interleukin-6 | | 205896 |
| 530 | IL6RA | Interleukin-6 receptor alpha chain | | 205897 |
| 531 | IL6RB | Interleukin-6 receptor beta chain | | 205898 |
| 532 | IL7 | Interleukin-7 | Secreted | 205899 |
| 533 | IL7RA | Interleukin-7 receptor alpha chain (IL-7R-alpha) (CD127,antigen) | Secreted; transmembrane | 205900 |
| 534 | IL8 | Interleukin-8 | | 205901 |
| 535 | IL9 | Interleukin-9 | | 205902 |
| 536 | IRBP | Interphotoreceptor retinoid-binding protein | Secreted (extracellular space, extracellular matrix, interphotoreceptor matrix) | 205903 |
| 537 | MMP1 | Interstitial collagenase | Secreted | 205904 |
| 538 | PPBI | Intestinal alkaline phosphatase | | 205905 |
| 539 | JAM1 | Junctional adhesion molecule A | Cell junction, tight junction (Single-pass type I membrane protein) | 205906 |
| 540 | JAM2 | Junctional adhesion molecule B | Cell junction, tight junction (Single-pass type I membrane protein) | 205907 |
| 541 | JAM3 | Junctional adhesion molecule C | Cell junction, tight junction (Single-pass type I membrane protein) | 205908 |
| 542 | JAML1 | Junctional adhesion molecule-like | | 205909 |
| 543 | KLK7 | Kallikrein-7 | | 205910 |
| 544 | CASK | Kappa-casein | Secreted | 205911 |
| 545 | KAZD1 | Kazal-type serine protease inhibitor domain-containing protein 1 | | 205912 |
| 546 | FGF7 | Keratinocyte growth factor | | 205913 |
| 547 | KI2L1 | Killer cell immunoglobulin-like receptor 2DL1 | Cell membrane (Single-pass type I membrane protein) | 205914 |
| 548 | KIRR2 | Kin of IRRE-like protein 2 | Cell membrane (Single-pass type I membrane protein) | 205915 |
| 549 | KNG1 | Kininogen-1 | | 205916 |
| 550 | SCF | Kit ligand | | 205917 |
| 551 | KTEL1 | KTEL motif-containing protein 1 (CAP10-like 46 kDa protein) | Endoplasmic reticulum lumen | 205918 |
| 552 | SPIT1 | Kunitz-type protease inhibitor 1 (Hepatocyte growth factor, activator inhibitor type 1) | Secreted | 205919 |
| 553 | SPIT2 | Kunitz-type protease inhibitor 2 | | 205920 |
| 554 | MFGM | Lactadherin | | 205921 |
| 555 | LCTL | Lactase-like protein | Endoplasmic reticulum membrane (Single-pass membrane protein) | 205922 |
| 556 | LPH | Lactase-phlorizin hydrolase (Lactase-glycosylceramidase) | Apical cell membrane (Single-pass type I membrane protein) (Brush border) | 205923 |
| 557 | TRFL | Lactotransferrin | | 205924 |
| 558 | LAMB1 | Laminin subunit beta-1 | | 205925 |
| 559 | LAMC1 | Laminin subunit gamma-1 | | 205926 |
| 560 | OXLA | L-amino-acid oxidase | | 205927 |
| 561 | LEPR | Leptin receptor | | 205928 |
| 562 | A2GL | Leucine-rich alpha-2-glycoprotein precursor (LRG) | Secreted | 205929 |
| 563 | LRRN1 | Leucine-rich repeat neuronal protein 1 | Membrane (Single-pass type I membrane protein) | 205930 |
| 564 | FLRT2 | Leucine-rich repeat transmembrane protein FLRT2 | Membrane (Single-pass type I membrane protein) | 205931 |
| 565 | LRC55 | Leucine-rich repeat-containing protein 55 | Membrane (Single-pass type I membrane protein) | 205932 |
| 566 | LIF | Leukemia inhibitory factor | | 205933 |
| 567 | CD45 | Leukocyte common antigen | Cell membrane (Single-pass type I membrane protein) | 205934 |
| 568 | LIRA3 | Leukocyte immunoglobulin-like receptor subfamily A member 3 | Secreted | 205935 |
| 569 | LEUK | Leukosialin | | 205936 |
| 570 | LCN1 | Lipocalin-1 | Secreted | 205937 |
| 571 | LBP | Lipopolysaccharide-binding protein | Secreted; Cytoplasmic granule membrane | 205938 |
| 572 | LIPL | Lipoprotein lipase | | 205939 |
| 573 | REG1A | Lithostathine 1 alpha (Pancreatic stone protein) (PSP) | Islet cells | 205940 |
| 574 | EST1 | Liver carboxylesterase 1 (Acyl coenzyme, A: cholesterol acyltransferase) (ACAT) | Endoplasmic reticulum lumen | 205941 |
| 575 | FCG2A | Low affinity immunoglobulin gamma Fc region receptor II-a | | 205942 |
| 576 | LDLR | Low-density lipoprotein receptor | | 205943 |
| 577 | LYAM1 | L-selectin | | 205944 |
| 578 | LUM | Lumican precursor (Keratan sulfate proteoglycan lumican) | Secreted (extracellular space, extracellular matrix) | 205945 |
| 579 | LU | Lutheran blood group glycoprotein | | 205946 |
| 580 | LSHB | Lutropin subunit beta (Luteinizing hormone subunit beta) | Secreted | 205947 |
| 581 | LYPD5 | Ly6/PLAUR domain-containing protein 5 | Secreted (extracellular space) | 205948 |
| 582 | LYPD6 | Ly6/PLAUR domain-containing protein 6 | Secreted (extracellular space) | 205949 |
| 583 | SLUR1 | Ly-6/uPAR-related protein 1 | Secreted | 205950 |
| 584 | LAG3 | Lymphocyte activation gene 3 protein | | 205951 |
| 585 | LY6D | Lymphocyte antigen 6D | | 205952 |
| 586 | LY75 | Lymphocyte antigen 75 | | 205953 |
| 587 | LY86 | Lymphocyte antigen 86 | Secreted (extracellular space) | 205954 |
| 588 | LFA3 | Lymphocyte function-associated antigen 3 | | 205955 |
| 589 | TNFB | Lymphotoxin-alpha (LT-alpha) (TNF-beta) (Tumor necrosis, factor ligand superfamily member 1) | Secreted; Membrane (The homotrimer is secreted. The heterotrimer is membrane) | 205956 |
| 590 | PPA6 | Lysophosphatidic acid phosphatase type 6 | Secreted | 205957 |
| 591 | PPAL | Lysosomal acid phosphatase | | 205958 |
| 592 | MA2B1 | Lysosomal alpha-mannosidase | Lysosome | 205959 |
| 593 | PPGB | Lysosomal protective protein | Lysosome | 205960 |
| 594 | LAMP1 | Lysosome-associated membrane glycoprotein 1 | | 205961 |
| 595 | LAMP2 | Lysosome-associated membrane glycoprotein 2 | | 205962 |
| 596 | LYSC | Lysozyme C | Lysosome | 205963 |
| 597 | CSF1 | Macrophage colony-stimulating factor 1 | | 205964 |
| 598 | MIP2A | Macrophage inflammatory protein 2-alpha (MIP2-alpha) | Secreted | 205965 |
| 599 | MIP2B | Macrophage inflammatory protein 2-beta (MIP2-beta) | Secreted | 205966 |
| 600 | HMR1 | Major histocompatibility complex class I-related gene protein | | 205967 |
| 601 | SG2A1 | Mammaglobin-B | | 205968 |
| 602 | MBL2 | Mannose-binding protein C | | 205969 |
| 603 | CBPA3 | Mast cell carboxypeptidase A | Secretory granules; Cytoplasmic vesicle | 205970 |
| 604 | MMP7 | Matrilysin | | 205971 |
| 605 | MGP | Matrix Gla protein | | 205972 |
| 606 | MMP9 | Matrix metalloproteinase-9 | | 205973 |
| 607 | PME17 | Melanocyte protein Pmel 17 | | 205974 |
| 608 | MIA | Melanoma-derived growth regulatory protein | Secreted | 205975 |
| 609 | TRFM | Melanotransferrin | | 205976 |
| 610 | MCP | Membrane cofactor protein (Trophoblast leukocyte common, antigen) (TLX) (CD46 antigen) | Acrosome inner membrane (Single-pass type I membrane protein) | 205977 |
| 611 | MBTP1 | Membrane-bound transcription factor site-1 protease | | 205978 |
| 612 | MSLN | Mesothelin | | 205979 |
| 613 | TIMP1 | Metalloproteinase inhibitor 1 | | 205980 |
| 614 | TIMP2 | Metalloproteinase inhibitor 2 | | 205981 |
| 615 | TIMP3 | Metalloproteinase inhibitor 3 (TIMP-3) | Secreted, extracellular space, extracellular matrix | 205982 |
| 616 | MICA | MHC class I polypeptide-related sequence A | | 205983 |
| 617 | MFAP4 | Microfibril-associated glycoprotein 4 | | 205984 |
| 618 | MK | Midkine | | 205985 |
| 619 | MIME | Mimecan (Osteoglycin) | Secreted (extracellular space, extracellular matrix) | 205986 |
| 620 | CD14 | Monocyte differentiation antigen CD14 | | 205987 |
| 621 | MUC1 | Mucin-1 | | 205988 |
| 622 | MUCL1 | Mucin-like protein 1 | | 205989 |
| 623 | MYP0 | Myelin P0 protein | | 205990 |
| 624 | MPZL3 | Myelin protein zero-like protein 3 | Membrane (Single-pass type I membrane protein) | 205991 |
| 625 | MAG | Myelin-associated glycoprotein | | 205992 |
| 626 | MOG | Myelin-oligodendrocyte glycoprotein | | 205993 |
| 627 | PRTN3 | Myeloblastin | | 205994 |
| 628 | PERM | Myeloperoxidase | | 205995 |
| 629 | ASPG | N(4)-(Beta-N-acetylglucosaminyl)-L-asparaginase (Glycosylasparaginase) | Lysosome | 205996 |
| 630 | GALNS | N-acetylgalactosamine-6-sulfatase | | 205997 |
| 631 | GNS | N-acetylglucosamine-6-sulfatase | | 205998 |
| 632 | PGRP2 | N-acetylmuramoyl-L-alanine amidase | | 205999 |
| 633 | ANFB | Natriuretic peptides B | | 206000 |
| 634 | CD244 | Natural killer cell receptor 2B4 (NKR2B4) | Membrane (Single-pass type I membrane protein) | 206001 |
| 635 | NEU1 | NEU 1 protein | | 206002 |
| 636 | L1CAM | Neural cell adhesion molecule L1 (N-CAM L1) (CD171 antigen) | Cell membrane (Single-pass type I membrane protein) | 206003 |
| 637 | NXPH3 | Neurexophilin-3 | Secreted | 206004 |
| 638 | NBL1 | Neuroblastoma suppressor of tumorigenicity 1 | | 206005 |
| 639 | 7B2 | Neuroendocrine protein 7B2 (Secretogranin-5) | Secreted, transport | 206006 |
| 640 | NLGNX | Neuroligin-4, X-linked (Neuroligin X) | Membrane (Single-pass type I membrane protein) | 206007 |
| 641 | NMB | Neuromedin-B | Secreted | 206008 |
| 642 | NETO2 | Neuropilin and tolloid-like protein 2 | | 206009 |
| 643 | NRP1 | Neuropilin-1 | | 206010 |
| 644 | NPTN | Neuroplastin | | 206011 |
| 645 | NTRI | Neurotrimin | | 206012 |
| 646 | MMP8 | Neutrophil collagenase | | 206013 |
| 647 | DEF1 | Neutrophil defensin 1 | | 206014 |
| 648 | DEF3 | Neutrophil defensin 3 | | 206015 |
| 649 | NGAL | Neutrophil gelatinase-associated lipocalin | | 206016 |
| 650 | NID1 | Nidogen-1 | Secreted (extracellular space, extracellular matrix, basement membrane) | 206017 |
| 651 | NID2 | Nidogen-2 | | 206018 |
| 652 | N2DL1 | NKG2D ligand 2 | | 206019 |
| 653 | CEAM5 | noembryonic antigen-related cell adhesion molecule 5 | Cell membrane (lipid anchor) | 206020 |
| 654 | RNAS2 | Non-secretory ribonuclease | | 206021 |
| 655 | SPHM | N-sulphoglucosamine sulphohydrolase | | 206022 |
| 656 | NTRK3 | NT-3 growth factor receptor | | 206023 |
| 657 | I20RA | nterleukin-20 receptor alpha chain | | 206024 |
| 658 | OLFL1 | Olfactomedin-like protein 1 | Secreted | 206025 |
| 659 | OMGP | Oligodendrocyte-myelin glycoprotein | | 206026 |
| 660 | ONCM | Oncostatin-M | | 206027 |
| 661 | OTOR | Otoraplin (Fibrocyte-derived protein) | Secreted | 206028 |
| 662 | AMYP | Pancreatic alpha-amylase | | 206029 |
| 663 | PAHO | Pancreatic prohormone | | 206030 |
| 664 | ISK1 | Pancreatic secretory trypsin inhibitor | | 206031 |
| 665 | LIPP | Pancreatic triacylglycerol lipase | Secreted | 206032 |
| 666 | PTHY | Parathyroid hormone precursor (Parathyrin) (PTH) | Secreted | 206033 |
| 667 | PEPA | Pepsin A | | 206034 |
| 668 | PI16 | Peptidase inhibitor 16 | | 206035 |
| 669 | YQ001 | Peptide | | 206036 |
| 670 | PYY | Peptide YY (PYY) (PYY-I) | Secreted | 206037 |
| 671 | PPIB | Peptidyl-prolyl cis-trans isomerase B | Endoplasmic reticulum lumen; Melanosome | 206038 |
| 672 | PERF | Perforin-1 | | 206039 |
| 673 | LCAT | Phosphatidylcholine-sterol acvltransferase | | 206040 |
| 674 | PEBP4 | Phosphatidylethanolamine-binding protein 4 | Lysosome | 206041 |
| 675 | PHLD | Phosphatidylinositol-glycan-specific phospholipase D | | 206042 |
| 676 | P3IP1 | Phosphoinositide-3-kinase-interacting protein 1 | Membrane (Single-pass type I membrane protein) | 206043 |
| 677 | PA21B | Phospholipase A2 | | 206044 |
| 678 | PA2GA | Phospholipase A2, membrane associated | Membrane | 206045 |
| 679 | PLTP | Phospholipid transfer protein | | 206046 |
| 680 | PLGF | Placenta growth factor | | 206047 |
| 681 | PP11 | Placental protein 11 | | 206048 |
| 682 | KLKB1 | Plasma kallikrein (Plasma prekallikrein) | Secreted | 206049 |
| 683 | IC1 | Plasma protease C1 inhibitor | Membrane (postsynaptic membrane) | 206050 |
| 684 | IPSP | Plasma serine protease inhibitor | | 206051 |
| 685 | PLMN | Plasminogen | | 206052 |
| 686 | PAI1 | Plasminogen activator inhibitor 1 | | 206053 |
| 687 | CXCL7 | Platelet basic protein (PBP) (C-X-C motif chemokine 7) | Secreted | 206054 |
| 688 | PECA1 | Platelet endothelial cell adhesion molecule (PECAM-1) | Membrane (Single-pass type I membrane protein) | 206055 |
| 689 | PLF4 | Platelet factor 4 | | 206056 |
| 690 | PF4V | Platelet factor 4 variant | | 206057 |
| 691 | GP1BA | Platelet glycoprotein Ib alpha chain | Membrane (Single-pass type I membrane protein) | 206058 |
| 692 | GP1BB | Platelet glycoprotein Ib beta chain | Membrane (Single-pass type I membrane protein) | 206059 |
| 693 | GPIX | Platelet glycoprotein IX(GPIX) (CD42a antigen) | Membrane (Single-pass type I membrane protein) | 206060 |
| 694 | GI24 | Platelet receptor Gi24 | Membrane (Single-pass type I membrane protein) | 206061 |
| 695 | PAFA | Platelet-activating factor acetylhydrolase | | 206062 |
| 696 | PDGFA | Platelet-derived growth factor subunit A | | 206063 |
| 697 | PDGFB | Platelet-derived growth factor subunit B | | 206064 |
| 698 | PTN | Pleiotrophin | | 206065 |
| 699 | PIGR | Polvmeric immunoglobulin receptor | | 206066 |
| 700 | PORIM | Porimin | | 206067 |
| 701 | PZP | Pregnancy zone protein | | 206068 |
| 702 | PSG1 | Pregnancy-specific beta-1-glycoprotein 1 | | 206069 |
| 703 | PSG2 | Pregnancy-specific beta-1-glycoprotein 2 | | 206070 |
| 704 | PCYXL | Prenylcysteine oxidase-like | | 206071 |
| 705 | SAP | Proactivator polypeptide | | 206072 |
| 706 | GPR97 | Probable G-protein coupled receptor 97 | Cell membrane (Multi-pass membrane protein) | 206073 |
| 707 | PCOC2 | Procollagen C-endopeptidase enhancer 2 | | 206074 |
| 708 | GON1 | Progonadoliberin-1 | | 206075 |
| 709 | PD1L2 | Programmed cell death 1 ligand 2 | | 206076 |
| 710 | PROK1 | Prokineticin-1 (Endocrine-gland-derived vascular endothelial, growth factor) | Secreted | 206077 |
| 711 | PROK2 | Prokineticin-2 (PK2) (Protein Bv8 homolog) | Secreted | 206078 |
| 712 | PRL | Prolactin | Secreted | 206079 |
| 713 | PRLR | Prolactin receptor | | 206080 |
| 714 | PIP | Prolactin-inducible protein (Prolactin-induced protein) | Secreted | 206081 |
| 715 | PRRT3 | Proline-rich transmembrane protein 3 | Membrane (Multi-pass membrane protein) | 206082 |
| 716 | P4HA1 | Prolyl 4-hydroxylase subunit alpha-1 | | 206083 |
| 717 | MOTI | Promotilin | | 206084 |
| 718 | NPY | Pro-neuropeptide Y | Secreted | 206085 |
| 719 | PROP | Properdin r (Complement factor P) | Secreted | 206086 |
| 720 | PCSK9 | Proprotein convertase subtilisin/kexin type 9 | | 206087 |
| 721 | REL2 | Prorelaxin H2 | | 206088 |
| 722 | PGH1 | Prostaglandin G/H synthase 1 | | 206089 |
| 723 | PTGDS | Prostaglandin-H2 D-isomerase | Rough endoplasmic reticulum. Nucleus membrane. Golgi apparatus. Cytoplasm, perin | 206090 |
| 724 | PSCA | Prostate stem cell antigen | | 206091 |
| 725 | KLK3 | Prostate-specific antigen (PSA) (Kallikrein-,3) | Secreted | 206092 |
| 726 | PPAP | Prostatic acid phosphatase | | 206093 |
| 727 | AMBP | Protein AMBP | | 206094 |
| 728 | ARMET | Protein ARMET | | 206095 |
| 729 | CREG1 | Protein CREG1 | | 206096 |
| 730 | PDIA1 | Protein disulfide-isomerase | | 206097 |
| 731 | PDIA3 | Protein disulfide-isomerase A3 | Endoplasmic reticulum lumen | 206098 |
| 732 | LMAN1 | Protein ERGIC-53 (ER-Golgi intermediate compartment 53 kDa, protein) | Endoplasmic reticulum-Golgi intermediate compartment membrane (Single-pass type) | 206099 |
| 733 | G6B | Protein G6b | Endoplasmic reticulum and Golgi apparatus (Isoforms A E and D); Cell membrane (Single-pass type I membrane protein) (Isoforms A and B) | 206100 |
| 734 | NELL2 | Protein kinase C-binding protein NELL2 | Secreted | 206101 |
| 735 | NOV | Protein NOV homolog (NovH) | Secreted | 206102 |
| 736 | PARM1 | Protein PARM-1 | Membrane (Single-pass type I membrane protein) | 206103 |
| 737 | ZPI | Protein Z-dependent protease inhibitor | | 206104 |
| 738 | PCDA2 | Protocadherin alpha-2 | | 206105 |
| 739 | PCDBA | Protocadherin beta 10 (PCDH-beta10 | Cell membrane (Single-pass type I membrane protein) | 206106 |
| 740 | LYAM3 | P-selectin | | 206107 |
| 741 | SFTA1 | Pulmonary surfactant-associated protein A1 | | 206108 |
| 742 | SFTA2 | Pulmonary surfactant-associated protein A2 | | 206109 |
| 743 | PLBL2 | Putative phospholipase B-like 2 | Lysosome lumen | 206110 |
| 744 | PTPRG | Receptor-type tyrosine-protein phosphatase gamma | Membrane (Single-pass type I membrane protein) | 206111 |
| 745 | REG3G | Regenerating islet-derived protein 3 gamma | Secreted | 206112 |
| 746 | REG4 | Regenerating islet-derived protein 4 | | 206113 |
| 747 | REL3 | Relaxin-3 | | 206114 |
| 748 | RENI | Renin | | 206115 |
| 749 | RISC | Retinoid-inducible serine carboxypeptidase | | 206116 |
| 750 | RET3 | Retinol-binding protein 3 | | 206117 |
| 751 | RET4 | Retinol-binding protein 4 | | 206118 |
| 752 | RNAS4 | Ribonuclease 4 | Secreted | 206119 |
| 753 | RNAS1 | Ribonuclease pancreatic | Secreted | 206120 |
| 754 | RIB1 | Ribonuclease, RNase A Family, 1 (Pancreatic) | | 206121 |
| 755 | PLOD1 | rocollagen-lysine,2-oxoglutarate 5-dioxygenase 1 | | 206122 |
| 756 | PRPC | Salivary acidic proline-rich phosphoprotein 1/2 | | 206123 |
| 757 | SRCH | Sarcoplasmic reticulum histidine-rich calcium-binding protein | Sarcoplasmic reticulum lumen | 206124 |
| 758 | C163B | Scavenger receptor cysteine-rich type 1 protein M160 | | 206125 |
| 759 | SOST | Sclerostin | | 206126 |
| 760 | SOSD1 | Sclerostin domain-containing protein 1 (Ectodermal BMP, inhibitor) | Secreted | 206127 |
| 761 | SCRG1 | Scrapie-responsive protein 1 | Secreted | 206128 |
| 762 | SFRP1 | Secreted frizzled-related protein 1 | | 206129 |
| 763 | SFRP2 | Secreted frizzled-related protein 2 (sFRP-2) | Secreted (Wnt signaling pathway) | 206130 |
| 764 | SFRP3 | Secreted frizzled-related protein 3 (sFRP-3) | Secreted (Wnt signaling pathway) | 206131 |
| 765 | SG1D4 | Secretoglobin family 1D member 4 (IFN-gamma-inducible, secretoglobin) | Secreted | 206132 |
| 766 | SCG1 | Secretogranin-1 | | 206133 |
| 767 | SEPP1 | Selenoprotein P | | 206134 |
| 768 | SEM4B | Semaphorin-4B | Membrane (Single-pass type I membrane protein) | 206135 |
| 769 | SEM6B | Semaphorin-6B (Semaphorin-Z) (Sema Z) | Membrane (Single-pass type I membrane protein) | 206136 |
| 770 | SEMG1 | Semenogelin-1 (Semenogelin I) (SGI) | Secreted | 206137 |
| 771 | SEMG2 | Semenogelin-2 | | 206138 |
| 772 | SRGN | Serglycin (Secretory granule proteoglycan core protein), (Platelet proteoglycan core protein) | Cytoplasmic granule | 206139 |
| 773 | ISK2 | serine protease inhibitor Kazal-type 2 | | 206140 |
| 774 | ISK6 | Serine protease inhibitor Kazal-type 6 | | 206141 |
| 775 | TRFE | Serotransferrin | | 206142 |
| 776 | ALBU | Serum albumin | Secreted | 206143 |
| 777 | SAA | Serum amyloid A protein (SAA) | Secreted | 206144 |
| 778 | SAA4 | Serum amyloid A-4 protein | Secreted | 206145 |
| 779 | SAMP | Serum amyloid P-component (SAP) | Secreted | 206146 |
| 780 | SHBG | Sex hormone-binding globulin (SHBG) (Sex steroid-binding, protein) (SBP) (Testis-specific androgen-binding protein) (ABP) | Secreted (In testis by Sertoli cells) | 206147 |
| 781 | NEUR1 | Sialidase-1 | Lysosome (membrane and lumen); cell membrane; cytoplasmic vesicle | 206148 |
| 782 | SIDT2 | SID1 transmembrane family member 2 | Membrane (Multi-pass membrane protein) | 206149 |
| 783 | SLAF5 | SLAM family member 5 | | 206150 |
| 784 | SLAF6 | SLAM family member 6 (NK-T-B-antigen) (NTB-A) | Membrane (Single-pass type I membrane protein) | 206151 |
| 785 | SLAF7 | SLAM family member 7 (CD2-like receptor-activating cytotoxic, cells) (CRACC) | Membrane (Single-pass type I membrane protein) | 206152 |
| 786 | SLAF8 | SLAM family member 8 | | 206153 |
| 787 | SMS | Somatostatin (Growth hormone release-inhibiting factor) | Secreted | 206154 |
| 788 | SOMA | Somatotropin | | 206155 |
| 789 | SPRC | SPARC | | 206156 |
| 790 | ASM | Sphingomyelin phosphodiesterase | Lysosome | 206157 |
| 791 | STC1 | Stanniocalcin-1 (STC-1) | Secreted | 206158 |
| 792 | STAT | Statherin | Secreted | 206159 |
| 793 | STS | Steryl-sulfatase | | 206160 |
| 794 | STIM2 | Stromal interaction molecule 2 | | 206161 |
| 795 | SMR3B | Submaxillary gland androgen-regulated protein 3 homolog B | Secreted | 206162 |
| 796 | SUMF1 | Sulfatase-modifying factor 1 | | 206163 |
| 797 | SFTPG | Surfactant-associated protein G | | 206164 |
| 798 | TPSN | Tapasin | | 206165 |
| 799 | TPSNR | Tapasin-related protein (TAPASIN-R) | Cell membrane (Single-pass type I membrane protein); Endoplasmic reticulum | 206166 |
| 800 | PPA5 | Tartrate-resistant acid phosphatase type 5 | | 206167 |
| 801 | CD7 | T-cell antigen CD7 | | 206168 |
| 802 | TVA2 | T-cell receptor alpha chain V region CTL-L17 | membrane | 206169 |
| 803 | TVA1 | T-cell receptor alpha chain V region HPB-MLT | | 206170 |
| 804 | TVA3 | T-cell receptor alpha chain V region PY14 | | 206171 |
| 805 | TVB2 | T-cell receptor beta chain V region CTL-L17 | membrane | 206172 |
| 806 | TVC | T-cell receptor gamma chain V region PT-gamma-1/2 | | 206173 |
| 807 | CD2 | T-cell surface antigen CD2 precursor (T-cell surface antigen T11/Leu-,5) | Membrane (Single-pass type I membrane protein) | 206174 |
| 808 | CD1A | T-cell surface glycoprotein CD1a | | 206175 |
| 809 | CD1E | T-cell surface glycoprotein CD1e | | 206176 |
| 810 | CD3D | T-cell surface glycoprotein CD3 delta chain | | 206177 |
| 811 | CD3E | T-cell surface glycoprotein CD3 epsilon chain (T-cell, surface antigen T3/Leu-4 epsilon chain) | Membrane (Single-pass type I membrane protein) | 206178 |
| 812 | CD3G | T-cell surface glycoprotein CD3 gamma chain | | 206179 |
| 813 | CD3Z | T-cell surface glycoprotein CD3 zeta chain (T-cell receptor, T3 zeta chain) (CD247 antigen) | Membrane (Single-pass type I membrane protein) | 206180 |
| 814 | CD4 | T-cell surface glycoprotein CD4 | | 206181 |
| 815 | CD5 | T-cell surface glycoprotein CD5 (Lymphocyte antigen T1/Leu-,1) (CD5 antigen) | Cell membrane (Single-pass type I membrane protein) | 206182 |
| 816 | CD8A | T-cell surface glycoprotein CD8 alpha chain | | 206183 |
| 817 | CD8B | T-cell surface glycoprotein CD8 beta chain | | 206184 |
| 818 | CD28 | T-cell-specific surface glycoprotein CD28 | Membrane (Single-pass type I membrane protein) | 206185 |
| 819 | TENA | Tenascin | | 206186 |
| 820 | TDGF1 | Teratocarcinoma-derived growth factor 1 | | 206187 |
| 821 | TICN2 | Testican-2 (SPARC/osteonectin, CWCV, and Kazal-like domains, proteoglycan 2) | Secreted, extracellular space, extracellular matrix | 206188 |
| 822 | TETN | Tetranectin (TN) (C-type lectin domain family 3-member B) | Secreted | 206189 |
| 823 | TGFR2 | TGF-beta receptor type-2 | Membrane (Single-pass type I membrane protein) | 206190 |
| 824 | TXD12 | Thioredoxin domain-containing protein 12 (Thioredoxin-like protein p19) | Endoplasmic reticulum lumen | 206191 |
| 825 | TXND4 | Thioredoxin domain-containing protein 4 (Endoplasmic, reticulum resident protein ERp44) | Endoplasmic reticulum lumen | 206192 |
| 826 | TRBM | Thrombomodulin | | 206193 |
| 827 | TPO | Thrombopoietin | | 206194 |
| 828 | TSP1 | Thrombospondin-1 | | 206195 |
| 829 | THY1 | Thy-1 membrane glycoprotein | | 206196 |
| 830 | THYG | Thyroglobulin (Tg) | Secreted | 206197 |
| 831 | TSHR | Thyrotropin receptor | | 206198 |
| 832 | TSHB | Thyrotropin subunit beta precursor (Thyroid-stimulating hormone, subunit beta) (TSH-beta) (TSH-B) | Secreted | 206199 |
| 833 | THBG | Thyroxine-binding globulin | Secreted | 206200 |
| 834 | TF | Tissue factor | | 206201 |
| 835 | TFPI1 | Tissue factor pathway inhibitor (TFPI) (Lipoprotein-, associated coagulation inhibitor) (LACI) | Secreted | 206202 |
| 836 | TFPI2 | Tissue factor pathway inhibitor 2 | | 206203 |
| 837 | TPA | Tissue-type plasminogen activator | | 206204 |
| 838 | CD80 | T-lymphocyte activation antigen CD80 | | 206205 |
| 839 | TM2D1 | TM2 domain-containing protein 1 (Beta-amyloid-binding, protein) | Membrane (Multi-pass membrane protein) | 206206 |
| 840 | TLR1 | Toll-like receptor 1 (Toll/interleukin-1 receptor-like, protein) (TIL) (CD281 antigen) | Cell membrane (Single-pass type I membrane protein); Cytoplasmic | 206207 |
| 841 | TLR3 | Toll-like receptor 3 (CD283 antigen) | Membrane (Single-pass type I membrane protein) | 206208 |
| 842 | TLR4 | Toll-like receptor 4 (hToll) (CD284 antigen) | Membrane (Single-pass type I membrane protein) | 206209 |
| 843 | TLR5 | Toll-like receptor 5 | Membrane (Single-pass type I membrane protein) | 206210 |
| 844 | TEFF | Tomoregulin-2 | | 206211 |
| 845 | TCO1 | Transcobalamin-1 (Transcobalamin I) (TCI) | Secreted | 206212 |
| 846 | TCO2 | Transcobalamin-2 | | 206213 |
| 847 | TGFB1 | Transforming growth factor beta-1 | Secreted | 206214 |
| 848 | SSRA | Translocon-associated protein subunit alpha | | 206215 |
| 849 | TMIG2 | Transmembrane and immunoglobulin domain-containing protein 2 | Membrane (Single-pass type I membrane protein) | 206216 |
| 850 | TMM25 | Transmembrane protein 25, Isoform 3 | Secreted; Signal; Transmembrane | 206217 |
| 851 | TMM46 | Transmembrane protein 46 | | 206218 |
| 852 | TMM66 | Transmembrane protein 66 | | 206219 |
| 853 | TMM9B | Transmembrane protein 9B | | 206220 |
| 854 | TTHY | Transthyretin | | 206221 |
| 855 | TFF1 | Trefoil factor 1 (pS2 protein) | Secreted | 206222 |
| 856 | TFF3 | Trefoil factor 3 | | 206223 |
| 857 | TPP1 | Tripeptidyl-peptidase 1 | | 206224 |
| 858 | TRY1 | Trypsin-1 (Trypsin I) (Cationic trypsinogen) | Secreted, extracellular space | 206225 |
| 859 | TRY2 | Trypsin-2 (Trypsin II) (Anionic trypsinogen) | Secreted, extracellular space | 206226 |
| 860 | TINAL | Tubulointerstitial nephritis antigen-like | | 206227 |
| 861 | TR10B | Tumor necrosis factor receptor superfamily member 10B | | 206228 |
| 862 | TNR10C | Tumor necrosis factor receptor superfamily member 10C | | 206229 |
| 863 | TR10D | Tumor necrosis factor receptor superfamily member 10D (Decoy, receptor 2) (DcR2) | Membrane; Single-pass type I membrane protein | 206230 |
| 864 | TR11B | Tumor necrosis factor receptor superfamily member 11B | Secreted | 206231 |
| 865 | TNR14 | Tumor necrosis factor receptor superfamily member 14 (Herpesvirus entry mediator A) | Membrane (Single-pass type I membrane protein) | 206232 |
| 866 | TNR16 | Tumor necrosis factor receptor superfamily member 16 (Low-, affinity nerve growth factor receptor) | Membrane (Single-pass type I membrane protein) | 206233 |
| 867 | TNR18 | Tumor necrosis factor receptor superfamily member 18(Glucocorticoid-induced TNFR-related protein) | Secreted | 206234 |
| 868 | TNR19 | Tumor necrosis factor receptor superfamily member 19 | Membrane (Single-pass type I membrane protein) | 206235 |
| 869 | TR19L | Tumor necrosis factor receptor superfamily member 19L | Cell membrane (Single-pass type I membrane protein); Cytoplasm | 206236 |
| 870 | TNR1A | Tumor necrosis factor receptor superfamily member 1A | | 206237 |
| 871 | TNR1B | Tumor necrosis factor receptor superfamily member 1B (Tumor, necrosis factor receptor 2) (TNF-R2) | Secreted | 206238 |
| 872 | TNR5 | Tumor necrosis factor receptor superfamily member 5 (CD40L, receptor) | Secreted | 206239 |
| 873 | TNR6B | Tumor necrosis factor receptor superfamily member 6B | Secreted | 206240 |
| 874 | TNR8 | Tumor necrosis factor receptor superfamily member 8 | Cell membrane (Single-pass type I membrane protein) (isoform 1); cytoplasm (isoform 2) | 206241 |
| 875 | TNR9 | Tumor necrosis factor receptor superfamily member 9 (4-1BB, ligand receptor) | Membrane (Single-pass type I membrane protein) | 206242 |
| 876 | EDAR | Tumor necrosis factor receptor superfamily member EDAR, (Anhidrotic ectodysplasin receptor 1) | Membrane (Single-pass type I membrane protein) | 206243 |
| 877 | TSG6 | Tumor necrosis factor-inducible gene 6 protein | | 206244 |
| 878 | TIE1 | Tyrosine-protein kinase receptor Tie-1 | Membrane (Single-pass type I membrane protein) | 206245 |
| 879 | CK083 | Uncharacterized protein C11orf83 | | 206246 |
| 880 | CQ099 | Uncharacterized protein C17orf99 | | 206247 |
| 881 | YK001 | Uncharacterized protein UNQ655/PRO1286 | Secreted; Signal | 206248 |
| 882 | UPAR | Urokinase plasminogen activator surface receptor | | 206249 |
| 883 | UROK | Urokinase-type plasminogen activator | | 206250 |
| 884 | UROM | Uromodulin | | 206251 |
| 885 | UTS2 | Urotensin-2 (Urotensin-II) | Secreted; Signal | 206252 |
| 886 | UTER | Uteroglobin | | 206253 |
| 887 | BPHL | Valacyclovir hydrolase | | 206254 |
| 888 | VCAM1 | Vascular cell adhesion protein 1 (V-CAM 1) (CD106 antigen) | Membrane; Single-pass type I membrane protein | 206255 |
| 889 | VEGFA | Vascular endothelial growth factor A (VEGF-A) | Secreted; Signal | 206256 |
| 890 | VEGFC | Vascular endothelial growth factor C (VEGF-C) (Vascular, endothelial growth factor-related protein) (VRP) (Flt4 ligand) | Secreted; Signal | 206257 |
| 891 | VGFR1 | Vascular endothelial growth factor receptor 1 | | 206258 |
| 892 | VGFR3 | Vascular endothelial growth factor receptor 3 | | 206259 |
| 893 | NEU2 | Vasopressin-neurophysin 2-copeptin | Secreted | 206260 |
| 894 | VCC1 | VEGF co-regulated chemokine 1 | | 206261 |
| 895 | CSPG2 | Versican core protein (Large fibroblast proteoglycan) | Secreted (extracellular space, extracellular matrix) | 206262 |
| 896 | VTDB | Vitamin D-binding protein | | 206263 |
| 897 | PROC | Vitamin K-dependent protein C | | 206264 |
| 898 | PROS | Vitamin K-dependent protein S | | 206265 |
| 899 | PROZ | Vitamin K-dependent protein Z | | 206266 |
| 900 | VMO1 | Vitelline membrane outer layer protein 1 homolog | Secreted; Signal | 206267 |
| 901 | VTNC | Vitronectin | | 206268 |
| 902 | VWF | von Willebrand factor (vWF) | Secreted; Localized to storage granules | 206269 |
| 903 | VSIG2 | V-set and immunoglobulin domain-containing protein 2 | | 206270 |
| 904 | VSIG4 | V-set and immunoglobulin domain-containing protein 4 | | 206271 |
| 905 | VSTM1 | V-set and transmembrane domain-containing protein 1 | | 206272 |
| 906 | WISP2 | WNT1-inducible-signaling pathway protein 2 precursor (WISP-2), (Connective tissue growth factor-like protein) (CTGF-L) | Secreted | 206273 |
| 907 | S39A6 | Zinc transporter ZIP6 (Zrt- and Irt-like protein 6) (ZIP-6) | Cell membrane (Multi-pass membrane protein) | 206274 |
| 908 | ZA2G | Zinc-alpha-2-glycoprotein | | 206275 |
| 909 | ZP2 | Zona pellucida sperm-binding protein 2 (Zona pellucida, glycoprotein ZP2) | Cell membrane (Single-pass type I membrane protein) | 206276 |
| 910 | ZG16 | Zymogen granule membrane protein 16 | | 206277 |

### Cleavage sites

In some embodiments, the effector module comprises a cleavage and/or processing feature. Representative examples are given in Table 3.

The effector module of the present invention may include at least one protein cleavage signal/site. The protein cleavage signal/site may be located at the N-terminus, the C-terminus, at any space between the N- and the C- termini such as, but not limited to, half-way between the N- and C-termini, between the N-terminus and the half-way point, between the half-way point and the C-terminus, and combinations thereof.

The effector module may include one or more cleavage signal(s)/site(s) of any proteinases. The proteinases may be a serine proteinase, a cysteine proteinase, an endopeptidease, a dipeptidease, a metalloproteinase, a glutamic proteinase, a threonine proteinase and an aspartic proteinase.

As a non-limiting example, U.S. Pat. NO.: 7,374,930 and U.S. application publication NO.: 2009/227660, use a furin cleavage site to cleave the N-terminal methionine of GLP-1 in the expression product from the Golgi apparatus of the cells. In one aspect, the effector module may include a furin cleavage site or a modified furin cleavage site. In one embodiment, the effector module may include at least one protein cleavage signal and/or site with the proviso that the payload is not GLP-1.

**Table 3. Proteinase and Cleavage Sites**

| Proteinase No. | Proteinase | Type | Sequence or type of cleavage | SEQ ID NO |
|---|---|---|---|---|
| 1 | Actinidain | | X-X-X-R/K*R/K (not V)-X-X-X | 206278 |
| 2 | Calpain-1 | Cysteine proteinase | | - |
| 3 | Carboxypeptidase A | | P1*X-X (P1 = K, R) | 206279 |
| 4 | Carboxypeptidase P | | X-X-X-X-X-X*(X is not S); or X-X-X-X-X-X*(X is not G); or X-X-X-X-X-X*(X is not P) | 206280-206282 |
| 5 | Carboxypeptidase Y | | X-X-X-X-X-Xa*Xa-X-X-X (Xa = F, W, H, Y; X=anv amino acid) | 206283 |
| 6 | Caspase-1 | Cysteine proteinase | F/W/Y/L-X-H/A/T-D* X (not P/E/D/Q/K)-X-X-X | 206284 |
| 7 | Caspase-2 | Cysteine proteinase | D-V-A-D*X (not P/E/D/Q/K)-X-X-X | 206285 |
| 8 | Caspase-3 | Cysteine proteinase | D-M-Q-D*X (not P/E/D/Q/K)-X-X-X | 206286 |
| 9 | Caspase-4 | Cysteine proteinase | L-E-V-D*X (not P/E/D/Q/K)-X-X-X | 206287 |
| 10 | Caspase-5 | Cysteine proteinase | L/W-E-H-D*X-X-X-X | 206288 |
| 11 | Caspase-6 | Cysteine proteinase | V-E-H/I-D*X (not P/E/D/Q/K)-X-X-X | 206289 |
| 12 | Caspase-7 | Cysteine proteinase | D-E-V-D*X (not P/E/D/Q/K)-X-X-X | 206290 |
| 13 | Caspase-8 | Cysteine proteinase | X-X-I-Q-A-D*S-X-X-X; or X-X-Xa-E-X-D*Xs-X-X-X (Xs = S, G, A; Xa = V, L, I, A) | 206291, 206292 |
| 14 | Caspase-9 | Cysteine proteinase | L-E-H-D*X-X-X-X | 206293 |
| 15 | Caspase-10 | Cysteine proteinase | I-E-A-D*X-X-X-X | 206294 |
| 16 | Cathepsin B | Cysteine proteinase | X-X-R-R *X-X-X-X | 206295 |
| 17 | Cathepsin C (dipeptidyl-peptidase I) | Cysteine proteinase | X-X (N-terminal dipeptide) *X (not P) | - |
| 18 | Cathepsin G | Cysteine proteinase | X-X-X-X-X- (E or K or W or F) *X-X-X-X | 206296 |
| 19 | Cathepsin H | | X-X-X-X-R*X-X-X-X | 206297 |
| 20 | Cathepsin K | Cysteine proteinase | X-X-L/M/F (not R)-X*X-X-X-X | 206298 |
| 21 | Cathepsin L | Cysteine proteinase | X-X-R-R *X-X-X-X | 206299 |
| 22 | Cathepsin S | | X-V-V-R*X-X-X-X | 206300 |
| 23 | Cathepsin V | | X-F/LN-R*X | 206301 |
| 24 | Clostripain (Clostridiopeptidase B) | Cysteine proteinase | X-X-X-X-R* P1' (P1' not E, D)-X-X | 206302 |
| 25 | Chymase | | X-X-X-F/Y/W/L*X-X-X-X | 206303 |
| 26 | Chymotrypsin | | P1*P1'- (P1 = aromatic (W, Y and F), P1' = nonspecific) | - |
| 27 | Elastase (neutrophil) | | X-X-X-V/A *X-X-X-X | 206304 |
| 28 | Elastase (pancreatic) | | X-X-X-X-X-A*X-X-X-X | 206305 |
| 29 | Elastase (leukocyte) | | V/A*X-X | - |
| 30 | Endoproteinase Arg-C | | K*K and R*K | - |
| 31 | Endoproteinase Glu-C (V8 protease) | | X-X-X-E* X-X-X (X= any amino acid); X-X-X-D* X-X-X (X= anv amino acid) | 206306, 206307 |
| 32 | Endoproteinase Lys-C | | X-X-X-X-X-K*X-X-X-X (X= anv amino acid) | 206308 |
| 33 | Endoproteinase Asp-N | Metallo proteinase | X-X-X-P1*D- X-X-X (P1=cysteic acid) | 206309 |
| 34 | Enterokinase | Serine proteinase | D-D-D-D-K*X-X-X | 206310 |
| 35 | Factor Xa | | I/A-E/D-G-R*X-X-X | 206311 |
| 36 | Formic acid | | X-X-X-D*X-X-X | 206312 |
| 37 | Furin | | X-X-R/K-R*X (small, hydrophilic)-X (aliphatic)-X-X | 206313 |
| 38 | Glutamyl-endopeptidase | | D-AN-PN-D*X (not P/D)-X (not P) | 206314 |
| 39 | Granzyme B | | I-E-P-D*X-X | 206315 |
| 40 | HRV 3C Protease | | L-E-V-L-F-Q*G-P | 206316 |
| 41 | Hydroxylamine (NH2OH) | | X-X-X-N*G-X-X-X | 206317 |
| 42 | Intein Site | | dithioTeritol cleavage | - |
| 43 | lodosobenzoic acid | | X-X-X-X-W*X-X-X-X | 206318 |
| 44 | Leucyl aminopeptidase (peptidase S; cytosol aminopeptidase; cathepsin III) | | X-X-X-L/P (not R/K) *P-X-X-X | 206319 |
| 45 | LysC Lysyl endopeptidase (Achromobacter proteinase I) | | X-X-X-X-K*X-X-X-X | 206320 |
| 46 | LysN Peptidyl-Lys metalloendopeptidase | | X-X-X-X*K-X-X-X-X | 206321 |
| 47 | Matrix metallopeptidase-2 | Metallo proteinase | X -D(L/F/N/I)- I (or V)-P (or V/I-V (or A-S (or G/A/E) *L (or M/I/Y/F)- R (or Y/K/M/I/V)-S (or A/G)- X | 206322 |
| 48 | Matrix metallopeptidase-3 | Metallo proteinase | X -N (or I)- K (or V/I/R)-P (orV/I)- F(or Y/L/M/A)- S (or E) * M (or I/K/Y/F)- M (or K/I/R) -M (orA)- X | 206323 |
| 49 | oligopeptidase A | | X-G-P-G/A*G/A-P-A-X | 206324 |
| 50 | Papain | Cysteine proteinase | X-X-X-R/K*R/K (not V)-X-X-X | 206325 |
| 51 | Pepsin | Aspartic proteinase | X-X-X-X-X-hydrophobic*hydrophobic-X-X-X (hydrophobic AA=F, Y, W, L) | 206326 |
| 52 | peptidyl-dipeptidase A (peptidase P) | | (X)n (oligopeptide)*X (not P)-X (not D/E) | 206327 |
| 53 | Phytepsin | | X-X-X-FN/I/L/A*FN/I/L/A-X-X-X | 206328 |
| 54 | Plasmin | Serine proteinase | X-X-X-K/R*X-X-X | 206329 |
| 55 | PreScission | | L-E-V-L-F-Q*G-P | 206330 |
| 56 | Proline-endopeptidase | | X-X-K/H/R-P*X (not P)-X-X-X | 206331 |
| 57 | Proteinase K | | P1*P1'- (P1 = aromatic, hydrophobic preferred) | - |
| 58 | Pyroglutamate aminopeptidase (bovine) | Cysteine proteinase | P1*P1 (P1 = 5-oxoPline or pyroEtamate) | - |
| 59 | Subtilisin | | P1*P1'- (P1 = neutral/acidic preferred) | - |
| 60 | signal peptidase I | | Cleavage of hydrophobic, N-terminal signal or leader sequences | - |
| 61 | SUMO Protease | | Recognize the tertiary structure of the ubiquitin-like (UBL) Ptein, SUMO | - |
| 62 | TEV protease | | E-N-L-Y-F-Q*G | 206332 |
| 63 | Thermolvsin | Metallo proteinase | X-X-X-X-X-X*L/ F/I/L/V/M/A-X-X-X | 206333 |
| 64 | Thrombin | | L-V-P-R*G-S | 206334 |
| 65 | Trypsin | Serine proteinase | X-X-X-K/R*X-X | 206335 |
| 66 | TAGZyme | | H-tag remoV by ExoPteolytic Digestion | - |

### Tags

In some embodiments, the effector module comprises a protein tag. Representative examples are given in Table 4.

The protein tag may be used for detecting and monitoring the process of the effector module. The effector module

may include one or more tags such as an epitope tag (e.g., a FLAG or hemagglutinin (HA) tag). A large number of protein tags may be used for the present effector modules. They include, but are not limited to, self-labeling polypeptide tags (e.g., haloalkane dehalogenase (halotag2 or halotag7), ACP tag, clip tag, MCP tag, snap tag), epitope tags (e.g., FLAG, HA, His, and Myc), fluorescent tags (e.g., green fluorescent protein (GFP), red fluorescent protein (RFP), yellow fluorescent protein (YFP), and its variants), bioluminescent tags (e.g. luciferase and its variants), affinity tags (e.g., maltose-binding protein

(MBP) tag, glutathione-S-transferase (GST) tag), immunogenic affinity tags (e.g., protein A/G, IRS, AU1, AU5, glu-glu, KT3, S-tag, HSV, VSV-G, Xpress and V5), and other tags (e.g., biotin (small molecule), StrepTag (Strepll), SBP, biotin carboxyl carrier protein (BCCP), , eXact, CBP, CYD, HPC, CBD intein-chitin binding domain, Trx, NorpA, and NusA).

In other embodiments, a tag may also be selected from those disclosed in U.S. Pat. NOs.: 8,999,897; 8,357,511; 7,094, 568; 5,011,912; 4,851,341; and 4,703,004; U.S. patent application publication NOs.: 2013/115635 and 2013/012687; and PCT application publication NO.: WO2013/091661.

In some aspects, a multiplicity of protein tags, either the same or different tags, may be used; each of the tags may be located at the same N- or C-terminus, whereas in other cases these tags may be located at each terminus.

**Table 4. Protein Tags**

| Tag No. | Protein Tag Name | SEQ ID NO |
|---|---|---|
| 1 | AviTag | 206336 |
| 2 | BCCP (Biotin Carboxyl Carrier Protein) | 206337 |
| 3 | Calmodulin tag | 206338 |
| 4 | chitin binding protein (CBP) | 206339 |
| 5 | E-tag | 206340 |
| 6 | Fc-tag | 206341 |
| 7 | FLAG-tag | 206342 |
| 8 | GFP (from vector pHT3AG) | 206343 |
| 9 | GFP variant (CFP from Cloning vector pSEVA247C) | 206344 |
| 10 | GFP variant (RFP from Cloning vector pSAT6-DEST-RFP-N1) | 206345 |
| 11 | GFP variant (YFP from Cloning vector pSEVA227Y) | 206346 |
| 12 | Glutathione-S-transferase (GST) | 206347 |
| 13 | Halo-tag | 206348 |
| 14 | HAT tag | 206349 |
| 15 | HA-tag | 206350 |
| 16 | Isopep-tag | 206351 |
| 17 | maltose binding protein (MBP) | 206352 |
| 18 | Myc-tag | 206353 |
| 19 | poly (Histidine) (His-tag) | 206354 |
| 20 | polyarginine -tag (Arg-tag) | 206355 |
| 21 | polyglutamate tag | 206356 |
| 22 | SBP tag | 206357 |
| 23 | Snoop-tag | 206358 |
| 24 | Softag I | 206359 |
| 25 | Spy tag | 206360 |
| 26 | S-tag | 206361 |
| 27 | Strep tag II | 206362 |
| 28 | TC tag | 206363 |
| 29 | Thioredoxin (TRX) | 206364 |
| 30 | V5-tag | 206365 |
| 31 | VSV-tag | 206366 |
| 32 | Xpress tag | 206367 |

### Targeting or penetrating peptides

In some embodiments, the effector module comprises a targeting and/or penetrating peptide. Representative examples are given in Tables 9 and 10.

Small targeting and/or penetrating peptides that selectively recognize cell surface markers (e.g. receptors, trans-membrane proteins, and extra-cellular matrix molecules) can be employed to target the effector module to the desired organs, tissues or cells. Short peptides (5-50 amino acid residues) synthesized *in vitro* and naturally occurring peptides, or analogs, variants, derivatives thereof, may be incorporated into the effector module for homing the effector module to the desired organs, tissues and cells, and/or subcellular locations inside the cells.

In some embodiments, a targeting sequence and/or penetrating peptide may be included in the effector module to drive the effector module to a target organ, or a tissue, or a cell (e.g., a cancer cell; See Tables 9 and 10). As non-limiting examples, such targeting sequences and/or penetrating peptides may include those for targeting the effector module to desired region of the central nervous system (e.g., U.S. Pat. NO.: 9,259,432; U.S. application publication NO.: 2015/259392); or adipose tissue (e.g., U.S. Pat. NOs.: 8,067,377 and 8,710,017); or prostate (e.g., U.S. patent publication NO.: 2016/0046668).

In other embodiments, a targeting and/or penetrating peptide may direct the effector module to a specific subcellular location inside a cell. As a non-limiting example, a mitochondria targeting peptide and/or a mitochondria membrane penetrating peptide may be included in the effector module to drive the effector module to the mitochondria of a cell. See e.g., U.S. Pat. NOs.: 9,260,495; 9,173,952 and 9,132,198; and U.S. application publication NO.: 2015/361140.

Naturally occurring small targeting and/or penetrating peptides that recognize specific tissues or cells bind cell surface molecules (e.g. receptors, trans-membrane proteins) with high affinity, which make them attractive trafficking moieties. Such peptides may include peptide toxins from microbes, insects (e.g. scorpion, honey bee, spider), animals (e.g. snake) and plants, and analogs, variants and derivatives thereof; and secreted peptide hormones, ligands and signal peptides.

In some aspects, analogs, variants and derivatives from natural toxins that abolish their cytotoxic activities may be used as targeting peptides. Exotoxin is a toxin secreted by bacteria. Many exotoxins have been shown to bind specific cell molecules. For example, enterotoxins, a group of protein toxins produced and secreted from bacterial organisms bind the mucosal (epithelial) cells of the intestinal wall. Enterotoxins may include, but are not limited to, *E. coli* heat stable enterotoxin (ST), Cholera toxin (CT), *E. coli* heat-labile enterotoxin (LT), *Bordetella pertussis*-derived pertussis toxin (PT), *Pseudomonas aeruginosa* exotoxin A (ETA), *Staphylococcus* enterotoxins, *Corynebacterium diphtheria*-derived diphtheria toxin, enterotoxin NSP4 from rotavirus. Other exotoxins include neurotoxins which affect the nervous system, cardiotoxins which affect the heart, pseudomonas exotoxins, Botulinum neurotoxins, shiga toxin, shiga-like toxin 1 and 2, Clostridium difficile toxins, Clostridium perfringens epsiolon toxin and anthrax toxin.

In addition to exotoxins, other toxins may include those isolated from plants such as maize RIP, gelonin, pokeweed antiviral protein, saporin, trichsanthin, ricin, abrin; scorpions such as Charybdotoxin ; spider such as PcTx1; cone snail such as PcTx1; sea anemone such as gigantoxin 1; honey bees such as mellitins, a group of water-soluble, cationic, amphipathic 26 amino acid alpha-helical peptides isolated from the venoms of honey bee *Apis mellifera (western* or European or big honey bee), *Apis florea* (little or dwarf honey bee), *Apis dorsata* (giant honey bee) and *Apis cerana* (oriental honey bee); snake venom toxins, bombesin which is originally isolated from the skin of toad, which binds g-protein couple gastrin releasing peptide receptors (e.g. BBR-1/2/3) in the gastric tract and brain. See e.g. Suchanek, G., et al., PNAS (1978) 75:701-704.

Peptides hormones and other signal peptides transfer important messages for cell to cell communications, which selectively bind cells that express their receptors with high affinity. In some aspects, peptide hormones may be included in the effector module. Such small peptide hormones and signal peptides may include, but are not limited to, adiponectin, adipose-derived hormone, agouti signaling peptide, allatostatin, amylin, angiotensin, atrial natriuretic peptide, bomben-like peptide, big gastrin, betatrophin, bradykinin, calcitonin, corticotrophin releasing hormone, cosyntrophin, endothelin, enteroglucagon, FGF, FNDC5, follicle-stimulating hormone, gastrin, ghrelin, glucagon and glucagon-like peptide, gonadotrophin, granulocyte colony stimulating factor, growth hormone, growth hormone releasing hormone, hepcidin, human chorionic gonadotrophin, human placental lactogen, incretin, insulin and insulin analogs, insulin-like growth factor, leptin, little gastrin, liraglutide, luteinizing hormone, melanocortin, minigastrin, alpha-melanocyte-stimulating hormone, neuropeptide Y, nerve growth factor (NGF), neurotrophin-3/4, NPH insulin, orexin, obestatin, osteocalcin, pancreatic hormone, parathyroid hormone, peptide hormone, peptide YY, prolactin, preprohormone, relaxi, renin, salcatonin, somatostatin (SST), secretin, substance P, sincalide, teleost leptins, temporin, tesamorelin, thyroid stimulating hormone, urocortin, vasoactive intestinal peptide (VIP), VGF and Vitellogenin.

Targeting and penetrating peptides may also be engineered biomimetic peptides and/or chemically modified small peptides. Numerous peptides with specific motifs and sequences that target specific cells and tissues with high affinity and selectivity in normal or diseased conditions are identified. A synthetic targeting peptide may be up to 30 amino acids in length, or may be longer. A targeting peptide generally has at least about 5 amino acids but may have fewer, for example, 4 amino acids, or 3 amino acids. Generally, a targeting peptide has any number of amino acids from about 6 to about 30 inclusive. The peptide may have 6, 7, 8, 9, 10, 11, 12, 13, 14, 15, 16, 17, 18, 19, 20, 21, 22, 23, 24, 25, 26, 27, 28, 29 or 30 amino acids. Generally, a targeting peptide may have 25 or fewer amino acids, for example, 20 or fewer, for example 15 or fewer.

A chimeric peptide may also be synthesized with fused amino acids from naturally occurring proteins and artificial amino acid sequences.

In addition to penetrating peptides listed in Table 5 and Table 6, newly developed cell penetrating peptides discussed in U.S. Pat. NOs.: 9,206,231; 9,110,059; 8,706,219; and 8,772,449; and U.S. application publication NOs.: 2016/089447; 2016/060296; 2016/060314; 2016/060312; 2016/060311; 2016/009772; 2016/002613; 2015/314011 and 2015/166621; and PCT application publication NOs.: WO2015/179691 and 2015/183044 may also be included in the effector module.

**Table 5. Targeting and/or Penetrating Peptides**

| Targeting Peptide No. | Gene | Targeted receptor | SEQ ID | Targeted tissues/cells | Category |
|---|---|---|---|---|---|
| 1 | CD4 binding peptide | CD4 | 206368 | anti-HIV; CD4 positive cells | Non-tumor |
| 2 | - | CD4 | 206369 | Non-tumor | Non-tumor |
| 3 | - | CD4 | 206370 | Non-tumor | Non-tumor |
| 4 | CTxB | Cholera enterotoxin subunit B; binds to ganglioside receptor (GM1) | 206371 | vaccine delivery to enhance immuno response; mucosa; Pever's patch M cells | Non-tumor |
| 5 | mNSP4 131K; NSP4 114-135 | Derived from rotaviral enterotoxin NSP4 | 206372 | vaccine | Non-tumor |
| 6 | NSP4 120-147 | Derived from rotaviral enterotoxin NSP4 | 206373 | vaccine | Non-tumor |
| 7 | NSP4 2-22 | Derived from rotaviral enterotoxin NSP4 | 206374 | vaccine | Non-tumor |
| 8 | NSP4 90-123 | Derived from rotaviral enterotoxin NSP4 | 206375 | vaccine | Non-tumor |
| 9 | Tet-1 peptide | - | 206376 | neuron; Alzheimer's disease | Non-tumor |
| 10 | bFGF | binds to fibroblast growth factor (FGF) receptor (FGFR) | 206377 | FGFR positive cancer cells | Tumor |
| 11 | DEVDG | caspase 3 | 206378 | Tumor | Tumor |
| 12 | CD44BP | CD44 | 206379 | cancer stem cells | Tumor |
| 13 | collagen peptide | collagen derived peptide | 206380 | metastatic melanoma cells | Tumor |
| 14 | DV3 | CXC chemokine receptor 4 (CXCR4) | 206381 | Tumor | Tumor |
| 15 | Bn (2-7) | Derived from bombesin, binds to bombesin receptor such as BBR-1/2/3) | 206382 | Tumor cells; DU145 tumor xenograft | Tumor |
| 16 | Bn(6-14) | Derived from bombesin, binds to bombesin receptor such as BBR-1/2/3) | 206383 | Tumor cells; DU145 tumor xenograft | Tumor |
| 17 | EAST1 | derived from *E. coli* enterotoxin; binds to GC-C | 206384 | colon cancer, irritable bowel movement and chronic constipation | Tumor |
| 18 | STh (6-19) | derived from *E. coli* enterotoxin; binds to GC-C | 206385 | colon cancer | Tumor |
| 19 | STh (2-19) | derived from *E. coli* enterotoxin; binds to GC-C | 206386 | colon cancer; bind to epithelial cells | Tumor |
| 20 | STp | derived from *E. coli* enterotoxin; binds to GC-C | 206387 | colon cancer, irritable bowel movement and chronic constipation | Tumor |
| 21 | | derived from *E. coli* enterotoxin; binds to GC-C | 206388 | nonsmall cell lung cancer | Tumor |
| 22 | LHRH peptide | derived from Luteinizing Hormone-Releasing Hormone (LHRH); modified | 206389 | various cancer cells | Tumor |
| 23 | STh | Derived from V. cholerae enterotoxin; Derived from V. mimicus enterotoxin; binds to GC-C | 206390 | colon cancer, irritable bowel movement and chronic constipation | Tumor |
| 24 | ST | Derived from Y. enterocolitica enterotoxin; binds to GC-C | 206391 | colon cancer, irritable bowel movement and chronic constipation | Tumor |
| 25 | FHBP | Fibronectin | 206392 | cancer stem cells | Tumor |
| 26 | PC34 | from phage display biopanning | 206393 | breast cancer cells | Tumor |
| 27 | PC65 | from phage display biopanning | 206394 | breast cancer cells | Tumor |
| 28 | PC73 | from phage display biopanning | 206395 | breast cancer cells | Tumor |
| 29 | PC82 | from phage display biopanning | 206396 | breast cancer cells | Tumor |
| 30 | SP90 | from phage display biopanning | 206397 | breast cancer cells | Tumor |
| 31 | SP94 | from phage display biopanning | 206398 | hepatocellular carcinoma cells | Tumor |
| 32 | FROP-1 | FROP-1 | 206399 | follicular thyroid carcinoma and other carcinoma | Tumor |
| 33 | cancer recognition peptide | Her-2 | 206400 | target to tumor HER-2 antigen; breast cancer, prostate cancer | Tumor |
| 34 | integrin (IBP) | Integrin | 206401 | cancer stem cells | Tumor |
| 35 | - | neuropilin-1 | 206402 | glioblastoma and endothelial cells | Tumor |
| 36 | VIP | Vasoactive intestinal peptide | 206403 | breast cancer | Tumor |
| 37 | ACPP-MMP-2 | - | 206404 | Proteases in breast cancer cells | Tumor |
| 38 | ACPP-MMP-2/9 | - | 206405 | Proteases in human fibrosarcoma | Tumor |
| 39 | alpha-melanocyte stimulating hormone peptide | - | | melanomas | Tumor |
| 40 | Antagonist G peptide | - | 206406 | small cell lung cancer | Tumor |
| 41 | CP15 | - | 206407 | colon cancer | Tumor |
| 42 | IRQ | - | 206408 | parenchymal cells | Tumor |
| 43 | L-peptide | - | 206409 | nasopharyngeal carcinoma cells | Tumor |
| 44 | p160 | - | 206410 | breast cancer | Tumor |
| 45 | RPMrel | - | 206411 | colon cancer tissue | Tumor |
| 46 | RPMrel | - | 206412 | colon cancer tissue | Tumor |
| 47 | VTW | - | 206413 | glioblastoma cells; brain tumor | Tumor |
| 48 | - | - | 206414 | fibrosarcomas | Tumor |
| 49 | - | Aminopeptidase A | 206415 | vasculature; breast tumor | Vasculature |
| 50 | IF7 | Anxa1 | 206416 | Tumor blood vessels; Melanoma and colorectal cancer | Vasculature |
| 51 | F3 peptide | bind to nucleolin | 206417 | Angiogenic blood vessels (endothelial cells) and tumor cells; leukemia tumor | Vasculature |
| 52 | CLT1 | CLC1 and fibronectin | 206418 | tumor blood vessels | Vasculature |
| 53 | CREKA | from phage display | 206419 | the blood vessels and stroma of tumor | Vasculature |
| 54 | - | MMP-2; MMP-9 | 206420 | Tumor blood vessels; Breast carcinoma | Vasculature |
| 55 | - | NG2 | 206421 | NG2-positive pericytes | Vasculature |
| 56 | - | NG2 | 206422 | NG2-positive pericytes | Vasculature |
| 57 | LyP-1 | P32/gC1qR | 206423 | Tumor cells, lymphatic endothelium | Vasculature |
| 58 | RGR | PDGFR-beta | 206424 | "Pericytes and endothelial cells; Pancreatic tumors and | |
| angiogenic islets | | | | | |
| " | Vasculature | | | | |
| 59 | AGR | - | 206425 | Lymphatic vessels | Vasculature |
| 60 | CLT2 | - | 206426 | tumor blood vessels | Vasculature |
| 61 | GX1 | - | 206427 | vasculature; gastric cancer | Vasculature |
| 62 | K237 | - | 206428 | K237 ligand, a peptide that can bind to the KDR receptors predominantly expressed on the surface of tumor neovasculature endothelial cells with high affinity and specificity and inhibit the VEGF-KDR angiogenic signal pathway, was conjugated to the aldehyde group of PEG chain using the N-terminal PEGylation technique | Vasculature |
| 63 | KAA | - | 206429 | vasculature; pancreatic tumors; Pericytes and endothelial cells | Vasculature |
| 64 | KAR | - | 206430 | vasculature; pancreatic tumors | Vasculature |
| 65 | KRK | - | 206431 | Angiogenic blood: vessels and tumor cells; Tumor blood vessels; skin carcinoma | Vasculature |
| 66 | LSD | - | 206432 | Lymphatic vessels | Vasculature |
| 67 | LyP-2 | - | 206433 | Lmphatic vessels | Vasculature |
| 68 | PEGA | - | 206434 | tumor blood vessels | Vasculature |
| 69 | REA | - | 206435 | Lymphatic vessels | Vasculature |
| 70 | RMS-II | - | 206436 | tumor blood vessels | Vasculature |
| 71 | RSR | - | 206437 | Pericytes and endothelial cells | Vasculature |
| 72 | SP5-52 | - | 206438 | vasculature; various cancer | Vasculature |
| 73 | TCP-1 | - | 206439 | tumor blood vessels; Orthotopic colorectal cancer and gastric cancer | Vasculature |
| 74 | - | - | 206440 | Blood vessels; tumor blood vessels; dysplastic skin | Vasculature |

**Table 6. Penetrating Peptides**

| Peptide No. | Name | SEQ ID | Peptide features | Source |
|---|---|---|---|---|
| 1 | TAT | 206444 | Trans-Activator of Transcription protein from HIV-1; cell penetrating peptide | Torchilin, Eur. J. Pharm. Biopharm., 2009, 71, 431-444 |
| 2 | Pep-1 | 206445 | designed; synthetic peptide; for penetrating cell membrane | Delehanty J et al., Therapeutic delivery, 2010, 1, 411-433 |
| 3 | RDP | 206446 | derived from rabies virus glycoprotein; neuron; brain-blood barrier; Parkinson's disease | Fu A et al., Pharm Res 2012, 29, 1562-1569 |
| 4 | RVG29 | 206447 | rabies viral glycoprotein; brain blood barrier; binds to acetylcholine receptor (AchR) | Delehanty J et al., Therapeutic delivery, 2010, 1, 411-433 |
| 5 | Ast 1 | 206448 | Insect neuropeptide allatostatin 1; cell penetrating | Delehanty J et al., Therapeutic delivery, 2010, 1, 411-433 |
| 6 | Penetratin | 206449 | protein derived; from the antennapedia transcription factor of Drosophila melanogaster; cell penetrating peptide | Regberg J et al, pharmaceuticals, 2012, 5, 991-1007 |
| 7 | pVEC | 206450 | protein derived; cell penetrating peptide | |
| 8 | MPG8 | 206451 | Chimeric peptide; cell penetrating peptide | |
| 9 | Transportan | 206452 | Chimeric peptide; cell penetrating peptide | |
| 10 | Transportan10 | 206453 | Chimeric, modified; cell penetrating peptide | |
| 11 | PepFect3 | 206454 | Chimeric, modified; cell penetrating peptide | |
| 12 | PepFect 6 | 206455 | Chimeric, modified; cell penetrating peptide | |
| 13 | PepFect 14 | 206456 | Chimeric, modified; cell penetrating peptide | |
| 14 | Polyarginine | 206457 | designed, synthetic peptide; cell penetrating | |
| 15 | Stearylpolyargi nine | 206458 | designed, synthetic peptide; cell penetrating | |
| 16 | Pep-3 | 206459 | designed, synthetic peptide; cell penetrating | |
| 17 | CADY | 206460 | designed, synthetic peptide; cell penetrating | |
| 18 | YTA2 | 206461 | designed, synthetic peptide; cell penetrating | |
| 19 | YTA4 | 206462 | designed, synthetic peptide; cell penetrating | |
| 20 | SynB1 | 206463 | protein derived, cell penetrating peptide | |
| 21 | SynB3 | 206464 | protein derived, cell penetrating peptide | |
| 22 | Maurocalcine | 206465 | protein derived, cell penetrating peptide | |
| 23 | PTD4 | 206466 | protein derived, cell penetrating peptide | |
| 24 | Angiopep-2 | 206467 | brain, penetrating the blood-brain barrier (BBB) | Zou L et al., Current Neuropharmacology, 2013, 11, 197-208 |
| 25 | Angiopep-5 | 206468 | brain, penetrating the blood-brain barrier (BBB) | |
| 26 | TAT (47-57) | 206469 | brain, penetrating the blood-brain barrier (BBB) | |
| 27 | (RXRRBR)2XB | 206470 | brain, penetrating the blood-brain barrier (BBB) | |
| 28 | SvnB 5 | 206471 | brain, BBB | |
| 29 | FGF4 | 206472 | FGF4-SOCS3 protected mice from lethal effects of staphylococcal enterotoxin B and lipopolysaccharide by reducing production of inflammatory cytokines and hemorrhagic necrosis brain | |
| 30 | TAT-10H | 206473 | brain, penetrating the blood-brain barrier (BBB) | |
| 31 | RVG-9R | 206474 | brain, BBB | |
| 32 | TAT-HA | 206475 | brain, BBB | |
| 33 | HC-[poly(K)] | 206476 | derived from tetanus toxin; Induce increased brain cell penetration | |
| 34 | CM18-TAT | 206477 | residues 1-7 of Cecropin-A, 2-12 of Melittin, and 47-57 of HIV-1 Tat protein | |
| 35 | TH-Lip | 206478 | an engineered α-helical cell penetrating peptide originated from peptide TK (AGYLLGKINLKKLAKL(Aib)LLIL-NH; tumor cell penetrating | |
| 36 | BR2 | 206479 | synthesized peptide; tumor cell penetrating | Lim et al., Plos One, 2013, 8, e66084 |
| 37 | Xentry | 206480 | derived from an N-terminal region of the X-protein of the hepatitis B virus | Montrose K et al., Sci Rep 2013, 3, 1661 |
| 38 | | 206481 | derived from the heparin-binding motif of human eosinophil cationic protein (ECP). | Fang et al., Plos One, 2013, 8, e57318 |
| 39 | | 206482 | ECP, cell penetrating peptide | |
| 40 | | 206483 | ECP, cell penetrating peptide | |
| 41 | | 206484 | ECP, cell penetrating peptide | |
| 42 | | 206485 | ECP, cell penetrating peptide | |
| 43 | | 206486 | ECP, cell penetrating peptide | |
| 44 | | 206487 | ECP, cell penetrating peptide | |
| 45 | | 206488 | ECP, cell penetrating peptide | |
| 46 | | 206489 | ECP, cell penetrating peptide | |
| 47 | KLA | 206490 | cell penetrating peptide | |
| 48 | | 206491 | integrin; targeting dendritic cells | US7820624 |
| 49 | | 206492 | integrin; targeting dendritic cells | US7820624 |
| 50 | | 206493 | integrin; targeting dendritic cells | US7820624 |
| 51 | | 206494 | integrin; targeting dendritic cells | US7820624 |
| 52 | | 206495 | | US7820624; SEQ ID NO 5 |
| 53 | | 206496 | Dendritic cells | US7820624; SEQ ID NO 6 |
| 54 | | 206497 | | US7820624; SEQ ID NO 7 |
| 55 | | 206498 | Dendritic cells | US7820624; SEQ ID NO 8 |
| 56 | | 206499 | Dendritic cells | US7820624; SEQ ID NO 9 |
| 57 | | 206500 | Dendritic cells | US7820624; SEQ ID NO 10 |
| 58 | | 206501 | Dendritic cells | US7820624; SEQ ID NO 11 |
| 59 | | 206502 | Dendritic cells | US7820624; SEQ ID NO 12 |
| 60 | | 206503 | Dendritic cells | US7820624; SEQ ID NO 13 |
| 61 | | 206504 | Dendritic cells | US7820624; SEQ ID NO 14 |
| 62 | | 206505 | Dendritic cells | US7820624; SEQ ID NO 15 |
| 63 | | 206506 | Dendritic cells | US7820624; SEQ ID NO 16 |
| 64 | | 206507 | Dendritic cells | US7820624; SEQ ID NO 17 |
| 65 | | 206508 | Dendritic cells | US7820624; SEQ ID NO 18 |
| 66 | | 206509 | Dendritic cells | US7820624; SEQ ID NO 19 |
| 67 | | 206510 | Dendritic cells | US7820624; SEQ ID NO20 |
| 68 | | 206511 | Dendritic cells | US7820624; SEQ ID NO21 |
| 69 | | 206512 | Dendritic cells | US7820624; SEQ ID NO 22 |
| 70 | | 206513 | Dendritic cells | US7820624; SEQ ID NO23 |
| 71 | | 206514 | Dendritic cells | US7820624; SEQ ID NO 24 |
| 72 | | 206515 | Dendritic cells | US7820624; SEQ ID NO 25 |
| 73 | | 206516 | Dendritic cells | US7820624; SEQ ID NO 26 |
| 74 | | 206517 | Dendritic cells | US7820624; SEQ ID NO27 |
| 75 | | 206518 | Dendritic cells | US7820624; SEQ ID NO28 |
| 76 | | 206519 | Dendritic cells | US7820624; SEQ ID NO 29 |
| 77 | | 206520 | Dendritic cells | US7820624; SEQ ID NO 30 |
| 78 | | 206521 | Dendritic cells | US7820624; SEQ ID NO 31 |
| 79 | | 206522 | Dendritic cells | US7820624; SEQ ID NO 32 |
| 80 | | 206523 | Dendritic cells | US7820624; SEQ ID NO 33 |
| 81 | | 206524 | Dendritic cells | US7820624; SEQ ID NO 34 |
| 82 | | 206525 | Dendritic cells | US7820624; SEQ ID NO 35 |
| 83 | | 206526 | Dendritic cells | US7820624; SEQ ID NO 36 |
| 84 | | 206527 | Dendritic cells | US7820624; SEQ ID NO 37 |
| 85 | | 206528 | Dendritic cells | US7820624; SEQ ID NO 38 |
| 86 | | 206529 | Dendritic cells | US7820624; SEQ ID NO 39 |
| 87 | | 206530 | Dendritic cells | US7820624; SEQ ID NO 40 |
| 88 | | 206531 | Dendritic cells | US7820624; SEQ ID NO 41 |
| 89 | | 206532 | Dendritic cells | US7820624; SEQ ID NO 42 |
| 90 | | 206533 | Dendritic cells | US7820624; SEQ ID NO 43 |
| 91 | | 206534 | Dendritic cells | US7820624; SEQ ID NO 44 |
| 92 | | 206535 | Dendritic cells | US7820624; SEQ ID NO 45 |
| 93 | | 206536 | Dendritic cells | US7820624; SEQ ID NO 47 |
| 94 | | 206537 | Dendritic cells | US7820624; SEQ ID NO 48 |
| 95 | | 206538 | Dendritic cells | US7820624; SEQ ID NO 51 |
| 96 | | 206539 | Dendritic cells | US7820624; SEQ ID NO 52 |
| 97 | | 206540 | Honey bee toxin (mellitin, 26AAs); target to integrin, enhance nuclear transport; membrane lytic peptides | Ogris, M., et al., J. Biol. Chem. (2001) 276:47550-47555 and Boeckle, S., et al., J. Control Release (2006) 112:240-248). |
| 98 | | 206541 | from apis florea; mellitin peptides 26AAs | US7943168; US8496945; US20130281658; SEQ ID NO 1 |
| 99 | | 206542 | mellitin derivative | US20130281658;SEQ ID NO 2 |
| 100 | | 206543 | mellitin derivative | US20130281658; SEQ ID NO 3 |
| 101 | | 206544 | mellitin derivative | US20130281658; SEQ ID NO 4 |
| 102 | | 206545 | mellitin derivative | US20130281658; SEQ ID NO 5 |
| 103 | | 206546 | mellitin derivative | US20130281658; SEQ ID NO 6 |
| 104 | | 206547 | leu-mellitin; apis florea | US20130281658; SEQ ID NO 7 |
| 105 | | 206548 | mellitin derivative | US20130281658; SEQ ID NO 8 |
| 106 | | 206549 | mellitin derivative | US20130281658; SEQ ID NO 9 |
| 107 | | 206550 | mellitin derivative | US20130281658; SEQ ID NO 10 |
| 108 | | 206551 | mellitin derivative | US20130281658; SEQ ID NO 11 |
| 109 | | 206552 | mellitin derivative | US20130281658; SEQ ID NO 12 |
| 110 | | 206553 | mellitin derivative | US20130281658; SEQ ID NO 13 |
| 111 | | 206554 | mellitin derivative | US20130281658; SEQ ID NO 14 |
| 112 | | 206555 | mellitin derivative | US20130281658; SEQ ID NO 15 |
| 113 | | 206556 | mellitin derivative | US20130281658; SEQ ID NO 16 |
| 114 | | 206557 | mellitin derivative | US20130281658; SEQ ID NO 17 |
| 115 | | 206558 | mellitin derivative | US20130281658; SEQ ID NO 18 |
| 116 | | 206559 | mellitin derivative | US20130281658; SEQ ID NO 19 |
| 117 | | 206560 | mellitin derivative | US20130281658; SEQ ID NO 20 |
| 118 | | 206561 | mellitin derivative | US20130281658; SEQ ID NO 21 |
| 119 | | 206562 | mellitin derivative | US20130281658; SEQ ID NO 76 |
| 120 | | 206563 | mellitin derivative | US20130281658; SEQ ID NO 77 |
| 121 | | 206564 | mellitin derivative | US20130281658; SEQ ID NO 78 |
| 122 | | 206565 | mellitin derivative | US20130281658; SEQ ID NO 79 |
| 123 | | 206566 | mellitin derivative | US20130281658; SEQ ID NO 80 |
| 124 | | 206567 | mellitin derivative | US20130281658; SEQ ID NO 81 |
| 125 | | 206568 | mellitin derivative | US20130281658; SEQ ID NO 82 |
| 126 | | 206569 | mellitin derivative | US20130281658; SEQ ID NO 83 |
| 127 | | 206570 | mellitin derivative | US20130281658; SEQ ID NO 84 |
| 128 | | 206571 | mellitin derivative | US20130281658; SEQ ID NO 85 |
| 129 | | 206572 | mellitin derivative | US20130281658; SEQ ID NO 86 |
| 130 | | 206573 | mellitin derivative | US20130281658; SEQ ID NO 87 |
| 131 | | 206574 | mellitin derivative | US20130281658; SEQ ID NO 88 |
| 132 | | 206575 | mellitin derivative | US20130281658; SEQ ID NO 89 |
| 133 | | 206576 | | US20130281658; SEQ ID NO 90 |
| 134 | | 206577 | reversed mellitin | US20130281658; SEQ ID NO 92 |
| 135 | | 206578 | reversed mellitin | US20130281658; SEQ ID NO 93 |
| 136 | | 206579 | mellitin derivative | US20130281658; SEQ ID NO 94 |
| 137 | | 206580 | normal melittin amino acid sequence is reversed and all amino acids are D-form amino acids (Glycine (G) is achiral) | US20130281658; SEQ ID NO 95 |
| 138 | | 206581 | mellitin derivative | US20130281658; SEQ ID NO 96 |
| 139 | | 206582 | cell penetrating peptides | SEQ ID NO 1 (US8242081; US7943581; US7579318) |
| 140 | | 206583 | cell penetrating peptides | SEQ ID NO 2 (US8242081; US7943581; US7579318) |
| 141 | | 206584 | cell penetrating peptides | SEQ ID NO 3 (US8242081; US7943581; US7579318) |
| 142 | | 206585 | cell penetrating peptides | SEQ ID NO 4 (US8242081; US7943581; US7579318) |
| 143 | | 206586 | cell penetrating peptides | SEQ ID NO 5 (US8242081; US7943581; US7579318) |
| 144 | | 206587 | cell penetrating peptides | SEQ ID NO 6 (US8242081; US7943581; US7579318) |
| 145 | | 206588 | cell penetrating peptides | SEQ ID NO 7 (US8242081; US7943581; US7579318) |
| 146 | | 206589 | cell penetrating peptides | SEQ ID NO 8 (US8242081; US7943581; US7579318) |
| 147 | | 206590 | cell penetrating peptides | SEQ ID NO 9 (US8242081; US7943581; US7579318) |
| 148 | | 206591 | cell penetrating peptides | SEQ ID NO 10 (US8242081; US7943581; US7579318) |
| 149 | | 206592 | cell penetrating peptides | SEQ ID NO 11 (US8242081; US7943581; US7579318) |
| 150 | | 206593 | cell penetrating peptides | SEQ ID NO 12 (US8242081; US7943581; US7579318) |
| 151 | | 206594 | cell penetrating peptides | SEQ ID NO 13 (US8242081; US7943581; US7579318) |
| 152 | | 206595 | penetrating peptide for coating nanoparticle, but not lipid based nanoparticle | US7943396; US8383423; SEQ ID NO 1 |
| 153 | | 206596 | penetrating peptide for coating nanoparticle, but not lipid based nanoparticle | US7943396; US8383423; SEQ ID NO 2 |
| 154 | | 206597 | penetrating peptide for coating nanoparticle, but not lipid based nanoparticle | US7943396; US8383423; SEQ ID NO 3 |
| 155 | | 206598 | penetrating peptide for coating nanoparticle, but not lipid based nanoparticle | US7943396; US8383423; SEQ ID NO 4 |
| 156 | | 206599 | penetrating peptide for coating nanoparticle, but not lipid based nanoparticle | US7943396; US8383423; SEQ ID NO 5 |
| 157 | | 206600 | CAP peptide; clustering; cancer | US8263133; SEQ ID NO 1 |
| 158 | | 206601 | CAP peptide; clustering; cancer | US8263133; SEQ ID NO 2 |
| 159 | | 206602 | CAP-RGD-ASA peptide; | US8263133; SEQ ID NO 3 |
| 160 | | 206603 | CAP-ASA peptide; | US8263133; SEQ ID NO 4 |
| 161 | | 206604 | CAP-RGD peptide; | US8263133; SEQ ID NO 5 |
| 162 | | 206605 | brain and heart | US8506928; SEQ ID NO 1 |
| 163 | | 206606 | brain and heart | US8506928; SEQ ID NO 2 |
| 164 | | 206607 | brain and heart | US8506928; SEQ ID NO 3 |
| 165 | | 206608 | brain and heart | US8506928; SEQ ID No 4 |
| 166 | | 206609 | brain and heart | US8506928; SEQ ID NO 5 |
| 167 | | 206610 | brain and heart | US8506928; SEQ ID NO 6 |
| 168 | | 206611 | brain and heart | US8506928; SEQ ID NO 7 |
| 169 | | 206612 | heart vasculature; cardiovascular diseases | US7501486; US20060160743; SEQ ID NO 1 |
| 170 | | 206613 | heart vasculature; cardiovascular diseases | US7501486; US20060160743; SEQ ID NO 2 |
| 171 | | 206614 | heart vasculature; cardiovascular diseases | US7501486; US20060160743; SEQ ID NO 3 |
| 172 | | 206615 | heart vasculature; cardiovascular diseases | US7501486; US20060160743; SEQ ID NO 4 |
| 173 | | 206616 | heart vasculature; cardiovascular diseases | US7501486; US20060160743; SEQ ID NO 5 |
| 174 | | 206617 | heart vasculature; cardiovascular diseases | US7501486; US20060160743; SEQ ID NO 6 |
| 175 | | 206618 | heart vasculature; cardiovascular diseases | US7501486; US20060160743; SEQ ID NO 7 |
| 176 | | 206619 | heart vasculature; cardiovascular diseases | US7501486; US20060160743; SEQ ID NO 8 |
| 177 | | 206620 | heart vasculature; cardiovascular diseases | US7501486; US20060160743; SEQ ID NO 9 |
| 178 | | 206621 | heart vasculature; cardiovascular diseases | US7501486; US20060160743; SEQ ID NO 10 |
| 179 | | 206622 | heart vasculature; cardiovascular diseases | US7501486; US20060160743; SEQ ID NO 11 |
| 180 | | 206623 | heart vasculature; cardiovascular diseases | US7501486; US20060160743; SEQ ID NO 12 |
| 181 | | 206624 | heart vasculature; cardiovascular diseases | US7501486; US20060160743; SEQ ID NO 13 |
| 182 | | 206625 | heart vasculature; cardiovascular diseases | US7501486; US20060160743; SEQ ID NO 14 |
| 183 | | 206626 | heart vasculature; cardiovascular diseases | US7501486; US20060160743; SEQ ID NO 15 |
| 184 | | 206627 | heart vasculature; cardiovascular diseases | US7501486; US20060160743; SEQ ID NO 16 |
| 185 | | 206628 | heart vasculature; cardiovascular diseases | US7501486; US20060160743; SEQ ID NO 17 |
| 186 | | 206629 | heart vasculature; cardiovascular diseases | US7501486; US20060160743; SEQ ID NO 18 |
| 187 | | 206630 | heart vasculature; cardiovascular diseases | US7501486; US20060160743; SEQ ID NO 19 |
| 188 | | 206631 | heart vasculature; cardiovascular diseases | US7501486; US20060160743; SEQ ID NO 20 |
| 189 | | 206632 | heart vasculature; cardiovascular diseases | US7501486; US20060160743; SEQ ID NO 21 |
| 190 | | 206633 | heart vasculature; cardiovascular diseases | US7501486; US20060160743; SEQ ID NO 22 |
| 191 | | 206634 | heart vasculature; cardiovascular diseases | US7501486; US20060160743; SEQ ID NO 23 |
| 192 | | 206635 | U87R cell binding peptide; | US6303573; SEQ ID NO 2 |
| 193 | | 206636 | U87R cell binding peptide; | US6303573; SEQ ID NO 3 |
| 194 | | 206637 | U87R cell binding peptide; | US6303573; SEQ ID NO 4 |
| 195 | | 206638 | U87R cell binding peptide; | US6303573; SEQ ID NO 9 |
| 196 | | 206639 | U87R cell binding peptide; | US6303573; SEQ ID NO 10 |
| 197 | | 206640 | U87R cell binding peptide; | US8530429; SEQ ID NO 1 |
| 198 | | 206641 | U87R cells binding peptide; | US8530429; SEQ ID NO 2 |
| 199 | | 206642 | U87R cells binding peptide; | US8530429; SEQ ID NO 3 |
| 200 | | 206643 | U87R cells binding peptide; | US8530429; SEQ ID NO 4 |
| 201 | | 206644 | U87R cells binding peptide; | US8530429; SEQ ID NO 5 |
| 202 | | 206645 | U87R cells binding peptide; | US8530429; SEQ ID NO 6 |
| 203 | | 206646 | U87R cells binding peptide; | US8530429; SEQ ID NO 7 |
| 204 | | 206647 | U87R cells binding peptide; | US8530429; SEQ ID NO 8 |
| 205 | | 206648 | U87R cells binding peptide; | US8530429; SEQ ID NO 9 |
| 206 | | 206649 | U87R cells binding peptide; | US8530429; SEQ ID NO 10 |
| 207 | | 206650 | BTIC-binding peptide; | US8530429; SEQ ID NO 11 |
| 208 | | 206651 | BTIC-binding peptide; | US8530429; SEQ ID NO 12 |
| 209 | | 206652 | BTIC-binding peptide; | US8530429; SEQ ID NO 13 |
| 210 | | 206653 | BTIC-binding peptide; | US8530429; SEQ ID NO 14 |
| 211 | | 206654 | BTIC-binding peptide; | US8530429; SEQ ID NO 15 |
| 212 | | 206655 | BTIC-binding peptide; | US8530429; SEQ ID NO 16 |
| 213 | | 206656 | skin penetrating and cell entering peptides; in particular penetrating stratum corneum layer and cell membrane. | US8518871; SEQ ID NO 1 |
| 214 | | 206657 | skin penetrating and cell entering peptides; in particular penetrating stratum corneum layer and cell membrane. | US8518871; SEQ ID NO 2 |
| 215 | | 206658 | skin penetrating and cell entering peptides; in particular penetrating stratum corneum layer and cell membrane. | US8518871; SEQ ID NO 3 |
| 216 | | 206659 | skin penetrating and cell entering peptides; in particular penetrating stratum corneum layer and cell membrane. | US8518871; SEQ ID NO 4 |
| 217 | | 206660 | skin penetrating and cell entering peptides; in particular penetrating stratum corneum layer and cell membrane. | US8518871; SEQ ID NO 5 |
| 218 | | 206661 | skin penetrating and cell entering peptides; in particular penetrating stratum corneum layer and cell membrane. | US8518871; SEQ ID NO 6 |
| 219 | | 206662 | skin penetrating and cell entering peptides; in particular penetrating stratum corneum layer and cell membrane. | US8518871; SEQ ID NO 7 |
| 220 | | 206663 | skin penetrating and cell entering peptides; in particular penetrating stratum corneum layer and cell membrane. | US8518871; SEQ ID NO 8 |
| 221 | | 206664 | skin penetrating and cell entering peptides; in particular penetrating stratum corneum layer and cell membrane. | US8518871; SEQ ID NO 9 |
| 222 | | 206665 | skin penetrating and cell entering peptides; in particular penetrating stratum corneum layer and cell membrane. | US8518871; SEQ ID NO 10 |
| 223 | | 206666 | skin penetrating and cell entering peptides; in particular penetrating stratum corneum layer and cell membrane. | US8518871; SEQ ID NO 11 |
| 224 | | 206667 | skin penetrating and cell entering peptides; in particular penetrating stratum corneum layer and cell membrane. | US8518871; SEQ ID NO 12 |
| 225 | | 206668 | skin penetrating and cell entering peptides; in particular penetrating stratum corneum layer and cell membrane. | US8518871; SEQ ID NO 13 |
| 226 | | 206669 | skin penetrating and cell entering peptides; in particular penetrating stratum corneum layer and cell membrane. | US8518871; SEQ ID NO 14 |
| 227 | | 206670 | skin penetrating and cell entering peptides; in particular penetrating stratum corneum layer and cell membrane. | US8518871; SEQ ID NO 15 |
| 228 | | 206671 | skin penetrating and cell entering peptides; in particular penetrating stratum corneum layer and cell membrane. | US8518871; SEQ ID NO 16 |
| 229 | | 206672 | skin penetrating and cell entering peptides; in particular penetrating stratum corneum layer and cell membrane. | US8518871; SEQ ID NO 17 |
| 230 | | 206673 | skin penetrating and cell entering peptides; in particular penetrating stratum corneum layer and cell membrane. | US8518871; SEQ ID NO 18 |
| 231 | | 206674 | F3 peptide; penetrating tumor blood and tumor cells | WO2003087124; SEQ ID NO. 9 |
| 232 | | 206675 | APA-binding peptide | SEQ ID NO 125 (WO2002020769) |
| 233 | | 206676 | APA-binding peptide | SEQ ID NO 126 (WO2002020769) |
| 234 | | 206677 | APA-binding peptide | SEQ ID NO 127(WO2002020769) |
| 235 | | 206678 | APA-binding peptide | SEQ ID NO 128(WO2002020769) |
| 236 | | 206679 | APA-binding peptide | SEQ ID NO 129(WO2002020769) |
| 237 | | 206680 | APA-binding peptide | SEQ ID NO 130(WO2002020769) |
| 238 | | 206681 | APA-binding peptide | SEQ ID NO 131(WO2002020769) |
| 239 | | 206682 | APA-binding peptide | SEQ ID NO 138(WO2002020769) |
| 240 | | 206683 | pancreatic targeting peptide | SEQ ID NO 139 (WO2002020769) |
| 241 | | 206684 | pancreatic targeting peptide | SEQ ID NO 140(WO2002020769) |
| 242 | | 206685 | APA-binding peptide | SEQ ID NO 141(WO2002020769) |
| 243 | | 206686 | APA-binding peptide | SEQ ID NO 142 (WO2002020769) |
| 244 | Buforin II | 206687 | cell penetrating peptide | SEQ ID NO 1(US20090186802) |
| 245 | DPV3 | 206688 | cell penetrating peptide | SEQ ID NO 2(US20090186802) |
| 246 | DPV6 | 206689 | cell penetrating peptide | SEQ ID NO 3(US20090186802) |
| 247 | DPV7 | 206690 | cell penetrating peptide | SEQ ID NO 4(US20090186802) |
| 248 | DPV7b | 206691 | cell penetrating peptide | SEQ ID NO 5(US20090186802) |
| 249 | DPV3/10 | 206692 | cell penetrating peptide | SEQ ID NO 6(US20090186802) |
| 250 | DPV10/6 | 206693 | cell penetrating peptide | SEQ ID NO 7(US20090186802) |
| 251 | DPV1047 | 206694 | cell penetrating peptide | SEQ ID NO 8(US20090186802) |
| 252 | DPV1048 | 206695 | cell penetrating peptide | SEQ ID NO 9 (US20090186802) |
| 253 | DPV10 | 206696 | cell penetrating peptide | SEQ ID NO 10 (US20090186802) |
| 254 | DPV15 | 206697 | cell penetrating peptide | SEQ ID NO 11(US20090186802) |
| 255 | DPV15b | 206698 | cell penetrating peptide | SEQ ID NO 12 (US20090186802) |
| 256 | GALA | 206699 | cell penetrating peptide | SEQ ID NO 13 (US20090186802) |
| 257 | Cβ | 206700 | Haptotactic peptide | SEQ ID NO 14 (US20090186802) |
| 258 | preCγ | 206701 | Haptotactic peptide | SEQ ID NO 15 (US20090186802) |
| 259 | CaE | 206702 | Haptotactic peptide | SEQ ID NO 16 (US20090186802) |
| 260 | hCT(9-32) | 206703 | Haptotactic peptide | SEQ ID NO 17 (US20090186802) |
| 261 | HN-1 | 206704 | Haptotactic peptide | SEQ ID NO 18 (US20090186802) |
| 262 | Influenza virus nucleoprotein (NLS) | 206705 | Haptotactic peptide | SEQ ID NO 19 (US20090186802) |
| 263 | KALA | 206706 | Haptotactic peptide | SEQ ID NO 20 (US20090186802) |
| 264 | K-FGF | 206707 | Haptotactic peptide | SEQ ID NO 21 (US20090186802) |
| 265 | Ku70 | 206708 | Haptotactic peptide | SEQ ID NO 22 (US20090186802) |
| 266 | MAP | 206709 | Haptotactic peptide | SEQ ID NO 23 (US20090186802) |
| 267 | MPG | 206710 | Haptotactic peptide | SEQ ID NO 24 (US20090186802) |
| 268 | MPM (IP/K-FGF) | 206711 | Haptotactic peptide | SEQ ID NO 25 (US20090186802) |
| 269 | N50 (NLS of NF-κB P50) | 206712 | Haptotactic peptide | SEQ ID NO 26 (US20090186802) |
| 270 | Pep-7 | 206713 | Haptotactic peptide | SEQ ID NO 28 (US20090186802) |
| 271 | Short Penetratin | 206714 | Haptotactic peptide | SEQ ID NO 30 (US20090186802) |
| 272 | pISL | 206715 | Haptotactic peptide | SEQ ID NO 33 (US20090186802) |
| 273 | Prion mouse PrPc1-28 | 206716 | Haptotactic peptide | SEQ ID NO 34 (US20090186802) |
| 274 | SAP | 206717 | Haptotactic peptide | SEQ ID NO 36 (US20090186802) |
| 275 | SV-40 (NLS) | 206718 | Haptotactic peptide | SEQ ID NO 37 (US20090186802) |
| 276 | SvnB4 | 206719 | Haptotactic peptide | SEQ ID NO 40 (US20090186802) |
| 277 | Transportan derivative | 206720 | Haptotactic peptide | SEQ ID NO 46 (US20090186802) |
| 278 | Transportan derivative | 206721 | Haptotactic peptide | SEQ ID NO 47 (US20090186802) |
| 279 | VP22 | 206722 | Haptotactic peptide | SEQ ID NO 48 (US20090186802) |
| 280 | VT5 | 206723 | Haptotactic peptide | SEQ ID NO 49 (US20090186802) |

### Linkers

In some embodiments, the effector module comprises a linker. Representative examples are given in Table 7 and Table 8.

The effector module of the present invention may optionally further comprise a linker region. The linker region serves primarily as a spacer between two or more polypeptides within the effector module. The "linker" or "spacer", as used herein, refers to a molecule or group of molecules that connects two molecules, or two parts of a molecule such as two domains of a recombinant protein.

In some embodiments, "Linker" (L) or "linker domain" or "linker region" or "linker module" or "peptide linker" as used herein refers to an oligo- or polypeptide region of from about 1 to 100 amino acids in length, which links together any of the domains/regions of the effector module (also called peptide linker). The peptide linker may be 1-40 amino acids in length, or 2-30 amino acids in length, or 20-80 amino acids in length, or 50-100 amino acids in length. Linker length may also be optimized depending on the type of payload utilized and based on the crystal structure of the payload. In some instances, a shorter linker length may be preferably selected. In some aspects, the peptide linker is made up of amino acids linked together by peptide bonds, preferably from 1 to 20 amino acids linked by peptide bonds, wherein the amino acids are selected from the 20 naturally occurring amino acids: Glycine (G), Alanine (A), Valine (V), Leucine (L), Isoleucine (I), Serine (S), Cysteine (C), Threonine (T), Methionine (M), Proline (P), Phenylalanine (F), Tyrosine (Y), Tryptophan (W), Histidine (H), Lysine (K), Arginine (R), Aspartate (D), Glutamic acid (E), Asparagine (N), and Glutamine (Q). One or more of these amino acids may be glycosylated, as is understood by those in the art. In some aspects, amino acids of a peptide linker may be selected from Alanine (A), Glycine (G), Proline (P), Asparagine (R), Serine (S), Glutamine (Q) and Lysine (K).

In one example, an artificially designed peptide linker may preferably be composed of a polymer of flexible residues like Glycine (G) and Serine (S) so that the adjacent protein domains are free to move relative to one another. Longer linkers may be used when it is desirable to ensure that two adjacent domains do not interfere with one another. The choice of a particular linker sequence may concern if it affects biological activity, stability, folding, targeting and/or pharmacokinetic features of the fusion construct.

In other examples, a peptide linker may be made up of a majority of amino acids that are sterically unhindered, such as Glycine (G) and Alanine (A). Exemplary linkers are polyglycines (such as (G)₄, (G)₅, (G)₈), poly(GA), and polyalanines. The linkers described herein are exemplary, and linkers that are much longer and which include other residues are contemplated by the present invention.

A linker sequence may be a natural linker derived from a multi-domain protein. A natural linker is a short peptide sequence that separates two different domains or motifs within a protein. Examples of proteins which may contain natural linkers or regions that can be used in the effector module may include, but are not limited to, those listed in Table 8.

In some aspects, linkers may be flexible or rigid. In other aspects, linkers may be cleavable or non- cleavable. As used herein, the terms "cleavable linker domain or region" or "cleavable peptide linker" are used interchangeably. In some embodiments, the linker sequence may be cleaved enzymatically and/or chemically. Examples of enzymes (e.g., proteinase/peptidase) useful for cleaving the peptide linker include, but are not limited, to Arg-C proteinase, Asp-N endopeptidase, chymotrypsin, clostripain, enterokinase, Factor Xa, glutamyl endopeptidase, Granzyme B, Achromobacter proteinase I, pepsin, proline endopeptidase, proteinase K, Staphylococcal peptidase I, thermolysin, thrombin, trypsin, and members of the Caspase family of proteolytic enzymes (e.g. Caspases 1-10). Chemical sensitive cleavage sites may also be included in a linker sequence. Examples of chemical cleavage reagents include, but are not limited to, cyanogen bromide, which cleaves methionine residues; N-chloro succinimide, iodobenzoic acid or BNPS-skatole [2-(2-nitrophenylsulfenyl)-3-methylindole], which cleaves tryptophan residues; dilute acids, which cleave at aspartyl-prolyl bonds; and e aspartic acid-proline acid cleavable recognition sites (i.e., a cleavable peptide linker comprising one or more D-P dipeptide moieties). The fusion module may include multiple regions encoding peptides of interest separated by one or more cleavable peptide linkers.

In other embodiments, a cleavable linker may be a "self-cleaving" linker peptide, such as 2 A linker (for example T2A), 2A-like linkers or functional equivalents thereof and combinations thereof. In some embodiments, the linkers include the picornaviral 2A-like linker, CHYSEL sequences of porcine teschovirus (P2A), Thosea asigna virus (T2A) or combinations, variants and functional equivalents thereof. Other linkers will be apparent to those skilled in the art and may be used in connection with alternate embodiments of the invention.

The linkers of the present invention may also be non-peptide linkers. For example, alkyl linkers such as -NH-(CH₂)a-C(O)-, wherein a=2-20 can be used. These alkyl linkers may further be substituted by any non-sterically hindering group such as lower alkyl (e.g., C₁-C₆) lower acyl, halogen (e.g., Cl, Br), CN, NH₂, phenyl, etc.

**Table 7. Artificial Linkers**

| Linker No. | Linker | Protease(s) | Source | SEQ ID NO |
|---|---|---|---|---|
| 1 | Artificial linker | - | US4946778 | - |
| 2 | Artificial linker | - | WO2012083424 | - |
| 3 | Artificial linker | - | WO2012083424 | - |
| 4 | Artificial linker | - | WO2012083424 | 206724 |
| 5 | Artificial linker | - | US4946778 | 206725 |
| 6 | Artificial linker | - | US4946778 | 206726 |
| 7 | Artificial linker | - | - | 206727 |
| 8 | Artificial linker | - | US5856456 | 206728 |
| 9 | Artificial linker | - | US4946778 | 206729 |
| 10 | Cleavable Disulfide | chymotrypsin | - | 206730 |
| 11 | Cleavable Disulfide | chymotrypsin | - | 206731 |
| 12 | Cleavable Disulfide | trypsin | - | 206732 |
| 13 | Cleavable Disulfide | factor Xa, thrombin, trypsin | - | 206733 |
| 14 | Cleavable Disulfide | trypsin | - | 206734 |
| 15 | Cleavable Disulfide | CSV | - | 206735 |
| 16 | Cleavable Disulfide | chymotrypsin, trypsin | - | 206736 |
| 17 | Cleavable Disulfide | trypsin | - | 206737 |
| 18 | Cleavable Disulfide | trypsin | - | 206738 |
| 19 | Cleavable Disulfide | trypsin | - | 206739 |
| 20 | Cleavable Disulfide | chymotrypsin, thrombin, trypsin | - | 206740 |
| 21 | Flexible G/S rich linker | - | - | - |
| 22 | Flexible G/S rich linker | - | - | - |
| 23 | Flexible G/S rich linker | - | - | - |
| 24 | Flexible G/S rich linker | - | - | - |
| 25 | Flexible G/S rich linker | - | - | - |
| 26 | Flexible G/S rich linker | - | - | - |
| 27 | Flexible G/S rich linker | - | - | 206741 |
| 28 | Flexible G/S rich linker | - | - | 206742 |
| 29 | Flexible G/S rich linker | - | - | 206743 |
| 30 | Flexible G/S rich linker | - | - | 206744 |
| 31 | Flexible G/S rich linker | - | - | 206745 |
| 32 | Flexible G/S rich linker | - | - | 206746 |
| 33 | Flexible G/S rich linker | - | - | 206747 |
| 34 | Flexible G/S rich linker | trypsin | - | 206748 |
| 35 | Flexible G/S rich linker | - | - | 206749 |
| 36 | Flexible G/S rich linker | chymotrypsin, trypsin | - | 206750 |
| 37 | Flexible G/S rich linker | trypsin | - | 206751 |
| 38 | Flexible G/S rich linker | - | - | 206752 |
| 39 | Flexible G/S rich linker | - | - | 206753 |
| 40 | Flexible G/S rich linker | - | - | 206754 |
| 41 | Rigid extended P-rich | - | - | 206755 |
| 42 | Rigid extended P-rich | - | - | 206756 |
| 43 | Rigid extended P-rich | - | - | 206757 |
| 44 | Rigid extended P-rich | - | - | 206758 |
| 45 | Rigid extended P-rich | - | - | 206759 |
| 46 | Rigid extended P-rich | - | - | 206760 |
| 47 | Rigid extended P-rich | - | - | 206761 |
| 48 | Rigid extended P-rich | - | - | 206762 |
| 49 | Rigid extended P-rich | - | - | 206763 |
| 50 | Rigid extended P-rich | - | - | 206764 |
| 51 | Rigid extended P-rich | - | - | 206765 |
| 52 | Rigid extended P-rich | - | - | 206766 |
| 53 | Rigid extended P-rich | - | - | 206767 |
| 54 | Rigid extended P-rich | - | - | 206768 |
| 55 | Rigid extended P-rich | - | - | 206769 |
| 56 | Rigid helical | - | - | 206770 |
| 57 | Rigid helical | trypsin | - | 206771 |
| 58 | Rigid helical | trypsin | - | 206772 |
| 59 | Rigid helical | trypsin | - | 206773 |
| 60 | Rigid helical | trypsin | - | 206774 |
| 61 | Rigid helical | trypsin | - | 206775 |
| 62 | Rigid helical | trypsin | - | 206776 |
| 63 | Rigid helical | trypsin | - | 206777 |
| 64 | Serine rich linker | - | US5525491 | 206778 |
| 65 | Serine rich linker | - | US5525491 | 206779 |
| 66 | Serine rich linker | - | US5525491 | 206780 |
| 67 | Serine rich linker | - | US5525491 | 206781 |
| 68 | Serine rich linker | - | US5525491 | 206782 |
| 69 | Serine rich linker | - | US5525491 | 206783 |
| 70 | Serine rich linker | - | US5525491 | 206784 |

**Table 8. Proteins containing natural linker regions**

| **Linker No.** | **Name** | **SEQ ID NO** | **Protease cleavage site; linker core** |
|---|---|---|---|
| 71 | 1,4-alpha-glucan-branching enzyme | - | |
| 72 | 1,4-alpha-glucan-branching enzyme | 206785 | chymotrypsin |
| 73 | 1,4-beta-n-acetylmuramidase | - | chymotrypsin, trypsin |
| 74 | 1,4-beta-n-acetylmuramidase | 206786 | |
| 75 | 1,4-beta-n-acetylmuramidase | 206787 | |
| 76 | 1,4-beta-n-acetylmuramidase | 206788 | trypsin |
| 77 | 1,4-beta-n-acetylmuramidase | 206789 | |
| 78 | 1,4-beta-n-acetylmuramidase | 206790 | |
| 79 | 1,4-beta-n-acetylmuramidase | 206791 | |
| 80 | 1,4-beta-n-acetylmuramidase | 206792 | chymotrypsin |
| 81 | 1,4-beta-n-acetylmuramidase | 206793 | chymotrypsin, trypsin |
| 82 | 1,4-beta-n-acetylmuramidase | 206794 | chymotrypsin, trypsin |
| 83 | 1,4-beta-n-acetylmuramidase | 206795 | |
| 84 | 150aa long hypothetical transcriptional regulator | 206796 | |
| 85 | 150aa long hypothetical transcriptional regulator | 206797 | chymotrypsin, trypsin |
| 86 | 1-deoxy-d-xylulose 5-phosphate reductoisomerase | 206798 | trypsin |
| 87 | 1-deoxy-d-xylulose 5-phosphate reductoisomerase | 206799 | chymotrypsin |
| 88 | 1-deoxy-d-xylulose 5-phosphate reductoisomerase | 206800 | chymotrypsin |
| 89 | 1-deoxy-d-xylulose 5-phosphate reductoisomerase | 206801 | |
| 90 | 235aa long hypothetical biotin-[acetyl-coa-carboxylase] ligase | 206802 | trypsin |
| 91 | 235aa long hypothetical biotin-[acetyl-coa-carboxylase] ligase | 206803 | |
| 92 | 235aa long hypothetical biotin-[acetyl-coa-carboxylase] ligase | 206804 | trypsin |
| 93 | 2-dehydropantoate 2-reductase | 206805 | |
| 94 | 2-dehydropantoate 2-reductase | 206806 | trypsin |
| 95 | 2-dehydropantoate 2-reductase | 206807 | |
| 96 | 2-dehydropantoate 2-reductase | 206808 | chymotrypsin |
| 97 | 2-dehydropantoate 2-reductase | 206809 | chymotrypsin, trypsin |
| 98 | 2-dehydropantoate 2-reductase | 206810 | |
| 99 | 2-dehydropantoate 2-reductase, putative | 206811 | chymotrypsin |
| 100 | 2-dehydropantoate 2-reductase, putative | 206812 | trypsin |
| 101 | 4-alpha-glucanotransferase | 206813 | trypsin |
| 102 | 4-alpha-glucanotransferase | 206814 | trypsin |
| 103 | 4-alpha-glucanotransferase | 206815 | chymotrypsin, trypsin |
| 104 | 4-diphosphocytidyl-2c-methyl-d-erythritol kinase | - | |
| 105 | 4-diphosphocytidyl-2c-methyl-d-erythritol kinase | 206816 | |
| 106 | 4-diphosphocytidyl-2c-methyl-d-erythritol kinase | 206817 | trypsin |
| 107 | 4-diphosphocytidyl-2c-methyl-d-erythritol kinase | 206818 | |
| 108 | 4-diphosphocytidyl-2c-methyl-d-erythritol kinase | 206819 | chymotrypsin, trypsin |
| 109 | 4-hydroxyphenylpyruvate dioxygenase | 206820 | chymotrypsin, trypsin |
| 110 | 5-13 amino acids from the N termini of human Ck and CH1 domains Linker | 206821 | |
| 111 | 5-13 amino acids from the N termini of human Ck and CH1 domains Linker | - | |
| 112 | 5-13 amino acids from the N termini of human Ck and CH1 domains Linker | 206822 | |
| 113 | 5-13 amino acids from the N termini of human Ck and CH1 domains Linker | 206823 | |
| 114 | 5-13 amino acids from the N termini of human Ck and CH1 domains Linker | 206824 | |
| 115 | 5-13 amino acids from the N termini of human Ck and CH1 domains Linker | 206825 | |
| 116 | 5'-exonuclease | 206826 | |
| 117 | 5-methyltetrahydropteroyltriglutamate-homocysteinemethyltransferase | - | trypsin |
| 118 | 5-methyltetrahydropteroyltriglutamate-homocysteinemethyltransferase | 206827 | trypsin |
| 119 | 5-methyltetrahydropteroyltriglutamate-homocysteinemethyltransferase | 206828 | trypsin |
| 120 | 5-methyltetrahydropteroyltriglutamate-homocysteinemethyltransferase | 206829 | trypsin |
| 121 | 5-methyltetrahydropteroyltriglutamate-homocysteinemethyltransferase | 206830 | trypsin |
| 122 | 5'-nucleotidase | 206831 | trypsin |
| 123 | 5'-nucleotidase | 206832 | |
| 124 | 5'-nucleotidase | 206833 | trypsin |
| 125 | 5'-nucleotidase | 206834 | chymotrypsin |
| 126 | 704aa long hypothetical glycosyltransferase | 206835 | |
| 127 | 704aa long hypothetical glycosyltransferase | 206836 | trypsin |
| 128 | 80 kda nuclear cap binding protein | 206837 | chymotrypsin, trypsin |
| 129 | 80 kda nuclear cap binding protein | 206838 | |
| 130 | 80 kda nuclear cap binding protein | 206839 | chymotrypsin, trypsin |
| 131 | 80 kda nuclear cap binding protein | 206840 | chymotrypsin, trypsin |
| 132 | Acetaldehyde dehydrogenase (acylating) | 206841 | |
| 133 | Acetaldehyde dehydrogenase (acylating) | 206842 | trypsin |
| 134 | Acetolactate synthase isozyme iii small subunit | 206843 | |
| 135 | Acetylcholine receptor protein, alpha chain | 206844 | trypsin |
| 136 | Acetylcholine receptor protein, beta chain | 206845 | chymotrypsin, trypsin |
| 137 | Aconitate hydratase 2 | 206846 | |
| 138 | Aconitate hydratase 2 | 206847 | chymotrypsin, trypsin |
| 139 | Aconitate hydratase 2 | 206848 | |
| 140 | Aconitate hydratase 2 | 206849 | chymotrypsin, trypsin |
| 141 | Aconitate hydratase 2 | 206850 | chymotrypsin, trypsin |
| 142 | Acriflavine resistance protein b | - | chymotrypsin |
| 143 | Acriflavine resistance protein b | - | |
| 144 | Acriflavine resistance protein b | - | |
| 145 | Acriflavine resistance protein b | - | trypsin |
| 146 | Acriflavine resistance protein b | - | |
| 147 | Acriflavine resistance protein b | 206851 | trypsin |
| 148 | Acriflavine resistance protein b | 206852 | chymotrypsin |
| 149 | Acriflavine resistance protein b | 206853 | chymotrypsin |
| 150 | Acriflavine resistance protein b | 206854 | |
| 151 | Acriflavine resistance protein b | 206855 | |
| 152 | Acriflavine resistance protein b | 206856 | trypsin |
| 153 | Acriflavine resistance protein b | 206857 | chymotrypsin, trypsin |
| 154 | Acriflavine resistance protein b | 206858 | chymotrypsin |
| 155 | Acriflavine resistance protein b | 206859 | chymotrypsin |
| 156 | Acriflavine resistance protein b | 206860 | trypsin |
| 157 | Acriflavine resistance protein b | 206861 | trypsin |
| 158 | Acriflavine resistance protein b | 206862 | trypsin |
| 159 | Acriflavine resistance protein b | 206863 | |
| 160 | Acriflavine resistance protein b | 206864 | chymotrypsin, trypsin |
| 161 | Acriflavine resistance protein b | 206865 | trypsin |
| 162 | Acriflavine resistance protein b | 206866 | |
| 163 | Acriflavine resistance protein b | 206867 | trypsin |
| 164 | Acriflavine resistance protein b | 206868 | trypsin |
| 165 | Acriflavine resistance protein b | 206869 | |
| 166 | Acriflavine resistance protein b | 206870 | trypsin |
| 167 | Acriflavine resistance protein b | 206871 | chymotrypsin, thrombin, trypsin |
| 168 | Acriflavine resistance protein b | 206872 | chymotrypsin, trypsin |
| 169 | Acriflavine resistance protein b | 206873 | chymotrypsin |
| 170 | Acriflavine resistance protein b | 206874 | trypsin |
| 171 | Acyl-coa thioesterase ii | 206875 | |
| 172 | Acyl-coa thioesterase ii | 206876 | chymotrypsin |
| 173 | Acyl-coa thioesterase ii | 206877 | chymotrypsin, trypsin |
| 174 | Acyl-coa thioesterase ii | 206878 | chymotrypsin, trypsin |
| 175 | Acyl-coa thioesterase ii | 206879 | trypsin |
| 176 | Acyl-coenzyme a thioesterase 4 | 206880 | trypsin |
| 177 | Acyl-coenzyme a thioesterase 4 | 206881 | chymotrypsin |
| 178 | Acyl-coenzyme a thioesterase 4 | 206882 | |
| 179 | Acyl-coenzyme a thioesterase 4 | 206883 | chymotrypsin |
| 180 | Acyl-coenzyme a thioesterase 4 | 206884 | chymotrypsin |
| 181 | Adenine glycosylase | 206885 | trypsin |
| 182 | Adenylate cyclase | 206886 | |
| 183 | Aerolysin | 206887 | |
| 184 | Aerolysin | 206888 | |
| 185 | Agglutinin | - | chymotrypsin |
| 186 | Agglutinin isolectin 1 | 206889 | trypsin |
| 187 | Agglutinin isolectin 1 | 206890 | |
| 188 | Aldehyde ferredoxin oxidoreductase | 206891 | trypsin |
| 189 | Aldehyde oxidoreductase | 206892 | trypsin |
| 190 | Aldehyde oxidoreductase | 206893 | chymotrypsin |
| 191 | Aldehyde oxidoreductase | 206894 | chymotrypsin |
| 192 | Aldehyde oxidoreductase | 206895 | |
| 193 | Aldehyde oxidoreductase | 206896 | chymotrypsin, factor xa, trypsin |
| 194 | Alkyl hydroperoxide reductase subunit f | 206897 | chymotrypsin |
| 195 | Alkyl hydroperoxide reductase subunit f | 206898 | |
| 196 | Alkyl hydroperoxide reductase subunit f | 206899 | |
| 197 | Alkyl hydroperoxide reductase subunit f | 206900 | trypsin |
| 198 | Alkyl hydroperoxide reductase subunit f | 206901 | trypsin |
| 199 | Alkyl hydroperoxide reductase subunit f | 206902 | chymotrypsin, trypsin |
| 200 | Alkyl hydroperoxide reductase subunit f | 206903 | trypsin |
| 201 | Alkyl hydroperoxide reductase subunit f | 206904 | trypsin |
| 202 | Alkyl hydroperoxide reductase subunit f | 206905 | |
| 203 | Alkyl hydroperoxide reductase subunit f | 206906 | chymotrypsin, trypsin |
| 204 | Allantoicase | 206907 | chymotrypsin |
| 205 | Allantoicase | 206908 | trypsin |
| 206 | Alliin lyase 1 | - | |
| 207 | Alliin lyase 1 | 206909 | chymotrypsin, trypsin |
| 208 | Alliin lyase 1 | 206910 | trypsin |
| 209 | Alliin lyase 1 | 206911 | |
| 210 | Alliin lyase 1 | 206912 | chymotrypsin, trypsin |
| 211 | Alpha amylase | 206913 | chymotrypsin |
| 212 | Alpha amylase | 206914 | trypsin |
| 213 | Alpha-actinin 1 | 206915 | |
| 214 | Alpha-actinin 1 | 206916 | |
| 215 | Alpha-adaptin c | 206917 | trypsin |
| 216 | Alpha-amylase | 206918 | chymotrypsin |
| 217 | Alpha-glucuronidase | - | |
| 218 | Alpha-glucuronidase | 206919 | chymotrypsin |
| 219 | Alpha-glucuronidase | 206920 | chymotrypsin |
| 220 | Alpha-glucuronidase | 206921 | chymotrypsin |
| 221 | Alpha-glucuronidase | 206922 | chymotrypsin, trypsin |
| 222 | Alpha-glucuronidase | 206923 | trypsin |
| 223 | Alpha-glucuronidase | 206924 | |
| 224 | Alpha-glucuronidase | 206925 | trypsin |
| 225 | Alpha-glucuronidase | 206926 | chymotrypsin |
| 226 | Alpha-glucuronidase | 206927 | chymotrypsin |
| 227 | Alpha-glucuronidase | 206928 | chymotrypsin, trypsin |
| 228 | Alpha-glucuronidase | 206929 | |
| 229 | Alpha-glucuronidase | 206930 | trypsin |
| 230 | Alpha-glucuronidase | 206931 | chymotrypsin, trypsin |
| 231 | Alpha-glucuronidase | 206932 | trypsin |
| 232 | Alpha-glucuronidase | 206933 | chymotrypsin, trypsin |
| 233 | Alpha-glucuronidase | 206934 | chymotrypsin |
| 234 | Alpha-glucuronidase | 206935 | trypsin |
| 235 | Alpha-glucuronidase | 206936 | chymotrypsin, trypsin |
| 236 | Alpha-glucuronidase | 206937 | chymotrypsin, trypsin |
| 237 | Alpha-glucuronidase | 206938 | chymotrypsin |
| 238 | Alpha-glucuronidase | 206939 | chymotrypsin, trypsin |
| 239 | Alpha-I-arabinofuranosidase b | 206940 | chymotrypsin |
| 240 | Alpha-mannosidase | 206941 | chymotrypsin |
| 241 | Alr2269 protein | 206942 | trypsin |
| 242 | Amp nucleosidase | 206943 | chymotrypsin |
| 243 | Amp nucleosidase | 206944 | |
| 244 | Amp nucleosidase | 206945 | chymotrypsin |
| 245 | Angiopoietin-1 receptor | - | |
| 246 | Angiopoietin-1 receptor | - | |
| 247 | Angiopoietin-1 receptor | - | |
| 248 | Angiopoietin-1 receptor | - | |
| 249 | Angiopoietin-1 receptor | 206946 | chymotrypsin |
| 250 | Angiopoietin-1 receptor | 206947 | trypsin |
| 251 | Angiopoietin-1 receptor | 206948 | chymotrypsin, trypsin |
| 252 | Angiopoietin-1 receptor | 206949 | |
| 253 | Angiopoietin-1 receptor | 206950 | |
| 254 | Angiopoietin-1 receptor | 206951 | |
| 255 | Angiopoietin-1 receptor | 206952 | chymotrypsin |
| 256 | Angiopoietin-1 receptor | 206953 | |
| 257 | Angiopoietin-1 receptor | 206954 | trypsin |
| 258 | Angiopoietin-1 receptor | 206955 | chymotrypsin |
| 259 | Angiopoietin-1 receptor | 206956 | chymotrypsin, trypsin |
| 260 | Angiopoietin-1 receptor | 206957 | trypsin |
| 261 | Angiopoietin-1 receptor | 206958 | chymotrypsin |
| 262 | Angiopoietin-1 receptor | 206959 | trypsin |
| 263 | Angiopoietin-1 receptor | 206960 | trypsin |
| 264 | Angiopoietin-1 receptor | 206961 | chymotrypsin, trypsin |
| 265 | Angiopoietin-1 receptor | 206962 | |
| 266 | Angiopoietin-1 receptor | 206963 | |
| 267 | Angiopoietin-1 receptor | 206964 | chymotrypsin, trypsin |
| 268 | Angiopoietin-1 receptor | 206965 | chymotrypsin, thrombin, trypsin |
| 269 | Angiopoietin-1 receptor | 206966 | chymotrypsin, trypsin |
| 270 | Annexin a2 | - | |
| 271 | Annexin a2 | 206967 | trypsin |
| 272 | Annexin a2 | 206968 | trypsin |
| 273 | Anthranilate phosphoribosyltransferase | 206969 | trypsin |
| 274 | Ap-2 complex subunit beta-2 | 206970 | |
| 275 | Archaeosine trna-guanine transglycosylase | - | |
| 276 | Archaeosine trna-guanine transglycosylase | 206971 | chymotrypsin |
| 277 | Archaeosine trna-guanine transglycosylase | 206972 | chymotrypsin |
| 278 | Archaeosine trna-guanine transglycosylase | 206973 | trypsin |
| 279 | Archaeosine trna-guanine transglycosylase | 206974 | trypsin |
| 280 | Archaeosine trna-guanine transglycosylase | 206975 | trypsin |
| 281 | Archaeosine trna-guanine transglycosylase | 206976 | trypsin |
| 282 | Archaeosine trna-guanine transglycosylase | 206977 | chymotrypsin, trypsin |
| 283 | Archeal exosome rna binding protein rrp4 | 206978 | chymotrypsin, trypsin |
| 284 | Archeal exosome rna binding protein rrp4 | 206979 | |
| 285 | Archeal exosome rna binding protein rrp4 | 206980 | chymotrypsin, trypsin |
| 286 | Arginyl-trna synthetase | - | |
| 287 | Arginyl-trna synthetase | 206981 | trypsin |
| 288 | Arginyl-trna synthetase | 206982 | chymotrypsin, trypsin |
| 289 | Arginyl-trna synthetase | 206983 | chymotrypsin, trypsin |
| 290 | Arrestin | 206984 | chymotrypsin |
| 291 | Arrestin | 206985 | trypsin |
| 292 | Arsenite oxidase | 206986 | chymotrypsin |
| 293 | Atp phosphoribosyltransferase | - | |
| 294 | Atp-dependent dna helicase | - | chymotrypsin |
| 295 | Atp-dependent dna helicase | 206987 | trypsin |
| 296 | Atp-dependent dna helicase | 206988 | |
| 297 | Atp-dependent dna helicase | 206989 | |
| 298 | Atp-dependent dna helicase | 206990 | |
| 299 | Atp-dependent dna helicase | 206991 | |
| 300 | Atp-dependent dna helicase | 206992 | trypsin |
| 301 | Atp-dependent dna helicase | 206993 | chymotrypsin |
| 302 | Atp-dependent dna helicase | 206994 | chymotrypsin, factor xa, trypsin |
| 303 | At-rich dna-binding protein | 206995 | trypsin |
| 304 | At-rich dna-binding protein | 206996 | chymotrypsin, trypsin |
| 305 | Axonin-1 | - | |
| 306 | Axonin-1 | - | |
| 307 | Axonin-1 | 206997 | trypsin |
| 308 | Axonin-1 | 206998 | |
| 309 | Axonin-1 | 206999 | trypsin |
| 310 | Axonin-1 | 207000 | |
| 311 | Axonin-1 | 207001 | |
| 312 | Axonin-1 | 207002 | trypsin |
| 313 | Axonin-1 | 207003 | chymotrypsin, trypsin |
| 314 | Bacilysin biosynthesis protein bacb | 207004 | chymotrypsin |
| 315 | Bacilysin biosynthesis protein bacb | 207005 | |
| 316 | Bacilysin biosynthesis protein bacb | 207006 | |
| 317 | Bacilysin biosynthesis protein bacb | 207007 | chymotrypsin |
| 318 | Bacilysin biosynthesis protein bacb | 207008 | chymotrypsin, trypsin |
| 319 | Bacteriophage mu transposase | 207009 | trypsin |
| 320 | Bacteriophage mu transposase | 207010 | chymotrypsin, trypsin |
| 321 | Benzoyl-coa-dihydrodiol lyase | 207011 | trypsin |
| 322 | Benzoyl-coa-dihydrodiol lyase | 207012 | chymotrypsin, trypsin |
| 323 | Benzoyl-coa-dihydrodiol lyase | 207013 | trypsin |
| 324 | Benzoyl-coa-dihydrodiol lyase | 207014 | trypsin |
| 325 | Benzoyl-coa-dihydrodiol lyase | 207015 | trypsin |
| 326 | Benzoylformate decarboxylase | 207016 | chymotrypsin |
| 327 | Benznylformate decarboxylase | 207017 | chymotrypsin, trypsin |
| 328 | Benzoylformate decarboxylase | 207018 | trypsin |
| 329 | Beta-amylase | 207019 | |
| 330 | Beta-galactosidase | - | |
| 331 | Beta-galactosidase | 207020 | |
| 332 | Beta-galactosidase | 207021 | |
| 333 | Beta-galactosidase | 207022 | chymotrypsin, trypsin |
| 334 | Beta-galactosidase | 207023 | trypsin |
| 335 | Beta-galactosidase | 207024 | |
| 336 | Beta-galactosidase | 207025 | |
| 337 | Beta-galactosidase | 207026 | |
| 338 | Beta-galactosidase | 207027 | |
| 339 | Beta-galactosidase | 207028 | trypsin |
| 340 | Beta-galactosidase | 207029 | |
| 341 | Beta-galactosidase | 207030 | chymotrypsin, trypsin |
| 342 | Beta-galactosidase | 207031 | |
| 343 | Beta-galactosidase | 207032 | |
| 344 | Beta-galactosidase | 207033 | |
| 345 | Beta-galactosidase | 207034 | chymotrypsin, trypsin |
| 346 | Beta-galactosidase | 207035 | |
| 347 | Beta-galactosidase | 207036 | chymotrypsin |
| 348 | Beta-galactosidase | 207037 | chymotrypsin, trypsin |
| 349 | Beta-galactosidase | 207038 | chymotrypsin, trypsin |
| 350 | Beta-galactosidase | 207039 | |
| 351 | Beta-galactosidase | 207040 | chymotrypsin |
| 352 | Beta-galactosidase | 207041 | chymotrypsin, trypsin |
| 353 | Beta-n-acetylhexosaminidase | - | trypsin |
| 354 | Beta-n-acetylhexosaminidase | 207042 | |
| 355 | Beta-n-acetylhexosaminidase | 207043 | trypsin |
| 356 | Beta-n-acetylhexosaminidase | 207044 | chymotrypsin, trypsin |
| 357 | Bifunctional nmn adenylyltransferase/nudix hydrolase | 207045 | chymotrypsin |
| 358 | Bifunctional purine biosynthesis protein purh | 207046 | chymotrypsin, trypsin |
| 359 | Biliverdin reductase a | - | |
| 360 | Biliverdin reductase a | - | |
| 361 | Biliverdin reductase a | 207047 | trypsin |
| 362 | Biliverdin reductase a | 207048 | trypsin |
| 363 | Biodegradative arginine decarboxylase | - | |
| 364 | Biodegradative arginine decarboxylase | 207049 | |
| 365 | Biodegradative arginine decarboxylase | 207050 | |
| 366 | Biodegradative arginine decarboxylase | 207051 | |
| 367 | Biodegradative arginine decarboxylase | 207052 | chymotrypsin |
| 368 | Biodegradative arginine decarboxylase | 207053 | chymotrypsin, trypsin |
| 369 | Biodegradative arginine decarboxylase | 207054 | |
| 370 | Biodegradative arginine decarboxylase | 207055 | trypsin |
| 371 | Biodegradative arginine decarboxylase | 207056 | trypsin |
| 372 | Biodegradative arginine decarboxylase | 207057 | chymotrypsin |
| 373 | Biodegradative arginine decarboxylase | 207058 | chymotrypsin, trypsin |
| 374 | Biodegradative arginine decarboxylase | 207059 | |
| 375 | Biodegradative arginine decarboxylase | 207060 | chymotrypsin, trypsin |
| 376 | Biotin carboxylase | 207061 | trypsin |
| 377 | Bowman-birk trypsin inhibitor | 207062 | chymotrypsin, trypsin |
| 378 | Bpt4 gene 59 helicase assembly protein | - | trypsin |
| 379 | Brca1-associated ring domain protein 1 | 207063 | chymotrypsin, trypsin |
| 380 | Brca1-associated ring domain protein 1 | 207064 | trypsin |
| 381 | Brca1-associated ring domain protein 1 | 207065 | chymotrypsin, trypsin |
| 382 | Breast cancer 2 | 207066 | chymotrypsin |
| 383 | Breast cancer 2 | 207067 | |
| 384 | Breast cancer 2 | 207068 | trypsin |
| 385 | Breast cancer 2 | 207069 | chymotrypsin |
| 386 | Breast cancer 2 | 207070 | |
| 387 | Breast cancer 2 | 207071 | chymotrypsin, trypsin |
| 388 | Butyrate response factor 2 | 207072 | chymotrypsin, trypsin |
| 389 | C4b-binding protein | - | chymotrypsin, trypsin |
| 390 | C4b-binding protein | 207073 | chymotrypsin |
| 391 | C5a peptidase | 207074 | |
| 392 | C5a peptidase | 207075 | chymotrypsin |
| 393 | C5a peptidase | 207076 | trypsin |
| 394 | C5a peptidase | 207077 | |
| 395 | C5a peptidase | 207078 | |
| 396 | C5a peptidase | 207079 | trypsin |
| 397 | C5a peptidase | 207080 | |
| 398 | C5a peptidase | 207081 | trypsin |
| 399 | C5a peptidase | 207082 | trypsin |
| 400 | C5a peptidase | 207083 | |
| 401 | C5a peptidase | 207084 | chymotrypsin, trypsin |
| 402 | C5a peptidase | 207085 | |
| 403 | C5a peptidase | 207086 | chymotrypsin, trypsin |
| 404 | Calcium-binding protein | 207087 | |
| 405 | Cara | 207088 | chymotrypsin, trypsin |
| 406 | Cara | 207089 | |
| 407 | Carbamoyl phosphate synthetase (small chain) | 207090 | |
| 408 | Carbamoyl phosphate synthetase (small chain) | 207091 | |
| 409 | Carbamoyl phosphate synthetase (small chain) | 207092 | |
| 410 | Carbamoyl phosphate synthetase (small chain) | 207093 | |
| 411 | Carbamoyl phosphate synthetase (small chain) | 207094 | chymotrypsin, trypsin |
| 412 | Carbon monoxide dehydrogenase/acetyl-coa synthase subunitalpha | 207095 | trypsin |
| 413 | Carboxypeptidase gp180 residues 503-882 | - | trypsin |
| 414 | Catabolite activation-like protein | 207096 | chymotrypsin |
| 415 | Catabolite activation-like protein | 207097 | |
| 416 | Catechol 2,3-dioxygenase | 207098 | chymotrypsin, trypsin |
| 417 | Cation-independent mannose 6-phosphate receptor | 207099 | chymotrypsin, trypsin |
| 418 | Cd3 epsilon and gamma ectodomain fragmentcomplex | 207100 | trypsin |
| 419 | Cd3 epsilon and gamma ectodomain fragmentcomplex | 207101 | trypsin |
| 420 | Cell filamentation protein | - | |
| 421 | Cell filamentation protein | 207102 | trypsin |
| 422 | Cell filamentation protein | 207103 | trypsin |
| 423 | Cellular coagulation factor xiii zymogen | - | |
| 424 | Cellular coagulation factor xiii zymogen | - | |
| 425 | Cellular coagulation factor xiii zymogen | - | |
| 426 | Cellular coagulation factor xiii zymogen | 207104 | |
| 427 | Cellular coagulation factor xiii zymogen | 207105 | |
| 428 | Cellular coagulation factor xiii zymogen | 207106 | chymotrypsin |
| 429 | Cellular coagulation factor xiii zymogen | 207107 | thrombin, trypsin |
| 430 | Cellular coagulation factor xiii zymogen | 207108 | |
| 431 | Cellular coagulation factor xiii zymogen | 207109 | trypsin |
| 432 | Cellular coagulation factor xiii zymogen | 207110 | trypsin |
| 433 | Cellular coagulation factor xiii zymogen | 207111 | chymotrypsin |
| 434 | Cellular coagulation factor xiii zymogen | 207112 | trypsin |
| 435 | Cellular coagulation factor xiii zymogen | 207113 | trypsin |
| 436 | Cellular coagulation factor xiii zymogen | 207114 | trypsin |
| 437 | Cellular coagulation factor xiii zymogen | 207115 | |
| 438 | Cellular coagulation factor xiii zymogen | 207116 | chymotrypsin, trypsin |
| 439 | Cellular coagulation factor xiii zymogen | 207117 | trypsin |
| 440 | Cellular coagulation factor xiii zymogen | 207118 | chymotrypsin |
| 441 | Cellular coagulation factor xiii zymogen | 207119 | chymotrypsin |
| 442 | Cellular coagulation factor xiii zymogen | 207120 | chymotrypsin |
| 443 | Cellular coagulation factor xiii zymogen | 207121 | chymotrypsin |
| 444 | Cellular coagulation factor xiii zymogen | 207122 | chymotrypsin, trypsin |
| 445 | Cellulase | 207123 | |
| 446 | Cellulase | 207124 | |
| 447 | Cellulase | 207125 | |
| 448 | Cellulase | 207126 | |
| 449 | Cellulase | 207127 | |
| 450 | Cellulase | 207128 | |
| 451 | Cellulase | 207129 | |
| 452 | Cellulase | 207130 | |
| 453 | Cellulase | 207131 | |
| 454 | Cellulase linker | 207132 | |
| 455 | Cellulase linker | 207133 | |
| 456 | Cellulase linker | 207134 | |
| 457 | Cellulase linker | 207135 | |
| 458 | Chaperone protein fimc | - | trypsin |
| 459 | Chaperone protein fimc | - | |
| 460 | Chaperone protein fimc | 207136 | trypsin |
| 461 | Chaperone protein fimc | 207137 | trypsin |
| 462 | Chaperone protein hscb | - | trypsin |
| 463 | Chaperone protein hscb | 207138 | chymotrypsin, trypsin |
| 464 | Cheb methylesterase | 207139 | trypsin |
| 465 | Cheb methylesterase | 207140 | trypsin |
| 466 | Cheb methylesterase | 207141 | trypsin |
| 467 | Chelatase, putative | 207142 | trypsin |
| 468 | Chemotaxis receptor methyltransferase cher | 207143 | trypsin |
| 469 | Chemotaxis receptor methyltransferase cher | 207144 | |
| 470 | Chemotaxis receptor methyltransferase cher | 207145 | chymotrypsin, trypsin |
| 471 | Cholesterol oxidase | 207146 | |
| 472 | Cholesterol oxidase | 207147 | trypsin |
| 473 | Cholesterol oxidase | 207148 | chymotrypsin |
| 474 | Cholesterol oxidase | 207149 | |
| 475 | Cholesterol oxidase | 207150 | trypsin |
| 476 | Cholesterol oxidase | 207151 | |
| 477 | Cholesterol oxidase | 207152 | chymotrypsin |
| 478 | Cholesterol oxidase | 207153 | trypsin |
| 479 | Cholesterol oxidase | 207154 | trypsin |
| 480 | Cholesterol oxidase | 207155 | chymotrypsin |
| 481 | Cholesterol oxidase | 207156 | |
| 482 | Cholesterol oxidase | 207157 | chymotrypsin |
| 483 | Chromatin structure-remodeling complex proteinrsc4 | - | trypsin |
| 484 | Chromatin structure-remodeling complex proteinrsc4 | 207158 | |
| 485 | Chromatin structure-remodeling complex proteinrsc4 | 207159 | chymotrypsin, trypsin |
| 486 | Chromatin structure-remodeling complex proteinrsc4 | 207160 | trypsin |
| 487 | Chromodomain-helicase-dna-binding protein 1 | 207161 | chymotrypsin |
| 488 | Chromodomain-helicase-dna-binding protein 1 | 207162 | chymotrypsin, trypsin |
| 489 | Colicin ia | 207163 | trypsin |
| 490 | Collagen adhesin | 207164 | chymotrypsin |
| 491 | Complement c3 beta chain | 207165 | chymotrypsin |
| 492 | Complement c3 beta chain | 207166 | |
| 493 | Complement c3 beta chain | 207167 | |
| 494 | Complement c3 beta chain | 207168 | trypsin |
| 495 | Complement decay-accelerating factor | - | |
| 496 | Complement factor h | - | trypsin |
| 497 | Complement receptor type 2 | 207169 | chymotrypsin, trypsin |
| 498 | Conserved hypothetical protein | 207170 | chymotrypsin, trypsin |
| 499 | Conserved hypothetical protein mth1747 | - | |
| 500 | Conserved hypothetical protein mth1747 | 207171 | chymotrypsin |
| 501 | Conserved hypothetical protein mth1747 | 207172 | |
| 502 | Conserved hypothetical protein mth1747 | 207173 | trypsin |
| 503 | Conserved hypothetical protein mth1747 | 207174 | trypsin |
| 504 | Conserved hypothetical protein mth1747 | 207175 | |
| 505 | Conserved hypothetical protein mth1747 | 207176 | trypsin |
| 506 | Conserved hypothetical protein mth1747 | 207177 | trypsin |
| 507 | Conserved protein (mth177) | 207178 | |
| 508 | Creatine amidinohydrolase | 207179 | |
| 509 | Cruciferin | 207180 | chymotrypsin |
| 510 | Cruciferin | 207181 | chymotrypsin, trypsin |
| 511 | Cruciferin | 207182 | |
| 512 | Cruciferin | 207183 | trypsin |
| 513 | Cruciferin | 207184 | chymotrypsin, factor xa, trypsin |
| 514 | Cruciferin | 207185 | chymotrypsin, trypsin |
| 515 | Cruciferin | 207186 | chymotrypsin, trypsin |
| 516 | Csl3 | 207187 | |
| 517 | Csl3 | 207188 | |
| 518 | Ctp synthase | 207189 | trypsin |
| 519 | Ctp synthase | 207190 | trypsin |
| 520 | Cullin homolog | - | trypsin |
| 521 | Cullin homolog | 207191 | trypsin |
| 522 | Cullin homolog | 207192 | chymotrypsin |
| 523 | Cullin homolog | 207193 | chymotrypsin, trypsin |
| 524 | Cullin homolog | 207194 | chymotrypsin, trypsin |
| 525 | Cullin homolog | 207195 | trypsin |
| 526 | Cyclin a2 | 207196 | chymotrypsin |
| 527 | Cysteine-rich secretory protein | 207197 | chymotrypsin, trypsin |
| 528 | Cytidine deaminase | 207198 | trypsin |
| 529 | Cytidine deaminase | 207199 | chymotrypsin |
| 530 | Cytidine deaminase | 207200 | chymotrypsin, trypsin |
| 531 | Cytochrome b-c1 complex subunit rieske, mitochondrial | 207201 | trypsin |
| 532 | Cytochrome c oxidase subunit 2 | - | |
| 533 | Cytochrome c oxidase subunit 2 | 207202 | chymotrypsin |
| 534 | Cytochrome c oxidase subunit 2 | 207203 | |
| 535 | Cytochrome c oxidase subunit 2 | 207204 | |
| 536 | Cytochrome c oxidase subunit 2 | 207205 | chymotrypsin, trypsin |
| 537 | Cytochrome c4 | - | |
| 538 | Cytochrome c4 | - | |
| 539 | D-aminopeptidase | 207206 | trypsin |
| 540 | Ddmc | 207207 | chymotrypsin |
| 541 | Ddmc | 207208 | chymotrypsin |
| 542 | Deltex protein | 207209 | chymotrypsin |
| 543 | Deoxyuridine 5'-triphosphate nucleotidohydrolase | 207210 | trypsin |
| 544 | Diaminopimelate epimerase | 207211 | chymotrypsin |
| 545 | Diaminopimelate epimerase | 207212 | trypsin |
| 546 | Diaminopimelate epimerase | 207213 | chymotrypsin, trypsin |
| 547 | Di-heme peroxidase | - | |
| 548 | Di-heme peroxidase | 207214 | trypsin |
| 549 | Dihydropyrimidine dehydrogenase | 207215 | |
| 550 | Dihydropyrimidine dehydrogenase | 207216 | trypsin |
| 551 | Dihydropyrimidine dehydrogenase | 207217 | trypsin |
| 552 | Dihydropyrimidine dehydrogenase | 207218 | |
| 553 | Dihydropyrimidine dehydrogenase | 207219 | chymotrypsin |
| 554 | Dihydropyrimidine dehydrogenase | 207220 | trypsin |
| 555 | Dihydropyrimidine dehydrogenase | 207221 | trypsin |
| 556 | Dihydropyrimidine dehydrogenase | 207222 | |
| 557 | Dihydropyrimidine dehydrogenase | 207223 | trypsin |
| 558 | Dihydropyrimidine dehydrogenase | 207224 | trypsin |
| 559 | Dihydropyrimidine dehydrogenase | 207225 | chymotrypsin |
| 560 | Dihydropyrimidine dehydrogenase | 207226 | trypsin |
| 561 | Dihydropyrimidine dehydrogenase | 207227 | chymotrypsin, trypsin |
| 562 | Dihydropyrimidine dehydrogenase | 207228 | trypsin |
| 563 | Dihydropyrimidine dehydrogenase | 207229 | |
| 564 | Dihydropyrimidine dehydrogenase | 207230 | trypsin |
| 565 | Dihydropyrimidine dehydrogenase | 207231 | trypsin |
| 566 | Dihydropyrimidine dehydrogenase | 207232 | chymotrypsin, trypsin |
| 567 | Dihydropyrimidine dehydrogenase | 207233 | trypsin |
| 568 | Dihydropyrimidine dehydrogenase | 207234 | |
| 569 | Dihydropyrimidine dehydrogenase | 207235 | trypsin |
| 570 | Dihydropyrimidine dehydrogenase | 207236 | |
| 571 | Dihydropyrimidine dehydrogenase | 207237 | |
| 572 | Dihydropyrimidine dehydrogenase | 207238 | chymotrypsin |
| 573 | Dihydropyrimidine dehydrogenase | 207239 | trypsin |
| 574 | Dihydropyrimidine dehydrogenase | 207240 | chymotrypsin |
| 575 | Dihydropyrimidine dehydrogenase | 207241 | chymotrypsin, trypsin |
| 576 | Dihydropyrimidine dehydrogenase | 207242 | chymotrypsin |
| 577 | Dihydropyrimidine dehydrogenase | 207243 | trypsin |
| 578 | Dihydropyrimidine dehydrogenase | 207244 | chymotrypsin, trypsin |
| 579 | Dihydropyrimidine dehydrogenase | 207245 | chymotrypsin, trypsin |
| 580 | Discoidin-1 subunit a | 207246 | trypsin |
| 581 | Discoidin-1 subunit a | 207247 | |
| 582 | Discoidin-1 subunit a | 207248 | chymotrypsin |
| 583 | Dissimilatory copper-containing nitritereductase | 207249 | chymotrypsin, trypsin |
| 584 | D-lactate dehydrogenase | - | |
| 585 | D-lactate dehydrogenase | 207250 | trypsin |
| 586 | D-lactate dehydrogenase | 207251 | trypsin |
| 587 | D-lactate dehydrogenase | 207252 | |
| 588 | D-lactate dehydrogenase | 207253 | chymotrypsin, trypsin |
| 589 | D-lactate dehydrogenase | 207254 | chymotrypsin, trypsin |
| 590 | D-lactate dehydrogenase | 207255 | chymotrypsin, trypsin |
| 591 | Dna damage-binding protein 1 | - | |
| 592 | Dna damage-binding protein 1 | 207256 | |
| 593 | Dna damage-binding protein 1 | 207257 | |
| 594 | Dna damage-binding protein 1 | 207258 | |
| 595 | Dna damage-binding protein 1 | 207259 | |
| 596 | Dna damage-binding protein 1 | 207260 | |
| 597 | Dna damage-binding protein 1 | 207261 | |
| 598 | Dna damage-binding protein 1 | 207262 | chymotrypsin, trypsin |
| 599 | Dna damage-binding protein 1 | 207263 | |
| 600 | Dna damage-binding protein 1 | 207264 | trypsin |
| 601 | Dna damage-binding protein 1 | 207265 | trypsin |
| 602 | Dna damage-binding protein 1 | 207266 | chymotrypsin |
| 603 | Dna damage-binding protein 1 | 207267 | |
| 604 | Dna damage-binding protein 1 | 207268 | chymotrypsin, trypsin |
| 605 | Dna damage-binding protein 1 | 207269 | |
| 606 | Dna damage-binding protein 1 | 207270 | chymotrypsin, trypsin |
| 607 | Dna damage-binding protein 1 | 207271 | trypsin |
| 608 | Dna damage-binding protein 1 | 207272 | trypsin |
| 609 | Dna damage-binding protein 1 | 207273 | chymotrypsin, trypsin |
| 610 | Dna damage-binding protein 1 | 207274 | chymotrypsin |
| 611 | Dna damage-binding protein 1 | 207275 | |
| 612 | Dna damage-binding protein 1 | 207276 | chymotrypsin, trypsin |
| 613 | Dna damage-binding protein 1 | 207277 | chymotrypsin, trypsin |
| 614 | Dna gyrase b | - | |
| 615 | Dna gyrase b | 207278 | |
| 616 | Dna gyrase b | 207279 | trypsin |
| 617 | Dna gyrase b | 207280 | |
| 618 | Dna gyrase b | 207281 | trypsin |
| 619 | Dna gyrase b | 207282 | chymotrypsin |
| 620 | Dna gyrase b | 207283 | |
| 621 | Dna gyrase b | 207284 | |
| 622 | Dna gyrase b | 207285 | trypsin |
| 623 | Dna gyrase b | 207286 | |
| 624 | Dna gyrase b | 207287 | chymotrypsin |
| 625 | Dna gyrase b | 207288 | chymotrypsin, trypsin |
| 626 | Dna ligase | 207289 | |
| 627 | Dna ligase | 207290 | trypsin |
| 628 | Dna ligase | 207291 | trypsin |
| 629 | Dna ligase | 207292 | chymotrypsin, trypsin |
| 630 | Dna ligase | 207293 | chymotrypsin |
| 631 | Dna mismatch repair protein muts | - | |
| 632 | Dna mismatch repair protein muts | - | |
| 633 | Dna mismatch repair protein muts | 207294 | trypsin |
| 634 | Dna mismatch repair protein muts | 207295 | chymotrypsin |
| 635 | Dna mismatch repair protein muts | 207296 | chymotrypsin |
| 636 | Dna mismatch repair protein muts | 207297 | trypsin |
| 637 | Dna mismatch repair protein muts | 207298 | trypsin |
| 638 | Dna polymerase | - | chymotrypsin |
| 639 | Dna polymerase | - | trypsin |
| 640 | Dna polymerase | 207299 | |
| 641 | Dna polymerase | 207300 | trypsin |
| 642 | Dna polymerase | 207301 | trypsin |
| 643 | Dna polymerase | 207302 | trypsin |
| 644 | Dna polymerase | 207303 | chymotrypsin |
| 645 | Dna polymerase | 207304 | chymotrypsin, trypsin |
| 646 | Dna polymerase | 207305 | trypsin |
| 647 | Dna polymerase | 207306 | chymotrypsin, trypsin |
| 648 | Dna polymerase alpha subunit b | 207307 | |
| 649 | Dna polymerase alpha subunit b | 207308 | trypsin |
| 650 | Dna polymerase alpha subunit b | 207309 | chymotrypsin |
| 651 | Dna polymerase alpha subunit b | 207310 | |
| 652 | Dna polymerase alpha subunit b | 207311 | |
| 653 | Dna polymerase alpha subunit b | 207312 | chymotrypsin, trypsin |
| 654 | Dna polymerase alpha subunit b | 207313 | |
| 655 | Dna polymerase alpha subunit b | 207314 | chymotrypsin |
| 656 | Dna polymerase alpha subunit b | 207315 | chymotrypsin |
| 657 | Dna polymerase alpha subunit b | 207316 | chymotrypsin, trypsin |
| 658 | Dna polymerase eta | - | |
| 659 | Dna polymerase eta | 207317 | |
| 660 | Dna polymerase eta | 207318 | chymotrypsin |
| 661 | Dna polymerase eta | 207319 | chymotrypsin, trypsin |
| 662 | Dna polymerase eta | 207320 | chymotrypsin, trypsin |
| 663 | Dna polymerase eta | 207321 | chymotrypsin |
| 664 | Dna polymerase i | - | |
| 665 | Dna polymerase i | - | |
| 666 | Dna polymerase i | 207322 | trypsin |
| 667 | Dna primase | - | |
| 668 | Dna primase | 207323 | trypsin |
| 669 | Dna primase | 207324 | |
| 670 | Dna primase | 207325 | trypsin |
| 671 | Dna primase | 207326 | |
| 672 | Dna primase | 207327 | trypsin |
| 673 | Dna primase | 207328 | trypsin |
| 674 | Dna primase | 207329 | chymotrypsin, trypsin |
| 675 | Dna primase/helicase | - | |
| 676 | Dna primase/helicase | 207330 | |
| 677 | Dna primase/helicase | 207331 | chymotrypsin |
| 678 | Dna primase/helicase | 207332 | trypsin |
| 679 | Dna primase/helicase | 207333 | chymotrypsin, trypsin |
| 680 | Dna primase/helicase | 207334 | trypsin |
| 681 | Dna primase/helicase | 207335 | chymotrypsin, trypsin |
| 682 | Dna primase/helicase | 207336 | |
| 683 | Dna primase/helicase | 207337 | chymotrypsin, trypsin |
| 684 | Dna primase/helicase | 207338 | chymotrypsin, trypsin |
| 685 | Dna primase/helicase | 207339 | chymotrypsin, trypsin |
| 686 | Dna topoisomerase 2 | - | |
| 687 | Dna topoisomerase 2 | - | |
| 688 | Dna topoisomerase 2 | - | trypsin |
| 689 | Dna topoisomerase 2 | 207340 | chymotrypsin |
| 690 | Dna topoisomerase 2 | 207341 | chymotrypsin, trypsin |
| 691 | Dna topoisomerase 2 | 207342 | chymotrypsin |
| 692 | Dna topoisomerase 2 | 207343 | chymotrypsin, trypsin |
| 693 | Dna topoisomerase 2 | 207344 | trypsin |
| 694 | Dna topoisomerase 2 | 207345 | chymotrypsin |
| 695 | Dna topoisomerase 2 | 207346 | trypsin |
| 696 | Dna topoisomerase 2 | 207347 | chymotrypsin, trypsin |
| 697 | Dna topoisomerase 2 | 207348 | chymotrypsin, trypsin |
| 698 | Dna topoisomerase i | 207349 | |
| 699 | Dna topoisomerase i | 207350 | trypsin |
| 700 | Dna topoisomerase i | 207351 | trypsin |
| 701 | Dna topoisomerase ii, alpha isozyme | - | |
| 702 | Dna topoisomerase ii, alpha isozyme | 207352 | |
| 703 | Dna topoisomerase ii, alpha isozyme | 207353 | |
| 704 | Dna topoisomerase ii, alpha isozyme | 207354 | |
| 705 | Dna topoisomerase ii, alpha isozyme | 207355 | chymotrypsin, trypsin |
| 706 | Dna topoisomerase ii, alpha isozyme | 207356 | trypsin |
| 707 | Dna topoisomerase ii, alpha isozyme | 207357 | trypsin |
| 708 | Dna topoisomerase ii, alpha isozyme | 207358 | chymotrypsin |
| 709 | Dna topoisomerase ii, alpha isozyme | 207359 | chymotrypsin, trypsin |
| 710 | Dna topoisomerase vi a subunit | 207360 | |
| 711 | Dna topoisomerase vi a subunit | 207361 | |
| 712 | Dna topoisomerase vi a subunit | 207362 | |
| 713 | Dna topoisomerase vi a subunit | 207363 | |
| 714 | Dna topoisomerase vi a subunit | 207364 | trypsin |
| 715 | Dna topoisomerase vi a subunit | 207365 | trypsin |
| 716 | Dna-3-methyladenine glycosylase 2 | 207366 | trypsin |
| 717 | Dna-binding response regulator mtra | 207367 | trypsin |
| 718 | Dna-directed rna polymerase beta chain | 207368 | |
| 719 | Dna-directed rna polymerase beta chain | 207369 | |
| 720 | Dna-directed rna polymerase beta chain | 207370 | chymotrypsin, trypsin |
| 721 | Dna-directed rna polymerase beta chain | 207371 | chymotrypsin |
| 722 | Dna-directed rna polymerase beta chain | 207372 | chymotrypsin, trypsin |
| 723 | Dna-directed rna polymerase beta chain | 207373 | trypsin |
| 724 | Dna-directed rna polymerase beta chain | 207374 | chymotrypsin |
| 725 | Dna-directed rna polymerase beta chain | 207375 | trypsin |
| 726 | Dna-directed rna polymerase ii 14.2 kda polypeptide | 207376 | |
| 727 | Dna-directed rna polymerase ii 14.2 kda polypeptide | 207377 | trypsin |
| 728 | Dna-directed rna polymerase, subunit e' (rpoe1) | 207378 | |
| 729 | Dna-directed rna polymerase, subunit e' (rpoe1) | 207379 | chymotrypsin |
| 730 | Dna-directed rna polymerases i, ii, and iii 27 kdapolypeptide | - | |
| 731 | Dna-directed rna polymerases i, ii, and iii 27 kdapolypeptide | 207380 | chymotrypsin |
| 732 | Dna-directed rna polymerases i, ii, and iii 27 kdapolypeptide | 207381 | |
| 733 | Dna-directed rna polymerases i, ii, and iii 27 kdapolypeptide | 207382 | chymotrypsin, trypsin |
| 734 | Dna-directed rna polymerases i, ii, and iii 27 kdapolypeptide | 207383 | trypsin |
| 735 | Drosophila neuroglian | 207384 | chymotrypsin, trypsin |
| 736 | Dystroglycan | 207385 | trypsin |
| 737 | Dystrophin | 207386 | |
| 738 | Dystrophin | 207387 | |
| 739 | Dystrophin | 207388 | chymotrypsin, trypsin |
| 740 | Dystrophin | 207389 | trypsin |
| 741 | Dystrophin | 207390 | |
| 742 | Dystrophin | 207391 | chymotrypsin, trypsin |
| 743 | Dystrophin | 207392 | |
| 744 | E2a dna-binding protein | 207393 | |
| 745 | E2a dna-binding protein | 207394 | chymotrypsin, trypsin |
| 746 | E3 sumo-protein ligase siz1 | 207395 | |
| 747 | E3 sumo-protein ligase siz1 | 207396 | trypsin |
| 748 | E3 sumo-protein ligase siz1 | 207397 | trypsin |
| 749 | Early switch protein xol-1 2.2k splice form | 207398 | trypsin |
| 750 | Egf-like module containing mucin-like hormonereceptor-like 2 precursor | 207399 | |
| 751 | Egf-like module containing mucin-like hormonereceptor-like 2 precursor | 207400 | chymotrypsin, trypsin |
| 752 | Elongation factor 1-gamma 1 | 207401 | |
| 753 | Elongation factor 1-gamma 1 | 207402 | |
| 754 | Elongation factor g | 207403 | |
| 755 | Elongation factor g | 207404 | trypsin |
| 756 | Elongation factor g | 207405 | |
| 757 | Elongation factor g | 207406 | chymotrypsin |
| 758 | Elongation factor g | 207407 | |
| 759 | Elongation factor g | 207408 | chymotrypsin |
| 760 | Elongation factor g | 207409 | trypsin |
| 761 | Elongation factor g | 207410 | chymotrypsin, trypsin |
| 762 | Elongation factor g | 207411 | chymotrypsin |
| 763 | Elongation factor g | 207412 | chymotrypsin, trypsin |
| 764 | Elongation factor p | 207413 | chymotrypsin |
| 765 | Elongation factor ts | 207414 | |
| 766 | Elongation factor ts | 207415 | trypsin |
| 767 | Elongation factor ts | 207416 | trypsin |
| 768 | Elongation factor tu (ef-tu) | 207417 | chymotrypsin, trypsin |
| 769 | Endoglucanase | 207418 | |
| 770 | Endonuclease pi-scei | 207419 | |
| 771 | Endonuclease pi-scei | 207420 | chymotrypsin |
| 772 | Endonuclease pi-scei | 207421 | trypsin |
| 773 | Endonuclease pi-scei | 207422 | trypsin |
| 774 | Endonuclease pi-scei | 207423 | |
| 775 | Endonuclease pi-scei | 207424 | trypsin |
| 776 | Endonuclease pi-scei | 207425 | trypsin |
| 777 | Endonuclease pi-scei | 207426 | trypsin |
| 778 | Endonuclease pi-scei | 207427 | chymotrypsin |
| 779 | Enterobactin synthetase component f | 207428 | chymotrypsin |
| 780 | Enterobactin synthetase component f | 207429 | chymotrypsin |
| 781 | Enterobactin synthetase component f | 207430 | trypsin |
| 782 | Enterobactin synthetase component f | 207431 | trypsin |
| 783 | Enterobactin synthetase component f | 207432 | chymotrypsin |
| 784 | Enterobactin synthetase component f | 207433 | |
| 785 | Enterobactin synthetase component f | 207434 | chymotrypsin |
| 786 | Enterobactin synthetase component f | 207435 | |
| 787 | Enterobactin synthetase component f | 207436 | chymotrypsin, trypsin |
| 788 | Enterochelin esterase | 207437 | chymotrypsin |
| 789 | Epo receptor | - | |
| 790 | Epo receptor | 207438 | |
| 791 | Erythrocyte binding antigen region ii | 207439 | |
| 792 | Erythrocyte binding antigen region ii | 207440 | chymotrypsin |
| 793 | Erythrocyte binding antigen region ii | 207441 | |
| 794 | Erythrocyte binding antigen region ii | 207442 | |
| 795 | Erythrocyte binding antigen region ii | 207443 | trypsin |
| 796 | E-selectin | 207444 | |
| 797 | Esterase esta | - | |
| 798 | Esterase esta | 207445 | |
| 799 | Esterase esta | 207446 | chymotrypsin |
| 800 | Eukaryotic peptide chain release factor gtp-bindingsubunit | 207447 | trypsin |
| 801 | Exonuclease i | - | trypsin |
| 802 | Exonuclease i | 207448 | trypsin |
| 803 | Fasciclin i | - | |
| 804 | Fasciclin i | 207449 | chymotrypsin |
| 805 | Fibrillin-1 | 207450 | |
| 806 | Fibrillin-1 | 207451 | trypsin |
| 807 | Fibrillin-1 | 207452 | trypsin |
| 808 | Fibrillin-1 | 207453 | |
| 809 | Fibrillin-1 | 207454 | trypsin |
| 810 | Fibronectin | 207455 | |
| 811 | Fibronectin | 207456 | chymotrypsin |
| 812 | Fibronectin | 207457 | chymotrypsin |
| 813 | Flagellar hook protein flge | 207458 | |
| 814 | Flagellar hook protein flge | 207459 | chymotrypsin |
| 815 | Flagellar hook protein flge | 207460 | chymotrypsin |
| 816 | Flagellar hook protein flge | 207461 | |
| 817 | Flagellar hook protein flge | 207462 | chymotrypsin |
| 818 | Flagellar hook protein flge | 207463 | |
| 819 | Flagellar hook protein flge | 207464 | |
| 820 | Flavohemoprotein | 207465 | chymotrypsin, trypsin |
| 821 | Focal adhesion kinase 1 | 207466 | chymotrypsin, trypsin |
| 822 | Folc bifunctional protein | 207467 | |
| 823 | Folc bifunctional protein | 207468 | trypsin |
| 824 | Folc bifunctional protein | 207469 | trypsin |
| 825 | Folc bifunctional protein | 207470 | |
| 826 | Folc bifunctional protein | 207471 | |
| 827 | Folc bifunctional protein | 207472 | |
| 828 | Folc bifunctional protein | 207473 | |
| 829 | Folc bifunctional protein | 207474 | chymotrypsin, trypsin |
| 830 | Follistatin | 207475 | chymotrypsin, trypsin |
| 831 | Formate dehydrogenase (large subunit) | - | chymotrypsin |
| 832 | Formate dehydrogenase (large subunit) | 207476 | |
| 833 | Formate dehydrogenase (large subunit) | 207477 | chymotrypsin |
| 834 | Formate dehydrogenase (large subunit) | 207478 | trypsin |
| 835 | Formate dehydrogenase (large subunit) | 207479 | chymotrypsin, trypsin |
| 836 | Formate dehydrogenase (large subunit) | 207480 | chymotrypsin, trypsin |
| 837 | Formate dehydrogenase (large subunit) | 207481 | |
| 838 | Formate dehydrogenase (large subunit) | 207482 | chymotrypsin |
| 839 | Formate dehydrogenase (large subunit) | 207483 | trypsin |
| 840 | Formate dehydrogenase (large subunit) | 207484 | chymotrypsin, trypsin |
| 841 | Formate dehydrogenase (large subunit) | 207485 | chymotrypsin, trypsin |
| 842 | Formate dehydrogenase (large subunit) | 207486 | chymotrypsin, trypsin |
| 843 | Formate dehydrogenase (large subunit) | 207487 | chymotrypsin, trypsin |
| 844 | Formate dehydrogenase, nitrate-inducible major subunit | 207488 | chymotrypsin, trypsin |
| 845 | Formate dehydrogenase, nitrate-inducible, major subunit | 207489 | |
| 846 | Formate dehydrogenase, nitrate-inducible, major subunit | 207490 | |
| 847 | Formate dehydrogenase, nitrate-inducible, major subunit | 207491 | chymotrypsin |
| 848 | Formate dehydrogenase, nitrate-inducible, major subunit | 207492 | |
| 849 | Formate dehydrogenase, nitrate-inducible, major subunit | 207493 | trypsin |
| 850 | Formate dehydrogenase, nitrate-inducible, major subunit | 207494 | trypsin |
| 851 | Formate dehydrogenase, nitrate-inducible, major subunit | 207495 | trypsin |
| 852 | Formate dehydrogenase, nitrate-inducible, major subunit | 207496 | chymotrypsin, trypsin |
| 853 | Formate dehydrogenase, nitrate-inducible, major subunit | 207497 | trypsin |
| 854 | Formate dehydrogenase, nitrate-inducible, major subunit | 207498 | chymotrypsin, trypsin |
| 855 | Formate dehydrogenase, nitrate-inducible, major subunit | 207499 | trypsin |
| 856 | Formate dehydrogenase, nitrate-inducible, major subunit | 207500 | trypsin |
| 857 | Formate dehydrogenase, nitrate-inducible, major subunit | 207501 | chymotrypsin, trypsin |
| 858 | Fumarvlacetoacetate hydrolase | 207502 | |
| 859 | Galactose oxidase | - | |
| 860 | Galactose oxidase | - | chymotrypsin |
| 861 | Galactose oxidase | - | |
| 862 | Galactose oxidase | - | trypsin |
| 863 | Galactose oxidase | - | |
| 864 | Galactose oxidase | - | |
| 865 | Galactose oxidase | 207503 | trypsin |
| 866 | Galactose oxidase | 207504 | trypsin |
| 867 | Galactose oxidase | 207505 | trypsin |
| 868 | Galactose oxidase | 207506 | |
| 869 | Galactose oxidase | 207507 | |
| 870 | Galactose oxidase | 207508 | chymotrypsin |
| 871 | Galactose oxidase | 207509 | |
| 872 | Galactose oxidase | 207510 | chymotrypsin |
| 873 | Galactose oxidase | 207511 | chymotrypsin |
| 874 | Galactose oxidase | 207512 | |
| 875 | Galactose oxidase | 207513 | trypsin |
| 876 | Galactose oxidase | 207514 | chymotrypsin |
| 877 | Galactose oxidase | 207515 | chymotrypsin |
| 878 | Galactose oxidase | 207516 | trypsin |
| 879 | Galactose oxidase | 207517 | |
| 880 | Galactose oxidase | 207518 | |
| 881 | Galactose oxidase | 207519 | chymotrypsin, trypsin |
| 882 | Galactose oxidase | 207520 | chymotrypsin, trypsin |
| 883 | Galactose oxidase | 207521 | chymotrypsin, trypsin |
| 884 | Galactose oxidase | 207522 | chymotrypsin |
| 885 | Galactose oxidase | 207523 | chymotrypsin |
| 886 | Galactose oxidase | 207524 | chymotrypsin |
| 887 | Galactose oxidase | 207525 | chymotrypsin, trypsin |
| 888 | Galactose oxidase | 207526 | chymotrypsin, trypsin |
| 889 | Gamma b-crystallin | 207527 | chymotrypsin |
| 890 | Gamma-delta t-cell receptor | 207528 | trypsin |
| 891 | Gelation factor | - | |
| 892 | Gelation factor | 207529 | |
| 893 | Gelation factor | 207530 | |
| 894 | Gelation factor | 207531 | trypsin |
| 895 | Gene activator apha | 207532 | trypsin |
| 896 | Gingipain r | 207533 | trypsin |
| 897 | Glucodextranase | 207534 | |
| 898 | Glucodextranase | 207535 | |
| 899 | Glucodextranase | 207536 | chymotrypsin |
| 900 | Glucosamine-fructose-6-phosphate aminotransferase | - | chymotrypsin |
| 901 | Glucosamine-fructose-6-phosphate aminotransferase | 207537 | trypsin |
| 902 | Glucosamine-fructose-6-phosphate aminotransferase | 207538 | |
| 903 | Glucosamine-fructose-6-phosphate aminotransferase | 207539 | |
| 904 | Glucosamine-fructose-6-phosphate aminotransferase | 207540 | trypsin |
| 905 | Glucosamine-fructose-6-phosphate aminotransferase | 207541 | |
| 906 | Glucosamine-fructose-6-phosphate aminotransferase | 207542 | trypsin |
| 907 | Glucosamine-fructose-6-phosphate aminotransferase | 207543 | trypsin |
| 908 | Glucosamine-fructose-6-phosphate aminotransferase | 207544 | trypsin |
| 909 | Glucosamine-fructose-6-phosphate aminotransferase | 207545 | chymotrypsin, trypsin |
| 910 | Glucosamine-fructose-6-phosphate aminotransferase | 207546 | chymotrypsin, trypsin |
| 911 | Glucose-1-phosphate adenylyltransferase smallsubunit | 207547 | |
| 912 | Glucose-1-phosphate adenylyltransferase smallsubunit | 207548 | chymotrypsin, trypsin |
| 913 | Glucose-6-phosphate isomerase | - | trypsin |
| 914 | Glucose-6-phosphate isomerase | - | |
| 915 | Glucose-6-phosphate isomerase | 207549 | trypsin |
| 916 | Glucose-6-phosphate isomerase | 207550 | chymotrypsin |
| 917 | Glucose-6-phosphate isomerase, conjectural | 207551 | chymotrypsin, trypsin |
| 918 | Glutamate dehydrogenase | 207552 | chymotrypsin |
| 919 | Glutamate dehydrogenase | 207553 | trypsin |
| 920 | Glutamate receptor interacting protein | 207554 | chymotrypsin |
| 921 | Glutamate synthase [nadph] large chain | 207555 | |
| 922 | Glutamate synthase [nadph] large chain | 207556 | |
| 923 | Glutamate synthase [nadph] large chain | 207557 | trypsin |
| 924 | Glutamate synthase [nadph] large chain | 207558 | trypsin |
| 925 | Glutamate synthase [nadph] large chain | 207559 | trypsin |
| 926 | Glutamate synthase [nadph] large chain | 207560 | chymotrypsin, trypsin |
| 927 | Glutamate synthase [nadph] large chain | 207561 | chymotrypsin, trypsin |
| 928 | Glutamine synthetase | 207562 | chymotrypsin, trypsin |
| 929 | Glutamine synthetase | 207563 | chymotrypsin, trypsin |
| 930 | Glutamyl-trna synthetase | 207564 | |
| 931 | Glutamyl-trna synthetase | 207565 | chymotrypsin, trypsin |
| 932 | Glutamyl-trna synthetase | 207566 | chymotrypsin |
| 933 | Glutamyl-trna synthetase | 207567 | trypsin |
| 934 | Glutamyl-trna synthetase | 207568 | |
| 935 | Glutamyl-trna synthetase | 207569 | chymotrypsin, trypsin |
| 936 | Glutamyl-trna synthetase | 207570 | trypsin |
| 937 | Glutamyl-trna synthetase | 207571 | trypsin |
| 938 | Glutaredoxin 2 | 207572 | trypsin |
| 939 | Glutathione s-transferase | 207573 | |
| 940 | Glutathione s-transferase | 207574 | chymotrypsin |
| 941 | Glutathione s-transferase | 207575 | |
| 942 | Glutathione s-transferase 1-6 | 207576 | chymotrypsin, trypsin |
| 943 | Glutathione s-transferase a1 | 207577 | chymotrypsin, trypsin |
| 944 | Glutathione s-transferase i | - | |
| 945 | Glutathione s-transferase i | 207578 | trypsin |
| 946 | Glutathione synthetase | 207579 | trypsin |
| 947 | Glutathione transferase gst1-4 | 207580 | |
| 948 | Glutathione transferase gst1-4 | 207581 | chymotrypsin |
| 949 | Glutathione transferase sigma class | 207582 | chymotrypsin, trypsin |
| 950 | Glycerol-3-phosphate dehydrogenase [nad(p)+] | 207583 | chymotrypsin |
| 951 | Glycine cleavage system transcriptional repressor, putative | 207584 | |
| 952 | Glycolipid-anchored surface protein 2 | 207585 | trypsin |
| 953 | Glycolipid-anchored surface protein 2 | 207586 | chymotrypsin, trypsin |
| 954 | Glycyl-trna synthetase | - | chymotrypsin, trypsin |
| 955 | Glycyl-trna synthetase | 207587 | |
| 956 | Glycyl-trna synthetase | 207588 | |
| 957 | Glycyl-trna synthetase | 207589 | trypsin |
| 958 | Glycyl-trna synthetase | 207590 | trypsin |
| 959 | Glycyl-trna synthetase | 207591 | trypsin |
| 960 | Glycyl-trna synthetase | 207592 | trypsin |
| 961 | Glycyl-trna synthetase | 207593 | chymotrypsin |
| 962 | Glycyl-trna synthetase | 207594 | chymotrypsin, trypsin |
| 963 | "Glycyl-trna synthetase | | |
| " | 207595 | chymotry psin, trypsin | |
| 964 | Growth hormone receptor | 207596 | |
| 965 | Growth hormone receptor | 207597 | chymotrypsin |
| 966 | Harmonin | 207598 | thrombin, trypsin |
| 967 | Hasr protein | 207599 | trypsin |
| 968 | Hasr protein | 207600 | |
| 969 | Hemin transport protein hems | 207601 | trypsin |
| 970 | Hemin transport protein hems | 207602 | trypsin |
| 971 | Hemin transport protein hems | 207603 | |
| 972 | Hemoglobin | 207604 | |
| 973 | Hemolytic lectin cel-iii | 207605 | trypsin |
| 974 | Hepatocyte nuclear factor 6 | 207606 | chymotrypsin, trypsin |
| 975 | Histidyl-trna synthetase | 207607 | chymotrypsin, trypsin |
| 976 | Hnh homing endonuclease | 207608 | trypsin |
| 977 | Hnh homing endonuclease | 207609 | trypsin |
| 978 | Hnh homing endonuclease | 207610 | trypsin |
| 979 | Homoserine dehydrogenase | 207611 | |
| 980 | Homoserine kinase | 207612 | |
| 981 | Homoserine kinase | 207613 | |
| 982 | Homoserine kinase | 207614 | |
| 983 | Homoserine kinase | 207615 | trypsin |
| 984 | Hth-type transcriptional regulator mqsa (ygit/b3021) | 207616 | |
| 985 | Hth-type transcriptional repressor vvoa | 207617 | |
| 986 | Hth-type transcriptional repressor yvoa | 207618 | trypsin |
| 987 | Human igg1 Middle Hinge Linker | 207619 | |
| 988 | Human igg1 Upper Hinge Linker | 207620 | |
| 989 | Human igg3 Middle Hinge Linker | 207621 | |
| 990 | Human igg3m15 Middle Hinge Linker | 207622 | |
| 991 | Human igg4 Lower Hinge Linker | 207623 | |
| 992 | Human igg4 Middle Hinge Linker | 207624 | |
| 993 | Human igg4 Upper Hinge Linker | 207625 | |
| 994 | Hybrid cluster protein | 207626 | |
| 995 | Hybrid cluster protein | 207627 | trypsin |
| 996 | Hybrid cluster protein | 207628 | chymotrypsin |
| 997 | Hybrid cluster protein | 207629 | chymotrypsin |
| 998 | Hybrid cluster protein | 207630 | chymotrypsin, trypsin |
| 999 | Hypothetical conserved protein, gk1056 | 207631 | trypsin |
| 1000 | Hypothetical membrane spanning protein | 207632 | chymotrypsin |
| 1001 | Hypothetical methylmalonyl-coa decarboxylase alpha subunit | 207633 | |
| 1002 | Hypothetical methylmalonyl-coa decarboxylase alpha subunit | 207634 | chymotrypsin |
| 1003 | Hypothetical methylmalonyl-coa decarboxylase alpha subunit | 207635 | chymotrypsin, trypsin |
| 1004 | Hypothetical methylmalonyl-coa decarboxylase alpha subunit | 207636 | trypsin |
| 1005 | Hypothetical methylmalonyl-coa decarboxylase alpha subunit | 207637 | |
| 1006 | Hypothetical methylmalonyl-coa decarboxylase alpha subunit | 207638 | chymotrypsin |
| 1007 | Hypothetical methylmalonyl-coa decarboxylase alpha subunit | 207639 | chymotrypsin, trypsin |
| 1008 | Hypothetical protein | - | |
| 1009 | Hypothetical protein | 207640 | |
| 1010 | Hypothetical protein ape0525 | - | |
| 1011 | Hypothetical protein ape0525 | 207641 | chymotrypsin, trypsin |
| 1012 | Hypothetical protein loc449832 | 207642 | |
| 1013 | Hypothetical protein loc449832 | 207643 | |
| 1014 | Hypothetical protein pa4388 | 207644 | chymotrypsin, trypsin |
| 1015 | Hypothetical protein pa5201 | - | |
| 1016 | Hypothetical protein pa5201 | - | |
| 1017 | Hypothetical protein pa5201 | - | trypsin |
| 1018 | Hypothetical protein pa5201 | 207645 | |
| 1019 | Hypothetical protein pa5201 | 207646 | trypsin |
| 1020 | Hypothetical protein pa5201 | 207647 | trypsin |
| 1021 | Hypothetical protein pa5201 | 207648 | |
| 1022 | Hypothetical protein pa5201 | 207649 | |
| 1023 | Hypothetical protein pa5201 | 207650 | chymotrypsin |
| 1024 | Hypothetical protein pa5201 | 207651 | trypsin |
| 1025 | Hypothetical protein pa5201 | 207652 | chymotrypsin, trypsin |
| 1026 | Hypothetical protein pa5201 | 207653 | trypsin |
| 1027 | Hypothetical protein pa5201 | 207654 | trypsin |
| 1028 | Hypothetical protein pa5201 | 207655 | trypsin |
| 1029 | Hypothetical protein pa5201 | 207656 | chymotrypsin, trypsin |
| 1030 | Hypothetical protein pa5201 | 207657 | thrombin, trypsin |
| 1031 | Hypothetical protein pa5201 | 207658 | trypsin |
| 1032 | Hypothetical protein pa5201 | 207659 | |
| 1033 | Hypothetical protein pa5201 | 207660 | chymotrypsin, trypsin |
| 1034 | Hypothetical protein pa5201 | 207661 | trypsin |
| 1035 | Hypothetical protein pa5201 | 207662 | chymotrypsin |
| 1036 | Hypothetical protein pa5201 | 207663 | chymotrypsin, trypsin |
| 1037 | Hypothetical protein pa5201 | 207664 | chymotrypsin, trypsin |
| 1038 | Hypothetical protein ph0495 | - | |
| 1039 | Hypothetical protein ph0495 | 207665 | |
| 1040 | Hypothetical protein ph0495 | 207666 | chymotrypsin, trypsin |
| 1041 | Hypothetical protein ph0495 | 207667 | chymotrypsin, trypsin |
| 1042 | Hypothetical protein ph0495 | 207668 | trypsin |
| 1043 | Hypothetical protein ph0510 | 207669 | chymotrypsin |
| 1044 | Hypothetical protein ph0510 | 207670 | chymotrypsin |
| 1045 | Hypothetical protein ph1313 | 207671 | |
| 1046 | Hypothetical protein ph1313 | 207672 | chymotrypsin, trypsin |
| 1047 | Hypothetical protein slr0953 | 207673 | |
| 1048 | Hypothetical protein slr0953 | 207674 | chymotrypsin |
| 1049 | Hypothetical protein slr0953 | 207675 | chymotrypsin |
| 1050 | Hypothetical protein slr0953 | 207676 | chymotrypsin |
| 1051 | Hypothetical protein slr0953 | 207677 | |
| 1052 | Hypothetical protein yigz | 207678 | trypsin |
| 1053 | Hypothetical protein yigz | 207679 | trypsin |
| 1054 | Hypothetical protein yjia | 207680 | |
| 1055 | Hypothetical protein yjia | 207681 | trypsin |
| 1056 | Hypothetical protein yjia | 207682 | |
| 1057 | Hypothetical protein yjia | 207683 | trypsin |
| 1058 | Hypothetical protein yjia | 207684 | chymotrypsin, trypsin |
| 1059 | Hypothetical trna/rrna methyltransferase yjfh | 207685 | |
| 1060 | Hypothetical trna/rrna methyltransferase yjfh | 207686 | chymotrypsin, trypsin |
| 1061 | Iclr transcriptional regulator | 207687 | chymotrypsin |
| 1062 | Iclr transcriptional regulator | 207688 | chymotrypsin, trypsin |
| 1063 | Iclr transcriptional regulator | 207689 | trypsin |
| 1064 | Iclr transcriptional regulator | 207690 | trypsin |
| 1065 | Integrase | 207691 | trypsin |
| 1066 | Interferon, alpha-inducible protein (clone ifi-15k) | 207692 | trypsin |
| 1067 | Interleukin-1 receptor, type i | - | |
| 1068 | Interleukin-1 receptor, type i | 207693 | |
| 1069 | Interleukin-1 receptor, type i | 207694 | chymotrypsin |
| 1070 | Interleukin-1 receptor, type i | 207695 | chymotrypsin |
| 1071 | Interleukin-12 subunit p40 | - | chymotrypsin |
| 1072 | Interleukin-12 subunit p40 | 207696 | |
| 1073 | Interleukin-12 subunit p40 | 207697 | trypsin |
| 1074 | Interleukin-12 subunit p40 | 207698 | trypsin |
| 1075 | Interleukin-12 subunit p40 | 207699 | |
| 1076 | Interleukin-12 subunit p40 | 207700 | chymotrypsin |
| 1077 | Interleukin-12 subunit p40 | 207701 | |
| 1078 | Interleukin-12 subunit p40 | 207702 | chymotrypsin, trypsin |
| 1079 | Interleukin-2 receptor alpha chain | 207703 | chymotrypsin |
| 1080 | Interleukin-2 receptor alpha chain | 207704 | trypsin |
| 1081 | Internalin b | - | |
| 1082 | Internalin b | 207705 | |
| 1083 | Internalin b | 207706 | |
| 1084 | Internalin b | 207707 | |
| 1085 | Internalin b | 207708 | chymotrypsin |
| 1086 | Internalin b | 207709 | trypsin |
| 1087 | Internalin b | 207710 | trypsin |
| 1088 | Internalin b | 207711 | chymotrypsin, trypsin |
| 1089 | Internalin b | 207712 | trypsin |
| 1090 | Internalin b | 207713 | chymotrypsin, trypsin |
| 1091 | Internalin b | 207714 | trypsin |
| 1092 | Internalin b | 207715 | |
| 1093 | Internalin b | 207716 | trypsin |
| 1094 | Intimin | - | |
| 1095 | Intimin | 207717 | chymotrypsin |
| 1096 | Intimin | 207718 | trypsin |
| 1097 | Intimin | 207719 | |
| 1098 | Intron-encoded dna endonuclease i-anii | 207720 | chymotrypsin |
| 1099 | Intron-encoded dna endonuclease i-anii | 207721 | chymotrypsin, trypsin |
| 1100 | Invasin | - | trypsin |
| 1101 | Invasin | 207722 | |
| 1102 | Invasin | 207723 | chymotrypsin, trypsin |
| 1103 | Invasin | 207724 | chymotrypsin |
| 1104 | Invasin | 207725 | |
| 1105 | Invasin | 207726 | |
| 1106 | Invasin | 207727 | |
| 1107 | Invasin | 207728 | chymotrypsin |
| 1108 | Invasin | 207729 | trypsin |
| 1109 | Invasin | 207730 | chymotrypsin, trypsin |
| 1110 | Invasin | 207731 | chymotrypsin |
| 1111 | Invasin | 207732 | chymotrypsin, trypsin |
| 1112 | Invasin | 207733 | trypsin |
| 1113 | Iron hydrogenase 1 | - | |
| 1114 | Iron hydrogenase 1 | 207734 | trypsin |
| 1115 | Iron hydrogenase 1 | 207735 | trypsin |
| 1116 | Iron hydrogenase 1 | 207736 | trypsin |
| 1117 | Iron hydrogenase 1 | 207737 | |
| 1118 | Iron hydrogenase 1 | 207738 | trypsin |
| 1119 | Iron hydrogenase 1 | 207739 | |
| 1120 | Iron hydrogenase 1 | 207740 | trypsin |
| 1121 | Iron hydrogenase 1 | 207741 | trypsin |
| 1122 | Iron hydrogenase 1 | 207742 | chymotrypsin |
| 1123 | Iron hydrogenase 1 | 207743 | |
| 1124 | Iron hydrogenase 1 | 207744 | |
| 1125 | Iron hydrogenase 1 | 207745 | chymotrypsin, trypsin |
| 1126 | Iron hydrogenase 1 | 207746 | chymotrypsin, trypsin |
| 1127 | Iron transport protein | 207747 | |
| 1128 | Isoflavanone 4'-o-methyltransferase | 207748 | chymotrypsin |
| 1129 | Isoflavanone 4'-o-methyltransferase | 207749 | trypsin |
| 1130 | Junctional adhesion molecule 1 | 207750 | trypsin |
| 1131 | Junctional adhesion molecule 1 | 207751 | trypsin |
| 1132 | Junctional adhesion molecule 1 | 207752 | |
| 1133 | Kanamycin nucleotidyltransferase | 207753 | chymotrypsin |
| 1134 | Kanamycin nucleotidyltransferase | 207754 | chymotrypsin |
| 1135 | Kanamycin nucleotidyltransferase | 207755 | |
| 1136 | Kanamycin nucleotidyltransferase | 207756 | |
| 1137 | Kelch-like protein 11 | 207757 | trypsin |
| 1138 | Kexin | - | |
| 1139 | Kexin | 207758 | |
| 1140 | Kexin | 207759 | trypsin |
| 1141 | Kexin | 207760 | |
| 1142 | Kexin | 207761 | chymotrypsin, trypsin |
| 1143 | Kexin | 207762 | chymotrypsin |
| 1144 | Kexin | 207763 | chymotrypsin, trypsin |
| 1145 | Kexin | 207764 | chymotrypsin, trypsin |
| 1146 | Ku70 | 207765 | trypsin |
| 1147 | Ku70 | 207766 | trypsin |
| 1148 | Ku70 | 207767 | trypsin |
| 1149 | Ku70 | 207768 | chymotrypsin, trypsin |
| 1150 | Ku80 | 207769 | trypsin |
| 1151 | Laccase-1 | 207770 | chymotrypsin |
| 1152 | Laccase-1 | 207771 | chymotrypsin |
| 1153 | Laccase-1 | 207772 | chymotrypsin |
| 1154 | Laccase-1 | 207773 | chymotrypsin, thrombin, trypsin |
| 1155 | Laminin | - | trypsin |
| 1156 | L-aspartate dehydrogenase | - | |
| 1157 | L-aspartate dehydrogenase | 207774 | chymotrypsin, trypsin |
| 1158 | L-aspartate dehydrogenase | 207775 | |
| 1159 | Leucine dehydrogenase | 207776 | |
| 1160 | Leucine dehydrogenase | 207777 | |
| 1161 | Light chain of hvhel10 antibody fragment (fab) | 207778 | trypsin |
| 1162 | Lin2111 protein | 207779 | trypsin |
| 1163 | Lin2111 protein | 207780 | trypsin |
| 1164 | Lipopolysaccharide-responsive and beige-like anchor protein | 207781 | chymotrypsin, thrombin, trypsin |
| 1165 | Lipopolysaccharide-responsive and beige-like anchor protein | 207782 | |
| 1166 | Lipovitellin (Iv-1n, Iv-1c) | 207783 | |
| 1167 | Lipovitellin (Iv-1n, Iv-1c) | 207784 | trypsin |
| 1168 | Lipovitellin (Iv-1n, Iv-1c) | 207785 | trypsin |
| 1169 | Lipovitellin (Iv-1n, Iv-1c) | 207786 | trypsin |
| 1170 | Lipovitellin (Iv-1n, Iv-1c) | 207787 | chymotrypsin, trypsin |
| 1171 | Lipoxygenase-1 | 207788 | |
| 1172 | Lipoxygenase-1 | 207789 | chymotrypsin |
| 1173 | Low affinity immunoglobulin gamma fc region receptor ii-a | 207790 | chymotrypsin |
| 1174 | Luciferase | 207791 | chymotrypsin, trypsin |
| 1175 | Lysr-type regulatory protein | 207792 | trypsin |
| 1176 | Macrolide-specific efflux protein maca | - | |
| 1177 | Macrolide-specific efflux protein maca | 207793 | chymotrypsin |
| 1178 | Macrolide-specific efflux protein maca | 207794 | |
| 1179 | Magnesium transporter, putative | 207795 | chymotrypsin |
| 1180 | Main hemagglutinin component | 207796 | |
| 1181 | Major centromere autoantigen b | 207797 | chymotrypsin, trypsin |
| 1182 | Major surface antigen p30 | 207798 | |
| 1183 | Major surface antigen p30 | 207799 | trypsin |
| 1184 | Major vault protein | 207800 | chymotrypsin, trypsin |
| 1185 | Major vault protein | 207801 | trypsin |
| 1186 | Maltose phosphorylase | 207802 | trypsin |
| 1187 | Maltose phosphorylase | 207803 | |
| 1188 | Maltose phosphorylase | 207804 | |
| 1189 | Maltose phosphorylase | 207805 | chymotrypsin, trypsin |
| 1190 | Maltose phosphorylase | 207806 | chymotrypsin, trypsin |
| 1191 | Manganese-dependent inorganic pyrophosphatase | 207807 | |
| 1192 | Manganese-dependent inorganic pyrophosphatase | 207808 | trypsin |
| 1193 | Mannan-binding lectin | 207809 | |
| 1194 | Mannan-binding lectin | 207810 | chymotrypsin |
| 1195 | Mannan-binding lectin | 207811 | |
| 1196 | Mannitol dehydrogenase | - | |
| 1197 | Mannitol dehydrogenase | 207812 | |
| 1198 | Membrane cofactor protein | - | trypsin |
| 1199 | Membrane cofactor protein | 207813 | chymotrypsin, trypsin |
| 1200 | Membrane-associated prostaglandin e synthase-2 | 207814 | chymotrypsin |
| 1201 | Membrane-associated prostaglandin e synthase-2 | 207815 | |
| 1202 | Membrane-associated prostaglandin e synthase-2 | 207816 | chymotrypsin |
| 1203 | Membrane-associated prostaglandin e synthase-2 | 207817 | trypsin |
| 1204 | Membrane-associated prostaglandin e synthase-2 | 207818 | chymotrypsin, trypsin |
| 1205 | Membrane-bound Ivtic murein transglycosylase a | 207819 | chymotrypsin, trypsin |
| 1206 | Methionyl-trna synthetase | 207820 | |
| 1207 | Methyl-accepting chemotaxis protein | - | |
| 1208 | Methyl-accepting chemotaxis protein | 207821 | trypsin |
| 1209 | Methyl-accepting chemotaxis protein | 207822 | trypsin |
| 1210 | Methyl-accepting chemotaxis protein | 207823 | chymotrypsin |
| 1211 | Methyl-coenzyme m reductase | 207824 | |
| 1212 | Methyl-coenzyme m reductase | 207825 | |
| 1213 | Methyl-coenzyme m reductase | 207826 | chymotrypsin |
| 1214 | Methyl-coenzyme m reductase | 207827 | trypsin |
| 1215 | Methylene tetrahydromethanopterin dehydrogenase | 207828 | |
| 1216 | Methylene tetrahydromethanopterin dehydrogenase | 207829 | trypsin |
| 1217 | Mg2+ transporter mgte | 207830 | chymotrypsin |
| 1218 | Mg2+ transporter mqte | 207831 | chymotrypsin |
| 1219 | Mg2+ transporter mgte | 207832 | chymotrypsin, trypsin |
| 1220 | Mitochondrial aconitase | 207833 | chymotrypsin, trypsin |
| 1221 | Mitochondrial aconitase | 207834 | chymotrypsin, trypsin |
| 1222 | Modification methylase taqi | - | |
| 1223 | Modification methylase taqi | - | |
| 1224 | Modification methylase taqi | 207835 | |
| 1225 | Modification methylase taqi | 207836 | |
| 1226 | Modification methylase taqi | 207837 | |
| 1227 | Modification methylase taqi | 207838 | chymotrypsin, trypsin |
| 1228 | Modification methylase taqi | 207839 | trypsin |
| 1229 | Modification methylase taqi | 207840 | trypsin |
| 1230 | Modification methylase taqi | 207841 | trypsin |
| 1231 | Modification methylase taqi | 207842 | chymotrypsin, trypsin |
| 1232 | Multidrug-efflux transporter 1 regulator | 207843 | |
| 1233 | Muramoyl-pentapeptide carboxypeptidase | 207844 | |
| 1234 | Mutl | 207845 | |
| 1235 | Mutl | 207846 | |
| 1236 | Mutl | 207847 | |
| 1237 | Mutl | 207848 | chymotrypsin, trypsin |
| 1238 | Mutl | 207849 | trypsin |
| 1239 | Mutl | 207850 | chymotrypsin, trypsin |
| 1240 | Mutl | 207851 | chymotrypsin |
| 1241 | Mutl | 207852 | trypsin |
| 1242 | Mutl | 207853 | chymotrypsin, trypsin |
| 1243 | Mutm (fpg) protein | 207854 | chymotrypsin, trypsin |
| 1244 | Mutm (fpg) protein | 207855 | chymotrypsin, trypsin |
| 1245 | Mutm (fpg) protein | 207856 | chymotrypsin, trypsin |
| 1246 | Mutm (fpg) protein | 207857 | chymotrypsin, trypsin |
| 1247 | Myotubularin-related protein 2 | - | |
| 1248 | Myotubularin-related protein 2 | 207858 | trypsin |
| 1249 | Myotubularin-related protein 2 | 207859 | chymotrypsin |
| 1250 | Myotubularin-related protein 2 | 207860 | |
| 1251 | Myotubularin-related protein 2 | 207861 | trypsin |
| 1252 | Myotubularin-related protein 2 | 207862 | chymotrypsin, trypsin |
| 1253 | N utilization substance protein a | - | |
| 1254 | N utilization substance protein a | 207863 | |
| 1255 | N utilization substance protein a | 207864 | trypsin |
| 1256 | N utilization substance protein a | 207865 | chymotrypsin, trypsin |
| 1257 | N-acetylglucosamine kinase | - | |
| 1258 | N-acetylglucosamine kinase | - | |
| 1259 | N-acetylglucosamine kinase | 207866 | trypsin |
| 1260 | N-acyl-d-glutamate deacylase | 207867 | |
| 1261 | N-acyl-d-glutamate deacylase | 207868 | |
| 1262 | N-acyl-d-glutamate deacylase | 207869 | |
| 1263 | N-acyl-d-glutamate deacylase | 207870 | |
| 1264 | N-acyl-d-glutamate deacylase | 207871 | chymotrypsin |
| 1265 | N-acyl-d-glutamate deacylase | 207872 | chymotrypsin |
| 1266 | N-acyl-d-glutamate deacylase | 207873 | trypsin |
| 1267 | Nad-dependent malic enzyme | 207874 | |
| 1268 | Nad-dependent malic enzyme | 207875 | chymotrypsin, trypsin |
| 1269 | Nadh peroxidase | - | |
| 1270 | Nadh peroxidase | - | |
| 1271 | Nadh peroxidase | - | |
| 1272 | Nadh peroxidase | 207876 | |
| 1273 | Nadh peroxidase | 207877 | |
| 1274 | Nadh peroxidase | 207878 | |
| 1275 | Nadh peroxidase | 207879 | |
| 1276 | Nadh peroxidase | 207880 | trypsin |
| 1277 | Nadh peroxidase | 207881 | chymotrypsin, trypsin |
| 1278 | Nadh pyrophosphatase | 207882 | chymotrypsin |
| 1279 | Naphthalene 1,2-dioxvgenase alpha subunit | 207883 | |
| 1280 | Naphthalene 1,2-dioxygenase alpha subunit | 207884 | chymotrypsin, trypsin |
| 1281 | Nedd8-activating enzyme e1 catalytic subunit | 207885 | chymotrypsin, trypsin |
| 1282 | Nedd8-activating enzyme e1 regulatory subunit | 207886 | chymotrypsin |
| 1283 | Nedd8-activating enzyme e1 regulatory subunit | 207887 | |
| 1284 | Nedd8-activating enzyme e1 regulatory subunit | 207888 | trypsin |
| 1285 | Nei endonuclease viii-like 1 | 207889 | trypsin |
| 1286 | Nei endonuclease viii-like 1 | 207890 | chymotrypsin, thrombin, trypsin |
| 1287 | Nei endonuclease viii-like 1 | 207891 | |
| 1288 | Nei endonuclease viii-like 1 | 207892 | chymotrypsin, thrombin, trypsin |
| 1289 | Neural cell adhesion molecule 2 | 207893 | chymotrypsin, trypsin |
| 1290 | Neural cell adhesion molecule 2 | 207894 | chymotrypsin |
| 1291 | Neural cell adhesion molecule 2 | 207895 | chymotrypsin |
| 1292 | Neural cell adhesion molecule 2 | 207896 | chymotrypsin, trypsin |
| 1293 | Neural cell adhesion molecule 2 | 207897 | |
| 1294 | Neuroplastin | 207898 | chymotrypsin |
| 1295 | Neuroplastin | 207899 | trypsin |
| 1296 | Neuroplastin | 207900 | trypsin |
| 1297 | Neutrophil cytosol factor 1 | 207901 | chymotrypsin |
| 1298 | Nickel responsive regulator | 207902 | |
| 1299 | Nifu-like protein 2, chloroplast | 207903 | |
| 1300 | Nitric oxide reductase | - | |
| 1301 | Nitric oxide reductase | 207904 | |
| 1302 | Nitric oxide reductase | 207905 | chymotrypsin, trypsin |
| 1303 | Nitric oxide reductase | 207906 | |
| 1304 | Nitric oxide reductase | 207907 | trypsin |
| 1305 | Nitric oxide reductase | 207908 | trypsin |
| 1306 | Nk receptor | 207909 | chymotrypsin |
| 1307 | Nuclear factor of activated t-cells, cytoplasmic2 | 207910 | |
| 1308 | Nucleolin rbd12 | 207911 | trypsin |
| 1309 | O-glcnacase naqj | 207912 | |
| 1310 | Orange carotenoid protein | - | |
| 1311 | Orange carotenoid protein | 207913 | trypsin |
| 1312 | Orange carotenoid protein | 207914 | chymotrypsin, trypsin |
| 1313 | Orn/lys/arg decarboxylase family protein | - | |
| 1314 | Orn/lys/arg decarboxylase family protein | 207915 | |
| 1315 | Orn/lys/arg decarboxylase family protein | 207916 | chymotrypsin |
| 1316 | Orn/lys/arg decarboxylase family protein | 207917 | chymotrypsin |
| 1317 | Orn/lys/arg decarboxylase family protein | 207918 | |
| 1318 | Orn/lys/arg decarboxylase family protein | 207919 | chymotrypsin, trypsin |
| 1319 | Orn/lys/arg decarboxylase family protein | 207920 | chymotrypsin |
| 1320 | Orn/lys/arg decarboxylase family protein | 207921 | trypsin |
| 1321 | Osteoclast-stimulating factor 1 | 207922 | |
| 1322 | Oxygen-independent coproporphyrinogen iii oxidase | 207923 | |
| 1323 | Oxygen-independent coproporphyrinogen iii oxidase | 207924 | |
| 1324 | Oxygen-independent coproporphyrinogen iii oxidase | 207925 | |
| 1325 | Oxygen-independent coproporphyrinogen iii oxidase | 207926 | trypsin |
| 1326 | Oxygen-independent coproporphyrinogen iii oxidase | 207927 | |
| 1327 | Oxygen-independent coproporphyrinogen iii oxidase | 207928 | chymotrypsin |
| 1328 | Oxygen-independent coproporphyrinogen iii oxidase | 207929 | |
| 1329 | Oxygen-independent coproporphyrinogen iii oxidase | 207930 | |
| 1330 | Oxygen-independent coproporphyrinogen iii oxidase | 207931 | chymotrypsin, trypsin |
| 1331 | Oxygen-independent coproporphyrinogen iii oxidase | 207932 | |
| 1332 | Paraneoplastic encephalomyelitis antigen hud | 207933 | trypsin |
| 1333 | Paraneoplastic encephalomyelitis antigen hud | 207934 | chymotrypsin |
| 1334 | Penicillin binding protein 4 | 207935 | trypsin |
| 1335 | Penicillin binding protein 4 | 207936 | |
| 1336 | Penicillin binding protein 4 | 207937 | trypsin |
| 1337 | Penicillin binding protein 4 | 207938 | |
| 1338 | Penicillin binding protein 4 | 207939 | trypsin |
| 1339 | Penicillin binding protein 4 | 207940 | chymotrypsin, trypsin |
| 1340 | Penicillin binding protein 4 | 207941 | chymotrypsin, trypsin |
| 1341 | Peptide-n(4)-(n-acetyl-beta-d-glucosaminyl)asparagineamidase f | - | |
| 1342 | Peptide-n(4)-(n-acetyl-beta-d-glucosaminyl)asparagineamidase f | 207942 | chymotrypsin, trypsin |
| 1343 | Peptide-n(4)-(n-acetyl-beta-d-glucosaminyl)asparagineamidase f | 207943 | chymotrypsin, trypsin |
| 1344 | Peptide-n(4)-(n-acetyl-beta-d-glucosaminyl)asparagineamidase f | 207944 | chymotrypsin, trypsin |
| 1345 | Peroxisomal primary amine oxidase | 207945 | trypsin |
| 1346 | Peroxisomal primary amine oxidase | 207946 | chymotrypsin, trypsin |
| 1347 | Peroxisome biogenesis factor 1 | 207947 | |
| 1348 | Pesticidial crystal protein cry2aa | 207948 | |
| 1349 | Pesticidial crystal protein crv2aa | 207949 | trypsin |
| 1350 | Pesticidial crystal protein crv2aa | 207950 | chymotrypsin |
| 1351 | Phase 1 flagellin | - | |
| 1352 | Phase 1 flagellin | 207951 | |
| 1353 | Phase 1 flagellin | 207952 | |
| 1354 | Phase 1 flagellin | 207953 | trypsin |
| 1355 | Phase 1 flagellin | 207954 | |
| 1356 | Phase 1 flagellin | 207955 | trypsin |
| 1357 | Phase 1 flagellin | 207956 | trypsin |
| 1358 | Phase 1 flagellin | 207957 | trypsin |
| 1359 | Phase 1 flagellin | 207958 | |
| 1360 | Phase 1 flagellin | 207959 | |
| 1361 | Phase 1 flagellin | 207960 | trypsin |
| 1362 | Phase 1 flagellin | 207961 | chymotrypsin, trypsin |
| 1363 | Phase 1 flagellin | 207962 | chymotrypsin, trypsin |
| 1364 | Phenylalanyl-trna synthetase beta chain | - | |
| 1365 | Phenylalanyl-trna synthetase beta chain | 207963 | |
| 1366 | Phenylalanyl-trna synthetase beta chain | 207964 | |
| 1367 | Phenylalanyl-trna synthetase beta chain | 207965 | |
| 1368 | Phenylalanyl-trna synthetase beta chain | 207966 | trypsin |
| 1369 | Phenylalanyl-trna synthetase beta chain | 207967 | chymotrypsin |
| 1370 | Phenylalanyl-trna synthetase beta chain | 207968 | trypsin |
| 1371 | Phenylalanyl-trna synthetase beta chain | 207969 | |
| 1372 | Phenylalanyl-trna synthetase beta chain | 207970 | |
| 1373 | Phenylalanyl-trna synthetase beta chain | 207971 | |
| 1374 | Phenylalanyl-trna synthetase beta chain | 207972 | chymotrypsin, trypsin |
| 1375 | Phenylalanyl-trna synthetase beta chain | 207973 | chymotrypsin |
| 1376 | Phenylalanyl-trna synthetase beta chain | 207974 | chymotrypsin, trypsin |
| 1377 | Phenylalanyl-trna synthetase beta chain | 207975 | chymotrypsin, trypsin |
| 1378 | Phosphatase | 207976 | |
| 1379 | Phosphatase | 207977 | |
| 1380 | Phosphatase | 207978 | trypsin |
| 1381 | Phosphatidylinositol transfer protein sec14p | - | chymotrypsin |
| 1382 | Phosphatidylinositol transfer protein sec14p | 207979 | chymotrypsin |
| 1383 | Phosphatidylinositol transfer protein sec14p | 207980 | chymotrypsin |
| 1384 | Phosphatidylserine synthase | 207981 | chymotrypsin |
| 1385 | Phosphatidylserine synthase | 207982 | trypsin |
| 1386 | Phosphatidylserine synthase | 207983 | chymotrypsin, trypsin |
| 1387 | Phosphoglycolate phosphatase | 207984 | trypsin |
| 1388 | Phosphoglycolate phosphatase | 207985 | chymotrypsin |
| 1389 | Phosphoglycolate phosphatase | 207986 | trypsin |
| 1390 | Phosphoglycolate phosphatase | 207987 | chymotrypsin, trypsin |
| 1391 | Phospholipase d | 207988 | trypsin |
| 1392 | Phospholipase d | 207989 | |
| 1393 | Phospholipase d | 207990 | |
| 1394 | Phosphoribosylamine--glycine ligase | 207991 | trypsin |
| 1395 | Phosphoribosylamine--glycine ligase | 207992 | chymotrypsin, trypsin |
| 1396 | Phosphotransferase system, enzyme i | 207993 | trypsin |
| 1397 | Photosystem ii d1 protease | 207994 | |
| 1398 | Photosystem ii d1 protease | 207995 | |
| 1399 | Photosystem ii d1 protease | 207996 | trypsin |
| 1400 | Photosystem ii d1 protease | 207997 | trypsin |
| 1401 | Photosystem ii d1 protease | 207998 | trypsin |
| 1402 | Phthalate dioxygenase reductase | 207999 | chymotrypsin, trypsin |
| 1403 | P-hydroxybenzoate hydroxylase | - | |
| 1404 | P-hydroxybenzoate hydroxylase | - | |
| 1405 | P-hydroxybenzoate hydroxylase | - | trypsin |
| 1406 | P-hydroxybenzoate hydroxylase | 208000 | |
| 1407 | P-hydroxybenzoate hydroxylase | 208001 | chymotrypsin |
| 1408 | P-hydroxybenzoate hydroxylase | 208002 | chymotrypsin |
| 1409 | P-hydroxybenzoate hydroxylase | 208003 | |
| 1410 | P-hydroxybenzoate hydroxylase | 208004 | trypsin |
| 1411 | P-hydroxybenzoate hydroxylase | 208005 | |
| 1412 | P-hydroxybenzoate hydroxylase | 208006 | |
| 1413 | P-hydroxybenzoate hydroxylase | 208007 | chymotrypsin |
| 1414 | P-hydroxybenzoate hydroxylase | 208008 | |
| 1415 | P-hydroxybenzoate hydroxylase | 208009 | chymotrypsin |
| 1416 | P-hydroxybenzoate hydroxylase | 208010 | |
| 1417 | P-hydroxybenzoate hydroxylase | 208011 | trypsin |
| 1418 | P-hydroxybenzoate hydroxylase | 208012 | trypsin |
| 1419 | P-hydroxybenzoate hydroxylase | 208013 | chymotrypsin, trypsin |
| 1420 | P-hydroxybenzoate hydroxylase | 208014 | |
| 1421 | P-hvdroxvbenzoate hydroxylase | 208015 | chymotrypsin |
| 1422 | P-hydroxybenzoate hydroxylase | 208016 | |
| 1423 | Phytase | - | |
| 1424 | Phytase | - | |
| 1425 | Phytase | 208017 | trypsin |
| 1426 | Phytase | 208018 | |
| 1427 | Phytase | 208019 | |
| 1428 | Phytase | 208020 | trypsin |
| 1429 | Phytase | 208021 | |
| 1430 | Phytase | 208022 | |
| 1431 | Phytase | 208023 | trypsin |
| 1432 | Phytase | 208024 | chymotrypsin, trypsin |
| 1433 | Pirin | - | trypsin |
| 1434 | Pirin | - | |
| 1435 | Pirin | 208025 | |
| 1436 | Pirin | 208026 | trypsin |
| 1437 | Pirin | 208027 | trypsin |
| 1438 | Pirin | 208028 | |
| 1439 | Pirin | 208029 | trypsin |
| 1440 | Pirin | 208030 | chymotrypsin, trypsin |
| 1441 | Poly(a) polymerase | 208031 | |
| 1442 | Poly(a) polymerase | 208032 | chymotrypsin |
| 1443 | Poly(a) polymerase | 208033 | trypsin |
| 1444 | Poly(a) polymerase | 208034 | chymotrypsin, trypsin |
| 1445 | Poly(a) polymerase | 208035 | chymotrypsin, trypsin |
| 1446 | Poly(a) polymerase | 208036 | |
| 1447 | Poly(a) polymerase | 208037 | chymotrypsin, trypsin |
| 1448 | Poly(a) polymerase | 208038 | trypsin |
| 1449 | Poly(a) polymerase | 208039 | chymotrypsin |
| 1450 | Poly(a) polymerase | 208040 | |
| 1451 | Poly(a) polymerase | 208041 | trypsin |
| 1452 | Poly(a) polymerase | 208042 | chymotrypsin, trypsin |
| 1453 | Poly(rc)-binding protein 2 | 208043 | |
| 1454 | Polymerase x | 208044 | |
| 1455 | Polymerase x | 208045 | chymotrypsin |
| 1456 | Polypeptide n-acetylgalactosaminyltransferase 2 | 208046 | |
| 1457 | Polypeptide n-acetylgalactosaminyltransferase 2 | 208047 | trypsin |
| 1458 | Polyphosphate kinase | 208048 | |
| 1459 | Polyphosphate kinase | 208049 | chymotrypsin, trypsin |
| 1460 | Polyphosphate kinase | 208050 | chymotrypsin |
| 1461 | Polypyrimidine tract-binding protein | 208051 | chymotrypsin, trypsin |
| 1462 | Porcine pancreatic spasmolytic polypeptide | 208052 | chymotrypsin |
| 1463 | Possible 3-mercaptopyruvate sulfurtransferase | - | chymotrypsin |
| 1464 | Possible 3-mercaptopyruvate sulfurtransferase | - | chymotrypsin |
| 1465 | Possible 3-mercaptopyruvate sulfurtransferase | 208053 | trypsin |
| 1466 | Possible 3-mercaptopyruvate sulfurtransferase | 208054 | |
| 1467 | Possible 3-mercaptopyruvate sulfurtransferase | 208055 | chymotrypsin, trypsin |
| 1468 | Postsynaptic densitv protein 95 | 208056 | chymotrypsin |
| 1469 | Postsynaptic densitv protein 95 | 208057 | chymotrypsin, trypsin |
| 1470 | Predicted sugar phosphatases of the hadsuperfamily | - | |
| 1471 | Predicted sugar phosphatases of the hadsuperfamily | 208058 | |
| 1472 | Predicted sugar phosphatases of the hadsuperfamily | 208059 | trypsin |
| 1473 | Predicted sugar phosphatases of the hadsuperfamily | 208060 | |
| 1474 | Predicted sugar phosphatases of the hadsuperfamily | 208061 | trypsin |
| 1475 | Predicted sugar phosphatases of the hadsuperfamily | 208062 | |
| 1476 | Predicted sugar phosphatases of the hadsuperfamily | 208063 | trypsin |
| 1477 | Predicted sugar phosphatases of the hadsuperfamily | 208064 | chymotrypsin |
| 1478 | Predicted sugar phosphatases of the hadsuperfamily | 208065 | chymotrypsin |
| 1479 | Preprotein translocase seca | - | |
| 1480 | Preprotein translocase seca | - | |
| 1481 | Preprotein translocase seca | 208066 | trypsin |
| 1482 | Preprotein translocase seca | 208067 | |
| 1483 | Preprotein translocase seca | 208068 | chymotrypsin, trypsin |
| 1484 | Preprotein translocase seca | 208069 | trypsin |
| 1485 | Preprotein translocase seca | 208070 | |
| 1486 | Preprotein translocase seca | 208071 | |
| 1487 | Preprotein translocase seca | 208072 | |
| 1488 | Preprotein translocase seca | 208073 | chymotrypsin |
| 1489 | Preprotein translocase seca | 208074 | chymotrypsin, trypsin |
| 1490 | Preprotein translocase seca | 208075 | trypsin |
| 1491 | Preprotein translocase seca | 208076 | chymotrypsin |
| 1492 | Preprotein translocase seca | 208077 | |
| 1493 | Preprotein translocase seca | 208078 | trypsin |
| 1494 | Preprotein translocase seca | 208079 | chymotrypsin |
| 1495 | Preprotein translocase seca | 208080 | trypsin |
| 1496 | Preprotein translocase seca | 208081 | chymotrypsin, trypsin |
| 1497 | Preprotein translocase seca | 208082 | chymotrypsin, trypsin |
| 1498 | Prfa | - | |
| 1499 | Probable 16s rrna-processing protein rimm | 208083 | chymotrypsin, trypsin |
| 1500 | Probable biphenyl-2,3-diol 1,2-dioxygenase bphc | 208084 | chymotrypsin, trypsin |
| 1501 | Probable chorismate mutase | - | |
| 1502 | Probable chorismate mutase | 208085 | |
| 1503 | Probable chorismate mutase | 208086 | |
| 1504 | Probable ferredoxin-dependent nitrite reductase nira | - | |
| 1505 | Probable ferredoxin-dependent nitrite reductase nira | - | chymotrypsin |
| 1506 | Probable ferredoxin-dependent nitrite reductase nira | 208087 | |
| 1507 | Probable ferredoxin-dependent nitrite reductase nira | 208088 | trypsin |
| 1508 | Probable ferredoxin-dependent nitrite reductase nira | 208089 | |
| 1509 | Probable ferredoxin-dependent nitrite reductase nira | 208090 | chymotrypsin |
| 1510 | Probable ferredoxin-dependent nitrite reductase nira | 208091 | chymotrypsin, trypsin |
| 1511 | Probable ferredoxin-dependent nitrite reductase nira | 208092 | chymotrypsin, trypsin |
| 1512 | Probable ferredoxin-dependent nitrite reductase nira | 208093 | chymotrypsin, trypsin |
| 1513 | Probable ferredoxin-dependent nitrite reductase nira | 208094 | |
| 1514 | Probable ferredoxin-dependent nitrite reductase nira | 208095 | |
| 1515 | Probable ferredoxin-dependent nitrite reductase nira | 208096 | |
| 1516 | Probable ferredoxin-dependent nitrite reductase nira | 208097 | trypsin |
| 1517 | Probable ferredoxin-dependent nitrite reductase nira | 208098 | |
| 1518 | Probable galactokinase | 208099 | trypsin |
| 1519 | Probable galactokinase | 208100 | |
| 1520 | Probable galactokinase | 208101 | trypsin |
| 1521 | Probable galactokinase | 208102 | chymotrypsin, trypsin |
| 1522 | Probable galactokinase | 208103 | trypsin |
| 1523 | Probable galactokinase | 208104 | trypsin |
| 1524 | Probable galactokinase | 208105 | chymotrypsin |
| 1525 | Probable galactokinase | 208106 | chymotrypsin, trypsin |
| 1526 | Probable galactokinase | 208107 | |
| 1527 | Probable galactokinase | 208108 | chymotrypsin |
| 1528 | Probable galactokinase | 208109 | |
| 1529 | Probable galactokinase | 208110 | chymotrypsin, trypsin |
| 1530 | Probable glutathione s-transferase | 208111 | |
| 1531 | Probable gst-related protein | 208112 | trypsin |
| 1532 | Probable hpr(ser) kinase/phosphatase | 208113 | chymotrypsin |
| 1533 | Probable thiosulfate sulfurtransferase | 208114 | |
| 1534 | Probable thiosulfate sulfurtransferase | 208115 | trypsin |
| 1535 | Probable thiosulfate sulfurtransferase | 208116 | |
| 1536 | Probable thiosulfate sulfurtransferase | 208117 | chymotrypsin |
| 1537 | Probable thiosulfate sulfurtransferase | 208118 | chymotrypsin |
| 1538 | Probable thiosulfate sulfurtransferase | 208119 | trypsin |
| 1539 | Probable thiosulfate sulfurtransferase | 208120 | trypsin |
| 1540 | Probable thiosulfate sulfurtransferase | 208121 | chymotrypsin, trypsin |
| 1541 | Probable trna pseudouridine synthase d | 208122 | |
| 1542 | Probable trna pseudouridine synthase d | 208123 | trypsin |
| 1543 | Probable trna pseudouridine synthase d | 208124 | trypsin |
| 1544 | Probable trna pseudouridine synthase d | 208125 | chymotrypsin, trypsin |
| 1545 | Probable trna pseudouridine synthase d | 208126 | chymotrypsin |
| 1546 | Probable trna pseudouridine synthase d | 208127 | chymotrypsin, trypsin |
| 1547 | Programed cell death protein 8 | - | trypsin |
| 1548 | Programed cell death protein 8 | - | |
| 1549 | Programed cell death protein 8 | 208128 | trypsin |
| 1550 | Programed cell death protein 8 | 208129 | trypsin |
| 1551 | Programed cell death protein 8 | 208130 | chymotrypsin |
| 1552 | Programed cell death protein 8 | 208131 | |
| 1553 | Programed cell death protein 8 | 208132 | |
| 1554 | Programed cell death protein 8 | 208133 | chymotrypsin, trypsin |
| 1555 | Programed cell death protein 8 | 208134 | trypsin |
| 1556 | Programed cell death protein 8 | 208135 | trypsin |
| 1557 | Programed cell death protein 8 | 208136 | trypsin |
| 1558 | Programed cell death protein 8 | 208137 | |
| 1559 | Programed cell death protein 8 | 208138 | |
| 1560 | Programed cell death protein 8 | 208139 | trypsin |
| 1561 | Programed cell death protein 8 | 208140 | trypsin |
| 1562 | Programed cell death protein 8 | 208141 | chymotrypsin, trypsin |
| 1563 | Programed cell death protein 8 | 208142 | chymotrypsin |
| 1564 | Programed cell death protein 8 | 208143 | trypsin |
| 1565 | Programed cell death protein 8 | 208144 | chymotrypsin |
| 1566 | Programed cell death protein 8 | 208145 | chymotrypsin, trypsin |
| 1567 | Proline oxidase | 208146 | chymotrypsin |
| 1568 | Prolyl-trna synthetase | 208147 | trypsin |
| 1569 | Prostaglandin g/h synthase 1 | - | |
| 1570 | Prostaglandin g/h synthase 1 | 208148 | |
| 1571 | Protease | 208149 | |
| 1572 | Protease | 208150 | |
| 1573 | Protease | 208151 | chymotrypsin, trypsin |
| 1574 | Protease degs | 208152 | chymotrypsin |
| 1575 | Protease degs | 208153 | trypsin |
| 1576 | Protease degs | 208154 | chymotrypsin, trypsin |
| 1577 | Protease degs | 208155 | |
| 1578 | Protease iii | - | |
| 1579 | Protease iii | - | trypsin |
| 1580 | Protease iii | 208156 | |
| 1581 | Protease iii | 208157 | |
| 1582 | Protease iii | 208158 | trypsin |
| 1583 | Protease iii | 208159 | |
| 1584 | Protease iii | 208160 | |
| 1585 | Protease iii | 208161 | |
| 1586 | Protease iii | 208162 | chymotrypsin, trypsin |
| 1587 | Protease iii | 208163 | trypsin |
| 1588 | Protease iii | 208164 | trypsin |
| 1589 | Protease iii | 208165 | chymotrypsin |
| 1590 | Protease iii | 208166 | |
| 1591 | Protease iii | 208167 | chymotrypsin, trypsin |
| 1592 | Protease iii | 208168 | chymotrypsin |
| 1593 | Protease iii | 208169 | trypsin |
| 1594 | Protease iii | 208170 | |
| 1595 | Protease iii | 208171 | trypsin |
| 1596 | Protease iii | 208172 | chymotrypsin |
| 1597 | Protease iii | 208173 | chymotrypsin, trypsin |
| 1598 | Protease iii | 208174 | chymotrypsin, trypsin |
| 1599 | Protease iii | 208175 | chymotrypsin, trypsin |
| 1600 | Protection of telomeres 1 | 208176 | |
| 1601 | Protection of telomeres 1 | 208177 | chymotrypsin, trypsin |
| 1602 | Protein (cd58) | 208178 | |
| 1603 | Protein (crp1) | 208179 | chymotrypsin, trypsin |
| 1604 | Protein (dna polymerase) | 208180 | |
| 1605 | Protein (dna polymerase) | 208181 | trypsin |
| 1606 | Protein (dna polymerase) | 208182 | trypsin |
| 1607 | Protein (electron transfer flavoprotein) | 208183 | |
| 1608 | Protein (electron transfer flavoprotein) | 208184 | trypsin |
| 1609 | Protein (ffh) | 208185 | chymotrypsin |
| 1610 | Protein (ffh) | 208186 | |
| 1611 | Protein (ffh) | 208187 | |
| 1612 | Protein (ffh) | 208188 | trypsin |
| 1613 | Protein (ffh) | 208189 | trypsin |
| 1614 | Protein (foki restriction endonuclease) | 208190 | trypsin |
| 1615 | Protein (foki restriction endonuclease) | 208191 | trypsin |
| 1616 | Protein (foki restriction endonuclease) | 208192 | chymotrypsin |
| 1617 | Protein (foki restriction endonuclease) | 208193 | trypsin |
| 1618 | Protein (foki restriction endonuclease) | 208194 | chymotrypsin, trypsin |
| 1619 | Protein (foki restriction endonuclease) | 208195 | chymotrypsin |
| 1620 | Protein (foki restriction endonuclease) | 208196 | |
| 1621 | Protein (foki restriction endonuclease) | 208197 | chymotrypsin, trypsin |
| 1622 | Protein (foki restriction endonuclease) | 208198 | chymotrypsin, trypsin |
| 1623 | Protein (neural cell adhesion molecule) | 208199 | chymotrypsin, trypsin |
| 1624 | Protein (neural cell adhesion molecule) | 208200 | |
| 1625 | Protein (neural cell adhesion molecule) | 208201 | chymotrypsin, trypsin |
| 1626 | Protein (nine-haem cytochrome c) | - | chymotrypsin |
| 1627 | Protein (nine-haem cytochrome c) | 208202 | |
| 1628 | Protein (nine-haem cytochrome c) | 208203 | chymotrypsin, trypsin |
| 1629 | Protein (nine-haem cytochrome c) | 208204 | trypsin |
| 1630 | Protein (nine-haem cytochrome c) | 208205 | chymotrypsin |
| 1631 | Protein (nine-haem cytochrome c) | 208206 | trypsin |
| 1632 | Protein (nine-haem cytochrome c) | 208207 | |
| 1633 | Protein (nine-haem cytochrome c) | 208208 | |
| 1634 | Protein (nine-haem cytochrome c) | 208209 | chymotrypsin, trypsin |
| 1635 | Protein (protease/helicase ns3) | 208210 | chymotrypsin |
| 1636 | Protein (protease/helicase ns3) | 208211 | |
| 1637 | Protein (protease/helicase ns3) | 208212 | |
| 1638 | Protein (protease/helicase ns3) | 208213 | chymotrypsin, trypsin |
| 1639 | Protein disulfide oxidoreductase | 208214 | trypsin |
| 1640 | Protein disulfide oxidoreductase | 208215 | trypsin |
| 1641 | Protein disulfide-isomerase a4 | 208216 | |
| 1642 | Protein kinase pkr | 208217 | trypsin |
| 1643 | Protein kinase pkr | 208218 | chymotrypsin, trypsin |
| 1644 | Protein tolb | - | |
| 1645 | Protein tolb | 208219 | |
| 1646 | Protein tolb | 208220 | |
| 1647 | Protein tolb | 208221 | chymotrypsin |
| 1648 | Protein tolb | 208222 | |
| 1649 | Protein tolb | 208223 | |
| 1650 | Protein tolb | 208224 | chymotrypsin, trypsin |
| 1651 | Protein translation elongation factor 1a | 208225 | |
| 1652 | Protein transport protein sec24 | - | trypsin |
| 1653 | Protein transport protein sec24 | 208226 | |
| 1654 | Protein transport protein sec24 | 208227 | trypsin |
| 1655 | Protein transport protein sec24 | 208228 | |
| 1656 | Protein transport protein sec24 | 208229 | |
| 1657 | Protein transport protein sec24 | 208230 | |
| 1658 | Protein transport protein sec24 | 208231 | chymotrypsin, trypsin |
| 1659 | Protein transport protein sec24 | 208232 | trypsin |
| 1660 | Protein transport protein sec24 | 208233 | |
| 1661 | Pseudouridine synthase cbf5 | - | |
| 1662 | Pseudouridine synthase cbf5 | 208234 | |
| 1663 | Pseudouridine synthase cbf5 | 208235 | |
| 1664 | Putative acetylglutamate synthase | 208236 | |
| 1665 | Putative acetylglutamate synthase | 208237 | chymotrypsin, trypsin |
| 1666 | Putative acetylglutamate synthase | 208238 | trypsin |
| 1667 | Putative family 31 glucosidase vici | 208239 | |
| 1668 | Putative family 31 glucosidase vici | 208240 | |
| 1669 | Putative family 31 glucosidase yici | 208241 | trypsin |
| 1670 | Putative glutathione transferase | 208242 | |
| 1671 | Putative glutathione transferase | 208243 | |
| 1672 | Putative glutathione transferase | 208244 | |
| 1673 | Putative gntr-family transcriptional regulator | 208245 | trypsin |
| 1674 | Putative gntr-family transcriptional regulator | 208246 | trypsin |
| 1675 | Putative gntr-family transcriptional regulator | 208247 | chymotrypsin, trypsin |
| 1676 | Putative hth-type transcriptional regulator ph0061 | 208248 | trypsin |
| 1677 | Putative hth-type transcriptional regulator ph1519 | 208249 | chymotrypsin |
| 1678 | Putative hth-type transcriptional regulator ph1519 | 208250 | chymotrypsin, trypsin |
| 1679 | Putative metallopeptidase | 208251 | trypsin |
| 1680 | Putative n-acetylmannosamine kinase | 208252 | |
| 1681 | Putative n-acetylmannosamine kinase | 208253 | |
| 1682 | Putative n-acetylmannosamine kinase | 208254 | |
| 1683 | Putative nadp oxidoreductase bf3122 | 208255 | |
| 1684 | Putative nadp oxidoreductase bf3122 | 208256 | chymotrypsin |
| 1685 | Putative nadp oxidoreductase bf3122 | 208257 | trypsin |
| 1686 | Putative nadp oxidoreductase bf3122 | 208258 | chymotrypsin, trypsin |
| 1687 | Putative oxidoreductase | 208259 | chymotrypsin, trypsin |
| 1688 | Putative secreted alpha-galactosidase | - | |
| 1689 | Putative secreted alpha-galactosidase | - | |
| 1690 | Putative secreted alpha-galactosidase | 208260 | |
| 1691 | Putative secreted alpha-galactosidase | 208261 | chymotrypsin |
| 1692 | Putative secreted alpha-galactosidase | 208262 | |
| 1693 | Putative tagatose-6-phosphate ketose/aldose isomerase | - | trypsin |
| 1694 | Putative tagatose-6-phosphate ketose/aldose isomerase | 208263 | chymotrypsin |
| 1695 | Putative tagatose-6-phosphate ketose/aldose isomerase | 208264 | chymotrypsin |
| 1696 | Putative tagatose-6-phosphate ketose/aldose isomerase | 208265 | chymotrypsin |
| 1697 | Putative transcriptional regulator gntr | 208266 | chymotrypsin |
| 1698 | Putative transcriptional repressor (tetr/acrr familv) | - | chymotrypsin, trypsin |
| 1699 | Putative transcriptional repressor (tetr/acrr family) | 208267 | chymotrypsin |
| 1700 | Putative uncharacterized protein | 208268 | |
| 1701 | Putative uncharacterized protein | 208269 | trypsin |
| 1702 | Putative uncharacterized protein | 208270 | chymotrypsin |
| 1703 | Putative uncharacterized protein | 208271 | |
| 1704 | Putative uncharacterized protein | 208272 | |
| 1705 | Putative uncharacterized protein | 208273 | trypsin |
| 1706 | Putative uncharacterized protein | 208274 | trypsin |
| 1707 | Putative uncharacterized protein | 208275 | chymotrypsin, trypsin |
| 1708 | Putative uncharacterized protein | 208276 | chymotrypsin |
| 1709 | Pyruvate decarboxylase | - | |
| 1710 | Pyruvate decarboxylase | 208277 | |
| 1711 | Pyruvate decarboxylase | 208278 | trypsin |
| 1712 | Pyruvate decarboxylase | 208279 | |
| 1713 | Pyruvate decarboxylase | 208280 | chymotrypsin |
| 1714 | Pyruvate decarboxylase | 208281 | trypsin |
| 1715 | Pyruvate dehydrogenase [lipoamide] kinase isozyme 2,mitochondrial | - | chymotrypsin |
| 1716 | Pyruvate dehydrogenase [lipoamide] kinase isozyme 2,mitochondrial | 208282 | chymotrypsin, trypsin |
| 1717 | Pyruvate dehydrogenase [lipoamide] kinase isozyme 2,mitochondrial | 208283 | |
| 1718 | Pyruvate dehydrogenase e1 component subunit beta, mitochondrial | 208284 | |
| 1719 | Pyruvate dehydrogenase e1 component subunit beta, mitochondrial | 208285 | chymotrypsin |
| 1720 | Pyruvate dehydrogenase e1 component subunit beta, mitochondrial | 208286 | chymotrypsin, trypsin |
| 1721 | Pyruvate phosphate dikinase | - | chymotrypsin |
| 1722 | Pyruvate phosphate dikinase | - | |
| 1723 | Pyruvate phosphate dikinase | 208287 | |
| 1724 | Pyruvate phosphate dikinase | 208288 | |
| 1725 | Pyruvate phosphate dikinase | 208289 | trypsin |
| 1726 | Pyruvate phosphate dikinase | 208290 | |
| 1727 | Pyruvate phosphate dikinase | 208291 | chymotrypsin |
| 1728 | Pyruvate phosphate dikinase | 208292 | |
| 1729 | Pyruvate phosphate dikinase | 208293 | trypsin |
| 1730 | Pyruvate phosphate dikinase | 208294 | trypsin |
| 1731 | Pyruvate phosphate dikinase | 208295 | |
| 1732 | Pyruvate phosphate dikinase | 208296 | |
| 1733 | Pyruvate-ferredoxin oxidoreductase | - | trypsin |
| 1734 | Pyruvate-ferredoxin oxidoreductase | 208297 | |
| 1735 | Pyruvate-ferredoxin oxidoreductase | 208298 | chymotrypsin, trypsin |
| 1736 | Pyruvate-ferredoxin oxidoreductase | 208299 | |
| 1737 | Pyruvate-ferredoxin oxidoreductase | 208300 | chymotrypsin |
| 1738 | Pyruvate-ferredoxin oxidoreductase | 208301 | |
| 1739 | Pyruvate-ferredoxin oxidoreductase | 208302 | |
| 1740 | Pyruvate-ferredoxin oxidoreductase | 208303 | chymotrypsin, trypsin |
| 1741 | Pyruvate-ferredoxin oxidoreductase | 208304 | trypsin |
| 1742 | Pyruvate-ferredoxin oxidoreductase | 208305 | |
| 1743 | Pyruvate-ferredoxin oxidoreductase | 208306 | trypsin |
| 1744 | Pyruvate-ferredoxin oxidoreductase | 208307 | trypsin |
| 1745 | Pyruvate-ferredoxin oxidoreductase | 208308 | chymotrypsin, trypsin |
| 1746 | Pyruvate-ferredoxin oxidoreductase | 208309 | trypsin |
| 1747 | Pyruvate-ferredoxin oxidoreductase | 208310 | chymotrypsin, trypsin |
| 1748 | Pyruvate-ferredoxin oxidoreductase | 208311 | trypsin |
| 1749 | Pyruvate-ferredoxin oxidoreductase | 208312 | trypsin |
| 1750 | Pyruvate-ferredoxin oxidoreductase | 208313 | chymotrypsin |
| 1751 | Pyruvate-ferredoxin oxidoreductase | 208314 | trypsin |
| 1752 | Pyruvate-ferredoxin oxidoreductase | 208315 | chymotrypsin, trypsin |
| 1753 | Quinohemoprotein amine dehydrogenase 60 kdasubunit | 208316 | |
| 1754 | Quinohemoprotein amine dehydrogenase 60 kdasubunit | 208317 | |
| 1755 | Quinohemoprotein amine dehydrogenase 60 kdasubunit | 208318 | |
| 1756 | Quinohemoprotein amine dehydrogenase 60 kdasubunit | 208319 | |
| 1757 | Quinohemoprotein amine dehydrogenase 60 kdasubunit | 208320 | |
| 1758 | Quinohemoprotein amine dehydrogenase 60 kdasubunit | 208321 | thrombin, trypsin |
| 1759 | Quinohemoprotein amine dehydrogenase 60 kdasubunit | 208322 | chymotrypsin |
| 1760 | Quinohemoprotein amine dehydrogenase 60 kdasubunit | 208323 | chymotrypsin |
| 1761 | Quinohemoprotein amine dehydrogenase 60 kdasubunit | 208324 | trypsin |
| 1762 | Quinohemoprotein amine dehydrogenase 60 kdasubunit | 208325 | chymotrypsin |
| 1763 | Rag1 | 208326 | |
| 1764 | Rag1 | 208327 | |
| 1765 | Receptor-type tyrosine-protein phosphatase mu | 208328 | chymotrypsin, trypsin |
| 1766 | Receptor-type tyrosine-protein phosphatase mu | 208329 | trypsin |
| 1767 | Recg | 208330 | trypsin |
| 1768 | Recg | 208331 | trypsin |
| 1769 | Recg | 208332 | |
| 1770 | Recg | 208333 | chymotrypsin |
| 1771 | Recg | 208334 | chymotrypsin, trypsin |
| 1772 | Recg | 208335 | trypsin |
| 1773 | Recg | 208336 | trypsin |
| 1774 | Recg | 208337 | trypsin |
| 1775 | Recg | 208338 | chymotrypsin, trypsin |
| 1776 | Recg | 208339 | trypsin |
| 1777 | Recg | 208340 | trypsin |
| 1778 | Recg | 208341 | chymotrypsin, trypsin |
| 1779 | Recombination endonuclease vii | 208342 | chymotrypsin, trypsin |
| 1780 | Recombining binding protein suppressor of hairless | 208343 | |
| 1781 | Restriction endonuclease | - | trypsin |
| 1782 | Restriction endonuclease | 208344 | chymotrypsin |
| 1783 | Restriction endonuclease | 208345 | |
| 1784 | Restriction endonuclease | 208346 | chymotrypsin, trypsin |
| 1785 | Retinaldehyde-binding protein 1 | - | |
| 1786 | Retinaldehyde-binding protein 1 | 208347 | |
| 1787 | Retinaldehyde-binding protein 1 | 208348 | chymotrypsin |
| 1788 | Retinoblastoma pocket | 208349 | trypsin |
| 1789 | Rfcs | - | |
| 1790 | Rfcs | - | |
| 1791 | Rfcs | 208350 | |
| 1792 | Rfcs | 208351 | trypsin |
| 1793 | Rfcs | 208352 | |
| 1794 | Rfcs | 208353 | chymotrypsin, trypsin |
| 1795 | Rfcs | 208354 | trypsin |
| 1796 | Rhamnogalacturonase b | 208355 | trypsin |
| 1797 | Rhamnogalacturonase b | 208356 | |
| 1798 | Rhamnogalacturonase b | 208357 | trypsin |
| 1799 | Rhamnogalacturonase b | 208358 | chymotrypsin, trypsin |
| 1800 | Rhamnogalacturonase b | 208359 | trypsin |
| 1801 | Rhodniin | 208360 | chymotrypsin |
| 1802 | Rhodniin | 208361 | |
| 1803 | Riboflavin synthase | 208362 | chymotrypsin, trypsin |
| 1804 | Ribonuclease d | 208363 | trypsin |
| 1805 | Ribonuclease d | 208364 | |
| 1806 | Ribonuclease d | 208365 | |
| 1807 | Ribonuclease ttha0252 | 208366 | |
| 1808 | Ribonuclease ttha0252 | 208367 | |
| 1809 | Ribonuclease ttha0252 | 208368 | |
| 1810 | Ribonuclease ttha0252 | 208369 | chymotrypsin |
| 1811 | Ribonuclease ttha0252 | 208370 | chymotrypsin |
| 1812 | Ribonuclease ttha0252 | 208371 | chymotrypsin, trypsin |
| 1813 | Ribonucleotide reductase r1 protein | 208372 | chymotrypsin |
| 1814 | Ribonucleotide reductase r1 protein | 208373 | chymotrypsin |
| 1815 | Ribonucleotide reductase r1 protein | 208374 | chymotrypsin |
| 1816 | Ribonucleotide reductase r1 protein | 208375 | chymotrypsin, trypsin |
| 1817 | Ribonucleotide reductase r1 protein | 208376 | chymotrypsin |
| 1818 | Ribonucleotide reductase r1 protein | 208377 | chymotrypsin, trypsin |
| 1819 | Ribosome maturation factor rimm | 208378 | |
| 1820 | Ribulose-1,5 bisphosphate carboxylase/oxygenase large subunit n-methyltransferase | - | trypsin |
| 1821 | Ribulose-1,5 bisphosphate carboxylase/oxygenase large subunit n-methyltransferase | 208379 | chymotrypsin |
| 1822 | Rna binding domain of rho transcription termination factor | 208380 | trypsin |
| 1823 | Rna binding protein zfa | 208381 | chymotrypsin, trypsin |
| 1824 | Rob transcription factor | 208382 | trypsin |
| 1825 | Rob transcription factor | 208383 | chymotrypsin, trypsin |
| 1826 | Rp2 lipase | 208384 | chymotrypsin, trypsin |
| 1827 | Rubrerythrin | 208385 | trypsin |
| 1828 | S-adenosylmethionine synthetase | 208386 | trypsin |
| 1829 | Sarcoplasmic/endoplasmic reticulum calcium atpase 1 | - | chymotrypsin |
| 1830 | Sarcoplasmic/endoplasmic reticulum calcium atpase 1 | 208387 | |
| 1831 | Sarcoplasmic/endoplasmic reticulum calcium atpase 1 | 208388 | trypsin |
| 1832 | Sarcoplasmic/endoplasmic reticulum calcium atpase 1 | 208389 | trypsin |
| 1833 | Sarcoplasmic/endoplasmic reticulum calcium atpase 1 | 208390 | |
| 1834 | Sarcoplasmic/endoplasmic reticulum calcium atpase 1 | 208391 | trypsin |
| 1835 | Sarcoplasmic/endoplasmic reticulum calcium atpase 1 | 208392 | |
| 1836 | Sarcoplasmic/endoplasmic reticulum calcium atpase 1 | 208393 | |
| 1837 | Sarcoplasmic/endoplasmic reticulum calcium atpase 1 | 208394 | |
| 1838 | Sarcoplasmic/endoplasmic reticulum calcium atpase 1 | 208395 | |
| 1839 | Sarcoplasmic/endoplasmic reticulum calcium atpase 1 | 208396 | chymotrypsin |
| 1840 | Sarcoplasmic/endoplasmic reticulum calcium atpase 1 | 208397 | |
| 1841 | Sarcoplasmic/endoplasmic reticulum calcium atpase 1 | 208398 | |
| 1842 | Sarcoplasmic/endoplasmic reticulum calcium atpase 1 | 208399 | chymotrypsin |
| 1843 | Sarcoplasmic/endoplasmic reticulum calcium atpase 1 | 208400 | |
| 1844 | Sarcoplasmic/endoplasmic reticulum calcium atpase 1 | 208401 | |
| 1845 | Sarcoplasmic/endoplasmic reticulum calcium atpase 1 | 208402 | trypsin |
| 1846 | Sarcoplasmic/endoplasmic reticulum calcium atpase 1 | 208403 | |
| 1847 | Sarcoplasmic/endoplasmic reticulum calcium atpase 1 | 208404 | trypsin |
| 1848 | Sarcoplasmic/endoplasmic reticulum calcium atpase 1 | 208405 | trypsin |
| 1849 | Sarcoplasmic/endoplasmic reticulum calcium atpase 1 | 208406 | trypsin |
| 1850 | Sarcoplasmic/endoplasmic reticulum calcium atpase 1 | 208407 | chymotrypsin |
| 1851 | Sarcoplasmic/endoplasmic reticulum calcium atpase 1 | 208408 | |
| 1852 | Sarcoplasmic/endoplasmic reticulum calcium atpase 1 | 208409 | trypsin |
| 1853 | Sarcoplasmic/endoplasmic reticulum calcium atpase 1 | 208410 | chymotrypsin |
| 1854 | Sarcoplasmic/endoplasmic reticulum calcium atpase 1 | 208411 | chymotrypsin, trypsin |
| 1855 | Scavenger mrna-decapping enzyme dcps | - | |
| 1856 | Scavenger mrna-decapping enzyme dcps | - | |
| 1857 | Scavenger mrna-decapping enzyme dcps | 208412 | chymotrypsin, trypsin |
| 1858 | Scavenger mrna-decapping enzyme dcps | 208413 | |
| 1859 | Sec18p (residues 22 - 210) | 208414 | trypsin |
| 1860 | Sec18p (residues 22 - 210) | 208415 | chymotrypsin, trypsin |
| 1861 | Sensor protein | 208416 | |
| 1862 | Sensor protein | 208417 | trypsin |
| 1863 | Septum site-determining protein minc | 208418 | trypsin |
| 1864 | Serine acetyltransferase | 208419 | trypsin |
| 1865 | Serine protease/ntpase/helicase ns3 | 208420 | chymotrypsin |
| 1866 | Serine protease/ntpase/helicase ns3 | 208421 | |
| 1867 | Serine protease/ntpase/helicase ns3 | 208422 | |
| 1868 | Seryl-trna synthetase | 208423 | |
| 1869 | Sialidase | 208424 | chymotrypsin, trypsin |
| 1870 | Sialidase b | - | |
| 1871 | Sialidase b | - | trypsin |
| 1872 | Sialidase b | 208425 | |
| 1873 | Sialidase b | 208426 | chymotrypsin, trypsin |
| 1874 | Sialidase b | 208427 | |
| 1875 | Sialidase b | 208428 | chymotrypsin |
| 1876 | Sialidase b | 208429 | chymotrypsin, trypsin |
| 1877 | Sialidase b | 208430 | chymotrypsin, trypsin |
| 1878 | Signal peptidase i | - | trypsin |
| 1879 | Signal peptidase i | 208431 | |
| 1880 | Signal peptidase i | 208432 | |
| 1881 | Signal peptidase i | 208433 | chymotrypsin |
| 1882 | Signal peptidase i | 208434 | trypsin |
| 1883 | Signal peptidase i | 208435 | |
| 1884 | Signal peptidase i | 208436 | |
| 1885 | Signal peptidase i | 208437 | |
| 1886 | Signal peptidase i | 208438 | trypsin |
| 1887 | Signal peptidase i | 208439 | chymotrypsin |
| 1888 | Signal peptidase i | 208440 | |
| 1889 | Signal recognition particle protein | 208441 | trypsin |
| 1890 | Signal transducer and activator of transcription1-alpha/beta | - | |
| 1891 | Signal transducer and activator of transcription1-alpha/beta | - | |
| 1892 | Signal transducer and activator of transcription1-alpha/beta | 208442 | chymotrypsin, trypsin |
| 1893 | Signal transducer and activator of transcription1-alpha/beta | 208443 | |
| 1894 | Signal transducer and activator of transcription1-alpha/beta | 208444 | chymotrypsin, trypsin |
| 1895 | Signal transducer and activator of transcription1-alpha/beta | 208445 | trypsin |
| 1896 | Signal transduction protein cbl | 208446 | chymotrypsin |
| 1897 | Signal transduction protein cbl | 208447 | trypsin |
| 1898 | Similar to rad54-like | - | |
| 1899 | Similar to rad54-like | - | chymotrypsin |
| 1900 | Similar to rad54-like | - | chymotrypsin, trypsin |
| 1901 | Similar to rad54-like | 208448 | |
| 1902 | Similar to rad54-like | 208449 | |
| 1903 | Similar to rad54-like | 208450 | |
| 1904 | Similar to rad54-like | 208451 | trypsin |
| 1905 | Similar to rad54-like | 208452 | chymotrypsin, trypsin |
| 1906 | Similar to rad54-like | 208453 | |
| 1907 | Similar to rad54-like | 208454 | trypsin |
| 1908 | Similar to rad54-like | 208455 | chymotrypsin |
| 1909 | Similar to rad54-like | 208456 | trypsin |
| 1910 | Similar to rad54-like | 208457 | chymotrypsin, trypsin |
| 1911 | Skd1 protein | - | |
| 1912 | Skd1 protein | 208458 | |
| 1913 | Skd1 protein | 208459 | chymotrypsin |
| 1914 | Skd1 protein | 208460 | chymotrypsin, trypsin |
| 1915 | Skd1 protein | 208461 | trypsin |
| 1916 | Skd1 protein | 208462 | |
| 1917 | SII1358 protein | 208463 | |
| 1918 | SII1358 protein | 208464 | chymotrypsin |
| 1919 | SII1358 protein | 208465 | chymotrypsin, trypsin |
| 1920 | SII1358 protein | 208466 | trypsin |
| 1921 | Soluble ifn alpha/beta receptor | 208467 | chymotrypsin |
| 1922 | Soluble ifn alpha/beta receptor | 208468 | chymotrypsin |
| 1923 | Sporozoite-specific sag protein | 208469 | trypsin |
| 1924 | Staphylococcal accessory regulator a homologue | 208470 | trypsin |
| 1925 | Staphylococcal nuclease domain-containing protein 1 | 208471 | chymotrypsin |
| 1926 | Staphylococcal nuclease domain-containing protein 1 | 208472 | |
| 1927 | Staphylococcal nuclease domain-containing protein 1 | 208473 | |
| 1928 | Staphylococcal nuclease domain-containing protein 1 | 208474 | |
| 1929 | Staphylococcal nuclease domain-containing protein 1 | 208475 | |
| 1930 | Staphylococcal nuclease domain-containing protein 1 | 208476 | chymotrypsin, trypsin |
| 1931 | Stat protein | 208477 | |
| 1932 | Stat protein | 208478 | |
| 1933 | Stat protein | 208479 | |
| 1934 | Stat protein | 208480 | chymotrypsin |
| 1935 | Stat protein | 208481 | |
| 1936 | Stat protein | 208482 | |
| 1937 | Stat protein | 208483 | trypsin |
| 1938 | Stat protein | 208484 | |
| 1939 | Stat protein | 208485 | chymotrypsin, trypsin |
| 1940 | Stat protein | 208486 | |
| 1941 | Stat protein | 208487 | chymotrypsin, trypsin |
| 1942 | Stat protein | 208488 | |
| 1943 | Stat protein | 208489 | trypsin |
| 1944 | Stat protein | 208490 | trypsin |
| 1945 | Stat protein | 208491 | trypsin |
| 1946 | Subtilisin-like protease | 208492 | chymotrypsin |
| 1947 | Succinyl-coa ligase [gdp-forming] alpha-chain, mitochondrial | 208493 | trypsin |
| 1948 | Succinyl-coa ligase [gdp-forming] alpha-chain, mitochondrial | 208494 | |
| 1949 | Succinyl-coa ligase [gdp-forming] alpha-chain, mitochondrial | 208495 | |
| 1950 | Succinyl-coa ligase [gdp-forming] alpha-chain, mitochondrial | 208496 | |
| 1951 | Succinyl-coa ligase [gdp-forming] alpha-chain, mitochondrial | 208497 | |
| 1952 | Succinyl-coa ligase [gdp-forming] alpha-chain, mitochondrial | 208498 | trypsin |
| 1953 | Succinyl-coa synthetase beta chain | - | |
| 1954 | Succinyl-coa synthetase beta chain | - | trypsin |
| 1955 | Succinyl-coa synthetase beta chain | 208499 | trypsin |
| 1956 | Succinyl-coa synthetase beta chain | 208500 | |
| 1957 | Succinyl-coa synthetase beta chain | 208501 | |
| 1958 | Succinyl-coa synthetase beta chain | 208502 | chymotrypsin |
| 1959 | Succinyl-coa synthetase beta chain | 208503 | |
| 1960 | Succinyl-coa synthetase beta chain | 208504 | trypsin |
| 1961 | Succinyl-coa:3-ketoacid-coenzyme a transferase | 208505 | chymotrypsin, trypsin |
| 1962 | Sulfurtransferase | 208506 | trypsin |
| 1963 | Super antigen smez-2 | 208507 | chymotrypsin, trypsin |
| 1964 | Superoxide dismutase 1 copper chaperone | 208508 | trypsin |
| 1965 | Surface layer protein | 208509 | trypsin |
| 1966 | Surface layer protein | 208510 | |
| 1967 | Surface layer protein | 208511 | chymotrypsin |
| 1968 | Surface layer protein | 208512 | chymotrypsin |
| 1969 | Surface layer protein | 208513 | |
| 1970 | Surface layer protein | 208514 | trypsin |
| 1971 | Surface layer protein | 208515 | chymotrypsin |
| 1972 | Surface layer protein | 208516 | |
| 1973 | T lymphocyte activation antigen | 208517 | |
| 1974 | T lymphocyte activation antigen | 208518 | trypsin |
| 1975 | T-cell receptor alpha chain c region | 208519 | chymotrypsin |
| 1976 | Terminal oxygenase component of carbazide | 208520 | chymotrypsin |
| 1977 | Tetanus neurotoxin | 208521 | chymotrypsin, trypsin |
| 1978 | Tetracycline repressor protein class d | 208522 | chymotrypsin |
| 1979 | The gdp-binding protein obg | 208523 | |
| 1980 | The gtp-binding protein obg | 208524 | |
| 1981 | The gtp-binding protein obg | 208525 | |
| 1982 | The gtp-binding protein obg | 208526 | trypsin |
| 1983 | Thioredoxin domain-containing protein 4 | 208527 | |
| 1984 | Thioredoxin domain-containing protein 4 | 208528 | trypsin |
| 1985 | Thiosulfate sulfurtransferase | - | |
| 1986 | Thiosulfate sulfurtransferase | 208529 | |
| 1987 | Thiosulfate sulfurtransferase | 208530 | chymotrypsin, trypsin |
| 1988 | Thiosulfate sulfurtransferase | 208531 | chymotrypsin, trypsin |
| 1989 | Thiosulfate sulfurtransferase | 208532 | chymotrypsin, trypsin |
| 1990 | Threonyl-trna synthetase | 208533 | |
| 1991 | Threonyl-trna synthetase | 208534 | trypsin |
| 1992 | Threonyl-trna synthetase | 208535 | trypsin |
| 1993 | Threonyl-trna synthetase | 208536 | trypsin |
| 1994 | Threonyl-trna synthetase | 208537 | |
| 1995 | Threonyl-trna synthetase | 208538 | |
| 1996 | Threonyl-trna synthetase | 208539 | chymotrypsin, trypsin |
| 1997 | Threonyl-trna synthetase | 208540 | trypsin |
| 1998 | Threonyl-trna synthetase | 208541 | |
| 1999 | Threonyl-trna synthetase 1 | 208542 | chymotrypsin |
| 2000 | Threonyl-trna synthetase 1 | 208543 | chymotrypsin |
| 2001 | Threonyl-trna synthetase 1 | 208544 | |
| 2002 | Threonyl-trna synthetase 1 | 208545 | trypsin |
| 2003 | Threonyl-trna synthetase 1 | 208546 | chymotrypsin |
| 2004 | Threonyl-trna synthetase 1 | 208547 | chymotrypsin |
| 2005 | Threonyl-trna synthetase 1 | 208548 | trypsin |
| 2006 | Threonyl-trna synthetase 1 | 208549 | chymotrypsin, trypsin |
| 2007 | Thrombospondin 1 | 208550 | chymotrypsin |
| 2008 | Tick-borne encephalitis virus glycoprotein | 208551 | chymotrypsin, trypsin |
| 2009 | Titin | 208552 | trypsin |
| 2010 | Titin | 208553 | trypsin |
| 2011 | Tlr1789 protein | 208554 | |
| 2012 | Tlr1789 protein | 208555 | trypsin |
| 2013 | Topoisomerase i | 208556 | chymotrypsin, trypsin |
| 2014 | Topoisomerase i | 208557 | trypsin |
| 2015 | Toxic shock syndrome toxin-1 | 208558 | |
| 2016 | Toxic shock syndrome toxin-1 | 208559 | |
| 2017 | Toxic shock syndrome toxin-1 | 208560 | |
| 2018 | Toxic shock syndrome toxin-1 | 208561 | chymotrypsin, trypsin |
| 2019 | T-plasminogen activator f1-g | - | |
| 2020 | T-plasminogen activator f1-q | 208562 | chymotrypsin, trypsin |
| 2021 | Tpsb transporter fhac | 208563 | |
| 2022 | Tpsb transporter fhac | 208564 | |
| 2023 | Tpsb transporter fhac | 208565 | chymotrypsin, trypsin |
| 2024 | Transcarbamylase | 208566 | chymotrypsin, trypsin |
| 2025 | Transcarbamylase | 208567 | chymotrypsin |
| 2026 | Transcription antiterminator lict | 208568 | |
| 2027 | Transcription elongation factor greb | 208569 | chymotrypsin, trypsin |
| 2028 | Transcription initiation factor iia gamma chain | 208570 | trypsin |
| 2029 | Transcription initiation factor iib | 208571 | chymotrypsin |
| 2030 | Transcription initiation factor iib | 208572 | |
| 2031 | Transcriptional regulator (ntrc family) | 208573 | chymotrypsin, trypsin |
| 2032 | Transcriptional regulator aefr | 208574 | |
| 2033 | Transcriptional regulator aefr | 208575 | |
| 2034 | Transcriptional regulator aefr | 208576 | trypsin |
| 2035 | Transcriptional regulator aefr | 208577 | trypsin |
| 2036 | Transcriptional regulator aefr | 208578 | chymotrypsin |
| 2037 | Transcriptional regulator, asnc family | 208579 | |
| 2038 | Transcriptional regulator, asnc family | 208580 | trypsin |
| 2039 | Transcriptional regulator, asnc family | 208581 | trypsin |
| 2040 | Transcriptional regulator, biotin repressor family | 208582 | trypsin |
| 2041 | Transcriptional regulator, crp/fnr family | 208583 | trypsin |
| 2042 | Transcriptional regulator, gntr family | 208584 | |
| 2043 | Transcriptional regulator, hth_3 family | 208585 | |
| 2044 | Transcriptional regulator, hth_3 family | 208586 | chymotrypsin |
| 2045 | Transcriptional regulator, hth_3 family | 208587 | |
| 2046 | Transcriptional regulator, hth_3 family | 208588 | |
| 2047 | Transcriptional regulator, hth_3 family | 208589 | trypsin |
| 2048 | Transcriptional regulator, laci family | 208590 | chymotrypsin, trypsin |
| 2049 | Transcriptional regulatory protein zrar | 208591 | |
| 2050 | Transcriptional regulatory protein zrar | 208592 | |
| 2051 | Transcriptional regulatory protein zrar | 208593 | trypsin |
| 2052 | Transcriptional regulatory protein zrar | 208594 | chymotrypsin |
| 2053 | Transcriptional regulatory protein zrar | 208595 | chymotrypsin |
| 2054 | Transcriptional regulatory protein zrar | 208596 | chymotrypsin, trypsin |
| 2055 | Transcriptional regulatory protein zrar | 208597 | chymotrypsin, trypsin |
| 2056 | Transferrin receptor protein | - | |
| 2057 | Transferrin receptor protein | 208598 | chymotrypsin |
| 2058 | Transferrin receptor protein | 208599 | |
| 2059 | Transferrin receptor protein | 208600 | trypsin |
| 2060 | Transferrin receptor protein | 208601 | chymotrypsin |
| 2061 | Translation initiation factor 5a | 208602 | |
| 2062 | Translation initiation factor 5a | 208603 | |
| 2063 | Translation initiation factor 5a | 208604 | chymotrypsin |
| 2064 | Translation initiation factor if2/eif5b | 208605 | chymotrypsin, trypsin |
| 2065 | Translation initiation factor if2/eif5b | 208606 | |
| 2066 | Transposable element mariner, complete cds | 208607 | chymotrypsin, trypsin |
| 2067 | Tricorn protease | 208608 | |
| 2068 | Tricorn protease | 208609 | trypsin |
| 2069 | Tricorn protease | 208610 | chymotrypsin, trypsin |
| 2070 | Trigger factor | 208611 | |
| 2071 | Trigger factor | 208612 | |
| 2072 | Trigger factor | 208613 | chymotrypsin, trypsin |
| 2073 | Trna cca-adding enzyme | - | trypsin |
| 2074 | Trna cca-adding enzyme | 208614 | chymotrypsin, trypsin |
| 2075 | Trna cca-adding enzyme | 208615 | |
| 2076 | Trna cca-adding enzyme | 208616 | trypsin |
| 2077 | Trna cca-adding enzyme | 208617 | chymotrypsin, trypsin |
| 2078 | Trna nucleotidyltransferase | 208618 | factor xa, trypsin |
| 2079 | Trna-splicing endonuclease | 208619 | trypsin |
| 2080 | Tt1467 protein | - | |
| 2081 | Tt1467 protein | 208620 | trypsin |
| 2082 | Tumor suppressor p53-binding protein 1 | 208621 | |
| 2083 | Tumor suppressor p53-binding protein 1 | 208622 | |
| 2084 | Tumor suppressor p53-binding protein 1 | 208623 | chymotrypsin |
| 2085 | Tumor suppressor p53-binding protein 1 | 208624 | chymotrypsin, trypsin |
| 2086 | Type a flavoprotein fpra | 208625 | trypsin |
| 2087 | Type a flavoprotein fpra | 208626 | chymotrypsin |
| 2088 | Type a flavoprotein fpra | 208627 | |
| 2089 | Type a flavoprotein fpra | 208628 | |
| 2090 | Type a flavoprotein fpra | 208629 | |
| 2091 | Type i restriction enzyme specificity protein mg438 | - | |
| 2092 | Type i restriction enzyme specificity protein mg438 | 208630 | chymotrypsin |
| 2093 | Type i restriction enzyme specificity protein mg438 | 208631 | chymotrypsin, trypsin |
| 2094 | Type i restriction-modification enzyme, s subunit | 208632 | |
| 2095 | Type i restriction-modification enzyme, s subunit | 208633 | trypsin |
| 2096 | Type i site-specific restriction-modificationsystem, r (restriction) subunit | 208634 | trypsin |
| 2097 | Type i site-specific restriction-modificationsystem, r (restriction) subunit | 208635 | trypsin |
| 2098 | Type i site-specific restriction-modificationsystem, r (restriction) subunit | 208636 | chymotrypsin, trypsin |
| 2099 | Type ii dna topoisomerase vi subunit b | 208637 | |
| 2100 | Type ii dna topoisomerase vi subunit b | 208638 | trypsin |
| 2101 | Type ii dna topoisomerase vi subunit b | 208639 | chymotrypsin |
| 2102 | Type ii dna topoisomerase vi subunit b | 208640 | chymotrypsin |
| 2103 | Type ii dna topoisomerase vi subunit b | 208641 | trypsin |
| 2104 | Type ii dna topoisomerase vi subunit b | 208642 | |
| 2105 | Type ii dna topoisomerase vi subunit b | 208643 | trypsin |
| 2106 | Type ii dna topoisomerase vi subunit b | 208644 | chymotrypsin, trypsin |
| 2107 | Type ii dna topoisomerase vi subunit b | 208645 | chymotrypsin, trypsin |
| 2108 | Type ii dna topoisomerase vi subunit b | 208646 | chymotrypsin, trypsin |
| 2109 | Type ii dna topoisomerase vi subunit b | 208647 | chymotrypsin, trypsin |
| 2110 | Type vi secretion system component | 208648 | chymotrypsin, trypsin |
| 2111 | Type vi secretion system component | 208649 | chymotrypsin, trypsin |
| 2112 | Type vi secretion system component | 208650 | chymotrypsin, thrombin, trypsin |
| 2113 | Tyrosine-protein kinase receptor ufo | 208651 | |
| 2114 | Tyrosine-protein kinase receptor ufo | 208652 | |
| 2115 | Tyrosine-protein kinase zap-70 | 208653 | |
| 2116 | Tyrosine-protein kinase zap-70 | 208654 | chymotrypsin, trypsin |
| 2117 | Tyrosyl-dna phosphodiesterase | 208655 | chymotrypsin |
| 2118 | Tvrosvl-dna phosphodiesterase | 208656 | trypsin |
| 2119 | Ubiquitin carboxyl-terminal hydrolase 7 | 208657 | |
| 2120 | Udp-galactopyranose mutase | 208658 | chymotrypsin, trypsin |
| 2121 | Udp-galactopyranose mutase | 208659 | chymotrypsin |
| 2122 | Udp-galactopyranose mutase | 208660 | chymotrypsin, trypsin |
| 2123 | Udp-galactopyranose mutase | 208661 | chymotrypsin, trypsin |
| 2124 | Udp-galactopyranose mutase | 208662 | chymotrypsin, trypsin |
| 2125 | Udp-glucose dehydrogenase | 208663 | trypsin |
| 2126 | Udp-n-acetylmuramate-l-alanine ligase | 208664 | |
| 2127 | Udp-n-acetylmuramate-l-alanine ligase | 208665 | chymotrypsin, trypsin |
| 2128 | Udp-n-acetylmuramoylalanine--d-glutamate ligase | 208666 | |
| 2129 | Udp-n-acetylmuramoylalanine--d-glutamate ligase | 208667 | chymotrypsin, trypsin |
| 2130 | Udp-n-acetylmuramoylalanine-d-glutamyl-lysine-d-alanyl-d-alanine ligase, murf protein | 208668 | |
| 2131 | Udp-n-acetylmuramoylalanyl-d-glutamate--2,6-diaminopimelate ligase | 208669 | |
| 2132 | Udp-n-acetylmuramoylalanyl-d-glutamate--2,6-diaminopimelate ligase | 208670 | chymotrypsin |
| 2133 | Udp-n-acetylmuramoylalanyl-d-glutamate--2,6-diaminopimelate ligase | 208671 | |
| 2134 | Udp-n-acetylmuramoylalanyl-d-glutamate--2,6-diaminopimelate ligase | 208672 | |
| 2135 | Udp-n-acetylmuramoylalanyl-d-glutamate--2,6-diaminopimelate ligase | 208673 | |
| 2136 | Udp-n-acetylmuramoylalanyl-d-glutamate--2,6-diaminopimelate ligase | 208674 | |
| 2137 | Udp-n-acetylmuramoylalanyl-d-glutamate--2,6-diaminopimelate ligase | 208675 | trypsin |
| 2138 | Uncharacterized conserved protein | 208676 | trypsin |
| 2139 | Uncharacterized conserved protein | 208677 | chymotrypsin, trypsin |
| 2140 | Uncharacterized gst-like protein vfcf | 208678 | chymotrypsin, trypsin |
| 2141 | Uncharacterized gst-like proteinprotein | 208679 | chymotrypsin |
| 2142 | Uncharacterized gst-like proteinprotein | 208680 | chymotrypsin, trypsin |
| 2143 | Uncharacterized gst-like proteinprotein | 208681 | chymotrypsin, trypsin |
| 2144 | Uncharacterized protein | 208682 | trypsin |
| 2145 | Uncharacterized protein | 208683 | trypsin |
| 2146 | Uncharacterized protein bt_1490 | 208684 | chymotrypsin, trypsin |
| 2147 | Uncharacterized protein ypfi | - | |
| 2148 | Uncharacterized protein ypfi | - | |
| 2149 | Uncharacterized protein ypfi | 208685 | chymotrypsin |
| 2150 | Uncharacterized protein ypfi | 208686 | |
| 2151 | Uncharacterized protein ypfi | 208687 | |
| 2152 | Uncharacterized protein ypfi | 208688 | |
| 2153 | Uncharacterized protein ypfi | 208689 | chymotrypsin |
| 2154 | Uncharacterized protein ypfi | 208690 | |
| 2155 | Uncharacterized protein ypfi | 208691 | |
| 2156 | Uncharacterized protein ypfi | 208692 | chymotrypsin, trypsin |
| 2157 | Uncharacterized protein ypfi | 208693 | trypsin |
| 2158 | Uncharacterized protein ypfi | 208694 | |
| 2159 | Uncharacterized protein ypfi | 208695 | chymotrypsin, trypsin |
| 2160 | Uncharacterized protein ypfi | 208696 | chymotrypsin |
| 2161 | Uncharacterized protein ypfi | 208697 | chymotrypsin, trypsin |
| 2162 | Uncharacterized protein ypfi | 208698 | chymotrypsin, trypsin |
| 2163 | Uncharacterized protein ypfi | 208699 | chymotrypsin |
| 2164 | Unknown protein | 208700 | chymotrypsin |
| 2165 | Unknown protein | 208701 | trypsin |
| 2166 | Upf0131 protein ykqa | 208702 | trypsin |
| 2167 | Upf0131 protein ykqa | 208703 | trypsin |
| 2168 | Upf0131 protein ykqa | 208704 | chymotrypsin, trypsin |
| 2169 | Upf0348 protein mj0951 | 208705 | |
| 2170 | Upf0348 protein mj0951 | 208706 | chymotrypsin |
| 2171 | Upf0348 protein mj0951 | 208707 | |
| 2172 | Upf0348 protein mj0951 | 208708 | |
| 2173 | Upf0348 protein mj0951 | 208709 | chymotrypsin |
| 2174 | Upf0348 protein mj0951 | 208710 | trypsin |
| 2175 | Upf0348 protein mj0951 | 208711 | chymotrypsin, trypsin |
| 2176 | Upf0348 protein mj0951 | 208712 | |
| 2177 | Ure2 protein | 208713 | chymotrypsin |
| 2178 | Uridine diphospho-n-acetylenolpyruvylglucosaminereductase | - | |
| 2179 | Uridine diphospho-n-acetylenolpyruvylglucosaminereductase | 208714 | chymotrypsin, trypsin |
| 2180 | Uridine diphospho-n-acetylenolpyruvylglucosaminereductase | 208715 | chymotrypsin, trypsin |
| 2181 | Uridine diphospho-n-acetylenolpyruvylglucosaminereductase | 208716 | trypsin |
| 2182 | Uridine diphospho-n-acetylenolpyruvylglucosaminereductase | 208717 | chymotrypsin, trypsin |
| 2183 | Urokinase plasminogen activator surface receptor | 208718 | trypsin |
| 2184 | Urokinase plasminogen activator surface receptor | 208719 | chymotrypsin, trypsin |
| 2185 | Vascular cell adhesion molecule-1 | 208720 | trypsin |
| 2186 | Vcp-like atpase | 208721 | trypsin |
| 2187 | Vcp-like atpase | 208722 | chymotrypsin, trypsin |
| 2188 | Viral casp8 and fadd-like apoptosis regulator | 208723 | chymotrypsin |
| 2189 | Vitamin k-dependent protein z | 208724 | chymotrypsin, trypsin |
| 2190 | Vp1 protein | 208725 | trypsin |
| 2191 | V-type atp synthase alpha chain | 208726 | |
| 2192 | Xaa-pro aminopeptidase | 208727 | chymotrypsin |
| 2193 | Xaa-pro aminopeptidase | 208728 | trypsin |
| 2194 | Xaa-pro aminopeptidase | 208729 | chymotrypsin, trypsin |
| 2195 | Xaa-pro aminopeptidase | 208730 | |
| 2196 | Xanthine dehydrogenase | 208731 | chymotrypsin |
| 2197 | Xanthine dehydrogenase | 208732 | chymotrypsin |
| 2198 | Xanthine dehydrogenase | 208733 | trypsin |
| 2199 | Xanthine dehydrogenase | 208734 | chymotrypsin |
| 2200 | X-prolyl dipeptidyl aminopetidase | - | trypsin |
| 2201 | X-prolyl dipeptidyl aminopetidase | - | |
| 2202 | X-prolyl dipeptidyl aminopetidase | - | |
| 2203 | X-prolyl dipeptidyl aminopetidase | - | chymotrypsin |
| 2204 | X-prolyl dipeptidyl aminopetidase | 208735 | chymotrypsin |
| 2205 | X-prolyl dipeptidyl aminopetidase | 208736 | trypsin |
| 2206 | X-prolyl dipeptidyl aminopetidase | 208737 | trypsin |
| 2207 | X-prolyl dipeptidyl aminopetidase | 208738 | |
| 2208 | X-prolyl dipeptidyl aminopetidase | 208739 | chymotrypsin, trypsin |
| 2209 | X-prolyl dipeptidyl aminopetidase | 208740 | chymotrypsin, trypsin |
| 2210 | X-prolyl dipeptidyl aminopetidase | 208741 | trypsin |
| 2211 | X-prolyl dipeptidyl aminopetidase | 208742 | chymotrypsin |
| 2212 | X-prolyl dipeptidyl aminopetidase | 208743 | |
| 2213 | X-prolyl dipeptidyl aminopetidase | 208744 | |
| 2214 | X-prolyl dipeptidyl aminopetidase | 208745 | trypsin |
| 2215 | X-prolyl dipeptidyl aminopetidase | 208746 | chymotrypsin, trypsin |
| 2216 | X-prolyl dipeptidyl aminopetidase | 208747 | chymotrypsin |
| 2217 | X-prolyl dipeptidyl aminopetidase | 208748 | chymotrypsin |
| 2218 | X-prolyl dipeptidyl aminopetidase | 208749 | chymotrypsin, trypsin |
| 2219 | X-prolyl dipeptidyl aminopetidase | 208750 | trypsin |
| 2220 | X-prolyl dipeptidyl aminopetidase | 208751 | chymotrypsin |
| 2221 | X-prolyl dipeptidyl aminopetidase | 208752 | trypsin |
| 2222 | X-prolyl dipeptidyl aminopetidase | 208753 | trypsin |
| 2223 | X-prolyl dipeptidyl aminopetidase | 208754 | chymotrypsin, trypsin |
| 2224 | X-prolyl dipeptidyl aminopetidase | 208755 | chymotrypsin, trypsin |
| 2225 | X-prolyl dipeptidyl aminopetidase | 208756 | trypsin |
| 2226 | X-prolyl dipeptidyl aminopetidase | 208757 | chymotrypsin, trypsin |
| 2227 | X-prolyl dipeptidyl aminopetidase | 208758 | trypsin |
| 2228 | Xylosidase/arabinosidase | 208759 | chymotrypsin, trypsin |
| 2229 | Xylosidase/arabinosidase | 208760 | chymotrypsin |
| 2230 | Xylosidase/arabinosidase | 208761 | chymotrypsin, trypsin |
| 2231 | Xylosidase/arabinosidase | 208762 | |
| 2232 | Xylosidase/arabinosidase | 208763 | chymotrypsin |
| 2233 | Xylosidase/arabinosidase | 208764 | chymotrypsin, trypsin |
| 2234 | Xylosidase/arabinosidase | 208765 | chymotrypsin |
| 2235 | Ykof | 208766 | chymotrypsin, trypsin |

### Embedded stimulus, signals or other regulatory moieties

### microRNA

microRNAs (or miRNA) are 19-25 nucleotide long noncoding RNAs that bind to the 3'UTR of nucleic acid molecules and down-regulate gene expression either by reducing nucleic acid molecule stability or by inhibiting translation. The polynucleotides of the invention may comprise one or more microRNA target sequences, microRNA sequences, or microRNA seeds. Such sequences may correspond to any known microRNA such as those taught in US Publication US2005/0261218 and US Publication US2005/0059005. As a non-limiting embodiment, known microRNAs, their sequences and their binding site sequences in the human genome are listed below in Table 9.

A microRNA sequence comprises a "seed" region, i.e., a sequence in the region of positions 2-8 of the mature microRNA, which sequence has perfect Watson-Crick complementarity to the miRNA target sequence. A microRNA seed may comprise positions 2-8 or 2-7 of the mature microRNA. In some embodiments, a microRNA seed may comprise 7 nucleotides (e.g., nucleotides 2-8 of the mature microRNA), wherein the seed-complementary site in the corresponding miRNA target is flanked by an adenine (A) opposed to microRNA position 1. In some embodiments, a microRNA seed may comprise 6 nucleotides (e.g., nucleotides 2-7 of the mature microRNA), wherein the seed-complementary site in the corresponding miRNA target is flanked by an adenine (A) opposed to microRNA position 1. See for example, Grimson A, Farh KK, Johnston WK, Garrett-Engele P, Lim LP, Bartel DP; Mol Cell. 2007 Jul 6;27(1):91-105. The bases of the microRNA seed have complete complementarity with the target sequence. By engineering microRNA target sequences into the polynucleotides encoding the effector modules, SREs or payloads of the invention one can target the molecule for degradation or reduced translation, provided the microRNA in question is available. This process will reduce the hazard of off target effects upon nucleic acid molecule delivery.

Identification of microRNA, microRNA target regions, and their expression patterns and role in biology have been reported (Bonauer et al., Curr Drug Targets 2010 11:943-949; Anand and Cheresh Curr Opin Hematol 2011 18:171-176; Contreras and Rao Leukemia 2012 26:404-413 (2011 Dec 20. doi: 10.1038/leu.2011.356); Bartel Cell 2009 136:215-233; Landgraf et al, Cell, 2007 129:1401-1414; Gentner and Naldini, Tissue Antigens. 2012 80:393-403 and all references therein).

For example, if the polynucleotide is not intended to be delivered to the liver but ends up there, then miR-122, a microRNA abundant in liver, can inhibit the expression of the polynucleotide if one or multiple target sites of miR-122 are engineered into the polynucleotide. Introduction of one or multiple binding sites for different microRNA can be engineered to further decrease the longevity, stability, and protein translation of a polynucleotide hence providing an additional layer of tenability beyond the stimulus selection, SRE design and payload variation.

As used herein, the term "microRNA site" refers to a microRNA target site or a microRNA recognition site, or any nucleotide sequence to which a microRNA binds or associates. It should be understood that "binding" may follow traditional Watson-Crick hybridization rules or may reflect any stable association of the microRNA with the target sequence at or adjacent to the microRNA site.

Conversely, for the purposes of the polynucleotides of the present invention, microRNA binding sites can be engineered out of (i.e. removed from) sequences in which they naturally occur in order to increase protein expression in specific tissues. For example, miR-122 binding sites may be removed to improve protein expression in the liver.

Regulation of expression in multiple tissues can be accomplished through introduction or removal or one or several microRNA binding sites.

Specifically, microRNAs are known to be differentially expressed in immune cells (also called hematopoietic cells), such as antigen presenting cells (APCs) (e.g. dendritic cells and macrophages), macrophages, monocytes, B lymphocytes, T lymphocytes, granulocytes, natural killer cells, etc. Immune cell specific microRNAs are involved in immunogenicity, autoimmunity, the immune -response to infection, inflammation, as well as unwanted immune response after gene therapy and tissue/organ transplantation. Immune cells specific microRNAs also regulate many aspects of development, proliferation, differentiation and apoptosis of hematopoietic cells (immune cells). For example, miR-142 and miR-146 are exclusively expressed in the immune cells, particularly abundant in myeloid dendritic cells. Introducing the miR-142 binding site into the 3'-UTR of a polypeptide of the present invention can selectively suppress the gene expression in the antigen presenting cells through miR-142 mediated mRNA degradation, limiting antigen presentation in professional APCs (e.g. dendritic cells) and thereby preventing antigen-mediated immune response after gene delivery (see, Annoni A et al., blood, 2009, 114, 5152-5161.)

In one embodiment, microRNAs binding sites that are known to be expressed in immune cells, in particular, the antigen presenting cells, can be engineered into the polynucleotides to suppress the expression of the polynucleotide in APCs through microRNA mediated RNA degradation, subduing the antigen-mediated immune response, while the expression of the polynucleotide is maintained in non-immune cells where the immune cell specific microRNAs are not expressed.

Many microRNA expression studies have been conducted, and are described in the art, to profile the differential expression of microRNAs in various cancer cells /tissues and other diseases. Some microRNAs are abnormally over-expressed in certain cancer cells and others are under-expressed. For example, microRNAs are differentially expressed in cancer cells (WO2008/154098, US2013/0059015, US2013/0042333, WO2011/157294); cancer stem cells (US2012/0053224); pancreatic cancers and diseases (US2009/0131348, US2011/0171646, US2010/0286232, US8389210); asthma and inflammation (US8415096); prostate cancer (US2013/0053264); hepatocellular carcinoma (WO2012/151212, US2012/0329672, WO2008/054828, US8252538); lung cancer cells (WO2011/076143, WO2013/033640, WO2009/070653, US2010/0323357); cutaneous T cell lymphoma (WO2013/011378); colorectal cancer cells (WO2011/0281756, WO2011/076142); cancer positive lymph nodes (WO2009/100430, US2009/0263803); nasopharyngeal carcinoma (EP2112235); chronic obstructive pulmonary disease (US2012/0264626, US2013/0053263); thyroid cancer (WO2013/066678); ovarian cancer cells (US2012/0309645, WO2011/095623); breast cancer cells (WO2008/154098, WO2007/081740, US2012/0214699), leukemia and lymphoma (WO2008/073915, US2009/0092974, US2012/0316081, US2012/0283310, WO2010/018563).

In one embodiment, microRNA may be used as described herein in support of the creation of tunable biocircuits.

In some embodiments, effector modules may be designed to encode (as a DNA or RNA or mRNA) one or more payloads, SREs and/or regulatory sequence such as a microRNA or microRNA binding site. In some embodiments, any of the encoded payloads or SREs may be stabilized or de-stabilized by mutation and then combined with one or more regulatory sequences to generate a dual or multi-tuned effector module or biocircuit system.

Each aspect or tuned modality may bring to the effector module or biocircuit a differentially tuned feature. For example, an SRE may represent a destabilizing domain, while mutations in the protein payload may alter its cleavage sites or dimerization properties or half-life and the inclusion of one or more microRNA or microRNA binding site may impart cellular detargeting or trafficking features. Consequently, the present invention embraces biocircuits which are multifactorial in their tenability.

Such biocircuits may be engineered to contain one, two, three, four or more tuned features.

Shown in FIG. 19B, for example is a multi-tuned effector module having such a microRNA feature.

**Table 9. microRNA Sequences**

| **microRNA name** | **miR SEQ ID** | **miR BS SEQ ID** | **Types of Tissues and/or Cells** |
|---|---|---|---|
| hsa-miR-1 | 208768 | 210810 | Heart and muscle |
| hsa-miR-10a-3p | 208769 | 210811 | Hematopoietic cells |
| hsa-miR-10a-5p | 208770 | 210812 | Hematopoietic cells |
| hsa-miR-10b-3p | 208771 | 210813 | Variety of tissues and cells |
| hsa-miR-10b-5p | 208772 | 210814 | Variety of tissues and cells |
| hsa-miR-15a-3p | 208773 | 210815 | Lymphocyte, blood, hematopoietic tissues (spleen) |
| hsa-miR-15a-5p | 208774 | 210816 | Lymphocyte, blood, hematopoietic tissues (spleen) |
| hsa-miR-15b-3p | 208775 | 210817 | Lymphocyte, blood, hematopoietic tissues (spleen) |
| hsa-miR-15b-5p | 208776 | 210818 | Lymphocyte, blood, hematopoietic tissues (spleen) |
| hsa-miR-16-1-3p | 208777 | 210819 | Blood, embryonic stem cells, hematopoietic tissues (spleen) |
| hsa-miR-16-2-3p | 208778 | 210820 | Lymphocyte, blood, hematopoietic tissues (spleen) |
| hsa-miR-16-5p | 208779 | 210821 | Variety of tissues, blood |
| hsa-miR-17-3p | 208780 | 210822 | Endothelial cells, embryonic stem cells |
| hsa-miR-17-5p | 208781 | 210823 | Kidnev, breast and endothelial cells |
| hsa-miR-18a-3p | 208782 | 210824 | Lung and endothelial cells |
| hsa-miR-18a-5p | 208783 | 210825 | Lung and endothelial cells |
| hsa-miR-18b-3p | 208784 | 210826 | Lung |
| hsa-miR-18b-5p | 208785 | 210827 | Lung |
| hsa-miR-19a-3p | 208786 | 210828 | Endothelial cells |
| hsa-miR-19a-5p | 208787 | 210829 | Endothelial cells |
| hsa-miR-19b-1-5p | 208788 | 210830 | Endothelial cells |
| hsa-miR-19b-2-5p | 208789 | 210831 | Endothelial cells |
| hsa-miR-19b-3p | 208790 | 210832 | Endothelial cells |
| hsa-miR-20a-3p | 208791 | 210833 | Kidnev, endothelial cells, osteogenic cells |
| hsa-miR-20a-5p | 208792 | 210834 | Kidney, endothelial cells, osteogenic cells |
| hsa-miR-20b-3p | 208793 | 210835 | Osteogenic cells |
| hsa-miR-20b-5p | 208794 | 210836 | Osteogenic cells |
| hsa-miR-21-3p | 208795 | 210837 | Blood (myeloid cells), glioblast, liver, vascular endothelial cells |
| hsa-miR-21-5p | 208796 | 210838 | Blood (myeloid cells), liver, endothelial cells |
| hsa-miR-22-3p | 208797 | 210839 | Variety of cells and tissues |
| hsa-miR-22-5p | 208798 | 210840 | Variety of cells and tissues |
| hsa-miR-23a-3p | 208799 | 210841 | Endothelial cells, brain(astrocyte), blood(erythroid) |
| hsa-miR-23a-5p | 208800 | 210842 | Endothelial cells, brain(astrocyte), blood(erythroid) |
| hsa-miR-23b-3p | 208801 | 210843 | Myeloid cells and blood |
| hsa-miR-23b-5p | 208802 | 210844 | Myeloid cells and blood |
| hsa-miR-23c | 208803 | 210845 | |
| hsa-miR-24-1-5p | 208804 | 210846 | Myeloid cells and lung |
| hsa-miR-24-2-5p | 208805 | 210847 | Myeloid cells and lung |
| hsa-miR-24-3p | 208806 | 210848 | Myeloid cells and lung |
| hsa-miR-25-3p | 208807 | 210849 | Embryonic stem cells, airway smooth muscle |
| hsa-miR-25-5p | 208808 | 210850 | Embryonic stem cells, airway smooth muscle |
| hsa-miR-26a-1-3p | 208809 | 210851 | Embryonic stem cells, Blood and other tissues |
| hsa-miR-26a-2-3p | 208810 | 210852 | Blood and other tissues |
| hsa-miR-26a-5p | 208811 | 210853 | Blood and other tissues |
| hsa-miR-26b-3p | 208812 | 210854 | Hematopoietic cells |
| hsa-miR-26b-5p | 208813 | 210855 | Hematopoietic cells |
| hsa-miR-27a-3p | 208814 | 210856 | Myeloid cells |
| hsa-miR-27a-5p | 208815 | 210857 | Myeloid cells |
| hsa-miR-27b-3p | 208816 | 210858 | Myeloid cells and vascular endothelial cells |
| hsa-miR-27b-5p | 208817 | 210859 | Myeloid cells and vascular endothelial cells |
| hsa-miR-28-3p | 208818 | 210860 | Blood (immune cells) |
| hsa-miR-28-5p | 208819 | 210861 | Blood (immune cells) |
| hsa-miR-29a-3p | 208820 | 210862 | Immune system |
| hsa-miR-29a-5p | 208821 | 210863 | Immune system |
| hsa-miR-29b-1-5p | 208822 | 210864 | Immune system |
| hsa-miR-29b-2-5p | 208823 | 210865 | Immune system |
| hsa-miR-29b-3p | 208824 | 210866 | Immune system |
| hsa-miR-29c-3p | 208825 | 210867 | Immune system |
| hsa-miR-29c-5p | 208826 | 210868 | Immune system |
| hsa-miR-30a-3p | 208827 | 210869 | Kidney and pancreatic cells |
| hsa-miR-30a-5p | 208828 | 210870 | CNS (prefrontal cortex), other tissues |
| hsa-miR-30b-3p | 208829 | 210871 | Kidney, adipose, CNS (prefrontal cortex) |
| hsa-miR-30b-5p | 208830 | 210872 | Kidney, adipose, CNS (prefrontal cortex) |
| hsa-miR-30c-1-3p | 208831 | 210873 | Kidney, adipose, CNS (prefrontal cortex) |
| hsa-miR-30c-2-3p | 208832 | 210874 | Kidney, adipose, CNS (prefrontal cortex) |
| hsa-miR-30c-5p | 208833 | 210875 | Kidney, adipose, CNS (prefrontal cortex) |
| hsa-miR-30d-3p | 208834 | 210876 | CNS (prefrontal cortex |
| hsa-miR-30d-5p | 208835 | 210877 | CNS (prefrontal cortex, embryoid body cells |
| hsa-miR-30e-3p | 208836 | 210878 | Myeloid cells and glia cells |
| hsa-miR-30e-5p | 208837 | 210879 | Myeloid cell and glia cells |
| hsa-miR-31-3p | 208838 | 210880 | |
| hsa-miR-31-5p | 208839 | 210881 | |
| hsa-miR-32-3p | 208840 | 210882 | Blood and glia |
| hsa-miR-32-5p | 208841 | 210883 | Blood and glia |
| hsa-miR-34a-3p | 208842 | 210884 | Breast, myeloid cells, ciliated epithelial cells |
| hsa-miR-34a-5p | 208843 | 210885 | Breast, myeloid cells, ciliated epithelial cells |
| hsa-miR-34b-3p | 208844 | 210886 | Ciliated epithelial cells |
| hsa-miR-34b-5p | 208845 | 210887 | Ciliated epithelial cells |
| hsa-miR-34c-3p | 208846 | 210888 | Ciliated epithelial cells, placenta |
| hsa-miR-34c-5p | 208847 | 210889 | Ciliated epithelial cells, placenta |
| hsa-miR-7-1-3p | 208848 | 210890 | Glioblast, brain, pancreas |
| hsa-miR-7-2-3p | 208849 | 210891 | Brain and pancreas |
| hsa-miR-7-5p | 208850 | 210892 | Brain |
| hsa-miR-92a-1-5p | 208851 | 210893 | Endothelial cells |
| hsa-miR-92a-2-5p | 208852 | 210894 | Endothelial cells |
| hsa-miR-92a-3p | 208853 | 210895 | Endothelial cells and CNS |
| hsa-miR-92b-3p | 208854 | 210896 | Endothelial cells and heart |
| hsa-miR-92b-5p | 208855 | 210897 | Endothelial cells and heart |
| hsa-miR-93-3p | 208856 | 210898 | Embryonic stem cells |
| hsa-miR-93-5p | 208857 | 210899 | Embryonic stem cells |
| hsa-miR-9-3p | 208858 | 210900 | Brain |
| hsa-miR-95 | 208859 | 210901 | |
| hsa-miR-9-5p | 208860 | 210902 | Brain |
| hsa-miR-96-3p | 208861 | 210903 | Stem cells |
| hsa-miR-96-5p | 208862 | 210904 | Stem cells |
| hsa-miR-98-3p | 208863 | 210905 | |
| hsa-miR-98-5p | 208864 | 210906 | |
| hsa-miR-99a-3p | 208865 | 210907 | Hematopoietic cells |
| hsa-miR-99a-5p | 208866 | 210908 | Hematopoietic cells |
| hsa-miR-99b-3p | 208867 | 210909 | Hematopoietic cells and embryonic stem cells |
| hsa-miR-99b-5p | 208868 | 210910 | Hematopoietic cells and embryonic stem cells |
| hsa-miR-100-3p | 208869 | 210911 | Hematopoietic cells, endothelial cells |
| hsa-miR-100-5p | 208870 | 210912 | Hematopoietic cells and endothelial cells |
| hsa-miR-101-3p | 208871 | 210913 | Endothelial cells |
| hsa-miR-101-5p | 208872 | 210914 | Endothelial cells |
| hsa-miR-103a-2-5p | 208873 | 210915 | Embryonic stem cells and a variety of cells and tissues |
| hsa-miR-103a-3p | 208874 | 210916 | Embryonic stem cells and a variety of cells and tissues |
| hsa-miR-103b | 208875 | 210917 | Variety of cells and tissues |
| hsa-miR-105-3p | 208876 | 210918 | Pancreatic cells |
| hsa-miR-105-5p | 208877 | 210919 | Pancreatic cells |
| hsa-miR-106a-3p | 208878 | 210920 | Osteogenic cells |
| hsa-miR-106a-5p | 208879 | 210921 | Osteogenic cells |
| hsa-miR-106b-3p | 208880 | 210922 | Embryonic stem cells |
| hsa-miR-106b-5p | 208881 | 210923 | Embryonic stem cells |
| hsa-miR-107 | 208882 | 210924 | Many tissues and brain hepatocytes/liver |
| hsa-miR-122-3p | 208883 | 210925 | Kidney and liver/hepatocytes |
| hsa-miR-122-5p | 208884 | 210926 | Liver/hepatocytes |
| hsa-miR-124-3p | 208885 | 210927 | Brain and plasma (exosomal) |
| hsa-miR-124-5p | 208886 | 210928 | Brain and plasma (circulating) |
| hsa-miR-125a-3p | 208887 | 210929 | Brain and hematopoietic cells |
| hsa-miR-125a-5p | 208888 | 210930 | Brain and hematopoietic cells |
| hsa-miR-125b-1-3p | 208889 | 210931 | Hematopoietic cells (monocytes), brain(neuron) |
| hsa-miR-125b-2-3p | 208890 | 210932 | Hematopoietic cells (monocytes), brain(neuron) |
| hsa-miR-125b-5p | 208891 | 210933 | Hematopoietic cells, brain (neuron) |
| hsa-miR-126-3p | 208892 | 210934 | Endothelial cells, lung |
| hsa-miR-126-5p | 208893 | 210935 | Endothelial cells, lung |
| hsa-miR-127-3p | 208894 | 210936 | Lung, placenta |
| hsa-miR-127-5p | 208895 | 210937 | Lung, placenta(islet) |
| hsa-miR-128 | 208896 | 210938 | Glioblast, brain |
| hsa-miR-129-1-3p | 208897 | 210939 | Multiple cell types |
| hsa-miR-129-2-3p | 208898 | 210940 | Multiple cell types |
| hsa-miR-129-5p | 208899 | 210941 | Liver(hepatocytes) |
| hsa-miR-130a-3p | 208900 | 210942 | Lung, monocvtes, vascular endothelial cells |
| hsa-miR-130a-5p | 208901 | 210943 | Lung, monocytes, vascular endothelial cells |
| hsa-miR-130b-3p | 208902 | 210944 | Lung, epidermal cells(keratinocytes) |
| hsa-miR-130b-5p | 208903 | 210945 | Lung, epidermal cells(keratinocytes) |
| hsa-miR-132-3p | 208904 | 210946 | Brain(neuron), immune cells |
| hsa-miR-132-5p | 208905 | 210947 | Brain(neuron), immune cells |
| hsa-miR-133a | 208906 | 210948 | Muscle, heart, epithelial cells (lung) |
| hsa-miR-133b | 208907 | 210949 | Muscle, heart, epithelial cells (lung) |
| hsa-miR-134 | 208908 | 210950 | Lung (epithelial) |
| hsa-miR-135a-3p | 208909 | 210951 | Brain, other tissues |
| hsa-miR-135a-5p | 208910 | 210952 | Brain, other tissues |
| hsa-miR-135b-3p | 208911 | 210953 | Brain, placenta, other tissues |
| hsa-miR-135b-5p | 208912 | 210954 | Brain, placenta, other tissues |
| hsa-miR-136-3p | 208913 | 210955 | Stem cells, placenta |
| hsa-miR-136-5p | 208914 | 210956 | Stem cells, placenta |
| hsa-miR-137 | 208915 | 210957 | Brain |
| hsa-miR-138-1-3p | 208916 | 210958 | Stem cells, epidermal cells (keratinocytes) |
| hsa-miR-138-2-3p | 208917 | 210959 | Stem cells |
| hsa-miR-138-5p | 208918 | 210960 | Stem cells |
| hsa-miR-139-3p | 208919 | 210961 | Hematocytes, brain |
| hsa-miR-139-5p | 208920 | 210962 | Hematocytes, brain |
| hsa-miR-140-3p | 208921 | 210963 | Airway smooth muscle |
| hsa-miR-140-5p | 208922 | 210964 | Cartilage (chondrocytes) |
| hsa-miR-141-3p | 208923 | 210965 | Variety of cells and tissues |
| hsa-miR-141-5p | 208924 | 210966 | Variety of cells and tissues |
| hsa-miR-142-3p | 208925 | 210967 | Myeloid cells, hematopoiesis, APC cells |
| hsa-miR-142-5p | 208926 | 210968 | Myeloid cells, hematopoiesis, APC cells |
| hsa-miR-143-3p | 208927 | 210969 | Vascular smooth muscle |
| hsa-miR-143-5p | 208928 | 210970 | Vascular smooth muscle, T-cells |
| hsa-miR-144-3p | 208929 | 210971 | Erythroid |
| hsa-miR-144-5p | 208930 | 210972 | Erythroid |
| hsa-miR-145-3p | 208931 | 210973 | Kidney, cartilage, vascular smooth muscle |
| hsa-miR-145-5p | 208932 | 210974 | Kidney, cartilage, vascular smooth muscle |
| hsa-miR-146a-3p | 208933 | 210975 | Immune cells, hematopoiesis |
| hsa-miR-146a-5p | 208934 | 210976 | Immune cells, hematopoiesis |
| hsa-miR-146b-3p | 208935 | 210977 | Immune cells |
| hsa-miR-146b-5p | 208936 | 210978 | Embryonic stem cells |
| hsa-miR-147a | 208937 | 210979 | Macrophage |
| hsa-miR-147b | 208938 | 210980 | Macrophage |
| hsa-miR-148a-3p | 208939 | 210981 | Hematopoietic cells |
| hsa-miR-148a-5p | 208940 | 210982 | Hematopoietic cells |
| hsa-miR-148b-3p | 208941 | 210983 | Neuron |
| hsa-miR-148b-5p | 208942 | 210984 | Neuron |
| hsa-miR-149-3p | 208943 | 210985 | Heart and brain |
| hsa-miR-149-5p | 208944 | 210986 | Heart and brain |
| hsa-miR-150-3p | 208945 | 210987 | Hematopoietic cells (lymphoid) |
| hsa-miR-150-5p | 208946 | 210988 | Hematopoietic cells (lymphoid) |
| hsa-miR-151a-3p | 208947 | 210989 | Neuron, fetal liver |
| hsa-miR-151a-5p | 208948 | 210990 | Neuron, fetal liver |
| hsa-miR-151b | 208949 | 210991 | Immune cells (B-cells) |
| hsa-miR-152 | 208950 | 210992 | Liver |
| hsa-miR-153 | 208951 | 210993 | Brain |
| hsa-miR-154-3p | 208952 | 210994 | Embryonic stem cells |
| hsa-miR-154-5p | 208953 | 210995 | Embryonic stem cells |
| hsa-miR-155-3p | 208954 | 210996 | T/B cells, monocytes, breast |
| hsa-miR-155-5p | 208955 | 210997 | T/B cells, monocytes, breast |
| hsa-miR-181a-2-3p | 208956 | 210998 | Glioblast, stem cells |
| hsa-miR-181a-3p | 208957 | 210999 | Glioblast, myeloid cells, embryonic stem cells |
| hsa-miR-181a-5p | 208958 | 211000 | Glioblast, myeloid cells, embryonic stem cells |
| hsa-miR-181b-3p | 208959 | 211001 | Glioblast, Embryonic stem cells, epidermal (keratinocytes) |
| hsa-miR-181b-5p | 208960 | 211002 | Glioblast, Embryonic stem cells, epidermal (keratinocytes) |
| hsa-miR-181c-3p | 208961 | 211003 | Brain, stem cells/progenitor |
| hsa-miR-181c-5p | 208962 | 211004 | Brain, stem cells/progenitor |
| hsa-miR-181d | 208963 | 211005 | Glia cells |
| hsa-miR-182-3p | 208964 | 211006 | Immune cells |
| hsa-miR-182-5p | 208965 | 211007 | Lung, immune cells |
| hsa-miR-183-3p | 208966 | 211008 | Brain |
| hsa-miR-183-5p | 208967 | 211009 | Brain |
| hsa-miR-184 | 208968 | 211010 | Blood, tongue, pancreas (islet) |
| hsa-miR-185-3p | 208969 | 211011 | |
| hsa-miR-185-5p | 208970 | 211012 | |
| hsa-miR-186-3p | 208971 | 211013 | Osteoblasts, heart |
| hsa-miR-186-5p | 208972 | 211014 | Osteoblasts, heart |
| hsa-miR-187-3p | 208973 | 211015 | |
| hsa-miR-187-5p | 208974 | 211016 | |
| hsa-miR-188-3p | 208975 | 211017 | Smooth muscle, central nervous system |
| hsa-miR-188-5p | 208976 | 211018 | Smooth muscle, central nervous system |
| hsa-miR-190a | 208977 | 211019 | Brain |
| hsa-miR-190b | 208978 | 211020 | Brain |
| hsa-miR-191-3p | 208979 | 211021 | |
| hsa-miR-191-5p | 208980 | 211022 | |
| hsa-miR-192-3p | 208981 | 211023 | Kidney |
| hsa-miR-192-5p | 208982 | 211024 | Kidney |
| hsa-miR-193a-3p | 208983 | 211025 | Variety of cells and tissues |
| hsa-miR-193a-5p | 208984 | 211026 | Variety of cells and tissues |
| hsa-miR-193b-3p | 208985 | 211027 | Many tissues/cells, semen |
| hsa-miR-193b-5p | 208986 | 211028 | Many tissues/cells, semen |
| hsa-miR-194-3p | 208987 | 211029 | Kidney, liver |
| hsa-miR-194-5p | 208988 | 211030 | Kidney, liver |
| hsa-miR-195-3p | 208989 | 211031 | Breast, pancreas (islet) |
| hsa-miR-195-5p | 208990 | 211032 | Breast, pancreas (islet) |
| hsa-miR-196a-3p | 208991 | 211033 | Pancreatic cells, endometrial tissues, mesenchymal stem cells |
| hsa-miR-196a-5p | 208992 | 211034 | Pancreatic cells, endometrial tissues, mesenchymal stem cells |
| hsa-miR-196b-3p | 208993 | 211035 | Endometrial tissues |
| hsa-miR-196b-5p | 208994 | 211036 | Endometrial tissues |
| hsa-miR-197-3p | 208995 | 211037 | Blood (myeloid), other tissues/cells |
| hsa-miR-197-5p | 208996 | 211038 | Blood (myeloid), other tissues/cells |
| hsa-miR-198 | 208997 | 211039 | Central nervous system(CNS) |
| hsa-miR-199a-3p | 208998 | 211040 | Liver, embryonic body cells, cardiomyocytes |
| hsa-miR-199a-5p | 208999 | 211041 | Liver, cardiomyocytes |
| hsa-miR-199b-3p | 209000 | 211042 | Liver, osteoblast |
| hsa-miR-199b-5p | 209001 | 211043 | Liver, osteoblast |
| hsa-miR-200a-3p | 209002 | 211044 | Epithelial cells, many other tissues |
| hsa-miR-200a-5p | 209003 | 211045 | Epithelial cells, many other tissues |
| hsa-miR-200b-3p | 209004 | 211046 | Epithelial cells, many other tissues |
| hsa-miR-200b-5p | 209005 | 211047 | Epithelial cells, many other tissues |
| hsa-miR-200c-3p | 209006 | 211048 | Epithelial cells, many other tissues, embryonic stem cells |
| hsa-miR-200c-5p | 209007 | 211049 | Epithelial cells, many other tissues, embryonic stem cells |
| hsa-miR-202-3p | 209008 | 211050 | Blood |
| hsa-miR-202-5p | 209009 | 211051 | Blood |
| hsa-miR-203a | 209010 | 211052 | Skin (epithelium) |
| hsa-miR-203b-3p | 209011 | 211053 | Skin specific (epithelium) |
| hsa-miR-203b-5p | 209012 | 211054 | Skin specific (epithelium) |
| hsa-miR-204-3p | 209013 | 211055 | Adipose, other tissues/cells, Kidney |
| hsa-miR-204-5p | 209014 | 211056 | Adipose, other tissues/cells, kidney |
| hsa-miR-205-3p | 209015 | 211057 | Blood(plasma) |
| hsa-miR-205-5p | 209016 | 211058 | Blood(plasma) |
| hsa-miR-206 | 209017 | 211059 | Muscle (cardiac and skeletal) |
| hsa-miR-208a | 209018 | 211060 | Heart (cardiomyocyte), muscle |
| hsa-miR-208b | 209019 | 211061 | Heart (cardiomyocyte), muscle |
| hsa-miR-210 | 209020 | 211062 | Kidney, heart, vascular endothelial cells |
| hsa-miR-211-3p | 209021 | 211063 | Melanocytes |
| hsa-miR-211-5p | 209022 | 211064 | Melanocytes |
| hsa-miR-212-3p | 209023 | 211065 | Brain(neuron), spleen |
| hsa-miR-212-5p | 209024 | 211066 | Brain(neuron), spleen |
| hsa-miR-214-3p | 209025 | 211067 | Immune cells, pancreas |
| hsa-miR-214-5p | 209026 | 211068 | Immune cells, pancreas |
| hsa-miR-215 | 209027 | 211069 | Variety of cells and tissues |
| hsa-miR-216a-3p | 209028 | 211070 | Kidney, pancreas |
| hsa-miR-216a-5p | 209029 | 211071 | Kidney, pancreas |
| hsa-miR-216b | 209030 | 211072 | |
| hsa-miR-217 | 209031 | 211073 | Endothelial cells |
| hsa-miR-218-1-3p | 209032 | 211074 | Endothelial cells |
| hsa-miR-218-2-3p | 209033 | 211075 | |
| hsa-miR-218-5p | 209034 | 211076 | |
| hsa-miR-219-1-3p | 209035 | 211077 | Brain, oligodendrocytes |
| hsa-miR-219-2-3p | 209036 | 211078 | Brain, oligodendrocytes |
| hsa-miR-219-5p | 209037 | 211079 | Brain, oligodendrocytes |
| hsa-miR-221-3p | 209038 | 211080 | Endothelial cells, immune cells |
| hsa-miR-221-5p | 209039 | 211081 | Endothelial cells, immune cells |
| hsa-miR-222-3p | 209040 | 211082 | Endothelial cells |
| hsa-miR-222-5p | 209041 | 211083 | Endothelial cells |
| hsa-miR-223-3p | 209042 | 211084 | Myeloid cells |
| hsa-miR-223-5p | 209043 | 211085 | Myeloid cells |
| hsa-miR-224-3p | 209044 | 211086 | Blood(plasma), ovary |
| hsa-miR-224-5p | 209045 | 211087 | Blood(plasma), ovary |
| hsa-miR-296-3p | 209046 | 211088 | Kidney, heart, lung, endothelial cells |
| hsa-miR-296-5p | 209047 | 211089 | Lung, liver, endothelial cells |
| hsa-miR-297 | 209048 | 211090 | Oocyte and prostate |
| hsa-miR-298 | 209049 | 211091 | |
| hsa-miR-299-3p | 209050 | 211092 | |
| hsa-miR-299-5p | 209051 | 211093 | |
| hsa-miR-300 | 209052 | 211094 | Osteoblast |
| hsa-miR-301a-3p | 209053 | 211095 | Embryonic stem cells |
| hsa-miR-301a-5p | 209054 | 211096 | Embryonic stem cells |
| hsa-miR-301b | 209055 | 211097 | |
| hsa-miR-302a-3p | 209056 | 211098 | Embryonic stem cells, lipid metabolism |
| hsa-miR-302a-5p | 209057 | 211099 | Embryonic stem cells, lipid metabolism |
| hsa-miR-302b-3p | 209058 | 211100 | Embryonic stem cells |
| hsa-miR-302b-5p | 209059 | 211101 | Embryonic stem cells |
| hsa-miR-302c-3p | 209060 | 211102 | Embryonic stem cells |
| hsa-miR-302c-5p | 209061 | 211103 | Embryonic stem cells |
| hsa-miR-302d-3p | 209062 | 211104 | Embryonic stem cells |
| hsa-miR-302d-5p | 209063 | 211105 | Embryonic stem cells |
| hsa-miR-302e | 209064 | 211106 | Embryonic body cells |
| hsa-miR-302f | 209065 | 211107 | |
| hsa-miR-320a | 209066 | 211108 | Blood, heart (myocardial) |
| hsa-miR-320b | 209067 | 211109 | Central nervous system |
| hsa-miR-320c | 209068 | 211110 | Chondrocyte |
| hsa-miR-320d | 209069 | 211111 | |
| hsa-miR-320e | 209070 | 211112 | Neural cells |
| hsa-miR-323a-3p | 209071 | 211113 | Neurons |
| hsa-miR-323a-5p | 209072 | 211114 | Neurons |
| hsa-miR-323b-3p | 209073 | 211115 | |
| hsa-miR-323b-5p | 209074 | 211116 | |
| hsa-miR-324-3p | 209075 | 211117 | Kidney |
| hsa-miR-324-5p | 209076 | 211118 | Neurons |
| hsa-miR-325 | 209077 | 211119 | Neurons, placenta |
| hsa-miR-326 | 209078 | 211120 | Neurons |
| hsa-miR-328 | 209079 | 211121 | Neuron, blood |
| hsa-miR-329 | 209080 | 211122 | Brain and platelet |
| hsa-miR-330-3p | 209081 | 211123 | |
| hsa-miR-330-5p | 209082 | 211124 | |
| hsa-miR-331-3p | 209083 | 211125 | |
| hsa-miR-331-5p | 209084 | 211126 | Lymphocytes |
| hsa-miR-335-3p | 209085 | 211127 | Kidney, breast |
| hsa-miR-335-5p | 209086 | 211128 | Kidney, breast |
| hsa-miR-337-3p | 209087 | 211129 | Lung |
| hsa-miR-337-5p | 209088 | 211130 | Lung |
| hsa-miR-338-3p | 209089 | 211131 | Epithelial cells, oligodendrocytes |
| hsa-miR-338-5p | 209090 | 211132 | Oligodendrocytes |
| hsa-miR-339-3p | 209091 | 211133 | Immune cell |
| hsa-miR-339-5p | 209092 | 211134 | Immune cell |
| hsa-miR-33a-3p | 209093 | 211135 | Pancreatic islet, lipid metabolism |
| hsa-miR-33a-5p | 209094 | 211136 | Pancreatic islet, lipid metabolism |
| hsa-miR-33b-3p | 209095 | 211137 | Lipid metabolism |
| hsa-miR-33b-5p | 209096 | 211138 | Lipid metabolism |
| hsa-miR-340-3p | 209097 | 211139 | |
| hsa-miR-340-5p | 209098 | 211140 | Embryoid body cells |
| hsa-miR-342-3p | 209099 | 211141 | Brain, circulating plasma |
| hsa-miR-342-5p | 209100 | 211142 | Circulating plasma |
| hsa-miR-345-3p | 209101 | 211143 | Hematopoietic cells |
| hsa-miR-345-5p | 209102 | 211144 | Hematopoietic cells |
| hsa-miR-346 | 209103 | 211145 | Immune cells |
| hsa-miR-361-3p | 209104 | 211146 | Blood, endothelial cells |
| hsa-miR-361-5p | 209105 | 211147 | Endothelial cells |
| hsa-miR-362-3p | 209106 | 211148 | |
| hsa-miR-362-5p | 209107 | 211149 | |
| hsa-miR-363-3p | 209108 | 211150 | Kidnev stem cell, blood cells |
| hsa-miR-363-5p | 209109 | 211151 | Kidney stem cell, blood cells |
| hsa-miR-365a-3p | 209110 | 211152 | |
| hsa-miR-365a-5p | 209111 | 211153 | |
| hsa-miR-365b-3p | 209112 | 211154 | |
| hsa-miR-365b-5p | 209113 | 211155 | |
| hsa-miR-367-3p | 209114 | 211156 | Embryonic stem cells |
| hsa-miR-367-5p | 209115 | 211157 | Embryonic stem cells |
| hsa-miR-369-3p | 209116 | 211158 | Stem cells |
| hsa-miR-369-5p | 209117 | 211159 | Stem cells |
| hsa-miR-370 | 209118 | 211160 | |
| hsa-miR-371a-3p | 209119 | 211161 | Serum |
| hsa-miR-371a-5p | 209120 | 211162 | Serum |
| hsa-miR-371b-3p | 209121 | 211163 | Serum |
| hsa-miR-371b-5p | 209122 | 211164 | Serum |
| hsa-miR-372 | 209123 | 211165 | Hematopoietic cells, lung, placental (blood) |
| hsa-miR-373-3p | 209124 | 211166 | |
| hsa-miR-373-5p | 209125 | 211167 | |
| hsa-miR-374a-3p | 209126 | 211168 | Muscle (myoblasts) |
| hsa-miR-374a-5p | 209127 | 211169 | Muscle (myoblasts) |
| hsa-miR-374b-3p | 209128 | 211170 | Muscle (myoblasts) |
| hsa-miR-374b-5p | 209129 | 211171 | Muscle (myoblasts) |
| hsa-miR-374c-3p | 209130 | 211172 | Muscle (myoblasts) |
| hsa-miR-374c-5p | 209131 | 211173 | Muscle (myoblasts) |
| hsa-miR-375 | 209132 | 211174 | Pancreas (islet) |
| hsa-miR-376a-2-5p | 209133 | 211175 | Hematopoietic cells (erythroid, platelet, and lymphoma) |
| hsa-miR-376a-3p | 209134 | 211176 | Hematopoietic cells (erythroid, platelet, and lymphoma) |
| hsa-miR-376a-5p | 209135 | 211177 | Hematopoietic cells (erythroid, platelet, and lymphoma) |
| hsa-miR-376b-3p | 209136 | 211178 | Blood |
| hsa-miR-376b-5p | 209137 | 211179 | Blood |
| hsa-miR-376c-3p | 209138 | 211180 | Trophoblast |
| hsa-miR-376c-5p | 209139 | 211181 | Trophoblast |
| hsa-miR-377-3p | 209140 | 211182 | Hematopoietic cells |
| hsa-miR-377-5p | 209141 | 211183 | Hematopoietic cells |
| hsa-miR-378a-3p | 209142 | 211184 | Ovary, lipid metabolism |
| hsa-miR-378a-5p | 209143 | 211185 | Ovary, placenta/trophoblast, lipid metabolism |
| hsa-miR-378b | 209144 | 211186 | Lipid metabolism |
| hsa-miR-378c | 209145 | 211187 | Lipid metabolism |
| hsa-miR-378d | 209146 | 211188 | Lipid metabolism |
| hsa-miR-378e | 209147 | 211189 | Lipid metabolism |
| hsa-miR-378f | 209148 | 211190 | Lipid metabolism |
| hsa-miR-378g | 209149 | 211191 | Lipid metabolism |
| hsa-miR-378h | 209150 | 211192 | Lipid metabolism |
| hsa-miR-378i | 209151 | 211193 | Lipid metabolism |
| hsa-miR-378j | 209152 | 211194 | Lipid metabolism |
| hsa-miR-379-3p | 209153 | 211195 | |
| hsa-miR-379-5p | 209154 | 211196 | |
| hsa-miR-380-3p | 209155 | 211197 | Brain |
| hsa-miR-380-5p | 209156 | 211198 | Brain, embryonic stem cells |
| hsa-miR-381-3p | 209157 | 211199 | Chondrogenesis, lung, brain |
| hsa-miR-381-5p | 209158 | 211200 | Chondrogenesis, lung, brain |
| hsa-miR-382-3p | 209159 | 211201 | Renal epithelial cells |
| hsa-miR-382-5p | 209160 | 211202 | Renal epithelial cells |
| hsa-miR-383 | 209161 | 211203 | Testes, brain (medulla) |
| hsa-miR-384 | 209162 | 211204 | Epithelial cells |
| hsa-miR-409-3p | 209163 | 211205 | |
| hsa-miR-409-5p | 209164 | 211206 | |
| hsa-miR-410 | 209165 | 211207 | Brain |
| hsa-miR-411-3p | 209166 | 211208 | |
| hsa-miR-411-5p | 209167 | 211209 | |
| hsa-miR-412 | 209168 | 211210 | |
| hsa-miR-421 | 209169 | 211211 | Endothelial cells |
| hsa-miR-422a | 209170 | 211212 | Plasma |
| hsa-miR-423-3p | 209171 | 211213 | Embryonic stem cells |
| hsa-miR-423-5p | 209172 | 211214 | Heart, embryonic stem cells |
| hsa-miR-424-3p | 209173 | 211215 | Endothelial cells |
| hsa-miR-424-5p | 209174 | 211216 | Endothelial cells |
| hsa-miR-425-3p | 209175 | 211217 | Brain |
| hsa-miR-425-5p | 209176 | 211218 | Brain |
| hsa-miR-429 | 209177 | 211219 | Epithelial cells |
| hsa-miR-431-3p | 209178 | 211220 | |
| hsa-miR-431-5p | 209179 | 211221 | |
| hsa-miR-432-3p | 209180 | 211222 | Myoblast |
| hsa-miR-432-5p | 209181 | 211223 | Myoblast |
| hsa-miR-433 | 209182 | 211224 | |
| hsa-miR-448 | 209183 | 211225 | Liver (hepatocytes) |
| hsa-miR-449a | 209184 | 211226 | Chondrocytes, ciliated epithelial cells |
| hsa-miR-449b-3p | 209185 | 211227 | Ciliated epithelial cells, other tissues |
| hsa-miR-449b-5p | 209186 | 211228 | Ciliated epithelial cells, other tissues |
| hsa-miR-449c-3p | 209187 | 211229 | |
| hsa-miR-449c-5p | 209188 | 211230 | |
| hsa-miR-450a-3p | 209189 | 211231 | |
| hsa-miR-450a-5p | 209190 | 211232 | |
| hsa-miR-450b-3p | 209191 | 211233 | |
| hsa-miR-450b-5p | 209192 | 211234 | |
| hsa-miR-451a | 209193 | 211235 | Heart, central nervous system, epithelial cells |
| hsa-miR-451b | 209194 | 211236 | Heart, central nervous system, epithelial cells |
| hsa-miR-452-3p | 209195 | 211237 | Myoblast |
| hsa-miR-452-5p | 209196 | 211238 | Myoblast |
| hsa-miR-454-3p | 209197 | 211239 | Embryoid body cells, central nervous system, monocytes |
| hsa-miR-454-5p | 209198 | 211240 | Embryoid body cells, central nervous system, monocytes |
| hsa-miR-455-3p | 209199 | 211241 | |
| hsa-miR-455-5p | 209200 | 211242 | |
| hsa-miR-466 | 209201 | 211243 | |
| hsa-miR-483-3p | 209202 | 211244 | |
| hsa-miR-483-5p | 209203 | 211245 | Cartilage (chondrocyte), fetal brain |
| hsa-miR-484 | 209204 | 211246 | |
| hsa-miR-485-3p | 209205 | 211247 | |
| hsa-miR-485-5p | 209206 | 211248 | |
| hsa-miR-486-3p | 209207 | 211249 | Ervthroid cells |
| hsa-miR-486-5p | 209208 | 211250 | Stem cells (adipose) |
| hsa-miR-487a | 209209 | 211251 | |
| hsa-miR-487b | 209210 | 211252 | |
| hsa-miR-488-3p | 209211 | 211253 | |
| hsa-miR-488-5p | 209212 | 211254 | |
| hsa-miR-489 | 209213 | 211255 | Mesenchymal stem cells |
| hsa-miR-490-3p | 209214 | 211256 | |
| hsa-miR-490-5p | 209215 | 211257 | |
| hsa-miR-491-3p | 209216 | 211258 | |
| hsa-miR-491-5p | 209217 | 211259 | |
| hsa-miR-492 | 209218 | 211260 | |
| hsa-miR-493-3p | 209219 | 211261 | Myeloid cells, pancreas (islet) |
| hsa-miR-493-5p | 209220 | 211262 | Myeloid cells, pancreas (islet) |
| hsa-miR-494 | 209221 | 211263 | Epithelial cells |
| hsa-miR-495-3p | 209222 | 211264 | Platelet |
| hsa-miR-495-5p | 209223 | 211265 | Platelet |
| hsa-miR-496 | 209224 | 211266 | Blood |
| hsa-miR-497-3p | 209225 | 211267 | |
| hsa-miR-497-5p | 209226 | 211268 | |
| hsa-miR-498 | 209227 | 211269 | |
| hsa-miR-500a-3p | 209228 | 211270 | |
| hsa-miR-500a-5p | 209229 | 211271 | |
| hsa-miR-500b | 209230 | 211272 | Blood (plasma) |
| hsa-miR-501-3p | 209231 | 211273 | |
| hsa-miR-501-5p | 209232 | 211274 | |
| hsa-miR-502-3p | 209233 | 211275 | |
| hsa-miR-502-5p | 209234 | 211276 | |
| hsa-miR-503-3p | 209235 | 211277 | Ovary |
| hsa-miR-503-5p | 209236 | 211278 | Ovary |
| hsa-miR-504 | 209237 | 211279 | |
| hsa-miR-505-3p | 209238 | 211280 | |
| hsa-miR-505-5p | 209239 | 211281 | |
| hsa-miR-506-3p | 209240 | 211282 | |
| hsa-miR-506-5p | 209241 | 211283 | |
| hsa-miR-507 | 209242 | 211284 | |
| hsa-miR-508-3p | 209243 | 211285 | |
| hsa-miR-508-5p | 209244 | 211286 | Endothelial progenitor cells (epcs) |
| hsa-miR-509-3-5p | 209245 | 211287 | Testis |
| hsa-miR-509-3p | 209246 | 211288 | |
| hsa-miR-509-5p | 209247 | 211289 | |
| hsa-miR-510 | 209248 | 211290 | Brain |
| hsa-miR-511 | 209249 | 211291 | Dendritic cells and macrophages |
| hsa-miR-512-3p | 209250 | 211292 | Embryonic stem cells, placenta |
| hsa-miR-512-5p | 209251 | 211293 | Embryonic stem cells, placenta, |
| hsa-miR-513a-3p | 209252 | 211294 | |
| hsa-miR-513a-5p | 209253 | 211295 | Endothelial cells |
| hsa-miR-513b | 209254 | 211296 | |
| hsa-miR-513c-3p | 209255 | 211297 | |
| hsa-miR-513c-5p | 209256 | 211298 | |
| hsa-miR-514a-3p | 209257 | 211299 | |
| hsa-miR-514a-5p | 209258 | 211300 | |
| hsa-miR-514b-3p | 209259 | 211301 | |
| hsa-miR-514b-5p | 209260 | 211302 | |
| hsa-miR-515-3p | 209261 | 211303 | |
| hsa-miR-515-5p | 209262 | 211304 | Placenta |
| hsa-miR-516a-3p | 209263 | 211305 | Frontal cortex |
| hsa-miR-516a-5p | 209264 | 211306 | Placenta |
| hsa-miR-516b-3p | 209265 | 211307 | |
| hsa-miR-516b-5p | 209266 | 211308 | |
| hsa-miR-517-5p | 209267 | 211309 | Placenta |
| hsa-miR-517a-3p | 209268 | 211310 | Placenta |
| hsa-miR-517b-3p | 209269 | 211311 | Placenta |
| hsa-miR-517c-3p | 209270 | 211312 | Placenta |
| hsa-miR-518a-3p | 209271 | 211313 | |
| hsa-miR-518a-5p | 209272 | 211314 | |
| hsa-miR-518b | 209273 | 211315 | Placenta |
| hsa-miR-518c-3p | 209274 | 211316 | Placenta |
| hsa-miR-518c-5p | 209275 | 211317 | Placenta |
| hsa-miR-518d-3p | 209276 | 211318 | |
| hsa-miR-518d-5p | 209277 | 211319 | |
| hsa-miR-518e-3p | 209278 | 211320 | |
| hsa-miR-518e-5p | 209279 | 211321 | |
| hsa-miR-518f-3p | 209280 | 211322 | Placenta |
| hsa-miR-518f-5p | 209281 | 211323 | Placenta |
| hsa-miR-519a-3p | 209282 | 211324 | Placenta |
| hsa-miR-519a-5p | 209283 | 211325 | Placenta |
| hsa-miR-519b-3p | 209284 | 211326 | |
| hsa-miR-519b-5p | 209285 | 211327 | |
| hsa-miR-519c-3p | 209286 | 211328 | |
| hsa-miR-519c-5p | 209287 | 211329 | |
| hsa-miR-519d | 209288 | 211330 | Placenta |
| hsa-miR-519e-3p | 209289 | 211331 | Placenta |
| hsa-miR-519e-5p | 209290 | 211332 | Placenta |
| hsa-miR-520a-3p | 209291 | 211333 | Placenta |
| hsa-miR-520a-5p | 209292 | 211334 | Placenta |
| hsa-miR-520b | 209293 | 211335 | |
| hsa-miR-520c-3p | 209294 | 211336 | |
| hsa-miR-520c-5p | 209295 | 211337 | |
| hsa-miR-520d-3p | 209296 | 211338 | |
| hsa-miR-520d-5p | 209297 | 211339 | |
| hsa-miR-520e | 209298 | 211340 | |
| hsa-miR-520f | 209299 | 211341 | |
| hsa-miR-520g | 209300 | 211342 | |
| hsa-miR-520h | 209301 | 211343 | Placental specific |
| hsa-miR-521 | 209302 | 211344 | |
| hsa-miR-522-3p | 209303 | 211345 | |
| hsa-miR-522-5p | 209304 | 211346 | |
| hsa-miR-523-3p | 209305 | 211347 | |
| hsa-miR-523-5p | 209306 | 211348 | |
| hsa-miR-524-3p | 209307 | 211349 | |
| hsa-miR-524-5p | 209308 | 211350 | Placental specific |
| hsa-miR-525-3p | 209309 | 211351 | Placental specific |
| hsa-miR-525-5p | 209310 | 211352 | Placental specific |
| hsa-miR-526a | 209311 | 211353 | Placental specific |
| hsa-miR-526b-3p | 209312 | 211354 | Placental specific |
| hsa-miR-526b-5p | 209313 | 211355 | Placental specific |
| hsa-miR-527 | 209314 | 211356 | |
| hsa-miR-532-3p | 209315 | 211357 | |
| hsa-miR-532-5p | 209316 | 211358 | |
| hsa-miR-539-3p | 209317 | 211359 | |
| hsa-miR-539-5p | 209318 | 211360 | |
| hsa-miR-541-3p | 209319 | 211361 | |
| hsa-miR-541-5p | 209320 | 211362 | |
| hsa-miR-542-3p | 209321 | 211363 | Monocytes |
| hsa-miR-542-5p | 209322 | 211364 | |
| hsa-miR-543 | 209323 | 211365 | |
| hsa-miR-544a | 209324 | 211366 | |
| hsa-miR-544b | 209325 | 211367 | |
| hsa-miR-545-3p | 209326 | 211368 | |
| hsa-miR-545-5p | 209327 | 211369 | |
| hsa-miR-548 | 209328 | 211370 | |
| hsa-miR-548-3p | 209329 | 211371 | |
| hsa-miR-548-5p | 209330 | 211372 | |
| hsa-miR-548a | 209331 | 211373 | Colorectal micrornaome |
| hsa-miR-548a-3p | 209332 | 211374 | Colorectal micrornaome |
| hsa-miR-548a-5p | 209333 | 211375 | Colorectal micrornaome |
| hsa-miR-548aa | 209334 | 211376 | Cervical tumor |
| hsa-miR-548ab | 209335 | 211377 | B-cells |
| hsa-miR-548ac | 209336 | 211378 | B-cells |
| hsa-miR-548ad | 209337 | 211379 | B-cells |
| hsa-miR-548ae | 209338 | 211380 | B-cells |
| hsa-miR-548ag | 209339 | 211381 | B-cells |
| hsa-miR-548ah-3p | 209340 | 211382 | B-cells |
| hsa-miR-548ah-5p | 209341 | 211383 | B-cells |
| hsa-miR-548ai | 209342 | 211384 | B-cells |
| hsa-miR-548ai-3p | 209343 | 211385 | B-cells |
| hsa-miR-548ai-5p | 209344 | 211386 | B-cells |
| hsa-miR-548ak | 209345 | 211387 | B-cells |
| hsa-miR-548al | 209346 | 211388 | B-cells |
| hsa-miR-548am-3p | 209347 | 211389 | B-cells |
| hsa-miR-548am-5p | 209348 | 211390 | B-cells |
| hsa-miR-548an | 209349 | 211391 | B-cells |
| hsa-miR-548ao-3p | 209350 | 211392 | |
| hsa-miR-548ao-5p | 209351 | 211393 | |
| hsa-miR-548ap-3p | 209352 | 211394 | |
| hsa-miR-548ap-5p | 209353 | 211395 | |
| hsa-miR-548aq-3p | 209354 | 211396 | |
| hsa-miR-548aq-5p | 209355 | 211397 | |
| hsa-miR-548ar-3p | 209356 | 211398 | |
| hsa-miR-548ar-5p | 209357 | 211399 | |
| hsa-miR-548as-3p | 209358 | 211400 | |
| hsa-miR-548as-5p | 209359 | 211401 | |
| hsa-miR-548at-3p | 209360 | 211402 | |
| hsa-miR-548at-5p | 209361 | 211403 | |
| hsa-miR-548au-3p | 209362 | 211404 | |
| hsa-miR-548au-5p | 209363 | 211405 | |
| hsa-miR-548av-3p | 209364 | 211406 | |
| hsa-miR-548av-5p | 209365 | 211407 | |
| hsa-miR-548aw | 209366 | 211408 | |
| hsa-miR-548ay-3p | 209367 | 211409 | Abnormal skin (psoriasis) |
| hsa-miR-548ay-5p | 209368 | 211410 | Abnormal skin (psoriasis) |
| hsa-miR-548az-3p | 209369 | 211411 | Abnormal skin (psoriasis) |
| hsa-miR-548az-5p | 209370 | 211412 | Abnormal skin (psoriasis) |
| hsa-miR-548b-3p | 209371 | 211413 | Colorectal micrornaome |
| hsa-miR-548b-5p | 209372 | 211414 | Immune cells, frontal cortex |
| hsa-miR-548c-3p | 209373 | 211415 | Colorectal micrornaome |
| hsa-miR-548c-5p | 209374 | 211416 | Immune cells, frontal cortex |
| hsa-miR-548d-3p | 209375 | 211417 | Colorectal micrornaome |
| hsa-miR-548d-5p | 209376 | 211418 | Colorectal micrornaome |
| hsa-miR-548e | 209377 | 211419 | Embryonic stem cells |
| hsa-miR-548f | 209378 | 211420 | Embryonic stem cells |
| hsa-miR-548g-3p | 209379 | 211421 | Embryonic stem cells |
| hsa-miR-548g-5p | 209380 | 211422 | Embryonic stem cells |
| hsa-miR-548h-3p | 209381 | 211423 | Embryonic stem cells |
| hsa-miR-548h-5p | 209382 | 211424 | Embryonic stem cells |
| hsa-miR-548i | 209383 | 211425 | Embryonic stem cells, immune cells |
| hsa-miR-548j | 209384 | 211426 | Immune cells |
| hsa-miR-548k | 209385 | 211427 | Embryonic stem cells |
| hsa-miR-548l | 209386 | 211428 | Embryonic stem cells |
| hsa-miR-548m | 209387 | 211429 | Embryonic stem cells |
| hsa-miR-548n | 209388 | 211430 | Embryonic stem cells, immune cells |
| hsa-miR-548o-3p | 209389 | 211431 | Embryonic stem cells |
| hsa-miR-548o-5p | 209390 | 211432 | Embryonic stem cells |
| hsa-miR-548p | 209391 | 211433 | Embryonic stem cells |
| hsa-miR-548g | 209392 | 211434 | |
| hsa-miR-548s | 209393 | 211435 | Melanoma micrornaome |
| hsa-miR-548t-3p | 209394 | 211436 | Melanoma micrornaome |
| hsa-miR-548t-5p | 209395 | 211437 | Melanoma micrornaome |
| hsa-miR-548u | 209396 | 211438 | Melanoma micrornaome |
| hsa-miR-548w | 209397 | 211439 | Melanoma micrornaome |
| hsa-miR-548v | 209398 | 211440 | |
| hsa-miR-548z | 209399 | 211441 | Cervical tumor |
| hsa-miR-549a | 209400 | 211442 | Colorectal micrornaome |
| hsa-miR-550a-3-5p | 209401 | 211443 | |
| hsa-miR-550a-3p | 209402 | 211444 | |
| hsa-miR-550a-5p | 209403 | 211445 | |
| hsa-miR-550b-2-5p | 209404 | 211446 | Cervical tumor |
| hsa-miR-550b-3p | 209405 | 211447 | Cervical tumor |
| hsa-miR-551a | 209406 | 211448 | |
| hsa-miR-551b-3p | 209407 | 211449 | Hepatocytes |
| hsa-miR-551b-5p | 209408 | 211450 | Hepatocytes |
| hsa-miR-552 | 209409 | 211451 | Colorectal micrornaome |
| hsa-miR-553 | 209410 | 211452 | Colorectal micrornaome |
| hsa-miR-554 | 209411 | 211453 | Colorectal micrornaome |
| hsa-miR-555 | 209412 | 211454 | Colorectal micrornaome |
| hsa-miR-556-3p | 209413 | 211455 | Colorectal micrornaome |
| hsa-miR-556-5p | 209414 | 211456 | Colorectal micrornaome |
| hsa-miR-557 | 209415 | 211457 | Liver (hepatocytes) |
| hsa-miR-558 | 209416 | 211458 | |
| hsa-miR-559 | 209417 | 211459 | |
| hsa-miR-561-3p | 209418 | 211460 | |
| hsa-miR-561-5p | 209419 | 211461 | |
| hsa-miR-562 | 209420 | 211462 | |
| hsa-miR-563 | 209421 | 211463 | Colorectal micrornaome |
| hsa-miR-564 | 209422 | 211464 | |
| hsa-miR-566 | 209423 | 211465 | |
| hsa-miR-567 | 209424 | 211466 | |
| hsa-miR-568 | 209425 | 211467 | Colorectal micrornaome |
| hsa-miR-569 | 209426 | 211468 | |
| hsa-miR-570-3p | 209427 | 211469 | |
| hsa-miR-570-5p | 209428 | 211470 | |
| hsa-miR-571 | 209429 | 211471 | Frontal cortex |
| hsa-miR-572 | 209430 | 211472 | Circulating microrna (in plasma) |
| hsa-miR-573 | 209431 | 211473 | Colorectal micrornaome |
| hsa-miR-574-3p | 209432 | 211474 | Blood (myeloid cells) |
| hsa-miR-574-5p | 209433 | 211475 | Semen |
| hsa-miR-575 | 209434 | 211476 | |
| hsa-miR-576-3p | 209435 | 211477 | Colorectal micrornaome |
| hsa-miR-576-5p | 209436 | 211478 | Cartilage/ chondrocyte |
| hsa-miR-577 | 209437 | 211479 | Colorectal micrornaome |
| hsa-miR-578 | 209438 | 211480 | Colorectal micrornaome |
| hsa-miR-579 | 209439 | 211481 | |
| hsa-miR-580 | 209440 | 211482 | |
| hsa-miR-581 | 209441 | 211483 | Liver(hepatocytes) |
| hsa-miR-582-3p | 209442 | 211484 | Cartilage/chondrocyte |
| hsa-miR-582-5p | 209443 | 211485 | |
| hsa-miR-583 | 209444 | 211486 | |
| hsa-miR-584-3p | 209445 | 211487 | |
| hsa-miR-584-5p | 209446 | 211488 | |
| hsa-miR-585 | 209447 | 211489 | |
| hsa-miR-586 | 209448 | 211490 | Colorectal micrornaome |
| hsa-miR-587 | 209449 | 211491 | Colorectal micrornaome |
| hsa-miR-588 | 209450 | 211492 | Colorectal micrornaome |
| hsa-miR-589-3p | 209451 | 211493 | Mesothelial cells |
| hsa-miR-589-5p | 209452 | 211494 | Mesothelial cells |
| hsa-miR-590-3p | 209453 | 211495 | Cardiomyocytes |
| hsa-miR-590-5p | 209454 | 211496 | Cardiomyocytes |
| hsa-miR-591 | 209455 | 211497 | |
| hsa-miR-592 | 209456 | 211498 | |
| hsa-miR-593-3p | 209457 | 211499 | |
| hsa-miR-593-5p | 209458 | 211500 | |
| hsa-miR-595 | 209459 | 211501 | |
| hsa-miR-596 | 209460 | 211502 | |
| hsa-miR-597 | 209461 | 211503 | Colorectal micrornaome |
| hsa-miR-598 | 209462 | 211504 | Blood (lymphocytes) |
| hsa-miR-599 | 209463 | 211505 | |
| hsa-miR-600 | 209464 | 211506 | Colorectal micrornaome |
| hsa-miR-601 | 209465 | 211507 | |
| hsa-miR-602 | 209466 | 211508 | Oocyte |
| hsa-miR-603 | 209467 | 211509 | |
| hsa-miR-604 | 209468 | 211510 | Colorectal micrornaome |
| hsa-miR-605 | 209469 | 211511 | Colorectal micrornaome |
| hsa-miR-606 | 209470 | 211512 | Colorectal micrornaome |
| hsa-miR-607 | 209471 | 211513 | Colorectal micrornaome |
| hsa-miR-608 | 209472 | 211514 | |
| hsa-miR-609 | 209473 | 211515 | Colorectal micrornaome |
| hsa-miR-610 | 209474 | 211516 | |
| hsa-miR-611 | 209475 | 211517 | |
| hsa-miR-612 | 209476 | 211518 | |
| hsa-miR-613 | 209477 | 211519 | Lipid metabolism |
| hsa-miR-614 | 209478 | 211520 | Circulating micrornas (in Plasma) |
| hsa-miR-615-3p | 209479 | 211521 | |
| hsa-miR-615-5p | 209480 | 211522 | |
| hsa-miR-616-3p | 209481 | 211523 | |
| hsa-miR-616-5p | 209482 | 211524 | |
| hsa-miR-617 | 209483 | 211525 | |
| hsa-miR-618 | 209484 | 211526 | |
| hsa-miR-619 | 209485 | 211527 | Colorectal micrornaome |
| hsa-miR-620 | 209486 | 211528 | Colorectal micrornaome |
| hsa-miR-621 | 209487 | 211529 | |
| hsa-miR-622 | 209488 | 211530 | |
| hsa-miR-623 | 209489 | 211531 | |
| hsa-miR-624-3p | 209490 | 211532 | Chondrocyte |
| hsa-miR-624-5p | 209491 | 211533 | Chondrocyte |
| hsa-miR-625-3p | 209492 | 211534 | Liver (hepatocytes), circulating (blood) |
| hsa-miR-625-5p | 209493 | 211535 | Liver (hepatocytes), circulating (blood) |
| hsa-miR-626 | 209494 | 211536 | Colorectal micrornaome |
| hsa-miR-627 | 209495 | 211537 | |
| hsa-miR-628-3p | 209496 | 211538 | |
| hsa-miR-628-5p | 209497 | 211539 | |
| hsa-miR-629-3p | 209498 | 211540 | |
| hsa-miR-629-5p | 209499 | 211541 | |
| hsa-miR-630 | 209500 | 211542 | Chondrocytes |
| hsa-miR-631 | 209501 | 211543 | Colorectal micrornaome |
| hsa-miR-632 | 209502 | 211544 | |
| hsa-miR-633 | 209503 | 211545 | |
| hsa-miR-634 | 209504 | 211546 | Cartilage/ chondrocyte |
| hsa-miR-635 | 209505 | 211547 | Colorectal micrornaome |
| hsa-miR-636 | 209506 | 211548 | |
| hsa-miR-637 | 209507 | 211549 | Colorectal micrornaome |
| hsa-miR-638 | 209508 | 211550 | |
| hsa-miR-639 | 209509 | 211551 | Colorectal micrornaome |
| hsa-miR-640 | 209510 | 211552 | |
| hsa-miR-641 | 209511 | 211553 | Cartilage/ chondrocyte |
| hsa-miR-642a-3p | 209512 | 211554 | Adipocyte |
| hsa-miR-642a-5p | 209513 | 211555 | Colorectal micrornaome |
| hsa-miR-642b-3p | 209514 | 211556 | Cervical tumor |
| hsa-miR-642b-5p | 209515 | 211557 | Cervical tumor |
| hsa-miR-643 | 209516 | 211558 | Colorectal micrornaome |
| hsa-miR-644a | 209517 | 211559 | |
| hsa-miR-645 | 209518 | 211560 | |
| hsa-miR-646 | 209519 | 211561 | |
| hsa-miR-647 | 209520 | 211562 | |
| hsa-miR-648 | 209521 | 211563 | Circulating micrornas (in Plasma) |
| hsa-miR-649 | 209522 | 211564 | Serum |
| hsa-miR-650 | 209523 | 211565 | |
| hsa-miR-651 | 209524 | 211566 | Colorectal micrornaome |
| hsa-miR-652-3p | 209525 | 211567 | |
| hsa-miR-652-5p | 209526 | 211568 | |
| hsa-miR-653 | 209527 | 211569 | Colorectal micrornaome |
| hsa-miR-654-3p | 209528 | 211570 | Colorectal micrornaome |
| hsa-miR-654-5p | 209529 | 211571 | Bone marrow |
| hsa-miR-655 | 209530 | 211572 | |
| hsa-miR-656 | 209531 | 211573 | |
| hsa-miR-657 | 209532 | 211574 | Oligodendrocytes |
| hsa-miR-658 | 209533 | 211575 | |
| hsa-miR-659-3p | 209534 | 211576 | Myoblast |
| hsa-miR-659-5p | 209535 | 211577 | Myoblast |
| hsa-miR-660-3p | 209536 | 211578 | Myoblast |
| hsa-miR-660-5p | 209537 | 211579 | Myoblast |
| hsa-miR-661 | 209538 | 211580 | |
| hsa-miR-662 | 209539 | 211581 | Endothelial progenitor cells, oocytes |
| hsa-miR-663a | 209540 | 211582 | |
| hsa-miR-663b | 209541 | 211583 | |
| hsa-miR-664a-3p | 209542 | 211584 | Embryonic stem cells |
| hsa-miR-664a-5p | 209543 | 211585 | Embryonic stem cells |
| hsa-miR-664b-3p | 209544 | 211586 | Embryonic stem cells |
| hsa-miR-664b-5p | 209545 | 211587 | Embryonic stem cells |
| hsa-miR-665 | 209546 | 211588 | |
| hsa-miR-668 | 209547 | 211589 | Keratinocytes |
| hsa-miR-670 | 209548 | 211590 | |
| hsa-miR-671-3p | 209549 | 211591 | |
| hsa-miR-671-5p | 209550 | 211592 | |
| hsa-miR-675-3p | 209551 | 211593 | |
| hsa-miR-675-5p | 209552 | 211594 | |
| hsa-miR-676-3p | 209553 | 211595 | Female reproductive tract |
| hsa-miR-676-5p | 209554 | 211596 | Female reproductive tract |
| hsa-miR-708-3p | 209555 | 211597 | |
| hsa-miR-708-5p | 209556 | 211598 | |
| hsa-miR-711 | 209557 | 211599 | |
| hsa-miR-718 | 209558 | 211600 | Blood |
| hsa-miR-744-3p | 209559 | 211601 | Heart |
| hsa-miR-744-5p | 209560 | 211602 | Embryonic stem cells, heart |
| hsa-miR-758-3p | 209561 | 211603 | Cholesterol regulation and brain |
| hsa-miR-758-5p | 209562 | 211604 | Cholesterol regulation and brain |
| hsa-miR-759 | 209563 | 211605 | |
| hsa-miR-760 | 209564 | 211606 | |
| hsa-miR-761 | 209565 | 211607 | |
| hsa-miR-762 | 209566 | 211608 | Corneal epithelial cells |
| hsa-miR-764 | 209567 | 211609 | Osteoblast |
| hsa-miR-765 | 209568 | 211610 | |
| hsa-miR-766-3p | 209569 | 211611 | Embryonic stem cells |
| hsa-miR-766-5p | 209570 | 211612 | Embryonic stem cells |
| hsa-miR-767-3p | 209571 | 211613 | |
| hsa-miR-767-5p | 209572 | 211614 | |
| hsa-miR-769-3p | 209573 | 211615 | |
| hsa-miR-769-5p | 209574 | 211616 | |
| hsa-miR-770-5p | 209575 | 211617 | |
| hsa-miR-802 | 209576 | 211618 | Brain, epithelial cells, hepatocytes |
| hsa-miR-873-3p | 209577 | 211619 | |
| hsa-miR-873-5p | 209578 | 211620 | |
| hsa-miR-874 | 209579 | 211621 | |
| hsa-miR-875-3p | 209580 | 211622 | |
| hsa-miR-875-5p | 209581 | 211623 | |
| hsa-miR-876-3p | 209582 | 211624 | |
| hsa-miR-876-5p | 209583 | 211625 | |
| hsa-miR-877-3p | 209584 | 211626 | |
| hsa-miR-877-5p | 209585 | 211627 | |
| hsa-miR-885-3p | 209586 | 211628 | Embryonic stem cells |
| hsa-miR-885-5p | 209587 | 211629 | Embryonic stem cells |
| hsa-miR-887 | 209588 | 211630 | |
| hsa-miR-888-3p | 209589 | 211631 | |
| hsa-miR-888-5p | 209590 | 211632 | |
| hsa-miR-889 | 209591 | 211633 | |
| hsa-miR-890 | 209592 | 211634 | Epididymis |
| hsa-miR-891a | 209593 | 211635 | Epididymis |
| hsa-miR-891b | 209594 | 211636 | Epididymis |
| hsa-miR-892a | 209595 | 211637 | Epididymis |
| hsa-miR-892b | 209596 | 211638 | Epididymis |
| hsa-miR-892c-3p | 209597 | 211639 | Epididymis |
| hsa-miR-892c-5p | 209598 | 211640 | Epididymis |
| hsa-miR-920 | 209599 | 211641 | Human testis |
| hsa-miR-921 | 209600 | 211642 | Human testis |
| hsa-miR-922 | 209601 | 211643 | Human testis, neuronal tissues |
| hsa-miR-924 | 209602 | 211644 | Human testis |
| hsa-miR-933 | 209603 | 211645 | Cervical cancer |
| hsa-miR-934 | 209604 | 211646 | Cervical cancer |
| hsa-miR-935 | 209605 | 211647 | Blood mononuclear cells |
| hsa-miR-936 | 209606 | 211648 | Skin |
| hsa-miR-937-3p | 209607 | 211649 | |
| hsa-miR-937-5p | 209608 | 211650 | |
| hsa-miR-938 | 209609 | 211651 | |
| hsa-miR-939-3p | 209610 | 211652 | Hepatocytes |
| hsa-miR-939-5p | 209611 | 211653 | Hepatocytes |
| hsa-miR-940 | 209612 | 211654 | Cervical cancer |
| hsa-miR-941 | 209613 | 211655 | Embryonic stem cells |
| hsa-miR-942 | 209614 | 211656 | |
| hsa-miR-943 | 209615 | 211657 | Cervical cancer |
| hsa-miR-944 | 209616 | 211658 | |
| hsa-miR-1178-3p | 209617 | 211659 | |
| hsa-miR-1178-5p | 209618 | 211660 | |
| hsa-miR-1179 | 209619 | 211661 | |
| hsa-miR-1180 | 209620 | 211662 | Sarcoma |
| hsa-miR-1181 | 209621 | 211663 | |
| hsa-miR-1182 | 209622 | 211664 | Placenta |
| hsa-miR-1183 | 209623 | 211665 | |
| hsa-miR-1184 | 209624 | 211666 | Hematopoietic cells |
| hsa-miR-1185-1-3p | 209625 | 211667 | Placenta |
| hsa-miR-1185-2-3p | 209626 | 211668 | Placenta |
| hsa-miR-1185-5p | 209627 | 211669 | Placenta |
| hsa-miR-1193 | 209628 | 211670 | |
| hsa-miR-1197 | 209629 | 211671 | |
| hsa-miR-1200 | 209630 | 211672 | |
| hsa-miR-1202 | 209631 | 211673 | |
| hsa-miR-1203 | 209632 | 211674 | |
| hsa-miR-1204 | 209633 | 211675 | |
| hsa-miR-1205 | 209634 | 211676 | |
| hsa-miR-1206 | 209635 | 211677 | |
| hsa-miR-1207-3p | 209636 | 211678 | |
| hsa-miR-1207-5p | 209637 | 211679 | |
| hsa-miR-1208 | 209638 | 211680 | |
| hsa-miR-1224-3p | 209639 | 211681 | |
| hsa-miR-1224-5p | 209640 | 211682 | |
| hsa-miR-1225-3p | 209641 | 211683 | |
| hsa-miR-1225-5p | 209642 | 211684 | |
| hsa-miR-1226-3p | 209643 | 211685 | |
| hsa-miR-1226-5p | 209644 | 211686 | |
| hsa-miR-1227-3p | 209645 | 211687 | Cartilage/chondrocytes |
| hsa-miR-1227-5p | 209646 | 211688 | Cartilage/chondrocytes |
| hsa-miR-1228-3p | 209647 | 211689 | Liver (hepatocytes) |
| hsa-miR-1228-5p | 209648 | 211690 | Liver (hepatocytes) |
| hsa-miR-1229-3p | 209649 | 211691 | |
| hsa-miR-1229-5p | 209650 | 211692 | |
| hsa-miR-1231 | 209651 | 211693 | |
| hsa-miR-1233-1-5p | 209652 | 211694 | Serum |
| hsa-miR-1233-3p | 209653 | 211695 | Serum |
| hsa-miR-1234-3p | 209654 | 211696 | Embryonic stem cell |
| hsa-miR-1234-5p | 209655 | 211697 | Embryonic stem cell |
| hsa-miR-1236-3p | 209656 | 211698 | Lymphatic endothelial cells |
| hsa-miR-1236-5p | 209657 | 211699 | Lymphatic endothelial cells |
| hsa-miR-1237-3p | 209658 | 211700 | Esophageal cell line KYSE-150R |
| hsa-miR-1237-5p | 209659 | 211701 | Esophageal cell line KYSE-150R |
| hsa-miR-1238-3p | 209660 | 211702 | |
| hsa-miR-1238-5p | 209661 | 211703 | |
| hsa-miR-1243 | 209662 | 211704 | Embryonic stem cells |
| hsa-miR-1244 | 209663 | 211705 | Embryonic stem cells |
| hsa-miR-1245a | 209664 | 211706 | Embryonic stem cells |
| hsa-miR-1245b-3p | 209665 | 211707 | Embryonic stem cells |
| hsa-miR-1245b-5p | 209666 | 211708 | Embryonic stem cells |
| hsa-miR-1246 | 209667 | 211709 | Embryonic stem cells, epithelial cells |
| hsa-miR-1247-3p | 209668 | 211710 | Embryoid body cells |
| hsa-miR-1247-5p | 209669 | 211711 | Embryoid body cells |
| hsa-miR-1248 | 209670 | 211712 | |
| hsa-miR-1249 | 209671 | 211713 | Liver (hepatocytes) |
| hsa-miR-1250 | 209672 | 211714 | Oligodendrocytes |
| hsa-miR-1251 | 209673 | 211715 | Embryonic stem cells |
| hsa-miR-1252 | 209674 | 211716 | Embryonic stem cells |
| hsa-miR-1253 | 209675 | 211717 | Embryonic stem cells |
| hsa-miR-1254 | 209676 | 211718 | Embryonic stem cells |
| hsa-miR-1255a | 209677 | 211719 | Embryonic stem cells |
| hsa-miR-1255b-2-3p | 209678 | 211720 | Embryonic stem cells |
| hsa-miR-1255b-5p | 209679 | 211721 | Embryonic stem cells |
| hsa-miR-1256 | 209680 | 211722 | Embryonic stem cells |
| hsa-miR-1257 | 209681 | 211723 | Embryonic stem cells |
| hsa-miR-1258 | 209682 | 211724 | Embryonic stem cells |
| hsa-miR-1260a | 209683 | 211725 | Periodontal tissue |
| hsa-miR-1260b | 209684 | 211726 | Periodontal tissue |
| hsa-miR-1261 | 209685 | 211727 | Embryonic stem cells |
| hsa-miR-1262 | 209686 | 211728 | Embryoid body cells |
| hsa-miR-1263 | 209687 | 211729 | Embryonic stem cells |
| hsa-miR-1264 | 209688 | 211730 | Embryonic stem cells |
| hsa-miR-1265 | 209689 | 211731 | Embryonic stem cells |
| hsa-miR-1266 | 209690 | 211732 | Embryonic stem cells |
| hsa-miR-1267 | 209691 | 211733 | Embryonic stem cells |
| hsa-miR-1268a | 209692 | 211734 | Embryonic stem cells |
| hsa-miR-1268b | 209693 | 211735 | Embryonic stem cells |
| hsa-miR-1269a | 209694 | 211736 | Embryoid body cells |
| hsa-miR-1269b | 209695 | 211737 | Embryoid body cells |
| hsa-miR-1270 | 209696 | 211738 | Embryonic stem cells |
| hsa-miR-1271-3p | 209697 | 211739 | Brain |
| hsa-miR-1271-5p | 209698 | 211740 | Brain |
| hsa-miR-1272 | 209699 | 211741 | Embryonic stem cells |
| hsa-miR-1273a | 209700 | 211742 | Embryonic stem cells |
| hsa-miR-1273c | 209701 | 211743 | |
| hsa-miR-1273d | 209702 | 211744 | Embryonic stem cells |
| hsa-miR-1273e | 209703 | 211745 | |
| hsa-miR-1273f | 209704 | 211746 | |
| hsa-miR-1273q-3p | 209705 | 211747 | |
| hsa-miR-1273q-5p | 209706 | 211748 | |
| hsa-miR-1275 | 209707 | 211749 | Embryonic stem cells |
| hsa-miR-1276 | 209708 | 211750 | Embryonic stem cells |
| hsa-miR-1277-3p | 209709 | 211751 | Embryoid body cells |
| hsa-miR-1277-5p | 209710 | 211752 | Embryoid body cells |
| hsa-miR-1278 | 209711 | 211753 | Embryonic stem cells |
| hsa-miR-1279 | 209712 | 211754 | Monocytes |
| hsa-miR-1281 | 209713 | 211755 | |
| hsa-miR-1282 | 209714 | 211756 | Embryonic stem cells |
| hsa-miR-1283 | 209715 | 211757 | Placenta |
| hsa-miR-1284 | 209716 | 211758 | |
| hsa-miR-1285-3p | 209717 | 211759 | |
| hsa-miR-1285-5p | 209718 | 211760 | |
| hsa-miR-1286 | 209719 | 211761 | Smooth muscle |
| hsa-miR-1287 | 209720 | 211762 | Embryoid body cells |
| hsa-miR-1288 | 209721 | 211763 | Embryonic stem cells |
| hsa-miR-1289 | 209722 | 211764 | Multiple cell types |
| hsa-miR-1290 | 209723 | 211765 | Embryoid body cells |
| hsa-miR-1291 | 209724 | 211766 | Hepatocytes |
| hsa-miR-1292-3p | 209725 | 211767 | |
| hsa-miR-1292-5p | 209726 | 211768 | |
| hsa-miR-1293 | 209727 | 211769 | Embryonic stem cells |
| hsa-miR-1294 | 209728 | 211770 | Embryonic stem cells |
| hsa-miR-1295a | 209729 | 211771 | |
| hsa-miR-1295b-3p | 209730 | 211772 | |
| hsa-miR-1295b-5p | 209731 | 211773 | |
| hsa-miR-1296 | 209732 | 211774 | |
| hsa-miR-1297 | 209733 | 211775 | Embryonic stem cells |
| hsa-miR-1298 | 209734 | 211776 | |
| hsa-miR-1299 | 209735 | 211777 | Embryonic stem cells |
| hsa-miR-1301 | 209736 | 211778 | |
| hsa-miR-1302 | 209737 | 211779 | |
| hsa-miR-1303 | 209738 | 211780 | Hepatocyte |
| hsa-miR-1304-3p | 209739 | 211781 | |
| hsa-miR-1304-5p | 209740 | 211782 | |
| hsa-miR-1305 | 209741 | 211783 | Embryonic stem cells |
| hsa-miR-1306-3p | 209742 | 211784 | Embryonic stem cells |
| hsa-miR-1306-5p | 209743 | 211785 | Embryonic stem cells |
| hsa-miR-1307-3p | 209744 | 211786 | Embryonic stem cells |
| hsa-miR-1307-5p | 209745 | 211787 | Embryonic stem cells |
| hsa-miR-1321 | 209746 | 211788 | |
| hsa-miR-1322 | 209747 | 211789 | |
| hsa-miR-1323 | 209748 | 211790 | Placenta |
| hsa-miR-1324 | 209749 | 211791 | |
| hsa-miR-1343 | 209750 | 211792 | |
| hsa-miR-1468 | 209751 | 211793 | |
| hsa-miR-1469 | 209752 | 211794 | |
| hsa-miR-1470 | 209753 | 211795 | |
| hsa-miR-1471 | 209754 | 211796 | |
| hsa-miR-1537 | 209755 | 211797 | |
| hsa-miR-1538 | 209756 | 211798 | Blood |
| hsa-miR-1539 | 209757 | 211799 | Esophageal cell line KYSE-150R |
| hsa-miR-1587 | 209758 | 211800 | B-cells |
| hsa-miR-1825 | 209759 | 211801 | |
| hsa-miR-1827 | 209760 | 211802 | |
| hsa-miR-1908 | 209761 | 211803 | |
| hsa-miR-1909-3p | 209762 | 211804 | |
| hsa-miR-1909-5p | 209763 | 211805 | |
| hsa-miR-1910 | 209764 | 211806 | Embryonic stem cells |
| hsa-miR-1911-3p | 209765 | 211807 | Embryonic stem cells, neural precursor |
| hsa-miR-1911-5p | 209766 | 211808 | Embryonic stem cells, neural precursor |
| hsa-miR-1912 | 209767 | 211809 | Embryonic stem cells, neural precursor |
| hsa-miR-1913 | 209768 | 211810 | Embryonic stem cells |
| hsa-miR-1914-3p | 209769 | 211811 | Embryonic stem cells |
| hsa-miR-1914-5p | 209770 | 211812 | Embryonic stem cells |
| hsa-miR-1915-3p | 209771 | 211813 | Embryonic stem cells |
| hsa-miR-1915-5p | 209772 | 211814 | Embryonic stem cells |
| hsa-miR-1972 | 209773 | 211815 | |
| hsa-miR-1973 | 209774 | 211816 | |
| hsa-miR-1976 | 209775 | 211817 | |
| hsa-miR-2052 | 209776 | 211818 | |
| hsa-miR-2053 | 209777 | 211819 | |
| hsa-miR-2054 | 209778 | 211820 | |
| hsa-miR-2110 | 209779 | 211821 | |
| hsa-miR-2113 | 209780 | 211822 | Embryonic stem cells |
| hsa-miR-2114-3p | 209781 | 211823 | Ovary, female reproductive tract |
| hsa-miR-2114-5p | 209782 | 211824 | Ovary, female reproductive tract |
| hsa-miR-2115-3p | 209783 | 211825 | Female reproductive tract |
| hsa-miR-2115-5p | 209784 | 211826 | Female reproductive tract |
| hsa-miR-2116-3p | 209785 | 211827 | |
| hsa-miR-2116-5p | 209786 | 211828 | |
| hsa-miR-2117 | 209787 | 211829 | |
| hsa-miR-2276 | 209788 | 211830 | |
| hsa-miR-2277-3p | 209789 | 211831 | Female reproductive tract |
| hsa-miR-2277-5p | 209790 | 211832 | Female reproductive tract |
| hsa-miR-2278 | 209791 | 211833 | |
| hsa-miR-2355-3p | 209792 | 211834 | Embryonic stem cells |
| hsa-miR-2355-5p | 209793 | 211835 | Embryonic stem cells |
| hsa-miR-2392 | 209794 | 211836 | B-cells |
| hsa-miR-2467-3p | 209795 | 211837 | |
| hsa-miR-2467-5p | 209796 | 211838 | |
| hsa-miR-2681-3p | 209797 | 211839 | |
| hsa-miR-2681-5p | 209798 | 211840 | |
| hsa-miR-2682-3p | 209799 | 211841 | |
| hsa-miR-2682-5p | 209800 | 211842 | |
| hsa-miR-2861 | 209801 | 211843 | Osteoblasts |
| hsa-miR-2909 | 209802 | 211844 | T-Lymphocytes |
| hsa-miR-2964a-3p | 209803 | 211845 | |
| hsa-miR-2964a-5p | 209804 | 211846 | |
| hsa-miR-3064-3p | 209805 | 211847 | |
| hsa-miR-3064-5p | 209806 | 211848 | |
| hsa-miR-3065-3p | 209807 | 211849 | Oligodendrocytes |
| hsa-miR-3065-5p | 209808 | 211850 | Oligodendrocytes |
| hsa-miR-3074-3p | 209809 | 211851 | |
| hsa-miR-3074-5p | 209810 | 211852 | |
| hsa-miR-3115 | 209811 | 211853 | |
| hsa-miR-3116 | 209812 | 211854 | Melanoma miRNAome |
| hsa-miR-3117-3p | 209813 | 211855 | Melanoma miRNAome |
| hsa-miR-3117-5p | 209814 | 211856 | Melanoma miRNAome |
| hsa-miR-3118 | 209815 | 211857 | Melanoma miRNAome |
| hsa-miR-3119 | 209816 | 211858 | Melanoma miRNAome |
| hsa-miR-3120-3p | 209817 | 211859 | Melanoma miRNAome |
| hsa-miR-3120-5p | 209818 | 211860 | Melanoma miRNAome |
| hsa-miR-3121-3p | 209819 | 211861 | Melanoma miRNAome |
| hsa-miR-3121-5p | 209820 | 211862 | Melanoma miRNAome |
| hsa-miR-3122 | 209821 | 211863 | Melanoma miRNAome |
| hsa-miR-3123 | 209822 | 211864 | Melanoma miRNAome |
| hsa-miR-3124-3p | 209823 | 211865 | Melanoma miRNAome, ovary |
| hsa-miR-3124-5p | 209824 | 211866 | Melanoma miRNAome, ovary |
| hsa-miR-3125 | 209825 | 211867 | Melanoma miRNAome |
| hsa-miR-3126-3p | 209826 | 211868 | Melanoma miRNAome, ovary |
| hsa-miR-3126-5p | 209827 | 211869 | Melanoma miRNAome, ovary |
| hsa-miR-3127-3p | 209828 | 211870 | Melanoma miRNAome |
| hsa-miR-3127-5p | 209829 | 211871 | Melanoma miRNAome |
| hsa-miR-3128 | 209830 | 211872 | Melanoma miRNAome |
| hsa-miR-3129-3p | 209831 | 211873 | Melanoma miRNAome, ovary |
| hsa-miR-3129-5p | 209832 | 211874 | Melanoma miRNAome, ovary |
| hsa-miR-3130-3p | 209833 | 211875 | Melanoma miRNAome, ovary |
| hsa-miR-3130-5p | 209834 | 211876 | Melanoma miRNAome, ovary |
| hsa-miR-3131 | 209835 | 211877 | Melanoma miRNAome |
| hsa-miR-3132 | 209836 | 211878 | Melanoma miRNAome |
| hsa-miR-3133 | 209837 | 211879 | Melanoma miRNAome |
| hsa-miR-3134 | 209838 | 211880 | Melanoma miRNAome |
| hsa-miR-3135a | 209839 | 211881 | Melanoma miRNAome |
| hsa-miR-3135b | 209840 | 211882 | B cells |
| hsa-miR-3136-3p | 209841 | 211883 | Melanoma miRNAome |
| hsa-miR-3136-5p | 209842 | 211884 | Melanoma miRNAome |
| hsa-miR-3137 | 209843 | 211885 | Melanoma miRNAome |
| hsa-miR-3138 | 209844 | 211886 | Melanoma miRNAome, ovary |
| hsa-miR-3139 | 209845 | 211887 | Melanoma miRNAome |
| hsa-miR-3140-3p | 209846 | 211888 | Melanoma miRNAome, ovary |
| hsa-miR-3140-5p | 209847 | 211889 | Melanoma miRNAome, ovary |
| hsa-miR-3141 | 209848 | 211890 | Melanoma miRNAome |
| hsa-miR-3142 | 209849 | 211891 | Melanoma miRNAome; immune cells |
| hsa-miR-3143 | 209850 | 211892 | Melanoma miRNAome |
| hsa-miR-3144-3p | 209851 | 211893 | Melanoma miRNAome, ovary |
| hsa-miR-3144-5p | 209852 | 211894 | Melanoma miRNAome, ovary |
| hsa-miR-3145-3p | 209853 | 211895 | Melanoma miRNAome |
| hsa-miR-3145-5p | 209854 | 211896 | Melanoma miRNAome |
| hsa-miR-3146 | 209855 | 211897 | Melanoma miRNAome |
| hsa-miR-3147 | 209856 | 211898 | Melanoma miRNAome |
| hsa-miR-3148 | 209857 | 211899 | Melanoma miRNAome |
| hsa-miR-3149 | 209858 | 211900 | Melanoma miRNAome, ovary |
| hsa-miR-3150a-3p | 209859 | 211901 | Melanoma miRNAome |
| hsa-miR-3150a-5p | 209860 | 211902 | Melanoma miRNAome |
| hsa-miR-3150b-3p | 209861 | 211903 | Melanoma miRNAome |
| hsa-miR-3150b-5p | 209862 | 211904 | Melanoma miRNAome |
| hsa-miR-3151 | 209863 | 211905 | Melanoma miRNAome |
| hsa-miR-3152-3p | 209864 | 211906 | Melanoma miRNAome, ovary |
| hsa-miR-3152-5p | 209865 | 211907 | Melanoma miRNAome, ovary |
| hsa-miR-3153 | 209866 | 211908 | Melanoma miRNAome |
| hsa-miR-3154 | 209867 | 211909 | Melanoma miRNAome |
| hsa-miR-3155a | 209868 | 211910 | Melanoma miRNAome |
| hsa-miR-3155b | 209869 | 211911 | B cells |
| hsa-miR-3156-3p | 209870 | 211912 | Melanoma miRNAome |
| hsa-miR-3156-5p | 209871 | 211913 | Melanoma miRNAome |
| hsa-miR-3157-3p | 209872 | 211914 | Melanoma miRNAome |
| hsa-miR-3157-5p | 209873 | 211915 | Melanoma miRNAome |
| hsa-miR-3158-3p | 209874 | 211916 | Melanoma miRNAome, ovary |
| hsa-miR-3158-5p | 209875 | 211917 | Melanoma miRNAome, ovary |
| hsa-miR-3159 | 209876 | 211918 | Melanoma miRNAome |
| hsa-miR-3160-3p | 209877 | 211919 | Melanoma miRNAome |
| hsa-miR-3160-5p | 209878 | 211920 | Melanoma miRNAome |
| hsa-miR-3161 | 209879 | 211921 | Melanoma miRNAome |
| hsa-miR-3162-3p | 209880 | 211922 | Melanoma miRNAome |
| hsa-miR-3162-5p | 209881 | 211923 | Melanoma miRNAome |
| hsa-miR-3163 | 209882 | 211924 | Melanoma miRNAome |
| hsa-miR-3164 | 209883 | 211925 | Melanoma miRNAome |
| hsa-miR-3165 | 209884 | 211926 | Melanoma miRNAome |
| hsa-miR-3166 | 209885 | 211927 | Melanoma miRNAome |
| hsa-miR-3167 | 209886 | 211928 | Melanoma miRNAome, ovary |
| hsa-miR-3168 | 209887 | 211929 | Melanoma miRNAome |
| hsa-miR-3169 | 209888 | 211930 | Melanoma miRNAome |
| hsa-miR-3170 | 209889 | 211931 | Melanoma miRNAome |
| hsa-miR-3171 | 209890 | 211932 | Melanoma miRNAome, ovary |
| hsa-miR-3173-3p | 209891 | 211933 | Melanoma miRNAome |
| hsa-miR-3173-5p | 209892 | 211934 | Melanoma miRNAome |
| hsa-miR-3174 | 209893 | 211935 | Melanoma miRNAome |
| hsa-miR-3175 | 209894 | 211936 | Melanoma miRNAome, ovary |
| hsa-miR-3176 | 209895 | 211937 | Melanoma miRNAome |
| hsa-miR-3177-3p | 209896 | 211938 | Melanoma miRNAome |
| hsa-miR-3177-5p | 209897 | 211939 | Melanoma miRNAome |
| hsa-miR-3178 | 209898 | 211940 | Melanoma miRNAome |
| hsa-miR-3179 | 209899 | 211941 | Melanoma miRNAome |
| hsa-miR-3180 | 209900 | 211942 | Melanoma miRNAome, ovary |
| hsa-miR-3180-3p | 209901 | 211943 | Breast tunor |
| hsa-miR-3180-5p | 209902 | 211944 | Breast tumor |
| hsa-miR-3181 | 209903 | 211945 | Melanoma miRNAome |
| hsa-miR-3182 | 209904 | 211946 | Melanoma miRNAome |
| hsa-miR-3183 | 209905 | 211947 | Melanoma miRNAome |
| hsa-miR-3184-3p | 209906 | 211948 | Melanoma miRNAome |
| hsa-miR-3184-5p | 209907 | 211949 | Melanoma miRNAome |
| hsa-miR-3185 | 209908 | 211950 | Melanoma miRNAome |
| hsa-miR-3186-3p | 209909 | 211951 | Melanoma miRNAome, ovary |
| hsa-miR-3186-5p | 209910 | 211952 | Melanoma miRNAome, ovary |
| hsa-miR-3187-3p | 209911 | 211953 | Melanoma miRNAome |
| hsa-miR-3187-5p | 209912 | 211954 | Melanoma miRNAome |
| hsa-miR-3188 | 209913 | 211955 | Melanoma miRNAome |
| hsa-miR-3189-3p | 209914 | 211956 | Melanoma miRNAome |
| hsa-miR-3189-5p | 209915 | 211957 | Melanoma miRNAome |
| hsa-miR-3190-3p | 209916 | 211958 | Melanoma miRNAome |
| hsa-miR-3190-5p | 209917 | 211959 | Melanoma miRNAome |
| hsa-miR-3191-3p | 209918 | 211960 | Melanoma miRNAome |
| hsa-miR-3191-5p | 209919 | 211961 | Melanoma miRNAome |
| hsa-miR-3192 | 209920 | 211962 | Melanoma miRNAome |
| hsa-miR-3193 | 209921 | 211963 | Melanoma miRNAome |
| hsa-miR-3194-3p | 209922 | 211964 | Melanoma miRNAome |
| hsa-miR-3194-5p | 209923 | 211965 | Melanoma miRNAome |
| hsa-miR-3195 | 209924 | 211966 | Melanoma miRNAome |
| hsa-miR-3196 | 209925 | 211967 | |
| hsa-miR-3197 | 209926 | 211968 | Melanoma miRNAome |
| hsa-miR-3198 | 209927 | 211969 | Melanoma miRNAome |
| hsa-miR-3199 | 209928 | 211970 | Melanoma miRNAome |
| hsa-miR-3200-3p | 209929 | 211971 | Melanoma miRNAome, ovary |
| hsa-miR-3200-5p | 209930 | 211972 | Melanoma miRNAome, ovary |
| hsa-miR-3201 | 209931 | 211973 | Melanoma miRNAome, |
| hsa-miR-3202 | 209932 | 211974 | Melanoma miRNAome, epithelial cell BEAS2B |
| hsa-miR-3529-3p | 209933 | 211975 | Breast tumor |
| hsa-miR-3529-5p | 209934 | 211976 | Breast tumor |
| hsa-miR-3591-3p | 209935 | 211977 | Breast tumor |
| hsa-miR-3591-5p | 209936 | 211978 | Breast tumor |
| hsa-miR-3605-3p | 209937 | 211979 | Reproductive tracts |
| hsa-miR-3605-5p | 209938 | 211980 | Reproductive tracts |
| hsa-miR-3606-3p | 209939 | 211981 | Cervical tumors |
| hsa-miR-3606-5p | 209940 | 211982 | Cervical tumors |
| hsa-miR-3607-3p | 209941 | 211983 | Cervical tumors |
| hsa-miR-3607-5p | 209942 | 211984 | Cervical tumors |
| hsa-miR-3609 | 209943 | 211985 | Cervical tumors |
| hsa-miR-3610 | 209944 | 211986 | Cervical tumors |
| hsa-miR-3611 | 209945 | 211987 | Cervical tumors |
| hsa-miR-3612 | 209946 | 211988 | Cervical tumors |
| hsa-miR-3613-3p | 209947 | 211989 | Cervical tumors |
| hsa-miR-3613-5p | 209948 | 211990 | Cervical tumors |
| hsa-miR-3614-3p | 209949 | 211991 | Cervical and breast tumors |
| hsa-miR-3614-5p | 209950 | 211992 | Cervical and breast tumors |
| hsa-miR-3615 | 209951 | 211993 | Cervical tumors |
| hsa-miR-3616-3p | 209952 | 211994 | Cervical tumors |
| hsa-miR-3616-5p | 209953 | 211995 | Cervical tumors |
| hsa-miR-3617-3p | 209954 | 211996 | Cervical tumors and psoriasis |
| hsa-miR-3617-5p | 209955 | 211997 | Cervical tumors and psoriasis |
| hsa-miR-3618 | 209956 | 211998 | Cervical tumors |
| hsa-miR-3619-3p | 209957 | 211999 | Breast tumors |
| hsa-miR-3619-5p | 209958 | 212000 | Breast tumors |
| hsa-miR-3620-3p | 209959 | 212001 | Cervical tumors |
| hsa-miR-3620-5p | 209960 | 212002 | Cervical tumors |
| hsa-miR-3621 | 209961 | 212003 | Cervical tumors |
| hsa-miR-3622a-3p | 209962 | 212004 | Breast tumors |
| hsa-miR-3622a-5p | 209963 | 212005 | Breast tumors |
| hsa-miR-3622b-3p | 209964 | 212006 | Cervical tumors |
| hsa-miR-3622b-5p | 209965 | 212007 | Cervical tumors |
| hsa-miR-3646 | 209966 | 212008 | Solid tumor |
| hsa-miR-3648 | 209967 | 212009 | Solid tumor |
| hsa-miR-3649 | 209968 | 212010 | Solid tumor |
| hsa-miR-3650 | 209969 | 212011 | Solid tumor |
| hsa-miR-3651 | 209970 | 212012 | Solid tumor |
| hsa-miR-3652 | 209971 | 212013 | Solid tumor |
| hsa-miR-3653 | 209972 | 212014 | Solid tumor |
| hsa-miR-3654 | 209973 | 212015 | Solid tumor |
| hsa-miR-3655 | 209974 | 212016 | Solid tumor |
| hsa-miR-3656 | 209975 | 212017 | Solid tumor |
| hsa-miR-3657 | 209976 | 212018 | Solid tumor |
| hsa-miR-3658 | 209977 | 212019 | Solid tumor |
| hsa-miR-3659 | 209978 | 212020 | Breast tumors |
| hsa-miR-3660 | 209979 | 212021 | Breast tumors |
| hsa-miR-3661 | 209980 | 212022 | Breast tumors |
| hsa-miR-3662 | 209981 | 212023 | |
| hsa-miR-3663-3p | 209982 | 212024 | |
| hsa-miR-3663-5p | 209983 | 212025 | |
| hsa-miR-3664-3p | 209984 | 212026 | Breast tumors |
| hsa-miR-3664-5p | 209985 | 212027 | Breast tumors |
| hsa-miR-3665 | 209986 | 212028 | Brain |
| hsa-miR-3666 | 209987 | 212029 | Brain |
| hsa-miR-3667-3p | 209988 | 212030 | Peripheral blood |
| hsa-miR-3667-5p | 209989 | 212031 | Peripheral blood |
| hsa-miR-3668 | 209990 | 212032 | Peripheral blood |
| hsa-miR-3669 | 209991 | 212033 | Peripheral blood |
| hsa-miR-3670 | 209992 | 212034 | Peripheral blood |
| hsa-miR-3671 | 209993 | 212035 | Peripheral blood |
| hsa-miR-3672 | 209994 | 212036 | Peripheral blood |
| hsa-miR-3673 | 209995 | 212037 | Peripheral blood |
| hsa-miR-3674 | 209996 | 212038 | Peripheral blood |
| hsa-miR-3675-3p | 209997 | 212039 | Peripheral blood |
| hsa-miR-3675-5p | 209998 | 212040 | Peripheral blood |
| hsa-miR-3676-3p | 209999 | 212041 | Peripheral blood |
| hsa-miR-3676-5p | 210000 | 212042 | Peripheral blood |
| hsa-miR-3677-3p | 210001 | 212043 | Peripheral blood |
| hsa-miR-3677-5p | 210002 | 212044 | Peripheral blood |
| hsa-miR-3678-3p | 210003 | 212045 | Peripheral blood |
| hsa-miR-3678-5p | 210004 | 212046 | Peripheral blood |
| hsa-miR-3679-3p | 210005 | 212047 | Peripheral blood |
| hsa-miR-3679-5p | 210006 | 212048 | Peripheral blood |
| hsa-miR-3680-3p | 210007 | 212049 | Peripheral blood |
| hsa-miR-3680-5p | 210008 | 212050 | Peripheral blood |
| hsa-miR-3681-3p | 210009 | 212051 | Peripheral blood |
| hsa-miR-3681-5p | 210010 | 212052 | Peripheral blood |
| hsa-miR-3682-3p | 210011 | 212053 | Peripheral blood |
| hsa-miR-3682-5p | 210012 | 212054 | Peripheral blood |
| hsa-miR-3683 | 210013 | 212055 | Peripheral blood |
| hsa-miR-3684 | 210014 | 212056 | Peripheral blood |
| hsa-miR-3685 | 210015 | 212057 | Peripheral blood |
| hsa-miR-3686 | 210016 | 212058 | Peripheral blood |
| hsa-miR-3687 | 210017 | 212059 | Peripheral blood |
| hsa-miR-3688-3p | 210018 | 212060 | Breast tumor |
| hsa-miR-3688-5p | 210019 | 212061 | Breast tumor |
| hsa-miR-3689a-3p | 210020 | 212062 | Female reproductive tract |
| hsa-miR-3689a-5p | 210021 | 212063 | Female reproductive tract and peripheral blood |
| hsa-miR-3689b-3p | 210022 | 212064 | Female reproductive tract and peripheral blood |
| hsa-miR-3689b-5p | 210023 | 212065 | Female reproductive tract |
| hsa-miR-3689c | 210024 | 212066 | B cells |
| hsa-miR-3689d | 210025 | 212067 | B cells |
| hsa-miR-3689e | 210026 | 212068 | B cells |
| hsa-miR-3689f | 210027 | 212069 | B cells |
| hsa-miR-3690 | 210028 | 212070 | Peripheral blood |
| hsa-miR-3691-3p | 210029 | 212071 | Peripheral blood |
| hsa-miR-3691-5p | 210030 | 212072 | Peripheral blood |
| hsa-miR-3692-3p | 210031 | 212073 | Peripheral blood |
| hsa-miR-3692-5p | 210032 | 212074 | Peripheral blood |
| hsa-miR-3713 | 210033 | 212075 | Neuroblastoma |
| hsa-miR-3714 | 210034 | 212076 | Neuroblastoma |
| hsa-miR-3907 | 210035 | 212077 | Female reproductive tract |
| hsa-miR-3908 | 210036 | 212078 | Female reproductive tract |
| hsa-miR-3909 | 210037 | 212079 | Female reproductive tract |
| hsa-miR-3910 | 210038 | 212080 | Female reproductive tract |
| hsa-miR-3911 | 210039 | 212081 | Breast tumor and female reproductive tract |
| hsa-miR-3912 | 210040 | 212082 | Female reproductive tract |
| hsa-miR-3913-3p | 210041 | 212083 | Breast tumor and female reproductive tract |
| hsa-miR-3913-5p | 210042 | 212084 | Breast tumor and female reproductive tract |
| hsa-miR-3914 | 210043 | 212085 | Breast tumor and female reproductive tract |
| hsa-miR-3915 | 210044 | 212086 | Female reproductive tract |
| hsa-miR-3916 | 210045 | 212087 | Female reproductive tract |
| hsa-miR-3917 | 210046 | 212088 | Female reproductive tract |
| hsa-miR-3918 | 210047 | 212089 | Female reproductive tract |
| hsa-miR-3919 | 210048 | 212090 | Female reproductive tract |
| hsa-miR-3920 | 210049 | 212091 | Female reproductive tract |
| hsa-miR-3921 | 210050 | 212092 | Female reproductive tract |
| hsa-miR-3922-3p | 210051 | 212093 | Breast tumor and female reproductive tract |
| hsa-miR-3922-5p | 210052 | 212094 | Breast tumor and female reproductive tract |
| hsa-miR-3923 | 210053 | 212095 | Female reproductive tract |
| hsa-miR-3924 | 210054 | 212096 | Female reproductive tract |
| hsa-miR-3925-3p | 210055 | 212097 | Breast tumor and female reproductive tract |
| hsa-miR-3925-5p | 210056 | 212098 | Breast tumor and female reproductive tract |
| hsa-miR-3926 | 210057 | 212099 | Female reproductive tract |
| hsa-miR-3927-3p | 210058 | 212100 | Female reproductive tract and psoriasis |
| hsa-miR-3927-5p | 210059 | 212101 | Female reproductive tract and psoriasis |
| hsa-miR-3928 | 210060 | 212102 | Female reproductive tract |
| hsa-miR-3929 | 210061 | 212103 | Female reproductive tract |
| hsa-miR-3934-3p | 210062 | 212104 | Abnormal skin (psoriasis) |
| hsa-miR-3934-5p | 210063 | 212105 | Abnormal skin (psoriasis) |
| hsa-miR-3935 | 210064 | 212106 | |
| hsa-miR-3936 | 210065 | 212107 | Breast tumor and lymphoblastic leukemia |
| hsa-miR-3937 | 210066 | 212108 | |
| hsa-miR-3938 | 210067 | 212109 | |
| hsa-miR-3939 | 210068 | 212110 | |
| hsa-miR-3940-3p | 210069 | 212111 | Breast tumor |
| hsa-miR-3940-5p | 210070 | 212112 | Breast tumor |
| hsa-miR-3941 | 210071 | 212113 | |
| hsa-miR-3942-3p | 210072 | 212114 | Breast tumor and lymphoblastic leukemia |
| hsa-miR-3942-5p | 210073 | 212115 | Breast tumor and lymphoblastic leukemia |
| hsa-miR-3943 | 210074 | 212116 | |
| hsa-miR-3944-3p | 210075 | 212117 | Breast tumor |
| hsa-miR-3944-5p | 210076 | 212118 | Breast tumor |
| hsa-miR-3945 | 210077 | 212119 | |
| hsa-miR-3960 | 210078 | 212120 | Osteoblast |
| hsa-miR-3972 | 210079 | 212121 | Acute Myeloid Leukemia |
| hsa-miR-3973 | 210080 | 212122 | Acute Myeloid Leukemia |
| hsa-miR-3974 | 210081 | 212123 | Acute Myeloid Leukemia |
| hsa-miR-3975 | 210082 | 212124 | Acute Myeloid Leukemia |
| hsa-miR-3976 | 210083 | 212125 | Acute Myeloid Leukemia |
| hsa-miR-3977 | 210084 | 212126 | Acute Myeloid Leukemia |
| hsa-miR-3978 | 210085 | 212127 | Acute Myeloid Leukemia |
| hsa-miR-4251 | 210086 | 212128 | Embryonic stem cells and neural precursors |
| hsa-miR-4252 | 210087 | 212129 | Embryonic stem cells and neural precursors |
| hsa-miR-4253 | 210088 | 212130 | Embryonic stem cells and neural precursors |
| hsa-miR-4254 | 210089 | 212131 | Embryonic stem cells and neural precursors |
| hsa-miR-4255 | 210090 | 212132 | Embryonic stem cells and neural precursors |
| hsa-miR-4256 | 210091 | 212133 | Embryonic stem cells and neural precursors |
| hsa-miR-4257 | 210092 | 212134 | Embryonic stem cells and neural precursors |
| hsa-miR-4258 | 210093 | 212135 | Embryonic stem cells and neural precursors |
| hsa-miR-4259 | 210094 | 212136 | Embryonic stem cells and neural precursors |
| hsa-miR-4260 | 210095 | 212137 | Embryonic stem cells and neural precursors |
| hsa-miR-4261 | 210096 | 212138 | Embryonic stem cells and neural precursors |
| hsa-miR-4262 | 210097 | 212139 | Embryonic stem cells and neural precursors |
| hsa-miR-4263 | 210098 | 212140 | Embryonic stem cells and neural precursors |
| hsa-miR-4264 | 210099 | 212141 | Embryonic stem cells and neural precursors |
| hsa-miR-4265 | 210100 | 212142 | Embryonic stem cells and neural precursors |
| hsa-miR-4266 | 210101 | 212143 | Embryonic stem cells and neural precursors |
| hsa-miR-4267 | 210102 | 212144 | Embryonic stem cells and neural precursors |
| hsa-miR-4268 | 210103 | 212145 | Embryonic stem cells and neural precursors |
| hsa-miR-4269 | 210104 | 212146 | Embryonic stem cells and neural precursors |
| hsa-miR-4270 | 210105 | 212147 | Embryonic stem cells and neural precursors |
| hsa-miR-4271 | 210106 | 212148 | Embryonic stem cells and neural precursors |
| hsa-miR-4272 | 210107 | 212149 | Embryonic stem cells and neural precursors |
| hsa-miR-4273 | 210108 | 212150 | |
| hsa-miR-4274 | 210109 | 212151 | Embryonic stem cells and neural precursors |
| hsa-miR-4275 | 210110 | 212152 | Embryonic stem cells and neural precursors |
| hsa-miR-4276 | 210111 | 212153 | Embryonic stem cells and neural precursors |
| hsa-miR-4277 | 210112 | 212154 | Embryonic stem cells and neural precursors |
| hsa-miR-4278 | 210113 | 212155 | Embryonic stem cells and neural precursors |
| hsa-miR-4279 | 210114 | 212156 | Embryonic stem cells and neural precursors |
| hsa-miR-4280 | 210115 | 212157 | Embryonic stem cells and neural precursors |
| hsa-miR-4281 | 210116 | 212158 | Embryonic stem cells and neural precursors |
| hsa-miR-4282 | 210117 | 212159 | Embryonic stem cells and neural precursors |
| hsa-miR-4283 | 210118 | 212160 | Embryonic stem cells and neural precursors |
| hsa-miR-4284 | 210119 | 212161 | Embryonic stem cells and neural precursors |
| hsa-miR-4285 | 210120 | 212162 | Embryonic stem cells and neural precursors |
| hsa-miR-4286 | 210121 | 212163 | Embryonic stem cells and neural precursors |
| hsa-miR-4287 | 210122 | 212164 | Embryonic stem cells and neural precursors |
| hsa-miR-4288 | 210123 | 212165 | Embryonic stem cells and neural precursors |
| hsa-miR-4289 | 210124 | 212166 | Embryonic stem cells and neural precursors |
| hsa-miR-4290 | 210125 | 212167 | Embryonic stem cells and neural precursors |
| hsa-miR-4291 | 210126 | 212168 | Embryonic stem cells and neural precursors |
| hsa-miR-4292 | 210127 | 212169 | Embryonic stem cells and neural precursors |
| hsa-miR-4293 | 210128 | 212170 | Embryonic stem cells and neural precursors |
| hsa-miR-4294 | 210129 | 212171 | Embryonic stem cells and neural precursors |
| hsa-miR-4295 | 210130 | 212172 | Embryonic stem cells and neural precursors |
| hsa-miR-4296 | 210131 | 212173 | Embryonic stem cells and neural precursors |
| hsa-miR-4297 | 210132 | 212174 | Embryonic stem cells and neural precursors |
| hsa-miR-4298 | 210133 | 212175 | Embryonic stem cells and neural precursors |
| hsa-miR-4299 | 210134 | 212176 | Embryonic stem cells and neural precursors |
| hsa-miR-4300 | 210135 | 212177 | Embryonic stem cells and neural precursors |
| hsa-miR-4301 | 210136 | 212178 | Embryonic stem cells and neural precursors |
| hsa-miR-4302 | 210137 | 212179 | Embryonic stem cells and neural precursors |
| hsa-miR-4303 | 210138 | 212180 | Embryonic stem cells and neural precursors |
| hsa-miR-4304 | 210139 | 212181 | Embryonic stem cells and neural precursors |
| hsa-miR-4305 | 210140 | 212182 | Embryonic stem cells and neural precursors |
| hsa-miR-4306 | 210141 | 212183 | Embryonic stem cells and neural precursors |
| hsa-miR-4307 | 210142 | 212184 | Embryonic stem cells and neural precursors |
| hsa-miR-4308 | 210143 | 212185 | Embryonic stem cells and neural precursors |
| hsa-miR-4309 | 210144 | 212186 | Embryonic stem cells and neural precursors |
| hsa-miR-4310 | 210145 | 212187 | Embryonic stem cells and neural precursors |
| hsa-miR-4311 | 210146 | 212188 | Embryonic stem cells and neural precursors |
| hsa-miR-4312 | 210147 | 212189 | Embryonic stem cells and neural precursors |
| hsa-miR-4313 | 210148 | 212190 | Embryonic stem cells and neural precursors |
| hsa-miR-4314 | 210149 | 212191 | Embryonic stem cells and neural precursors |
| hsa-miR-4315 | 210150 | 212192 | Embryonic stem cells and neural precursors |
| hsa-miR-4316 | 210151 | 212193 | Embryonic stem cells and neural precursors |
| hsa-miR-4317 | 210152 | 212194 | Embryonic stem cells and neural precursors |
| hsa-miR-4318 | 210153 | 212195 | Embryonic stem cells and neural precursors |
| hsa-miR-4319 | 210154 | 212196 | Embryonic stem cells and neural precursors |
| hsa-miR-4320 | 210155 | 212197 | Embryonic stem cells and neural precursors |
| hsa-miR-4321 | 210156 | 212198 | Embryonic stem cells and neural precursors |
| hsa-miR-4322 | 210157 | 212199 | Embryonic stem cells and neural precursors |
| hsa-miR-4323 | 210158 | 212200 | Embryonic stem cells and neural precursors |
| hsa-miR-4324 | 210159 | 212201 | Embryonic stem cells and neural precursors |
| hsa-miR-4325 | 210160 | 212202 | Embryonic stem cells and neural precursors |
| hsa-miR-4326 | 210161 | 212203 | Embryonic stem cells and neural precursors |
| hsa-miR-4327 | 210162 | 212204 | Embryonic stem cells and neural precursors |
| hsa-miR-4328 | 210163 | 212205 | Embryonic stem cells and neural precursors |
| hsa-miR-4329 | 210164 | 212206 | Embryonic stem cells and neural precursors |
| hsa-miR-4330 | 210165 | 212207 | Embryonic stem cells and neural precursors |
| hsa-miR-4417 | 210166 | 212208 | B cells |
| hsa-miR-4418 | 210167 | 212209 | B cells |
| hsa-miR-4419a | 210168 | 212210 | B cells |
| hsa-miR-4419b | 210169 | 212211 | B cells |
| hsa-miR-4420 | 210170 | 212212 | B cells |
| hsa-miR-4421 | 210171 | 212213 | B cells |
| hsa-miR-4422 | 210172 | 212214 | Breast tumor and B cells |
| hsa-miR-4423-3p | 210173 | 212215 | Breast tumor, B cells and skin (psoriasis) |
| hsa-miR-4423-5p | 210174 | 212216 | Breast tumor B cells and skin (psoriasis) |
| hsa-miR-4424 | 210175 | 212217 | B cells |
| hsa-miR-4425 | 210176 | 212218 | B cells |
| hsa-miR-4426 | 210177 | 212219 | B cells |
| hsa-miR-4427 | 210178 | 212220 | B cells |
| hsa-miR-4428 | 210179 | 212221 | B cells |
| hsa-miR-4429 | 210180 | 212222 | B cells |
| hsa-miR-4430 | 210181 | 212223 | B cells |
| hsa-miR-4431 | 210182 | 212224 | B cells |
| hsa-miR-4432 | 210183 | 212225 | B cells |
| hsa-miR-4433-3p | 210184 | 212226 | B cells |
| hsa-miR-4433-5p | 210185 | 212227 | B cells |
| hsa-miR-4434 | 210186 | 212228 | B cells |
| hsa-miR-4435 | 210187 | 212229 | B cells |
| hsa-miR-4436a | 210188 | 212230 | Breast tumor and B cells |
| hsa-miR-4436b-3p | 210189 | 212231 | Breast tumor |
| hsa-miR-4436b-5p | 210190 | 212232 | Breast tumor |
| hsa-miR-4437 | 210191 | 212233 | B cells |
| hsa-miR-4438 | 210192 | 212234 | B cells |
| hsa-miR-4439 | 210193 | 212235 | B cells |
| hsa-miR-4440 | 210194 | 212236 | B cells |
| hsa-miR-4441 | 210195 | 212237 | B cells |
| hsa-miR-4442 | 210196 | 212238 | B cells |
| hsa-miR-4443 | 210197 | 212239 | B cells |
| hsa-miR-4444 | 210198 | 212240 | B cells |
| hsa-miR-4445-3p | 210199 | 212241 | B cells |
| hsa-miR-4445-5p | 210200 | 212242 | B cells |
| hsa-miR-4446-3p | 210201 | 212243 | Breast tumor and B cells |
| hsa-miR-4446-5p | 210202 | 212244 | Breast tumor and B cells |
| hsa-miR-4447 | 210203 | 212245 | B cells |
| hsa-miR-4448 | 210204 | 212246 | B cells |
| hsa-miR-4449 | 210205 | 212247 | B cells |
| hsa-miR-4450 | 210206 | 212248 | B cells |
| hsa-miR-4451 | 210207 | 212249 | B cells |
| hsa-miR-4452 | 210208 | 212250 | B cells |
| hsa-miR-4453 | 210209 | 212251 | B cells |
| hsa-miR-4454 | 210210 | 212252 | B cells |
| hsa-miR-4455 | 210211 | 212253 | B cells |
| hsa-miR-4456 | 210212 | 212254 | B cells |
| hsa-miR-4457 | 210213 | 212255 | B cells |
| hsa-miR-4458 | 210214 | 212256 | B cells |
| hsa-miR-4459 | 210215 | 212257 | B cells |
| hsa-miR-4460 | 210216 | 212258 | B cells |
| hsa-miR-4461 | 210217 | 212259 | B cells |
| hsa-miR-4462 | 210218 | 212260 | B cells |
| hsa-miR-4463 | 210219 | 212261 | B cells |
| hsa-miR-4464 | 210220 | 212262 | B cells |
| hsa-miR-4465 | 210221 | 212263 | B cells |
| hsa-miR-4466 | 210222 | 212264 | B cells |
| hsa-miR-4467 | 210223 | 212265 | Breast tumor and B cells |
| hsa-miR-4468 | 210224 | 212266 | B cells |
| hsa-miR-4469 | 210225 | 212267 | Breast tumor and B cells |
| hsa-miR-4470 | 210226 | 212268 | B cells |
| hsa-miR-4471 | 210227 | 212269 | Breast tumor and B cells |
| hsa-miR-4472 | 210228 | 212270 | B cells |
| hsa-miR-4473 | 210229 | 212271 | B cells |
| hsa-miR-4474-3p | 210230 | 212272 | Breast tumor, lymphoblastic leukemia and B cells |
| hsa-miR-4474-5p | 210231 | 212273 | Breast tumor, lymphoblastic leukemia and B cells |
| hsa-miR-4475 | 210232 | 212274 | B cells |
| hsa-miR-4476 | 210233 | 212275 | B cells |
| hsa-miR-4477a | 210234 | 212276 | B cells |
| hsa-miR-4477b | 210235 | 212277 | B cells |
| hsa-miR-4478 | 210236 | 212278 | B cells |
| hsa-miR-4479 | 210237 | 212279 | B cells |
| hsa-miR-4480 | 210238 | 212280 | B cells |
| hsa-miR-4481 | 210239 | 212281 | B cells |
| hsa-miR-4482-3p | 210240 | 212282 | B cells |
| hsa-miR-4482-5p | 210241 | 212283 | B cells |
| hsa-miR-4483 | 210242 | 212284 | B cells |
| hsa-miR-4484 | 210243 | 212285 | B cells |
| hsa-miR-4485 | 210244 | 212286 | B cells |
| hsa-miR-4486 | 210245 | 212287 | B cells |
| hsa-miR-4487 | 210246 | 212288 | B cells |
| hsa-miR-4488 | 210247 | 212289 | B cells |
| hsa-miR-4489 | 210248 | 212290 | Breast tumor and B cells |
| hsa-miR-4490 | 210249 | 212291 | B cells |
| hsa-miR-4491 | 210250 | 212292 | B cells |
| hsa-miR-4492 | 210251 | 212293 | B cells |
| hsa-miR-4493 | 210252 | 212294 | B cells |
| hsa-miR-4494 | 210253 | 212295 | B cells |
| hsa-miR-4495 | 210254 | 212296 | B cells |
| hsa-miR-4496 | 210255 | 212297 | B cells |
| hsa-miR-4497 | 210256 | 212298 | B cells |
| hsa-miR-4498 | 210257 | 212299 | B cells |
| hsa-miR-4499 | 210258 | 212300 | B cells |
| hsa-miR-4500 | 210259 | 212301 | B cells |
| hsa-miR-4501 | 210260 | 212302 | B cells |
| hsa-miR-4502 | 210261 | 212303 | B cells |
| hsa-miR-4503 | 210262 | 212304 | B cells |
| hsa-miR-4504 | 210263 | 212305 | B cells |
| hsa-miR-4505 | 210264 | 212306 | B cells |
| hsa-miR-4506 | 210265 | 212307 | B cells |
| hsa-miR-4507 | 210266 | 212308 | B cells |
| hsa-miR-4508 | 210267 | 212309 | B cells |
| hsa-miR-4509 | 210268 | 212310 | B cells |
| hsa-miR-4510 | 210269 | 212311 | B cells |
| hsa-miR-4511 | 210270 | 212312 | B cells |
| hsa-miR-4512 | 210271 | 212313 | B cells |
| hsa-miR-4513 | 210272 | 212314 | B cells |
| hsa-miR-4514 | 210273 | 212315 | B cells |
| hsa-miR-4515 | 210274 | 212316 | B cells |
| hsa-miR-4516 | 210275 | 212317 | B cells |
| hsa-miR-4517 | 210276 | 212318 | B cells |
| hsa-miR-4518 | 210277 | 212319 | B cells |
| hsa-miR-4519 | 210278 | 212320 | B cells |
| hsa-miR-4520a-3p | 210279 | 212321 | Breast tumor and B cells, skin (psoriasis) |
| hsa-miR-4520a-5p | 210280 | 212322 | Breast tumor and B cells, skin (psoriasis) |
| hsa-miR-4520b-3p | 210281 | 212323 | Breast tumor |
| hsa-miR-4520b-5p | 210282 | 212324 | Breast tumor |
| hsa-miR-4521 | 210283 | 212325 | B cells |
| hsa-miR-4522 | 210284 | 212326 | B cells |
| hsa-miR-4523 | 210285 | 212327 | B cells |
| hsa-miR-4524a-3p | 210286 | 212328 | Breast tumor and B cells, skin (psoriasis) |
| hsa-miR-4524a-5p | 210287 | 212329 | Breast tumor and B cells, skin (psoriasis) |
| hsa-miR-4524b-3p | 210288 | 212330 | Breast tumor and B cells, skin (psoriasis) |
| hsa-miR-4524b-5p | 210289 | 212331 | Breast tumor and B cells, skin (psoriasis) |
| hsa-miR-4525 | 210290 | 212332 | B cells |
| hsa-miR-4526 | 210291 | 212333 | Breast tumor and B cells |
| hsa-miR-4527 | 210292 | 212334 | B cells |
| hsa-miR-4528 | 210293 | 212335 | B cells |
| hsa-miR-4529-3p | 210294 | 212336 | Breast tumor and B cells |
| hsa-miR-4529-5p | 210295 | 212337 | Breast tumor and B cells |
| hsa-miR-4530 | 210296 | 212338 | B cells |
| hsa-miR-4531 | 210297 | 212339 | B cells |
| hsa-miR-4532 | 210298 | 212340 | B cells |
| hsa-miR-4533 | 210299 | 212341 | B cells |
| hsa-miR-4534 | 210300 | 212342 | B cells |
| hsa-miR-4535 | 210301 | 212343 | B cells |
| hsa-miR-4536-3p | 210302 | 212344 | B cells |
| hsa-miR-4536-5p | 210303 | 212345 | B cells |
| hsa-miR-4537 | 210304 | 212346 | B cells |
| hsa-miR-4538 | 210305 | 212347 | B cells |
| hsa-miR-4539 | 210306 | 212348 | B cells |
| hsa-miR-4540 | 210307 | 212349 | B cells |
| hsa-miR-4632-3p | 210308 | 212350 | Breast tumor |
| hsa-miR-4632-5p | 210309 | 212351 | Breast tumor |
| hsa-miR-4633-3p | 210310 | 212352 | Breast tumor |
| hsa-miR-4633-5p | 210311 | 212353 | Breast tumor |
| hsa-miR-4634 | 210312 | 212354 | Breast tumor |
| hsa-miR-4635 | 210313 | 212355 | Breast tumor |
| hsa-miR-4636 | 210314 | 212356 | Breast tumor |
| hsa-miR-4637 | 210315 | 212357 | Breast tumor and lymphoblastic leukemia |
| hsa-miR-4638-3p | 210316 | 212358 | Breast tumor |
| hsa-miR-4638-5p | 210317 | 212359 | Breast tumor |
| hsa-miR-4639-3p | 210318 | 212360 | Breast tumor |
| hsa-miR-4639-5p | 210319 | 212361 | Breast tumor |
| hsa-miR-4640-3p | 210320 | 212362 | Breast tumor |
| hsa-miR-4640-5p | 210321 | 212363 | Breast tumor |
| hsa-miR-4641 | 210322 | 212364 | Breast tumor |
| hsa-miR-4642 | 210323 | 212365 | Breast tumor |
| hsa-miR-4643 | 210324 | 212366 | Breast tumor |
| hsa-miR-4644 | 210325 | 212367 | Breast tumor |
| hsa-miR-4645-3p | 210326 | 212368 | Breast tumor |
| hsa-miR-4645-5p | 210327 | 212369 | Breast tumor |
| hsa-miR-4646-3p | 210328 | 212370 | Breast tumor |
| hsa-miR-4646-5p | 210329 | 212371 | Breast tumor |
| hsa-miR-4647 | 210330 | 212372 | Breast tumor |
| hsa-miR-4648 | 210331 | 212373 | Breast tumor |
| hsa-miR-4649-3p | 210332 | 212374 | Breast tumor |
| hsa-miR-4649-5p | 210333 | 212375 | Breast tumor |
| hsa-miR-4650-3p | 210334 | 212376 | Breast tumor |
| hsa-miR-4650-5p | 210335 | 212377 | Breast tumor |
| hsa-miR-4651 | 210336 | 212378 | Breast tumor |
| hsa-miR-4652-3p | 210337 | 212379 | Breast tumor |
| hsa-miR-4652-5p | 210338 | 212380 | Breast tumor |
| hsa-miR-4653-3p | 210339 | 212381 | Breast tumor |
| hsa-miR-4653-5p | 210340 | 212382 | Breast tumor |
| hsa-miR-4654 | 210341 | 212383 | Breast tumor |
| hsa-miR-4655-3p | 210342 | 212384 | Breast tumor |
| hsa-miR-4655-5p | 210343 | 212385 | Breast tumor |
| hsa-miR-4656 | 210344 | 212386 | Breast tumor |
| hsa-miR-4657 | 210345 | 212387 | Breast tumor |
| hsa-miR-4658 | 210346 | 212388 | Breast tumor |
| hsa-miR-4659a-3p | 210347 | 212389 | Breast tumor |
| hsa-miR-4659a-5p | 210348 | 212390 | Breast tumor |
| hsa-miR-4659b-3p | 210349 | 212391 | Breast tumor |
| hsa-miR-4659b-5p | 210350 | 212392 | Breast tumor |
| hsa-miR-4660 | 210351 | 212393 | Breast tumor |
| hsa-miR-4661-3p | 210352 | 212394 | Breast tumor |
| hsa-miR-4661-5p | 210353 | 212395 | Breast tumor |
| hsa-miR-4662a-3p | 210354 | 212396 | Breast tumor, psoriasis |
| hsa-miR-4662a-5p | 210355 | 212397 | Breast tumor, psoriasis |
| hsa-miR-4662b | 210356 | 212398 | Breast tumor |
| hsa-miR-4663 | 210357 | 212399 | Breast tumor |
| hsa-miR-4664-3p | 210358 | 212400 | Breast tumor |
| hsa-miR-4664-5p | 210359 | 212401 | Breast tumor |
| hsa-miR-4665-3p | 210360 | 212402 | Breast tumor |
| hsa-miR-4665-5p | 210361 | 212403 | Breast tumor |
| hsa-miR-4666a-3p | 210362 | 212404 | Breast tumor |
| hsa-miR-4666a-5p | 210363 | 212405 | Breast tumor |
| hsa-miR-4666b | 210364 | 212406 | |
| hsa-miR-4667-3p | 210365 | 212407 | Breast tumor |
| hsa-miR-4667-5p | 210366 | 212408 | Breast tumor |
| hsa-miR-4668-3p | 210367 | 212409 | Breast tumor |
| hsa-miR-4668-5p | 210368 | 212410 | Breast tumor |
| hsa-miR-4669 | 210369 | 212411 | Breast tumor |
| hsa-miR-4670-3p | 210370 | 212412 | Breast tumor |
| hsa-miR-4670-5p | 210371 | 212413 | Breast tumor |
| hsa-miR-4671-3p | 210372 | 212414 | Breast tumor |
| hsa-miR-4671-5p | 210373 | 212415 | Breast tumor |
| hsa-miR-4672 | 210374 | 212416 | Breast tumor |
| hsa-miR-4673 | 210375 | 212417 | Breast tumor |
| hsa-miR-4674 | 210376 | 212418 | Breast tumor |
| hsa-miR-4675 | 210377 | 212419 | Breast tumor |
| hsa-miR-4676-3p | 210378 | 212420 | Breast tumor |
| hsa-miR-4676-5p | 210379 | 212421 | Breast tumor |
| hsa-miR-4677-3p | 210380 | 212422 | Breast tumor, psoriasis |
| hsa-miR-4677-5p | 210381 | 212423 | Breast tumor, psoriasis |
| hsa-miR-4678 | 210382 | 212424 | Breast tumor |
| hsa-miR-4679 | 210383 | 212425 | Breast tumor |
| hsa-miR-4680-3p | 210384 | 212426 | Breast tumor |
| hsa-miR-4680-5p | 210385 | 212427 | Breast tumor |
| hsa-miR-4681 | 210386 | 212428 | Breast tumor |
| hsa-miR-4682 | 210387 | 212429 | Breast tumor |
| hsa-miR-4683 | 210388 | 212430 | Breast tumor |
| hsa-miR-4684-3p | 210389 | 212431 | Breast tumor |
| hsa-miR-4684-5p | 210390 | 212432 | Breast tumor |
| hsa-miR-4685-3p | 210391 | 212433 | Breast tumor |
| hsa-miR-4685-5p | 210392 | 212434 | Breast tumor |
| hsa-miR-4686 | 210393 | 212435 | Breast tumor |
| hsa-miR-4687-3p | 210394 | 212436 | Breast tumor |
| hsa-miR-4687-5p | 210395 | 212437 | Breast tumor |
| hsa-miR-4688 | 210396 | 212438 | Breast tumor |
| hsa-miR-4689 | 210397 | 212439 | Breast tumor |
| hsa-miR-4690-3p | 210398 | 212440 | Breast tumor |
| hsa-miR-4690-5p | 210399 | 212441 | Breast tumor |
| hsa-miR-4691-3p | 210400 | 212442 | Breast tumor |
| hsa-miR-4691-5p | 210401 | 212443 | Breast tumor |
| hsa-miR-4692 | 210402 | 212444 | Breast tumor |
| hsa-miR-4693-3p | 210403 | 212445 | Breast tumor |
| hsa-miR-4693-5p | 210404 | 212446 | Breast tumor |
| hsa-miR-4694-3p | 210405 | 212447 | Breast tumor |
| hsa-miR-4694-5p | 210406 | 212448 | Breast tumor |
| hsa-miR-4695-3p | 210407 | 212449 | Breast tumor |
| hsa-miR-4695-5p | 210408 | 212450 | Breast tumor |
| hsa-miR-4696 | 210409 | 212451 | Breast tumor |
| hsa-miR-4697-3p | 210410 | 212452 | Breast tumor |
| hsa-miR-4697-5p | 210411 | 212453 | Breast tumor |
| hsa-miR-4698 | 210412 | 212454 | Breast tumor |
| hsa-miR-4699-3p | 210413 | 212455 | Breast tumor |
| hsa-miR-4699-5p | 210414 | 212456 | Breast tumor |
| hsa-miR-4700-3p | 210415 | 212457 | Breast tumor |
| hsa-miR-4700-5p | 210416 | 212458 | Breast tumor |
| hsa-miR-4701-3p | 210417 | 212459 | Breast tumor |
| hsa-miR-4701-5p | 210418 | 212460 | Breast tumor |
| hsa-miR-4703-3p | 210419 | 212461 | Breast tumor |
| hsa-miR-4703-5p | 210420 | 212462 | Breast tumor |
| hsa-miR-4704-3p | 210421 | 212463 | Breast tumor |
| hsa-miR-4704-5p | 210422 | 212464 | Breast tumor |
| hsa-miR-4705 | 210423 | 212465 | Breast tumor |
| hsa-miR-4706 | 210424 | 212466 | Breast tumor |
| hsa-miR-4707-3p | 210425 | 212467 | Breast tumor |
| hsa-miR-4707-5p | 210426 | 212468 | Breast tumor |
| hsa-miR-4708-3p | 210427 | 212469 | Breast tumor |
| hsa-miR-4708-5p | 210428 | 212470 | Breast tumor |
| hsa-miR-4709-3p | 210429 | 212471 | Breast tumor |
| hsa-miR-4709-5p | 210430 | 212472 | Breast tumor |
| hsa-miR-4710 | 210431 | 212473 | Breast tumor |
| hsa-miR-4711-3p | 210432 | 212474 | Breast tumor |
| hsa-miR-4711-5p | 210433 | 212475 | Breast tumor |
| hsa-miR-4712-3p | 210434 | 212476 | Breast tumor |
| hsa-miR-4712-5p | 210435 | 212477 | Breast tumor |
| hsa-miR-4713-3p | 210436 | 212478 | Breast tumor |
| hsa-miR-4713-5p | 210437 | 212479 | Breast tumor |
| hsa-miR-4714-3p | 210438 | 212480 | Breast tumor |
| hsa-miR-4714-5p | 210439 | 212481 | Breast tumor |
| hsa-miR-4715-3p | 210440 | 212482 | Breast tumor |
| hsa-miR-4715-5p | 210441 | 212483 | Breast tumor |
| hsa-miR-4716-3p | 210442 | 212484 | Breast tumor |
| hsa-miR-4716-5p | 210443 | 212485 | Breast tumor |
| hsa-miR-4717-3p | 210444 | 212486 | Breast tumor |
| hsa-miR-4717-5p | 210445 | 212487 | Breast tumor |
| hsa-miR-4718 | 210446 | 212488 | Breast tumor |
| hsa-miR-4719 | 210447 | 212489 | Breast tumor |
| hsa-miR-4720-3p | 210448 | 212490 | Breast tumor |
| hsa-miR-4720-5p | 210449 | 212491 | Breast tumor |
| hsa-miR-4721 | 210450 | 212492 | Breast tumor |
| hsa-miR-4722-3p | 210451 | 212493 | Breast tumor |
| hsa-miR-4722-5p | 210452 | 212494 | Breast tumor |
| hsa-miR-4723-3p | 210453 | 212495 | Breast tumor |
| hsa-miR-4723-5p | 210454 | 212496 | Breast tumor |
| hsa-miR-4724-3p | 210455 | 212497 | Breast tumor |
| hsa-miR-4724-5p | 210456 | 212498 | Breast tumor |
| hsa-miR-4725-3p | 210457 | 212499 | Breast tumor |
| hsa-miR-4725-5p | 210458 | 212500 | Breast tumor |
| hsa-miR-4726-3p | 210459 | 212501 | Breast tumor |
| hsa-miR-4726-5p | 210460 | 212502 | Breast tumor |
| hsa-miR-4727-3p | 210461 | 212503 | Breast tumor |
| hsa-miR-4727-5p | 210462 | 212504 | Breast tumor |
| hsa-miR-4728-3p | 210463 | 212505 | Breast tumor |
| hsa-miR-4728-5p | 210464 | 212506 | Breast tumor |
| hsa-miR-4729 | 210465 | 212507 | Breast tumor |
| hsa-miR-4730 | 210466 | 212508 | Breast tumor |
| hsa-miR-4731-3p | 210467 | 212509 | Breast tumor |
| hsa-miR-4731-5p | 210468 | 212510 | Breast tumor |
| hsa-miR-4732-3p | 210469 | 212511 | Breast tumor |
| hsa-miR-4732-5p | 210470 | 212512 | Breast tumor |
| hsa-miR-4733-3p | 210471 | 212513 | Breast tumor |
| hsa-miR-4733-5p | 210472 | 212514 | Breast tumor |
| hsa-miR-4734 | 210473 | 212515 | Breast tumor |
| hsa-miR-4735-3p | 210474 | 212516 | Breast tumor |
| hsa-miR-4735-5p | 210475 | 212517 | Breast tumor |
| hsa-miR-4736 | 210476 | 212518 | Breast tumor |
| hsa-miR-4737 | 210477 | 212519 | Breast tumor |
| hsa-miR-4738-3p | 210478 | 212520 | Breast tumor |
| hsa-miR-4738-5p | 210479 | 212521 | Breast tumor |
| hsa-miR-4739 | 210480 | 212522 | Breast tumor |
| hsa-miR-4740-3p | 210481 | 212523 | Breast tumor |
| hsa-miR-4740-5p | 210482 | 212524 | Breast tumor |
| hsa-miR-4741 | 210483 | 212525 | Breast tumor, psoriasis |
| hsa-miR-4742-3p | 210484 | 212526 | Breast tumor, psoriasis |
| hsa-miR-4742-5p | 210485 | 212527 | Breast tumor |
| hsa-miR-4743-3p | 210486 | 212528 | Breast tumor |
| hsa-miR-4743-5p | 210487 | 212529 | Breast tumor |
| hsa-miR-4744 | 210488 | 212530 | Breast tumor |
| hsa-miR-4745-3p | 210489 | 212531 | Breast tumor |
| hsa-miR-4745-5p | 210490 | 212532 | Breast tumor |
| hsa-miR-4746-3p | 210491 | 212533 | Breast tumor |
| hsa-miR-4746-5p | 210492 | 212534 | Breast tumor |
| hsa-miR-4747-3p | 210493 | 212535 | Breast tumor |
| hsa-miR-4747-5p | 210494 | 212536 | Breast tumor |
| hsa-miR-4748 | 210495 | 212537 | Breast tumor |
| hsa-miR-4749-3p | 210496 | 212538 | Breast tumor |
| hsa-miR-4749-5p | 210497 | 212539 | Breast tumor |
| hsa-miR-4750-3p | 210498 | 212540 | Breast tumor |
| hsa-miR-4750-5p | 210499 | 212541 | Breast tumor |
| hsa-miR-4751 | 210500 | 212542 | Breast tumor |
| hsa-miR-4752 | 210501 | 212543 | Breast tumor |
| hsa-miR-4753-3p | 210502 | 212544 | Breast tumor |
| hsa-miR-4753-5p | 210503 | 212545 | Breast tumor |
| hsa-miR-4754 | 210504 | 212546 | Breast tumor |
| hsa-miR-4755-3p | 210505 | 212547 | Breast tumor |
| hsa-miR-4755-5p | 210506 | 212548 | Breast tumor |
| hsa-miR-4756-3p | 210507 | 212549 | Breast tumor |
| hsa-miR-4756-5p | 210508 | 212550 | Breast tumor |
| hsa-miR-4757-3p | 210509 | 212551 | Breast tumor |
| hsa-miR-4757-5p | 210510 | 212552 | Breast tumor |
| hsa-miR-4758-3p | 210511 | 212553 | Breast tumor |
| hsa-miR-4758-5p | 210512 | 212554 | Breast tumor |
| hsa-miR-4759 | 210513 | 212555 | Breast tumor |
| hsa-miR-4760-3p | 210514 | 212556 | Breast tumor |
| hsa-miR-4760-5p | 210515 | 212557 | Breast tumor |
| hsa-miR-4761-3p | 210516 | 212558 | Breast tumor |
| hsa-miR-4761-5p | 210517 | 212559 | Breast tumor |
| hsa-miR-4762-3p | 210518 | 212560 | Breast tumor |
| hsa-miR-4762-5p | 210519 | 212561 | Breast tumor |
| hsa-miR-4763-3p | 210520 | 212562 | Breast tumor |
| hsa-miR-4763-5p | 210521 | 212563 | Breast tumor |
| hsa-miR-4764-3p | 210522 | 212564 | Breast tumor |
| hsa-miR-4764-5p | 210523 | 212565 | Breast tumor |
| hsa-miR-4765 | 210524 | 212566 | Breast tumor |
| hsa-miR-4766-3p | 210525 | 212567 | Breast tumor |
| hsa-miR-4766-5p | 210526 | 212568 | Breast tumor |
| hsa-miR-4767 | 210527 | 212569 | Breast tumor |
| hsa-miR-4768-3p | 210528 | 212570 | Breast tumor |
| hsa-miR-4768-5p | 210529 | 212571 | Breast tumor |
| hsa-miR-4769-3p | 210530 | 212572 | Breast tumor |
| hsa-miR-4769-5p | 210531 | 212573 | Breast tumor |
| hsa-miR-4770 | 210532 | 212574 | Breast tumor |
| hsa-miR-4771 | 210533 | 212575 | Breast tumor |
| hsa-miR-4772-3p | 210534 | 212576 | Breast tumor, blood mononuclear cells |
| hsa-miR-4772-5p | 210535 | 212577 | Breast tumor, blood mononuclear cells |
| hsa-miR-4773 | 210536 | 212578 | Breast tumor |
| hsa-miR-4774-3p | 210537 | 212579 | Breast tumor and Lymphoblastic leukemia |
| hsa-miR-4774-5p | 210538 | 212580 | Breast tumor and Lymphoblastic leukemia |
| hsa-miR-4775 | 210539 | 212581 | Breast tumor |
| hsa-miR-4776-3p | 210540 | 212582 | Breast tumor |
| hsa-miR-4776-5p | 210541 | 212583 | Breast tumor |
| hsa-miR-4777-3p | 210542 | 212584 | Breast tumor |
| hsa-miR-4777-5p | 210543 | 212585 | Breast tumor |
| hsa-miR-4778-3p | 210544 | 212586 | Breast tumor |
| hsa-miR-4778-5p | 210545 | 212587 | Breast tumor |
| hsa-miR-4779 | 210546 | 212588 | Breast tumor |
| hsa-miR-4780 | 210547 | 212589 | Breast tumor |
| hsa-miR-4781-3p | 210548 | 212590 | Breast tumor |
| hsa-miR-4781-5p | 210549 | 212591 | Breast tumor |
| hsa-miR-4782-3p | 210550 | 212592 | Breast tumor |
| hsa-miR-4782-5p | 210551 | 212593 | Breast tumor |
| hsa-miR-4783-3p | 210552 | 212594 | Breast tumor |
| hsa-miR-4783-5p | 210553 | 212595 | Breast tumor |
| hsa-miR-4784 | 210554 | 212596 | Breast tumor |
| hsa-miR-4785 | 210555 | 212597 | Breast tumor |
| hsa-miR-4786-3p | 210556 | 212598 | Breast tumor |
| hsa-miR-4786-5p | 210557 | 212599 | Breast tumor |
| hsa-miR-4787-3p | 210558 | 212600 | Breast tumor |
| hsa-miR-4787-5p | 210559 | 212601 | Breast tumor |
| hsa-miR-4788 | 210560 | 212602 | Breast tumor |
| hsa-miR-4789-3p | 210561 | 212603 | Breast tumor |
| hsa-miR-4789-5p | 210562 | 212604 | Breast tumor |
| hsa-miR-4790-3p | 210563 | 212605 | Breast tumor |
| hsa-miR-4790-5p | 210564 | 212606 | Breast tumor |
| hsa-miR-4791 | 210565 | 212607 | Breast tumor |
| hsa-miR-4792 | 210566 | 212608 | Breast tumor |
| hsa-miR-4793-3p | 210567 | 212609 | Breast tumor |
| hsa-miR-4793-5p | 210568 | 212610 | Breast tumor |
| hsa-miR-4794 | 210569 | 212611 | Breast tumor |
| hsa-miR-4795-3p | 210570 | 212612 | Breast tumor |
| hsa-miR-4795-5p | 210571 | 212613 | Breast tumor |
| hsa-miR-4796-3p | 210572 | 212614 | Breast tumor |
| hsa-miR-4796-5p | 210573 | 212615 | Breast tumor |
| hsa-miR-4797-3p | 210574 | 212616 | Breast tumor |
| hsa-miR-4797-5p | 210575 | 212617 | Breast tumor |
| hsa-miR-4798-3p | 210576 | 212618 | Breast tumor |
| hsa-miR-4798-5p | 210577 | 212619 | Breast tumor |
| hsa-miR-4799-3p | 210578 | 212620 | Breast tumor |
| hsa-miR-4799-5p | 210579 | 212621 | Breast tumor |
| hsa-miR-4800-3p | 210580 | 212622 | Breast tumor |
| hsa-miR-4800-5p | 210581 | 212623 | Breast tumor |
| hsa-miR-4801 | 210582 | 212624 | Breast tumor |
| hsa-miR-4802-3p | 210583 | 212625 | Breast tumor, psoriasis |
| hsa-miR-4802-5p | 210584 | 212626 | Breast tumor, psoriasis |
| hsa-miR-4803 | 210585 | 212627 | Breast tumor |
| hsa-miR-4804-3p | 210586 | 212628 | Breast tumor |
| hsa-miR-4804-5p | 210587 | 212629 | Breast tumor |
| hsa-miR-4999-3p | 210588 | 212630 | |
| hsa-miR-4999-5p | 210589 | 212631 | |
| hsa-miR-499a-3p | 210590 | 212632 | Heart, cardiac stem cells |
| hsa-miR-499a-5p | 210591 | 212633 | Heart, cardiac stem cells |
| hsa-miR-499b-3p | 210592 | 212634 | Heart, cardiac stem cells |
| hsa-miR-499b-5p | 210593 | 212635 | Heart, cardiac stem cells |
| hsa-miR-5000-3p | 210594 | 212636 | Lymphoblastic leukemia |
| hsa-miR-5000-5p | 210595 | 212637 | Lymphoblastic leukemia |
| hsa-miR-5001-3p | 210596 | 212638 | |
| hsa-miR-5001-5p | 210597 | 212639 | |
| hsa-miR-5002-3p | 210598 | 212640 | |
| hsa-miR-5002-5p | 210599 | 212641 | |
| hsa-miR-5003-3p | 210600 | 212642 | |
| hsa-miR-5003-5p | 210601 | 212643 | |
| hsa-miR-5004-3p | 210602 | 212644 | |
| hsa-miR-5004-5p | 210603 | 212645 | |
| hsa-miR-5006-3p | 210604 | 212646 | Lymphoblastic leukemia |
| hsa-miR-5006-5p | 210605 | 212647 | Lymphoblastic leukemia |
| hsa-miR-5007-3p | 210606 | 212648 | |
| hsa-miR-5007-5p | 210607 | 212649 | |
| hsa-miR-5008-3p | 210608 | 212650 | |
| hsa-miR-5008-5p | 210609 | 212651 | |
| hsa-miR-5009-3p | 210610 | 212652 | |
| hsa-miR-5009-5p | 210611 | 212653 | |
| hsa-miR-5010-3p | 210612 | 212654 | |
| hsa-miR-5010-5p | 210613 | 212655 | |
| hsa-miR-5011-3p | 210614 | 212656 | |
| hsa-miR-5011-5p | 210615 | 212657 | |
| hsa-miR-5047 | 210616 | 212658 | |
| hsa-miR-5087 | 210617 | 212659 | |
| hsa-miR-5088 | 210618 | 212660 | |
| hsa-miR-5089-3p | 210619 | 212661 | |
| hsa-miR-5089-5p | 210620 | 212662 | |
| hsa-miR-5090 | 210621 | 212663 | |
| hsa-miR-5091 | 210622 | 212664 | |
| hsa-miR-5092 | 210623 | 212665 | |
| hsa-miR-5093 | 210624 | 212666 | |
| hsa-miR-5094 | 210625 | 212667 | |
| hsa-miR-5095 | 210626 | 212668 | |
| hsa-miR-5096 | 210627 | 212669 | |
| hsa-miR-5100 | 210628 | 212670 | Salivary gland |
| hsa-miR-5186 | 210629 | 212671 | Lymphoblastic leukemia |
| hsa-miR-5187-3p | 210630 | 212672 | Lymphoblastic leukemia, skin (psoriasis) |
| hsa-miR-5187-5p | 210631 | 212673 | Lymphoblastic leukemia, skin (psoriasis) |
| hsa-miR-5188 | 210632 | 212674 | Lymphoblastic leukemia |
| hsa-miR-5189 | 210633 | 212675 | Lymphoblastic leukemia |
| hsa-miR-5190 | 210634 | 212676 | Lymphoblastic leukemia |
| hsa-miR-5191 | 210635 | 212677 | Lymphoblastic leukemia |
| hsa-miR-5192 | 210636 | 212678 | Lymphoblastic leukemia |
| hsa-miR-5193 | 210637 | 212679 | Lymphoblastic leukemia |
| hsa-miR-5194 | 210638 | 212680 | Lymphoblastic leukemia |
| hsa-miR-5195-3p | 210639 | 212681 | Lymphoblastic leukemia |
| hsa-miR-5195-5p | 210640 | 212682 | Lymphoblastic leukemia |
| hsa-miR-5196-3p | 210641 | 212683 | Lymphoblastic leukemia |
| hsa-miR-5196-5p | 210642 | 212684 | Lymphoblastic leukemia |
| hsa-miR-5197-3p | 210643 | 212685 | Lymphoblastic leukemia |
| hsa-miR-5197-5p | 210644 | 212686 | Lymphoblastic leukemia |
| hsa-miR-5571-3p | 210645 | 212687 | Salivary gland |
| hsa-miR-5571-5p | 210646 | 212688 | Salivary gland |
| hsa-miR-5572 | 210647 | 212689 | Salivary gland |
| hsa-miR-5579-3p | 210648 | 212690 | |
| hsa-miR-5579-5p | 210649 | 212691 | |
| hsa-miR-5580-3p | 210650 | 212692 | |
| hsa-miR-5580-5p | 210651 | 212693 | |
| hsa-miR-5581-3p | 210652 | 212694 | |
| hsa-miR-5581-5p | 210653 | 212695 | |
| hsa-miR-5582-3p | 210654 | 212696 | |
| hsa-miR-5582-5p | 210655 | 212697 | |
| hsa-miR-5583-3p | 210656 | 212698 | |
| hsa-miR-5583-5p | 210657 | 212699 | |
| hsa-miR-5584-3p | 210658 | 212700 | |
| hsa-miR-5584-5p | 210659 | 212701 | |
| hsa-miR-5585-3p | 210660 | 212702 | |
| hsa-miR-5585-5p | 210661 | 212703 | |
| hsa-miR-5586-3p | 210662 | 212704 | |
| hsa-miR-5586-5p | 210663 | 212705 | |
| hsa-miR-5587-3p | 210664 | 212706 | |
| hsa-miR-5587-5p | 210665 | 212707 | |
| hsa-miR-5588-3p | 210666 | 212708 | |
| hsa-miR-5588-5p | 210667 | 212709 | |
| hsa-miR-5589-3p | 210668 | 212710 | |
| hsa-miR-5589-5p | 210669 | 212711 | |
| hsa-miR-5590-3p | 210670 | 212712 | |
| hsa-miR-5590-5p | 210671 | 212713 | |
| hsa-miR-5591-3p | 210672 | 212714 | |
| hsa-miR-5591-5p | 210673 | 212715 | |
| hsa-miR-5680 | 210674 | 212716 | |
| hsa-miR-5681a | 210675 | 212717 | |
| hsa-miR-5681b | 210676 | 212718 | |
| hsa-miR-5682 | 210677 | 212719 | |
| hsa-miR-5683 | 210678 | 212720 | |
| hsa-miR-5684 | 210679 | 212721 | |
| hsa-miR-5685 | 210680 | 212722 | |
| hsa-miR-5686 | 210681 | 212723 | |
| hsa-miR-5687 | 210682 | 212724 | |
| hsa-miR-5688 | 210683 | 212725 | |
| hsa-miR-5689 | 210684 | 212726 | |
| hsa-miR-5690 | 210685 | 212727 | |
| hsa-miR-5691 | 210686 | 212728 | |
| hsa-miR-5692a | 210687 | 212729 | |
| hsa-miR-5692b | 210688 | 212730 | |
| hsa-miR-5692c | 210689 | 212731 | |
| hsa-miR-5693 | 210690 | 212732 | |
| hsa-miR-5694 | 210691 | 212733 | |
| hsa-miR-5695 | 210692 | 212734 | |
| hsa-miR-5696 | 210693 | 212735 | |
| hsa-miR-5697 | 210694 | 212736 | |
| hsa-miR-5698 | 210695 | 212737 | |
| hsa-miR-5699 | 210696 | 212738 | |
| hsa-miR-5700 | 210697 | 212739 | |
| hsa-miR-5701 | 210698 | 212740 | |
| hsa-miR-5702 | 210699 | 212741 | |
| hsa-miR-5703 | 210700 | 212742 | |
| hsa-miR-5704 | 210701 | 212743 | |
| hsa-miR-5705 | 210702 | 212744 | |
| hsa-miR-5706 | 210703 | 212745 | |
| hsa-miR-5707 | 210704 | 212746 | |
| hsa-miR-5708 | 210705 | 212747 | |
| hsa-miR-5739 | 210706 | 212748 | Endothelial cells |
| hsa-miR-5787 | 210707 | 212749 | Fibroblast |
| hsa-miR-6068 | 210708 | 212750 | Endothelial cells |
| hsa-miR-6069 | 210709 | 212751 | Endothelial cells |
| hsa-miR-6070 | 210710 | 212752 | A colorectal microRNAome |
| hsa-miR-6071 | 210711 | 212753 | Endothelial cells |
| hsa-miR-6072 | 210712 | 212754 | Endothelial cells |
| hsa-miR-6073 | 210713 | 212755 | Endothelial cells |
| hsa-miR-6074 | 210714 | 212756 | Endothelial cells |
| hsa-miR-6075 | 210715 | 212757 | Endothelial cells |
| hsa-miR-6076 | 210716 | 212758 | Endothelial cells |
| hsa-miR-6077 | 210717 | 212759 | Endothelial cells |
| hsa-miR-6078 | 210718 | 212760 | Endothelial cells |
| hsa-miR-6079 | 210719 | 212761 | Endothelial cells |
| hsa-miR-6080 | 210720 | 212762 | Endothelial cells |
| hsa-miR-6081 | 210721 | 212763 | Endothelial cells |
| hsa-miR-6082 | 210722 | 212764 | Endothelial cells |
| hsa-miR-6083 | 210723 | 212765 | Endothelial cells |
| hsa-miR-6084 | 210724 | 212766 | Endothelial cells |
| hsa-miR-6085 | 210725 | 212767 | Endothelial cells |
| hsa-miR-6086 | 210726 | 212768 | Embryonic stem cells |
| hsa-miR-6087 | 210727 | 212769 | Embryonic stem cells |
| hsa-miR-6088 | 210728 | 212770 | Embryonic stem cells |
| hsa-miR-6089 | 210729 | 212771 | Embryonic stem cells |
| hsa-miR-6090 | 210730 | 212772 | Embryonic stem cells |
| hsa-miR-6124 | 210731 | 212773 | |
| hsa-miR-6125 | 210732 | 212774 | |
| hsa-miR-6126 | 210733 | 212775 | |
| hsa-miR-6127 | 210734 | 212776 | |
| hsa-miR-6128 | 210735 | 212777 | |
| hsa-miR-6129 | 210736 | 212778 | |
| hsa-miR-6130 | 210737 | 212779 | |
| hsa-miR-6131 | 210738 | 212780 | |
| hsa-miR-6132 | 210739 | 212781 | |
| hsa-miR-6133 | 210740 | 212782 | |
| hsa-miR-6134 | 210741 | 212783 | |
| hsa-miR-6165 | 210742 | 212784 | |
| hsa-miR-6499-3p | 210743 | 212785 | Abnormal skin (psoriasis) |
| hsa-miR-6499-5p | 210744 | 212786 | Abnormal skin (psoriasis) |
| hsa-miR-6500-3p | 210745 | 212787 | Abnormal skin (psoriasis) |
| hsa-miR-6500-5p | 210746 | 212788 | Abnormal skin (psoriasis) |
| hsa-miR-6501-3p | 210747 | 212789 | Abnormal skin (psoriasis) |
| hsa-miR-6501-5p | 210748 | 212790 | Abnormal skin (psoriasis) |
| hsa-miR-6502-3p | 210749 | 212791 | Abnormal skin (psoriasis) |
| hsa-miR-6502-5p | 210750 | 212792 | Abnormal skin (psoriasis) |
| hsa-miR-6503-3p | 210751 | 212793 | Abnormal skin (psoriasis) |
| hsa-miR-6503-5p | 210752 | 212794 | Abnormal skin (psoriasis) |
| hsa-miR-6504-3p | 210753 | 212795 | Abnormal skin (psoriasis) |
| hsa-miR-6504-5p | 210754 | 212796 | Abnormal skin (psoriasis) |
| hsa-miR-6505-3p | 210755 | 212797 | Abnormal skin (psoriasis) |
| hsa-miR-6505-5p | 210756 | 212798 | Abnormal skin (psoriasis) |
| hsa-miR-6506-3p | 210757 | 212799 | Abnormal skin (psoriasis) |
| hsa-miR-6506-5p | 210758 | 212800 | Abnormal skin (psoriasis) |
| hsa-miR-6507-3p | 210759 | 212801 | Abnormal skin (psoriasis) |
| hsa-miR-6507-5p | 210760 | 212802 | Abnormal skin (psoriasis) |
| hsa-miR-6508-3p | 210761 | 212803 | Abnormal skin (psoriasis) |
| hsa-miR-6508-5p | 210762 | 212804 | Abnormal skin (psoriasis) |
| hsa-miR-6509-3p | 210763 | 212805 | Abnormal skin (psoriasis) |
| hsa-miR-6509-5p | 210764 | 212806 | Abnormal skin (psoriasis) |
| hsa-miR-6510-3p | 210765 | 212807 | Abnormal skin (psoriasis) |
| hsa-miR-6510-5p | 210766 | 212808 | Abnormal skin (psoriasis) |
| hsa-miR-6511a-3p | 210767 | 212809 | Abnormal skin (psoriasis) and epididymis |
| hsa-miR-6511a-5p | 210768 | 212810 | Abnormal skin (psoriasis) and epididymis |
| hsa-miR-6511b-3p | 210769 | 212811 | Epididymis |
| hsa-miR-6511b-5p | 210770 | 212812 | Epididymis |
| hsa-miR-6512-3p | 210771 | 212813 | Abnormal skin (psoriasis) |
| hsa-miR-6512-5p | 210772 | 212814 | Abnormal skin (psoriasis) |
| hsa-miR-6513-3p | 210773 | 212815 | Abnormal skin (psoriasis) |
| hsa-miR-6513-5p | 210774 | 212816 | Abnormal skin (psoriasis) |
| hsa-miR-6514-3p | 210775 | 212817 | Abnormal skin (psoriasis) |
| hsa-miR-6514-5p | 210776 | 212818 | Abnormal skin (psoriasis) |
| hsa-miR-6515-3p | 210777 | 212819 | Abnormal skin (psoriasis) and epididymis |
| hsa-miR-6515-5p | 210778 | 212820 | Abnormal skin (psoriasis) and epididymis |
| hsa-miR-6715a-3p | 210779 | 212821 | Epididymis |
| hsa-miR-6715b-3p | 210780 | 212822 | Epididymis |
| hsa-miR-6715b-5p | 210781 | 212823 | Epididymis |
| hsa-miR-6716-3p | 210782 | 212824 | Epididymis |
| hsa-miR-6716-5p | 210783 | 212825 | Epididymis |
| hsa-miR-6717-5p | 210784 | 212826 | Epididymis |
| hsa-miR-6718-5p | 210785 | 212827 | Epididymis |
| hsa-miR-6719-3p | 210786 | 212828 | Epididymis |
| hsa-miR-6720-3p | 210787 | 212829 | Epididymis |
| hsa-miR-6721-5p | 210788 | 212830 | Epididymis |
| hsa-miR-6722-3p | 210789 | 212831 | Epididymis |
| hsa-miR-6722-5p | 210790 | 212832 | Epididymis |
| hsa-miR-6723-5p | 210791 | 212833 | Epididymis |
| hsa-miR-6724-5p | 210792 | 212834 | Epididymis |
| hsa-let-7a-2-3p | 210793 | 212835 | Embrvonic stem cells, lung, myeloid cells |
| hsa-let-7a-3p | 210794 | 212836 | Embryonic stem cells, lung |
| hsa-let-7a-5p | 210795 | 212837 | Embrvonic stem cells, lung |
| hsa-let-7b-3p | 210796 | 212838 | Epithelial cells, endothelial cells (vascular) |
| hsa-let-7b-5p | 210797 | 212839 | Epithelial cells, endothelial cells (vascular) |
| hsa-let-7c | 210798 | 212840 | Dendritic cells |
| hsa-let-7d-3p | 210799 | 212841 | Embryonic stem cells |
| hsa-let-7d-5p | 210800 | 212842 | Embryonic stem cells |
| hsa-let-7e-3p | 210801 | 212843 | Immune cells |
| hsa-let-7e-5p | 210802 | 212844 | Immune cells |
| hsa-let-7f-1-3p | 210803 | 212845 | Immune cells (T cells) |
| hsa-let-7f-2-3p | 210804 | 212846 | Immune cells (T cells) |
| hsa-let-7f-5p | 210805 | 212847 | Immune cells (T cells) |
| hsa-let-7q-3p | 210806 | 212848 | Hematopoietic cells, adipose, smooth muscle cells |
| hsa-let-7q-5p | 210807 | 212849 | Hematopoietic cells, adipose, smooth muscle cells |
| hsa-let-7i-3p | 210808 | 212850 | Immune cells |
| hsa-let-7i-5p | 210809 | 212851 | Immune cells |

### Construct Optimization to reduce basal expression

Biocircuit constructs are to be further optimized to reduce or eliminate the basal expression in the absence of ligands. In some embodiments, an interfering RNA may be used to reduce the basal expression. Other RNA regulatory elements may also be introduced to the construct, for example, by incorporating AU-rich mRNA destabilizing elements (ARE) into the 3' untranslated region (3'UTR) of the construct (Maitra et al., RNA, 2008, 14(5): 950-959).

A construct may be test with different promoters or mutated promoters. The promoter that gives the least "leaky" expression may be used. In some embodiments, one or more suppressor binding sites may be inserted to the constructs. The suppressor proteins bind to the construct and suppress the expression of the construct in the absence of the stimulus.

Additionally, constructs encoding proteins which can attenuate the transgene activity may also be co-expressed with the biocircuits.

In some embodiments, effector modules of the present invention may include one or more degrons to tune expression. As used herein, a "degron" refers to a minimal sequence within a protein that is sufficient for the recognition and the degradation by the proteolytic system. An important property of degrons is that they are transferrable, that is, appending a degron to a sequence confers degradation upon the sequence. In some embodiments, the degron may be appended to the destabilizing domains, the payload or both. Incorporation of the degron within the effector module of the invention, confers additional protein instability_to the effector module and may be used to minimize basal expression. In some embodiments, the degron may be an N-degron, a phospho degron, a heat inducible degron, a photosensitive degron, an oxygen dependent degron. As a non-limiting example, the degron may be an Ornithine decarboxylase degron as described by Takeuchi et al. (Takeuchi J et al. (2008). Biochem J. 2008 Mar 1;410(2):401-7). Other examples of degrons useful in the present invention include degrons described in International patent publication Nos. WO2017004022, WO2016210343, and WO2011062962.

### III. PHARMACEUTICAL COMPOSITIONS

The present teachings further comprise pharmaceutical compositions comprising one or more effector modules of the present invention, and optionally at least one pharmaceutically acceptable excipient or inert ingredient.

As used herein the term "pharmaceutical composition" refers to a preparation of one or more of the effector modules described herein, or pharmaceutically acceptable salts thereof, optionally with other chemical components such as physiologically suitable carriers and excipients.

The term "excipient" or "inactive ingredient" refers to an inert or inactive substance added to a pharmaceutical composition to further facilitate administration of a compound. Non-limiting examples of such inert ingredients are disclosed herein under Formulations.

In some embodiments, compositions are administered to humans, human patients or subjects. For the purposes of the present disclosure, the phrase "active ingredient" generally refers to the effector module to be delivered as described herein.

Although the descriptions of pharmaceutical compositions provided herein are principally directed to pharmaceutical compositions which are suitable for administration to humans, it will be understood by the skilled artisan that such compositions are generally suitable for administration to any other animal, e.g., to non-human animals, e.g. non-human mammals. Subjects to which administration of the pharmaceutical compositions is contemplated include, but are not limited to, non-human mammals, including agricultural animals such as cattle, horses, chickens and pigs, domestic animals such as cats, dogs, or research animals such as mice, rats, rabbits, dogs and non-human primates.

A pharmaceutical composition in accordance with the invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient or inert ingredient, and/or any additional ingredients in a pharmaceutical composition in accordance with the invention will vary, depending upon the identity, size, and/or condition of the subject treated and further depending upon the route by which the composition is to be administered. By way of example, the composition may comprise between 0.1% and 100%, e.g., between 0.5 and 50%, between 1-30%, between 5-80%, at least 80% (w/w) active ingredient.

Efficacy of treatment or amelioration of disease can be assessed, for example by measuring disease progression, disease remission, symptom severity, reduction in pain, quality of life, dose of a medication required to sustain a treatment effect, level of a disease marker or any other measurable parameter appropriate for a given disease being treated or targeted for prevention. It is well within the ability of one skilled in the art to monitor efficacy of treatment or prevention by measuring any one of such parameters, or any combination of parameters. In connection with the administration of compositions of the present invention, "effective against" for example a cancer, indicates that administration in a clinically appropriate manner results in a beneficial effect for at least a statistically significant fraction of patients, such as an improvement of symptoms, a cure, a reduction in disease load, reduction in tumor mass or cell numbers, extension of life, improvement in quality of life, or other effect generally recognized as positive by medical doctors familiar with treating the particular type of cancer.

A treatment or preventive effect is evident when there is a statistically significant improvement in one or more parameters of disease status, or by a failure to worsen or to develop symptoms where they would otherwise be anticipated. As an example, a favorable change of at least 10% in a measurable parameter of disease, and preferably at least 20%, 30%, 40%, 50% or more can be indicative of effective treatment. Efficacy for a given composition or formulation of the present invention can also be judged using an experimental animal model for the given disease as known in the art. When using an experimental animal model, efficacy of treatment is evidenced when a statistically significant change is observed.

### Therapeutic Uses

### Cancer

Various cancers may be treated with pharmaceutical compositions and effector modules of the present invention. As used herein, the term "cancer" refers to any of various malignant neoplasms characterized by the proliferation of anaplastic cells that tend to invade surrounding tissue and metastasize to new body sites and also refers to the pathological condition characterized by such malignant neoplastic growths. Cancers may be tumors or hematological malignancies, and include but are not limited to, all types of lymphomas/leukemias, carcinomas and sarcomas, such as those cancers or tumors found in the anus, bladder, bile duct, bone, brain, breast, cervix, colon/rectum, endometrium, esophagus, eye, gallbladder, head and neck, liver, kidney, larynx, lung, mediastinum (chest), mouth, ovaries, pancreas, penis, prostate, skin, small intestine, stomach, spinal marrow, tailbone, testicles, thyroid and uterus.

Types of carcinomas which may be treated with the compositions of the present invention include, but are not limited to, papilloma/carcinoma, choriocarcinoma, endodermal sinus tumor, teratoma, adenoma/adenocarcinoma, melanoma, fibroma, lipoma, leiomyoma, rhabdomyoma, mesothelioma, angioma, osteoma, chondroma, glioma, lymphoma/leukemia, squamous cell carcinoma, small cell carcinoma, large cell undifferentiated carcinomas, basal cell carcinoma and sinonasal undifferentiated carcinoma.

Types of carcinomas which may be treated with the compositions of the present invention include, but are not limited to, soft tissue sarcoma such as alveolar soft part sarcoma, angiosarcoma, dermatofibrosarcoma, desmoid tumor, desmoplastic small round cell tumor, extraskeletal chondrosarcoma, extraskeletal osteosarcoma, fibrosarcoma, hemangiopericytoma, hemangiosarcoma, Kaposi's sarcoma, leiomyosarcoma, liposarcoma, lymphangiosarcoma, lymphosarcoma, malignant fibrous histiocytoma, neurofibrosarcoma, rhabdomyosarcoma, synovial sarcoma, and Askin's tumor, Ewing's sarcoma (primitive neuroectodermal tumor), malignant hemangioendothelioma, malignant schwannoma, osteosarcoma, and chondrosarcoma.

As a non-limiting example, the carcinoma which may be treated may be Acute granulocytic leukemia, Acute lymphocytic leukemia, Acute myelogenous leukemia, Adenocarcinoma, Adenosarcoma, Adrenal cancer, Adrenocortical carcinoma, Anal cancer, Anaplastic astrocytoma, Angiosarcoma, Appendix cancer, Astrocytoma, Basal cell carcinoma, B-Cell lymphoma), Bile duct cancer, Bladder cancer, Bone cancer, Bowel cancer, Brain cancer, Brain stem glioma, Brain tumor, Breast cancer, Carcinoid tumors, Cervical cancer, Cholangiocarcinoma, Chondrosarcoma, Chronic lymphocytic leukemia, Chronic myelogenous leukemia, Colon cancer, Colorectal cancer, Craniopharyngioma, Cutaneous lymphoma, Cutaneous melanoma, Diffuse astrocytoma, Ductal carcinoma in situ, Endometrial cancer, Ependymoma, Epithelioid sarcoma, Esophageal cancer, Ewing sarcoma, Extrahepatic bile duct cancer, Eye cancer, Fallopian tube cancer, Fibrosarcoma, Gallbladder cancer, Gastric cancer, Gastrointestinal cancer, Gastrointestinal carcinoid cancer, Gastrointestinal stromal tumors, General, Germ cell tumor, Glioblastoma multiforme, Glioma, Hairy cell leukemia, Head and neck cancer, Hemangioendothelioma, Hodgkin lymphoma, Hodgkin's disease, Hodgkin's lymphoma, Hypopharyngeal cancer, Infiltrating ductal carcinoma, Infiltrating lobular carcinoma, Inflammatory breast cancer, Intestinal Cancer, Intrahepatic bile duct cancer, Invasive / infiltrating breast cancer, Islet cell cancer, Jaw cancer, Kaposi sarcoma, Kidney cancer, Laryngeal cancer, Leiomyosarcoma, Leptomeningeal metastases, Leukemia, Lip cancer, Liposarcoma, Liver cancer, Lobular carcinoma in situ, Low-grade astrocytoma, Lung cancer, Lymph node cancer, Lymphoma, Male breast cancer, Medullary carcinoma, Medulloblastoma, Melanoma, Meningioma, Merkel cell carcinoma, Mesenchymal chondrosarcoma, Mesenchymous, Mesothelioma, Metastatic breast cancer, Metastatic melanoma, Metastatic squamous neck cancer, Mixed gliomas, Mouth cancer, Mucinous carcinoma, Mucosal melanoma, Multiple myeloma, Nasal cavity cancer, Nasopharyngeal cancer, Neck cancer, Neuroblastoma, Neuroendocrine tumors, Non-Hodgkin lymphoma, Non-Hodgkin's lymphoma, Non-small cell lung cancer, Oat cell cancer, Ocular cancer, Ocular melanoma, Oligodendroglioma, Oral cancer, Oral cavity cancer, Oropharyngeal cancer, Osteogenic sarcoma, Osteosarcoma, Ovarian cancer, Ovarian epithelial cancer, Ovarian germ cell tumor, Ovarian primary peritoneal carcinoma, Ovarian sex cord stromal tumor, Paget's disease, Pancreatic cancer, Papillary carcinoma, Paranasal sinus cancer, Parathyroid cancer, Pelvic cancer, Penile cancer, Peripheral nerve cancer, Peritoneal cancer, Pharyngeal cancer, Pheochromocytoma, Pilocytic astrocytoma, Pineal region tumor, Pineoblastoma, Pituitary gland cancer, Primary central nervous system lymphoma, Prostate cancer, Rectal cancer, Renal cell cancer, Renal pelvis cancer, Rhabdomyosarcoma, Salivary gland cancer, Sarcoma, Sarcoma, bone, Sarcoma, soft tissue, Sarcoma, uterine, Sinus cancer, Skin cancer, Small cell lung cancer, Small intestine cancer, Soft tissue sarcoma, Spinal cancer, Spinal column cancer, Spinal cord cancer, Spinal tumor, Squamous cell carcinoma, Stomach cancer, Synovial sarcoma, T-cell lymphoma), Testicular cancer, Throat cancer, Thymoma / thymic carcinoma, Thyroid cancer, Tongue cancer, Tonsil cancer, Transitional cell cancer, Transitional cell cancer, Transitional cell cancer, Triple-negative breast cancer, Tubal cancer, Tubular carcinoma, Ureteral cancer, Ureteral cancer, Urethral cancer, Uterine adenocarcinoma, Uterine cancer, Uterine sarcoma, Vaginal cancer, and Vulvar cancer.

### Combination treatments

The invention further relates to the use of pharmaceutical compositions, and effector modules of the present invention for treating one or more forms of cancer, in combination with other pharmaceuticals and/or other therapeutic methods, e.g., with known pharmaceuticals and/or known therapeutic methods, such as, for example, those which are currently employed for treating these disorders. For example, the pharmaceutical compositions or effector modules of the present invention can also be administered in conjunction with one or more additional anti-cancer treatments, such as biological, chemotherapy and radiotherapy. Accordingly, a treatment can include, for example, imatinib (Gleevac), all-trans-retinoic acid, a monoclonal antibody treatment (gemtuzumab, ozogamicin), chemotherapy (for example, chlorambucil, prednisone, prednisolone, vincristine, cytarabine, clofarabine, farnesyl transferase inhibitors, decitabine, inhibitors of MDR1), rituximab, interferon-a, anthracycline drugs (such as daunorubicin or idarubicin), L-asparaginase, doxorubicin, cyclophosphamide, doxorubicin, bleomycin, fludarabine, etoposide, pentostatin, or cladribine), bone marrow transplant, stem cell transplant, radiation therapy, anti-metabolite drugs (methotrexate and 6-mercaptopurine), or any therapeutic antibody.

### Combinations with radiation

Radiation therapy (also called radiotherapy, X-ray therapy, or irradiation) is the use of ionizing radiation to kill cancer cells and shrink tumors. Radiation therapy can be administered externally via external beam radiotherapy (EBRT) or internally via brachytherapy. The effects of radiation therapy are localized and confined to the region being treated. Radiation therapy may be used to treat almost every type of solid tumor, including cancers of the brain, breast, cervix, larynx, lung, pancreas, prostate, skin, stomach, uterus, or soft tissue sarcomas. Radiation is also used to treat leukemia and lymphoma.

### Combination with chemotherapy

Chemotherapy is the treatment of cancer with drugs that can destroy cancer cells. In current usage, the term "chemotherapy" usually refers to cytotoxic drugs which affect rapidly dividing cells in general, in contrast with targeted therapy. Chemotherapy drugs interfere with cell division in various possible ways, e.g. with the duplication of DNA or the separation of newly formed chromosomes. Most forms of chemotherapy target all rapidly dividing cells and are not specific to cancer cells, although some degree of specificity may come from the inability of many cancer cells to repair DNA damage, while normal cells generally can.

Most chemotherapy regimens are given in combination. Exemplary chemotherapeutic agents include , but are not limited to, 5-FU Enhancer, 9-AC, AG2037, AG3340, Aggrecanase Inhibitor, Aminoglutethimide, Amsacrine (m-AMSA), Asparaginase, Azacitidine, Batimastat (BB94), BAY 12-9566, BCH-4556, Bis-Naphtalimide, Busulfan, Capecitabine, Carboplatin, Carmustaine+Polifepr Osan, cdk4/cdk2 inhibitors, Chlorombucil, CI-994, Cisplatin, Cladribine, CS-682, Cytarabine HCl, D2163, Dactinomycin, Daunorubicin HCl, DepoCyt, Dexifosamide, Docetaxel, Dolastain, Doxifluridine, Doxorubicin, DX8951f, E 7070, EGFR, Epirubicin, Erythropoietin, Estramustine phosphate sodium, Etoposide (VP16-213), Farnesyl Transferase Inhibitor, FK 317, Flavopiridol, Floxuridine, Fludarabine, Fluorouracil (5-FU), Flutamide, Fragyline, Gemcitabine, Hexamethylmelamine (HMM), Hydroxyurea (hydroxycarbamide), Ifosfamide, Interferon Alfa-2a, Interferon Alfa-2b, Interleukin-2, Irinotecan, ISI 641, Krestin, Lemonal DP 2202, Leuprolide acetate (LHRH-releasing factor analogue), Levamisole, LiGLA (lithium-gamma linolenate), Lodine Seeds, Lometexol, Lomustine (CCNU), Marimistat, Mechlorethamine HCl (nitrogen mustard), Megestrol acetate, Meglamine GLA, Mercaptopurine, Mesna, Mitoguazone (methyl-GAG; methyl glyoxal bis-guanylhydrazone; MGBG), Mitotane (o.p'-DDD), Mitoxantrone, Mitoxantrone HCl, MMI 270, MMP, MTA/LY 231514, Octreotide, ODN 698, OK-432, Oral Platinum, Oral Taxoid, Paclitaxel (TAXOL.RTM.), PARP Inhibitors, PD 183805, Pentostatin (2' deoxycoformycin), PKC 412, Plicamycin, Procarbazine HCl, PSC 833, Ralitrexed, RAS Farnesyl Transferase Inhibitor, RAS Oncogene Inhibitor, Semustine (methyl-CCNU), Streptozocin, Suramin, Tamoxifen citrate, Taxane Analog, Temozolomide, Teniposide (VM-26), Thioguanine, Thiotepa, Topotecan, Tyrosine Kinase, UFT (Tegafur/Uracil), Valrubicin, Vinblastine sulfate, Vindesine sulfate, VX-710, VX-853, YM 116, ZD 0101, ZD 0473/Anormed, ZD 1839, ZD 9331.

### Immuno-oncology and Cell therapies

Recent progress in the field of cancer immunology has allowed the development of several approaches to help the immune system keep the cancer at bay. Such immunotherapy approaches include the targeting of cancer antigens through monoclonal antibodies or through adoptive transfer of ex vivo engineered T cells (e.g., which contain chimeric antigen receptors or engineered T cell receptors).

In some embodiments, pharmaceutical compositions, or effector modules of the present invention may be used in the modulation or alteration or exploitation of the immune system to target one or more cancers. This approach may also be considered with other such biological approaches, e.g., immune response modifying therapies such as the administration of interferons, interleukins, colony-stimulating factors, other monoclonal antibodies, vaccines, gene therapy, and nonspecific immunomodulating agents are also envisioned as anti-cancer therapies to be combined with the pharmaceutical compositions or effector modules of the present invention.

Cancer immunotherapy refers to a diverse set of therapeutic strategies designed to induce the patient's own immune system to fight the cancer.

### Cell Therapies

There are several types of cellular immunotherapies, including tumor infiltrating lymphocyte (TIL) therapy, genetically engineered T cells bearing chimeric antigen receptors (CARs), and recombinant TCR technology.

According to the present invention, the effector modules of the present invetion may be used in the development and implementation of cell therapies such as adoptive cell therapy. Certain effector modules useful in cell therapy are given in FIGs. 8-13.

The payloads of the present invention are CD19 chimeric antigen receptors (CARs), which when transduced into immune cells (e.g., T cells and NK cells), can re-direct the immune cells against the target (e.g., a tumor cell) which expresses CD19.

As used herein, the term "chimeric antigen receptor (CAR)" refers to a synthetic receptor that mimics the TCR on the surface of T cells. In general, a CAR is composed of an extracellular targeting domain, a transmembrane domain/region and an intracellular signaling/activation domain. In a standard CAR receptor, the components: the extracellular targeting domain, transmembrane domain and intracellular signaling/activation domain, are linearly constructed as a single fusion protein. The extracellular region comprises a targeting domain/moiety (e.g., a scFv) that recognizes a specific tumor antigen or other tumor cell-surface molecules. The intracellular region may contain a signaling domain of TCR complex (e.g., the signal region of CD3ζ), and/or one or more costimulatory signaling domains, such as those from CD28, 4-1BB (CD137) and OX-40 (CD134). For example, a "first-generation CAR" only has the CD3ζ signaling domain, whereas, a second-generation CARs has a CD3ζsignal domain plus one costimulatory signaling domain, and a third-generation CARs having CD3ζ signal domain plus two or more costimulatory signaling domains. A CAR, when expressed by a T cell, endows the T cell with antigen specificity determined by the extracellular targeting moiety of the CAR. It is also desirable to add one or more elements such as homing and suicide genes to develop a more competent and safer architecture of CAR, which has given rise to the so called the fourth-generation CAR.

In some embodiments, the extracellular targeting domain is joined through the hinge (also called space domain or spacer) and transmembrane regions to an intracellular signaling domain. The hinge may need to be varied to optimize the potency of CAR expressing cells towards the cancer cells due to the size of the target protein where the targeting moiety binds, and the size and affinity of the targeting domain itself. Upon recognition and binding of the targeting moiety to the target cell, the intracellular signaling domain leads to an activation signal for the CAR T cell, which is further amplified by the "second signal" from one or more intracellular costimulatory domains. The CAR T cell, once activated, can destroy the target cell.

In some embodiments, the CAR of the present invention may be split into two parts, each part is linked a dimerizing domain, such that an input that triggers the dimerization promotes assembly of the intact functional receptor. Wu and Lim recently reported a split CAR in which the extracellular CD19 binding domain and the intracellular signaling element are separated and linked to the FKBP domain and the FRB* (T2089L mutant of FKBP-rapamycin binding) domain that heterodimerize in the presence of the rapamycin analog AP21967. The split receptor is assembled in the presence of AP21967 and together with the specific antigen binding, activates T cells (Wu et al., Science, 2015, 625(6258): aab4077).

In some embodiments, the CAR of the present invention may be designed as an inducible CAR. Sakemura et al recently reported the incorporation of a Tet-On inducible system to the CD19 CAR construct. The CD19 CAR is activated only in the presence of doxycycline (Dox). Sakemura reported that Tet-CD19CAR T cells in the presence of Dox were cytotoxic against CD19⁺ cell lines and had equivalent cytokine production and proliferation upon CD19 stimulation, compared with conventional CD19CAR T cells (Sakemura et al., Cancer Immuno. Res., 2016, Jun 21, Epub ahead of print). In one example, this Tet-CAR may be the payload of the effector module under the control of DDs of the invention. The dual systems provide more flexibility to turn-on and off the CAR expression in transduced T cells.

According to the present invention, the payload of the present invention may be a first-generation CAR, or a second-generation CAR, or a third-generation CAR, or a fourth-generation CAR. Representative effector module embodiments comprising CAR constructs are illustrated in FIG. 13-18.

In accordance with the invention, the extracellular target moiety of a CAR may be any agent that recognizes and binds to CD19 with high specificity and affinity. The target moiety may be an antibody and variants thereof that specifically bind to CD19, or a peptide aptamer selected from a random sequence pool based on its ability to bind to CD19, or a variant or fragment thereof that can bind to CD19, or a CD19 antigen recognition domain from native T- cell receptor (TCR), or a natural ligand of CD19.

In some embodiments, the targeting domain of a CAR may be a Ig NAR, a Fab fragment, a Fab' fragment, a F(ab)'2 fragment, a F(ab)'3 fragment, Fv, a single chain variable fragment (scFv), a bis-scFv, a (scFv)2, a minibody, a diabody, a triabody, a tetrabody, a disulfide stabilized Fv protein (dsFv), a unitbody, a nanobody, or an antigen binding region derived from an antibody that specifically recognizes CD19. In one embodiment, the targeting moiety is a scFv antibody. The scFv domain, when it is expressed on the surface of a CAR T cell and subsequently binds to CD19 on a cancer cell, is able to maintain the CAR T cell in proximity to the cancer cell and to trigger the activation of the T cell. A scFv can be generated using routine recombinant DNA technology techniques and is discussed in the present invention.

In some embodiments, the targeting moiety of a CAR construct may be a natural ligand of CD19, or a variant and/or fragment thereof capable of binding CD19.

As non-limiting examples, the targeting moiety of the present invention may be a CD19 antigen binding domain (U.S. Pat. NO.: 9, 328, 156); or the anti-CD19 scFv antibody of SEQ ID NO.: 20 in US Pat. NO.: 9,102,761.

The intracellular domain of a CAR fusion polypeptide, after binding to its target molecule, transmits a signal to the immune effector cell, activating at least one of the normal effector functions of immune effector cells, including cytolytic activity (e.g., cytokine secretion) or helper activity. Therefore, the intracellular domain comprises an "intracellular signaling domain" of a T cell receptor (TCR). In some embodiments, the intracellular signaling domain of the present invention may contain signaling motifs which are known as immunoreceptor tyrosine-based activation motifs (ITAMs). In some embodiments, the intracellular region of the present invention further comprises one or more costimulatory signaling domains which provide additional signals to the immune effector cells. These costimulatory signaling domains, in combination with the signaling domain can further improve expansion, activation, memory, persistence, and tumor-eradicating efficiency of CAR engineered immune cells (e.g., CAR T cells). In some cases, the costimulatory signaling region contains 1, 2, 3, or 4 cytoplasmic domains of one or more intracellular signaling and /or costimulatory molecules. In some embodiments, the intracellular region of the present invention may comprise a functional signaling domain from a protein selected from the group consisting of an MHC class I molecule, a TNF receptor protein, an immunoglobulin-like protein, a cytokine receptor, an integrin, a signaling lymphocytic activation protein (SLAM) such as CD48, CD229, 2B4, CD84, NTB-A, CRACC, BLAME,CD2F-10, SLAMF6, SLAMF7, an activating NK cell receptor, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), SLAMF7, NKp80 (KLRF1), NKp44, NKp30, NKp46, CD19, CD4, CD8alpha, CD8beta, IL2R beta, IL2R gamma, IL7R alpha, IL-15Ra, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, NKD2C SLP76, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, CD270 (HVEM), GADS, SLP-76, PAG/Cbp, CD19a, a ligand that specifically binds with CD83, DAP 10, TRIM, ZAP70, Killer immunoglobulin receptors (KIRs) such as KIR2DL1, KIR2DL2/L3, KIR2DL4, KIR2DL5A, KIR2DL5B, KIR2DS1, KIR2DS2, KIR2DS3, KIR2DS4, KIR2DS5, KlR3DL1/S1, KIR3DL2, KIR3DL3, and KIR2DP1; lectin related NK cell receptors such as Ly49, Ly49A, and Ly49C.

In some embodiments, the CAR of the present invention may comprise a transmembrane domain. As used herein, the term "Transmembrane domain (TM)" refers broadly to an amino acid sequence of about 15 residues in length which spans the plasma membrane. More preferably, a transmembrane domain includes at least 20, 21, 22, 23, 24, 25, 26, 27, 28, 29, 30, 31, 32, 33, 34, 35, 36, 37, 38, 39, 40, 41, 42, 43, 44, or 45 amino acid residues and spans the plasma membrane. In some embodiments, the transmembrane domain of the present invention may be derived either from a natural or from a synthetic source. The transmembrane domain of a CAR may be derived from any naturally membrane-bound or transmembrane protein. In some embodiments, the transmembrane domain of the present invention may be selected from the group consisting of a CD8a transmembrane domain, a CD4 transmembrane domain, a CD 28 transmembrane domain, a CTLA-4 transmembrane domain, a PD-1 transmembrane domain, and a human IgG4 Fc region.

In some embodiments, the CAR of the present invention may comprise an optional hinge region (also called spacer). A hinge sequence is a short sequence of amino acids that facilitates flexibility of the extracellular targeting domain that moves the target binding domain away from the effector cell surface to enable proper cell/cell contact, target binding and effector cell activation (Patel et al., Gene Therapy, 1999; 6: 412-419). The hinge sequence may be positioned between the targeting moiety and the transmembrane domain. The hinge sequence can be any suitable sequence derived or obtained from any suitable molecule. The hinge sequence may be derived from all or part of an immunoglobulin (e.g., IgGI, IgG2, IgG3, IgG4) hinge region, i.e., the sequence that falls between the CHI and CH2 domains of an immunoglobulin, e.g., an IgG4 Fc hinge, the extracellular regions of type 1 membrane proteins such as CD8α CD4, CD28 and CD7, which may be a wild type sequence or a derivative.

In some embodiments, the CAR of the present invention may comprise one or more linkers selected from Table 7 or 8.

The amino acid sequences of CD19 CAR and its components are presented in Table 10. In Table 10, the transmembrane domain is italicized and underlined to differentiate it from the adjacent sequence components. The mutations in the sequences are in bold.

**Table 10. CD19 CAR constructs**

| **Description** | **Amino Acid Sequence** | **Amino Acid SEQ ID NO** | **Nucleic Acid SEQ ID NO** |
|---|---|---|---|
| CD19 scFv | DIQMTQTTSSLSASLGDRVTISCRASQDISKYLNWYQQKPD GTVKLLIYHTSRLHSGVPSRFSGSGSGTDYSLTISNLEQEDI ATYFCQQGNTLPYTFGGGTKLEITGGGGSGGGGSGGGGS EVKLQESGPGLVAPSQSLSVTCTVSGVSLPDYGVSWIRQP PRKGLEWLGVIWGSETTYYNSALKSRLTIIKDNSKSQVFLKM NSLQTDDTAIYYCAKHYYYGGSYAMDYWGQGTSVTVSS | 213405 | 23418-213422 |
| CD8α hinge--TM | TTTPAPRPPTPAPTIASQPLSLRPEACRPAAGGAVHTRGLD FACDIYIWAPLAGTCGVLLLSLVITLYC | 213406 | 213423-213428 |
| CD3 zeta signaling domain | RVKFSRSADAPAYKQGQNQLYNELNLGRREEYDVLDKRRG RDPEMGGKPRRKNPQEGLYNELQKDKMAEAYSEIGMKGE RRRGKGHDGLYQGLSTATKDTYDALHMQALPPR | 213407 | 213429-213433 |
| 4-1BB intracellular signaling domain | KRGRKKLLYIFKQPFMRPVQTTQEEDGCSCRFPEEEEGGC EL | 213408 | 213434-213438 |
| CD8a leader | MALPVTALLLPLALLLHAARP | 213409 | 213439-213443 |
| FKBP (F36V, L106P) | GVQVETISPGDGRTFPKRGQTCVVHYTGMLEDGKKVDSSR DRNKPFKFMLGKQEVIRGWEEGVAQMSVGQRAKLTISPDY AYGATGHPGIIPPHATLVFDVELLKPE | 213274 | 213444-213445 |
| FKBP (E31G, F36V, R71G, K105E) | GVQVETISPGDGRTFPKRGQTCVVHYTGMLGDGKKVDSSR DRNKPFKFMLGKQEVIRGWEEGVAQMSVGQGAKLTISPDY | 213297 | 213446 |
| | AYGATGHPGIIPPHATLVFDVELLELE | | |
| ecDHFR (R12Y, Y100I) | ISLIAALAVDYVIGMENAMPWNLPADLAWFKRNTLNKPVIMG RHTWESIGRPLPGRKNIILSSQPGTDDRVTWVKSVDEAIAA CGDVPEIMVIGGGRVIEQFLPKAQKLYLTHIDAEVEGDTHFP DYEPDDWESVFSEFHDADAQNSHSYCFEILERR | 213275 | 213447-213448 |
| ecDHFR (R12H, E129K) | ISLIAALAVDHVIGMENAMPWNLPADLAWFKRNTLNKPVIMG RHTWESIGRPLPGRKNIILSSQPGTDDRVTWVKSVDEAIAA CGDVPEIMVIGGGRVYEQFLPKAQKLYLTHIDAEVEGDTHF PDYKPDDWESVFSEFHDADAQNSHSYCFEILERR | 213410 | 213449 |
| Linker | GGSGG | 213290 | 213395-213396 |
| OT-CD19 CAR-001 (CD8a leader; CD19 scFV (FM63); CD8a hinge+TM; 41BB; CD3zeta) | MALPVTALLLPLALLLHAARPDIQMTQTTSSLSASLGDRVTIS CRASQDISKYLNWYQQKPDGTVKLLIYHTSRLHSGVPSRFS GSGSGTDYSLTISNLEQEDIATYFCQQGNTLPYTFGGGTKL EITGGGGSGGGGSGGGGSEVKLQESGPGLVAPSQSLSVT CTVSGVSLPDYGVSWIRQPPRKGLEWLGVIWGSETTYYNS ALKSRLTIIKDNSKSQVFLKMNSLQTDDTAIYYCAKHYYYGG SYAMDYWGQGTSVTVSSTTTPAPRPPTPAPTIASQPLSLRP EACRPAAGGAVHTRGLDFACDIYIWAPLAGTCGVLLLSLVIT LYCKRGRKKLLYIFKQPFMRPVQTTQEEDGCSCRFPEEEE GGCELRVKFSRSADAPAYKQGQNQLYNELNLGRREEYDVL DKRRGRDPEMGGKPRRKNPQEGLYNELQKDKMAEAYSEI GMKGERRRGKGHDGLYQGLSTATKDTYDALHMQALPPR | 213411 | 213450 |
| OT-CD19 CAR-002 (CD8a leader; CD19 scFV; FKBP (F36V, L106P); CD8a hinge+TM; 41BB; CD3zeta) | MALPVTALLLPLALLLHAARPDIQMTQTTSSLSASLGDRVTIS CRASQDISKYLNWYQQKPDGTVKLLIYHTSRLHSGVPSRFS GSGSGTDYSLTISNLEQEDIATYFCQQGNTLPYTFGGGTKL EITGGGGSGGGGSGGGGSEVKLQESGPGLVAPSQSLSVT CTVSGVSLPDYGVSWIRQPPRKGLEWLGVIWGSETTYYNS ALKSRLTIIKDNSKSQVFLKMNSLQTDDTAIYYCAKHYYYGG SYAMDYWGQGTSVTVSSGVQVETISPGDGRTFPKRGQTC VVHYTGMLEDGKKVDSSRDRNKPFKFMLGKQEVIRGWEE GVAQMSVGQRAKLTISPDYAYGATGHPGIIPPHATLVFDVEL LKPETTTPAPRPPTPAPTIASQPLSLRPEACRPAAGGAVHT RGLDFACDIYIWAPLAGTCGVLLLSLVITLYCKRGRKKLLYIF KQPFMRPVQTTQEEDGCSCRFPEEEEGGCELRVKFSRSA DAPAYKQGQNQLYNELNLGRREEYDVLDKRRGRDPEMGG KPRRKNPQEGLYNELQKDKMAEAYSEIGMKGERRRGKGH DGLYQGLSTATKDTYDALHMQALPPR | 213412 | 213451 |
| OT-CD 19CAR-003 (CD8a leader; CD19 scFV; ecDHFR (R12Y, Y100I); CD8a hinge+TM; 41BB; CD3zeta) | MALPVTALLLPLALLLHAARPDIQMTQTTSSLSASLGDRVTIS CRASQDISKYLNWYQQKPDGTVKLLIYHTSRLHSGVPSRFS GSGSGTDYSLTISNLEQEDIATYFCQQGNTLPYTFGGGTKL EITGGGGSGGGGSGGGGSEVKLQESGPGLVAPSQSLSVT CTVSGVSLPDYGVSWIRQPPRKGLEWLGVIWGSETTYYNS ALKSRLTIIKDNSKSQVFLKMNSLQTDDTAIYYCAKHYYYGG SYAMDYWGQGTSVTVSSISLIAALAVDYVIGMENAMPWNLP ADLAWFKRNTLNKPVIMGRHTWESIGRPLPGRKNIILSSQP GTDDRVTWVKSVDEAIAACGDVPEIMVIGGGRVIEQFLPKA QKLYLTHIDAEVEGDTHFPDYEPDDWESVFSEFHDADAQN SHSYCFEILERRTTTPAPRPPTPAPTIASQPLSLRPEACRPA AGGAVHTRGLDFACDIYIWAPLAGTCGVLLLSLVITLYCKRG RKKLLYIFKQPFMRPVQTTQEEDGCSCRFPEEEEGGCELR VKFSRSADAPAYKQGQNQLYNELNLGRREEYDVLDKRRGR DPEMGGKPRRKNPQEGLYNELQKDKMAEAYSEIGMKGER RRGKGHDGLYQGLSTATKDTYDALHMQALPPR | 213413 | 213452 |
| OT-CD19 CAR-004 (CD8a leader; CD19 scFV; CD8a hinge FKBP (F36V, L106P) CD8a TM- CD8 hinge seq following TM; 41BB; CD3zeta) | MALPVTALLLPLALLLHAARPDIQMTQTTSSLSASLGDRVTIS CRASQDISKYLNWYQQKPDGTVKLLIYHTSRLHSGVPSRFS GSGSGTDYSLTISNLEQEDIATYFCQQGNTLPYTFGGGTKL EITGGGGSGGGGSGGGGSEVKLQESGPGLVAPSQSLSVT CTVSGVSLPDYGVSWIRQPPRKGLEWLGVIWGSETTYYNS ALKSRLTIIKDNSKSQVFLKMNSLQTDDTAIYYCAKHYYYGG SYAMDYWGQGTSVTVSSTTTPAPRPPTPAPTIASQPLSLRP EACRPAAGGAVHTRGLDFACDGVQVETISPGDGRTFPKRG QTCVVHYTGMLEDGKKVDSSRDRNKPFKFMLGKQEVIRG WEEGVAQMSVGQRAKLTISPDYAYGATGHPGIIPPHATLVF DVELLKPEIYIWAPLAGTCGVLLLSLVITLYCKRGRKKLLYIFK QPFMRPVQTTQEEDGCSCRFPEEEEGGCELRVKFSRSAD APAYKQGQNQLYNELNLGRREEYDVLDKRRGRDPEMGGK PRRKNPQEGLYNELQKDKMAEAYSEIGMKGERRRGKGHD GLYQGLSTATKDTYDALHMQALPPR | 213414 | 213453 |
| OT-CD19 CAR-005 (CD8a leader; CD19 scFV; ecDHFR (R12Y; Y100I); 41BB; CD3zeta) | MALPVTALLLPLALLLHAARPDIQMTQTTSSLSASLGDRVTIS CRASQDISKYLNWYQQKPDGTVKLLIYHTSRLHSGVPSRFS GSGSGTDYSLTISNLEQEDIATYFCQQGNTLPYTFGGGTKL EITGGGGSGGGGSGGGGSEVKLQESGPGLVAPSQSLSVT CTVSGVSLPDYGVSWIRQPPRKGLEWLGVIWGSETTYYNS ALKSRLTIIKDNSKSQVFLKMNSLQTDDTAIYYCAKHYYYGG SYAMDYWGQGTSVTVSSTTTPAPRPPTPAPTIASQPLSLRP EACRPAAGGAVHTRGLDFACDISLIAALAVDYVIGMENAMP WNLPADLAWFKRNTLNKPVIMGRHTWESIGRPLPGRKNIIL SSQPGTDDRVTWVKSVDEAIAACGDVPEIMVIGGGRVIEQF LPKAQKLYLTHIDAEVEGDTHFPDYEPDDWESVFSEFHDAD AQNSHSYCFEILERRIYIWAPLAGTCGVLLLSLVITLYCKRGR KKLLYIFKQPFMRPVQTTQEEDGCSCRFPEEEEGGCELRVK FSRSADAPAYKQGQNQLYNELNLGRREEYDVLDKRRGRDP EMGGKPRRKNPQEGLYNELQKDKMAEAYSEIGMKGERRR GKGHDGLYQGLSTATKDTYDALHMQALPPR | 213415 | 213454 |
| OT-CD19C CAR-006 (CD8a leader; CD19 scFV; CD8a hinge+TM; 41BB; CD3zeta; linker1 (GGSGG) ecDHFR (R12H; E129K)) | MALPVTALLLPLALLLHAARPDIQMTQTTSSLSASLGDRVTIS CRASQDISKYLNWYQQKPDGTVKLLIYHTSRLHSGVPSRFS GSGSGTDYSLTISNLEQEDIATYFCQQGNTLPYTFGGGTKL EITGGGGSGGGGSGGGGSEVKLQESGPGLVAPSQSLSVT CTVSGVSLPDYGVSWIRQPPRKGLEWLGVIWGSETTYYNS ALKSRLTIIKDNSKSQVFLKMNSLQTDDTAIYYCAKHYYYGG SYAMDYWGQGTSVTVSSTTTPAPRPPTPAPTIASQPLSLRP EACRPAAGGAVHTRGLDFACDIYIWAPLAGTCGVLLLSLVIT LYCKRGRKKLLYIFKQPFMRPVQTTQEEDGCSCRFPEEEE GGCELRVKFSRSADAPAYKQGQNQLYNELNLGRREEYDVL DKRRGRDPEMGGKPRRKNPQEGLYNELQKDKMAEAYSEI GMKGERRRGKGHDGLYQGLSTATKDTYDALHMQALPPRG GSGGISLIAALAVDHVIGMENAMPWNLPADLAWFKRNTLNK PVIMGRHTWESIGRPLPGRKNIILSSQPGTDDRVTWVKSVD EAIAACGDVPEIMVIGGGRVYEQFLPKAQKLYLTHIDAEVEG DTHFPDYKPDDWESVFSEFHDADAQNSHSYCFEILERR | 213416 | 213455 |
| OT-CD19C-007 (CD8a leader; CD19 scFV; CD8a hinge+TM; 41BB; CD3zeta; linker1 (GGSGG); FKBP (E31G, F36V, R71G, K105E)) | MALPVTALLLPLALLLHAARPDIQMTQTTSSLSASLGDRVTIS CRASQDISKYLNWYQQKPDGTVKLLIYHTSRLHSGVPSRFS GSGSGTDYSLTISNLEQEDIATYFCQQGNTLPYTFGGGTKL EITGGGGSGGGGSGGGGSEVKLQESGPGLVAPSQSLSVT CTVSGVSLPDYGVSWIRQPPRKGLEWLGVIWGSETTYYNS ALKSRLTIIKDNSKSQVFLKMNSLQTDDTAIYYCAKHYYYGG SYAMDYWGQGTSVTVSSTTTPAPRPPTPAPTIASQPLSLRP EACRPAAGGAVHTRGLDFACDIYIWAPLAGTCGVLLLSLVIT LYCKRGRKKLLYIFKQPFMRPVQTTQEEDGCSCRFPEEEE GGCELRVKFSRSADAPAYKQGQNQLYNELNLGRREEYDVL DKRRGRDPEMGGKPRRKNPQEGLYNELQKDKMAEAYSEI | 213417 | 213456 |
| | GMKGERRRGKGHDGLYQGLSTATKDTYDALHMQALPPRG GSGGGVQVETISPGDGRTFPKRGQTCVVHYTGMLGDGKK VDSSRDRNKPFKFMLGKQEVIRGWEEGVAQMSVGQGAKL TISPDYAYGATGHPGIIPPHATLVFDVELLELE | | |

In one embodiment, the chimeric antigen receptor (CAR) of the present invention may be a conditionally active CAR. A wild type protein or domain thereof, such as those described herein may be used to generate a conditionally active biologic protein which are reversibly or irreversibly inactivated at the wild type normal physiological conditions as well as to such conditionally active biologic proteins and domains and uses of such conditional active biologic proteins and domains are provided. Such methods and conditionally active proteins are taught in, for example, International Publication No. WO2016033331. As a non-limiting example, the CAR comprises at least one antigen specific targeting region evolved from a wild type protein or a domain thereof and one or more of a decrease in activity in the assay at the normal physiological condition compared to the antigen specific targeting region of the wild-type protein or a domain thereof, and an increase in activity in the assay under the aberrant condition compared to the antigen specific targeting region of the wild-type protein or a domain thereof.

### IV. FORMULATIONS

The compositions of the present invention may be formulated in any manner suitable for delivery. The formulation may be, but is not limited to, nanoparticles, poly (lactic-co-glycolic acid) (PLGA) microspheres, lipidoids, lipoplex, liposome, polymers, carbohydrates (including simple sugars), cationic lipids and combinations thereof.

In one embodiment, the formulation is a nanoparticle which may comprise at least one lipid. The lipid may be selected from, but is not limited to, DLin-DMA, DLin-K-DMA, 98N12-5, C12-200, DLin-MC3-DMA, DLin-KC2-DMA, DODMA, PLGA, PEG, PEG-DMG and PEGylated lipids. In another aspect, the lipid may be a cationic lipid such as, but not limited to, DLin-DMA, DLin-D-DMA, DLin-MC3-DMA, DLin-KC2-DMA and DODMA.

For polynucleotides of the invention, the formulation may be selected from any of those taught, for example, in International Application PCT/US2012/069610.

### Inactive ingredients

In some embodiments, pharmaceutical or other formulations may comprise at least one excipient which is an inactive ingredient. As used herein, the term "inactive ingredient" refers to one or more inactive agents included in formulations. In some embodiments, all, none or some of the inactive ingredients which may be used in the formulations of the present invention may be approved by the US Food and Drug Administration (FDA). A non-exhaustive list of inactive ingredients includes, 1,2,6-Hexanetriol, 1,2-Dimyristoyl-Sn-Glycero-3-(Phospho-S-(1-Glycerol)), 1,2-Dimyristoyl-Sn-Glycero-3-Phosphocholine, 1,2-Dioleoyl-Sn-Glycero-3-Phosphocholine, 1,2-Dipalmitoyl-Sn-Glycero-3-(Phospho-Rac-(1-Glycerol)), 1,2-Distearoyl-Sn-Glycero-3-(Phospho-Rac-(1-Glycerol)), 1,2-Distearoyl-Sn-Glycero-3-Phosphocholine, 1-O-Tolylbiguanide, 2-Ethyl-1,6-Hexanediol, Acetic Acid, Acetic Acid, Glacial, Acetic Anhydride, Acetone, Acetone Sodium Bisulfite, Acetylated Lanolin Alcohols, Acetylated Monoglycerides, Acetylcysteine, Acetyltryptophan, DL-, Acrylates Copolymer, Acrylic Acid-Isooctyl Acrylate Copolymer, Acrylic Adhesive 788, Activated Charcoal, Adcote 72A103, Adhesive Tape, Adipic Acid, Aerotex Resin 3730, Alanine, Albumin Aggregated, Albumin Colloidal, Albumin Human, Alcohol, Alcohol, Dehydrated, Alcohol, Denatured, Alcohol, Diluted, Alfadex, Alginic Acid, Alkyl Ammonium Sulfonic Acid Betaine, Alkyl Aryl Sodium Sulfonate, Allantoin, Allyl .Alpha.-Ionone, Almond Oil, Alpha.-Terpineol, Alpha.-Tocopherol, Alpha.-Tocopherol Acetate, DI-, Alpha.-Tocopherol, DI-, Aluminum Acetate, Aluminum Chlorhydroxy Allantoinate, Aluminum Hydroxide, Aluminum Hydroxide - Sucrose, Hydrated, Aluminum Hydroxide Gel, Aluminum Hydroxide Gel F 500, Aluminum Hydroxide Gel F 5000, Aluminum Monostearate, Aluminum Oxide, Aluminum Polyester, Aluminum Silicate, Aluminum Starch Octenylsuccinate, Aluminum Stearate, Aluminum Subacetate, Aluminum Sulfate Anhydrous, Amerchol C, Amerchol-Cab, Aminomethylpropanol, Ammonia, Ammonia Solution, Ammonia Solution, Strong, Ammonium Acetate, Ammonium Hydroxide, Ammonium Lauryl Sulfate, Ammonium Nonoxynol-4 Sulfate, Ammonium Salt Of C-12-C-15 Linear Primary Alcohol Ethoxylate, Ammonium Sulfate, Ammonyx, Amphoteric-2, Amphoteric-9, Anethole, Anhydrous Citric Acid, Anhydrous Dextrose, Anhydrous Lactose, Anhydrous Trisodium Citrate, Aniseed Oil, Anoxid Sbn, Antifoam, Antipyrine, Apaflurane, Apricot Kernel Oil Peg-6 Esters, Aquaphor, Arginine, Arlacel, Ascorbic Acid, Ascorbyl Palmitate, Aspartic Acid, Balsam Peru, Barium Sulfate, Beeswax, Beeswax, Synthetic, Beheneth-10, Bentonite, Benzalkonium Chloride, Benzenesulfonic Acid, Benzethonium Chloride, Benzododecinium Bromide, Benzoic Acid, Benzyl Alcohol, Benzyl Benzoate, Benzyl Chloride, Betadex, Bibapcitide, Bismuth Subgallate, Boric Acid, Brocrinat, Butane, Butyl Alcohol, Butyl Ester Of Vinyl Methyl Ether/Maleic Anhydride Copolymer (125000 Mw), Butyl Stearate, Butylated Hydroxyanisole, Butylated Hydroxytoluene, Butylene Glycol, Butylparaben, Butyric Acid, C20-40 Pareth-24, Caffeine, Calcium, Calcium Carbonate, Calcium Chloride, Calcium Gluceptate, Calcium Hydroxide, Calcium Lactate, Calcobutrol, Caldiamide Sodium, Caloxetate Trisodium, Calteridol Calcium, Canada Balsam, Caprylic/Capric Triglyceride, Caprylic/Capric/Stearic Triglyceride, Captan, Captisol, Caramel, Carbomer 1342, Carbomer 1382, Carbomer 934, Carbomer 934p, Carbomer 940, Carbomer 941, Carbomer 980, Carbomer 981, Carbomer Homopolymer Type B (Allyl Pentaerythritol Crosslinked), Carbomer Homopolymer Type C (Allyl Pentaerythritol Crosslinked), Carbon Dioxide, Carboxy Vinyl Copolymer, Carboxymethylcellulose, Carboxymethylcellulose Sodium, Carboxypolymethylene, Carrageenan, Carrageenan Salt, Castor Oil, Cedar Leaf Oil, Cellulose, Cellulose, Microcrystalline, Cerasynt-Se, Ceresin, Ceteareth-12, Ceteareth-15, Ceteareth-30, Cetearyl Alcohol/Ceteareth-20, Cetearyl Ethylhexanoate, Ceteth-10, Ceteth-2, Ceteth-20, Ceteth-23, Cetostearyl Alcohol, Cetrimonium Chloride, Cetyl Alcohol, Cetyl Esters Wax, Cetyl Palmitate, Cetylpyridinium Chloride, Chlorobutanol, Chlorobutanol Hemihydrate, Chlorobutanol, Anhydrous, Chlorocresol, Chloroxylenol, Cholesterol, Choleth, Choleth-24, Citrate, Citric Acid, Citric Acid Monohydrate, Citric Acid, Hydrous, Cocamide Ether Sulfate, Cocamine Oxide, Coco Betaine, Coco Diethanolamide, Coco Monoethanolamide, Cocoa Butter, Coco-Glycerides, Coconut Oil, Coconut Oil, Hydrogenated, Coconut Oil/Palm Kernel Oil Glycerides, Hydrogenated, Cocoyl Caprylocaprate, Cola Nitida Seed Extract, Collagen, Coloring Suspension, Corn Oil, Cottonseed Oil, Cream Base, Creatine, Creatinine, Cresol, Croscarmellose Sodium, Crospovidone, Cupric Sulfate, Cupric Sulfate Anhydrous, Cyclomethicone, Cyclomethicone/Dimethicone Copolyol, Cysteine, Cysteine Hydrochloride, Cysteine Hydrochloride Anhydrous, Cysteine, DI-, D&C Red No. 28, D&C Red No. 33, D&C Red No. 36, D&C Red No. 39, D&C Yellow No. 10, Dalfampridine, Daubert 1-5 Pestr (Matte) 164z, Decyl Methyl Sulfoxide, Dehydag Wax Sx, Dehydroacetic Acid, Dehymuls E, Denatonium Benzoate, Deoxycholic Acid, Dextran, Dextran 40, Dextrin, Dextrose, Dextrose Monohydrate, Dextrose Solution, Diatrizoic Acid, Diazolidinyl Urea, Dichlorobenzyl Alcohol, Dichlorodifluoromethane, Dichlorotetrafluoroethane, Diethanolamine, Diethyl Pyrocarbonate, Diethyl Sebacate, Diethylene Glycol Monoethyl Ether, Diethylhexyl Phthalate, Dihydroxyaluminum Aminoacetate, Diisopropanolamine, Diisopropyl Adipate, Diisopropyl Dilinoleate, Dimethicone 350, Dimethicone Copolyol, Dimethicone Mdx4-4210, Dimethicone Medical Fluid 360, Dimethyl Isosorbide, Dimethyl Sulfoxide, Dimethylaminoethyl Methacrylate - Butyl Methacrylate - Methyl Methacrylate Copolymer, Dimethyldioctadecylammonium Bentonite, Dimethylsiloxane/Methylvinylsiloxane Copolymer, Dinoseb Ammonium Salt, Dipalmitoylphosphatidylglycerol, DI-, Dipropylene Glycol, Disodium Cocoamphodiacetate, Disodium Laureth Sulfosuccinate, Disodium Lauryl Sulfosuccinate, Disodium Sulfosalicylate, Disofenin, Divinylbenzene Styrene Copolymer, Dmdm Hydantoin, Docosanol, Docusate Sodium, Duro-Tak 280-2516, Duro-Tak 387-2516, Duro-Tak 80-1196, Duro-Tak 87-2070, Duro-Tak 87-2194, Duro-Tak 87-2287, Duro-Tak 87-2296, Duro-Tak 87-2888, Duro-Tak 87-2979, Edetate Calcium Disodium, Edetate Disodium, Edetate Disodium Anhydrous, Edetate Sodium, Edetic Acid, Egg Phospholipids, Entsufon, Entsufon Sodium, Epilactose, Epitetracycline Hydrochloride, Essence Bouquet 9200, Ethanolamine Hydrochloride, Ethyl Acetate, Ethyl Oleate, Ethylcelluloses, Ethylene Glycol, Ethylene Vinyl Acetate Copolymer, Ethylenediamine, Ethylenediamine Dihydrochloride, Ethylene-Propylene Copolymer, Ethylene-Vinyl Acetate Copolymer (28% Vinyl Acetate), Ethylene-Vinyl Acetate Copolymer (9% Vinylacetate), Ethylhexyl Hydroxystearate, Ethylparaben, Eucalyptol, Exametazime, Fat, Edible, Fat, Hard, Fatty Acid Esters, Fatty Acid Pentaerythriol Ester, Fatty Acids, Fatty Alcohol Citrate, Fatty Alcohols, Fd&C Blue No. 1, Fd&C Green No. 3, Fd&C Red No. 4, Fd&C Red No. 40, Fd&C Yellow No. 10 (Delisted), Fd&C Yellow No. 5, Fd&C Yellow No. 6, Ferric Chloride, Ferric Oxide, Flavor 89-186, Flavor 89-259, Flavor Df-119, Flavor Df-1530, Flavor Enhancer, Flavor Fig 827118, Flavor Raspberry Pfc-8407, Flavor Rhodia Pharmaceutical No. Rf 451, Fluorochlorohydrocarbons, Formaldehyde, Formaldehyde Solution, Fractionated Coconut Oil, Fragrance 3949-5, Fragrance 520a, Fragrance 6.007, Fragrance 91-122, Fragrance 9128-Y, Fragrance 93498g, Fragrance Balsam Pine No. 5124, Fragrance Bouquet 10328, Fragrance Chemoderm 6401-B, Fragrance Chemoderm 6411, Fragrance Cream No. 73457, Fragrance Cs-28197, Fragrance Felton 066m, Fragrance Firmenich 47373, Fragrance Givaudan Ess 9090/1c, Fragrance H-6540, Fragrance Herbal 10396, Fragrance Nj-1085, Fragrance P O FI-147, Fragrance Pa 52805, Fragrance Pera Derm D, Fragrance Rbd-9819, Fragrance Shaw Mudge U-7776, Fragrance Tf 044078, Fragrance Ungerer Honeysuckle K 2771, Fragrance Ungerer N5195, Fructose, Gadolinium Oxide, Galactose, Gamma Cyclodextrin, Gelatin, Gelatin, Crosslinked, Gelfoam Sponge, Gellan Gum (Low Acyl), Gelva 737, Gentisic Acid, Gentisic Acid Ethanolamide, Gluceptate Sodium, Gluceptate Sodium Dihydrate, Gluconolactone, Glucuronic Acid, Glutamic Acid, DI-, Glutathione, Glycerin, Glycerol Ester Of Hydrogenated Rosin, Glyceryl Citrate, Glyceryl Isostearate, Glyceryl Laurate, Glyceryl Monostearate, Glyceryl Oleate, Glyceryl Oleate/Propylene Glycol, Glyceryl Palmitate, Glyceryl Ricinoleate, Glyceryl Stearate, Glyceryl Stearate - Laureth-23, Glyceryl Stearate/Peg Stearate, Glyceryl Stearate/Peg-100 Stearate, Glyceryl Stearate/Peg-40 Stearate, Glyceryl Stearate-Stearamidoethyl Diethylamine, Glyceryl Trioleate, Glycine, Glycine Hydrochloride, Glycol Distearate, Glycol Stearate, Guanidine Hydrochloride, Guar Gum, Hair Conditioner (18n195-1m), Heptane, Hetastarch, Hexylene Glycol, High Density Polyethylene, Histidine, Human Albumin Microspheres, Hyaluronate Sodium, Hydrocarbon, Hydrocarbon Gel, Plasticized, Hydrochloric Acid, Hydrochloric Acid, Diluted, Hydrocortisone, Hydrogel Polymer, Hydrogen Peroxide, Hydrogenated Castor Oil, Hydrogenated Palm Oil, Hydrogenated Palm/Palm Kernel Oil Peg-6 Esters, Hydrogenated Polybutene 635-690, Hydroxide lon, Hydroxyethyl Cellulose, Hydroxyethylpiperazine Ethane Sulfonic Acid, Hydroxymethyl Cellulose, Hydroxyoctacosanyl Hydroxystearate, Hydroxypropyl Cellulose, Hydroxypropyl Methylcellulose 2906, Hydroxypropyl-Bcyclodextrin, Hypromellose 2208 (15000 Mpa.S), Hypromellose 2910 (15000 Mpa.S), Hypromelloses, Imidurea, Iodine, lodoxamic Acid, lofetamine Hydrochloride, Irish Moss Extract, Isobutane, Isoceteth-20, Isoleucine, Isooctyl Acrylate, Isopropyl Alcohol, Isopropyl Isostearate, Isopropyl Myristate, Isopropyl Myristate - Myristyl Alcohol, Isopropyl Palmitate, Isopropyl Stearate, Isostearic Acid, Isostearyl Alcohol, Isotonic Sodium Chloride Solution, Jelene, Kaolin, Kathon Cg, Kathon Cg II, Lactate, Lactic Acid, Lactic Acid, DI-, Lactic Acid, L-, Lactobionic Acid, Lactose, Lactose Monohydrate, Lactose, Hydrous, Laneth, Lanolin, Lanolin Alcohol - Mineral Oil, Lanolin Alcohols, Lanolin Anhydrous, Lanolin Cholesterols, Lanolin Nonionic Derivatives, Lanolin, Ethoxylated, Lanolin, Hydrogenated, Lauralkonium Chloride, Lauramine Oxide, Laurdimonium Hydrolyzed Animal Collagen, Laureth Sulfate, Laureth-2, Laureth-23, Laureth-4, Lauric Diethanolamide, Lauric Myristic Diethanolamide, Lauroyl Sarcosine, Lauryl Lactate, Lauryl Sulfate, Lavandula Angustifolia Flowering Top, Lecithin, Lecithin Unbleached, Lecithin, Egg, Lecithin, Hydrogenated, Lecithin, Hydrogenated Soy, Lecithin, Soybean, Lemon Oil, Leucine, Levulinic Acid, Lidofenin, Light Mineral Oil, Light Mineral Oil (85 Ssu), Limonene, (+/-)-, Lipocol Sc-15, Lysine, Lysine Acetate, Lysine Monohydrate, Magnesium Aluminum Silicate, Magnesium Aluminum Silicate Hydrate, Magnesium Chloride, Magnesium Nitrate, Magnesium Stearate, Maleic Acid, Mannitol, Maprofix, Mebrofenin, Medical Adhesive Modified S-15, Medical Antiform A-F Emulsion, Medronate Disodium, Medronic Acid, Meglumine, Menthol, Metacresol, Metaphosphoric Acid, Methanesulfonic Acid, Methionine, Methyl Alcohol, Methyl Gluceth-10, Methyl Gluceth-20, Methyl Gluceth-20 Sesquistearate, Methyl Glucose Sesquistearate, Methyl Laurate, Methyl Pyrrolidone, Methyl Salicylate, Methyl Stearate, Methylboronic Acid, Methylcellulose (4000 Mpa.S), Methylcelluloses, Methylchloroisothiazolinone, Methylene Blue, Methylisothiazolinone, Methylparaben, Microcrystalline Wax, Mineral Oil, Mono And Diglyceride, Monostearyl Citrate, Monothioglycerol, Multisterol Extract, Myristyl Alcohol, Myristyl Lactate, Myristyl-.Gamma.-Picolinium Chloride, N-(Carbamoyl-Methoxy Peg-40)-1,2-Distearoyl-Cephalin Sodium, N,N-Dimethylacetamide, Niacinamide, Nioxime, Nitric Acid, Nitrogen, Nonoxynol Iodine, Nonoxynol-15, Nonoxynol-9, Norflurane, Oatmeal, Octadecene-1/Maleic Acid Copolymer, Octanoic Acid, Octisalate, Octoxynol-1, Octoxynol-40, Octoxynol-9, Octyldodecanol, Octylphenol Polymethylene, Oleic Acid, Oleth-10/Oleth-5, Oleth-2, Oleth-20, Oleyl Alcohol, Oleyl Oleate, Olive Oil, Oxidronate Disodium, Oxyquinoline, Palm Kernel Oil, Palmitamine Oxide, Parabens, Paraffin, Paraffin, White Soft, Parfum Creme 45/3, Peanut Oil, Peanut Oil, Refined, Pectin, Peg 6-32 Stearate/Glycol Stearate, Peg Vegetable Oil, Peg-100 Stearate, Peg-12 Glyceryl Laurate, Peg-120 Glyceryl Stearate, Peg-120 Methyl Glucose Dioleate, Peg-15 Cocamine, Peg-150 Distearate, Peg-2 Stearate, Peg-20 Sorbitan Isostearate, Peg-22 Methyl Ether/Dodecyl Glycol Copolymer, Peg-25 Propylene Glycol Stearate, Peg-4 Dilaurate, Peg-4 Laurate, Peg-40 Castor Oil, Peg-40 Sorbitan Diisostearate, Peg-45/Dodecyl Glycol Copolymer, Peg-5 Oleate, Peg-50 Stearate, Peg-54 Hydrogenated Castor Oil, Peg-6 Isostearate, Peg-60 Castor Oil, Peg-60 Hydrogenated Castor Oil, Peg-7 Methyl Ether, Peg-75 Lanolin, Peg-8 Laurate, Peg-8 Stearate, Pegoxol 7 Stearate, Pentadecalactone, Pentaerythritol Cocoate, Pentasodium Pentetate, Pentetate Calcium Trisodium, Pentetic Acid, Peppermint Oil, Perflutren, Perfume 25677, Perfume Bouquet, Perfume E-1991, Perfume Gd 5604, Perfume Tana 90/42 Scba, Perfume W-1952-1, Petrolatum, Petrolatum, White, Petroleum Distillates, Phenol, Phenol, Liquefied, Phenonip, Phenoxyethanol, Phenylalanine, Phenylethyl Alcohol, Phenylmercuric Acetate, Phenylmercuric Nitrate, Phosphatidyl Glycerol, Egg, Phospholipid, Phospholipid, Egg, Phospholipon 90g, Phosphoric Acid, Pine Needle Oil (Pinus Sylvestris), Piperazine Hexahydrate, Plastibase-50w, Polacrilin, Polidronium Chloride, Poloxamer 124, Poloxamer 181, Poloxamer 182, Poloxamer 188, Poloxamer 237, Poloxamer 407, Poly(Bis(P-Carboxyphenoxy) Propane Anhydride): Sebacic Acid, Poly(Dimethylsiloxane/Methylvinylsiloxane/Methylhydrogensiloxane) Dimethylvinyl Or Dimethylhydroxy Or Trimethyl Endblocked, Poly(DI-Lactic-Co-Glycolic Acid), (50:50, Poly(DI-Lactic-Co-Glycolic Acid), Ethyl Ester Terminated, (50:50, Polyacrylic Acid (250000 Mw), Polybutene (1400 Mw), Polycarbophil, Polyester, Polyester Polyamine Copolymer, Polyester Rayon, Polyethylene Glycol 1000, Polyethylene Glycol 1450, Polyethylene Glycol 1500, Polyethylene Glycol 1540, Polyethylene Glycol 200, Polyethylene Glycol 300, Polyethylene Glycol 300-1600, Polyethylene Glycol 3350, Polyethylene Glycol 400, Polyethylene Glycol 4000, Polyethylene Glycol 540, Polyethylene Glycol 600, Polyethylene Glycol 6000, Polyethylene Glycol 8000, Polyethylene Glycol 900, Polyethylene High Density Containing Ferric Oxide Black (<1%), Polyethylene Low Density Containing Barium Sulfate (20-24%), Polyethylene T, Polyethylene Terephthalates, Polyglactin, Polyglyceryl-3 Oleate, Polyglyceryl-4 Oleate, Polyhydroxyethyl Methacrylate, Polyisobutylene, Polyisobutylene (1100000 Mw), Polyisobutylene (35000 Mw), Polyisobutylene 178-236, Polyisobutylene 241-294, Polyisobutylene 35-39, Polyisobutylene Low Molecular Weight, Polyisobutylene Medium Molecular Weight, Polyisobutylene/Polybutene Adhesive, Polylactide, Polyols, Polyoxyethylene - Polyoxypropylene 1800, Polyoxyethylene Alcohols, Polyoxyethylene Fatty Acid Esters, Polyoxyethylene Propylene, Polyoxyl 20 Cetostearyl Ether, Polyoxyl 35 Castor Oil, Polyoxyl 40 Hydrogenated Castor Oil, Polyoxyl 40 Stearate, Polyoxyl 400 Stearate, Polyoxyl 6 And Polyoxyl 32 Palmitostearate, Polyoxyl Distearate, Polyoxyl Glyceryl Stearate, Polyoxyl Lanolin, Polyoxyl Palmitate, Polyoxyl Stearate, Polypropylene, Polypropylene Glycol, Polyquaternium-10, Polyquaternium-7 (70/30 Acrylamide/Dadmac, Polysiloxane, Polysorbate 20, Polysorbate 40, Polysorbate 60, Polysorbate 65, Polysorbate 80, Polyurethane, Polyvinyl Acetate, Polyvinyl Alcohol, Polyvinyl Chloride, Polyvinyl Chloride-Polyvinyl Acetate Copolymer, Polyvinylpyridine, Poppy Seed Oil, Potash, Potassium Acetate, Potassium Alum, Potassium Bicarbonate, Potassium Bisulfite, Potassium Chloride, Potassium Citrate, Potassium Hydroxide, Potassium Metabisulfite, Potassium Phosphate, Dibasic, Potassium Phosphate, Monobasic, Potassium Soap, Potassium Sorbate, Povidone Acrylate Copolymer, Povidone Hydrogel, Povidone K17, Povidone K25, Povidone K29/32, Povidone K30, Povidone K90, Povidone K90f, Povidone/Eicosene Copolymer, Povidones, Ppg-12/Smdi Copolymer, Ppg-15 Stearyl Ether, Ppg-20 Methyl Glucose Ether Distearate, Ppg-26 Oleate, Product Wat, Proline, Promulgen D, Promulgen G, Propane, Propellant A-46, Propyl Gallate, Propylene Carbonate, Propylene Glycol, Propylene Glycol Diacetate, Propylene Glycol Dicaprylate, Propylene Glycol Monolaurate, Propylene Glycol Monopalmitostearate, Propylene Glycol Palmitostearate, Propylene Glycol Ricinoleate, Propylene Glycol/Diazolidinyl Urea/Methylparaben/Propylparben, Propylparaben, Protamine Sulfate, Protein Hydrolysate, Pvm/Ma Copolymer, Quaternium-15, Quaternium-15 Cis-Form, Quaternium-52, Ra-2397, Ra-3011, Saccharin, Saccharin Sodium, Saccharin Sodium Anhydrous, Safflower Oil, Sd Alcohol 3a, Sd Alcohol 40, Sd Alcohol 40-2, Sd Alcohol 40b, Sepineo P 600, Serine, Sesame Oil, Shea Butter, Silastic Brand Medical Grade Tubing, Silastic Medical Adhesive, Silicone Type A, Silica, Dental, Silicon, Silicon Dioxide, Silicon Dioxide, Colloidal, Silicone, Silicone Adhesive 4102, Silicone Adhesive 4502, Silicone Adhesive Bio-Psa Q7-4201, Silicone Adhesive Bio-Psa Q7-4301, Silicone Emulsion, Silicone/Polyester Film Strip, Simethicone, Simethicone Emulsion, Sipon Ls 20np, Soda Ash, Sodium Acetate, Sodium Acetate Anhydrous, Sodium Alkyl Sulfate, Sodium Ascorbate, Sodium Benzoate, Sodium Bicarbonate, Sodium Bisulfate, Sodium Bisulfite, Sodium Borate, Sodium Borate Decahydrate, Sodium Carbonate, Sodium Carbonate Decahydrate, Sodium Carbonate Monohydrate, Sodium Cetostearyl Sulfate, Sodium Chlorate, Sodium Chloride, Sodium Chloride Injection, Sodium Chloride Injection, Bacteriostatic, Sodium Cholesteryl Sulfate, Sodium Citrate, Sodium Cocoyl Sarcosinate, Sodium Desoxycholate, Sodium Dithionite, Sodium Dodecylbenzenesulfonate, Sodium Formaldehyde Sulfoxylate, Sodium Gluconate, Sodium Hydroxide, Sodium Hypochlorite, Sodium Iodide, Sodium Lactate, Sodium Lactate, L-, Sodium Laureth-2 Sulfate, Sodium Laureth-3 Sulfate, Sodium Laureth-5 Sulfate, Sodium Lauroyl Sarcosinate, Sodium Lauryl Sulfate, Sodium Lauryl Sulfoacetate, Sodium Metabisulfite, Sodium Nitrate, Sodium Phosphate, Sodium Phosphate Dihydrate, Sodium Phosphate, Dibasic, Sodium Phosphate, Dibasic, Anhydrous, Sodium Phosphate, Dibasic, Dihydrate, Sodium Phosphate, Dibasic, Dodecahydrate, Sodium Phosphate, Dibasic, Heptahydrate, Sodium Phosphate, Monobasic, Sodium Phosphate, Monobasic, Anhydrous, Sodium Phosphate, Monobasic, Dihydrate, Sodium Phosphate, Monobasic, Monohydrate, Sodium Polyacrylate (2500000 Mw), Sodium Pyrophosphate, Sodium Pyrrolidone Carboxylate, Sodium Starch Glycolate, Sodium Succinate Hexahydrate, Sodium Sulfate, Sodium Sulfate Anhydrous, Sodium Sulfate Decahydrate, Sodium Sulfite, Sodium Sulfosuccinated Undecyclenic Monoalkylolamide, Sodium Tartrate, Sodium Thioglycolate, Sodium Thiomalate, Sodium Thiosulfate, Sodium Thiosulfate Anhydrous, Sodium Trimetaphosphate, Sodium Xylenesulfonate, Somay 44, Sorbic Acid, Sorbitan, Sorbitan Isostearate, Sorbitan Monolaurate, Sorbitan Monooleate, Sorbitan Monopalmitate, Sorbitan Monostearate, Sorbitan Sesquioleate, Sorbitan Trioleate, Sorbitan Tristearate, Sorbitol, Sorbitol Solution, Soybean Flour, Soybean Oil, Spearmint Oil, Spermaceti, Squalane, Stabilized Oxychloro Complex, Stannous 2-Ethylhexanoate, Stannous Chloride, Stannous Chloride Anhydrous, Stannous Fluoride, Stannous Tartrate, Starch, Starch 1500, Pregelatinized, Starch, Corn, Stearalkonium Chloride, Stearalkonium Hectorite/Propylene Carbonate, Stearamidoethyl Diethylamine, Steareth-10, Steareth-100, Steareth-2, Steareth-20, Steareth-21, Steareth-40, Stearic Acid, Stearic Diethanolamide, Stearoxytrimethylsilane, Steartrimonium Hydrolyzed Animal Collagen, Stearyl Alcohol, Sterile Water For Inhalation, Styrene/Isoprene/Styrene Block Copolymer, Succimer, Succinic Acid, Sucralose, Sucrose, Sucrose Distearate, Sucrose Polyesters, Sulfacetamide Sodium, Sulfobutylether .Beta.-Cyclodextrin, Sulfur Dioxide, Sulfuric Acid, Sulfurous Acid, Surfactol Qs, Tagatose, D-, Talc, Tall Oil, Tallow Glycerides, Tartaric Acid, Tartaric Acid, DI-, Tenox, Tenox-2, Tert-Butyl Alcohol, Tert-Butyl Hydroperoxide, Tert-Butylhydroquinone, Tetrakis(2-Methoxyisobutylisocyanide)Copper(I) Tetrafluoroborate, Tetrapropyl Orthosilicate, Tetrofosmin, Theophylline, Thimerosal, Threonine, Thymol, Tin, Titanium Dioxide, Tocopherol, Tocophersolan, Triacetin, Tricaprylin, Trichloromonofluoromethane, Trideceth-10, Triethanolamine Lauryl Sulfate, Trifluoroacetic Acid, Triglycerides, Medium Chain, Trihydroxystearin, Trilaneth-4 Phosphate, Trilaureth-4 Phosphate, Trisodium Citrate Dihydrate, Trisodium Hedta, Triton 720, Triton X-200, Trolamine, Tromantadine, Tromethamine, Tryptophan, Tyloxapol, Tyrosine, Undecylenic Acid, Union 76 Amsco-Res 6038, Urea, Valine, Vegetable Oil, Vegetable Oil Glyceride, Hydrogenated, Vegetable Oil, Hydrogenated, Versetamide, Viscarin, Viscose/Cotton, Vitamin E, Wax, Emulsifying, Wecobee Fs, White Ceresin Wax, White Wax, Xanthan Gum, Zinc, Zinc Acetate, Zinc Carbonate, Zinc Chloride, and/or Zinc Oxide.

### V. DOSING AND ADMINISTRATION AND DELIVERY

### Dosing

The present invention provides methods comprising administering any one or more effector modules to a subject in need thereof. These may be administered to a subject using any amount and any route of administration effective for preventing or treating or imaging a disease, disorder, and/or condition (e.g., a disease, disorder, and/or condition relating to working memory deficits). The exact amount required will vary from subject to subject, depending on the species, age, and general condition of the subject, the severity of the disease, the particular composition, its mode of administration, its mode of activity, and the like.

Compositions in accordance with the invention are typically formulated in dosage unit form for ease of administration and uniformity of dosage. It will be understood, however, that the total daily usage of the compositions of the present invention may be decided by the attending physician within the scope of sound medical judgment. The specific therapeutically effective, prophylactically effective, or appropriate imaging dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

### Administration

The pharmaceutical compositions, or effector modules of the present invention may be administered by any route to achieve a therapeutically effective outcome. These include, but are not limited to enteral (into the intestine), gastroenteral, epidural (into the dura matter), oral (by way of the mouth), transdermal, peridural, intracerebral (into the cerebrum), intracerebroventricular (into the cerebral ventricles), epicutaneous (application onto the skin), intradermal, (into the skin itself), subcutaneous (under the skin), nasal administration (through the nose), intravenous (into a vein), intravenous bolus, intravenous drip, intraarterial (into an artery), intramuscular (into a muscle), intracardiac (into the heart), intraosseous infusion (into the bone marrow), intrathecal (into the spinal canal), intraperitoneal, (infusion or injection into the peritoneum), intravesical infusion, intravitreal, (through the eye), intracavernous injection (into a pathologic cavity) intracavitary (into the base of the penis), intravaginal administration, intrauterine, extra-amniotic administration, transdermal (diffusion through the intact skin for systemic distribution), transmucosal (diffusion through a mucous membrane), transvaginal, insufflation (snorting), sublingual, sublabial, enema, eye drops (onto the conjunctiva), in ear drops, auricular (in or by way of the ear), buccal (directed toward the cheek), conjunctival, cutaneous, dental (to a tooth or teeth), electro-osmosis, endocervical, endosinusial, endotracheal, extracorporeal, hemodialysis, infiltration, interstitial, intra-abdominal, intra-amniotic, intra-articular, intrabiliary, intrabronchial, intrabursal, intracartilaginous (within a cartilage), intracaudal (within the cauda equine), intracisternal (within the cisterna magna cerebellomedularis), intracorneal (within the cornea), dental intracornal, intracoronary (within the coronary arteries), intracorporus cavernosum (within the dilatable spaces of the corporus cavernosa of the penis), intradiscal (within a disc), intraductal (within a duct of a gland), intraduodenal (within the duodenum), intradural (within or beneath the dura), intraepidermal (to the epidermis), intraesophageal (to the esophagus), intragastric (within the stomach), intragingival (within the gingivae), intraileal (within the distal portion of the small intestine), intralesional (within or introduced directly to a localized lesion), intraluminal (within a lumen of a tube), intralymphatic (within the lymph), intramedullary (within the marrow cavity of a bone), intrameningeal (within the meninges), intramyocardial (within the myocardium), intraocular (within the eye), intraovarian (within the ovary), intrapericardial (within the pericardium), intrapleural (within the pleura), intraprostatic (within the prostate gland), intrapulmonary (within the lungs or its bronchi), intrasinal (within the nasal or periorbital sinuses), intraspinal (within the vertebral column), intrasynovial (within the synovial cavity of a joint), intratendinous (within a tendon), intratesticular (within the testicle), intrathecal (within the cerebrospinal fluid at any level of the cerebrospinal axis), intrathoracic (within the thorax), intratubular (within the tubules of an organ), intratumor (within a tumor), intratympanic (within the aurus media), intravascular (within a vessel or vessels), intraventricular (within a ventricle), iontophoresis (by means of electric current where ions of soluble salts migrate into the tissues of the body), irrigation (to bathe or flush open wounds or body cavities), laryngeal (directly upon the larynx), nasogastric (through the nose and into the stomach), occlusive dressing technique (topical route administration which is then covered by a dressing which occludes the area), ophthalmic (to the external eye), oropharyngeal (directly to the mouth and pharynx), parenteral, percutaneous, periarticular, peridural, perineural, periodontal, rectal, respiratory (within the respiratory tract by inhaling orally or nasally for local or systemic effect), retrobulbar (behind the pons or behind the eyeball), intramyocardial (entering the myocardium), soft tissue, subarachnoid, subconjunctival, submucosal, topical, transplacental (through or across the placenta), transtracheal (through the wall of the trachea), transtympanic (across or through the tympanic cavity), ureteral (to the ureter), urethral (to the urethra), vaginal, caudal block, diagnostic, nerve block, biliary perfusion, cardiac perfusion, photopheresis or spinal.

### Parenteral and injectable administration

In some embodiments, pharmaceutical compositions, or effector modules of the present invention may be administered parenterally. Liquid dosage forms for oral and parenteral administration include, but are not limited to, pharmaceutically acceptable emulsions, microemulsions, solutions, suspensions, syrups, and/or elixirs. In addition to active ingredients, liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, oral compositions can include adjuvants such as wetting agents, emulsifying and suspending agents, sweetening, flavoring, and/or perfuming agents. In certain embodiments for parenteral administration, compositions are mixed with solubilizing agents such as CREMOPHOR^{®}, alcohols, oils, modified oils, glycols, polysorbates, cyclodextrins, polymers, and/or combinations thereof. In other embodiments, surfactants are included such as hydroxypropylcellulose.

Injectable preparations, for example, sterile injectable aqueous or oleaginous suspensions may be formulated according to the known art using suitable dispersing agents, wetting agents, and/or suspending agents. Sterile injectable preparations may be sterile injectable solutions, suspensions, and/or emulsions in nontoxic parenterally acceptable diluents and/or solvents, for example, as a solution in 1,3-butanediol. Among the acceptable vehicles and solvents that may be employed are water, Ringer's solution, U.S.P., and isotonic sodium chloride solution. Sterile, fixed oils are conventionally employed as a solvent or suspending medium. For this purpose, any bland fixed oil can be employed including synthetic mono- or diglycerides. Fatty acids such as oleic acid can be used in the preparation of injectables.

Injectable formulations may be sterilized, for example, by filtration through a bacterial-retaining filter, and/or by incorporating sterilizing agents in the form of sterile solid compositions which can be dissolved or dispersed in sterile water or other sterile injectable medium prior to use.

In order to prolong the effect of active ingredients, it is often desirable to slow the absorption of active ingredients from subcutaneous or intramuscular injections. This may be accomplished by the use of liquid suspensions of crystalline or amorphous material with poor water solubility. The rate of absorption of active ingredients depends upon the rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are prepared by entrapping the drug in liposomes or microemulsions which are compatible with body tissues.

### Rectal and vaginal administration

In some embodiments, pharmaceutical compositions or effector modules of the present invention may be administered rectally and/or vaginally. Compositions for rectal or vaginal administration are typically suppositories which can be prepared by mixing compositions with suitable non-irritating excipients such as cocoa butter, polyethylene glycol or a suppository wax which are solid at ambient temperature but liquid at body temperature and therefore melt in the rectum or vaginal cavity and release the active ingredient.

### Oral administration

In some embodiments, pharmaceutical compositions or effector modules of the present invention may be administered orally. Solid dosage forms for oral administration include capsules, tablets, pills, powders, and granules. In such solid dosage forms, an active ingredient is mixed with at least one inert, pharmaceutically acceptable excipient such as sodium citrate or dicalcium phosphate and/or fillers or extenders (*e.g*. starches, lactose, sucrose, glucose, mannitol, and silicic acid), binders (*e.g*. carboxymethylcellulose, alginates, gelatin, polyvinylpyrrolidinone, sucrose, and acacia), humectants (*e.g*. glycerol), disintegrating agents (*e.g*. agar, calcium carbonate, potato or tapioca starch, alginic acid, certain silicates, and sodium carbonate), solution retarding agents (*e.g*. paraffin), absorption accelerators (*e.g*. quaternary ammonium compounds), wetting agents (*e.g*. cetyl alcohol and glycerol monostearate), absorbents (*e.g*. kaolin and bentonite clay), and lubricants (*e.g*. talc, calcium stearate, magnesium stearate, solid polyethylene glycols, sodium lauryl sulfate), and mixtures thereof. In the case of capsules, tablets and pills, the dosage form may comprise buffering agents.

### Topical or transdermal administration

As described herein, pharmaceutical compositions or effector modules of the present invention may be formulated for administration topically. The skin may be an ideal target site for delivery as it is readily accessible. Three routes are commonly considered to deliver pharmaceutical compositions or effector modules of the present invention to the skin: (i) topical application (*e.g*. for local/regional treatment and/or cosmetic applications); (ii) intradermal injection (*e.g*. for local/regional treatment and/or cosmetic applications); and (iii) systemic delivery (*e.g*. for treatment of dermatologic diseases that affect both cutaneous and extracutaneous regions). Pharmaceutical compositions or effector modules of the present invention can be delivered to the skin by several different approaches known in the art.

In some embodiments, the invention provides for a variety of dressings (e.g., wound dressings) or bandages (e.g., adhesive bandages) for conveniently and/or effectively carrying out methods of the present invention. Typically dressing or bandages may comprise sufficient amounts of pharmaceutical compositions or effector modules of the present invention described herein to allow users to perform multiple treatments.

Dosage forms for topical and/or transdermal administration may include ointments, pastes, creams, lotions, gels, powders, solutions, sprays, inhalants and/or patches. Generally, active ingredients are admixed under sterile conditions with pharmaceutically acceptable excipients and/or any needed preservatives and/or buffers. Additionally, the present invention contemplates the use of transdermal patches, which often have the added advantage of providing controlled delivery of pharmaceutical compositions or effector modules of the present invention to the body. Such dosage forms may be prepared, for example, by dissolving and/or dispensing pharmaceutical compositions, or effector modules in the proper medium. Alternatively, or additionally, rates may be controlled by either providing rate controlling membranes and/or by dispersing pharmaceutical compositions, or effector modules in a polymer matrix and/or gel.

Formulations suitable for topical administration include, but are not limited to, liquid and/or semi liquid preparations such as liniments, lotions, oil in water and/or water in oil emulsions such as creams, ointments and/or pastes, and/or solutions and/or suspensions.

Topically-administrable formulations may, for example, comprise from about 1% to about 10% (w/w) active ingredient, although the concentration of active ingredient may be as high as the solubility limit of the active ingredient in the solvent. Formulations for topical administration may further comprise one or more of the additional ingredients described herein.

### Depot administration

As described herein, in some embodiments, pharmaceutical compositions or effector modules of the present invention are formulated in depots for extended release. Generally, specific organs or tissues ("target tissues") are targeted for administration.

In some aspects of the invention, pharmaceutical compositions or effector modules of the present invention are spatially retained within or proximal to target tissues. Provided are method of providing pharmaceutical compositions, or effector modules to target tissues of mammalian subjects by contacting target tissues (which comprise one or more target cells) with pharmaceutical compositions, or effector modules under conditions such that they are substantially retained in target tissues, meaning that at least 10, 20, 30, 40, 50, 60, 70, 80, 85, 90, 95, 96, 97, 98, 99, 99.9, 99.99 or greater than 99.99% of the composition is retained in the target tissues. Advantageously, retention is determined by measuring the amount of pharmaceutical compositions, or effector modules that enter one or more target cells. For example, at least 1%, 5%, 10%, 20%, 30%, 40%, 50%, 60%, 70%, 80%, 85%, 90%, 95%, 96%, 97%, 98%, 99%, 99.9%, 99.99% or greater than 99.99% of pharmaceutical compositions, or effector modules administered to subjects are present intracellularly at a period of time following administration. For example, intramuscular injection to mammalian subjects may be performed using aqueous compositions comprising pharmaceutical compositions or effector modules of the present invention and one or more transfection reagent, and retention is determined by measuring the amount of pharmaceutical compositions,or effector modules present in muscle cells.

Certain aspects of the invention are directed to methods of providing pharmaceutical compositions or effector modules of the present invention to a target tissues of mammalian subjects, by contacting target tissues (comprising one or more target cells) with pharmaceutical compositions, or effector modules under conditions such that they are substantially retained in such target tissues. Pharmaceutical compositions, or effector modules comprise enough active ingredient such that the effect of interest is produced in at least one target cell. In some embodiments, pharmaceutical compositions, or effector modules generally comprise one or more cell penetration agents, although "naked" formulations (such as without cell penetration agents or other agents) are also contemplated, with or without pharmaceutically acceptable carriers.

In some embodiments, the amount of a growth factor present in cells in a tissue is desirably increased. Preferably, this increase in growth factor is spatially restricted to cells within the target tissue. Thus, provided are methods of increasing the amount of growth factor of interest in tissues of mammalian subjects. In some embodiments, formulations are provided comprising pharmaceutical compositions, or effector modules characterized in that the unit quantity provided has been determined to produce a desired level of growth factor of interest in a substantial percentage of cells contained within predetermined volumes of target tissue.

In some embodiments, formulations comprise a plurality of different pharmaceutical compositions, or effector modules, where one or more than one targets biomolecules of interest. Optionally, formulations may also comprise cell penetration agents to assist in the intracellular delivery of pharmaceutical compositions, or effector modules. In such embodiments, determinations are made of compound and/or composition dose required to target biomolecules of interest in substantial percentages of cells contained within predetermined volumes of the target tissue (generally, without targeting biomolecules of interest in adjacent or distal tissues.) Determined doses are then introduced directly into subject tissues. In some embodiments, the invention provides for pharmaceutical compositions or effector modules to be delivered in more than one administration or by split dose administration.

### Pulmonary administration

In some embodiments, pharmaceutical compositions or effector modules of the present invention may be prepared, packaged, and/or sold in formulations suitable for pulmonary administration. In some embodiments, such administration is via the buccal cavity. In some embodiments, formulations may comprise dry particles comprising active ingredients. In such embodiments, dry particles may have a diameter in the range from about 0.5 nm to about 7 nm or from about 1 nm to about 6 nm. In some embodiments, formulations may be in the form of dry powders for administration using devices comprising dry powder reservoirs to which streams of propellant may be directed to disperse such powder. In some embodiments, self-propelling solvent/powder dispensing containers may be used. In such embodiments, active ingredients may be dissolved and/or suspended in low-boiling propellant in sealed containers. Such powders may comprise particles wherein at least 98% of the particles by weight have diameters greater than 0.5 nm and at least 95% of the particles by number have diameters less than 7 nm. Alternatively, at least 95% of the particles by weight have a diameter greater than 1 nm and at least 90% of the particles by number have a diameter less than 6 nm. Dry powder compositions may include a solid fine powder diluent such as sugar and are conveniently provided in a unit dose form.

Low boiling propellants generally include liquid propellants having a boiling point of below 65 °F at atmospheric pressure. Generally, propellants may constitute 50% to 99.9% (w/w) of the composition, and active ingredient may constitute 0.1% to 20% (w/w) of the composition. Propellants may further comprise additional ingredients such as liquid non-ionic and/or solid anionic surfactant and/or solid diluent (which may have particle sizes of the same order as particles comprising active ingredients).

Pharmaceutical compositions formulated for pulmonary delivery may provide active ingredients in the form of droplets of solution and/or suspension. Such formulations may be prepared, packaged, and/or sold as aqueous and/or dilute alcoholic solutions and/or suspensions, optionally sterile, comprising active ingredients, and may conveniently be administered using any nebulization and/or atomization device. Such formulations may further comprise one or more additional ingredients including, but not limited to, a flavoring agent such as saccharin sodium, a volatile oil, a buffering agent, a surface active agent, and/or a preservative such as methylhydroxybenzoate. Droplets provided by this route of administration may have an average diameter in the range from about 0.1 nm to about 200 nm.

### Intranasal, nasal and buccal administration

In some embodiments, pharmaceutical compositions or effector modules of the present invention may be administered nasally and/or intranasaly. In some embodiments, formulations described herein as being useful for pulmonary delivery may also be useful for intranasal delivery. In some embodiments, formulations for intranasal administration comprise a coarse powder comprising the active ingredient and having an average particle from about 0.2 µm to 500 µm. Such formulations are administered in the manner in which snuff is taken, *i.e*. by rapid inhalation through the nasal passage from a container of the powder held close to the nose.

Formulations suitable for nasal administration may, for example, comprise from about as little as 0.1% (w/w) and as much as 100% (w/w) of active ingredient, and may comprise one or more of the additional ingredients described herein. A pharmaceutical composition may be prepared, packaged, and/or sold in a formulation suitable for buccal administration. Such formulations may, for example, be in the form of tablets and/or lozenges made using conventional methods, and may, for example, 0.1% to 20% (w/w) active ingredient, the balance comprising an orally dissolvable and/or degradable composition and, optionally, one or more of the additional ingredients described herein. Alternately, formulations suitable for buccal administration may comprise powders and/or an aerosolized and/or atomized solutions and/or suspensions comprising active ingredients. Such powdered, aerosolized, and/or aerosolized formulations, when dispersed, may comprise average particle and/or droplet sizes in the range of from about 0.1 nm to about 200 nm, and may further comprise one or more of any additional ingredients described herein.

### Ophthalmic or otic administration

In some embodiments, pharmaceutical compositions or effector modules of the present invention may be prepared, packaged, and/or sold in formulations suitable for ophthalmic and/or otic administration. Such formulations may, for example, be in the form of eye and/or ear drops including, for example, a 0.1/1.0% (w/w) solution and/or suspension of the active ingredient in aqueous and/or oily liquid excipients. Such drops may further comprise buffering agents, salts, and/or one or more other of any additional ingredients described herein. Other ophthalmically-administrable formulations which are useful include those which comprise active ingredients in microcrystalline form and/or in liposomal preparations. Subretinal inserts may also be used as forms of administration.

### Delivery

### Naked delivery

Pharmaceutical compositions or effector modules of the present invention may be delivered to cells, tissues, organs and/or organisms in naked form. As used herein in, the term "naked" refers to pharmaceutical compositions, or effector modules delivered free from agents or modifications which promote transfection or permeability. The naked pharmaceutical compositions or effector modules may be delivered to the cells, tissues, organs and/or organisms using routes of administration known in the art and described herein. In some embodiments, naked delivery may include formulation in a simple buffer such as saline or PBS.

### Formulated delivery

In some embodiments, pharmaceutical compositions or effector modules of the present invention may be formulated, using methods described herein. Formulations may comprise pharmaceutical compositions, or effector modules which may be modified and/or unmodified. Formulations may further include, but are not limited to, cell penetration agents, pharmaceutically acceptable carriers, delivery agents, bioerodible or biocompatible polymers, solvents, and/or sustained-release delivery depots. Formulations of the present invention may be delivered to cells using routes of administration known in the art and described herein.

pharmaceutical compositions, or effector modules may also be formulated for direct delivery to organs or tissues in any of several ways in the art including, but not limited to, direct soaking or bathing, via a catheter, by gels, powder, ointments, creams, gels, lotions, and/or drops, by using substrates such as fabric or biodegradable materials coated or impregnated with compositions, and the like.

### Detectable agents and Labels

The effector modules may be associated with or bound to one or more radioactive agents or detectable agents.

These agents include various organic small molecules, inorganic compounds, nanoparticles, enzymes or enzyme substrates, fluorescent materials, luminescent materials (e.g., luminol), bioluminescent materials (e.g., luciferase, luciferin, and aequorin), chemiluminescent materials, radioactive materials (e.g., ¹⁸F, ⁶⁷Ga, ^{81m}Kr, ⁸²Rb, ¹¹¹In, 1231, ¹³³Xe, ²⁰¹Tl, ¹²⁵I, ³⁵S, ¹⁴C, ³H, or ^{99m}Tc (e.g., as pertechnetate (technetate(VII), TcO₄₋)), and contrast agents (e.g., gold (e.g., gold nanoparticles), gadolinium (e.g., chelated Gd), iron oxides (e.g., superparamagnetic iron oxide (SPIO), monocrystalline iron oxide nanoparticles (MIONs), and ultrasmall superparamagnetic iron oxide (USPIO)), manganese chelates (e.g., Mn-DPDP), barium sulfate, iodinated contrast media (iohexol), microbubbles, or perfluorocarbons). Such optically-detectable labels include for example, without limitation, 4-acetamido-4'-isothiocyanatostilbene-2,2'disulfonic acid; acridine and derivatives (e.g., acridine and acridine isothiocyanate); 5-(2'-aminoethyl)aminonaphthalene-1-sulfonic acid (EDANS); 4-amino-N-[3-vinylsulfonyl)phenyl]naphthalimide-3,5 disulfonate; N-(4-anilino-l-naphthyl)maleimide; anthranilamide; BODIPY; Brilliant Yellow; coumarin and derivatives (e.g., coumarin, 7-amino-4-methylcoumarin (AMC, Coumarin 120), and 7-amino-4-trifluoromethylcoumarin (Coumarin 151)); cyanine dyes; cyanosine; 4',6-diaminidino-2-phenylindole (DAPI); 5' 5"-dibromopyrogallol-sulfonaphthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]-naphthalene-1-sulfonyl chloride (DNS, dansylchloride); 4-dimethylaminophenylazophenyl-4'-isothiocyanate (DABITC); eosin and derivatives (e.g., eosin and eosin isothiocyanate); erythrosin and derivatives (e.g., erythrosin B and erythrosin isothiocyanate); ethidium; fluorescein and derivatives (e.g., 5-carboxyfluorescein (FAM), 5-(4,6-dichlorotriazin-2-yl)aminofluorescein (DTAF), 2',7'-dimethoxy-4'5'-dichloro-6-carboxyfluorescein, fluorescein, fluorescein isothiocyanate, X-rhodamine-5-(and-6)-isothiocyanate (QFITC or XRITC), and fluorescamine); 2-[2-[3-[[1,3-dihydro-1,1-dimethyl-3-(3-sulfopropyl)-2H-benz[e]indol-2-ylidene]ethylidene]-2-[4-(ethoxycarbonyl)-1-piperazinyl]-1-cyclopenten-1-yl]ethenyl]-1,1-dimethyl-3-(3-sulforpropyl)-1H-benz[e]indolium hydroxide, inner salt, compound with n,n-diethylethanamine(1:1) (IR144); 5-chloro-2-[2-[3-[(5-chloro-3-ethyl-2(3H)-benzothiazolylidene)ethylidene]-2-(diphenylamino)-1-cyclopenten-1-yl]ethenyl]-3-ethyl benzothiazolium perchlorate (IR140); Malachite Green isothiocyanate; 4-methylumbelliferone orthocresolphthalein; nitrotyrosine; pararosaniline; Phenol Red; B-phycoerythrin; o-phthaldialdehyde; pyrene and derivatives(e.g., pyrene, pyrene butyrate, and succinimidyl 1-pyrene); butyrate quantum dots; Reactive Red 4 (CIBACRON^{™} Brilliant Red 3B-A); rhodamine and derivatives (e.g., 6-carboxy-X-rhodamine (ROX), 6-carboxyrhodamine (R6G), lissamine rhodamine B sulfonyl chloride rhodarnine (Rhod), rhodamine B, rhodamine 123, rhodamine X isothiocyanate, sulforhodamine B, sulforhodamine 101, sulfonyl chloride derivative of sulforhodamine 101 (Texas Red), N,N,N',N'tetramethyl-6-carboxyrhodamine (TAMRA) tetramethyl rhodamine, and tetramethyl rhodamine isothiocyanate (TRITC)); riboflavin; rosolic acid; terbium chelate derivatives; Cyanine-3 (Cy3); Cyanine-5 (Cy5); cyanine-5.5 (Cy5.5), Cyanine-7 (Cy7); IRD 700; IRD 800; Alexa 647; La Jolta Blue; phthalo cyanine; and naphthalo cyanine.

In some embodiments, the detectable agent may be a non-detectable precursor that becomes detectable upon activation (e.g., fluorogenic tetrazine-fluorophore constructs (e.g., tetrazine-BODIPY FL, tetrazine-Oregon Green 488, or tetrazine-BODIPY TMR-X) or enzyme activatable fluorogenic agents (e.g., PROSENSE^{®} (VisEn Medical))). In vitro assays in which the enzyme labeled compositions can be used include, but are not limited to, enzyme linked immunosorbent assays (ELISAs), immunoprecipitation assays, immunofluorescence, enzyme immunoassays (EIA), radioimmunoassays (RIA), and Western blot analysis.

### VI. DELIVERY MODALITIES AND/OR VECTORS

The effector modules of the present invention may be delivered using one or more modalities. The present invention also provides vectors that package polynucleotides of the invention encoding effector modules, SREs (DDs) and payload constructs, and combinations thereof. Vectors of the present invention may also be used to deliver the packaged polynucleotides to a cell, a local tissue site or a subject. These vectors may be of any kind, including DNA vectors, RNA vectors, plasmids, viral vectors and particles. Viral vector technology is well known and described in Sambrook et al. (2001, Molecular Cloning: A Laboratory Manual, Cold Spring Harbor Laboratory, New York). Viruses, which are useful as vectors include, but are not limited to lentiviral vectors, adenoviral vectors, adeno-associated viral (AAV) vectors, herpes simplex viral vectors, retroviral vectors, oncolytic viruses, and the like.

In general, vectors contain an origin of replication functional in at least one organism, a promoter sequence and convenient restriction endonuclease site, and one or more selectable markers e.g. a drug resistance gene.

As used herein a promoter is defined as a DNA sequence recognized by transcription machinery of the cell, required to initiate specific transcription of the polynucleotide sequence of the present invention. Vectors can comprise native or non-native promoters operably linked to the polynucleotides of the invention. The promoters selected may be strong, weak, constitutive, inducible, tissue specific, development stage-specific, and/or organism specific. One example of a suitable promoter is the immediate early cytomegalovirus (CMV) promoter, comprising the sequence of SEQ ID NO.: 213364. This promoter sequence is a strong constitutive promoter sequence capable of driving high levels of expression of polynucleotide sequence that is operatively linked to it. Another example of a preferred promoter is Elongation Growth Factor-1. Alpha (EF-1. alpha), comprising the sequence of SEQ ID NO.: 213365. Other constitutive promoters may also be used, including, but not limited to simian virus 40 (SV40), mouse mammary tumor virus (MMTV), human immunodeficiency virus (HIV), long terminal repeat (LTR), promoter, an avian leukemia virus promoter, an Epstein-Barr virus immediate early promoter, a Rous sarcoma virus promoter as well as human gene promoters including, but not limited to the phosphoglycerate kinase (PGK) promoter , comprising the sequence of SEQ ID NO.: 213366, actin promoter, the myosin promoter, the hemoglobin promoter, the Ubiquitin C (Ubc) promoter, the human U6 small nuclear protein promoter and the creatine kinase promoter. In some instances, inducible promoters such as but not limited to metallothionine promoter, glucocorticoid promoter, a progesterone promoter, and a tetracycline promoter may be used.

In some embodiments, the optimal promoter may be selected based on its ability to achieve minimal expression of the SREs and payloads of the invention in the absence of the ligand and detectable expression in the presence of the ligand.

Additional promoter elements e.g. enhancers may be used to regulate the frequency of transcriptional initiation. Such regions may be located 10-100 base pairs upstream or downstream of the start site. In some instances, two or more promoter elements may be used to cooperatively or independently activate transcription.

In some embodiments, the recombinant expression vector may comprise regulatory sequences, such as transcription and translation initiation and termination codons, which are specific to the type of host cell into which the vector is to be introduced.

In some embodiments, the vector of the invention may comprise one or more payloads taught herein, wherein the two or more payloads may be included in one effector module. In this case, the two or more payloads are tuned by the same stimulus simultaneously. In other embodiments, the vector of the invention may comprise two or more effector modules, wherein each effector module comprises a different payload. In this case, the two or more effector modules and payloads are tuned by different stimuli, providing separately independent regulation of the two or more components.

### Lentiviral vehicles/particles

In some embodiments, lentiviral vehicles/particles may be used as delivery modalities. Lentiviruses are subgroup of the *Retroviridae* family of viruses, named because reverse transcription of viral RNA genomes to DNA is required before integration into the host genome. As such, the most important features of lentiviral vehicles/particles are the integration of their genetic material into the genome of a target/host cell. Some examples of lentivirus include the Human Immunodeficiency Viruses: HIV-1 and HIV-2, the Simian Immunodeficiency Virus (SIV), feline immunodeficiency virus (FIV), bovine immunodeficiency virus (BIV), Jembrana Disease Virus (JDV), equine infectious anemia virus (EIAV), equine infectious anemia virus, visna-maedi and caprine arthritis encephalitis virus (CAEV).

Typically, lentiviral particles making up the gene delivery vehicle are replication defective on their own (also referred to as "self-inactivating"). Lentiviruses are able to infect both dividing and non-dividing cells by virtue of the entry mechanism through the intact host nuclear envelope (Naldini L et al., Curr. Opin. Biotechnol, 1998, 9: 457-463). Recombinant lentiviral vehicles/particles have been generated by multiply attenuating the HIV virulence genes, for example, the genes Env, Vif, Vpr, Vpu, Nef and Tat are deleted making the vector biologically safe. Correspondingly, lentiviral vehicles, for example, derived from HIV-1/HIV-2 can mediate the efficient delivery, integration and long-term expression of transgenes into non-dividing cells. As used herein, the term "recombinant" refers to a vector or other nucleic acid containing both lentiviral sequences and non-lentiviral retroviral sequences.

Lentiviral particles may be generated by co-expressing the virus packaging elements and the vector genome itself in a producer cell such as human HEK293T cells. These elements are usually provided in three or four separate plasmids. The producer cells are co-transfected with plasmids that encode lentiviral components including the core (i.e. structural proteins) and enzymatic components of the virus, and the envelope protein(s) (referred to as the packaging systems), and a plasmid that encodes the genome including a foreign transgene, to be transferred to the target cell, the vehicle itself (also referred to as the transfer vector). In general, the plasmids or vectors are included in a producer cell line. The plasmids/vectors are introduced via transfection, transduction or infection into the producer cell line. Methods for transfection, transduction or infection are well known by those of skill in the art. As non-limiting example, the packaging and transfer constructs can be introduced into producer cell lines by calcium phosphate transfection, lipofection or electroporation, generally together with a dominant selectable marker, such as neo, DHFR, Gln synthetase or ADA, followed by selection in the presence of the appropriate drug and isolation of clones.

The producer cell produces recombinant viral particles that contain the foreign gene, for example, the effector module of the present invention. The recombinant viral particles are recovered from the culture media and titrated by standard methods used by those of skill in the art. The recombinant lentiviral vehicles can be used to infect target cells.

Cells that can be used to produce high-titer lentiviral particles may include, but are not limited to, HEK293T cells, 293G cells, STAR cells (Relander et al., Mol. Ther., 2005, 11: 452-459), FreeStyle^{™} 293 Expression System (ThermoFisher, Waltham, MA), and other HEK293T-based producer cell lines (e.g., Stewart et al., Hum Gene Ther._2011, 22(3):357-369; Lee et al., Biotechnol Bioeng, 2012, 10996): 1551-1560; Throm et al., Blood. 2009, 113(21): 5104-5110).

In some aspects, the envelope proteins may be heterologous envelop proteins from other viruses, such as the G protein of vesicular stomatitis virus (VSV G) or baculoviral gp64 envelop proteins. The VSV-G glycoprotein may especially be chosen among species classified in the vesiculovirus genus: *Carajas virus* (CJSV), *Chandipura virus* (CHPV), Cocal *virus* (COCV), *Isfahan virus* (ISFV), *Maraba virus* (MARAV), *Piry virus* (PIRYV), *Vesicular stomatitis Alagoas virus* (VSAV), *Vesicular stomatitis Indiana virus* (VSIV) *and Vesicular stomatitis New Jersey virus* (VSNJV) and/or stains provisionally classified in the vesiculovirus genus as *Grass carp rhabdovirus, BeAn* 157575 *virus* (BeAn 157575), *Boteke virus* (BTKV), *Calchaqui virus* (CQIV), *Eel virus American* (EVA), *Gray Lodge virus* (GLOV), *Jurona virus* (JURY), *Klamath virus* (KLAV), *Kwatta virus* (KWAV), *La Joya virus* (LJV), *Malpais Spring virus* (MSPV), *Mount Elgon bat virus* (MEBV), *Perinet virus* (PERV), *Pike fry rhabdovirus* (PFRV), *Porton virus* (PORV), *Radi virus* (RADIV), *Spring viremia of carp virus* (SVCV), *Tupaia virus* (TUPV), *Ulcerative disease rhabdovirus* (UDRV) and *Yug Bogdanovac virus* (YBV). The gp64 or other baculoviral env protein can be derived from *Autographa californica* nucleopolyhedrovirus (AcMNPV), *Anagrapha falcifera* nuclear polyhedrosis virus, *Bombyx mori* nuclear polyhedrosis virus, *Choristoneura fumiferana* nucleopolyhedrovirus, *Orgyia pseudotsugata* single capsid nuclear polyhedrosis virus, *Epiphyas postvittana* nucleopolyhedrovirus, *Hyphantria cunea* nucleopolyhedrovirus, *Galleria mellonella* nuclear polyhedrosis virus, Dhori virus, Thogoto virus, *Antheraea pemyi* nucleopolyhedrovirus or Batken virus.

Other elements provided in lentiviral particles may comprise retroviral LTR (long-terminal repeat) at either 5' or 3' terminus, a retroviral export element, optionally a lentiviral reverse response element (RRE), a promoter or active portion thereof, and a locus control region (LCR) or active portion thereof. The effector module is linked to the vector.

Methods for generating recombinant lentiviral particles are discussed in the art, for example, U.S. Pat. NOs.: 8, 846, 385; 7,745, 179; 7,629,153; 7,575,924; 7,179, 903; and 6, 808, 905.

Lentivirus vectors used may be selected from, but are not limited to pLVX, pLenti, pLenti6, pLJM1, FUGW, pWPXL, pWPI, pLenti CMV puro DEST, pLJM1-EGFP, pULTRA, plnducer20, pHIV-EGFP, pCW57.1, pTRPE, pELPS, pRRL, and pLionll.

Lentiviral vehicles are plasmid-based or virus-based and are known in the art (See, U.S. Pat. NOs. 9, 260, 725; 9,068,199; 9,023,646; 8,900,858; 8,748,169; 8,709,799; 8,420,104; 8,329,462; 8,076,106; 6,013,516; and 5,994,136).

### Adeno-associated viral particles

Delivery of any of the effector modules, SREs or payload constructs of the present invention may be achieved using recombinant adeno-associated viral (rAAV) vectors. Such vectors or viral particles may be designed to utilize any of the known serotype capsids or combinations of serotype capsids. Capsids may include but not limited to AAV1, AAV2, AAV2G9, AAV3, AAV3a, AAV3b, AAV3-3, AAV4, AAV4-4, AAV5, AAV6, AAV6.1, AAV6.2, AAV6.1.2, AAV7, AAV7.2, AAV8, AAV9, AAV9.11, AAV9.13, AAV9.16, AAV9.24, AAV9.45, AAV9.47, AAV9.61, AAV9.68, AAV9.84, AAV9.9, AAV10, AAV11, AAV12, AAV16.3, AAV24.1, AAV27.3, AAV42.12, AAV42-1b, AAV42-2, AAV42-3a, AAV42-3b, AAV42-4, AAV42-5a, AAV42-5b, AAV42-6b, AAV42-8, AAV42-10, AAV42-11, AAV42-12, AAV42-13, AAV42-15, AAV42-aa, AAV43-1, AAV43-12, AAV43-20, AAV43-21, AAV43-23, AAV43-25, AAV43-5, AAV44.1, AAV44.2, AAV44.5, AAV223.1, AAV223.2, AAV223.4, AAV223.5, AAV223.6, AAV223.7, AAV1-7/rh.48, AAV1-8/rh.49, AAV2-15/rh.62, AAV2-3/rh.61, AAV2-4/rh.50, AAV2-5/rh.51, AAV3.1/hu.6, AAV3.1/hu.9, AAV3-9/rh.52, AAV3-11/rh.53, AAV4-8/r11.64, AAV4-9/rh.54, AAV4-19/rh.55, AAV5-3/rh.57, AAV5-22/rh.58, AAV7.3/hu.7, AAV16.8/hu.10, AAV16.12/hu.11, AAV29.3/bb.1, AAV29.5/bb.2, AAV106.1/hu.37, AAV114.3/hu.40, AAV127.2/hu.41, AAV127.5/hu.42, AAV128.3/hu.44, AAV130.4/hu.48, AAV145.1/hu.53, AAV145.5/hu.54, AAV145.6/hu.55, AAV161.10/hu.60, AAV161.6/hu.61, AAV33.12/hu.17, AAV33.4/hu.15, AAV33.8/hu.16, AAV52/hu.19, AAV52.1/hu.20, AAV58.2/hu.25, AAVA3.3, AAVA3.4, AAVA3.5, AAVA3.7, AAVC1, AAVC2, AAVC5, AAV-DJ, AAV-DJ8, AAVF3, AAVF5, AAVH2, AAVH6, AAVLK03, AAVH-1/hu.1, AAVH-5/hu.3, AAVLG-10/rh.40, AAVLG-4/rh.38, AAVLG-9/hu.39, AAVN721-8/rh.43, AAVCh.5, AAVCh.5R1, AAVcy.2, AAVcy.3, AAVcy.4, AAVcy.5, AAVCy.5R1, AAVCy.5R2, AAVCy.5R3, AAVCy.5R4, AAVcy.6, AAVhu.1, AAVhu.2, AAVhu.3, AAVhu.4, AAVhu.5, AAVhu.6, AAVhu.7, AAVhu.9, AAVhu.10, AAVhu.11, AAVhu.13, AAVhu.15, AAVhu.16, AAVhu.17, AAVhu.18, AAVhu.20, AAVhu.21, AAVhu.22, AAVhu.23.2, AAVhu.24, AAVhu.25, AAVhu.27, AAVhu.28, AAVhu.29, AAVhu.29R, AAVhu.31, AAVhu.32, AAVhu.34, AAVhu.35, AAVhu.37, AAVhu.39, AAVhu.40, AAVhu.41, AAVhu.42, AAVhu.43, AAVhu.44, AAVhu.44R1, AAVhu.44R2, AAVhu.44R3, AAVhu.45, AAVhu.46, AAVhu.47, AAVhu.48, AAVhu.48R1, AAVhu.48R2, AAVhu.48R3, AAVhu.49, AAVhu.51, AAVhu.52, AAVhu.54, AAVhu.55, AAVhu.56, AAVhu.57, AAVhu.58, AAVhu.60, AAVhu.61, AAVhu.63, AAVhu.64, AAVhu.66, AAVhu.67, AAVhu.14/9, AAVhu.t 19, AAVrh.2, AAVrh.2R, AAVrh.8, AAVrh.8R, AAVrh.10, AAVrh.12, AAVrh.13, AAVrh.13R, AAVrh.14, AAVrh.17, AAVrh.18, AAVrh.19, AAVrh.20, AAVrh.21, AAVrh.22, AAVrh.23, AAVrh.24, AAVrh.25, AAVrh.31, AAVrh.32, AAVrh.33, AAVrh.34, AAVrh.35, AAVrh.36, AAVrh.37, AAVrh.37R2, AAVrh.38, AAVrh.39, AAVrh.40, AAVrh.46, AAVrh.48, AAVrh.48.1, AAVrh.48.1.2, AAVrh.48.2, AAVrh.49, AAVrh.51, AAVrh.52, AAVrh.53, AAVrh.54, AAVrh.56, AAVrh.57, AAVrh.58, AAVrh.61, AAVrh.64, AAVrh.64R1, AAVrh.64R2, AAVrh.67, AAVrh.73, and/or AAVrh.74.

In one embodiment, the AAV serotype may be or have a sequence as described in United States Publication No. US20030138772, such as, but not limited to, AAV1 (SEQ ID NO: 6 and 64 of US20030138772), AAV2 (SEQ ID NO: 7 and 70 of US20030138772), AAV3 (SEQ ID NO: 8 and 71 of US20030138772), AAV4 (SEQ ID NO: 63 of US20030138772), AAV5 (SEQ ID NO: 114 of US20030138772), AAV6 (SEQ ID NO: 65 of US20030138772), AAV7 (SEQ ID NO: 1-3 of US20030138772), AAV8 (SEQ ID NO: 4 and 95 of US20030138772), AAV9 (SEQ ID NO: 5 and 100 of US20030138772), AAV10 (SEQ ID NO: 117 of US20030138772), AAV11 (SEQ ID NO: 118 of US20030138772), AAV12 (SEQ ID NO: 119 of US20030138772), AAVrh10 (amino acids 1 to 738 of SEQ ID NO: 81 of US20030138772) or variants thereof. Non limiting examples of variants include SEQ ID NOs: 9, 27-45, 47-62, 66-69, 73-81, 84-94, 96, 97, 99, 101-113 of US20030138772.

In one embodiment, the AAV serotype may be or may have a sequence as described in United States Publication No. US20150159173, such as, but not limited to, AAV2 (SEQ ID NO: 7 and 23 of US20150159173), rh20 (SEQ ID NO: 1 of US20150159173), rh32/33 (SEQ ID NO: 2 of US20150159173), rh39 (SEQ ID NO: 3, 20 and 36 of US20150159173), rh46 (SEQ ID NO: 4 and 22 of US20150159173), rh73 (SEQ ID NO: 5 of US20150159173), rh74 (SEQ ID NO: 6 of US20150159173), AAV6.1 (SEQ ID NO: 29 of US20150159173), rh.8 (SEQ ID NO: 41 of US20150159173), rh.48.1 (SEQ ID NO: 44 of US20150159173), hu.44 (SEQ ID NO: 45 of US20150159173), hu.29 (SEQ ID NO: 42 of US20150159173), hu.48 (SEQ ID NO: 38 of US20150159173), rh54 (SEQ ID NO: 49 of US20150159173), AAV2 (SEQ ID NO: 7 of US20150159173), cy.5 (SEQ ID NO: 8 and 24 of US20150159173), rh.10 (SEQ ID NO: 9 and 25 of US20150159173), rh.13 (SEQ ID NO: 10 and 26 of US20150159173), AAV1 (SEQ ID NO: 11 and 27 of US20150159173), AAV3 (SEQ ID NO: 12 and 28 of US20150159173), AAV6 (SEQ ID NO: 13 and 29 of US20150159173), AAV7 (SEQ ID NO: 14 and 30 of US20150159173), AAV8 (SEQ ID NO: 15 and 31 of US20150159173), hu.13 (SEQ ID NO: 16 and 32 of US20150159173), hu.26 (SEQ ID NO: 17 and 33 of US20150159173), hu.37 (SEQ ID NO: 18 and 34 of US20150159173), hu.53 (SEQ ID NO: 19 and 35 of US20150159173), rh.43 (SEQ ID NO: 21 and 37 of US20150159173), rh2 (SEQ ID NO: 39 of US20150159173), rh.37 (SEQ ID NO: 40 of US20150159173), rh.64 (SEQ ID NO: 43 of US20150159173), rh.48 (SEQ ID NO: 44 of US20150159173), ch.5 (SEQ ID NO 46 of US20150159173), rh.67 (SEQ ID NO: 47 of US20150159173), rh.58 (SEQ ID NO: 48 of US20150159173), or variants thereof including, but not limited to Cy5R1, Cy5R2, Cy5R3, Cy5R4, rh.13R, rh.37R2, rh.2R, rh.8R, rh.48.1, rh.48.2, rh.48.1.2, hu.44R1, hu.44R2, hu.44R3, hu.29R, ch.5R1, rh64R1, rh64R2, AAV6.2, AAV6.1, AAV6.12, hu.48R1, hu.48R2, and hu.48R3.

In one embodiment, the AAV serotype may be or have the sequence as described in United States Patent No. US 7,198,951, such as, but not limited to, AAV9 (SEQ ID NO: 1-3 of US 7,198,951), AAV2 (SEQ ID NO: 4 of US 7,198,951), AAV1 (SEQ ID NO: 5 of US 7,198,951), AAV3 (SEQ ID NO: 6 of US 7,198,951), and AAV8 (SEQ ID NO: 7).

In one embodiment, the AAV serotype may be or have a mutation in the AAV9 sequence as described by N Pulicherla et al. (Molcular Therapy 19(6):1070-1078 (2011)), such as but not limited to, AAV9.9, AAV9.11, AAV9.13, AAV9.16, AAV9.24, AAV9.45, AAV9.47, AAV9.61, AAV9.68, AAV9.84.

In one embodiment, the AAV serotype may be or have a sequence as described in United States Patent No. US 6,156,303, such as, but not limited to, AAV3B (SEQ ID NO: 1 and 10 of US 6,156,303), AAV6 (SEQ ID NO: 2, 7 and 11 of US 6,156,303), AAV2 (SEQ ID NO: 3 and 8 of US 6,156,303), AAV3A (SEQ ID NO: 4 and 9, of US 6,156,303), or derivatives thereof.

In one embodiment, the AAV serotype may be or may have a sequence as described in United States Publication No. US20140359799, such as, but not limited to, AAV8 (SEQ ID NO: 1 of US20140359799), AAVDJ (SEQ ID NO: 2 and 3 of US20140359799), or variants thereof.

In one embodiment, the AAV serotype may be or have the sequence of AAV4 as described in International Publication No. WO1998011244, such as, but not limited to AAV4 (SEQ ID NO: 1-20 of WO1998011244).

In one embodiment, the AAV serotype may be or have a mutation in the AAV2 sequence to generate AAV2G9 as described in International Publication No. WO2014144229.

In one embodiment, the AAV serotype may be or have a sequence as described in International Publication WO2005033321, such as, but not limited to AAV1 (SEQ ID NO: 202 and 219 of WO2005033321), AAV2 (SEQ ID NO: 211 and 221 of WO2005033321), AAV3-3 (SEQ ID NO: 200 and 217 of WO2005033321), AAV4-4 (SEQ ID NO: 201 and 218 of WO2005033321), AAV5 (SEQ ID NO: 216 and 199 of WO2005033321), AAV6 (SEQ ID NO: 203 and 220 of WO2005033321), AAV7 (SEQ ID NO: 213 and 222 of WO2005033321), AAV8 (SEQ ID NO: 214 and 223 of WO2005033321), hu.14/AAV9 (SEQ ID NO: 3 and 123 of WO2005033321), hu.17 (SEQ ID NO: 83 of WO2005033321), hu.6 (SEQ ID NO: 84 of WO2005033321), hu.42 (SEQ ID NO: 85 of WO2005033321), rh.38 (SEQ ID NO: 86 of WO2005033321), hu.40 (SEQ ID NO: 87 of WO2005033321), hu.37 (SEQ ID NO: 88 of WO2005033321), rh.40 (SEQ ID NO: 92 of WO2005033321), rh.52 (SEQ ID NO: 96 of WO2005033321), rh.53 (SEQ ID NO: 97 of WO2005033321), rh.49 (SEQ ID NO: 103 of WO2005033321), rh.51 (SEQ ID NO: 104 of WO2005033321), rh.57 (SEQ ID NO: 105 of WO2005033321), rh.58 (SEQ ID NO: 106 of WO2005033321), rh.61 (SEQ ID NO: 107 of WO2005033321), rh.50 (SEQ ID NO: 108 of WO2005033321), rh.43 (SEQ ID NO: 163 of WO2005033321), rh.62 (SEQ ID NO: 114 of WO2005033321), rh.48 (SEQ ID NO: 115 of WO2005033321), 4-9/rh.54 (SEQ ID NO: 116 of WO2005033321), 4-19/rh.55 (SEQ ID NO: 117 of WO2005033321), hu.31 (SEQ ID NO: 121 of WO2005033321), hu.32 (SEQ ID NO: 122 of WO2005033321), hu.34 (SEQ ID NO: 125 of WO2005033321), hu.45 (SEQ ID NO: 127 of WO2005033321), hu.47 (SEQ ID NO: 128 of WO2005033321), hu.13 (SEQ ID NO: 129 of WO2005033321), hu.28 (SEQ ID NO: 130 of WO2005033321), hu.29 (SEQ ID NO: 132 of WO2005033321), hu.19 (SEQ ID NO: 133 of WO2005033321), hu.20 (SEQ ID NO: 134 of WO2005033321), hu.21 (SEQ ID NO: 135 of WO2005033321), hu.23.2 (SEQ ID NO: 137 of WO2005033321), hu.22 (SEQ ID NO: 138 of WO2005033321), hu.27 (SEQ ID NO: 140 of WO2005033321), hu.4 (SEQ ID NO: 141 of WO2005033321), hu.2 (SEQ ID NO: 143 of WO2005033321), hu.1 (SEQ ID NO: 144 of WO2005033321), hu.3 (SEQ ID NO: 145 of WO2005033321), hu.25 (SEQ ID NO: 146 of WO2005033321), hu.15 (SEQ ID NO: 147 of WO2005033321), hu.16 (SEQ ID NO: 148 of WO2005033321), hu.18 (SEQ ID NO: 149 of WO2005033321), hu.7 (SEQ ID NO: 150 of WO2005033321), hu.11 (SEQ ID NO: 153 of WO2005033321), hu.9 (SEQ ID NO: 155 of WO2005033321), hu.10 (SEQ ID NO: 156 of WO2005033321), hu.48 (SEQ ID NO: 157 of WO2005033321), hu.44 (SEQ ID NO: 144 of WO2005033321), hu.46 (SEQ ID NO: 159 of WO2005033321), hu.43 (SEQ ID NO: 160 of WO2005033321), hu.35 (SEQ ID NO: 164 of WO2005033321), hu.24 (SEQ ID NO: 136 of WO2005033321), rh.64 (SEQ ID NO: 99 of WO2005033321), hu.41 (SEQ ID NO: 91 of WO2005033321), hu.39 (SEQ ID NO: 102 of WO2005033321), hu.67 (SEQ ID NO: 198 of WO2005033321), hu.66 (SEQ ID NO: 197 of WO2005033321), hu.51 (SEQ ID NO: 190 of WO2005033321), hu.52 (SEQ ID NO: 191 of WO2005033321), hu.49 (SEQ ID NO: 189 of WO2005033321), hu.56 (SEQ ID NO: 192 of WO2005033321), hu.57 (SEQ ID NO: 193 of WO2005033321), hu.58 (SEQ ID NO: 194 of WO2005033321), hu.63 (SEQ ID NO: 195 of WO2005033321), hu.64 (SEQ ID NO: 196), hu.60 (SEQ ID NO: 184 of WO2005033321), hu.61 (SEQ ID NO: 185 of WO2005033321), hu.53 (SEQ ID NO: 186 of WO2005033321), hu.55 (SEQ ID NO: 187 of WO2005033321), hu.54 (SEQ ID NO: 188 of WO2005033321), hu.6 (SEQ ID NO: 84 of WO2005033321), rh.56 (SEQ ID NO: 152 of WO2005033321), or variants thereof. Non limiting examples of variants include SEQ ID NOs: 1, 2, 4-82, 89, 90, 93-95, 98, 100, 101, 109-113, 118-120, 124, 126, 131, 139, 142, 151,154, 158, 161, 162, 165-183, 202, 204-212, 215, 219, 224-236 of WO2005033321.

AAV vectors include not only single stranded vectors but self-complementary AAV vectors (scAAVs). scAAV vectors contain DNA which anneals together to form double stranded vector genome. By skipping second strand synthesis, scAAVs allow for rapid expression in the cell.

The rAAV vectors may be manufactured by standard methods in the art such as by triple transfection, in sf9 insect cells or in suspension cell cultures of human cells such as HEK293 cells.

The effector modules may be encoded in one or more viral genomes to be packaged in the AAV capsids taught herein.

Such vector or viral genomes may also include, in addition to at least one or two ITRs (inverted terminal repeats), certain regulatory elements necessary for expression from the vector or viral genome. Such regulatory elements are well known in the art and include for example promoters, introns, spacers, stuffer sequences, and the like.

The effector modules of the invention may be administered in one or more AAV particles.

In some embodiments, the effector modules may be administered in one or more AAV particles. In some embodiments, more than one effector module or SRE may be encoded in a viral genome.

### Retroviral vehicles/particles (γ-retroviral vectors)

In some embodiments, retroviral vehicles/particles may be used to deliver the effector modules of the present invention. Retroviral vectors (RVs) allow the permanent integration of a transgene in target cells. In addition to lentiviral vectors based on complex HIV-1/2, retroviral vectors based on simple gamma-retroviruses have been widely used to deliver therapeutic genes and demonstrated clinically as one of the most efficient and powerful gene delivery systems capable of transducing a broad range of cell types. Example species of Gamma retroviruses include the murine leukemia viruses (MLVs) and the feline leukemia viruses (FeLV).

In some embodiments, gamma-retroviral vectors derived from a mammalian gamma-retrovirus such as murine leukemia viruses (MLVs), are recombinant. The MLV families of gamma retroviruses include the ecotropic, amphotropic, xenotropic and polytropic subfamilies. Ecotropic viruses are able to infect only murine cells using mCAT-1 receptor. Examples of ecotropic viruses are Moloney MLV and AKV. Amphotropic viruses infect murine, human and other species through the Pit-2 receptor. One example of an amphotropic virus is the 4070A virus. Xenotropic and polytropic viruses utilize the same (Xpr1) receptor, but differ in their species tropism. Xenotropic viruses such as NZB-9-1 infect human and other species but not murine species, whereas polytropic viruses such as focus-forming viruses (MCF) infect murine, human and other species.

Gamma-retroviral vectors may be produced in packaging cells by co-transfecting the cells with several plasmids including one encoding the retroviral structural and enzymatic (gag-pol) polyprotein, one encoding the envelope (env) protein, and one encoding the vector mRNA comprising polynucleotide encoding the compositions of the present invention that is to be packaged in newly formed viral particles.

In some aspects, the recombinant gamma-retroviral vectors are pseudotyped with envelope proteins from other viruses. Envelope glycoproteins are incorporated in the outer lipid layer of the viral particles which can increase/alter the cell tropism. Exemplary envelop proteins include the gibbon ape leukemia virus envelope protein (GALV) or vesicular stomatitis virus G protein (VSV-G), or Simian endogenous retrovirus envelop protein, or Measles Virus H and F proteins, or Human immunodeficiency virus gp120 envelop protein, or cocal vesiculovirus envelop protein (See, e.g., U.S. application publication NO.: 2012/164118). In other aspects, envelope glycoproteins may be genetically modified to incorporate targeting/binding ligands into gamma-retroviral vectors, binding ligands including, but not limited to, peptide ligands, single chain antibodies and growth factors (Waehler et al., Nat. Rev. Genet. 2007, 8(8):573-587). These engineered glycoproteins can retarget vectors to cells expressing their corresponding target moieties. In other aspects, a "molecular bridge" may be introduced to direct vectors to specific cells. The molecular bridge has dual specificities: one end can recognize viral glycoproteins, and the other end can bind to the molecular determinant on the target cell. Such molecular bridges, for example ligand-receptor, avidin-biotin, and chemical conjugations, monoclonal antibodies and engineered fusogenic proteins, can direct the attachment of viral vectors to target cells for transduction (Yang et al., Biotechnol. Bioeng., 2008, 101(2): 357-368; and Maetzig et al., Viruses, 2011, 3, 677-713).

In some embodiments, the recombinant gamma-retroviral vectors are self-inactivating (SIN) gammaretroviral vectors. The vectors are replication incompetent. SIN vectors may harbor a deletion within the 3' U3 region initially comprising enhancer/promoter activity. Furthermore, the 5' U3 region may be replaced with strong promoters (needed in the packaging cell line) derived from Cytomegalovirus or RSV, or an internal promotor of choice, and/or an enhancer element. The choice of the internal promotors may be made according to specific requirements of gene expression needed for a particular purpose of the invention.

In some embodiments, polynucleotides encoding the effector module are inserted within the recombinant viral genome. The other components of the viral mRNA of a recombinant gamma-retroviral vector may be modified by insertion or removal of naturally occurring sequences (e.g., insertion of an IRES, insertion of a heterologous polynucleotide encoding a polypeptide or inhibitory nucleic acid of interest, shuffling of a more effective promoter from a different retrovirus or virus in place of the wild-type promoter and the like). In some examples, the recombinant gamma-retroviral vectors may comprise modified packaging signal, and/or primer binding site (PBS), and/or 5'-enhancer/promoter elements in the U3-region of the 5'- long terminal repeat (LTR), and/or 3'-SIN elements modified in the U3-region of the 3'-LTR. These modifications may increase the titers and the ability of infection.

Gammaretroviral vectors suitable for delivering effector modules of the present invention may be selected from those disclosed in U.S. Pat. NOs.: 8,828,718; 7,585,676; 7,351,585; U.S. application publication NO.: 2007/048285; PCT application publication NOs.: WO2010/113037; WO2014/121005; WO2015/056014; and EP Pat. NOs.: EP1757702; EP1757703.

### Oncolytic Viral vector

In some embodiments, polynucleotides of present invention may be packaged into oncolytic viruses. As used herein, the term "oncolytic virus" refers to a virus that preferentially infects and kills cancer cells such as vaccine viruses. An oncolytic virus can occur naturally or can be a genetically modified virus such as oncolytic adenovirus, and oncolytic herpes virus.

In some embodiments, oncolytic vaccine viruses may include viral particles of a thymidine kinase (TK)-deficient, granulocyte macrophage (GM)-colony stimulating factor (CSF)-expressing, replication-competent vaccinia virus vector sufficient to induce oncolysis of cells in the tumor; See e.g., US Pat. NO.: 9,226,977.

### Messenger RNA (mRNA)

In some embodiments, the effector modules of the invention may be designed as a messenger RNA (mRNA). As used herein, the term "messenger RNA" (mRNA) refers to any polynucleotide which encodes a polypeptide of interest and which is capable of being translated to produce the encoded polypeptide of interest *in vitro, in vivo, in situ* or *ex vivo.* Such mRNA molecules may have the structural components or features of any of those taught in International Application number PCT/US2013/030062.

Polynucleotides of the invention may also be designed as taught in, for example, Ribostem Limited in United Kingdom patent application serial number 0316089.2 filed on July 9, 2003 now abandoned, PCT application number PCT/GB2004/002981 filed on July 9, 2004 published as WO2005005622, United States patent application national phase entry serial number 10/563,897 filed on June 8, 2006 published as US20060247195 now abandoned, and European patent application national phase entry serial number EP2004743322 filed on July 9, 2004 published as EP1646714 now withdrawn; Novozymes, Inc. in PCT application number PCT/US2007/88060 filed on December 19, 2007 published as WO2008140615, United States patent application national phase entry serial number 12/520,072 filed on July 2, 2009 published as US20100028943 and European patent application national phase entry serial number EP2007874376 filed on July 7, 2009 published as EP2104739; University of Rochester in PCT application number PCT/US2006/46120 filed on December 4, 2006 published as WO2007064952 and United States patent application serial number 11/606,995 filed on December 1, 2006 published as US20070141030; BioNTech AG in European patent application serial number EP2007024312 filed December 14, 2007 now abandoned, PCT application number PCT/EP2008/01059 filed on December 12, 2008 published as WO2009077134, European patent application national phase entry serial number EP2008861423 filed on June 2, 2010 published as EP2240572, United States patent application national phase entry serial number 12/,735,060 filed November 24, 2010 published as US20110065103, German patent application serial number DE 10 2005 046 490 filed September 28, 2005, PCT application PCT/EP2006/0448 filed September 28, 2006 published as WO2007036366, national phase European patent EP1934345 published March, 21, 2012 and national phase US patent application serial number 11/992,638 filed August 14, 2009 published as 20100129877; Immune Disease Institute Inc. in United States patent application serial number 13/088,009 filed April 15, 2011 published as US20120046346 and PCT application PCT/US2011/32679 filed April 15, 2011 published as WO20110130624; Shire Human Genetic Therapeutics in United States patent application serial number 12/957,340 filed on November 20, 2010 published as US20110244026; Sequitur Inc. in PCT application PCT/US1998/019492 filed on September 18, 1998 published as WO1999014346; The Scripps Research Institute in PCT application number PCT/US2010/00567 filed on February 24, 2010 published as WO2010098861, and United States patent application national phase entry serial number 13/203,229 filed November 3, 2011 published as US20120053333; Ludwig-Maximillians University in PCT application number PCT/EP2010/004681 filed on July 30, 2010 published as WO2011012316; Cellscript Inc. in United States patent number 8,039,214 filed June 30, 2008 and granted October 18, 2011, United States patent application serial numbers 12/962,498 filed on December 7, 2010 published as US20110143436, 12/962,468 filed on December 7, 2010 published as US20110143397, 13/237,451 filed on September 20, 2011 published as US20120009649, and PCT applications PCT/US2010/59305 filed December 7, 2010 published as WO2011071931 and PCT/US2010/59317 filed on December 7, 2010 published as WO2011071936; The Trustees of the University of Pennsylvania in PCT application number PCT/US2006/32372 filed on August 21, 2006 published as WO2007024708, and United States patent application national phase entry serial number 11/990,646 filed on March 27, 2009 published as US20090286852; Curevac GMBH in German patent application serial numbers DE10 2001 027 283.9 filed June 5, 2001, DE10 2001 062 480.8 filed December 19, 2001, and DE 20 2006 051 516 filed October 31, 2006 all abandoned, European patent numbers EP1392341 granted March 30, 2005 and EP1458410 granted January 2, 2008, PCT application numbers PCT/EP2002/06180 filed June 5, 2002 published as WO2002098443, PCT/EP2002/14577 filed on December 19, 2002 published as WO2003051401, PCT/EP2007/09469 filed on December 31, 2007 published as WO2008052770, PCT/EP2008/03033 filed on April 16, 2008 published as WO2009127230, PCT/EP2006/004784 filed on May 19, 2005 published as WO2006122828, PCT/EP2008/00081 filed on January 9, 2007 published as WO2008083949, and United States patent application serial numbers 10/729,830 filed on December 5, 2003 published as US20050032730, 10/870,110 filed on June 18, 2004 published as US20050059624, 11/914,945 filed on July 7, 2008 published as US20080267873, 12/446,912 filed on October 27, 2009 published as US2010047261 now abandoned, 12/522,214 filed on January 4, 2010 published as US20100189729, 12/787,566 filed on May 26, 2010 published as US20110077287, 12/787,755 filed on May 26, 2010 published as US20100239608, 13/185,119 filed on July 18, 2011 published as US20110269950, and 13/106,548 filed on May 12, 2011 published as US20110311472.

In some embodiments, the effector modules may be designed as self amplifying RNA. "Self amplifying RNA" as used herein refers to RNA molecules that can replicate in the host resulting in the increase in the amount of the RNA and the protein encoded by the RNA. Such self amplifying RNA may have structural features or components of any of those taught in International Patent Application Publication No. WO2011005799.

### VII. METHODS AND USES

The effector modules of the present invention may be utilized in a large variety of applications including, but not limited to, therapeutics, diagnosis and prognosis, etc.

### VIII. DEFINITIONS

Activity: As used herein, the term "activity" refers to the condition in which things are happening or being done. Compositions of the invention may have activity and this activity may involve one or more biological events. Biological events may include cell signaling events. Biological events may include cell signaling events associated protein interactions with one or more corresponding proteins, receptors, small molecules or any of the biocircuit components described herein.

Administered in combination: As used herein, the term "administered in combination" or "combined administration" refers to simultaneous exposure of one or more subjects to two or more agents administered at the same time or within an interval such that the subject is at some point in time simultaneously exposed to both and/or such that there may be an overlap in the effect of each agent on the patient. In some embodiments, at least one dose of one or more agents is administered within about 24 hours, 12 hours, 6 hours, 3 hours, 1 hour, 30 minutes, 15 minutes, 10 minutes, 5 minutes, or 1 minute of at least one dose of one or more other agents. In some embodiments, administration occurs in overlapping dosage regimens. As used herein, the term "dosage regimen" refers to a plurality of doses spaced apart in time. Such doses may occur at regular intervals or may include one or more hiatus in administration. In some embodiments, the administration of individual doses of one or more compositions of the present invention, as described herein, are spaced sufficiently closely together such that a combinatorial (e.g., a synergistic) effect is achieved.

Animal: As used herein, the term "animal" refers to any member of the animal kingdom. In some embodiments, "animal" refers to humans at any stage of development. In some embodiments, "animal" refers to non-human animals at any stage of development. In certain embodiments, the non-human animal is a mammal (e.g., a rodent, a mouse, a rat, a rabbit, a monkey, a dog, a cat, a sheep, cattle, a primate, or a pig). In some embodiments, animals include, but are not limited to, mammals, birds, reptiles, amphibians, fish, and worms. In some embodiments, the animal is a transgenic animal, genetically-engineered animal, or a clone.

Antigens of interest or desired antigens: As used herein, the terms "antigens of interest" or "desired antigens" refers to those proteins and/or other biomolecules provided herein that are immunospecifically bound or interact with antibodies and/or fragments, mutants, variants, and/or alterations thereof described herein.

Approximately: As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

Associated with: As used herein, the terms "associated with," "conjugated," "linked," "attached," and "tethered," when used with respect to two or more moieties, mean that the moieties are physically associated or connected with one another, either directly or via one or more additional moieties that serve as linking agents, to form a structure that is sufficiently stable so that the moieties remain physically associated under the conditions in which the structure is used, e.g., physiological conditions. An "association" need not be strictly through direct covalent chemical bonding. It may also suggest ionic or hydrogen bonding or a hybridization based connectivity sufficiently stable such that the "associated" entities remain physically associated.

Biomolecule: As used herein, the term "biomolecule" is any natural molecule which is amino acid-based, nucleic acid-based, carbohydrate-based or lipid-based, and the like.

Biologically active: As used herein, the phrase "biologically active" refers to a characteristic of any substance that has activity in a biological system and/or organism. For instance, a substance that, when administered to an organism, has a biological effect on that organism, is considered to be biologically active. Compounds and/or compositions may be considered biologically active if even a portion of is biologically active or mimics an activity considered to biologically relevant.

Biological system: As used herein, the term "biological system" refers to a group of organs, tissues, cells, intracellular components, proteins, nucleic acids, molecules (including, but not limited to biomolecules) that function together to perform a certain biological task within cellular membranes, cellular compartments, cells, tissues, organs, organ systems, multicellular organisms, or any biological entity. Biological systems may be cell signaling pathways comprising intracellular and/or extracellular cell signaling biomolecules.

Candidate antibody: As used herein, the term "candidate antibody" refers to an antibody from a pool of one or more antibody from which one or more desired antibodies may be selected.

Cellular matrix: As used herein, the term "cellular matrix" refers to the biochemical and structural environment associated with the outer portion of the cell membrane. Such cell membranes may also include platelet membranes. Components of the cellular matrix may include, but are not limited to proteoglycans, carbohydrate molecules, integral membrane proteins, glycolipids and the like.

Compound: As used herein, the term "compound," refers to a distinct chemical entity. The term may be used herein to refer to peptides, proteins, protein complexes, nucleic acids, polynucleotides of the invention. Those of skill in the art appreciate that some compounds exist in different such forms, show different properties and/or activities (including, but not limited to biological activities). In such cases it is within the ordinary skill of those in the art to select or avoid particular forms of the compound for use in accordance with the present invention. For example, compounds that contain asymmetrically substituted carbon atoms can be isolated in optically active or racemic forms. Methods on how to prepare optically active forms from optically active starting materials are known in the art, such as by resolution of racemic mixtures or by stereoselective synthesis.

Conserved: As used herein, the term "conserved" refers to nucleotides or amino acid residues of polynucleotide or polypeptide sequences, respectively, that are those that occur unaltered in the same position of two or more sequences being compared. Nucleotides or amino acids that are relatively conserved are those that are conserved among more related sequences than nucleotides or amino acids appearing elsewhere in the sequences.

In some embodiments, two or more sequences are said to be "completely conserved" if they are 100% identical to one another. In some embodiments, two or more sequences are said to be "highly conserved" if they are at least 70% identical, at least 80% identical, at least 90% identical, or at least 95% identical to one another. In some embodiments, two or more sequences are said to be "highly conserved" if they are about 70% identical, about 80% identical, about 90% identical, about 95%, about 98%, or about 99% identical to one another. In some embodiments, two or more sequences are said to be "conserved" if they are at least 30% identical, at least 40% identical, at least 50% identical, at least 60% identical, at least 70% identical, at least 80% identical, at least 90% identical, or at least 95% identical to one another. In some embodiments, two or more sequences are said to be "conserved" if they are about 30% identical, about 40% identical, about 50% identical, about 60% identical, about 70% identical, about 80% identical, about 90% identical, about 95% identical, about 98% identical, or about 99% identical to one another. Conservation of sequence may apply to the entire length of an oligonucleotide or polypeptide or may apply to a portion, region or feature thereof.

In one embodiment, conserved sequences are not contiguous. Those skilled in the art are able to appreciate how to achieve alignment when gaps in contiguous alignment are present between sequences, and to align corresponding residues not withstanding insertions or deletions present.

CRISPR-Cas system: As used herein, the term "CRISPR-Cas system" in general refers collectively to components/elements involved in directing the activity of CRISPR-associated ("Cas") proteins, including sequences encoding a Cas gene, a tracr (trans-activating CRISPR) sequence (e.g. tracrRNA or an active partial tracrRNA), a tracr-complementary sequence (encompassing a "direct repeat" and a tracrRNA-processed partial direct repeat in the context of an endogenous CRISPR system), a target (guide) sequence (also referred to as a "spacer" in the context of an endogenous CRISPR system), or other sequences and transcripts from a CRISPR locus.

CRISPR interference (CRISPRi): As used herein, the term "CRISPRi" refers to a genetic perturbation technique that allows for sequence-specific repression or activation of gene expression in prokaryotic and eukaryotic cells using CRISPR complexes. CRISPRi regulates gene expression primarily on the transcriptional level.

Delivery: As used herein, "delivery" refers to the act or manner of delivering a compound, substance, entity, moiety, cargo or payload.

Delivery Agent: As used herein, "delivery agent" refers to any agent which facilitates, at least in part, the in vivo delivery of one or more substances (including, but not limited to compounds and/or compositions of the present invention) to a cell, subject or other biological system cells.

Desired antibody: As used herein, the term "desired antibody" refers to an antibody that is sought after, in some cases from a pool of candidate antibodies.

Destabilized: As used herein, the term "destable," "destabilize," "destabilizing region", or "destabilizing domain" means a region or molecule that is less stable than a starting, reference, wild-type or native form of the same region or molecule.

Detectable label: As used herein, "detectable label" refers to one or more markers, signals, or moieties which are attached, incorporated or associated with another entity, which markers, signals or moieties are readily detected by methods known in the art including radiography, fluorescence, chemiluminescence, enzymatic activity, absorbance, immunological detection and the like. Detectable labels may include radioisotopes, fluorophores, chromophores, enzymes, dyes, metal ions, ligands, biotin, avidin, streptavidin and haptens, quantum dots, polyhistidine tags, myc tags, flag tags, human influenza hemagglutinin (HA) tags and the like. Detectable labels may be located at any position in the entity with which they are attached, incorporated or associated. For example, when attached, incorporated in or associated with a peptide or protein, they may be within the amino acids, the peptides, or proteins, or located at the N- or C- termini.

Distal: As used herein, the term "distal" means situated away from the center or away from a point or region of interest.

Engineered: As used herein, embodiments of the invention are "engineered" when they are designed to have a feature or property, whether structural or chemical, that varies from a starting point, wild type or native molecule.

Epitope: As used herein, an "epitope" refers to a surface or region on a molecule that is capable of interacting with components of another molecule. In some embodiments, when referring to a protein or protein module, an epitope may comprise a linear stretch of amino acids or a three dimensional structure formed by folded amino acid chains.

Expression: As used herein, "expression" of a nucleic acid sequence refers to one or more of the following events: (1) production of an RNA template from a DNA sequence (e.g., by transcription); (2) processing of an RNA transcript (e.g., by splicing, editing, 5' cap formation, and/or 3' end processing); (3) translation of an RNA into a polypeptide or protein; (4) folding of a polypeptide or protein; and (5) post-translational modification of a polypeptide or protein.

Extracellular matrix: As used herein, the term, "extracellular matrix," or "ECM" refers to the area surrounding cells and/or the area between cells that typically comprises structural proteins as well as cell signaling molecules. Components of the extracellular matrix may include, but are not limited to proteins, nucleic acids, membranes, lipids and sugars that may be directly or indirectly associated with structural components of the extracellular environments. Structural components of the extracellular matrix may include, but are not limited to proteins, polysaccharides (e.g. hyaluronic acid,) glycosaminoglycans and proteoglycans (e.g. heparin sulfate, chondroitin sulfate and keratin sulfate.) Such structural components may include, but are not limited to fibrous components (e.g. collagens and elastins,) fibrillins, fibronectin, laminins, agrin, perlecan, decorin and the like.

*Ex Vivo:* As used herein, the term *"ex vivo"* refers to events that occur outside an organism (e.g., animal, plant, or microbe or cell or tissue thereof).

Feature: As used herein, a "feature" refers to a characteristic, a property, or a distinctive element.

Formulation: As used herein, a "formulation" includes at least a compound and/or composition of the present invention and a delivery agent.

Fragment: A "fragment," as used herein, refers to a portion. For example, fragments of proteins may comprise polypeptides obtained by digesting full-length protein. In some embodiments, a fragment of a protein includes at least 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 150, 200, 250 or more amino acids. In some embodiments, fragments of an antibody include portions of an antibody.

Functional: As used herein, a "functional" biological molecule is a biological entity with a structure and in a form in which it exhibits a property and/or activity by which it is characterized.

Guide RNA (gRNA): As used herein, the term "guide RNA" refers to a RNA molecule used in conjunction with a CRISPR associated system. The guide RNA may be composed of two RNA molecules, i.e., one RNA ("crRNA") which hybridizes to a target sequence and provides sequence specificity, and one RNA, the "tracrRNA", which is capable of hybridizing to the crRNA and forming a duplex with crRNA upon hybridization. As used herein, the term "tracrRNA" refers to the endogenous bacterial RNA that links the crRNA to the Cas9 nuclease and can bind any crRNA.

Homology: As used herein, the term "homology" refers to the overall relatedness between polymeric molecules, e.g. between nucleic acid molecules (e.g. DNA molecules and/or RNA molecules) and/or between polypeptide molecules. In some embodiments, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% identical or similar. The term "homologous" necessarily refers to a comparison between at least two sequences (polynucleotide or polypeptide sequences). In accordance with the invention, two polynucleotide sequences are considered to be homologous if the polypeptides they encode are at least about 50%, 60%, 70%, 80%, 90%, 95%, or even 99% for at least one stretch of at least about 20 amino acids. In some embodiments, homologous polynucleotide sequences are characterized by the ability to encode a stretch of at least 4-5 uniquely specified amino acids. For polynucleotide sequences less than 60 nucleotides in length, homology is typically determined by the ability to encode a stretch of at least 4-5 uniquely specified amino acids. In accordance with the invention, two protein sequences are considered to be homologous if the proteins are at least about 50%, 60%, 70%, 80%, or 90% identical for at least one stretch of at least about 20 amino acids. In many embodiments, homologous protein may show a large overall degree of homology and a high degree of homology over at least one short stretch of at least 3, 4, 5, 6, 7, 8, 9, 10, 11, 12, 13, 14, 15, 16, 17, 18, 19, 20, 25, 30, 35, 40, 45, 50 or more amino acids. In many embodiments, homologous proteins share one or more characteristic sequence elements. As used herein, the term "characteristic sequence element" refers to a motif present in related proteins. In some embodiments, the presence of such motifs correlates with a particular activity (such as biological activity).

Identity: As used herein, the term "identity" refers to the overall relatedness between polymeric molecules, e.g., between oligonucleotide molecules (e.g. DNA molecules and/or RNA molecules) and/or between polypeptide molecules. Calculation of the percent identity of two polynucleotide sequences, for example, may be performed by aligning the two sequences for optimal comparison purposes (e.g., gaps can be introduced in one or both of a first and a second nucleic acid sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using methods such as those described in Computational Molecular Biology, Lesk, A. M., ed., Oxford University Press, New York, 1988; Biocomputing: Informatics and Genome Projects, Smith, D. W., ed., Academic Press, New York, 1993; Sequence Analysis in Molecular Biology, von Heinje, G., Academic Press, 1987; Computer Analysis of Sequence Data, Part I, Griffin, A. M., and Griffin, H. G., eds., Humana Press, New Jersey, 1994; and Sequence Analysis Primer, Gribskov, M. and Devereux, J., eds., M Stockton Press, New York, 1991. For example, the percent identity between two nucleotide sequences can be determined, for example using the algorithm of Meyers and Miller (CABIOS, 1989, 4:11-17), which has been incorporated into the ALIGN program (version 2.0) using a PAM120 weight residue table, a gap length penalty of 12 and a gap penalty of 4. The percent identity between two nucleotide sequences can, alternatively, be determined using the GAP program in the GCG software package using an NWSgapdna.CMP matrix. Methods commonly employed to determine percent identity between sequences include, but are not limited to those disclosed in Carillo, H., and Lipman, D., SIAM J Applied Math., 48:1073 (1988). Techniques for determining identity are codified in publicly available computer programs. Exemplary computer software to determine homology between two sequences include, but are not limited to, GCG program package, Devereux, J., et al., Nucleic Acids Research, 12(1), 387 (1984)), BLASTP, BLASTN, and FASTA Altschul, S. F. et al., J. Molec. Biol., 215, 403 (1990)).

Inhibit expression of a gene: As used herein, the phrase "inhibit expression of a gene" means to cause a reduction in the amount of an expression product of the gene. The expression product may be RNA transcribed from the gene (e.g. mRNA) or a polypeptide translated from mRNA transcribed from the gene. Typically, a reduction in the level of mRNA results in a reduction in the level of a polypeptide translated therefrom. The level of expression may be determined using standard techniques for measuring mRNA or protein.

*In situ*: As used herein, the term *"in situ"* refers to events that occur in the original, natural, or existing environment e.g. within an organism.

*In vitro*: As used herein, the term *"in vitro"* refers to events that occur in an artificial environment, e.g., in a test tube or reaction vessel, in cell culture, in a Petri dish, etc., rather than within an organism (e.g., animal, plant, or microbe).

*In vivo*: As used herein, the term *"in vivo"* refers to events that occur within an organism (e.g., animal, plant, or microbe or cell or tissue thereof).

Isolated: As used herein, the term "isolated" is synonymous with "separated", but carries with it the inference separation was carried out by the hand of man. In one embodiment, an isolated substance or entity is one that has been separated from at least some of the components with which it was previously associated (whether in nature or in an experimental setting). Isolated substances may have varying levels of purity in reference to the substances from which they have been associated. Isolated substances and/or entities may be separated from at least about 10%, about 20%, about 30%, about 40%, about 50%, about 60%, about 70%, about 80%, about 90%, or more of the other components with which they were initially associated. In some embodiments, isolated agents are more than about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components.

Substantially isolated: By "substantially isolated" is meant that the compound is substantially separated from the environment in which it was formed or detected. Partial separation can include, for example, a composition enriched in the compound of the present disclosure. Substantial separation can include compositions containing at least about 50%, at least about 60%, at least about 70%, at least about 80%, at least about 90%, at least about 95%, at least about 97%, or at least about 99% by weight of the compound of the present disclosure, or salt thereof. Methods for isolating compounds and their salts are routine in the art. In some embodiments, isolation of a substance or entity includes disruption of chemical associations and/or bonds. In some embodiments, isolation includes only the separation from components with which the isolated substance or entity was previously combined and does not include such disruption.

Linker: As used herein, a linker refers to a moiety that connects two or more domains, moieties or entities. In one embodiment, a linker may comprise 10 or more atoms. In a further embodiment, a linker may comprise a group of atoms, e.g., 10-1,000 atoms, and can be comprised of the atoms or groups such as, but not limited to, carbon, amino, alkylamino, oxygen, sulfur, sulfoxide, sulfonyl, carbonyl, and imine. In some embodiments, a linker may comprise one or more nucleic acids comprising one or more nucleotides. In some embodiments, the linker may comprise an amino acid, peptide, polypeptide or protein. In some embodiments, a moiety bound by a linker may include, but is not limited to an atom, a chemical group, a nucleoside, a nucleotide, a nucleobase, a sugar, a nucleic acid, an amino acid, a peptide, a polypeptide, a protein, a protein complex, a payload (e.g., a therapeutic agent). or a marker (including, but not limited to a chemical, fluorescent, radioactive or bioluminescent marker). The linker can be used for any useful purpose, such as to form multimers or conjugates, as well as to administer a payload, as described herein. Examples of chemical groups that can be incorporated into the linker include, but are not limited to, alkyl, alkenyl, alkynyl, amido, amino, ether, thioether, ester, alkylene, heteroalkylene, aryl, or heterocyclyl, each of which can be optionally substituted, as described herein. Examples of linkers include, but are not limited to, unsaturated alkanes, polyethylene glycols (e.g., ethylene or propylene glycol monomeric units, e.g., diethylene glycol, dipropylene glycol, triethylene glycol, tripropylene glycol, tetraethylene glycol, or tetraethylene glycol), and dextran polymers, Other examples include, but are not limited to, cleavable moieties within the linker, such as, for example, a disulfide bond (-S-S-) or an azo bond (-N=N-), which can be cleaved using a reducing agent or photolysis. Non-limiting examples of a selectively cleavable bonds include an amido bond which may be cleaved for example by the use of tris(2-carboxyethyl)phosphine (TCEP), or other reducing agents, and/or photolysis, as well as an ester bond which may be cleaved for example by acidic or basic hydrolysis.

Modified: As used herein, the term "modified" refers to a changed state or structure of a molecule or entity as compared with a parent or reference molecule or entity. Molecules may be modified in many ways including chemically, structurally, and functionally. In some embodiments, compounds and/or compositions of the present invention are modified by the introduction of non-natural amino acids.

Mutation: As used herein, the term "mutation" refers to a change and/or alteration. In some embodiments, mutations may be changes and/or alterations to proteins (including peptides and polypeptides) and/or nucleic acids (including polynucleic acids). In some embodiments, mutations comprise changes and/or alterations to a protein and/or nucleic acid sequence. Such changes and/or alterations may comprise the addition, substitution and or deletion of one or more amino acids (in the case of proteins and/or peptides) and/or nucleotides (in the case of nucleic acids and or polynucleic acids). In embodiments wherein mutations comprise the addition and/or substitution of amino acids and/or nucleotides, such additions and/or substitutions may comprise 1 or more amino acid and/or nucleotide residues and may include modified amino acids and/or nucleotides.

Naturally occurring: As used herein, "naturally occurring" means existing in nature without artificial aid, or involvement of the hand of man.

Niche: As used herein, the term "niche" refers to a place, zone and/or habitat. As used herein, the term "cell niche" refers to a unique set of physiologic conditions in a cellular system within a tissue, organ or organ system within or derived from a mammalian organism. A cell niche may occur in vivo, in vitro, ex vivo, or in situ. Given the complex nature and the dynamic processes involved in growth factor signaling, a cell niche may be characterized functionally, spatially or temporally or may be used to refer to any environment that encompasses one or more cells.

Non-human vertebrate: As used herein, a "non-human vertebrate" includes all vertebrates except Homo sapiens, including wild and domesticated species. Examples of non-human vertebrates include, but are not limited to, mammals, such as alpaca, banteng, bison, camel, cat, cattle, deer, dog, donkey, gayal, goat, guinea pig, horse, llama, mule, pig, rabbit, reindeer, sheep water buffalo, and yak.

Off-target: As used herein, "off target" refers to any unintended effect on any one or more target, gene and/or cellular transcript.

Operably linked: As used herein, the phrase "operably linked" refers to a functional connection between two or more molecules, constructs, transcripts, entities, moieties or the like.

Paratope: As used herein, a "paratope" refers to the antigen-binding site of an antibody.

Passive adsorption: As used herein, "passive adsorption" refers to a method of immobilizing solid-phase reactants on one or more surfaces (e.g. membranes, dishes, culture dishes, assay plates, etc.) Immobilization typically occurs due to affinity between such reactants and surface components.

Patient: As used herein, "patient" refers to a subject who may seek or be in need of treatment, requires treatment, is receiving treatment, will receive treatment, or a subject who is under care by a trained (e.g., licensed) professional for a particular disease or condition.

Peptide: As used herein, the term "peptide" refers to a chain of amino acids that is less than or equal to about 50 amino acids long, e.g., about 5, 10, 15, 20, 25, 30, 35, 40, 45, or 50 amino acids long.

Pharmaceutically acceptable: The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

Pharmaceutically acceptable excipients: As used herein, the term "pharmaceutically acceptable excipient," as used herein, refers to any ingredient other than active agents (e.g., as described herein) present in pharmaceutical compositions and having the properties of being substantially nontoxic and non-inflammatory in subjects. In some embodiments, pharmaceutically acceptable excipients are vehicles capable of suspending and/or dissolving active agents. Excipients may include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspending or dispersing agents, sweeteners, and waters of hydration. Exemplary excipients include, but are not limited to: butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E, vitamin C, and xylitol.

Pharmaceutically acceptable salts: Pharmaceutically acceptable salts of the compounds described herein are forms of the disclosed compounds wherein the acid or base moiety is in its salt form (e.g., as generated by reacting a free base group with a suitable organic acid). Examples of pharmaceutically acceptable salts include, but are not limited to, mineral or organic acid salts of basic residues such as amines; alkali or organic salts of acidic residues such as carboxylic acids; and the like. Representative acid addition salts include acetate, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Pharmaceutically acceptable salts include the conventional non-toxic salts, for example, from non-toxic inorganic or organic acids. In some embodiments a pharmaceutically acceptable salt is prepared from a parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418, Pharmaceutical Salts: Properties, Selection, and Use, P.H. Stahl and C.G. Wermuth (eds.), Wiley-VCH, 2008, and Berge et al., Journal of Pharmaceutical Science, 66, 1-19 (1977). Pharmaceutically acceptable solvate: The term "pharmaceutically acceptable solvate," as used herein, refers to a crystalline form of a compound wherein molecules of a suitable solvent are incorporated in the crystal lattice. For example, solvates may be prepared by crystallization, recrystallization, or precipitation from a solution that includes organic solvents, water, or a mixture thereof. Examples of suitable solvents are ethanol, water (for example, mono-, di-, and tri-hydrates), N-methylpyrrolidinone (NMP), dimethyl sulfoxide (DMSO), N, N'-dimethylformamide (DMF), N, N'-dimethylacetamide (DMAC), 1,3-dimethyl-2-imidazolidinone (DMEU), 1,3-dimethyl-3,4,5,6-tetrahydro-2-(1H)-pyrimidinone (DMPU), acetonitrile (ACN), propylene glycol, ethyl acetate, benzyl alcohol, 2-pyrrolidone, benzyl benzoate, and the like. When water is the solvent, the solvate is referred to as a "hydrate." In some embodiments, the solvent incorporated into a solvate is of a type or at a level that is physiologically tolerable to an organism to which the solvate is administered (e.g., in a unit dosage form of a pharmaceutical composition).

Pharmacokinetic: As used herein, "pharmacokinetic" refers to any one or more properties of a molecule or compound as it relates to the determination of the fate of substances administered to living organisms. Pharmacokinetics are divided into several areas including the extent and rate of absorption, distribution, metabolism and excretion. This is commonly referred to as ADME where: (A) Absorption is the process of a substance entering the blood circulation; (D) Distribution is the dispersion or dissemination of substances throughout the fluids and tissues of the body; (M) Metabolism (or Biotransformation) is the irreversible transformation of parent compounds into daughter metabolites; and (E) Excretion (or Elimination) refers to the elimination of the substances from the body. In rare cases, some drugs irreversibly accumulate in body tissue.

Physicochemical: As used herein, "physicochemical" means of or relating to a physical and/or chemical property.

Preventing: As used herein, the term "preventing" refers to partially or completely delaying onset of an infection, disease, disorder and/or condition; partially or completely delaying onset of one or more symptoms, features, or clinical manifestations of a particular infection, disease, disorder, and/or condition; partially or completely delaying onset of one or more symptoms, features, or manifestations of a particular infection, disease, disorder, and/or condition; partially or completely delaying progression from an infection, a particular disease, disorder and/or condition; and/or decreasing the risk of developing pathology associated with the infection, the disease, disorder, and/or condition.

Prodrug: The present disclosure also includes prodrugs of the compounds described herein. As used herein, "prodrugs" refer to any substance, molecule or entity which is in a form predicate for that substance, molecule or entity to act as a therapeutic upon chemical or physical alteration. Prodrugs may be covalently bonded or sequestered in some way until converted into the active drug moiety prior to, upon or after administration to a mammalian subject. Prodrugs can be prepared by modifying functional groups present in the compounds in such a way that the modifications are cleaved, either in routine manipulation or in vivo, to the parent compounds. Prodrugs include, among other things, compounds wherein hydroxyl, amino, sulfhydryl, or carboxyl groups are bonded to any group that, when administered to a mammalian subject, cleaves to form a free hydroxyl, amino, sulfhydryl, or carboxyl group respectively. Preparation and use of prodrugs is discussed in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

Proliferate: As used herein, the term "proliferate" means to grow, expand, replicate or increase or cause to grow, expand, replicate or increase. "Proliferative" means having the ability to proliferate. "Anti-proliferative" means having properties counter to or in opposition to proliferative properties.

Protein of interest: As used herein, the terms "proteins of interest" or "desired proteins" include those provided herein and fragments, mutants, variants, and alterations thereof.

Proximal: As used herein, the term "proximal" means situated nearer to the center or to a point or region of interest.

Purified: As used herein, the term "purify" means to make substantially pure or clear from unwanted components, material defilement, admixture or imperfection. "Purified" refers to the state of being pure. "Purification" refers to the process of making pure.

Region: As used herein, the term "region" refers to a zone or general area. In some embodiments, when referring to a protein or protein module, a region may comprise a linear sequence of amino acids along the protein or protein module or may comprise a three dimensional area, an epitope and/or a cluster of epitopes. In some embodiments, regions comprise terminal regions. As used herein, the term "terminal region" refers to regions located at the ends or termini of a given agent. When referring to proteins, terminal regions may comprise N- and/or C-termini. N-termini refer to the end of a protein comprising an amino acid with a free amino group. C-termini refer to the end of a protein comprising an amino acid with a free carboxyl group. N- and/or C-terminal regions may there for comprise the N- and/or C-termini as well as surrounding amino acids. In some embodiments, N- and/or C-terminal regions comprise from about 3 amino acids to about 30 amino acids, from about 5 amino acids to about 40 amino acids, from about 10 amino acids to about 50 amino acids, from about 20 amino acids to about 100 amino acids and/or at least 100 amino acids. In some embodiments, N-terminal regions may comprise any length of amino acids that includes the N-terminus, but does not include the C-terminus. In some embodiments, C-terminal regions may comprise any length of amino acids, that include the C-terminus, but do not comprise the N-terminus.

Region of antibody recognition: As used herein, the term "region of antibody recognition" refers to one or more regions on one or more antigens or between two or more antigens that are specifically recognized and bound by corresponding antibodies. Regions of antibody recognition may comprise 1, 2, 3, 4, 5, 6, 7, 8, 9 or at least 10 amino acid residues. Regions of antibody recognition may comprise a junction between two proteins or between two domains of the same protein that are in close proximity to one another.

Sample: As used herein, the term "sample" refers to an aliquot or portion taken from a source and/or provided for analysis or processing. In some embodiments, a sample is from a biological source such as a tissue, cell or component part (e.g. a body fluid, including but not limited to blood, mucus, lymphatic fluid, synovial fluid, cerebrospinal fluid, saliva, amniotic fluid, amniotic cord blood, urine, vaginal fluid and semen). In some embodiments, a sample may be or comprise a homogenate, lysate or extract prepared from a whole organism or a subset of its tissues, cells or component parts, or a fraction or portion thereof, including but not limited to, for example, plasma, serum, spinal fluid, lymph fluid, the external sections of the skin, respiratory, intestinal, and genitourinary tracts, tears, saliva, milk, blood cells, tumors, organs. In some embodiments, a sample is or comprises a medium, such as a nutrient broth or gel, which may contain cellular components, such as proteins or nucleic acid molecule. In some embodiments, a "primary" sample is an aliquot of the source. In some embodiments, a primary sample is subjected to one or more processing (e.g., separation, purification, etc.) steps to prepare a sample for analysis or other use.

Signal Sequences: As used herein, the phrase "signal sequences" refers to a sequence which can direct the transport or localization of a protein.

Single unit dose: As used herein, a "single unit dose" is a dose of any therapeutic administered in one dose/at one time/single route/single point of contact, i.e., single administration event. In some embodiments, a single unit dose is provided as a discrete dosage form (e.g., a tablet, capsule, patch, loaded syringe, vial, etc.).

Similarity: As used herein, the term "similarity" refers to the overall relatedness between polymeric molecules, e.g. between polynucleotide molecules (e.g. DNA molecules and/or RNA molecules) and/or between polypeptide molecules. Calculation of percent similarity of polymeric molecules to one another can be performed in the same manner as a calculation of percent identity, except that calculation of percent similarity takes into account conservative substitutions as is understood in the art.

Split dose: As used herein, a "split dose" is the division of single unit dose or total daily dose into two or more doses.

Stable: As used herein "stable" refers to a compound or entity that is sufficiently robust to survive isolation to a useful degree of purity from a reaction mixture, and preferably capable of formulation into an efficacious therapeutic agent.

Stabilized: As used herein, the term "stabilize", "stabilized," "stabilized region" means to make or become stable. In some embodiments, stability is measured relative to an absolute value. In some embodiments, stability is measured relative to a secondary status or state or to a reference compound or entity.

Stimulus response element (SRE): the term "stimulus response element (SRE), as used herein, is a component of an effector module which is joined, attached, linked to or associated with one or more payloads of the effector module and in some instances is responsible for the responsive nature of the effector module to one or more stimuli. As used herein, the "responsive" nature of an SRE to a stimulus may be characterized by a covalent or non-covalent interaction, a direct or indirect association or a structural or chemical reaction to the stimulus. Further, the response of any SRE to a stimulus may be a matter of degree or kind. The response may be a partial response. The response may be a reversible response. The response may ultimately lead to a regulated signal or output. Such output signal may be of a relative nature to the stimulus, e.g., producing a modulatory effect of between 1 and 100 or a factored increase or decrease such as 2-fold, 3-fold, 4-fold, 5-fold, 10-fold or more. One non-limiting example of an SRE is a destabilizing domain (DD).

Subject: As used herein, the term "subject" or "patient" refers to any organism to which a composition in accordance with the invention may be administered, e.g., for experimental, diagnostic, prophylactic, and/or therapeutic purposes. Typical subjects include animals (e.g., mammals such as mice, rats, rabbits, non-human primates, and humans) and/or plants.

Substantially: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

Substantially equal: As used herein as it relates to time differences between doses, the term means plus/minus 2%.

Substantially simultaneously: As used herein and as it relates to plurality of doses, the term typically means within about 2 seconds.

Suffering from: An individual who is "suffering from" a disease, disorder, and/or condition has been diagnosed with or displays one or more symptoms of a disease, disorder, and/or condition.

Susceptible to: An individual who is "susceptible to" a disease, disorder, and/or condition has not been diagnosed with and/or may not exhibit symptoms of the disease, disorder, and/or condition but harbors a propensity to develop a disease or its symptoms. In some embodiments, an individual who is susceptible to a disease, disorder, and/or condition (for example, cancer) may be characterized by one or more of the following: (1) a genetic mutation associated with development of the disease, disorder, and/or condition; (2) a genetic polymorphism associated with development of the disease, disorder, and/or condition; (3) increased and/or decreased expression and/or activity of a protein and/or nucleic acid associated with the disease, disorder, and/or condition; (4) habits and/or lifestyles associated with development of the disease, disorder, and/or condition; (5) a family history of the disease, disorder, and/or condition; and (6) exposure to and/or infection with a microbe associated with development of the disease, disorder, and/or condition. In some embodiments, an individual who is susceptible to a disease, disorder, and/or condition will develop the disease, disorder, and/or condition. In some embodiments, an individual who is susceptible to a disease, disorder, and/or condition will not develop the disease, disorder, and/or condition.

Synthetic: The term "synthetic" means produced, prepared, and/or manufactured by the hand of man. Synthesis of polynucleotides or polypeptides or other molecules of the present invention may be chemical or enzymatic.

Targeted Cells: As used herein, "targeted cells" refers to any one or more cells of interest. The cells may be found in vitro, in vivo, ex vivo, in situ or in the tissue or organ of an organism. The organism may be an animal, preferably a mammal, more preferably a human and most preferably a patient.

Target site: The term "target site" as used herein, refers to a region or area targeted by a given compound, composition or method of the invention. Target sites may include, but are not limited to cells, tissues, organs, organ systems, niches and the like.

Therapeutic Agent: The term "therapeutic agent" refers to any agent that, when administered to a subject, has a therapeutic, diagnostic, and/or prophylactic effect and/or elicits a desired biological and/or pharmacological effect. Therapeutic agents of the present invention include any of the biocircuit components taught herein either alone or in combination with other therapeutic agents.

Therapeutically effective amount: As used herein, the term "therapeutically effective amount" means an amount of an agent to be delivered (e.g., nucleic acid, drug, therapeutic agent, diagnostic agent, prophylactic agent, etc.) that is sufficient, when administered to a subject suffering from or susceptible to an infection, disease, disorder, and/or condition, to treat, improve symptoms of, diagnose, prevent, and/or delay the onset of the infection, disease, disorder, and/or condition. In some embodiments, a therapeutically effective amount is provided in a single dose. In some embodiments, a therapeutically effective amount is administered in a dosage regimen comprising a plurality of doses. Those skilled in the art will appreciate that in some embodiments, a unit dosage form may be considered to comprise a therapeutically effective amount of a particular agent or entity if it comprises an amount that is effective when administered as part of such a dosage regimen.

Therapeutically effective outcome: As used herein, the term "therapeutically effective outcome" means an outcome that is sufficient in a subject suffering from or susceptible to an infection, disease, disorder, and/or condition, to treat, improve symptoms of, diagnose, prevent, and/or delay the onset of the infection, disease, disorder, and/or condition.

Total daily dose: As used herein, a "total daily dose" is an amount given or prescribed in a 24 hr period. It may be administered as a single unit dose.

Treating: As used herein, the term "treating" refers to partially or completely alleviating, ameliorating, improving, relieving, delaying onset of, inhibiting progression of, reducing severity of, and/or reducing incidence of one or more symptoms or features of a particular infection, disease, disorder, and/or condition. For example, "treating" cancer may refer to inhibiting survival, growth, and/or spread of a tumor. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition.

*Tune:* As used herein, the term "tune" means to adjust, balance or adapt one thing in response to a stimulus or toward a particular outcome. In one non-limiting example, the SREs and/or DDs, balance or adapt the function or structure of compositions to which they are appended, attached or associated with in response to particular stimuli and/or environments.

Unmodified: As used herein, "unmodified" refers to any substance, compound or molecule prior to being changed in any way. Unmodified may, but does not always, refer to the wild type or native form of a biomolecule or entity. Molecules or entities may undergo a series of modifications whereby each modified product may serve as the "unmodified" starting or reference molecule or entity for a subsequent modification.

### EXAMPLES

### Reference Example 1. Screening method to identify ligand responsive SREs or DDs

### Study design

To engineer constructs that display ligand dependent stability, a candidate ligand binding domain (LBD) is selected and a cell-based screen using yellow fluorescent protein (YFP) as a reporter for protein stability is designed to identify mutants of the candidate LBD possessing the desired characteristics of a destabilizing domain: low protein levels in the absence of a ligand of the LBD, (i.e., low basal stability), large dynamic range, robust and predictable dose-response behavior, and rapid kinetics of degradation (Banaszynski, et al., (2006) Cell; 126(5): 995-1004). The candidate LBD binds to a desired ligand but not endogenous signaling molecules.

The candidate LBD sequence (as a template) is first mutated using a combination of nucleotide analog mutagenesis and error-prone PCR, to generate libraries of mutants based on the template candidate domain sequence. The libraries generated are cloned in-frame at either the 5'- or 3'-ends of the YFP gene, and a retroviral expression system is used to stably transduce the libraries of YFP fusions into NIH3T3 fibroblasts.

### Screening Strategy I

The transduced NIH3T3 cells are subjected to three to four rounds of sorting using fluorescence-activated cell sorting (FACS) to screen the libraries of candidate DDs. Transduced NIH3T3 cells are cultured in the absence of the high affinity ligand of the ligand binding domain (LBD), and cells that exhibit low levels of YFP expression are selected through FACS. The selected cell population is cultured in the presence of the high affinity ligand of the ligand binding domain for a period of time (e.g., 24 hours), at which point cells are sorted again by FACS. Cells that exhibit high levels of YFP expression are selected through FACS and the selected cell population is split into two groups and treated again with the high affinity ligand of the ligand binding domain at different concentrations; one group treated with the lower concentration of the ligand and the other treated with a high concentration of the ligand, for a period of time (e.g., 24 hours), at which point cells are sorted again by FACS. Cells expressing mutants that are responsive to lower concentrations of the ligand are isolated.

The isolated cells responsible to the lower concentration of the ligand are treated with the ligand again and cells exhibiting low fluorescence levels are collected 4 hours following removal of the ligand from the media. This fourth sorting is designed to enrich cells that exhibit fast kinetics of degradation (Iwamoto et al., Chem Biol. 2010 Sep 24; 17(9): 981-988).

The collected cells after four rounds of sorting are recovered. The identified candidate cells are harvested and the genomic DNA is extracted. The candidate DDs are amplified by PCR and isolated. The candidate DDs are sequenced and compared to the LBD template to identify the actual mutations in candidate DDs.

### Screening Strategy II

The selected cell population is subject to additional one or more sorts by FACS in the absence of high affinity ligand of LBD and cells that exhibit low levels of YFP expression are selected for further analysis. Cells are treated with high affinity ligand of the ligand binding domain, for a period of time (e.g. 24 hours), and sorted again by FACS. Cells expressing high levels of YFP are selected for through FACS. Cells with high expression of YFP are treated with ligand again and cells exhibiting low fluorescence levels are collected 4 hours following removal of the ligand from the media to enrich cells that exhibit fast kinetics of degradation. Any of the sorting steps may be repeated to identify DDs with ligand dependent stability.

### Reference Example 2. E. coli DHFR (ecDHFR)-TMP driven assays

The ecDHFR plus trimethoprim system may be used to screen and or identify useful constructs. It was first described in 2010 (Iwamoto et al., Chem Biol. 2010; 17(9):981-8).

Briefly, using this system, mutants of E. coli dihydrofolate reductase (ecDHFR) protein are engineered to be degraded when expressed in mammalian cells. When a destabilizing domain (DD) is fused to a protein of interest, its instability is conferred to the fused protein. Trimethoprim (TMP) is a high-affinity ligand for ecDHFR that stabilizes fusion proteins in a dose-dependent manner. The ability of TMP to cross the blood-brain barrier (BBB) enables the tunable regulation of proteins expressed in cells found within the mammalian central nervous system. The ecDHFR-TMP DD system can work in parallel to the existing FKBP/Shld1-based DD system, allowing simultaneous regulation of two proteins independently.

### Reference Example 3. Regulation of protein expression in mammalian cells and/or systems

### Regulation of protein expression in mammalian cells

Standard molecular cloning techniques are used to generate a fusion of the signal response element (SRE), e.g., a destabilization domain (DD) and the payload of interest (POI) into a retroviral plasmid, generating a SRE-POI construct. An epitope tag, FLAG-tag is appended to the protein of interest. The plasmids are packaged into viral particles following standard viral infection protocol. Harvested viral particles proceed immediately to creation of cell lines to test regulation of the POI expression in response to the ligand; or are frozen at -80 °C for future use.

To create a mammalian cell line containing the integrated SRE-POI transgene, NIH3T3 cells are plated and 3mL filtered viral supernatant is added to the culture media. After four hours of infection, the viral media is replaced with 3T3 culture media. 48 hours after infection, cells with stable integration of the SRE-POI construct are selected by FACS and a fluorescent marker.

To test for ligand-dependent control of protein stability, equal numbers of cells with stable integration of the SRE-POI construct are plated in two cell culture plates; a high affinity ligand of the SRE is added in one plate and the other one is added with an equal volume of ethanol as a control. After incubation for 4 to 24 hours, the POI stability is assayed using either flow cytometry or western blotting.

A dose-response experiment using varying concentrations of the ligand and a time course assay is performed to test the ligand-dependent protein expression levels.

### Regulation of protein expression in living animals (mice)

The SRE-POI construct and cells with stable integration of the SRE-POI construct are created following the same procedure as discussed above. A tumor xenograft is used as a mechanism to delivery transgene in mice (Banaszynski et al., Nat. Med., 2008, 14:1123-1127).

*Day 1*: Culture cells containing a stably integrated SRE-POI transgene to 80% confluence and count number of cells. Adjust the number of cells to implant per animal depending on the growth of the cell line in animals.

*Day 2*: Trypsinize, quench with complete media, and spin cells. Wash cells three times with PBS, and resuspend cells in 100 µL (10,000 cells per µL) of DMEM (no FBS) per animal. Xenograft cells subcutaneously (or at desired location) into mice anesthetized with isoflurane (2%).

After transplanted cells form stable grafts, treatment of the ligand of the SRE begins. The ligand is reconstituted in an injectable solution (e.g., 9:1 PEG400: Tween 80) at various concentrations, and injected intravenously to a test animal. A control animal is injected with the injection vehicle only.

*Day 1 after the injection*: The expression level of the POI is analyzed for testing experimental SRE-POI stability. For direct protein measurement, tumors are removed and the tumor tissue amount is standardized for tumor samples from each test animal. Tumor tissues are homogenized and assayed for protein levels *via* ELISA or immunoblotting using antibodies specific to the POI.

Mice are dosed with the ligand every 48 hours. SRE-POI stabilization is periodically analyzed for the phenotypic and/or functional effects of protein stabilization. For example, the tumor xenograft regression/size is measured as functional effects of POI stabilization dependent on the ligand treatment.

### Reference Example 4. DD regulated IL2 expression

FKBP (L106P) and ecDHFR (R12Y, Y100I) are well-characterized destabilizing domains which can confer instability to fusion partners (e.g., a POI). The instability is reversed by a synthetic ligand named Shield-1 that binds to FKBP; TMP that binds to DHFR. An IL2 polypeptide was linked to either FKBP (L106P) or ecDHFR (R12Y, Y100I). IL2 constructs were cloned into pLVX-IRES-Puro lentiviral vectors. An IL2 signal sequence was inserted at the N terminus of the construct.

To evaluate dependence of IL2 levels on Shield-1 dose, HCT116 cells were plated onto a 96-well plate and treated with varying concentrations of Shield-1. Media was then collected from cells and IL2 levels were quantified using IL2 ELISA (FIG. 20). IL2 increased with increase in Shield-1 concentration and plateaued at higher Shield-1 dose levels. The EC₅₀ of Shield-1 was determined to be 50 nM

### Reference Example 5. DD regulated IL12 expression

Several FKBP (DD)-IL12 and DHFR (DD)-IL12 constructs (as shown in Table 18) were cloned into pLVX-IRES-Puro lentiviral vectors. FKBP(DD) is positioned at either N-terminus (Construct ID # OT-IL12-001, OT-IL12-002 and OT-IL12-003) or C-terminus (OT-IL12-004, OT-IL12-005 and OT-IL12-009) of the fusion construct. ecDHFR (R12Y, Y100I) is located at the N-terminal end of the fusion construct (OT-IL12-007). A p40 signal sequence was inserted next to the DD or IL-12. In several constructs, a furin protease cleavage site or a modified furin site was included.

HEK293T cells were transiently transfected with 200 ng or 1µg FKBP-IL12 plasmids (OT-IL12-001, OT-IL12-002, OT-IL12-003, OT-IL12-004, and OT-IL12-005), and treated with 10µM Shield-1 or left untreated for 6 hours. Culture media was collected from transfected cells and diluted 1:50 to measure IL12 levels using p40 ELISA. Average IL12 ELISA readings are presented in Table 11.

**Table 11. IL12 induction after transient transfection**

| **Construct ID** | **10µM Shield-1** | **No Shield-1** |
|---|---|---|
| OT-IL 12-001 | 1748.95 | 1289.61 |
| OT-IL12-002 | 50.73 | 18.01 |
| OT-IL12-003 | 2138.25 | 1762.55 |
| OT-IL12-004 | 1567.62 | 385.95 |
| OT-IL12-005 | 2670.80 | 1188.42 |
| HEK293T | -22.04 | -12.92 |

Treatment with Shield-1 resulted in a significant increase in IL12 over untreated with OT-IL12-004, and OT-IL12-005 constructs. OT-IL12-001, and OT-IL12-003 showed only modest increase in IL12 levels following Shield-1 treatment.

IL12 levels were also measured in cells following stable transfection. 500,000 cells stably transduced with OT-IL12-004 were plated in a 12 well plate and incubated overnight in growth media consisting of Dulbecco's Modified Eagle medium (DMEM) containing 10% fetal bovine serum (FBS). The next day the cells were treated with 1µM Shield-1 or left untreated for 6 or 24 hours. Following treatment with Shield-1, growth media was collected from the cells and diluted 10, 40, 160 or 640 fold and FKBP-IL12 levels were quantified using IL12-p40 ELISA assay. Average IL12 ELISA readings are presented in Table 12.

**Table 12. IL12 induction after stable transfection**

| **Media dilution (fold)** | **6 hours** | | **24 hours** | |
|---|---|---|---|---|
| | **1µM Shield-1** | **No Shield-1** | **1µM Shield-1** | **No Shield-1** |
| 10 | 0.58 | 0.17 | 1.33 | 0.28 |
| 40 | 0.26 | 0.10 | 0.79 | 0.12 |
| 160 | 0.12 | 0.08 | 0.31 | 0.09 |
| 640 | 0.08 | 0.09 | 0.12 | 0.08 |

IL12 induction following both 6 and 24 hours of Shield-1 treatment was most prominent at the lowest dilution factor of 10. A mild induction of IL12 induction was also observed at media dilution factor of 40.

To evaluate Shield-1 dependent FKBP-IL12 induction over time, 2 million cells were plated in growth medium and incubated overnight in the presence of 1µM Shield-1 or left untreated. Cells were then incubated for an additional 2-72 hours and growth media was collected for the cells at all time points. Growth media was diluted 400 fold and IL12 levels were measured using IL12 p40 ELISA. Average IL12 ELISA readings are presented in Table 13.

**Table 13. IL12 induction over time**

| **Time (hrs)** | **1µM Shield-1** | **No Shield-1** |
|---|---|---|
| 2 | 0.1774 | 0.12615 |
| 4 | 0.2567 | 0.1359 |
| 6 | 0.29085 | 0.12655 |
| 8 | 0.2752 | 0.1385 |
| 24 | 0.99475 | 0.1819 |
| 48 | 1.78525 | 0.23145 |
| 72 | 1.6288 | 0.25955 |

IL12 expression in Shield-1 treated cells was higher than untreated following 24, 48 and 72 hours after Shield-1 treatment.

To evaluate the dependence of FKBP-IL12 production on Shield-1 dose levels, OT-IL12-004 transduced HEK293T cells were plated at different densities (40,000 cells, 20,000 cells, 10,000 cells or 5,000 cells per well) onto a 96-well plate. Following overnight incubation, cells were treated with growth medium containing 0 to 10µM Shield-1 for 24 hours. Media was then collected, diluted 400 fold and FKBP-IL12 levels were measured using IL12-p40 ELISA. Average IL12 ELISA readings are presented in Table 14.

**Table 14. Dose and cell number dependent IL12 induction**

| **Shield-1 (µM)** | **40000 cells/well** | **20000 cells/well** | **10000 cells/well** | **5000 cells/well** |
|---|---|---|---|---|
| 10.00 | 623.77 | 656.70 | 214.11 | 193.62 |
| 3.33 | 670.64 | 618.10 | 273.74 | 207.55 |
| 1.11 | 677.27 | 872.24 | 322.56 | 203.71 |
| 0.37 | 368.17 | 582.71 | 250.49 | 172.50 |
| 0.12 | 197.29 | 343.34 | 156.98 | 95.92 |
| 0.04 | 171.50 | 205.68 | 63.79 | 48.89 |
| 0.01 | 117.25 | 103.56 | 13.30 | -2.35 |
| 0.00 | 66.34 | 60.58 | 2.11 | -8.53 |
| 0.00 | 100.43 | 39.55 | -13.58 | -21.76 |
| 0.00 | 83.49 | 7.92 | -21.76 | -26.97 |

A dose dependent IL12 induction was observed at all cell numbers tested.IL12 induction increased with Shield-1 up to a dose of 1µM; following which IL12 induction plateaued. Notably, greater IL12 induction was observed at 2000 and 4000 cells/well.

### Reference Example 6. FKBP and ecDHFR regulated IL12 mediated functions

HEK-Blue sensor cells (InvivoGen, San Diego, CA) were utilized to evaluate whether DD regulated IL12 is capable of regulating signaling downstream of IL12. In these cells, the IL12 receptor, STAT4 and downstream transcriptional elements are tied to a reporter gene such that IL12 signaling can be monitored. To evaluate production of functional IL12, one million HEK 293T were transfected with 200ng of OT-IL-12-003 plasmid using Lipofectamine 2000 (Thermo Fisher Scientific, Waltham, MA). 48 hours after transfection, cells were treated with growth media containing 10µM Shield-1, incubated for another 24 hours, following which, media was collected. 50,000 HEK 293 Blue sensor cells were plated onto 96 well plates and incubated overnight with media (at different dilutions) from Shield-1 treated OT-IL12-003 expressing HEK293T cells. After overnight incubation, 20 µl media was removed from each well and incubated with 180 µl Quanti-Blue reagent (InvivoGen, San Diego, CA) for 30 minutes at 37∘C. Absorption was measured at 620 nm using a spectrophotometer. To generate a standard curve, 180 µl Quanti-Blue reagent was mixed with 20 µl of recombinant IL12 at following concentrations 500, 250, 125, 62.5, 31.25, 15.62, 7.8 and 3.9 pg/ml. Functional IL12 concentrations were determined by comparing the optical density of each sample with IL12 standard curve. Measurable levels of functional IL12 were reached with 640 fold dilutions of IL12 containing growth media and further plateaued at higher concentrations of the media (FIG. 21A).

The dependence of functional IL12 production on the dose of Shield-1 used was also evaluated. 10,000 HEK293T cells stably transduced with OT-IL-12-004 were plated onto 96 well plated and treated with growth media containing 10, 3.33, 1.11, 0.37, 0.12, 0.04, 0.01, 0.005, 0.002 or 0 µM Shield-1 for 24 hours. Following Shield-1 treatment, media from cells was diluted 200 fold and 20µL of the diluted media was added to HEK Blue sensor cells. After overnight incubation, 20 µl of media was removed from each well and incubated with 180 µl Quanti-Blue reagent (InvivoGen, San Diego, CA) for 30 minutes at 37∘C. Absorption was measured at 620 nm using a spectrophotometer. To generate a standard curve, 180 µl Quanti-Blue reagent was mixed with 20 µl of recombinant IL12 at following concentrations 500, 250, 125, 62.5, 31.25, 15.62, 7.8 and 3.9 pg/ml. Functional IL12 concentrations were determined by comparing the optical density of each sample with IL12 standard curve. A dose dependent increase in the levels of functional IL12 levels was observed (FIG. 21B).

### Reference Example 7. DD regulated luciferase

DD regulated luciferase can be used to track cells *in vivo* e.g. T cells. Firefly luciferase or Renilla luciferase may be utilized as the payload. HCT-116 cells were stably transduced with the constitutive (OT-RLuc-001) or DD regulated constructs (OT-RLuc-002, OT-RLuc-003, OT-RLuc-004, OT-RLuc-005 and OT-RLuc-006). Cells were treated with 1µM Shield-1, or 10µM Trimethoprim or vehicle control for 24 hours and luciferase expression and activity was measured. Luciferase expression was measured via western blotting using Anti-Renilla luciferase and anti- Firefly luciferase antibodies (Abcam, Cambridge, UK). Blots were also probed with anti-GAPDH antibody to ensure even protein loading in all samples. As expected, the constitutive luciferase construct (OT-RLuc-001) showed expression of Renilla luciferase both in the presence and absence of ligand. In contrast, OT-RLuc-003 showed strong Shield-1 dependent stabilization of Renilla luciferase. OT-RLuc-004, 005 and 006 showed modest stabilization of Renilla luciferase in the presence of their corresponding ligand, while OT-FLuc-002 showed modest stabilization of firefly luciferase with the addition of Shield-1 (FIG. 22A).

Ligand dependent activity of Renilla and firefly luciferase constructs was also measured in using coelentrazine and luciferin substrates respectively. Cells were treated with1µM Shield-1, or 10µM Trimethoprim or vehicle control for 24 hours, lysed with assay lysis buffer and incubated with the luciferase substrate. Luciferase activity was measured as luminescence reading using a luminometer and the values were compared to control comprising of lysis buffer and substrate. In this assay, all DD regulated showed ligand dependent increase in luciferase activity compared to control. As expected, the constitutive construct OT-RLuc-001 showed high luciferase activity both in the presence and absence of ligand (FIG. 22B).

### Reference Example 8. Regulation of glucagon expression in mammalian cells and/or systems for the treatment of hypoglycemia

Hypoglycemia refers to a condition when the blood glucose levels are below normal. Blood glucose levels can be restored by administering glucagon. To generate biocircuits for the treatment of hypoglycemia, SREs fused to the payload of interest, glucagon, are cloned into retroviral vectors using standard molecular cloning techniques. Optional furin cleavage sites are included in the SREs to induce secretion of glucagon in non-secretory cells. NIH3T3 cells are transfected with retroviral vectors. Transfected cells are treated with high affinity ligand of the SRE and the glucagon expression is measured in the media using western blotting. Changes in glucose levels in the media is measured using mass spectrophotometry. An increase in glucagon protein levels and a concomitant increase in glucose levels in the media indicates that the ligand is able to stabilize the construct thereby allowing glucagon expression.

### Reference Example 9. SRE-based Cas9 expression systems

CRISPR/Cas9 technology has been widely used to generate heritable genomic changes. However, constitutive expression of Cas9 can result in toxicity and off-target effects. These limitations may be overcome by generating a regulatable Cas9 expression system where the Cas9 is fused to an SRE. Lentiviral vectors consisting of a first vector where a single guide RNA (sgRNA) targeting a gene of interest is driven by a constitutive promoter, and a second vector containing Cas9 fused at its N-terminal with a ligand-responsive SRE under the control of a suitable promoter are generated (see FIG. 19A and FIG. 19B). The constructs are then transduced into target cells such as cancer cells and embryonic stem cells. The expression levels of Cas9 and target gene of interest are measured in the presence or absence of varying doses of the ligand specific to the SRE, using western blot and RT-PCR. Cas9 expression is expected to be below detection levels in untreated cells and cells are expected to show a ligand dose-dependent increase in Cas9 expression. In contrast, the expression levels of target gene of interest are expected to be high in the untreated cells and below detection levels in ligand treated cells.

### Reference Example 10. Ex vivo expansion of stem cells for engraftment using SRE regulated expansion factors

To generate biocircuits for the expansion of stem cells, SREs are fused to the payload of interest, a stem cell expansion factor such as GM-CSF, IL-3, Il-6, SCF, FL, or TPO. SRE fusion constructs are cloned into retroviral vectors and transduced into freshly isolated CD34⁺ hematopoietic stem cells. Cells are then treated with high affinity ligand of the SRE and the stem cell expansion factor expression is measured using western blotting. An increase in expansion factor protein level indicates that the ligand is able to stabilize the construct thereby allowing its expression. Growth of the transduced hematopoietic stem cells over time is also monitored to test the functionality of the construct.

### Reference Example 11. Regulation of microbiome using SREs or DDs

Microorganisms, e.g. bacteria used in microbiome therapy may be programmed to die at a specific time, after the delivery of gene or genes, and/or after the host has experienced the therapeutic effect. Standard molecular cloning techniques are used to generate a fusion of the signal response element (SRE), e.g., a destabilization domain (DD) and the payload of interest (POI), a bacterial toxin gene into an appropriate bacterial vector, generating a SRE-toxin construct. To facilitate monitoring of *in vivo* colonization by transformed bacteria, a detectable label, luciferase is also appended to the construct. Bacteria are transformed with the SRE-POI construct following standard transformation protocol and successfully transformed clones are sequence verified.

Colony forming unit (CFU) cell viability assays are used to measure ligand dependent protein stabilization and functionality of the SRE-toxin construct. Two overnight cultures are grown under survival conditions i.e. in the absence of ligand, following which a high affinity ligand of the SRE is added to one plate and the other plate is treated with an appropriate vehicle control. Samples are collected every two hours, serially diluted and spotted onto agar plates with appropriate survival signals. CFU and survival ratios are calculated.

To test functionality of SRE-toxin construct *in vivo,* mice are administered bacteria expressing SRE-toxin by oral gavage for up to 1 week. After successful colonization of the bacteria has been confirmed using bioluminescence imaging, treatment with the ligand of the SRE begins. Test group mice are injected with various concentrations of the ligand, while a control group is injected with the appropriate vehicle control. Ligand dependent expression of the toxin in the test group is expected to result in the death of colonized bacteria as measured by the loss of bioluminescence following ligand injection.

### Reference Example 12. Regulatable expression of biomolecules in the liver

The liver may be exploited as a biofactory for the production of hepatic and non-hepatic proteins such as coagulation factors, insulin, growth hormones, cytokines, and enzymes. To generate regulatable biofactory, liver tropic adeno-associated virus vectors (rAAVs) such as rAAV2 and rAAV8 are engineered with SRE fused to payload of interest e.g. immunomodulatory cytokine, IL10. rAAV vector formulations are injected into mice via the tail vein. The number of injections and the dose of rAAV formulation delivered is optimized to the payload of interest. A few days after the delivery of the rAAV, mice are injected with the ligand specific to the SRE or appropriate vehicle control. Serum is isolated from the mice and IL10 levels are measured. IL10 levels are expected to be high in ligand treated mice and below detection in in vehicle control treated mice.

### Reference Example 13. Regulation of ammonia metabolizing enzymes by SRE based-ammonia biosensors

Ammonia is a key nitrogen source and a metabolic intermediate. Yet, excess ammonia can be detrimental to cell growth, especially in artificial growth environments such as bioreactors. SREs that utilize ammonia as the ligand and contain payloads that are capable of metabolizing ammonia may be useful in mitigating the negative consequences of ammonia. To engineer constructs that display ammonia dependent stability, a candidate ammonia binding domain is selected and a screened as described in Example 1 to identify a binding domain with the desired characteristics of a destabilizing domain. An ammonia metabolizing enzyme such as Carbamoyl Phosphate Synthetase 1 is selected as the Payload of interest (POI). Next, standard molecular cloning techniques are used to generate a fusion of the signal response element (SRE), e.g., ammonia sensitive-destabilization domain and the POI. Constructs are packaged into lentiviral vectors and NIH3T3 cells are transfected with lentiviral vectors. Transfected cells are treated with ammonium and the POI expression is measured using western blotting. Changes in intracellular ammonia and related metabolites is measure using mass spectrophotometry. An increase in POI protein levels in the and a concomitant increase in intracellular levels of ammonia metabolism products indicates that ammonia is able to stabilize the construct thereby allowing its expression.

### Reference Example 14. Regulation of protein expression in transgenic animals (mice)

Standard molecular cloning techniques are used to generate a fusion of the signal response element (SRE), e.g., a destabilization domain (DD) and the payload of interest (POI) into a retroviral plasmid, generating a SRE-POI construct. The SRE-POI constructs are then microinjected into fertilized embryos and transplanted into pseudo pregnant female mice. Resulting offspring are genotyped to identify mice carrying transgene and mice positive for the transgene. To test the expression level of the SRE-POI transgene, mice are dosed with the ligand and POI protein stabilization is analyzed. Functional effects of POI stabilization dependent on the ligand treatment are also evaluated

### Reference Example 15. DD regulated IL15

To test ligand dependent IL15 production, 1 million HEK-293T cells were plated in a 6-well plate in growth media containing DMEM and 10% FBS and incubated overnight at 37∘C at 5% CO2. Cells were then transfected with 100ng of OT-IL15-001(constitutive) or OT-IL15-002 (ecDHFR-IL15) using Lipofectamine 2000 and incubated for 48 hrs. Following the incubation, media was exchanged for growth medium containing 10µM Trimethoprim or vehicle control and further incubated for 24 hrs. Media was collected and the undiluted media samples or media samples diluted 4, 16, 256, 1024, 4096 or 16384-fold were tested using human IL-15 ELISA. Average IL15 ELISA readings are presented in Table 15.

**Table 15. DD-IL15 induction**

| **Media dilution (fold)** | **Vehicle** | **10 µM TMP** |
|---|---|---|
| 1 | 0.396 | 0.820 |
| 4 | 0.154 | 0.287 |
| 16 | 0.074 | 0.116 |
| 64 | 0.056 | 0.073 |
| 256 | 0.053 | 0.057 |
| 1024 | 0.053 | 0.048 |
| 4096 | 0.049 | 0.049 |
| 16384 | 0.050 | 0.049 |

The 64-fold, 16-fold, 4-fold diluted, and undiluted media samples showed IL15 levels greater than vehicle control, suggesting Trimethoprim dependent stabilization of IL15 at these dilutions.

### Example 16. DD regulated CD19 chimeric antigen receptor

A CD19 CAR fusion polypeptide was linked to either FKBP-DD or ecDHFR -DD and the constructs were cloned into pLVX-IRES-Puro vector. FKBP, and ecDHFR were positioned either between the CD19 scFv and the CD8αhinge (OT-CD19C-002, OT-CD19C-003), between the CD8αhinge and the transmembrane domain (OT-CD19C-004, OT-CD19C-005) or at the C terminus of the construct (OT-CD19C-006 and OT-CD19C-007). A constitutively expressed CAR construct, OT-CD19C-001 was used as a positive control.

To test ligand dependent expression of DD-CD19 CAR constructs, 1 million HEK 293T cells were cultured in growth medium containing DMEM and 10% FBS and transfected with CAR constructs using Lipofectamine 2000. 48 hours after transfection, cells were treated with 1µM or10µM Shield-1, 10µM Trimethoprim, 1µM Methotrexate, or vehicle control and incubated for 24 hours. Cells were harvested, lysed and immunoblotted for CD3 Zeta, a component of the CAR, using anti-CD247 (BD Pharmingen, Franklin Lanes, NJ) and Alexa 555-conjugated-goat-anti mouse antibody (red) (Li-Cor, Lincoln, NE). Lysates were also immunoblotted for Actin and probed with Alexa 488-conjugated secondary antibody (green) to confirm uniform protein loading in all the samples. Compared to the untreated control, OT-CD19C-002 and OT-CD19C-003 showed increased levels of CD3 Zeta in the presence of ligands, Shield-1 and TMP respectively, indicating the stabilization of the CD19 CAR (FIG. 23A). As shown in FIG. 23B, OT- CD19C-007 showed an increase in CD3 Zeta levels in the presence of Shield-1 and Methotrexate, indicating a ligand-dependent stabilization of CD19 CAR. As expected, the constutively expressed, OT-CD19C-001 showed strong expression of CD19 CAR in the absence of ligand treatment.

Lysates from cells expressing CD19 CAR constructs were also immunoblotted for 4 1-BB, a component of the CAR. As shown in FIG. 23C, OT-CD19C-003, OT-CD19C-006 and OT-CD19C-007 showed increase in 4-1BB expression levels with the treatment of corresponding ligands- TMP and Shield-1, as compared to 4-1BB levels in the absence of ligand, indicating a ligand dependent stabilization of CD19 CAR.

Surface expression of DD-CD19 CAR constructs in HEK 293T cells was measured using Fluorescence activated cell sorting (FACS) with Protein L-Biotin-Strepavidin-Allophycocyanin which binds to the kappa light chain of the CAR (ThermoFisher Scientific, Waltham, MA). Cells were treated with 1µM Shield-1, 1µM Methotrexate, 10µM Trimethoprim or vehicle control for 24 hours and subject to FACS analysis. As shown in FIG. 23D, surface expression of OT-CD19C-002 with FKBP-DD was detected only in the presence of Shield-1, while OT-CD19C-003 with ecDHFR-DD showed surface expression only in the presence of Trimethoprim. As expected, constitutively expressed construct OT-C19C-001 showed high expression both in ligand and control vehicle treated cells.

### MISCELLANEOUS

In the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or the entire group members are present in, employed in, or otherwise relevant to a given product or process.

It is also noted that the term "comprising" is intended to be open and permits but does not require the inclusion of additional elements or steps. When the term "comprising" is used herein, the term "consisting of' is thus also encompassed and disclosed.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

## Claims

1. An effector module comprising
a first component and a second component, wherein said first component is a stimulus response element (SRE) comprising an FKBP or E.coli DHFR (ecDHFR) destabilizing domain (DD), wherein the destabilizing domain is a FKBP destabilizing domain that comprises mutations F36V and L106P and comprises SEQ ID NO: 213274; a FKBP destabilizing domain that comprises mutations E31G, F36V, R71G, and K105E, and comprises SEQ ID NO: 213297; a ecDHFR destabilizing domain that comprises mutations R12Y and Y100I and comprises SEQ ID NO: 213275; a ecDHFR destabilizing domain that comprises R12H and E129K and comprises SEQ ID NO: 213410; or a sequence having at least 95% sequence identity to any of said amino acid sequences
and
wherein said second component is a payload construct comprising a CD19 CAR payload; and wherein said effector module is responsive to at least one stimulus.

2. The effector module of claim 1, wherein the destabilizing domain is selected from the group consisting of a FKBP destabilizing domain that comprises mutations F36V and L106P and comprises SEQ ID NO: 213274; a FKBP destabilizing domain that comprises mutations E31G, F36V, R71G, and K105E, and comprises SEQ ID NO: 213297; a ecDHFR destabilizing domain that comprises mutations R12Y and Y100I and comprises SEQ ID NO: 213275; and a ecDHFR destabilizing domain that comprises R12H and E129K and comprises SEQ ID NO: 213410.

3. The effector module of claim 1, wherein the effector module comprises one of the amino acid sequences listed in Table 10.

4. The effector module of claim 1, wherein:
(a) the effector module further comprises a signal sequence selected from those listed in Table 2; wherein, optionally:
(i) the effector module further comprises a cleavage and/or processing feature selected from those listed in Table 3;
(ii) the effector module further comprises a targeting and/or penetrating peptide selected from those listed in Tables 5 or 6; and/or
(iii) the effector module further comprises a linker selected from those listed in Tables 7 and 8;
(b) the effector module further comprises a cleavage and/or processing feature selected from those listed in Table 3; the effector module further comprises a targeting and/or penetrating peptide selected from those listed in Tables 5 or 6; and/or the effector module further comprises a linker selected from those listed in Tables 7 and 8; or
(c) the effector module comprises an organizational pattern selected from the group of those given in Figures 2, 3, 4, 5, and 6.

5. The effector module of any one of claims 1-4, wherein the at least one stimulus is selected from the group comprising: Shield-1, Methotrexate (MTX), and Trimethoprim (TMP).

6. A polynucleotide encoding the effector module of any one of claims 1-5.

7. The polynucleotide of claim 6, wherein:
(a) at least one region of said polynucleotide is codon optimized; wherein, optionally, the region encoding said first component of the effector module is codon optimized, or the region encoding said second component of the effector module is codon optimized; or
(b) the polynucleotide is a messenger RNA (mRNA).

8. An expression vector comprising the polynucleotide of any of claims 6-7, wherein, optionally, the expression vector is a viral vector selected from a lentiviral vector, adenoviral vector, adeno-associated viral (AAV) vector, herpes simplex viral vector, retroviral vector, and oncolytic viral vector.

9. A cell comprising the expression vector of claim 8.

10. A pharmaceutical composition comprising:
(i) the effector module of any one of claims 1-5;
(ii) the polynucleotide of any one of claims 6-7;
(iii) the expression vector of claim 8; or
(iv) the cell of claim 9;
wherein the pharmaceutical composition is for use in a method of treating a disease or disorder in a subject, comprising administration of the pharmaceutical composition; wherein, optionally, the pharmaceutical composition is administered via an intravenous (into a vein) route.

11. A regulatable human T cell or T cell population engineered to express an effector module, wherein,
the effector module comprises a chimeric antigen receptor (CAR) that recognizes a CD19 antigen and a FKBP or ecDHFR destabilizing domain (DD), wherein the destabilizing domain is a FKBP destabilizing domain that comprises mutations F36V and L106P and comprises SEQ ID NO: 213274; a FKBP destabilizing domain that comprises mutations E31G, F36V, R71G, and K105E, and comprises SEQ ID NO: 213297; a ecDHFR destabilizing domain that comprises mutations R12Y and Y100I and comprises SEQ ID NO: 213275; a ecDHFR destabilizing domain that comprises R12H and E129K and comprises SEQ ID NO: 213410; or a sequence having at least 95% sequence identity to any of said amino acid sequences;
wherein optionally the destabilizing domain is defined as in claim 2, or the effector module is defined as in claim 3, and
wherein, optionally:
(a) the T-cells are primary T-cells;
(b) the T cell is selected from the group consisting of cytotoxic T-cells, helper T-cells, memory T-cells, regulatory T-cells, tissue infiltrating lymphocytes and combinations thereof; or
(c) the cell population is obtained from a subject suffering from, being treated for, diagnosed with, at risk of developing, or suspected of having a disorder selected from the group consisting of a hyperproliferative condition (including cancer), an immune disorder (including an autoimmune disorder), and graft vs. host disease.

12. A method of producing a regulatable human T-cell or population thereof, the method comprising,
(a) contacting an isolated population of T-cells with a polynucleotide encoding one or more effector modules such that the effector module can be expressed in the contacted population;
(b) causing the level of the payload encoded by said one or more effector modules to be modulated upon exposure of the expressed effector module with one or more stimuli;
wherein at least one of the one or more effector modules comprises a stimulus response element (SRE) and a payload construct, wherein the payload construct comprises a CD19 CAR payload and the SRE comprises a FKBP or ecDHFR destabilizing domain (DD), wherein the destabilizing domain is a FKBP destabilizing domain that comprises mutations F36V and L106P and comprises SEQ ID NO: 213274; a FKBP destabilizing domain that comprises mutations E31G, F36V, R71G, and K105E, and comprises SEQ ID NO: 213297; a ecDHFR destabilizing domain that comprises mutations R12Y and Y100I and comprises SEQ ID NO: 213275; a ecDHFR destabilizing domain that comprises R12H and E129K and comprises SEQ ID NO: 213410; or a sequence having at least 95% sequence identity to any of said amino acid sequences,
wherein optionally the destabilizing domain is defined as in claim 2, or the effector module is defined as in claim 3,
wherein, optionally:
the level of the payload encoded by said one or more effector modules is upregulated upon exposure to the stimulus.

13. The method of claim 12, wherein the one or more stimuli is selected from the group comprising: Shield-1, Methotrexate (MTX), and Trimethoprim (TMP).

14. The regulatable human T cell or T cell population of claim 11 for use in a method of treating a patient in need thereof, the method comprising administration of the regulatable human T cell or T cell population; wherein, optionally:
(a) the treatment comprises adoptive immunotherapy; wherein, further optionally, the method further comprises expanding the regulatable human T cell or T cell population prior to the step of administration; or
(b) the method further comprises expanding the regulatable human T cell or T cell population prior to the step of administration.

## Patentansprüche

1. Effektormodul, umfassend:
eine erste Komponente und eine zweite Komponente, wobei die erste Komponente ein Stimulus-Response-Element (SRE) ist, umfassend eine FKBP- oder E-coli-DHFR(ecDHFR)-destabilisierende Domäne (DD), wobei die destabilisierende Domäne eine FKBP-destabilisierende Domäne ist, die die Mutationen F36V und L106P umfasst und SEQ ID NO: 213274 umfasst; eine FKBP-destabilisierende Domäne, die die Mutationen E31G, F36V, R71G und K105E umfasst und SEQ ID NO: 213297 umfasst; eine ecDHFR-destabilisierende Domäne, die die Mutationen R12Y und Y100I umfasst und SEQ ID NO: 213275 umfasst; eine ecDHFR-destabilisierende Domäne, die R12H und E129K umfasst und SEQ ID NO: 213410 umfasst; oder eine Sequenz, die mindestens 95 % Sequenzidentität zu einer der Aminosäuresequenzen aufweist
und
wobei die zweite Komponente ein Nutzlastkonstrukt ist, das eine CD19-CAR-Nutzlast umfasst; und wobei das Effektormodul auf mindestens einen Stimulus anspricht.

2. Effektormodul nach Anspruch 1, wobei die destabilisierende Domäne aus der Gruppe bestehend aus einer FKBPdestabilisierenden Domäne, die die Mutationen F36V und L106P umfasst und SEQ ID NO: 213274 umfasst; einer FKBPdestabilisierenden Domäne, die die Mutationen E31G, F36V, R71G und K105E umfasst und SEQ ID NO: 213297 umfasst; einer ecDHFRdestabilisierenden Domäne, die Mutationen R12Y umfasst und Y100I und SEQ ID NO: 213275 umfasst; und einer ecDHFR-destabilisierende Domäne, die R12H und E129K umfasst und SEQ ID NO: 213410 umfasst, ausgewählt ist.

3. Effektormodul nach Anspruch 1, wobei das Effektormodul eine der in Tabelle 10 aufgeführten Aminosäuresequenzen umfasst.

4. Effektormodul nach Anspruch 1, wobei:
(a) das Effektormodul ferner eine Signalfolge umfasst, die aus den in Tabelle 2 aufgeführten ausgewählt ist; wobei optional:
(i) das Effektormodul ferner ein Spaltungs- und/oder Verarbeitungsmerkmal umfasst, das aus den in Tabelle 3 aufgeführten ausgewählt ist;
(ii) das Effektormodul ferner ein zielgerichtetes und/oder penetrierendes Peptid umfasst, das aus den in den Tabellen 5 oder 6 aufgeführten ausgewählt ist; und/oder
(iii) das Effektormodul ferner einen Linker umfasst, der aus den in den Tabellen 7 und 8 aufgeführten ausgewählt ist;
(b) das Effektormodul ferner ein Spaltungs- und/oder Verarbeitungsmerkmal umfasst, das aus den in Tabelle 3 aufgeführten ausgewählt ist; das Effektormodul ferner ein zielgerichtetes und/oder penetrierendes Peptid umfasst, das aus den in den Tabellen 5 oder 6 aufgeführten ausgewählt ist; und/oder das Effektormodul ferner einen Linker umfasst, der aus den in den Tabellen 7 und 8 aufgeführten ausgewählt ist; oder
(c) das Effektormodul ein Organisationsmuster umfasst, das aus der in den Figuren 2, 3, 4, 5 und 6 angegebenen Gruppe ausgewählt ist.

5. Effektormodul nach einem der Ansprüche 1-4, wobei der mindestens ein Stimulus aus der Gruppe der folgenden ausgewählt ist: Shield-1, Methotrexat (MTX) und Trimethoprim (TMP) .

6. Polynukleotid, das für das Effektormodul nach einem der Ansprüche 1-5 codiert.

7. Polynukleotid nach Anspruch 6, wobei:
(a) mindestens eine Region des Polynukleotids Codon-optimiert ist; wobei optional die Region, die für die erste Komponente des Effektormoduls codiert, Codon-optimiert ist, oder die Region, die für die zweite Komponente des Effektormoduls codiert, Codon-optimiert ist; oder
(b) das Polynukleotid eine Boten-RNA (mRNA) ist.

8. Expressionsvektor, umfassend das Polynukleotid nach einem der Ansprüche 6-7, wobei optional der Expressionsvektor ein viraler Vektor ist, ausgewählt aus einem lentiviralen Vektor, adenoviralen Vektor, adeno-assoziierten viralen (AAV) Vektor, Herpes-Simplex-viralen Vektor, retroviralen Vektor und onkolytischen viralen Vektor.

9. Zelle, umfassend den Expressionsvektor nach Anspruch 8.

10. Pharmazeutische Zusammensetzung, umfassend:
(i) das Effektormodul nach einem der Ansprüche 1-5;
(ii) das Polynukleotid nach einem der Ansprüche 6-7;
(iii) den Expressionsvektor nach Anspruch 8; oder
(iv) die Zelle nach Anspruch 9;
wobei die pharmazeutische Zusammensetzung zur Verwendung in einem Verfahren zur Behandlung einer Krankheit oder eines Leidens eines Patienten, umfassend die Verabreichung der pharmazeutischen Zusammensetzung, bestimmt ist, wobei die pharmazeutische Zusammensetzung optional auf intravenösem Weg (in eine Vene) verabreicht wird.

11. Regulationsfähige humane T-Zelle oder T-Zell-Population, die ein Effektormodul exprimiert, wobei
das Effektormodul einen chimären Antigenrezeptor (CAR) umfasst, der ein CD19-Antigen und eine FKBP- oder ecDHFR - destabilisierende Domäne (DD) erkennt, wobei die destabilisierende Domäne eine FKBP-destabilisierende Domäne ist, die die Mutationen F36V und L106P umfasst und SEQ ID NO: 213274 umfasst; eine FKBP-destabilisierende Domäne, die die Mutationen E31G, F36V, R71G und K105E umfasst und SEQ ID NO: 213297 umfasst; eine ecDHFR-destabilisierende Domäne, die die Mutationen R12Y und Y100I umfasst und SEQ ID NO: 213275 umfasst; eine ecDHFR-destabilisierende Domäne, die R12H und E129K umfasst und SEQ ID NO: 213410 umfasst; oder eine Sequenz, die mindestens 95 % Sequenzidentität zu einer der Aminosäuresequenzen aufweist;
wobei optional die destabilisierende Domäne wie in Anspruch 2 definiert ist, oder das Effektormodul wie in Anspruch 3 definiert ist, und
wobei, optional:
(a) die T-Zellen primäre T-Zellen sind;
(b) die T-Zelle aus der Gruppe bestehend aus zytotoxischen T-Zellen, Helfer-T-Zellen, Gedächtnis-T-Zellen, regulatorischen T-Zellen, gewebeinfiltrierenden Lymphozyten und Kombinationen davon ausgewählt ist; oder
(c) die Zellpopulation von einer Person mit einem Leiden erlangt wird, gegen das sie behandelt wird, bei dem sie ein Risiko für die Entwicklung dieses Leidens hat oder bei dem das Vorliegen eines Leidens vermutet wird, das Leiden aus der Gruppe bestehend aus einem hyperproliferativen Zustand (einschließlich Krebs), einer Immunstörung (einschließlich einer Autoimmunerkrankung) und Graft-vs.-Host-Erkrankung.

12. Verfahren zur Herstellung einer regulationsfähigen humanen T-Zelle oder einer Population davon, das Verfahren umfassend,
(a) Inkontaktbringen einer isolierten Population von T-Zellen mit einem Polynukleotid, das für ein oder mehrere Effektormodule codiert, derart, dass das Effektormodul in der kontaktierten Population exprimiert werden kann;
(b) Veranlassen, dass das Ausmaß der von dem einen oder den mehreren Effektormodulen kodierten Nutzlast moduliert wird, wenn das exprimierte Effektormodul einem oder mehreren Stimuli ausgesetzt wird;
wobei mindestens eines des einen oder der mehreren Effektormodule ein Stimulus-Response-Element (SRE) und ein Nutzlastkonstrukt umfasst, wobei das Nutzlastkonstrukt eine CD19-CAR-Nutzlast umfasst und das SRE eine FKBP- oder ecDHFR - destabilisierende Domäne (DD) umfasst, wobei die destabilisierende Domäne eine FKBP-destabilisierende Domäne ist, die die Mutationen F36V und L106P umfasst und SEQ ID NO: 213274 umfasst; eine FKBP-destabilisierende Domäne, die die Mutationen E31G, F36V, R71G und K105E umfasst und SEQ ID NO: 213297 umfasst; eine ecDHFR-destabilisierende Domäne, die die Mutationen R12Y und Y100I umfasst und SEQ ID NO: 213275 umfasst; eine ecDHFR-destabilisierende Domäne, die R12H und E129K umfasst und SEQ ID NO: 213410 umfasst; oder eine Sequenz, die mindestens 95 % Sequenzidentität zu einer der Aminosäuresequenzen aufweist,
wobei optional die destabilisierende Domäne wie in Anspruch 2 definiert ist, oder das Effektormodul wie in Anspruch 3 definiert ist,
wobei, optional:
das Ausmaß der von dem einen oder den mehreren Effektormodulen kodierten Nutzlast bei Einwirkung des Stimulus hochreguliert wird.

13. Verfahren nach Anspruch 12, wobei der eine oder die mehreren Stimuli ausgewählt der Gruppe der folgenden angehört: Shield-1, Methotrexat (MTX) und Trimethoprim (TMP).

14. Regulationsfähige humane T-Zelle oder T-Zell-Population nach Anspruch 11 zur Verwendung in einem Verfahren zur Behandlung eines Patienten, der diese benötigt, wobei das Verfahren die Verabreichung der regulationsfähigen humanen T-Zelle oder T-Zell-Population umfasst; wobei, optional:
(a) die Behandlung eine adoptive Immuntherapie umfasst; wobei das Verfahren ferner optional umfasst, dass die regulationsfähige humane T-Zelle oder T-Zell-Population vor dem Schritt der Verabreichung expandiert wird; oder
(b) das Verfahren ferner ein Expandieren der regulationsfähigen humanen T-Zelle oder T-Zell-Population vor dem Schritt der Verabreichung umfasst.

## Revendications

1. Module effecteur comprenant
un premier composant et un second composant, dans lequel ledit premier composant est un élément de réponse au stimulus (SRE) comprenant un domaine déstabilisant (DD) de FKBP ou de DHFR de E.coli (ecDHFR), dans lequel le domaine déstabilisant étant un domaine déstabilisant de FKBP qui comprend les mutations F36V et L106P et comprend la SEQ ID NO : 213274 ; un domaine déstabilisant de FKBP qui comprend les mutations E31G, F36V, R71G et K105E et comprend la SEQ ID NO : 213297 ; un domaine déstabilisant d'ecDHFR qui comprend les mutations R12Y et Y100I et comprend la SEQ ID NO : 213275 ; un domaine déstabilisant d'ecDHFR qui comprend R12H et E129K et comprend la SEQ ID NO : 213410 ; ou une séquence ayant au moins 95 % d'identité de séquence avec l'une quelconque desdites séquences d'acides aminés
et
dans lequel ledit second composant est une construction de charge utile comprenant une charge utile de CAR de CD19 ; et dans lequel ledit module effecteur est sensible à au moins un stimulus.

2. Module effecteur selon la revendication 1, dans lequel le domaine déstabilisant est choisi dans le groupe constitué par un domaine déstabilisant de FKBP qui comprend les mutations F36V et L106P et comprend la SEQ ID NO : 213274 ; un domaine déstabilisant de FKBP qui comprend les mutations E31G, F36V, R71G et K105E et comprend la SEQ ID NO : 213297 ; un domaine déstabilisant d'ecDHFR qui comprend les mutations R12Y et Y100I et comprend la SEQ ID NO : 213275 ; et un domaine déstabilisant d'ecDHFR qui comprend R12H et E129K et comprend la SEQ ID NO : 213410.

3. Module effecteur selon la revendication 1, dans lequel le module effecteur comprend l'une des séquences d'acides aminés répertoriées dans le tableau 10.

4. Module effecteur selon la revendication 1, dans lequel :
(a) le module effecteur comprend également une séquence de signal choisie parmi celles répertoriées dans le tableau 2 ; dans lequel, éventuellement :
(i) le module effecteur comprend également une caractéristique de clivage et/ou de traitement choisie parmi celles répertoriées dans le tableau 3 ;
(ii) le module effecteur comprend également un peptide de ciblage et/ou de pénétration choisi parmi ceux énumérés dans les tableaux 5 ou 6 ; et/ou
(iii) le module effecteur comprend également un lieur choisi parmi ceux répertoriés dans les tableaux 7 et 8 ;
(b) le module effecteur comprend également une caractéristique de clivage et/ou de traitement choisie parmi celles répertoriées dans le tableau 3 ; le module effecteur comprend également un peptide de ciblage et/ou de pénétration choisi parmi ceux répertoriés dans les tableaux 5 ou 6 ; et/ou le module effecteur comprend également un lieur choisi parmi ceux répertoriés dans les tableaux 7 et 8 ; ou
(c) le module effecteur comprend un modèle d'organisation choisi dans le groupe de ceux donnés dans les figures 2, 3, 4, 5 et 6.

5. Module effecteur selon l'une quelconque des revendications 1 à 4, dans lequel l'au moins un stimulus est choisi dans le groupe comprenant : Shield-1, Méthotrexate (MTX) et Triméthoprime (TMP).

6. Polynucléotide codant pour le module effecteur selon l'une quelconque des revendications 1 à 5.

7. Polynucléotide selon la revendication 6, dans lequel :
(a) au moins une région dudit polynucléotide est optimisée par codons ; dans lequel, éventuellement, la région codant pour ledit premier composant du module effecteur est optimisée par codons, ou la région codant pour ledit second composant du module effecteur est optimisée par codons ; ou
(b) le polynucléotide est un ARN messager (ARNm).

8. Vecteur d'expression comprenant le polynucléotide selon l'une quelconque des revendications 6 et 7, dans lequel, éventuellement, le vecteur d'expression est un vecteur viral choisi parmi un vecteur lentiviral, un vecteur adénoviral, un vecteur viral adéno-associé (AAV), un vecteur viral de l'herpès simplex, un vecteur rétroviral et un vecteur viral oncolytique.

9. Cellule comprenant le vecteur d'expression selon la revendication 8.

10. Composition pharmaceutique comprenant :
(i) le module effecteur selon l'une quelconque des revendications 1 à 5 ;
(ii) le polynucléotide selon l'une quelconque des revendications 6 et 7 ;
(iii) le vecteur d'expression selon la revendication 8 ; ou
(iv) la cellule selon la revendication 9 ;
dans laquelle la composition pharmaceutique est pour une utilisation dans un procédéde traitement d'une maladie ou d'un trouble chez un sujet, comprenant l'administration de la composition pharmaceutique ; dans laquelle, éventuellement, la composition pharmaceutique est administrée par voie intraveineuse (dans une veine).

11. Cellule T humaine régulable ou une population de cellules T conçue pour exprimer un module effecteur, dans laquelle,
le module effecteur comprend un récepteur d'antigène chimérique (CAR) qui reconnait un antigène de CD19 et un domaine déstabilisant (DD) de FKBP ou d'ecDHFR, dans laquelle le domaine déstabilisant est un domaine déstabilisant de FKBP qui comprend les mutations F36V et L106P et comprend la SEQ ID NO : 213274 ; un domaine déstabilisant de FKBP qui comprend les mutations E31G, F36V, R71G et K105E et comprend la SEQ ID NO : 213297 ; un domaine déstabilisant d'ecDHFR qui comprend les mutations R12Y et Y100I et comprend la SEQ ID NO : 213275 ; un domaine déstabilisant d'ecDHFR qui comprend R12H et E129K et comprend la SEQ ID NO : 213410 ; ou une séquence ayant au moins 95 % d'identité de séquence avec l'une quelconque desdites séquences d'acides aminés ;
dans laquelle éventuellement le domaine déstabilisant est défini comme dans la revendication 2, ou le module effecteur est défini comme dans la revendication 3, et
dans lequel, éventuellement :
(a) les cellules T sont des cellules T primaires ;
(b) la cellule T est choisie dans le groupe constitué par les cellules T cytotoxiques, les cellules T auxiliaires, les cellules T mémoires, les cellules T régulatrices, les cellules infiltrant les tissus et des combinaisons de celles-ci ; ou
(c) la population de cellules est obtenue à partir d'un sujet souffrant, traité, diagnostiqué, risquant de développer ou suspecté d'avoir un trouble choisi dans le groupe constitué d'une condition hyperproliférative (y compris le cancer), d'un trouble immunitaire (y compris un trouble auto-immun) et d'une maladie du greffon contre l'hôte.

12. Procédé de production d'une cellule T humaine régulable ou d'une population de celles-ci, le procédé comprenant,
(a) la mise en contact d'une population isolée de cellules T avec un polynucléotide codant pour un ou plusieurs modules effecteurs de telle sorte que le module effecteur puisse être exprimé dans la population contactée ;
(b) le fait d'amener la modulation du niveau de la charge utile codée par ledit un ou lesdits plusieurs modules effecteurs lors de l'exposition du module effecteur exprimé à un ou plusieurs stimuli ;
dans lequel à moins un de l'un ou des plusieurs modules effecteurs comprend un élément de réponse au stimulus (SRE) et une construction de charge utile dans lequel la construction de charge utile comprend une charge utile de CAR de CD19 et le SRE comprend un domaine déstabilisant (DD) de FKBP ou d'ecDHFR, dans lequel le domaine déstabilisant est un domaine déstabilisant de FKBP qui comprend les mutations F36V et L106P et comprend la SEQ ID NO : 213274 ; un domaine déstabilisant de FKBP qui comprend les mutations E31G, F36V, R71G et K105E et comprend la SEQ ID NO : 213297 ; un domaine déstabilisant d'ecDHFR qui comprend les mutations R12Y et Y100I et comprend la SEQ ID NO : 213275 ; un domaine déstabilisant d'ecDHFR qui comprend R12H et E129K et comprend la SEQ ID NO : 213410 ; ou une séquence ayant au moins 95 % d'identité de séquence avec l'une quelconque desdites séquences d'acides aminés,
dans lequel éventuellement le domaine déstabilisant est défini comme dans la revendication 2, ou le module effecteur est défini comme dans la revendication 3,
dans lequel, éventuellement :
le niveau de la charge utile codée par ledit un ou lesdits plusieurs modules effecteurs est régulé à la hausse lors de l'exposition au stimulus.

13. Procédé selon la revendication 12, dans lequel l'un ou les plusieurs stimuli sont choisis dans le groupe comprenant : Shield-1, Méthotrexate (MTX) et Triméthoprime (TMP).

14. Cellule T humaine régulable ou population de cellules T selon la revendication 11, pour une utilisation dans un procédé de traitement d'un patient en ayant besoin, le procédé comprenant l'administration de la cellule T humaine régulable ou de la population de cellules T ; dans laquelle, éventuellement :
(a) le traitement comprend une immunothérapie adoptive ; dans laquelle, également, facultativement, le procédé comprend l'expansion de la cellule T humaine régulable ou de la population de cellules T avant l'étape d'administration ; ou
(b) le procédé comprend également l'expansion de la cellule T humaine régulable ou de la population de cellules T avant l'étape d'administration.
